# EUROPEAN PATENT APPLICATION

(11) **EP 2 261 367 A2**
(43) Date of publication of application: **15.12.2010**
(21) Application number: 10008262.7
(22) Date of filing: 26.11.2008
(51) Int. Cl.: C12Q 1/68

(54) **Gene expression markers for inflammatory bowel disease**

(30) Priority: 29.11.2007 US 991203 P; 22.05.2008 WO PCT/US2008/064562; 17.09.2008 US 192268 P
(62) Divisional of application: 08855762.4
(71) Applicant: Genentech, Inc., South San Francisco CA 94080-4990 (US); The University Court of The University of Edinburgh of Old College, Edinburgh EH8 9YL (GB)
(72) Inventor: Abbas, Alexander R., San Carlos, CA 94070 (US); Clark, Hilary, San Francisco, CA 94131 (US); Diehl, Lauri, Los Altos, CA 94024 (US); Lees, Charles, Linlithgow, West Lothian EH49 7EL Scotland (GB); Noble, Colin L., Edinburgh Midlothian EH5 3QF Scotland (GB); Satsangi, Jack, Edinburgh Midlothian EH10 5ET Scotland (GB)
(74) Representative: Walton, Seán Malcolm

(57) **Abstract**

The present invention relates to methods of gene expression profiling for inflammatory bowel disease pathogenesis, in which the differential expression in a test sample from a mammalian subject of one or more IBD markers relative to a control is determined, wherein the differential expression in the text sample is indicative of an IBD in the mammalian subject from which the test sample was obtained.

SEQUENCE LISTING

<110> GENENTECH, INC; The University Court of The University of Edinburgh of Old College.

<120> GENE EXPRESSION MARKERS FOR INFLAMMATORY BOWEL DISEASE

<130> SMW/FP6706188

<140> EP
<141> 2008-11-26

<150> EP 08855762.4
<151> 2008-11-26

<150> PCT/US08/85045
<151> 2008-11-26

<150> 61/192,268
<151> 2008-09-17

<150> PCT/US08/64562
<151> 2008-05-22

<150> 60/991,203
<151> 2007-11-29

<160> 269

<170> PatentIn version 3.5

<210> 1
<211> 475
<212> DNA
<213> Homo sapiens

<400> 1
acacatctgc tcctgctctc tctcctccag cgaccctagc catgagaacc ctcaccatcc 60

tcactgctgt tctcctcgtg gccctccagg ccaaggctga gccactccaa gctgaggatg 120

atccactgca ggcaaaagct tatgaggctg atgcccagga gcagcgtggg gcaaatgacc 180

aggactttgc cgtctccttt gcagaggatg caagctcaag tcttagagct ttgggctcaa 240

caagggcttt cacttgccat tgcagaaggt cctgttattc aacagaatat tcctatggga 300

cctgcactgt catgggtatt aaccacagat tctgctgcct ctgagggatg agaacagaga 360

gaaatatatt cataatttac tttatgacct agaaggaaac tgtcgtgtgt cccatacatt 420

gccatcaact ttgtttcctc atctcaaata aagtcctttc agcaaaaaaa aaaaa 475

<210> 2
<211> 100
<212> PRT
<213> Homo sapiens

<400> 2
Met Arg Thr Leu Thr Ile Leu Thr Ala Val Leu Leu Val Ala Leu Gln

1               5                   10                  15

Ala Lys Ala Glu Pro Leu Gln Ala Glu Asp Asp Pro Leu Gln Ala Lys
            20                  25                  30

Ala Tyr Glu Ala Asp Ala Gln Glu Gln Arg Gly Ala Asn Asp Gln Asp
        35                  40                  45

Phe Ala Val Ser Phe Ala Glu Asp Ala Ser Ser Ser Leu Arg Ala Leu
    50                  55                  60

Gly Ser Thr Arg Ala Phe Thr Cys His Cys Arg Arg Ser Cys Tyr Ser
65                  70                  75                  80

Thr Glu Tyr Ser Tyr Gly Thr Cys Thr Val Met Gly Ile Asn His Arg
                85                  90                  95

Phe Cys Cys Leu
            100

<210> 3
<211> 449
<212> DNA
<213> Homo sapiens

<400> 3
atatccactc ctgctctccc tcctgcaggt gaccccagcc atgaggacca tcgccatcct      60

tgctgccatt ctcctggtgg ccctgcaggc ccaggctgag tcactccagg aaagagctga     120

tgaggctaca acccagaagc agtctgggga agacaaccag gaccttgcta tctcctttgc     180

aggaaatgga ctctctgctc ttagaacctc aggttctcag gcaagagcca cctgctattg     240

ccgaaccggc cgttgtgcta cccgtgagtc cctctccggg gtgtgtgaaa tcagtggccg     300

cctctacaga ctctgctgtc gctgagcttc ctagatagaa accaaagcag tgcaagattc     360

agttcaaggt cctgaaaaaa gaaaaacatt ttactctgtg taccttgtgt ctttctaaat     420

ttctctctcc aaaataaagt tcaagcatt                                       449

<210> 4
<211> 94
<212> PRT
<213> Homo sapiens

<400> 4
Met Arg Thr Ile Ala Ile Leu Ala Ala Ile Leu Leu Val Ala Leu Gln
1               5                   10                  15

Ala Gln Ala Glu Ser Leu Gln Glu Arg Ala Asp Glu Ala Thr Thr Gln

                20                  25                  30

Lys Gln Ser Gly Glu Asp Asn Gln Asp Leu Ala Ile Ser Phe Ala Gly
        35                  40                  45

Asn Gly Leu Ser Ala Leu Arg Thr Ser Gly Ser Gln Ala Arg Ala Thr
    50                  55                  60

Cys Tyr Cys Arg Thr Gly Arg Cys Ala Thr Arg Glu Ser Leu Ser Gly
65                  70                  75                  80

Val Cys Glu Ile Ser Gly Arg Leu Tyr Arg Leu Cys Cys Arg
                85                  90

<210> 5
<211> 1726
<212> DNA
<213> Homo sapiens

<400> 5
gtcaggttca tggttacgaa gctgctgacc ccaggatccc agcccgtggg agagaagggg      60

gtctctgaca gcccccaccc ctccccactg ccagatcctt attgggtctg agtttcaggg     120

gtggggcccc agctggaggt tataaaacag ctcaatcggg gagtacaacc ttcggtttct     180

cttcggggaa agctgctttc agcgcacacg ggaagatatc agaaacatcc taggatcagg     240

acaccccaga tcttctcaac tggaaccacg aaggctgttt cttccacaca gcactttgat     300

ctccatttaa gcaggcacct ctgtcctgcg ttccggagct gcgttcccga tggtcctcct     360

ttggctcacg ctgctcctga tcgccctgcc ctgtctcctg caaacgaagg aagatccaaa     420

cccaccaatc acgaacctaa ggatgaaagc aaaggctcag cagttgacct gggaccttaa     480

cagaaatgtg accgatatcg agtgtgttaa agacgccgac tattctatgc cggcagtgaa     540

caatagctat tgccagtttg gagcaatttc cttatgtgaa gtgaccaact acaccgtccg     600

agtggccaac ccaccattct ccacgtggat cctcttccct gagaacagtg ggaagccttg     660

ggcaggtgcg gagaatctga cctgctggat tcatgacgtg gatttcttga gctgcagctg     720

ggcggtaggc ccgggggccc ccgcggacgt ccagtacgac ctgtacttga acgttgccaa     780

caggcgtcaa cagtacgagt gtcttcacta caaaacggat gctcagggaa cacgtatcgg     840

gtgtcgtttc gatgacatct ctcgactctc cagcggttct caaagttccc acatcctggt     900

gcggggcagg agcgcagcct tcggtatccc ctgcacagat aagtttgtcg tcttttcaca     960

gattgagata ttaactccac ccaacatgac tgcaaagtgt aataagacac attcctttat    1020

gcactggaaa atgagaagtc atttcaatcg caaatttcgc tatgagcttc agatacaaaa    1080

gagaatgcag cctgtaatca cagaacaggt cagagacaga acctccttcc agctactcaa 1140

tcctggaacg tacacagtac aaataagagc ccgggaaaga gtgtatgaat tcttgagcgc 1200

ctggagcacc ccccagcgct tcgagtgcga ccaggaggag ggcgcaaaca cacgtgcctg 1260

gcggacgtcg ctgctgatcg cgctggggac gctgctggcc ctggtctgtg tcttcgtgat 1320

ctgcagaagg tatctggtga tgcagagact ctttccccgc atccctcaca tgaaagaccc 1380

catcggtgac agcttccaaa acgacaagct ggtggtctgg gaggcgggca aagccggcct 1440

ggaggagtgt ctggtgactg aagtacaggt cgtgcagaaa acttgagact ggggttcagg 1500

gcttgtgggg gtctgcctca atctccctgg ccgggccagg cgcctgcaca gactggctgc 1560

tggacctgcg cacgcagccc aggaatggac attcctaacg ggtggtgggc atgggagatg 1620

cctgtgtaat ttcgtccgaa gctgccagga agaagaacag aactttgtgt gtttatttca 1680

tgataaagtg attttttttt ttttaaccca aaaaaaaaaa aaaaaa 1726

<210> 6
<211> 378
<212> PRT
<213> Homo sapiens

<400> 6
Met Val Leu Leu Trp Leu Thr Leu Leu Leu Ile Ala Leu Pro Cys Leu
1               5                   10                  15

Leu Gln Thr Lys Glu Asp Pro Asn Pro Pro Ile Thr Asn Leu Arg Met
            20                  25                  30

Lys Ala Lys Ala Gln Gln Leu Thr Trp Asp Leu Asn Arg Asn Val Thr
        35                  40                  45

Asp Ile Glu Cys Val Lys Asp Ala Asp Tyr Ser Met Pro Ala Val Asn
    50                  55                  60

Asn Ser Tyr Cys Gln Phe Gly Ala Ile Ser Leu Cys Glu Val Thr Asn
65                  70                  75                  80

Tyr Thr Val Arg Val Ala Asn Pro Pro Phe Ser Thr Trp Ile Leu Phe
                85                  90                  95

Pro Glu Asn Ser Gly Lys Pro Trp Ala Gly Ala Glu Asn Leu Thr Cys
            100                 105                 110

Trp Ile His Asp Val Asp Phe Leu Ser Cys Ser Trp Ala Val Gly Pro
        115                 120                 125

Gly Ala Pro Ala Asp Val Gln Tyr Asp Leu Tyr Leu Asn Val Ala Asn
    130                 135                 140

Arg Arg Gln Gln Tyr Glu Cys Leu His Tyr Lys Thr Asp Ala Gln Gly
145                 150                 155                 160

Thr Arg Ile Gly Cys Arg Phe Asp Asp Ile Ser Arg Leu Ser Ser Gly
                165                 170                 175

Ser Gln Ser Ser His Ile Leu Val Arg Gly Arg Ser Ala Ala Phe Gly
            180                 185                 190

Ile Pro Cys Thr Asp Lys Phe Val Val Phe Ser Gln Ile Glu Ile Leu
        195                 200                 205

Thr Pro Pro Asn Met Thr Ala Lys Cys Asn Lys Thr His Ser Phe Met
    210                 215                 220

His Trp Lys Met Arg Ser His Phe Asn Arg Lys Phe Arg Tyr Glu Leu
225                 230                 235                 240

Gln Ile Gln Lys Arg Met Gln Pro Val Ile Thr Glu Gln Val Arg Asp
                245                 250                 255

Arg Thr Ser Phe Gln Leu Leu Asn Pro Gly Thr Tyr Thr Val Gln Ile
            260                 265                 270

Arg Ala Arg Glu Arg Val Tyr Glu Phe Leu Ser Ala Trp Ser Thr Pro
        275                 280                 285

Gln Arg Phe Glu Cys Asp Gln Glu Glu Gly Ala Asn Thr Arg Ala Trp
    290                 295                 300

Arg Thr Ser Leu Leu Ile Ala Leu Gly Thr Leu Leu Ala Leu Val Cys
305                 310                 315                 320

Val Phe Val Ile Cys Arg Arg Tyr Leu Val Met Gln Arg Leu Phe Pro
                325                 330                 335

Arg Ile Pro His Met Lys Asp Pro Ile Gly Asp Ser Phe Gln Asn Asp
            340                 345                 350

Lys Leu Val Val Trp Glu Ala Gly Lys Ala Gly Leu Glu Glu Cys Leu
        355                 360                 365

Val Thr Glu Val Gln Val Val Gln Lys Thr

    370                 375

<210> 7
<211> 2308
<212> DNA
<213> Homo sapiens

<400> 7
gagagactgg atggacccac aagggtgaca gcccaggcgg accgatcttc ccatcccaca 60

tcctccggcg cgatgccaaa aagaggctga cggcaactgg gccttctgca gagaaagacc 120

tccgcttcac tgccccggct ggtcccaagg gtcaggaaga tggattcata cctgctgatg 180

tggggactgc tcacgttcat catggtgcct ggctgccagg cagagctctg tgacgatgac 240

ccgccagaga tcccacacgc cacattcaaa gccatggcct acaaggaagg aaccatgttg 300

aactgtgaat gcaagagagg tttccgcaga ataaaaagcg ggtcactcta tatgctctgt 360

acaggaaact ctagccactc gtcctgggac aaccaatgtc aatgcacaag ctctgccact 420

cggaacacaa cgaaacaagt gacacctcaa cctgaagaac agaaagaaag gaaaaccaca 480

gaaatgcaaa gtccaatgca gccagtggac caagcgagcc ttccaggtca ctgcagggaa 540

cctccaccat gggaaaatga agccacagag agaatttatc atttcgtggt ggggcagatg 600

gtttattatc agtgcgtcca gggatacagg gctctacaca gaggtcctgc tgagagcgtc 660

tgcaaaatga cccacgggaa gacaaggtgg acccagcccc agctcatatg cacaggtgaa 720

atggagacca gtcagtttcc aggtgaagag aagcctcagg caagccccga aggccgtcct 780

gagagtgaga cttcctgcct cgtcacaaca acagattttc aaatacagac agaaatggct 840

gcaaccatgg agacgtccat atttacaaca gagtaccagg tagcagtggc cggctgtgtt 900

ttcctgctga tcagcgtcct cctcctgagt gggctcacct ggcagcggag acagaggaag 960

agtagaagaa caatctagaa aaccaaaaga acaagaattt cttggtaaga agccgggaac 1020

agacaacaga agtcatgaag cccaagtgaa atcaaaggtg ctaaatggtc gcccaggaga 1080

catccgttgt gcttgcctgc gttttggaag ctctgaagtc acatcacagg acacggggca 1140

gtggcaacct tgtctctatg ccagctcagt cccatcagag agcgagcgct acccacttct 1200

aaatagcaat ttcgccgttg aagaggaagg gcaaaaccac tagaactctc catcttattt 1260

tcatgtatat gtgttcatta aagcatgaat ggtatggaac tctctccacc ctatatgtag 1320

tataaagaaa agtaggttta cattcatctc attccaactt cccagttcag gagtcccaag 1380

gaaagcccca gcactaacgt aaatacacaa cacacacact ctaccctata caactggaca 1440

ttgtctgcgt ggttcctttc tcagccgctt ctgactgctg attctcccgt tcacgttgcc 1500

taataaacat ccttcaagaa ctctgggctg ctacccagaa atcattttac ccttggctca 1560

atcctctaag ctaaccccct tctactgagc cttcagtctt gaatttctaa aaaacagagg 1620

ccatggcaga ataatctttg ggtaacttca aaacggggca gccaaaccca tgaggcaatg 1680

tcaggaacag aaggatgaat gaggtcccag gcagagaatc atacttagca aagttttacc 1740

tgtgcgttac taattggcct ctttaagagt tagtttcttt gggattgcta tgaatgatac 1800

cctgaatttg gcctgcacta atttgatgtt tacaggtgga cacacaaggt gcaaatcaat 1860

gcgtacgttt cctgagaagt gtctaaaaac accaaaaagg gatccgtaca ttcaatgttt 1920

atgcaaggaa ggaaagaaag aaggaagtga agagggagaa gggatggagg tcacactggt 1980

agaacgtaac cacggaaaag agcgcatcag gcctggcacg gtggctcagg cctataaccc 2040

cagctcccta ggagaccaag gcgggagcat ctcttgaggc caggagtttg agaccagcct 2100

gggcagcata gcaagacaca tccctacaaa aaattagaaa ttggctggat gtggtggcat 2160

acgcctgtag tcctagccac tcaggaggct gaggcaggag gattgcttga gcccaggagt 2220

tcgaggctgc agtcagtcat gatggcacca ctgcactcca gcctgggcaa cagagcaaga 2280

tcctgtcttt aaggaaaaaa agacaagg 2308

<210> 8
<211> 272
<212> PRT
<213> Homo sapiens

<400> 8
Met Asp Ser Tyr Leu Leu Met Trp Gly Leu Leu Thr Phe Ile Met Val
1               5                   10                  15

Pro Gly Cys Gln Ala Glu Leu Cys Asp Asp Asp Pro Pro Glu Ile Pro
            20                  25                  30

His Ala Thr Phe Lys Ala Met Ala Tyr Lys Glu Gly Thr Met Leu Asn
        35                  40                  45

Cys Glu Cys Lys Arg Gly Phe Arg Arg Ile Lys Ser Gly Ser Leu Tyr
    50                  55                  60

Met Leu Cys Thr Gly Asn Ser Ser His Ser Ser Trp Asp Asn Gln Cys
65                  70                  75                  80

Gln Cys Thr Ser Ser Ala Thr Arg Asn Thr Thr Lys Gln Val Thr Pro
                85                  90                  95

Gln Pro Glu Glu Gln Lys Glu Arg Lys Thr Thr Glu Met Gln Ser Pro
            100                 105                 110

Met Gln Pro Val Asp Gln Ala Ser Leu Pro Gly His Cys Arg Glu Pro
        115                 120                 125

Pro Pro Trp Glu Asn Glu Ala Thr Glu Arg Ile Tyr His Phe Val Val
    130                 135                 140

Gly Gln Met Val Tyr Tyr Gln Cys Val Gln Gly Tyr Arg Ala Leu His
145                 150                 155                 160

Arg Gly Pro Ala Glu Ser Val Cys Lys Met Thr His Gly Lys Thr Arg
                165                 170                 175

Trp Thr Gln Pro Gln Leu Ile Cys Thr Gly Glu Met Glu Thr Ser Gln
            180                 185                 190

Phe Pro Gly Glu Glu Lys Pro Gln Ala Ser Pro Glu Gly Arg Pro Glu
        195                 200                 205

Ser Glu Thr Ser Cys Leu Val Thr Thr Thr Asp Phe Gln Ile Gln Thr
    210                 215                 220

Glu Met Ala Ala Thr Met Glu Thr Ser Ile Phe Thr Thr Glu Tyr Gln
225                 230                 235                 240

Val Ala Val Ala Gly Cys Val Phe Leu Leu Ile Ser Val Leu Leu Leu
                245                 250                 255

Ser Gly Leu Thr Trp Gln Arg Arg Gln Arg Lys Ser Arg Arg Thr Ile
            260                 265                 270

<210> 9
<211> 807
<212> DNA
<213> Homo sapiens

<400> 9
aaaccatacc atatcccacc agagagtgac tcctgattgc ctcctcaagt cgcagacact      60

atgctgcctc ccatggccct gcccagtgta tcttggatgc tgctttcctg cctcatgctg     120

ctgtctcagg ttcaaggtga agaaccccag agggaactgc cctctgcacg gatccgctgt     180

cccaaaggct ccaaggccta tggctcccac tgctatgcct tgtttttgtc accaaaatcc     240

tggacagatg cagatctggc ctgccagaag cggccctctg gaaacctggt gtctgtgctc     300

agtggggctg agggatcctt cgtgtcctcc ctggtgaaga gcattggtaa cagctactca     360

tacgtctgga ttgggctcca tgaccccaca cagggcaccg agcccaatgg agaaggttgg     420

gagtggagta gcagtgatgt gatgaattac tttgcatggg agagaaatcc ctccaccatc     480

tcaagccccg gccactgtgc gagcctgtcg agaagcacag catttctgag gtggaaagat 540

tataactgta atgtgaggtt accctatgtc tgcaagttca ctgactagtg caggagggaa 600

gtcagcagcc tgtgtttggt gtgcaactca tcatgggcat gagaccagtg tgaggactca 660

ccctggaaga gaatattcgc ttaattcccc caacctgacc acctcattct tatctttctt 720

ctgtttcttc ctccccgctg tcatttcagt ctcttcattt tgtcatacgg cctaaggctt 780

taaagagcaa taaaattttt agtctgc 807

<210> 10
<211> 175
<212> PRT
<213> Homo sapiens

<400> 10
Met Leu Pro Pro Met Ala Leu Pro Ser Val Ser Trp Met Leu Leu Ser
1               5                   10                  15

Cys Leu Met Leu Leu Ser Gln Val Gln Gly Glu Glu Pro Gln Arg Glu
            20                  25                  30

Leu Pro Ser Ala Arg Ile Arg Cys Pro Lys Gly Ser Lys Ala Tyr Gly
        35                  40                  45

Ser His Cys Tyr Ala Leu Phe Leu Ser Pro Lys Ser Trp Thr Asp Ala
    50                  55                  60

Asp Leu Ala Cys Gln Lys Arg Pro Ser Gly Asn Leu Val Ser Val Leu
65                  70                  75                  80

Ser Gly Ala Glu Gly Ser Phe Val Ser Ser Leu Val Lys Ser Ile Gly
                85                  90                  95

Asn Ser Tyr Ser Tyr Val Trp Ile Gly Leu His Asp Pro Thr Gln Gly
            100                 105                 110

Thr Glu Pro Asn Gly Glu Gly Trp Glu Trp Ser Ser Ser Asp Val Met
        115                 120                 125

Asn Tyr Phe Ala Trp Glu Arg Asn Pro Ser Thr Ile Ser Ser Pro Gly
    130                 135                 140

His Cys Ala Ser Leu Ser Arg Ser Thr Ala Phe Leu Arg Trp Lys Asp
145                 150                 155                 160

Tyr Asn Cys Asn Val Arg Leu Pro Tyr Val Cys Lys Phe Thr Asp

165                 170                 175

<210> 11
<211> 773
<212> DNA
<213> Homo sapiens

<400> 11
aagccacctc aagtggacaa ggcacttacc aacagagatt gctgatttgc tccttaagca      60

agagattcac tgccgctaag catggctcag accaactcgt tcttcatgct gatctcctcc     120

ctgatgttcc tgtctctgag ccaaggccag gagtcccaga cagagctgcc taatccccga     180

atcagctgcc cagaaggcac caatgcctat cgctcctact gctactactt taatgaagac     240

cctgagacct gggttgatgc agatctctat tgccagaaca tgaattcagg caacctggtg     300

tctgtgctca cccaggcgga gggtgccttc gtggcctcac tgattaagga gagtagcact     360

gatgacagca atgtctggat tggcctccat gacccaaaaa agaaccgccg ctggcactgg     420

agtagtgggt ccctggtctc ctacaagtcc tgggacactg gatccccgag cagtgctaat     480

gctggctact gtgcaagcct gacttcatgc tcaggattca agaaatggaa ggatgaatct     540

tgtgagaaga agttctcctt tgtttgcaag ttcaaaaact agaggaagct gaaaaatgga     600

tgtctagaac tggtcctgca attactatga agtcaaaaat taaactagac tatgtctcca     660

actcagttca gaccatctcc tccctaatga gtttgcatcg ctgatcttca gtaccttcac     720

ctgtctcagt ctctagagcc ctgaaaaata aaaacaaact tatttttatc cag            773

<210> 12
<211> 166
<212> PRT
<213> Homo sapiens

<400> 12
Met Ala Gln Thr Asn Ser Phe Phe Met Leu Ile Ser Ser Leu Met Phe
1               5                   10                  15

Leu Ser Leu Ser Gln Gly Gln Glu Ser Gln Thr Glu Leu Pro Asn Pro
            20                  25                  30

Arg Ile Ser Cys Pro Glu Gly Thr Asn Ala Tyr Arg Ser Tyr Cys Tyr
        35                  40                  45

Tyr Phe Asn Glu Asp Pro Glu Thr Trp Val Asp Ala Asp Leu Tyr Cys
    50                  55                  60

Gln Asn Met Asn Ser Gly Asn Leu Val Ser Val Leu Thr Gln Ala Glu
65                  70                  75                  80

Gly Ala Phe Val Ala Ser Leu Ile Lys Glu Ser Ser Thr Asp Asp Ser
                85                  90                  95

Asn Val Trp Ile Gly Leu His Asp Pro Lys Lys Asn Arg Arg Trp His
            100                 105                 110

Trp Ser Ser Gly Ser Leu Val Ser Tyr Lys Ser Trp Asp Thr Gly Ser
        115                 120                 125

Pro Ser Ser Ala Asn Ala Gly Tyr Cys Ala Ser Leu Thr Ser Cys Ser
    130                 135                 140

Gly Phe Lys Lys Trp Lys Asp Glu Ser Cys Glu Lys Lys Phe Ser Phe
145                 150                 155                 160

Val Cys Lys Phe Lys Asn
                165

<210> 13
<211> 2693
<212> DNA
<213> Homo sapiens

<400> 13
ggagagaaat ggctgaaagt ggaaacccgg agcggtttgg ggcgcttcgc aaagttctcc      60

agctcggcga gccggcggtg tgtctaccag aggctggggg ccagtccatc tggtcctgcc     120

cgacgccact tccaccccgc gagctctggg aaaggtctct acgtgacgcc aagggccgtc     180

gcggccccgg ggagagggat cgaaagactc actctgcgag gtgtttacac tggaggtggg     240

ggacgggaag ggactgggca aagttacctg gactcaacta aattcctgcg cttgcttagt     300

gttgacattt gaataggatc cgagatgttc tgcttttatc ccgggcagcc ctgatatgtc     360

agcaacaggc tgaggttcca gacctgaaat cgcactgcaa gaaaaagggg tttgctgaac     420

taactccaag ctggtgtgcc tagcgtccgc gcggctgccg gcccaagagc tggagtcacc     480

atggcggccg gagttgcggc ctggctgcct tttgcccggg ctgcggccat cgggtggatg     540

ccggtggcca actgccccat gcccctggcc ccggccgaca agaacaagcg gcaggatgag     600

ctgattgtcc tcaacgtgag tgggcggagg ttccagacct ggaggaccac gctggagcgc     660

tacccggaca ccctgctggg cagcacggag aaggagttct tcttcaacga ggacaccaag     720

gagtacttct tcgaccggga ccccgaggtg ttccgctgcg tgctcaactt ctaccgcacg     780

gggaagctgc actacccgcg ctacgagtgc atctctgcct acgacgacga gctggccttc     840

tacggcatcc tcccggagat catcggggac tgctgctacg aggagtacaa ggaccgcaag     900

agggagaacg ccgagcggct catggacgac aacgactcgg agaacaacca ggagtccatg 960
ccctcgctca gcttccgcca gaccatgtgg cgggccttcg agaaccccca caccagcacg 1020
ctggccctgg tcttctacta cgtgactggc ttcttcatcg ctgtctcggt catcaccaac 1080
gtggtggaga cggtgccgtg cggcacggtc ccgggcagca aggagctgcc gtgcggggag 1140
cgctactcgg tggccttctt ctgcctggac acggcgtgcg tcatgatctt caccgtggag 1200
tacctcctgc ggctcttcgc ggctcccagc cgctaccgct tcatccgcag cgtcatgagc 1260
atcatcgacg tggtggccat catgccctac tacatcggtc tggtcatgac caacaacgag 1320
gacgtgtccg gcgccttcgt cacgctccgg gtcttccgcg tcttcaggat cttcaagttt 1380
tcccgccact cccagggcct gcggatcctg ggctacacac tgaagagctg tgcctccgaa 1440
ctgggctttc ttctcttctc cctcaccatg gccatcatca tctttgccac tgtgatgttt 1500
tatgccgaga agggctcctc ggccagcaag ttcacaagca tccctgcctc gttttggtac 1560
accattgtca ccatgaccac actggggtac ggagacatgg tgcctaagac gattgcaggg 1620
aagatcttcg gctccatctg ctccttgagt ggcgtcctgg tcattgccct gccagtccct 1680
gtgattgttt ccaactttag ccggatttac caccagaatc agagagctga taaacgcagg 1740
gcacaaaaga aggcccgcct tgccaggatc cgtgtggcca aaacaggcag ttcgaatgca 1800
tacctgcaca gcaagcgcaa cgggctcctc aacgaggcgc tggagctgac gggcacccca 1860
gaagaggagc acatgggcaa gaccacctca ctcatcgaga gccagcatca tcacctgctg 1920
cactgcctgg aaaaaaccac tgggttgtcc tatcttgtgg atgatcccct gttatctgta 1980
cgaacctcca ccatcaagaa ccacgagttt attgatgagc agatgtttga gcagaactgc 2040
atggagagtt caatgcagaa ctacccatcc acaagaagtc cctcactgtc cagccaccca 2100
ggcctcacta ccacctgctg ctcccgtcgt agtaagaaga ccacacacct gcccaattct 2160
aacctgccag ctactcgcct gcgcagcatg caagagctca gcacgatcca catccagggc 2220
agtgagcagc cctccctcac aaccagtcgc tccagcctta atttgaaagc agacgacgga 2280
ctgagaccaa actgcaaaac atcccagatc accacagcca tcatcagcat ccccactccc 2340
ccagcgctaa ccccagaggg ggaaagtcgg ccaccccctg ccagcccagg ccccaacacg 2400
aacattcctt ccatagccag caatgttgtc aaggtctccg ccttgtaaaa ccactggaca 2460
gagggccaga gtgggtagtg gggaatgaag gggactggca tgttggtggg tggtcactga 2520
gaccactccc tccccccttt ccccactatt tctgcctgcc ccattgtacc cctagcactg 2580
agacttgtgc ctggaaggaa aaagaggtag caaaggggca cctgaggttc acgctgctag 2640
cgtggacata gccctgtgat actgcatctc actggggcca gaggaaggga ggg 2693

<210> 14
<211> 655
<212> PRT
<213> Homo sapiens

<400> 14

<210> 15
<211> 799
<212> DNA
<213> Homo sapiens

<400> 15
cactcccaaa gaactgggta ctcaacactg agcagatctg ttctttgagc taaaaaccat      60

gtgctgtacc aagagtttgc tcctggctgc tttgatgtca gtgctgctac tccacctctg     120

cggcgaatca gaagcagcaa gcaactttga ctgctgtctt ggatacacag accgtattct     180

tcatcctaaa tttattgtgg gcttcacacg gcagctggcc aatgaaggct gtgacatcaa     240

tgctatcatc tttcacacaa agaaaaagtt gtctgtgtgc gcaaatccaa aacagacttg    300

ggtgaaatat attgtgcgtc tcctcagtaa aaaagtcaag aacatgtaaa aactgtggct    360

tttctggaat ggaattggac atagcccaag aacagaaaga accttgctgg ggttggaggt    420

ttcacttgca catcatggag ggtttagtgc ttatctaatt tgtgcctcac tggacttgtc    480

caattaatga agttgattca tattgcatca tagtttgctt tgtttaagca tcacattaaa    540

gttaaactgt attttatgtt atttatagct gtaggttttc tgtgtttagc tatttaatac    600

taattttcca taagctattt tggtttagtg caaagtataa aattatattt gggggggaat    660

aagattatat ggactttctt gcaagcaaca agctattttt taaaaaaact atttaacatt    720

cttttgttta tattgttttg tctcctaaat tgttgtaatt gcattataaa ataagaaaaa    780

cattaataag acaaatatt                                                 799

<210> 16
<211> 96
<212> PRT
<213> Homo sapiens

<400> 16
Met Cys Cys Thr Lys Ser Leu Leu Leu Ala Ala Leu Met Ser Val Leu
1               5                   10                  15

Leu Leu His Leu Cys Gly Glu Ser Glu Ala Ala Ser Asn Phe Asp Cys
            20                  25                  30

Cys Leu Gly Tyr Thr Asp Arg Ile Leu His Pro Lys Phe Ile Val Gly
        35                  40                  45

Phe Thr Arg Gln Leu Ala Asn Glu Gly Cys Asp Ile Asn Ala Ile Ile
    50                  55                  60

Phe His Thr Lys Lys Lys Leu Ser Val Cys Ala Asn Pro Lys Gln Thr
65                  70                  75                  80

Trp Val Lys Tyr Ile Val Arg Leu Leu Ser Lys Lys Val Lys Asn Met
                85                  90                  95

<210> 17
<211> 970
<212> DNA
<213> Homo sapiens

<400> 17
   gaagcgggaa agggccacaa tggggtctgg gaggtggcgg gggcggagcg gggatgtcca     60
   gccacgtcgc tttgttttcc cacgctagga gctaccacaa caggtgctgc gagccgtaag    120
   cgccccccac ccgcgctatg ggctcggacg cctgggtggg cctttggcgg ccacaccggc 180
   cccgcggccc catcgcggcg cactacggag gccccgggcc caaatacaag ctgccgccca 240
   acaccggcta cgccctgcat gacccgtcgc ggccgcgcgc ccccgccttc accttcggcg 300
   cgcgcttccc cacgcagcag acgacgtgcg gccccgggcc aggccacctg gtgcccgctc 360
   gcatgaccgt gcgcggcacc gacggcgccc ccgcctactc catctacggc cgcccacgcc 420
   gctcagcgcc cttcctcact ccgggacctg gcaggtactt cccggagcga gcggggaacg 480
   cgacgtaccc cagtgcgcct cggcacacca ttgctccccg aaactggggt gtccaggcgg 540
   aacagcagag cccaggtccc gcggcctata cggtgccctc gctcttgggt ccgcgcgtca 600
   tcggcaaagt ctccgcccca acttgctcca tctacggccg cagagcggct ggcagtttct 660
   tcgaggacct cagcaagacc ccgggcccct gcgcctatca ggtcgtgagt ccaggggtct 720
   acaagtcccg ggccccccag ttcacgattc tggcgcggac ttcgctcccc caagacaaca 780
   ctcggaagcc agggcccgcg gcctacaacg tggatcagca ccggaagccc cgcggctgga 840
   gtttcgggat ccggcactcg gactacctgg ccccgctggt gaccgacgcg gacaactgac 900
   ccgccaggcg ggagcggccc cacacgtgtt tgcttaaagt ctgcgagtcc gcaaaaaaaa 960
   aaaaaaaaaa 970

<210> 18
<211> 253
<212> PRT
<213> Homo sapiens

<400> 18
Met Gly Ser Asp Ala Trp Val Gly Leu Trp Arg Pro His Arg Pro Arg
1               5                   10                  15

Gly Pro Ile Ala Ala His Tyr Gly Gly Pro Gly Pro Lys Tyr Lys Leu
            20                  25                  30

Pro Pro Asn Thr Gly Tyr Ala Leu His Asp Pro Ser Arg Pro Arg Ala
        35                  40                  45

Pro Ala Phe Thr Phe Gly Ala Arg Phe Pro Thr Gln Gln Thr Thr Cys
    50                  55                  60

Gly Pro Gly Pro Gly His Leu Val Pro Ala Arg Met Thr Val Arg Gly
65                  70                  75                  80

Thr Asp Gly Ala Pro Ala Tyr Ser Ile Tyr Gly Arg Pro Arg Arg Ser
                85                  90                  95

Ala Pro Phe Leu Thr Pro Gly Pro Gly Arg Tyr Phe Pro Glu Arg Ala
            100                 105                 110

Gly Asn Ala Thr Tyr Pro Ser Ala Pro Arg His Thr Ile Ala Pro Arg
        115                 120                 125

Asn Trp Gly Val Gln Ala Glu Gln Gln Ser Pro Gly Pro Ala Ala Tyr
    130                 135                 140

Thr Val Pro Ser Leu Leu Gly Pro Arg Val Ile Gly Lys Val Ser Ala
145                 150                 155                 160

Pro Thr Cys Ser Ile Tyr Gly Arg Arg Ala Ala Gly Ser Phe Phe Glu
                165                 170                 175

Asp Leu Ser Lys Thr Pro Gly Pro Cys Ala Tyr Gln Val Val Ser Pro
            180                 185                 190

Gly Val Tyr Lys Ser Arg Ala Pro Gln Phe Thr Ile Leu Ala Arg Thr
        195                 200                 205

Ser Leu Pro Gln Asp Asn Thr Arg Lys Pro Gly Pro Ala Ala Tyr Asn
    210                 215                 220

Val Asp Gln His Arg Lys Pro Arg Gly Trp Ser Phe Gly Ile Arg His
225                 230                 235                 240

Ser Asp Tyr Leu Ala Pro Leu Val Thr Asp Ala Asp Asn
                245                 250

<210> 19
<211> 1498
<212> DNA
<213> Homo sapiens

<400> 19
accaaacctc ttcgaggcac aaggcacaac aggctgctct gggattctct tcagccaatc      60

ttcattgctc aagtgtctga agcagccatg gcagaagtac ctgagctcgc cagtgaaatg     120

atggcttatt acagtggcaa tgaggatgac ttgttctttg aagctgatgg ccctaaacag     180

atgaagtgct ccttccagga cctggacctc tgccctctgg atggcggcat ccagctacga     240

atctccgacc accactacag caagggcttc aggcaggccg cgtcagttgt tgtggccatg     300

gacaagctga ggaagatgct ggttccctgc ccacagacct tccaggagaa tgacctgagc     360

accttctttc ccttcatctt tgaagaagaa cctatcttct tcgacacatg ggataacgag     420

gcttatgtgc acgatgcacc tgtacgatca ctgaactgca cgctccggga ctcacagcaa    480

aaaagcttgg tgatgtctgg tccatatgaa ctgaaagctc tccacctcca gggacaggat    540

atggagcaac aagtggtgtt ctccatgtcc tttgtacaag gagaagaaag taatgacaaa    600

atacctgtgg ccttgggcct caaggaaaag aatctgtacc tgtcctgcgt gttgaaagat    660

gataagccca ctctacagct ggagagtgta gatcccaaaa attacccaaa gaagaagatg    720

gaaaagcgat ttgtcttcaa caagatagaa atcaataaca agctggaatt tgagtctgcc    780

cagttcccca actggtacat cagcacctct caagcagaaa acatgcccgt cttcctggga    840

gggaccaaag gcggccagga tataactgac ttcaccatgc aatttgtgtc ttcctaaaga    900

gagctgtacc cagagagtcc tgtgctgaat gtggactcaa tccctagggc tggcagaaag    960

ggaacagaaa ggtttttgag tacggctata gcctggactt tcctgttgtc tacaccaatg   1020

cccaactgcc tgccttaggg tagtgctaag aggatctcct gtccatcagc caggacagtc   1080

agctctctcc tttcagggcc aatccccagc ccttttgttg agccaggcct ctctcacctc   1140

tcctactcac ttaaagcccg cctgacagaa accacggcca catttggttc taagaaaccc   1200

tctgtcattc gctcccacat tctgatgagc aaccgcttcc ctatttattt atttatttgt   1260

ttgtttgttt tattcattgg tctaatttat tcaaaggggg caagaagtag cagtgtctgt   1320

aaaagagcct agtttttaat agctatggaa tcaattcaat ttggactggt gtgctctctt   1380

taaatcaagt cctttaatta agactgaaaa tatataagct cagattattt aaatgggaat   1440

atttataaat gagcaaatat catactgttc aatggttctg aaataaactt cactgaag     1498

<210> 20
<211> 269
<212> PRT
<213> Homo sapiens

<400> 20
Met Ala Glu Val Pro Glu Leu Ala Ser Glu Met Met Ala Tyr Tyr Ser
1               5                   10                  15

Gly Asn Glu Asp Asp Leu Phe Phe Glu Ala Asp Gly Pro Lys Gln Met
            20                  25                  30

Lys Cys Ser Phe Gln Asp Leu Asp Leu Cys Pro Leu Asp Gly Gly Ile
        35                  40                  45

Gln Leu Arg Ile Ser Asp His His Tyr Ser Lys Gly Phe Arg Gln Ala
    50                  55                  60

Ala Ser Val Val Val Ala Met Asp Lys Leu Arg Lys Met Leu Val Pro
65                  70                  75                  80

Cys Pro Gln Thr Phe Gln Glu Asn Asp Leu Ser Thr Phe Phe Pro Phe
                85                  90                  95

Ile Phe Glu Glu Glu Pro Ile Phe Phe Asp Thr Trp Asp Asn Glu Ala
            100                 105                 110

Tyr Val His Asp Ala Pro Val Arg Ser Leu Asn Cys Thr Leu Arg Asp
        115                 120                 125

Ser Gln Gln Lys Ser Leu Val Met Ser Gly Pro Tyr Glu Leu Lys Ala
    130                 135                 140

Leu His Leu Gln Gly Gln Asp Met Glu Gln Gln Val Val Phe Ser Met
145                 150                 155                 160

Ser Phe Val Gln Gly Glu Glu Ser Asn Asp Lys Ile Pro Val Ala Leu
                165                 170                 175

Gly Leu Lys Glu Lys Asn Leu Tyr Leu Ser Cys Val Leu Lys Asp Asp
            180                 185                 190

Lys Pro Thr Leu Gln Leu Glu Ser Val Asp Pro Lys Asn Tyr Pro Lys
        195                 200                 205

Lys Lys Met Glu Lys Arg Phe Val Phe Asn Lys Ile Glu Ile Asn Asn
    210                 215                 220

Lys Leu Glu Phe Glu Ser Ala Gln Phe Pro Asn Trp Tyr Ile Ser Thr
225                 230                 235                 240

Ser Gln Ala Glu Asn Met Pro Val Phe Leu Gly Gly Thr Lys Gly Gly
                245                 250                 255

Gln Asp Ile Thr Asp Phe Thr Met Gln Phe Val Ser Ser
            260                 265

<210> 21
<211> 1103
<212> DNA
<213> Homo sapiens

<400> 21
cacagagccc gggccgcagg cacctcctcg ccagctcttc cgctcctctc acagccgcca      60

gacccgcctg ctgagcccca tggcccgcgc tgctctctcc gccgccccca gcaatccccg     120

gctcctgcga gtggcactgc tgctcctgct cctggtagcc gctggccggc gcgcagcagg     180

agcgtccgtg gccactgaac tgcgctgcca gtgcttgcag accctgcagg gaattcaccc 240

caagaacatc caaagtgtga acgtgaagtc ccccggaccc cactgcgccc aaaccgaagt 300

catagccaca ctcaagaatg ggcggaaagc ttgcctcaat cctgcatccc ccatagttaa 360

gaaaatcatc gaaaagatgc tgaacagtga caaatccaac tgaccagaag ggaggaggaa 420

gctcactggt ggctgttcct gaaggaggcc ctgcccttat aggaacagaa gaggaaagag 480

agacacagct gcagaggcca cctggattgt gcctaatgtg tttgagcatc gcttaggaga 540

agtcttctat ttatttattt attcattagt tttgaagatt ctatgttaat attttaggtg 600

taaaataatt aagggtatga ttaactctac ctgcacactg tcctattata ttcattcttt 660

ttgaaatgtc aaccccaagt tagttcaatc tggattcata tttaatttga aggtagaatg 720

ttttcaaatg ttctccagtc attatgttaa tatttctgag gagcctgcaa catgccagcc 780

actgtgatag aggctggcgg atccaagcaa atggccaatg agatcattgt gaaggcaggg 840

gaatgtatgt gcacatctgt tttgtaactg tttagatgaa tgtcagttgt tatttattga 900

aatgatttca cagtgtgtgg tcaacatttc tcatgttgaa actttaagaa ctaaaatgtt 960

ctaaatatcc cttggacatt ttatgtcttt cttgtaaggc atactgcctt gtttaatggt 1020

agttttacag tgtttctggc ttagaacaaa ggggcttaat tattgatgtt ttcatagaga 1080

atataaaaat aaagcactta tag 1103

<210> 22
<211> 107
<212> PRT
<213> Homo sapiens

<400> 22
Met Ala Arg Ala Ala Leu Ser Ala Ala Pro Ser Asn Pro Arg Leu Leu
1               5                   10                  15

Arg Val Ala Leu Leu Leu Leu Leu Leu Val Ala Ala Gly Arg Arg Ala
            20                  25                  30

Ala Gly Ala Ser Val Ala Thr Glu Leu Arg Cys Gln Cys Leu Gln Thr
        35                  40                  45

Leu Gln Gly Ile His Pro Lys Asn Ile Gln Ser Val Asn Val Lys Ser
    50                  55                  60

Pro Gly Pro His Cys Ala Gln Thr Glu Val Ile Ala Thr Leu Lys Asn
65                  70                  75                  80

Gly Arg Lys Ala Cys Leu Asn Pro Ala Ser Pro Ile Val Lys Lys Ile

85                  90                  95

Ile Glu Lys Met Leu Asn Ser Asp Lys Ser Asn
            100                 105

<210> 23
<211> 1103
<212> DNA
<213> Homo sapiens

<400> 23
cacagagccc gggccgcagg cacctcctcg ccagctcttc cgctcctctc acagccgcca      60

gacccgcctg ctgagcccca tggcccgcgc tgctctctcc gccgccccca gcaatccccg     120

gctcctgcga gtggcactgc tgctcctgct cctggtagcc gctggccggc gcgcagcagg     180

agcgtccgtg gccactgaac tgcgctgcca gtgcttgcag accctgcagg gaattcaccc     240

caagaacatc caaagtgtga acgtgaagtc ccccggaccc cactgcgccc aaaccgaagt     300

catagccaca ctcaagaatg ggcggaaagc ttgcctcaat cctgcatccc ccatagttaa     360

gaaaatcatc gaaaagatgc tgaacagtga caaatccaac tgaccagaag ggaggaggaa     420

gctcactggt ggctgttcct gaaggaggcc ctgcccttat aggaacagaa gaggaaagag     480

agacacagct gcagaggcca cctggattgt gcctaatgtg tttgagcatc gcttaggaga     540

agtcttctat ttatttattt attcattagt tttgaagatt ctatgttaat attttaggtg     600

taaaataatt aagggtatga ttaactctac ctgcacactg tcctattata ttcattcttt     660

ttgaaatgtc aaccccaagt tagttcaatc tggattcata tttaatttga aggtagaatg     720

ttttcaaatg ttctccagtc attatgttaa tatttctgag gagcctgcaa catgccagcc     780

actgtgatag aggctggcgg atccaagcaa atggccaatg agatcattgt gaaggcaggg     840

gaatgtatgt gcacatctgt tttgtaactg tttagatgaa tgtcagttgt tatttattga     900

aatgatttca cagtgtgtgg tcaacatttc tcatgttgaa actttaagaa ctaaaatgtt     960

ctaaatatcc cttggacatt ttatgtcttt cttgtaaggc atactgcctt gtttaatggt    1020

agttttacag tgtttctggc ttagaacaaa ggggcttaat tattgatgtt ttcatagaga    1080

atataaaaat aaagcactta tag                                            1103

<210> 24
<211> 107
<212> PRT
<213> Homo sapiens

<400> 24
Met Ala Arg Ala Ala Leu Ser Ala Ala Pro Ser Asn Pro Arg Leu Leu
1               5                   10                  15

Arg Val Ala Leu Leu Leu Leu Leu Leu Val Ala Ala Gly Arg Arg Ala
            20                  25                  30

Ala Gly Ala Ser Val Ala Thr Glu Leu Arg Cys Gln Cys Leu Gln Thr
        35                  40                  45

Leu Gln Gly Ile His Pro Lys Asn Ile Gln Ser Val Asn Val Lys Ser
    50                  55                  60

Pro Gly Pro His Cys Ala Gln Thr Glu Val Ile Ala Thr Leu Lys Asn
65                  70                  75                  80

Gly Arg Lys Ala Cys Leu Asn Pro Ala Ser Pro Ile Val Lys Lys Ile
                85                  90                  95

Ile Glu Lys Met Leu Asn Ser Asp Lys Ser Asn
            100                 105

<210> 25
<211> 5243
<212> DNA
<213> Homo sapiens

<400> 25
agcgtgagac tcgcgccctc cggcacggaa aaggccaggc gacaggtgtc gcttgaaaag      60

actgggcttg tccttgctgg tgcatgcgtc gtcggcctct gggcagcagg tttacaaagg     120

aggaaaacga cttcttctag attttttttt cagtttcttc tataaatcaa aacatctcaa     180

aatggagacc taaaatcctt aaagggactt agtctaatct cgggaggtag ttttgtgcat     240

gggtaaacaa attaagtatt aactggtgtt ttactatcca aagaatgcta attttataaa     300

catgatcgag ttatataagg tataccataa tgagtttgat tttgaatttg atttgtggaa     360

ataaaggaaa agtgattcta gctggggcat attgttaaag catttttttc agagttggcc     420

aggcagtctc ctactggcac attctcccat tatgtagaat agaaatagta cctgtgtttg     480

ggaaagattt taaaatgagt gacagttatt tggaacaaag agctaataat caatccactg     540

caaattaaag aaacatgcag atgaaagttt tgacacatta aaatacttct acagtgacaa     600

agaaaaatca agaacaaagc tttttgatat gtgcaacaaa tttagaggaa gtaaaaagat     660

aaatgtgatg attggtcaag aaattatcca gttatttaca aggccactga tattttaaac     720

gtccaaaagt ttgtttaaat gggctgttac cgctgagaat gatgaggatg agaatgatgg     780

ttgaaggtta cattttagga aatgaagaaa cttagaaaat taatataaag acagtgatga     840

atacaaagaa gatttttata acaatgtgta aaatttttgg ccagggaaag gaatattgaa     900

gttagataca attacttacc tttgagggaa ataattgttg gtaatgagat gtgatgtttc 960
tcctgccacc tggaaacaaa gcattgaagt ctgcagttga aaagcccaac gtctgtgaga 1020
tccaggaaac catgcttgca aaccactggt aaaaaaaaaa aaaaaaaaaa aaaaaagcca 1080
cagtgacttg cttattggtc attgctagta ttatcgactc agaacctctt tactaatggc 1140
tagtaaatca taattgagaa attctgaatt ttgacaaggt ctctgctgtt gaaatggtaa 1200
atttattatt ttttttgtca tgataaattc tggttcaagg tatgctatcc atgaaataat 1260
ttctgaccaa aactaaattg atgcaatttg attatccatc ttagcctaca gatggcatct 1320
ggtaactttt gactgtttta aaaaataaat ccactatcag agtagatttg atgttggctt 1380
cagaaacatt tagaaaaaca aaagttcaaa aatgttttca ggaggtgata agttgaataa 1440
ctctacaatg ttagttcttt gagggggaca aaaaatttaa aatctttgaa aggtcttatt 1500
ttacagccat atctaaatta tcttaagaaa atttttaaca aagggaatga aatatatatc 1560
atgattctgt ttttccaaaa gtaacctgaa tatagcaatg aagttcagtt ttgttattgg 1620
tagtttgggc agagtctctt tttgcagcac ctgttgtcta ccataattac agaggacatt 1680
tccatgttct agccaagtat actattagaa taaaaaaact taacattgag ttgcttcaac 1740
agcatgaaac tgagtccaaa agaccaaatg aacaaacaca ttaatctctg attatttatt 1800
ttaaatagaa tatttaattg tgtaagatct aatagtatca ttatacttaa gcaatcatat 1860
tcctgatgat ctatgggaaa taactattat ttaattaata ttgaaaccag gttttaagat 1920
gtgttagcca gtcctgttac tagtaaatct ctttatttgg agagaaattt tagattgttt 1980
tgttctcctt attagaagga ttgtagaaag aaaaaaatga ctaattggag aaaaattggg 2040
gatatatcat atttcactga attcaaaatg tcttcagttg taaatcttac cattatttta 2100
cgtacctcta agaaataaaa gtgcttctaa ttaaaatatg atgtcattaa ttatgaaata 2160
cttcttgata acagaagttt taaaatagcc atcttagaat cagtgaaata tggtaatgta 2220
ttattttcct cctttgagtt aggtcttgtg cttttttttc ctggccacta aatttcacaa 2280
tttccaaaaa gcaaaataaa catattctga atatttttgc tgtgaaacac ttgacagcag 2340
agctttccac catgaaaaga agcttcatga gtcacacatt acatctttgg gttgattgaa 2400
tgccactgaa acattctagt agcctggaga agttgaccta cctgtggaga tgcctgccat 2460
taaatggcat cctgatggct taatacacat cactcttctg tgaagggttt taattttcaa 2520
cacagcttac tctgtagcat catgtttaca ttgtatgtat aaagattata caaaggtgca 2580
attgtgtatt tcttccttaa aatgtatcag tataggattt agaatctcca tgttgaaact 2640
ctaaatgcat agaaataaaa ataataaaaa atttttcatt ttggcttttc agcctagtat 2700
taaaactgat aaaagcaaag ccatgcacaa aactacctcc ctagagaaag gctagtccct 2760

tttcttcccc attcatttca ttatgaacat agtagaaaac agcatattct tatcaaattt 2820

gatgaaaagc gccaacacgt ttgaactgaa atacgacttg tcatgtgaac tgtaccgaat 2880

gtctacgtat tccacttttc ctgctggggt tcctgtctca gaaaggagtc ttgctcgtgc 2940

tggtttctat tacactggtg tgaatgacaa ggtcaaatgc ttctgttgtg gcctgatgct 3000

ggataactgg aaaagaggag acagtcctac tgaaaagcat aaaaagttgt atcctagctg 3060

cagattcgtt cagagtctaa attccgttaa caacttggaa gctacctctc agcctacttt 3120

tccttcttca gtaacaaatt ccacacactc attacttccg ggtacagaaa acagtggata 3180

tttccgtggc tcttattcaa actctccatc aaatcctgta aactccagag caaatcaaga 3240

tttttctgcc ttgatgagaa gttcctacca ctgtgcaatg aataacgaaa atgccagatt 3300

acttactttt cagacatggc cattgacttt tctgtcgcca acagatctgg caaaagcagg 3360

cttttactac ataggacctg gagacagagt ggcttgcttt gcctgtggtg gaaaattgag 3420

caattgggaa ccgaaggata atgctatgtc agaacacctg agacattttc ccaaatgccc 3480

atttatagaa aatcagcttc aagacacttc aagatacaca gtttctaatc tgagcatgca 3540

gacacatgca gcccgcttta aaacattctt taactggccc tctagtgttc tagttaatcc 3600

tgagcagctt gcaagtgcgg gtttttatta tgtgggtaac agtgatgatg tcaaatgctt 3660

ttgctgtgat ggtggactca ggtgttggga atctggagat gatccatggg ttcaacatgc 3720

caagtggttt ccaaggtgtg agtacttgat aagaattaaa ggacaggagt tcatccgtca 3780

agttcaagcc agttaccctc atctacttga acagctgcta tccacatcag acagcccagg 3840

agatgaaaat gcagagtcat caattatcca ttttgaacct ggagaagacc attcagaaga 3900

tgcaatcatg atgaatactc ctgtgattaa tgctgccgtg gaaatgggct ttagtagaag 3960

cctggtaaaa cagacagttc agagaaaaat cctagcaact ggagagaatt atagactagt 4020

caatgatctt gtgttagact tactcaatgc agaagatgaa ataagggaag aggagagaga 4080

aagagcaact gaggaaaaag aatcaaatga tttattatta atccggaaga atagaatggc 4140

actttttcaa catttgactt gtgtaattcc aatcctggat agtctactaa ctgccggaat 4200

tattaatgaa caagaacatg atgttattaa acagaagaca cagacgtctt tacaagcaag 4260

agaactgatt gatacgattt tagtaaaagg aaatattgca gccactgtat tcagaaactc 4320

tctgcaagaa gctgaagctg tgttatatga gcatttattt gtgcaacagg acataaaata 4380

tattcccaca gaagatgttt cagatctacc agtggaagaa caattgcgga gactacaaga 4440

agaaagaaca tgtaaagtgt gtatggacaa agaagtgtcc atagtgttta ttccttgtgg 4500

tcatctagta gtatgcaaag attgtgctcc ttctttaaga aagtgtccta tttgtaggag 4560

tacaatcaag ggtacagttc gtacatttct ttcatgaaga agaaccaaaa catcgtctaa 4620

actttagaat taatttatta aatgtattat aactttaact tttatcctaa tttggtttcc 4680

ttaaaatttt tatttattta caactcaaaa aacattgttt tgtgtaacat atttatatat 4740

gtatctaaac catatgaaca tatatttttt agaaactaag agaatgatag gcttttgttc 4800

ttatgaacga aaaagaggta gcactacaaa cacaatattc aatcaaaatt tcagcattat 4860

tgaaattgta agtgaagtaa aacttaagat atttgagtta acctttaaga attttaaata 4920

ttttggcatt gtactaatac cgggaacatg aagccaggtg tggtggtatg tgcctgtagt 4980

cccaggctga ggcaagagaa ttacttgagc ccaggagttt gaatccatcc tgggcagcat 5040

actgagaccc tgcctttaaa aacaaacaga acaaaaacaa aacaccaggg acacatttct 5100

ctgtcttttt tgatcagtgt cctatacatc gaaggtgtgc atatatgttg aatgacattt 5160

tagggacatg gtgtttttat aaagaattct gtgagaaaaa atttaataaa gcaacaaaaa 5220

ttactcttaa aaaaaaaaaa aaa 5243

<210> 26
<211> 604
<212> PRT
<213> Homo sapiens

<400> 26
Met Asn Ile Val Glu Asn Ser Ile Phe Leu Ser Asn Leu Met Lys Ser
1               5                   10                  15

Ala Asn Thr Phe Glu Leu Lys Tyr Asp Leu Ser Cys Glu Leu Tyr Arg
            20                  25                  30

Met Ser Thr Tyr Ser Thr Phe Pro Ala Gly Val Pro Val Ser Glu Arg
        35                  40                  45

Ser Leu Ala Arg Ala Gly Phe Tyr Tyr Thr Gly Val Asn Asp Lys Val
    50                  55                  60

Lys Cys Phe Cys Cys Gly Leu Met Leu Asp Asn Trp Lys Arg Gly Asp
65                  70                  75                  80

Ser Pro Thr Glu Lys His Lys Lys Leu Tyr Pro Ser Cys Arg Phe Val
                85                  90                  95

Gln Ser Leu Asn Ser Val Asn Asn Leu Glu Ala Thr Ser Gln Pro Thr
            100                 105                 110

Phe Pro Ser Ser Val Thr Asn Ser Thr His Ser Leu Leu Pro Gly Thr
        115                 120                 125

Glu Asn Ser Gly Tyr Phe Arg Gly Ser Tyr Ser Asn Ser Pro Ser Asn
    130                 135                 140

Pro Val Asn Ser Arg Ala Asn Gln Asp Phe Ser Ala Leu Met Arg Ser
145                 150                 155                 160

Ser Tyr His Cys Ala Met Asn Asn Glu Asn Ala Arg Leu Leu Thr Phe
                165                 170                 175

Gln Thr Trp Pro Leu Thr Phe Leu Ser Pro Thr Asp Leu Ala Lys Ala
            180                 185                 190

Gly Phe Tyr Tyr Ile Gly Pro Gly Asp Arg Val Ala Cys Phe Ala Cys
        195                 200                 205

Gly Gly Lys Leu Ser Asn Trp Glu Pro Lys Asp Asn Ala Met Ser Glu
    210                 215                 220

His Leu Arg His Phe Pro Lys Cys Pro Phe Ile Glu Asn Gln Leu Gln
225                 230                 235                 240

Asp Thr Ser Arg Tyr Thr Val Ser Asn Leu Ser Met Gln Thr His Ala
                245                 250                 255

Ala Arg Phe Lys Thr Phe Phe Asn Trp Pro Ser Ser Val Leu Val Asn
            260                 265                 270

Pro Glu Gln Leu Ala Ser Ala Gly Phe Tyr Tyr Val Gly Asn Ser Asp
        275                 280                 285

Asp Val Lys Cys Phe Cys Cys Asp Gly Gly Leu Arg Cys Trp Glu Ser
    290                 295                 300

Gly Asp Asp Pro Trp Val Gln His Ala Lys Trp Phe Pro Arg Cys Glu
305                 310                 315                 320

Tyr Leu Ile Arg Ile Lys Gly Gln Glu Phe Ile Arg Gln Val Gln Ala
                325                 330                 335

Ser Tyr Pro His Leu Leu Glu Gln Leu Leu Ser Thr Ser Asp Ser Pro
            340                 345                 350

Gly Asp Glu Asn Ala Glu Ser Ser Ile Ile His Phe Glu Pro Gly Glu
        355                 360                 365

Asp His Ser Glu Asp Ala Ile Met Met Asn Thr Pro Val Ile Asn Ala
    370                 375                 380

Ala Val Glu Met Gly Phe Ser Arg Ser Leu Val Lys Gln Thr Val Gln
385                 390                 395                 400

Arg Lys Ile Leu Ala Thr Gly Glu Asn Tyr Arg Leu Val Asn Asp Leu
                405                 410                 415

Val Leu Asp Leu Leu Asn Ala Glu Asp Glu Ile Arg Glu Glu Glu Arg
            420                 425                 430

Glu Arg Ala Thr Glu Glu Lys Glu Ser Asn Asp Leu Leu Leu Ile Arg
        435                 440                 445

Lys Asn Arg Met Ala Leu Phe Gln His Leu Thr Cys Val Ile Pro Ile
    450                 455                 460

Leu Asp Ser Leu Leu Thr Ala Gly Ile Ile Asn Glu Gln Glu His Asp
465                 470                 475                 480

Val Ile Lys Gln Lys Thr Gln Thr Ser Leu Gln Ala Arg Glu Leu Ile
                485                 490                 495

Asp Thr Ile Leu Val Lys Gly Asn Ile Ala Ala Thr Val Phe Arg Asn
            500                 505                 510

Ser Leu Gln Glu Ala Glu Ala Val Leu Tyr Glu His Leu Phe Val Gln
        515                 520                 525

Gln Asp Ile Lys Tyr Ile Pro Thr Glu Asp Val Ser Asp Leu Pro Val
    530                 535                 540

Glu Glu Gln Leu Arg Arg Leu Gln Glu Glu Arg Thr Cys Lys Val Cys
545                 550                 555                 560

Met Asp Lys Glu Val Ser Ile Val Phe Ile Pro Cys Gly His Leu Val
                565                 570                 575

Val Cys Lys Asp Cys Ala Pro Ser Leu Arg Lys Cys Pro Ile Cys Arg
            580                 585                 590

Ser Thr Ile Lys Gly Thr Val Arg Thr Phe Leu Ser
        595                 600

<210> 27
<211> 1400
<212> DNA
<213> Homo sapiens

<400> 27
cggcaaaaaa aaaaggcgta agaattttga agctatgttc aaaggtatcc ttcagagtgg      60

attggataac ttcgtgataa accacatgct aaagaacaac gtggctggac aaacatctat     120

ccagacccta gtacctaata cggatcaaaa gtcgaccagt gtaaaaaaag acaaccacaa     180

aaaaaaaaca gttaagatgt tggaatacct gggcaaagat gttcttcatg gtgtttttaa     240

ttatttggca aaacacgatg ttctgacatt gaaggaagag gaaaagaaaa aatattatga     300

tgccaaaatt gaagacaagg ccctgatctt ggtagactct ttgcgaaaga atcgcgtggc     360

tcatcaaatg tttacccaaa cacttctcaa tatggaccaa aagatcacca gtgtaaaacc     420

tcttctgcaa atcgaggctg gaccacctga gtcagcagaa tctacaaata tactcaaact     480

ttgtcctcgt gaagaattcc tgagactgtg taaaaaaaat catgatgaga tctatccaat     540

aaaaaagaga gaggaccgca gacgcctggc tctcatcata tgcaatacaa agtttgatca     600

cctgcctgca aggaatgggg ctcactatga catcgtgggg atgaaaaggc tgcttcaagg     660

cctgggctac actgtggttg acgaaaagaa tctcacagcc agggatatgg agtcagtgct     720

gagggcattt gctgccagac cagagcacaa gtcctctgac agcacgttct tggtactcat     780

gtctcatggc atcctagagg gaatctgcgg aactgcgcat aaaaagaaaa aaccggatgt     840

gctgctttat gacaccatct tccagatatt caacaaccgc aactgcctca gtctaaagga     900

caaacccaag gtcatcattg tccaggcctg cagaggtgaa aaacatgggg aactctgggt     960

cagagactct ccagcatcct tggcagtcat ctcttcacag tcatctgaga acctggaggc    1020

agattctgtt tgcaagatcc acgaggagaa ggacttcatt gctttctgtt cttcaacacc    1080

acataacgtg tcctggagag accgcacaag gggctccatc ttcattacgg aactcatcac    1140

atgcttccag aaatattctt gctgctgcca cctaatggaa atatttcgga aggtacagaa    1200

atcatttgaa gttccacagg ctaaagccca gatgcccacc atagaacgag caaccttgac    1260

aagagatttc tacctctttc ctggcaattg aaaatgaaac cacaggcagc ccagccctcc    1320

tctgtcaaca tcaaagagca catttaccag tatagcttgc atagtcaata tttggtattt    1380

caataaaagt aaagactgta                                                1400


<210> 28
<211> 418
<212> PRT
<213> Homo sapiens

<400> 28

Met Phe Lys Gly Ile Leu Gln Ser Gly Leu Asp Asn Phe Val Ile Asn
1               5                   10                  15

His Met Leu Lys Asn Asn Val Ala Gly Gln Thr Ser Ile Gln Thr Leu
            20                  25                  30

Val Pro Asn Thr Asp Gln Lys Ser Thr Ser Val Lys Lys Asp Asn His
        35                  40                  45

Lys Lys Lys Thr Val Lys Met Leu Glu Tyr Leu Gly Lys Asp Val Leu
    50                  55                  60

His Gly Val Phe Asn Tyr Leu Ala Lys His Asp Val Leu Thr Leu Lys
65                  70                  75                  80

Glu Glu Glu Lys Lys Lys Tyr Tyr Asp Ala Lys Ile Glu Asp Lys Ala
                85                  90                  95

Leu Ile Leu Val Asp Ser Leu Arg Lys Asn Arg Val Ala His Gln Met
            100                 105                 110

Phe Thr Gln Thr Leu Leu Asn Met Asp Gln Lys Ile Thr Ser Val Lys
        115                 120                 125

Pro Leu Leu Gln Ile Glu Ala Gly Pro Pro Glu Ser Ala Glu Ser Thr
    130                 135                 140

Asn Ile Leu Lys Leu Cys Pro Arg Glu Glu Phe Leu Arg Leu Cys Lys
145                 150                 155                 160

Lys Asn His Asp Glu Ile Tyr Pro Ile Lys Lys Arg Glu Asp Arg Arg
                165                 170                 175

Arg Leu Ala Leu Ile Ile Cys Asn Thr Lys Phe Asp His Leu Pro Ala
            180                 185                 190

Arg Asn Gly Ala His Tyr Asp Ile Val Gly Met Lys Arg Leu Leu Gln
        195                 200                 205

Gly Leu Gly Tyr Thr Val Val Asp Glu Lys Asn Leu Thr Ala Arg Asp
    210                 215                 220

Met Glu Ser Val Leu Arg Ala Phe Ala Ala Arg Pro Glu His Lys Ser
225                 230                 235                 240

Ser Asp Ser Thr Phe Leu Val Leu Met Ser His Gly Ile Leu Glu Gly

245                 250                 255

Ile Cys Gly Thr Ala His Lys Lys Lys Lys Pro Asp Val Leu Leu Tyr
            260                 265                 270

Asp Thr Ile Phe Gln Ile Phe Asn Asn Arg Asn Cys Leu Ser Leu Lys
        275                 280                 285

Asp Lys Pro Lys Val Ile Ile Val Gln Ala Cys Arg Gly Glu Lys His
    290                 295                 300

Gly Glu Leu Trp Val Arg Asp Ser Pro Ala Ser Leu Ala Val Ile Ser
305                 310                 315                 320

Ser Gln Ser Ser Glu Asn Leu Glu Ala Asp Ser Val Cys Lys Ile His
                325                 330                 335

Glu Glu Lys Asp Phe Ile Ala Phe Cys Ser Ser Thr Pro His Asn Val
            340                 345                 350

Ser Trp Arg Asp Arg Thr Arg Gly Ser Ile Phe Ile Thr Glu Leu Ile
        355                 360                 365

Thr Cys Phe Gln Lys Tyr Ser Cys Cys Cys His Leu Met Glu Ile Phe
    370                 375                 380

Arg Lys Val Gln Lys Ser Phe Glu Val Pro Gln Ala Lys Ala Gln Met
385                 390                 395                 400

Pro Thr Ile Glu Arg Ala Thr Leu Thr Arg Asp Phe Tyr Leu Phe Pro
                405                 410                 415

Gly Asn

<210> 29
<211> 5802
<212> DNA
<213> Homo sapiens

<400> 29
tttatagcag cagtagaaat ataccaccct agaggacaca cctcctttta gctaggtacc      60

tataaatgtc caggattttc tattcaattg agaagaaccc agcaaaatgg ggatctccac     120

agtcatcctt gaaatgtgtc ttttatgggg acaagttcta tctacaggtg ggtggatccc     180

aaggactaca gactacgctt cactgattcc ctcggaggtg cccttggatc caactgtagc     240

agaaggttct ccatttccct cggagtcgac cctggagtca actgtagcag aaggttctcc 300
gatttccttg gagtcaaccc tggagtcaac cgtagcagaa ggttctctga ttccctcaga 360
gtcaaccctg gagtcaactg tagcagaagg atctgattct ggtttggccc tgaggctggt 420
gaatggagat ggcaggtgtc agggccgagt ggagatccta taccgaggct cctggggcac 480
cgtgtgtgat gacagctggg acaccaatga tgccaacgtg gtctgtaggc agctgggttg 540
tggctgggcc atgtcagctc caggaaatgc ctggtttggc cagggctcag gacccattgc 600
cctggatgat gtgcgctgct caggacacga atcctacctg tggagctgcc cccacaatgg 660
ctggctctcc cataactgtg gccatggtga agatgctggt gttatctgct cagctgccca 720
gcctcagtca acactcaggc cagaaagttg gcctgtcagg atatcaccac ctgtacccac 780
agaaggatct gaatccagtt tggccctgag gctggtgaat ggaggcgaca ggtgtcgagg 840
ccgagtggag gtcctatacc gaggctcctg gggcaccgtg tgtgatgact actgggacac 900
caatgatgcc aatgtggtct gcaggcagct gggctgtggc tgggccatgt cagccccagg 960
aaatgcccag tttggccagg gctcaggacc cattgtcctg gatgatgtgc gctgctcagg 1020
acatgagtcc tacctgtgga gctgccccca caatggctgg ctcacccaca actgtggcca 1080
tagtgaagac gctggtgtca tctgctcagc tccccagtcc cggccgacac ccagcccaga 1140
tacttggccg acctcacatg catcaacagc aggacctgaa tccagtttgg ccctgaggct 1200
ggtgaatgga ggtgacaggt gtcagggccg agtggaggtc ctataccgag gctcctgggg 1260
caccgtgtgt gatgatagct gggacaccag tgacgccaat gtggtctgcc ggcagctggg 1320
ctgtggctgg gccacgtcag ccccaggaaa tgcccggttt ggccagggtt caggacccat 1380
tgtcctggat gacgtgcgct gctcaggcta tgagtcctac ctgtggagct gcccccacaa 1440
tggctggctc tcccataact gtcagcacag tgaagacgct ggtgtcatct gctcagctgc 1500
ccactcctgg tcgacgccca gtccagacac attgccgacc atcaccttgc ctgcatcgac 1560
agtaggatct gaatccagtt tggccctgag gctggtgaat ggaggtgaca ggtgtcaggg 1620
ccgagtggag gtcctatacc aaggctcctg gggcaccgtg tgcgatgaca gctgggacac 1680
caatgatgcc aatgtcgtct gcaggcaact gggctgtggc tgggccatgt cagccccagg 1740
aaatgcccgg tttggtcagg gctcaggacc cattgtcctg gatgatgtgc gctgctcagg 1800
acacgagtct tacctgtgga gctgccccca caatggctgg ctctcccaca actgtggcca 1860
tagtgaagac gctggtgtca tctgctcagc ttcccagtcc cggccaacac ctagtccaga 1920
cacttggcca acctcacatg catcaacagc aggatctgaa tccagtttgg ccctgaggct 1980
ggtgaatgga ggtgacaggt gtcagggccg agtggaggtc ctataccgag gctcctgggg 2040
caccgtgtgt gatgactact gggacaccaa tgatgccaat gtggtttgca ggcagctggg 2100

ctgtggctgg gccatgtcag ccccaggaaa tgcccggttt ggccagggtt caggacccat 2160

tgtcctggat gatgtgcgct gctcaggaca tgagtcctat ctgtggagct gcccccacaa 2220

tggctggctc tcccacaact gtggccatca tgaagacgct ggtgtcatct gctcagcttc 2280

ccagtcccag ccgacaccca gcccagacac ttggccaacc tcacatgcat caacagcagg 2340

atctgaatcc agtttggccc tgaggctggt gaatggaggt gacaggtgtc agggccgagt 2400

ggaggtccta taccgaggct cctggggcac cgtgtgtgat gactactggg acaccaatga 2460

tgccaatgtg gtttgcaggc agctgggctg tggctgggcc acgtcagccc caggaaatgc 2520

ccggtttggc cagggttcag gacccattgt cctggatgat gtgcgctgct caggacatga 2580

gtcctatctg tggagctgcc cccacaatgg ctggctctcc cacaactgtg gccatcatga 2640

agacgctggt gtcatctgct cagcttccca gtcccagccg acacccagcc cagacacttg 2700

gccaacctct cgtgcatcaa cagcaggatc tgaatccact ttggccctga gactggtgaa 2760

tggaggtgac aggtgtcgag gccgagtgga ggtcctatac caaggctcct ggggcaccgt 2820

gtgtgatgac tactgggaca ccaatgatgc caacgtggtc tgcaggcagc tgggctgtgg 2880

ctgggccatg tcagccccag gaaatgccca gtttggccag ggctcaggac ccattgtcct 2940

ggatgatgtg cgctgctcag gacacgagtc ttacctgtgg agctgccccc acaatggctg 3000

gctctcccac aactgtggcc atcatgaaga tgctggtgtc atctgctcag ctgctcagtc 3060

ccagtcaacg cccaggccag atacttggct gaccaccaac ttaccggcat tgacagtagg 3120

atctgaatcc agtttggctc tgaggctggt gaatggaggt gacaggtgtc gaggccgagt 3180

ggaggtcctg tatcgaggct cctggggaac cgtgtgtgat gacagctggg acaccaatga 3240

tgccaatgtg gtctgcaggc agctgggctg tggctgggcc atgtcggccc caggaaatgc 3300

ccggtttggc cagggctcag gacccattgt cctggatgat gtgcgctgct cagggaatga 3360

gtcctacctg tggagctgcc cccacaaagg ctggctcacc cacaactgtg gccatcacga 3420

agacgctggt gtcatctgct cagccaccca aataaattct actacgacag attggtggca 3480

tccaacaact acaaccactg caagaccctc ttcaaattgt ggtggcttct tattctatgc 3540

cagtgggaca ttctccagcc catcctaccc tgcatactac cccaacaatg ctaagtgtgt 3600

ttgggaaata gaagtgaatt ctggttatcg cataaacctg ggcttcagta atctgaaatt 3660

ggaggcacac cataactgca gttttgatta tgttgaaatc tttgatggat cattgaatag 3720

cagtctcctg ctggggaaaa tctgtaatga taccaggcaa atatttacat cttcttacaa 3780

ccgaatgacc attcactttc gaagtgacat cagtttccaa aacactggct tttggcttgg 3840

gtataactcc ttcccaagcg atgccacctt gaggttggtc aatttaaatt catcctatgg 3900

tctatgtgcc gggcgtgtag aaatttacca tggtggcacc tgggggacag tttgtgatga 3960

ctcctggacc attcaggaag ctgaggtggt ctgcagacag ctagggtgtg gacgtgcagt 4020

ttcagccctt ggaaatgcat attttggctc tggctctggc cccatcaccc tggacgatgt 4080

agagtgctca gggacggaat ccactctctg gcagtgccgg aaccgaggct ggttctccca 4140

caactgtaat catcgtgaag atgctggtgt catctgctca ggaaaccatc tatcgacacc 4200

tgctcctttt ctcaacatca cccgtccaaa cacagattat tcctgcggag gcttcctatc 4260

ccaaccatca ggggactttt ccagcccatt ctatcccggg aactatccaa acaatgccaa 4320

gtgtgtgtgg gacattgagg tgcaaaacaa ctaccgtgtg actgtgatct tcagagatgt 4380

ccagcttgaa ggtggctgca actatgatta tattgaagtt ttcgatggcc cctaccgcag 4440

ttcccctctc attgctcgag tttgtgatgg ggccagaggc tccttcactt cttcctccaa 4500

cttcatgtcc attcgcttca tcagtgacca cagcatcaca aggagagggt tccgggctga 4560

gtactactcc agtccctcca atgacagcac caacctgctc tgtctgccaa atcacatgca 4620

agccagtgtg agcaggagct atctccaatc cttgggcttt tctgccagtg accttgtcat 4680

ttccacctgg aatggatact acgagtgtcg gccccagata acgccgaacc tggtgatatt 4740

cacaattccc tactcaggct gcggcacctt caagcaggca gacaatgaca ccatcgacta 4800

ttccaacttc ctcacagcag ctgtctcagg tggcatcatc aagaggagga cagacctccg 4860

tattcacgtc agctgcagaa tgcttcagaa cacctgggtc gacaccatgt acattgctaa 4920

tgacaccatc cacgttgcta ataacaccat ccaggtcgag gaagtccagt atggcaattt 4980

tgacgtgaac atttcctttt atacttcctc atctttcttg tatcctgtga ccagccgccc 5040

ttactacgtg gacctgaacc aggacttgta cgttcaggct gaaatcctcc attctgatgc 5100

tgtactgacc ttgtttgtgg acacctgcgt ggcatcacca tactccaatg acttcacgtc 5160

tttgacttat gatctaatcc ggagtggatg cgtgagggat gacacctacg gaccctactc 5220

ctcgccatct cttcgcattg cccgcttccg gttcagggcc ttccacttcc tgaaccgctt 5280

cccctccgtg tacctgcgtt gtaaaatggt ggtgtgcaga gcgtatgacc cctcttcccg 5340

ctgctaccga ggctgtgtgt tgaggtcgaa gagggatgtg ggctcctacc aggaaaaggt 5400

ggacgtcgtc ctgggtccca tccagctgca gaccccccca cgccgagaag aggagcctcg 5460

gtaggtggtc gctctcagac cccactgtcc accggggcgc agacccctga ctcggggact 5520

tgggatgttc ctcttggtgt catattccaa ctcagattga gccctacatt gtgctgcacc 5580

tggtcatacg gagttgaatc agacctggtt cccgcctccc ccaaggctca tggtccttgg 5640

aggacccgtt gcagggtgag gtcaagagag ttctgacctg gatggcccat agacctgacg 5700

tcccagaatc catgcttctc atctgcaaaa tgaaaatgtc aatacttact tcttagcact 5760

gttgagaggg ttacttacat aaaggaattt tggtgaaact gc                    5802

<210> 30
<211> 1785
<212> PRT
<213> Homo sapiens

<400> 30
Met Gly Ile Ser Thr Val Ile Leu Glu Met Cys Leu Leu Trp Gly Gln
1               5                   10                  15

Val Leu Ser Thr Gly Gly Trp Ile Pro Arg Thr Thr Asp Tyr Ala Ser
            20                  25                  30

Leu Ile Pro Ser Glu Val Pro Leu Asp Pro Thr Val Ala Glu Gly Ser
        35                  40                  45

Pro Phe Pro Ser Glu Ser Thr Leu Glu Ser Thr Val Ala Glu Gly Ser
    50                  55                  60

Pro Ile Ser Leu Glu Ser Thr Leu Glu Ser Thr Val Ala Glu Gly Ser
65                  70                  75                  80

Leu Ile Pro Ser Glu Ser Thr Leu Glu Ser Thr Val Ala Glu Gly Ser
                85                  90                  95

Asp Ser Gly Leu Ala Leu Arg Leu Val Asn Gly Asp Gly Arg Cys Gln
            100                 105                 110

Gly Arg Val Glu Ile Leu Tyr Arg Gly Ser Trp Gly Thr Val Cys Asp
        115                 120                 125

Asp Ser Trp Asp Thr Asn Asp Ala Asn Val Val Cys Arg Gln Leu Gly
    130                 135                 140

Cys Gly Trp Ala Met Ser Ala Pro Gly Asn Ala Trp Phe Gly Gln Gly
145                 150                 155                 160

Ser Gly Pro Ile Ala Leu Asp Asp Val Arg Cys Ser Gly His Glu Ser
                165                 170                 175

Tyr Leu Trp Ser Cys Pro His Asn Gly Trp Leu Ser His Asn Cys Gly
            180                 185                 190

His Gly Glu Asp Ala Gly Val Ile Cys Ser Ala Ala Gln Pro Gln Ser
        195                 200                 205

Thr Leu Arg Pro Glu Ser Trp Pro Val Arg Ile Ser Pro Pro Val Pro
    210                 215                 220

Thr Glu Gly Ser Glu Ser Ser Leu Ala Leu Arg Leu Val Asn Gly Gly
225                 230                 235                 240

Asp Arg Cys Arg Gly Arg Val Glu Val Leu Tyr Arg Gly Ser Trp Gly
                245                 250                 255

Thr Val Cys Asp Asp Tyr Trp Asp Thr Asn Asp Ala Asn Val Val Cys
            260                 265                 270

Arg Gln Leu Gly Cys Gly Trp Ala Met Ser Ala Pro Gly Asn Ala Gln
        275                 280                 285

Phe Gly Gln Gly Ser Gly Pro Ile Val Leu Asp Asp Val Arg Cys Ser
    290                 295                 300

Gly His Glu Ser Tyr Leu Trp Ser Cys Pro His Asn Gly Trp Leu Thr
305                 310                 315                 320

His Asn Cys Gly His Ser Glu Asp Ala Gly Val Ile Cys Ser Ala Pro
                325                 330                 335

Gln Ser Arg Pro Thr Pro Ser Pro Asp Thr Trp Pro Thr Ser His Ala
            340                 345                 350

Ser Thr Ala Gly Pro Glu Ser Ser Leu Ala Leu Arg Leu Val Asn Gly
        355                 360                 365

Gly Asp Arg Cys Gln Gly Arg Val Glu Val Leu Tyr Arg Gly Ser Trp
    370                 375                 380

Gly Thr Val Cys Asp Asp Ser Trp Asp Thr Ser Asp Ala Asn Val Val
385                 390                 395                 400

Cys Arg Gln Leu Gly Cys Gly Trp Ala Thr Ser Ala Pro Gly Asn Ala
                405                 410                 415

Arg Phe Gly Gln Gly Ser Gly Pro Ile Val Leu Asp Asp Val Arg Cys
            420                 425                 430

Ser Gly Tyr Glu Ser Tyr Leu Trp Ser Cys Pro His Asn Gly Trp Leu
        435                 440                 445

Ser His Asn Cys Gln His Ser Glu Asp Ala Gly Val Ile Cys Ser Ala
    450                 455                 460

Ala His Ser Trp Ser Thr Pro Ser Pro Asp Thr Leu Pro Thr Ile Thr
465                 470                 475                 480

Leu Pro Ala Ser Thr Val Gly Ser Glu Ser Ser Leu Ala Leu Arg Leu
                485                 490                 495

Val Asn Gly Gly Asp Arg Cys Gln Gly Arg Val Glu Val Leu Tyr Gln
            500                 505                 510

Gly Ser Trp Gly Thr Val Cys Asp Asp Ser Trp Asp Thr Asn Asp Ala
        515                 520                 525

Asn Val Val Cys Arg Gln Leu Gly Cys Gly Trp Ala Met Ser Ala Pro
    530                 535                 540

Gly Asn Ala Arg Phe Gly Gln Gly Ser Gly Pro Ile Val Leu Asp Asp
545                 550                 555                 560

Val Arg Cys Ser Gly His Glu Ser Tyr Leu Trp Ser Cys Pro His Asn
                565                 570                 575

Gly Trp Leu Ser His Asn Cys Gly His Ser Glu Asp Ala Gly Val Ile
            580                 585                 590

Cys Ser Ala Ser Gln Ser Arg Pro Thr Pro Ser Pro Asp Thr Trp Pro
        595                 600                 605

Thr Ser His Ala Ser Thr Ala Gly Ser Glu Ser Ser Leu Ala Leu Arg
    610                 615                 620

Leu Val Asn Gly Gly Asp Arg Cys Gln Gly Arg Val Glu Val Leu Tyr
625                 630                 635                 640

Arg Gly Ser Trp Gly Thr Val Cys Asp Asp Tyr Trp Asp Thr Asn Asp
                645                 650                 655

Ala Asn Val Val Cys Arg Gln Leu Gly Cys Gly Trp Ala Met Ser Ala
            660                 665                 670

Pro Gly Asn Ala Arg Phe Gly Gln Gly Ser Gly Pro Ile Val Leu Asp
        675                 680                 685

Asp Val Arg Cys Ser Gly His Glu Ser Tyr Leu Trp Ser Cys Pro His

690                 695                 700

Asn Gly Trp Leu Ser His Asn Cys Gly His His Glu Asp Ala Gly Val
705                 710                 715                 720

Ile Cys Ser Ala Ser Gln Ser Gln Pro Thr Pro Ser Pro Asp Thr Trp
                725                 730                 735

Pro Thr Ser His Ala Ser Thr Ala Gly Ser Glu Ser Ser Leu Ala Leu
            740                 745                 750

Arg Leu Val Asn Gly Gly Asp Arg Cys Gln Gly Arg Val Glu Val Leu
        755                 760                 765

Tyr Arg Gly Ser Trp Gly Thr Val Cys Asp Asp Tyr Trp Asp Thr Asn
    770                 775                 780

Asp Ala Asn Val Val Cys Arg Gln Leu Gly Cys Gly Trp Ala Thr Ser
785                 790                 795                 800

Ala Pro Gly Asn Ala Arg Phe Gly Gln Gly Ser Gly Pro Ile Val Leu
                805                 810                 815

Asp Asp Val Arg Cys Ser Gly His Glu Ser Tyr Leu Trp Ser Cys Pro
            820                 825                 830

His Asn Gly Trp Leu Ser His Asn Cys Gly His His Glu Asp Ala Gly
        835                 840                 845

Val Ile Cys Ser Ala Ser Gln Ser Gln Pro Thr Pro Ser Pro Asp Thr
    850                 855                 860

Trp Pro Thr Ser Arg Ala Ser Thr Ala Gly Ser Glu Ser Thr Leu Ala
865                 870                 875                 880

Leu Arg Leu Val Asn Gly Gly Asp Arg Cys Arg Gly Arg Val Glu Val
                885                 890                 895

Leu Tyr Gln Gly Ser Trp Gly Thr Val Cys Asp Asp Tyr Trp Asp Thr
            900                 905                 910

Asn Asp Ala Asn Val Val Cys Arg Gln Leu Gly Cys Gly Trp Ala Met
        915                 920                 925

Ser Ala Pro Gly Asn Ala Gln Phe Gly Gln Gly Ser Gly Pro Ile Val
    930                 935                 940

Leu Asp Asp Val Arg Cys Ser Gly His Glu Ser Tyr Leu Trp Ser Cys
945                 950                 955                 960

Pro His Asn Gly Trp Leu Ser His Asn Cys Gly His His Glu Asp Ala
                965                 970                 975

Gly Val Ile Cys Ser Ala Ala Gln Ser Gln Ser Thr Pro Arg Pro Asp
            980                 985                 990

Thr Trp Leu Thr Thr Asn Leu Pro  Ala Leu Thr Val Gly  Ser Glu Ser
        995                 1000                 1005

Ser Leu  Ala Leu Arg Leu Val  Asn Gly Gly Asp Arg  Cys Arg Gly
    1010                 1015                 1020

Arg Val  Glu Val Leu Tyr Arg  Gly Ser Trp Gly Thr  Val Cys Asp
    1025                 1030                 1035

Asp Ser  Trp Asp Thr Asn Asp  Ala Asn Val Val Cys  Arg Gln Leu
    1040                 1045                 1050

Gly Cys  Gly Trp Ala Met Ser  Ala Pro Gly Asn Ala  Arg Phe Gly
    1055                 1060                 1065

Gln Gly  Ser Gly Pro Ile Val  Leu Asp Asp Val Arg  Cys Ser Gly
    1070                 1075                 1080

Asn Glu  Ser Tyr Leu Trp Ser  Cys Pro His Lys Gly  Trp Leu Thr
    1085                 1090                 1095

His Asn  Cys Gly His His Glu  Asp Ala Gly Val Ile  Cys Ser Ala
    1100                 1105                 1110

Thr Gln  Ile Asn Ser Thr Thr  Thr Asp Trp Trp His  Pro Thr Thr
    1115                 1120                 1125

Thr Thr  Thr Ala Arg Pro Ser  Ser Asn Cys Gly Gly  Phe Leu Phe
    1130                 1135                 1140

Tyr Ala  Ser Gly Thr Phe Ser  Ser Pro Ser Tyr Pro  Ala Tyr Tyr
    1145                 1150                 1155

Pro Asn  Asn Ala Lys Cys Val  Trp Glu Ile Glu Val  Asn Ser Gly
    1160                 1165                 1170

Tyr Arg Ile Asn Leu Gly Phe Ser Asn Leu Lys Leu Glu Ala His
    1175                1180                1185

His Asn Cys Ser Phe Asp Tyr Val Glu Ile Phe Asp Gly Ser Leu
    1190                1195                1200

Asn Ser Ser Leu Leu Leu Gly Lys Ile Cys Asn Asp Thr Arg Gln
    1205                1210                1215

Ile Phe Thr Ser Ser Tyr Asn Arg Met Thr Ile His Phe Arg Ser
    1220                1225                1230

Asp Ile Ser Phe Gln Asn Thr Gly Phe Leu Ala Trp Tyr Asn Ser
    1235                1240                1245

Phe Pro Ser Asp Ala Thr Leu Arg Leu Val Asn Leu Asn Ser Ser
    1250                1255                1260

Tyr Gly Leu Cys Ala Gly Arg Val Glu Ile Tyr His Gly Gly Thr
    1265                1270                1275

Trp Gly Thr Val Cys Asp Asp Ser Trp Thr Ile Gln Glu Ala Glu
    1280                1285                1290

Val Val Cys Arg Gln Leu Gly Cys Gly Arg Ala Val Ser Ala Leu
    1295                1300                1305

Gly Asn Ala Tyr Phe Gly Ser Gly Ser Gly Pro Ile Thr Leu Asp
    1310                1315                1320

Asp Val Glu Cys Ser Gly Thr Glu Ser Thr Leu Trp Gln Cys Arg
    1325                1330                1335

Asn Arg Gly Trp Phe Ser His Asn Cys Asn His Arg Glu Asp Ala
    1340                1345                1350

Gly Val Ile Cys Ser Gly Asn His Leu Ser Thr Pro Ala Pro Phe
    1355                1360                1365

Leu Asn Ile Thr Arg Pro Asn Thr Asp Tyr Ser Cys Gly Gly Phe
    1370                1375                1380

Leu Ser Gln Pro Ser Gly Asp Phe Ser Ser Pro Phe Tyr Pro Gly
    1385                1390                1395

Asn Tyr Pro Asn Asn Ala Lys Cys Val Trp Asp Ile Glu Val Gln
    1400                1405                1410

Asn Asn Tyr Arg Val Thr Val Ile Phe Arg Asp Val Gln Leu Glu
    1415                1420                1425

Gly Gly Cys Asn Tyr Asp Tyr Ile Glu Val Phe Asp Gly Pro Tyr
    1430                1435                1440

Arg Ser Ser Pro Leu Ile Ala Arg Val Cys Asp Gly Ala Arg Gly
    1445                1450                1455

Ser Phe Thr Ser Ser Ser Asn Phe Met Ser Ile Arg Phe Ile Ser
    1460                1465                1470

Asp His Ser Ile Thr Arg Arg Gly Phe Arg Ala Glu Tyr Tyr Ser
    1475                1480                1485

Ser Pro Ser Asn Asp Ser Thr Asn Leu Leu Cys Leu Pro Asn His
    1490                1495                1500

Met Gln Ala Ser Val Ser Arg Ser Tyr Leu Gln Ser Leu Gly Phe
    1505                1510                1515

Ser Ala Ser Asp Leu Val Ile Ser Thr Trp Asn Gly Tyr Tyr Glu
    1520                1525                1530

Cys Arg Pro Gln Ile Thr Pro Asn Leu Val Ile Phe Thr Ile Pro
    1535                1540                1545

Tyr Ser Gly Cys Gly Thr Phe Lys Gln Ala Asp Asn Asp Thr Ile
    1550                1555                1560

Asp Tyr Ser Asn Phe Leu Thr Ala Ala Val Ser Gly Gly Ile Ile
    1565                1570                1575

Lys Arg Arg Thr Asp Leu Arg Ile His Val Ser Cys Arg Met Leu
    1580                1585                1590

Gln Asn Thr Trp Val Asp Thr Met Tyr Ile Ala Asn Asp Thr Ile
    1595                1600                1605

His Val Ala Asn Asn Thr Ile Gln Val Glu Glu Val Gln Tyr Gly
    1610                1615                1620

Asn Phe Asp Val Asn Ile Ser Phe Tyr Thr Ser Ser Ser Phe Leu

1625                1630                1635

Tyr Pro Val Thr Ser Arg Pro Tyr Tyr Val Asp Leu Asn Gln Asp
    1640                1645                1650

Leu Tyr Val Gln Ala Glu Ile Leu His Ser Asp Ala Val Leu Thr
    1655                1660                1665

Leu Phe Val Asp Thr Cys Val Ala Ser Pro Tyr Ser Asn Asp Phe
    1670                1675                1680

Thr Ser Leu Thr Tyr Asp Leu Ile Arg Ser Gly Cys Val Arg Asp
    1685                1690                1695

Asp Thr Tyr Gly Pro Tyr Ser Ser Pro Ser Leu Arg Ile Ala Arg
    1700                1705                1710

Phe Arg Phe Arg Ala Phe His Phe Leu Asn Arg Phe Pro Ser Val
    1715                1720                1725

Tyr Leu Arg Cys Lys Met Val Val Cys Arg Ala Tyr Asp Pro Ser
    1730                1735                1740

Ser Arg Cys Tyr Arg Gly Cys Val Leu Arg Ser Lys Arg Asp Val
    1745                1750                1755

Gly Ser Tyr Gln Glu Lys Val Asp Val Val Leu Gly Pro Ile Gln
    1760                1765                1770

Leu Gln Thr Pro Pro Arg Arg Glu Glu Glu Pro Arg
    1775                1780                1785

<210> 31
<211> 4966
<212> DNA
<213> Homo sapiens

<400> 31
gtagatgcag tccgccgccg ccgctgcctc agccagcaat gcaagattag atctctaaat      60

gcagcaaaac actgcctgaa aacagaccgg cccgcgcagc aagcagacat ttcacggtgc     120

gctggggaag cttcaaaata tatctgtgac tctgtcttcg ttgctcttca tccccatcaa     180

tttcatcacg ggaggcgagc agcaagtaag aatttcactt tcggatctgc ctagagacac     240

acctccctgc tccctccccc actcgatgtg aagagtattc cggagtctcc gggcgggagt     300

agatttgcag caccctagcg ggagcgagga aaacctactg attctttagc tcattatcat     360

ctctcccaga cgagatttcc ttcttatcgc ctgcctcatc gctcaagttt gagcctcccg 420
aagtccgggc gggagagacg aaacccctgg ctcaccccca gccgcaggaa gccaccgcct 480
tgctccaagc ccctgcagct ctgctgcacc gcagcttctc acccagtgcg gatgctgtag 540
atcaacaggt tcagggaact tgagcagaat aaggagagac caccgggtgc cgcagctcgg 600
gtgcagaggg aaaaaaggac ccatagactt gtggctcgcg tcgcgcgcgc acgctgcgcc 660
agggccccag gctggcgcgc actccctctc tggctcctcc agtccgattg ctcctgcccc 720
caccttacag gtctgggatg tacctttcca tctgttgctg ctttcttcta tgggcccctg 780
ccctcactct caagaacctc aactactccg tgccggagga gcaaggggcc ggcacggtga 840
tcgggaacat cggcagggat gctcgactgc agcctgggct tccgcctgca gagcgcggcg 900
gcggagggcg cagcaagtcg ggtagctacc gggtgctgga gaactccgca ccgcacctgc 960
tggacgtgga cgcagacagc gggctcctct acaccaagca gcgcatcgac cgcgagtccc 1020
tgtgccgcca caatgccaag tgccagctgt ccctcgaggt gttcgccaac gacaaggaga 1080
tctgcatgat caaggtagag atccaggaca tcaacgacaa cgcgccctcc ttctcctcgg 1140
accagatcga aatggacatc tcggagaacg ctgctccggg cacccgcttc cccctcacca 1200
gcgcacatga ccccgacgcc ggcgagaatg ggctccgcac ctacctgctc acgcgcgacg 1260
atcacggcct ctttggactg gacgttaagt cccgcggcga cggcaccaag ttcccagaac 1320
tggtcatcca gaaggctctg gaccgcgagc aacagaatca ccatacgctc gtgctgactg 1380
ccctggacgg tggcgagcct ccacgttccg ccaccgtaca gatcaacgtg aaggtgattg 1440
actccaacga caacagcccg gtcttcgagg cgccatccta cttggtggaa ctgcccgaga 1500
acgctccgct gggtacagtg gtcatcgatc tgaacgccac cgacgccgat gaaggtccca 1560
atggtgaagt gctctactct ttcagcagct acgtgcctga ccgcgtgcgg gagctcttct 1620
ccatcgaccc caagaccggc ctaatccgtg tgaagggcaa tctggactat gaggaaaacg 1680
ggatgctgga gattgacgtg caggcccgag acctggggcc taaccctatc ccagcccact 1740
gcaaagtcac ggtcaagctc atcgaccgca acgacaatgc gccgtccatc ggtttcgtct 1800
ccgtgcgcca gggggcgctg agcgaggccg cccctcccgg caccgtcatc gccctggtgc 1860
gggtcactga ccgggactct ggcaagaacg gacagctgca gtgtcgggtc ctaggcggag 1920
gagggacggg cggcggcggg ggcctgggcg ggcccggggg ttccgtcccc ttcaagcttg 1980
aggagaacta cgacaacttc tacacggtgg tgactgaccg cccgctggac cgcgagacac 2040
aagacgagta caacgtgacc atcgtggcgc gggacggggg ctctcctccc ctcaactcca 2100
ccaagtcgtt cgcgatcaag attctagacg agaacgacaa cccgcctcgg ttcaccaaag 2160
ggctctacgt gcttcaggtg cacgagaaca acatcccggg agagtacctg ggctctgtgc 2220

tcgcccagga tcccgacctg ggccagaacg gcaccgtatc ctactctatc ctgccctcgc 2280

acatcggcga cgtgtctatc tacacctatg tgtctgtgaa tcccacgaac ggggccatct 2340

acgccctgcg ctcctttaac ttcgagcaga ccaaggcttt tgagttcaag gtgcttgcta 2400

aggactcggg ggcgcccgcg cacttggaga gcaacgccac ggtgagggtg acagtgctag 2460

acgtgaatga caacgcgcca gtgatcgtgc tccccacgct gcagaacgac accgcggagc 2520

tgcaggtgcc gcgcaacgct ggcctgggct atctggtgag cactgtgcgc gccctagaca 2580

gcgacttcgg cgagagcggg cgtctcacct acgagatcgt ggacggcaac gacgaccacc 2640

tgtttgagat cgacccgtcc agcggcgaga tccgcacgct gcaccctttc tgggaggacg 2700

tgacgcccgt ggtggagctg gtggtgaagg tgaccgacca cggcaagcct accctgtccg 2760

cagtggccaa gctcatcatc cgctcggtga gcggatccct tcccgagggg gtaccacggg 2820

tgaatggcga gcagcaccac tgggacatgt cgctgccgct catcgtgact ctgagcacta 2880

tctccatcat cctcctagcg gccatgatca ccatcgccgt caagtgcaag cgcgagaaca 2940

aggagatccg cacttacaac tgccgcatcg ccgagtacag ccacccgcag ctgggtgggg 3000

gcaagggcaa gaagaagaag atcaacaaaa atgatatcat gctggtgcag agcgaagtgg 3060

aggagaggaa cgccatgaac gtcatgaacg tggtgagcag cccctccctg gccacctccc 3120

ccatgtactt cgactaccag acccgcctgc ccctcagctc gccccggtcg gaggtgatgt 3180

atctcaaacc ggcctccaac aacctgactg tccctcaggg gcacgcgggc tgccacacca 3240

gcttcaccgg acaagggact aatgcaagcg agacccctgc cactcggatg tccataattc 3300

agacagacaa ttttcccgca gagcccaatt acatgggcag caggcagcag tttgttcaaa 3360

gtatttcagt agctccacgt ttaaggaccc agaaagagcc agcctgagag acagtgggca 3420

cggggacagt gatcaggctg acagtgacca agacactaac aaaggctcct gctgtgacat 3480

gtctgttagg gaggcactca agatgaaaac tacttcaact aaaagccaac cacttgaaca 3540

agaaccagaa gagtgtgtta attgcacaga tgaatgccga gtgcttggtc attctgacag 3600

gtgctggatg ccacagttcc ctgcagccaa tcaggctgaa aatgcagatt accgcacaaa 3660

tctctttgta cctacagttg aagctaatgt tgagactgag acttacgaaa ctgtgaatcc 3720

cactgggaaa aagacttttt gtacatttgg aaaagacaag cgagagcaca ctattctcat 3780

tgccaacgtt aaaccttatt taaaagccaa acgtgccctg agccctctcc tccaagaggt 3840

cccctcagca tcaagcagcc caaccaaggc gtgcatcgag ccttgcacct caacaaaagg 3900

ctccctggat ggctgtgaag caaaaccagg agccctggct gaagcaagca gtcagtactt 3960

gcccactgac agtcaatatc tgtcacctag taagcaacca agagaccctc ccttcatggc 4020

ttccgatcag atggcaaggg tctttgcaga tgtgcattcc agagccagcc gggattccag 4080

tgagatgggt gctgttcttg agcagcttga ccaccccaac agggatctgg gcagagagtc 4140

tgtggatgca gaggaagttg tgagagaaat tgataagctt ttgcaagact gccggggaaa 4200

cgaccctgtg gctgtgagaa agtgaaaaaa gaaaaaaaaa aaggcattgg cattttcttg 4260

tctcttctgt tgatttaaaa atgatccctc ctggtgataa cccattttac agggatgaag 4320

aaagaccaat gctgctttaa ggcttttagt gaacatctga agtgcccaca agtatgttct 4380

ttccactgct gatttctttt tcagagataa caatggtttc gttttgacca aacttgtatt 4440

aggacagaat taatgatgct taaagagaaa agaaaaaaag agagaagaaa aaggagagat 4500

gaaaaaggag gatgaggaga agaattacct tttgacaatc tgttaggaag gtatgcagtg 4560

tgagaactga agtatttctg atcactctca gactgtcctc cgtgatttat gctgacttaa 4620

ctgtttacct ataaacccca tacaaagcag ggtcataatt tgtgatctgt ggtggatttc 4680

tagcagtcat cacaggcttc tactgaaagt cctgaaaaga ccttgcagta gtccaagcta 4740

caccaaacat taacacatat ttgtggtaaa catttctgta taaagttacc tgacacacat 4800

ataaacacaa ggaacattcc atatcattag tcgaaaacaa aaacaaaaaa aaaacctttg 4860

gtcatttgta agacatctca tgtcatataa aagttaaatg taaaaagata cagtccattt 4920

tgtcctgcac acacgtagac taattcacgt caaaaaaaaa aaaaaa 4966

<210> 32
<211> 889
<212> PRT
<213> Homo sapiens

<400> 32
Met Tyr Leu Ser Ile Cys Cys Cys Phe Leu Leu Trp Ala Pro Ala Leu
1               5                   10                  15

Thr Leu Lys Asn Leu Asn Tyr Ser Val Pro Glu Glu Gln Gly Ala Gly
            20                  25                  30

Thr Val Ile Gly Asn Ile Gly Arg Asp Ala Arg Leu Gln Pro Gly Leu
        35                  40                  45

Pro Pro Ala Glu Arg Gly Gly Gly Gly Arg Ser Lys Ser Gly Ser Tyr
    50                  55                  60

Arg Val Leu Glu Asn Ser Ala Pro His Leu Leu Asp Val Asp Ala Asp
65                  70                  75                  80

Ser Gly Leu Leu Tyr Thr Lys Gln Arg Ile Asp Arg Glu Ser Leu Cys
                85                  90                  95

Arg His Asn Ala Lys Cys Gln Leu Ser Leu Glu Val Phe Ala Asn Asp
            100                 105                 110

Lys Glu Ile Cys Met Ile Lys Val Glu Ile Gln Asp Ile Asn Asp Asn
        115                 120                 125

Ala Pro Ser Phe Ser Ser Asp Gln Ile Glu Met Asp Ile Ser Glu Asn
    130                 135                 140

Ala Ala Pro Gly Thr Arg Phe Pro Leu Thr Ser Ala His Asp Pro Asp
145                 150                 155                 160

Ala Gly Glu Asn Gly Leu Arg Thr Tyr Leu Leu Thr Arg Asp Asp His
                165                 170                 175

Gly Leu Phe Gly Leu Asp Val Lys Ser Arg Gly Asp Gly Thr Lys Phe
            180                 185                 190

Pro Glu Leu Val Ile Gln Lys Ala Leu Asp Arg Glu Gln Gln Asn His
        195                 200                 205

His Thr Leu Val Leu Thr Ala Leu Asp Gly Gly Glu Pro Pro Arg Ser
    210                 215                 220

Ala Thr Val Gln Ile Asn Val Lys Val Ile Asp Ser Asn Asp Asn Ser
225                 230                 235                 240

Pro Val Phe Glu Ala Pro Ser Tyr Leu Val Glu Leu Pro Glu Asn Ala
                245                 250                 255

Pro Leu Gly Thr Val Val Ile Asp Leu Asn Ala Thr Asp Ala Asp Glu
            260                 265                 270

Gly Pro Asn Gly Glu Val Leu Tyr Ser Phe Ser Ser Tyr Val Pro Asp
        275                 280                 285

Arg Val Arg Glu Leu Phe Ser Ile Asp Pro Lys Thr Gly Leu Ile Arg
    290                 295                 300

Val Lys Gly Asn Leu Asp Tyr Glu Glu Asn Gly Met Leu Glu Ile Asp
305                 310                 315                 320

Val Gln Ala Arg Asp Leu Gly Pro Asn Pro Ile Pro Ala His Cys Lys
                325                 330                 335

Val Thr Val Lys Leu Ile Asp Arg Asn Asp Asn Ala Pro Ser Ile Gly
            340                 345                 350

Phe Val Ser Val Arg Gln Gly Ala Leu Ser Glu Ala Ala Pro Pro Gly
        355                 360                 365

Thr Val Ile Ala Leu Val Arg Val Thr Asp Arg Asp Ser Gly Lys Asn
    370                 375                 380

Gly Gln Leu Gln Cys Arg Val Leu Gly Gly Gly Gly Thr Gly Gly Gly
385                 390                 395                 400

Gly Gly Leu Gly Gly Pro Gly Gly Ser Val Pro Phe Lys Leu Glu Glu
                405                 410                 415

Asn Tyr Asp Asn Phe Tyr Thr Val Val Thr Asp Arg Pro Leu Asp Arg
            420                 425                 430

Glu Thr Gln Asp Glu Tyr Asn Val Thr Ile Val Ala Arg Asp Gly Gly
        435                 440                 445

Ser Pro Pro Leu Asn Ser Thr Lys Ser Phe Ala Ile Lys Ile Leu Asp
    450                 455                 460

Glu Asn Asp Asn Pro Pro Arg Phe Thr Lys Gly Leu Tyr Val Leu Gln
465                 470                 475                 480

Val His Glu Asn Asn Ile Pro Gly Glu Tyr Leu Gly Ser Val Leu Ala
                485                 490                 495

Gln Asp Pro Asp Leu Gly Gln Asn Gly Thr Val Ser Tyr Ser Ile Leu
            500                 505                 510

Pro Ser His Ile Gly Asp Val Ser Ile Tyr Thr Tyr Val Ser Val Asn
        515                 520                 525

Pro Thr Asn Gly Ala Ile Tyr Ala Leu Arg Ser Phe Asn Phe Glu Gln
    530                 535                 540

Thr Lys Ala Phe Glu Phe Lys Val Leu Ala Lys Asp Ser Gly Ala Pro
545                 550                 555                 560

Ala His Leu Glu Ser Asn Ala Thr Val Arg Val Thr Val Leu Asp Val
                565                 570                 575

Asn Asp Asn Ala Pro Val Ile Val Leu Pro Thr Leu Gln Asn Asp Thr
            580                 585                 590

Ala Glu Leu Gln Val Pro Arg Asn Ala Gly Leu Gly Tyr Leu Val Ser
        595                 600                 605

Thr Val Arg Ala Leu Asp Ser Asp Phe Gly Glu Ser Gly Arg Leu Thr
    610                 615                 620

Tyr Glu Ile Val Asp Gly Asn Asp Asp His Leu Phe Glu Ile Asp Pro
625                 630                 635                 640

Ser Ser Gly Glu Ile Arg Thr Leu His Pro Phe Trp Glu Asp Val Thr
                645                 650                 655

Pro Val Val Glu Leu Val Val Lys Val Thr Asp His Gly Lys Pro Thr
            660                 665                 670

Leu Ser Ala Val Ala Lys Leu Ile Ile Arg Ser Val Ser Gly Ser Leu
        675                 680                 685

Pro Glu Gly Val Pro Arg Val Asn Gly Glu Gln His His Trp Asp Met
    690                 695                 700

Ser Leu Pro Leu Ile Val Thr Leu Ser Thr Ile Ser Ile Ile Leu Leu
705                 710                 715                 720

Ala Ala Met Ile Thr Ile Ala Val Lys Cys Lys Arg Glu Asn Lys Glu
                725                 730                 735

Ile Arg Thr Tyr Asn Cys Arg Ile Ala Glu Tyr Ser His Pro Gln Leu
            740                 745                 750

Gly Gly Gly Lys Gly Lys Lys Lys Lys Ile Asn Lys Asn Asp Ile Met
        755                 760                 765

Leu Val Gln Ser Glu Val Glu Glu Arg Asn Ala Met Asn Val Met Asn
    770                 775                 780

Val Val Ser Ser Pro Ser Leu Ala Thr Ser Pro Met Tyr Phe Asp Tyr
785                 790                 795                 800

Gln Thr Arg Leu Pro Leu Ser Ser Pro Arg Ser Glu Val Met Tyr Leu
                805                 810                 815

Lys Pro Ala Ser Asn Asn Leu Thr Val Pro Gln Gly His Ala Gly Cys

820                 825                 830

His Thr Ser Phe Thr Gly Gln Gly Thr Asn Ala Ser Glu Thr Pro Ala
        835                 840                 845

Thr Arg Met Ser Ile Ile Gln Thr Asp Asn Phe Pro Ala Glu Pro Asn
    850                 855                 860

Tyr Met Gly Ser Arg Gln Gln Phe Val Gln Ser Ile Ser Val Ala Pro
865                 870                 875                 880

Arg Leu Arg Thr Gln Lys Glu Pro Ala
                885

<210> 33
<211> 733
<212> DNA
<213> Homo sapiens

<400> 33
aaacagcagg aaatagaaac ttaagagaaa tacacacttc tgagaaactg aaacgacagg      60

ggaaaggagg tctcactgag caccgtccca gcatccggac accacagcgg cccttcgctc     120

cacgcagaaa accacacttc tcaaaccttc actcaacact tccttcccca aagccagaag     180

atgcacaagg aggaacatga ggtggctgtg ctgggggcac cccccagcac catccttcca     240

aggtccaccg tgatcaacat ccacagcgag acctccgtgc ccgaccatgt cgtctggtcc     300

ctgttcaaca ccctcttctt gaactggtgc tgtctgggct tcatagcatt cgcctactcc     360

gtgaagtcta gggacaggaa gatggttggc gacgtgaccg gggcccaggc ctatgcctcc     420

accgccaagt gcctgaacat ctgggccctg attctgggca tcctcatgac cattggattc     480

atcctgttac tggtattcgg ctctgtgaca gtctaccata ttatgttaca gataatacag     540

gaaaaacggg gttactagta gccgcccata gcctgcaacc tttgcactcc actgtgcaat     600

gctggccctg cacgctgggg ctgttgcccc tgcccccttg gtcctgcccc tagatacagc     660

agtttatacc cacacacctg tctacagtgt cattcaataa agtgcacgtg cttgtgaaaa     720

aaaaaaaaaa aaa                                                        733

<210> 34
<211> 125
<212> PRT
<213> Homo sapiens

<400> 34
Met His Lys Glu Glu His Glu Val Ala Val Leu Gly Ala Pro Pro Ser
1               5                   10                  15

Thr Ile Leu Pro Arg Ser Thr Val Ile Asn Ile His Ser Glu Thr Ser
            20                  25                  30

Val Pro Asp His Val Val Trp Ser Leu Phe Asn Thr Leu Phe Leu Asn
        35                  40                  45

Trp Cys Cys Leu Gly Phe Ile Ala Phe Ala Tyr Ser Val Lys Ser Arg
    50                  55                  60

Asp Arg Lys Met Val Gly Asp Val Thr Gly Ala Gln Ala Tyr Ala Ser
65                  70                  75                  80

Thr Ala Lys Cys Leu Asn Ile Trp Ala Leu Ile Leu Gly Ile Leu Met
                85                  90                  95

Thr Ile Gly Phe Ile Leu Leu Leu Val Phe Gly Ser Val Thr Val Tyr
            100                 105                 110

His Ile Met Leu Gln Ile Ile Gln Glu Lys Arg Gly Tyr
        115                 120                 125

<210> 35
<211> 894
<212> DNA
<213> Homo sapiens

<400> 35
gcccgtcttc gtgtctcctc cctccctcgc cttcctcctt cctagctcct ctcctccagg      60

gccagactga gcccaggttg atttcaggcg gacaccaata gactccacag cagctccagg     120

agcccagaca ccggcggcca gaagcaaggc taggagctgc tgcagccatg tcggccctca     180

gcctcctcat tctgggcctg ctcacggcag tgccacctgc cagctgtcag caaggcctgg     240

ggaaccttca gccctggatg cagggcctta tcgcggtggc cgtgttcctg gtcctcgttg     300

caatcgcctt tgcagtcaac cacttctggt gccaggagga gccggagcct gcacacatga     360

tcctgaccgt cggaaacaag gcagatggag tcctggtggg aacagatgga aggtactctt     420

cgatggcggc cagtttcagg tccagtgagc atgagaatgc ctatgagaat gtgcccgagg     480

aggaaggcaa ggtccgcagc accccgatgt aaccttctct gtggctccaa ccccaagact     540

cccaggcaca tgggatggat gtccagtgct accacccaag ccccctcctt ctttgtgtgg     600

aatctgcaat agtgggctga ctccctccag ccccatgccg gccctacccg cccttgaagt     660

atagccagcc aaggttggag ctcagaccgt gtctaggttg gggctcggct gtggccctgg     720

ggtctcctgc tcagctcaga agagccttct ggagaggaca gtcagctgag cacctcccat     780

cctgctcaca cgtccttccc cataactatg gaaatggccc taatttctgt gaaataaaga     840

ctttttgtat ttctggggct gaggctcagc aacagcccct caggcttcca gtga           894

<210> 36
<211> 114
<212> PRT
<213> Homo sapiens

<400> 36
Met Ser Ala Leu Ser Leu Leu Ile Leu Gly Leu Leu Thr Ala Val Pro
1               5                   10                  15

Pro Ala Ser Cys Gln Gln Gly Leu Gly Asn Leu Gln Pro Trp Met Gln
            20                  25                  30

Gly Leu Ile Ala Val Ala Val Phe Leu Val Leu Val Ala Ile Ala Phe
        35                  40                  45

Ala Val Asn His Phe Trp Cys Gln Glu Glu Pro Glu Pro Ala His Met
    50                  55                  60

Ile Leu Thr Val Gly Asn Lys Ala Asp Gly Val Leu Val Gly Thr Asp
65                  70                  75                  80

Gly Arg Tyr Ser Ser Met Ala Ala Ser Phe Arg Ser Ser Glu His Glu
                85                  90                  95

Asn Ala Tyr Glu Asn Val Pro Glu Glu Glu Gly Lys Val Arg Ser Thr
            100                 105                 110

Pro Met

<210> 37
<211> 5395
<212> DNA
<213> Homo sapiens

<400> 37
aattcactaa tgcattctgc tctttttgag agcacagctt ctcagatgtg ctccttggag      60

ctggtgtgca gtgtcctgac tgtaagatca agtccaaacc tgttttggaa ttgaggaaac     120

ttctcttttg atctcagccc ttggtggtcc aggtcttcat gctgctgtgg gtgatattac     180

tggtcctggc tcctgtcagt ggacagtttg caaggacacc caggcccatt attttcctcc     240

agcctccatg gaccacagtc ttccaaggag agagagtgac cctcacttgc aagggatttc     300

gcttctactc accacagaaa acaaaatggt accatcggta ccttgggaaa gaaatactaa     360

gagaaacccc agacaatatc cttgaggttc aggaatctgg agagtacaga tgccaggccc 420
agggctcccc tctcagtagc cctgtgcact tggatttttc ttcagcttcg ctgatcctgc 480
aagctccact ttctgtgttt gaaggagact ctgtggttct gaggtgccgg gcaaaggcgg 540
aagtaacact gaataatact atttacaaga atgataatgt cctggcattc cttaataaaa 600
gaactgactt ccatattcct catgcatgtc tcaaggacaa tggtgcatat cgctgtactg 660
gatataagga aagttgttgc cctgtttctt ccaatacagt caaaatccaa gtccaagagc 720
catttacacg tccagtgctg agagccagct ccttccagcc catcagcggg aacccagtga 780
ccctgacctg tgagacccag ctctctctag agaggtcaga tgtcccgctc cggttccgct 840
tcttcagaga tgaccagacc ctgggattag gctggagtct ctccccgaat ttccagatta 900
ctgccatgtg gagtaaagat tcagggttct actggtgtaa ggcagcaaca atgccttaca 960
gcgtcatatc tgacagcccg agatcctgga tacaggtgca gatccctgca tctcatcctg 1020
tcctcactct cagccctgaa aaggctctga attttgaggg aaccaaggtg acacttcact 1080
gtgaaaccca ggaagattct ctgcgcactt tgtacaggtt ttatcatgag ggtgtccccc 1140
tgaggcacaa gtcagtccgc tgtgaaaggg gagcatccat cagcttctca ctgactacag 1200
agaattcagg gaactactac tgcacagctg acaatggcct tggcgccaag cccagtaagg 1260
ctgtgagcct ctcagtcact gttcccgtgt ctcatcctgt cctcaacctc agctctcctg 1320
aggacctgat tttgagggga gccaaggtga cacttcactg tgaagcccag agaggttcac 1380
tccccatcct gtaccagttt catcatgagg gtgctgccct ggagcgtagg tcggccaact 1440
ctgcaggagg agtggccatc agcttctctc tgactgcaga gcattcaggg aactactact 1500
gcacagctga caatggcttt ggcccccagc gcagtaaggc ggtgagcctc tccgtcactg 1560
tccctgtgtc tcatcctgtc ctcaccctca gctctgctga ggccctgact tttgaaggag 1620
ccactgtgac acttcactgt gaagtccaga gaggttcccc acaaatccta taccagtttt 1680
atcatgagga catgcccctg tggagcagct caacaccctc tgtgggaaga gtgtccttca 1740
gcttctctct gactgaagga cattcaggga attactactg cacagctgac aatggctttg 1800
gtccccagcg cagtgaagtg gtgagccttt ttgtcactgt tccagtgtct cgccccatcc 1860
tcaccctcag ggttcccagg gcccaggctg tggtggggga cctgctggag cttcactgtg 1920
aggccccgag aggctctccc ccaatcctgt actggtttta tcatgaggat gtcaccctgg 1980
ggagcagctc agccccctct ggaggagaag cttctttcaa cctctctctg actgcagaac 2040
attctggaaa ctactcatgt gaggccaaca atggcctagt ggcccagcac agtgacacaa 2100
tatcactcag tgttatagtt ccagtatctc gtcccatcct caccttcagg gctcccaggg 2160
cccaggctgt ggtgggggac ctgctggagc ttcactgtga ggccctgaga ggctcctccc 2220

caatcctgta ctggttttat catgaagatg tcaccctggg taagatctca gccccctctg 2280

gaggaggggc ctccttcaac ctctctctga ctacagaaca ttctggaatc tactcctgtg 2340

aggcagacaa tggtctggag gcccagcgca gtgagatggt gacactgaaa gttgcagttc 2400

cggtgtctcg cccggtcctc accctcaggg ctcccgggac ccatgctgcg gtgggggacc 2460

tgctggagct tcactgtgag gccctgagag gctctcccct gatcctgtac cggttttttc 2520

atgaggatgt caccctagga aataggtcgt ccccctctgg aggagcgtcc ttaaacctct 2580

ctctgactgc agagcactct ggaaactact cctgtgaggc cgacaatggc ctcggggccc 2640

agcgcagtga gacagtgaca ctttatatca cagggctgac cgcgaacaga agtggccctt 2700

ttgccacagg agtcgccggg ggcctgctca gcatagcagg ccttgctgcg ggggcactgc 2760

tgctctactg ctggctctcg agaaaagcag ggagaaagcc tgcctctgac cccgccagga 2820

gcccttcaga ctcggactcc caagagccca cctatcacaa tgtaccagcc tgggaagagc 2880

tgcaaccagt gtacactaat gcaaatccta gaggagaaaa tgtggtttac tcagaagtac 2940

ggatcatcca agagaaaaag aaacatgcag tggcctctga ccccaggcat ctcaggaaca 3000

agggttcccc tatcatctac tctgaagtta aggtggcgtc aaccccggtt tccggatccc 3060

tgttcttggc ttcctcagct cctcacagat gagtccacac gtctctccaa ctgctgtttc 3120

agcctctgca ccccaaagtt ccccttgggg gagaagcagc attgaagtgg gaagatttag 3180

gctgccccag accatatcta ctggcctttg tttcacatgt cctcattctc agtctgacca 3240

gaatgcaggg ccctgctgga ctgtcacctg tttcccagtt aaagccctga ctggcaggtt 3300

ttttaatcca gtggcaaggt gctcccactc cagggcccag cacatctcct ggattcctta 3360

gtgggcttca gctgtggttg ctgttctgag tactgctctc atcacacccc cacagagggg 3420

gtcttaccac acaaagggag agtgggcctt caggagatgc cgggctggcc taacagctca 3480

ggtgctccta aactccgaca cagagttcct gctttgggtg gatgcatttc tcaattgtca 3540

tcagcctggt ggggctactg cagtgtgctg ccaaatggga cagcacacag cctgtgcaca 3600

tgggacatgt gatgggtctc cccacggggg ctgcatttca cactcctcca cctgtctcaa 3660

actctaaggt cggcacttga caccaaggta acttctctcc tgctcatgtg tcagtgtcta 3720

cctgcccaag taagtggctt tcatacacca agtcccaagt tcttcccatc ctaacagaag 3780

taacccagca agtcaaggcc aggaggacca ggggtgcaga cagaacacat actggaacac 3840

aggaggtgct caattactat ttgactgact gactgaatga atgaatgaat gaggaagaaa 3900

actgtgggta atcaaactgg cataaaatcc agtgcactcc ctaggaaatc cgggaggtat 3960

tctggcttcc ctaagaaaca atggaagaga aggagcttgg atgaggaaac tgttcagcaa 4020

gaggaagggc ttctcacact ttcatgtgct tgtggatcac ctgaggatcc tgtgaaaata 4080

cagatactga ttcagtgggt ctgcgtagag cctgagactg ccattctaac atgttcccag 4140

gggatgctga tgctgctggc cctgggactg cactgcatgc atgtgaagcc ctataggtct 4200

cagcagaggc ccatggagag ggaatgtgtg gctctggctg cccagggccc aactcggttc 4260

acacggatcg tgctgctccc tggccagcct ttggccacag caccaccagc tgctgttgct 4320

gagagagctt cttctctgtg acatgttggc tttcatcagc caccctggga agcggaaagt 4380

agctgccact atctttgttt ccccacctca ggcctcacac tttcccatga aaagggtgaa 4440

tgtatataac ctgagccctc tccattcaga gttgttctcc catctctgag caatgggatg 4500

ttctgttccg cttttatgat atccatcaca tcttatcttg atctttgctc ccagtggatt 4560

gtacagtgat gacttttaag ccccacggcc ctgaaataaa atccttccaa gggcattgga 4620

agctcactcc acctgaacca tggcttttca tgcttccaag tgtcagggcc ttgcccagat 4680

agacagggct ggctctgctg ccccaacctt tcaaggagga aaccagacac ctgagacagg 4740

agcctgtatg cagcccagtg cagccttgca gaggacaagg ctggaggcat ttgtcatcac 4800

tacagatatg caactaaaat agacgtggag caagagaaat gcattcccac cgaggccgct 4860

tttttaggcc tagttgaaag tcaagaagga cagcagcaag cataggctca ggattaaaga 4920

aaaaaatctg ctcacagtct gttctggagg tcacatcacc aacaaagctc acgccctatg 4980

cagttctgag aaggtggagg caccaggctc aaaagaggaa atttagaatt tctcattggg 5040

agagtaaggt acccccatcc cagaatgata actgcacagt ggcagaacaa actccaccct 5100

aatgtgggtg gaccccgtcc agtctgttga aggcctgaat gtaacaaaag ggcttattct 5160

tcctcaagta agggggaact cctgctttgg gctgggacat aagtttttct gctttcagac 5220

gcaaactgaa aaatggctct tcttgggtct tgagcttgct ggcatatgga ctgaaagaaa 5280

ctatgctatt ggatctcctg gatctccagc ttgctgactg cagatcttga gatatgtcag 5340

cctctacagt cacaagagct aattcattct aataaaccaa tctttctgta aaaaa 5395

<210> 38
<211> 977
<212> PRT
<213> Homo sapiens

<400> 38
Met Leu Leu Trp Val Ile Leu Leu Val Leu Ala Pro Val Ser Gly Gln
1               5                   10                  15

Phe Ala Arg Thr Pro Arg Pro Ile Ile Phe Leu Gln Pro Pro Trp Thr
            20                  25                  30

Thr Val Phe Gln Gly Glu Arg Val Thr Leu Thr Cys Lys Gly Phe Arg
        35                  40                  45

Phe Tyr Ser Pro Gln Lys Thr Lys Trp Tyr His Arg Tyr Leu Gly Lys
    50                  55                  60

Glu Ile Leu Arg Glu Thr Pro Asp Asn Ile Leu Glu Val Gln Glu Ser
65                  70                  75                  80

Gly Glu Tyr Arg Cys Gln Ala Gln Gly Ser Pro Leu Ser Ser Pro Val
                85                  90                  95

His Leu Asp Phe Ser Ser Ala Ser Leu Ile Leu Gln Ala Pro Leu Ser
            100                 105                 110

Val Phe Glu Gly Asp Ser Val Val Leu Arg Cys Arg Ala Lys Ala Glu
        115                 120                 125

Val Thr Leu Asn Asn Thr Ile Tyr Lys Asn Asp Asn Val Leu Ala Phe
    130                 135                 140

Leu Asn Lys Arg Thr Asp Phe His Ile Pro His Ala Cys Leu Lys Asp
145                 150                 155                 160

Asn Gly Ala Tyr Arg Cys Thr Gly Tyr Lys Glu Ser Cys Cys Pro Val
                165                 170                 175

Ser Ser Asn Thr Val Lys Ile Gln Val Gln Glu Pro Phe Thr Arg Pro
            180                 185                 190

Val Leu Arg Ala Ser Ser Phe Gln Pro Ile Ser Gly Asn Pro Val Thr
        195                 200                 205

Leu Thr Cys Glu Thr Gln Leu Ser Leu Glu Arg Ser Asp Val Pro Leu
    210                 215                 220

Arg Phe Arg Phe Phe Arg Asp Asp Gln Thr Leu Gly Leu Gly Trp Ser
225                 230                 235                 240

Leu Ser Pro Asn Phe Gln Ile Thr Ala Met Trp Ser Lys Asp Ser Gly
                245                 250                 255

Phe Tyr Trp Cys Lys Ala Ala Thr Met Pro Tyr Ser Val Ile Ser Asp
            260                 265                 270

Ser Pro Arg Ser Trp Ile Gln Val Gln Ile Pro Ala Ser His Pro Val

275                 280                 285

Leu Thr Leu Ser Pro Glu Lys Ala Leu Asn Phe Glu Gly Thr Lys Val
    290                 295                 300

Thr Leu His Cys Glu Thr Gln Glu Asp Ser Leu Arg Thr Leu Tyr Arg
305                 310                 315                 320

Phe Tyr His Glu Gly Val Pro Leu Arg His Lys Ser Val Arg Cys Glu
                325                 330                 335

Arg Gly Ala Ser Ile Ser Phe Ser Leu Thr Thr Glu Asn Ser Gly Asn
            340                 345                 350

Tyr Tyr Cys Thr Ala Asp Asn Gly Leu Gly Ala Lys Pro Ser Lys Ala
        355                 360                 365

Val Ser Leu Ser Val Thr Val Pro Val Ser His Pro Val Leu Asn Leu
    370                 375                 380

Ser Ser Pro Glu Asp Leu Ile Phe Glu Gly Ala Lys Val Thr Leu His
385                 390                 395                 400

Cys Glu Ala Gln Arg Gly Ser Leu Pro Ile Leu Tyr Gln Phe His His
                405                 410                 415

Glu Gly Ala Ala Leu Glu Arg Arg Ser Ala Asn Ser Ala Gly Gly Val
            420                 425                 430

Ala Ile Ser Phe Ser Leu Thr Ala Glu His Ser Gly Asn Tyr Tyr Cys
        435                 440                 445

Thr Ala Asp Asn Gly Phe Gly Pro Gln Arg Ser Lys Ala Val Ser Leu
    450                 455                 460

Ser Val Thr Val Pro Val Ser His Pro Val Leu Thr Leu Ser Ser Ala
465                 470                 475                 480

Glu Ala Leu Thr Phe Glu Gly Ala Thr Val Thr Leu His Cys Glu Val
                485                 490                 495

Gln Arg Gly Ser Pro Gln Ile Leu Tyr Gln Phe Tyr His Glu Asp Met
            500                 505                 510

Pro Leu Trp Ser Ser Ser Thr Pro Ser Val Gly Arg Val Ser Phe Ser
        515                 520                 525

Phe Ser Leu Thr Glu Gly His Ser Gly Asn Tyr Tyr Cys Thr Ala Asp
    530                 535                 540

Asn Gly Phe Gly Pro Gln Arg Ser Glu Val Val Ser Leu Phe Val Thr
545                 550                 555                 560

Val Pro Val Ser Arg Pro Ile Leu Thr Leu Arg Val Pro Arg Ala Gln
                565                 570                 575

Ala Val Val Gly Asp Leu Leu Glu Leu His Cys Glu Ala Pro Arg Gly
            580                 585                 590

Ser Pro Pro Ile Leu Tyr Trp Phe Tyr His Glu Asp Val Thr Leu Gly
        595                 600                 605

Ser Ser Ser Ala Pro Ser Gly Gly Glu Ala Ser Phe Asn Leu Ser Leu
    610                 615                 620

Thr Ala Glu His Ser Gly Asn Tyr Ser Cys Glu Ala Asn Asn Gly Leu
625                 630                 635                 640

Val Ala Gln His Ser Asp Thr Ile Ser Leu Ser Val Ile Val Pro Val
                645                 650                 655

Ser Arg Pro Ile Leu Thr Phe Arg Ala Pro Arg Ala Gln Ala Val Val
            660                 665                 670

Gly Asp Leu Leu Glu Leu His Cys Glu Ala Leu Arg Gly Ser Ser Pro
        675                 680                 685

Ile Leu Tyr Trp Phe Tyr His Glu Asp Val Thr Leu Gly Lys Ile Ser
    690                 695                 700

Ala Pro Ser Gly Gly Gly Ala Ser Phe Asn Leu Ser Leu Thr Thr Glu
705                 710                 715                 720

His Ser Gly Ile Tyr Ser Cys Glu Ala Asp Asn Gly Leu Glu Ala Gln
                725                 730                 735

Arg Ser Glu Met Val Thr Leu Lys Val Ala Val Pro Val Ser Arg Pro
            740                 745                 750

Val Leu Thr Leu Arg Ala Pro Gly Thr His Ala Ala Val Gly Asp Leu
        755                 760                 765

Leu Glu Leu His Cys Glu Ala Leu Arg Gly Ser Pro Leu Ile Leu Tyr
    770                 775                 780

Arg Phe Phe His Glu Asp Val Thr Leu Gly Asn Arg Ser Ser Pro Ser
785                 790                 795                 800

Gly Gly Ala Ser Leu Asn Leu Ser Leu Thr Ala Glu His Ser Gly Asn
                805                 810                 815

Tyr Ser Cys Glu Ala Asp Asn Gly Leu Gly Ala Gln Arg Ser Glu Thr
            820                 825                 830

Val Thr Leu Tyr Ile Thr Gly Leu Thr Ala Asn Arg Ser Gly Pro Phe
        835                 840                 845

Ala Thr Gly Val Ala Gly Gly Leu Leu Ser Ile Ala Gly Leu Ala Ala
    850                 855                 860

Gly Ala Leu Leu Leu Tyr Cys Trp Leu Ser Arg Lys Ala Gly Arg Lys
865                 870                 875                 880

Pro Ala Ser Asp Pro Ala Arg Ser Pro Ser Asp Ser Asp Ser Gln Glu
                885                 890                 895

Pro Thr Tyr His Asn Val Pro Ala Trp Glu Glu Leu Gln Pro Val Tyr
            900                 905                 910

Thr Asn Ala Asn Pro Arg Gly Glu Asn Val Val Tyr Ser Glu Val Arg
        915                 920                 925

Ile Ile Gln Glu Lys Lys Lys His Ala Val Ala Ser Asp Pro Arg His
    930                 935                 940

Leu Arg Asn Lys Gly Ser Pro Ile Ile Tyr Ser Glu Val Lys Val Ala
945                 950                 955                 960

Ser Thr Pro Val Ser Gly Ser Leu Phe Leu Ala Ser Ser Ala Pro His
                965                 970                 975

Arg

<210> 39
<211> 3457
<212> DNA
<213> Homo sapiens

<400> 39
gggacgcccg ggcggccctg aaggggacgg ggcggcccca gtcggaggtc gcagggagct 60
ccgcccccga ctcggtataa gagctgggcc cggcccacgg cggcggcggc ggcggcggag 120
agagctggct cagggcgtcc gctaggctcg gacgacctgc tgagcctccc aaaccgcttc 180
cataaggctt tgcctttcca acttcagcta cagtgttagc taagtttgga aagaaggaaa 240
aaagaaaatc cctgggcccc ttttcttttg ttctttgcca aagtcgtcgt tgtagtcttt 300
ttgcccaagg ctgttgtgtt tttagaggtc ctatctccag ttccttgcac tcctgttaac 360
aagcacctca gcgagagcag cagcagcgat agcagccgca gaagagccag cggggtcgcg 420
tagtgtcatg accagggcgg gagatcacaa ccgccagaga ggatgctgtg gatccttggc 480
cgactacctg acctctgcaa aattccttct ctaccttggt cattctctct ctacttgggg 540
agatcggatg tggcactttg cggtgtctgt gtttctggta gagctctatg gaaacagcct 600
ccttttgaca gcagtctacg ggctggtggt ggcagggtct gttctggtcc tgggagccat 660
catcggtgac tgggtggaca agaatgctag acttaaagtg gcccagacct cgctggtggt 720
acagaatgtt tcagtcatcc tgtgtggaat catcctgatg atggttttct tacataaaca 780
tgagcttctg accatgtacc atggatgggt tctcacttcc tgctatatcc tgatcatcac 840
tattgcaaat attgcaaatt tggccagtac tgctactgca atcacaatcc aaagggattg 900
gattgttgtt gttgcaggag aagacagaag caaactagca aatatgaatg ccacaatacg 960
aaggattgac cagttaacca acatcttagc ccccatggct gttggccaga ttatgacatt 1020
tggctcccca gtcatcggct gtggctttat ttcgggatgg aacttggtat ccatgtgcgt 1080
ggagtacgtt ctgctctgga aggtttacca gaaaacccca gctctagctg tgaaagctgg 1140
tcttaaagaa gaggaaactg aattgaaaca gctgaattta cacaaagata ctgagccaaa 1200
acccctggag ggaactcatc taatgggtgt gaaagactct aacatccatg agcttgaaca 1260
tgagcaagag cctacttgtg cctcccagat ggctgagccc ttccgtacct tccgagatgg 1320
atgggtctcc tactacaacc agcctgtgtt tctggctggc atgggtcttg ctttccttta 1380
tatgactgtc ctgggctttg actgcatcac cacagggtac gcctacactc agggactgag 1440
tggttccatc ctcagtattt tgatgggagc atcagctata actggaataa tgggaactgt 1500
agcttttact tggctacgtc gaaaatgtgg tttggttcgg acaggtctga tctcaggatt 1560
ggcacagctt tcctgtttga tcttgtgtgt gatctctgta ttcatgcctg gaagccccct 1620
ggacttgtcc gtttctcctt ttgaagatat ccgatcaagg ttcattcaag gagagtcaat 1680
tacacctacc aagatacctg aaattacaac tgaaatatac atgtctaatg ggtctaattc 1740
tgctaatatt gtcccggaga caagtcctga atctgtgccc ataatctctg tcagtctgct 1800

gtttgcaggc gtcattgctg ctagaatcgg tctttggtcc tttgatttaa ctgtgacaca 1860

gttgctgcaa gaaaatgtaa ttgaatctga aagaggcatt ataaatggtg tacagaactc 1920

catgaactat cttcttgatc ttctgcattt catcatggtc atcctggctc caaatcctga 1980

agcttttggc ttgctcgtat tgatttcagt ctcctttgtg gcaatgggcc acattatgta 2040

tttccgattt gcccaaaata ctctgggaaa caagctcttt gcttgcggtc ctgatgcaaa 2100

agaagttagg aaggaaaatc aagcaaatac atctgttgtt tgagacagtt taactgttgc 2160

tatcctgtta ctagattata tagagcacat gtgcttattt tgtactgcag aattccaata 2220

aatggctggg tgttttgctc tgtttttacc acagctgtgc cttgagaact aaaagctgtt 2280

taggaaacct aagtcagcag aaattaactg attaatttcc cttatgttga ggcatggaaa 2340

aaaaattgga aaagaaaaac tcagtttaaa tacggagact ataatgataa cactgaattc 2400

ccctatttct catgagtaga tacaatctta cgtaaaagag tggttagtca cgtgaattca 2460

gttatcattt gacagattct tatctgtact agaattcaga tatgtcagtt ttctgcaaaa 2520

ctcactcttg ttcaagacta gctaatttat ttttttgcat cttagttatt tttaaaaaca 2580

aattcttcaa gtatgaagac taaattttga taactaatat tatccttatt gatcctattg 2640

atcttaaggt atttacatgt atgtggaaaa acaaaacact taactagaat tctctaataa 2700

ggtttatggt ttagcttaaa gagcaccttt gtatttttat tatcagatgg ggcaacatat 2760

tgtatgaagc atatgtagca cttcacagca tggttatcat gtaagctgca ggtagaagca 2820

cacactcagg tagaatattt ttatttttac tgttttatac ccagaagtta tttctacatt 2880

aagctgtaaa gtagatttat cacacaatga ctgcatacag acttcaaata tgtcaatagt 2940

ttggtcatag aacctagaag ccaaaagcca cacagaaggg caagaatccc aatttaactc 3000

atgttatcat cattagtgat ctgtgttgta gaacatgagg gtgtaagcct tcagcctggc 3060

aagttacatg tagaaagccc acacttgtga aggttttgtt ttacaaatca cttgatttaa 3120

gttctacagc aagaatattc ataaaagtat ccctttcaaa tgcctttgag aagaatagaa 3180

gaaaaaaagt ttgtatatat tttaaaaaat tgttttaaaa gtcagtttgc aacatgtctg 3240

taccaagatg gtactttgcc ttaaccgttt atatgcactt tcatggagac tgcaatacgt 3300

tgctatgagc actttcttta tccttggagt ttaatccttt gcttcatctt tctacagtat 3360

gacataatga tttgctatgt tgtaaaatct ttgtaaaaaa tttctatata aaaatatttt 3420

gaaaatctta aaaaaaaaaa aaaaaaaaaa aaaaaaa 3457

<210> 40
<211> 571
<212> PRT

<213> Homo sapiens

<400> 40

<210> 41
<211> 2213
<212> DNA
<213> Homo sapiens

<400> 41
gctctagaac tagtggatcc cccgggctgc aggaattctc taaagaagcc cctgggagca      60

cagctcatca ccatggactg gacctggagg ttcctctttg tggtggcagc agctacaggt     120

gtccagtccc aggtgcagct ggtgcagtct ggggctgagg tgaagaagcc tgggtcctcg     180

gtgaaggtct cctgcaaggc ttctggaggc accttcagca gctatgctat cagctgggtg     240

cgacaggccc ctggacaagg gcttgagtgg atgggaggga tcatccctat ctttggtaca     300

gcaaactacg cacagaagtt ccagggcaga gtcacgatta ccgcggacga atccacgagc     360

acagcctaca tggagctgag cagcctgaga tctgaggaca cggccgtgta ttactgtgcg     420

aaaaccggga tcctggggcc gtatagcagt ggctggtacc cgaactcgga ctactactac     480

tacggtatgg acgtctgggg ccaagggacc acggtcaccg tctcctcagg gagtgcatcc     540

gccccaaccc ttttccccct cgtctcctgt gagaattccc cgtcggatac gagcagcgtg     600

gccgttggct gcctcgcaca ggacttcctt cccgactcca tcactttctc ctggaaatac     660

aagaacaact ctgacatcag cagcacccgg ggcttcccat cagtcctgag agggggcaag     720

tacgcagcca cctcacaggt gctgctgcct tccaaggacg tcatgcaggg cacagacgaa     780

cacgtggtgt gcaaagtcca gcaccccaac ggcaacaaag aaaagaacgt gcctcttcca     840

gtgattgctg agctgcctcc caaagtgagc gtcttcgtcc caccccgcga cggcttcttc     900

ggcaaccccc gcagcaagtc caagctcatc tgccaggcca cgggtttcag tccccggcag 960

attcaggtgt cctggctgcg cgaggggaag caggtggggt ctggcgtcac cacggaccag 1020

gtgcaggctg aggccaaaga gtctgggccc acgacctaca aggtgaccag cacactgacc 1080

atcaaagaga gcgactggct cagccagagc atgttcacct gccgcgtgga tcacaggggc 1140

ctgaccttcc agcagaatgc gtcctccatg tgtgtccccg atcaagacac agccatccgg 1200

gtcttcgcca tccccccatc ctttgccagc atcttcctca ccaagtccac caagttgacc 1260

tgcctggtca cagacctgac cacctatgac agcgtgacca tctcctggac ccgccagaat 1320

ggcgaagctg tgaaaaccca caccaacatc tccgagagcc accccaatgc cactttcagc 1380

gccgtgggtg aggccagcat ctgcgaggat gactggaatt ccggggagag gttcacgtgc 1440

accgtgaccc acacagacct gccctcgcca ctgaagcaga ccatctcccg gcccaagggg 1500

gtggccctgc acaggcccga tgtctacttg ctgccaccag cccgggagca gctgaacctg 1560

cgggagtcgg ccaccatcac gtgcctggtg acgggcttct ctcccgcgga cgtcttcgtg 1620

cagtggatgc agagggggca gcccttgtcc ccggagaagt atgtgaccag cgccccaatg 1680

cctgagcccc aggccccagg ccggtacttc gcccacagca tcctgaccgt gtccgaagag 1740

gaatggaaca cgggggagac ctacacctgc gtggtggccc atgaggccct gcccaacagg 1800

gtcaccgaga ggaccgtgga caagtccacc gagggggagg tgagcgccga cgaggagggc 1860

tttgagaacc tgtgggccac cgcctccacc ttcatcgtcc tcttcctcct gagcctcttc 1920

tacagtacca ccgtcacctt gttcaaggtg aaatgatccc aacagaagaa catcggagac 1980

cagagagagg aactcaaagg ggcgctgcct ccgggtctgg ggtcctggcc tgcgtggcct 2040

gttggcacgt gtttctcttc ccgcccggcc tccagttgtg tgctctcaca caggcttcct 2100

tctcgaccgg caggggctgg ctggcttgca ggccacgagg tgggctctac cccacactgc 2160

tttgctgtgt atacgcttgt tgccctgaaa taaatatgca cattttatcc atg 2213

<210> 42
<211> 627
<212> PRT
<213> Homo sapiens

<400> 42

<210> 43
<211> 947
<212> DNA
<213> Homo sapiens

<400> 43
ccatccctga gatcttttta taaaaaaccc agtctttgct gaccagacaa agcataccag      60

atctcaccag agagtcctag gggactacag aaggaaaaag acaagaggca gtaggatatc     120

tgtgtgtcct cccgctgacc acacttcctt tagtgacccg attgcctcct caagtcgcag     180

acactatgct gcctcccatg gccctgccca gtgtgtcctg gatgctgctt tcctgcctca     240

ttctcctgtg tcaggttcaa ggtgaagaaa cccagaagga actgccctct ccacggatca     300

gctgtcccaa aggctccaag gcctatggct ccccctgcta tgccttgttt ttgtcaccaa     360

aatcctggat ggatgcagat ctggcttgcc agaagcggcc ctctggaaaa ctggtgtctg     420

tgctcagtgg ggctgaggga tccttcgtgt cctccctggt gaggagcatt agtaacagct     480

attcatacat ctggattggg ctccatgacc ccacacaggg ctctgagcct gatggagatg     540

gatgggagtg gagtagcact gatgtgatga attactttgc atgggagaaa aatccctcca     600

ccatcttaaa ccctggccac tgtgggagcc tgtcaagaag cacaggattt ctgaagtgga     660

aagattataa ctgtgatgca aagttaccct atgtctgcaa gttcaaggac tagggcaggt     720

gggaagtcag cagcctgagc ttggcgtgca gctcatcatg gacatgagac cagtgtgaag     780

actcaccctg gaagagaata ttctccccaa actgccctac ctgactacct tgtcatgatc     840

ctccttcttt ttcctttttc ttcaccttca tttcaggctt ttctctgtct tccatgtctt     900

gagatctcag agaataataa taaaaatgtt actttatacg taaaaaa                  947

<210> 44
<211> 175
<212> PRT
<213> Homo sapiens

<400> 44
Met Leu Pro Pro Met Ala Leu Pro Ser Val Ser Trp Met Leu Leu Ser
1               5                   10                  15

Cys Leu Ile Leu Leu Cys Gln Val Gln Gly Glu Glu Thr Gln Lys Glu
            20                  25                  30

Leu Pro Ser Pro Arg Ile Ser Cys Pro Lys Gly Ser Lys Ala Tyr Gly
        35                  40                  45

Ser Pro Cys Tyr Ala Leu Phe Leu Ser Pro Lys Ser Trp Met Asp Ala
    50                  55                  60

Asp Leu Ala Cys Gln Lys Arg Pro Ser Gly Lys Leu Val Ser Val Leu
65                  70                  75                  80

Ser Gly Ala Glu Gly Ser Phe Val Ser Ser Leu Val Arg Ser Ile Ser
                85                  90                  95

Asn Ser Tyr Ser Tyr Ile Trp Ile Gly Leu His Asp Pro Thr Gln Gly
            100                 105                 110

Ser Glu Pro Asp Gly Asp Gly Trp Glu Trp Ser Ser Thr Asp Val Met
        115                 120                 125

Asn Tyr Phe Ala Trp Glu Lys Asn Pro Ser Thr Ile Leu Asn Pro Gly
    130                 135                 140

His Cys Gly Ser Leu Ser Arg Ser Thr Gly Phe Leu Lys Trp Lys Asp
145                 150                 155                 160

Tyr Asn Cys Asp Ala Lys Leu Pro Tyr Val Cys Lys Phe Lys Asp
                165                 170                 175

<210> 45
<211> 3033
<212> DNA
<213> Homo sapiens

<400> 45
aacacaactg gcacatctct tttctcatct cttgaaaaaa accaacagag aaaaaagtac 60

cttgagaata aaggtaatga ttaatctgtc aggcacaaaa gggattgttt tggggatttc 120

gggttctaag tcgcagattc aaacaaatag cagcgaacag ggaatgacag ttccaccaga 180

agacgattaa gccacagcct ctaattggaa cggcatttgt acagtcagag actcttacca 240

gacatctcca ggaatctgtg agccattgtc aaaacgtcca ttttcatctg gctgtgaaag 300

tgaggaccac aacaggtagg tattggtaga aacaggagtc ctcagagaag ccccaagatg 360

cagcctgagg gagcagaaaa gggaaaaagc ttcaagcaga gactggtctt gaagagcagc 420

ttagcgaaag aaaccctctc tgagttcttg ggcacgttca tcttgattgt ccttggatgt 480

ggctgtgttg cccaagctat tctcagtcga ggacgttttg gaggggtcat cactatcaat 540

gttggatttt caatggcagt tgcaatggcc atttatgtgg ctggcggtgt ctctggtggt 600

cacatcaacc cagctgtgtc tttagcaatg tgtctctttg gacggatgaa atggttcaaa 660

ttgccatttt atgtgggagc ccagttcttg ggagcctttg tgggggctgc aaccgtcttt 720

ggcatttact atgatggact tatgtccttt gctggtggaa aactgctgat cgtgggagaa 780

aatgcaacag cacacatttt tgcaacatac ccagctccgt atctatctct ggcgaacgca 840

tttgcagatc aagtggtggc caccatgata ctcctcataa tcgtctttgc catctttgac 900

tccagaaact tgggagcccc cagaggccta gagcccattg ccatcggcct cctgattatt 960

gtcattgctt cctccctggg actgaacagt ggctgtgcca tgaacccagc tcgagacctg 1020

agtcccagac ttttcactgc cttggcaggc tgggggtttg aagtcttcag agctggaaac 1080

aacttctggt ggattcctgt agtgggccct ttggttggtg ctgtcattgg aggcctcatc 1140

tatgttcttg tcattgaaat ccaccatcca gagcctgact cagtctttaa gacagaacaa 1200

tctgaggaca aaccagagaa atatgaactc agtgtcatca tgtagtggca tgctcagctc 1260

tggatttgca gtcagtttgg gattctcttc agaaagatgg catctaagtg tctgtgttct 1320

tgtaagcctg aggtggaatc cacccagttt tgtctgctag ccatatggga catctaattg 1380

gaaaagcatc tgcataaaag tttggaaaca atgaccactt ctctaccatt gtcccccacc 1440

cccacccccc agaataacgc tgactgtccc ctgaaacagc cttctctcct gccctgttta 1500

tttcatcctc gatgggaatt cttgctaggt aagcactaat aactcggcat cttgacgata 1560

gtcccatttg ggtggtttca gctgcactat ctgtatgaaa tggtgtcacc aaaacccttt 1620

tcttcagtat cgacaaagat tacattctga gtaccaacca aaccctaaat tgaaagacaa 1680

aactatggtt tcagtcaaca tattcatgaa ttagggagct aatgggttaa gcttccagtt 1740

cccgctatgc tactggattt gtataaatac tgatattctc caaacctagt ggtgtaggga 1800

gcaagagaat gcagctggaa ggcacaaggg gaggacattg tggcattcag aaactgcagg 1860

agacaagatg aatttgagaa gccaaatgga atttttaatg gaaaccattt atcagattaa 1920

tctcttgctc tcctgcattt tagaggacac caattaattt cctggtcttt agtatataat 1980

aacctaaaat accattgtaa cctcagtcat gaaaaataca tcactctgtc tttttagctc 2040

aaatgtattt tcctaattgc ccacttgaga acagacattt gacaagttat atcaacgact 2100

gtgcttgtcc attattttac acatgcccta gaagccaaaa ctgaaagcca ctggatcctg 2160

gtctagctga atcttcagag tgggaggtct ccaaaaagat attaccttat tgggcttaac 2220

aattcacaag gcactttcac acccattatc taatttaatc ctcataatga ctatgtgagg 2280

caaatgccac attgcccatt tttcagataa agaaacaaaa tcttagggaa gataagttga 2340

gttgtccaag agcacactga aagttgaatg ttatctaatg cattcctcta cctttcagaa 2400

gatcagtagc tggctgagaa tctttgccaa atcttccttg ctagccagaa gtggaattgg 2460

cagcttctag aatatgtaca cctctggaca aaatgttcct caatcttaag atacaaagac 2520

cctcattgtc tgggtctatt cccacactta ctgagtacag atgaaggaaa gtggtagcaa 2580

tttaatcata actttcattt gctgaaaaac attatgagaa ggcctccctt cctaagccac 2640

ctctggtctt gctaagtctt gatcttgctt cctgccagca ccaaacatta cattcagggg 2700

atttcctctg gctcagtctt ttccccttga agttctctaa tagatgttac ttttgacaaa 2760

agatcgccta tgagttacaa gcaccagggg atgctctaca tcaagggatg caccttcagt 2820

caaactgtca aaaagcccag aattcccaaa ggcattaggt ttcccaactg ctttgtgctg 2880

atatcagaac agcagaaatt aaatgtgaaa tgtttctgat gacttatgtt ctacaatcta 2940

tggacatacg ggattttttt ttcttgcttt gaagctacct ggatatttcc tatttgaaat 3000

aaaattgttc ggtcattgtt gaaaaaaaaa aaa 3033

<210> 46
<211> 295
<212> PRT
<213> Homo sapiens

<400> 46
Met Gln Pro Glu Gly Ala Glu Lys Gly Lys Ser Phe Lys Gln Arg Leu
1               5                   10                  15

Val Leu Lys Ser Ser Leu Ala Lys Glu Thr Leu Ser Glu Phe Leu Gly
            20                  25                  30

Thr Phe Ile Leu Ile Val Leu Gly Cys Gly Cys Val Ala Gln Ala Ile
        35                  40                  45

Leu Ser Arg Gly Arg Phe Gly Gly Val Ile Thr Ile Asn Val Gly Phe
    50                  55                  60

Ser Met Ala Val Ala Met Ala Ile Tyr Val Ala Gly Gly Val Ser Gly
65                  70                  75                  80

Gly His Ile Asn Pro Ala Val Ser Leu Ala Met Cys Leu Phe Gly Arg
                85                  90                  95

Met Lys Trp Phe Lys Leu Pro Phe Tyr Val Gly Ala Gln Phe Leu Gly
            100                 105                 110

Ala Phe Val Gly Ala Ala Thr Val Phe Gly Ile Tyr Tyr Asp Gly Leu
        115                 120                 125

Met Ser Phe Ala Gly Gly Lys Leu Leu Ile Val Gly Glu Asn Ala Thr
    130                 135                 140

Ala His Ile Phe Ala Thr Tyr Pro Ala Pro Tyr Leu Ser Leu Ala Asn
145                 150                 155                 160

Ala Phe Ala Asp Gln Val Val Ala Thr Met Ile Leu Leu Ile Ile Val
                165                 170                 175

Phe Ala Ile Phe Asp Ser Arg Asn Leu Gly Ala Pro Arg Gly Leu Glu
            180                 185                 190

Pro Ile Ala Ile Gly Leu Leu Ile Ile Val Ile Ala Ser Ser Leu Gly
        195                 200                 205

Leu Asn Ser Gly Cys Ala Met Asn Pro Ala Arg Asp Leu Ser Pro Arg
    210                 215                 220

Leu Phe Thr Ala Leu Ala Gly Trp Gly Phe Glu Val Phe Arg Ala Gly
225                 230                 235                 240

Asn Asn Phe Trp Trp Ile Pro Val Val Gly Pro Leu Val Gly Ala Val
                245                 250                 255

Ile Gly Gly Leu Ile Tyr Val Leu Val Ile Glu Ile His His Pro Glu
            260                 265                 270

Pro Asp Ser Val Phe Lys Thr Glu Gln Ser Glu Asp Lys Pro Glu Lys
        275                 280                 285

Tyr Glu Leu Ser Val Ile Met

290                 295

<210> 47
<211> 2844
<212> DNA
<213> Homo sapiens

<400> 47
atgaggcccg gcctctcatt tctcctagcc cttctgttct tccttggcca agctgcaggg      60

gatttggggg atgtgggacc tccaattccc agccccggct tcagctcttt cccaggtgtt     120

gactccagct ccagcttcag ctccagctcc aggtcgggct ccagctccag ccgcagctta     180

ggcagcggag gttctgtgtc ccagttgttt tccaatttca ccggctccgt ggatgaccgt     240

gggacctgcc agtgctctgt ttccctgcca gacaccacct ttcccgtgga cagagtggaa     300

cgcttggaat tcacagctca tgttctttct cagaagtttg agaaagaact ttccaaagtg     360

agggaatatg tccaattaat tagtgtgtat gaaaagaaac tgttaaacct aactgtccga     420

attgacatca tggagaagga taccatttct tacactgaac tggacttcga gctgatcaag     480

gtagaagtga aggagatgga aaaactggtc atacagctga aggagagttt tggtggaagc     540

tcagaaattg ttgaccagct ggaggtggag ataagaaata tgactctctt ggtagagaag     600

cttgagacac tagacaaaaa caatgtcctt gccattcgcc gagaaatcgt ggctctgaag     660

accaagctga aagagtgtga ggcctctaaa gatcaaaaca cccctgtcgt ccaccctcct     720

cccactccag ggagctgtgg tcatggtggt gtggtgaaca tcagcaaacc gtctgtggtt     780

cagctcaact ggagagggtt ttcttatcta tatggtgctt ggggtaggga ttactctccc     840

cagcatccaa acaaaggact gtattgggtg gcgccattga atacagatgg gagactgttg     900

gagtattata gactgtacaa cacactggat gatttgctat tgtatataaa tgctcgagag     960

ttgcggatca cctatggcca aggtagtggt acagcagttt acaacaacaa catgtacgtc    1020

aacatgtaca acaccgggaa tattgccaga gttaacctga ccaccaacac gattgctgtg    1080

actcaaactc tccctaatgc tgcctataat aaccgctttt catatgctaa tgttgcttgg    1140

caagatattg actttgctgt ggatgagaat ggattgtggg ttatttattc aactgaagcc    1200

agcactggta acatggtgat tagtaaactc aatgacacca cacttcaggt gctaaacact    1260

tggtatacca agcagtataa accatctgct tctaacgcct tcatggtatg tggggttctg    1320

tatgccaccc gtactatgaa caccagaaca gaagagattt tttactatta tgacacaaac    1380

acagggaaag agggcaaact agacattgta atgcataaga tgcaggaaaa agtgcagagc    1440

attaactata acccttttga ccagaaactt tatgtctata acgatggtta ccttctgaat    1500

tatgatcttt ctgtcttgca gaagccccag taagctgttt aggagttagg gtgaaagaga    1560

aaatgtttgt tgaaaaaata gtcttctcca cttacttaga tatctgcagg ggtgtctaaa 1620

agtgtgttca ttttgcagca atgtttaggt gcatagttct accacactag agatctagga 1680

catttgtctt gatttggtga gttctcttgg gaatcatctg cctcttcagg cgcattttgc 1740

aataaagtct gtctagggtg ggattgtcag aggtctaggg gcactgtggg cctagtgaag 1800

cctactgtga ggaggcttca ctagaagcct taaattagga attaaggaac ttaaaactca 1860

gtatggcgtc tagggattct ttgtacagga aatattgccc aatgactagt cctcatccat 1920

gtagcaccac taattcttcc atgcctggaa gaaacctggg gacttagtta ggtagattaa 1980

tatctggagc tcctcgaggg accaaatctc caactttttt ttcccctcac tagcacctgg 2040

aatgatgctt tgtatgtggc agataagtaa atttggcatg cttatatatt ctacatctgt 2100

aaagtgctga gttttatgga gagaggcctt tttatgcatt aaattgtaca tggcaaataa 2160

atcccagaag gatctgtaga tgaggcacct gctttttctt ttctctcatt gtccacctta 2220

ctaaaagtca gtagaatctt ctacctcata acttccttcc aaaggcagct cagaagatta 2280

gaaccagact tactaaccaa ttccaccccc caccaacccc cttctactgc ctactttaaa 2340

aaaattaata gttttctatg gaactgatct aagattagaa aaattaattt tctttaattt 2400

cattatgaac ttttatttac atgactctaa gactataaga aaatctgatg gcagtgacaa 2460

agtgctagca tttattgtta tctaataaag accttggagc atatgtgcaa cttatgagtg 2520

tatcagttgt tgcatgtaat ttttgccttt gtttaagcct ggaacttgta agaaaatgaa 2580

aatttaattt ttttttctag gacgagctat agaaaagcta ttgagagtat ctagttaatc 2640

agtgcagtag ttggaaacct tgctggtgta tgtgatgtgc ttctgtgctt ttgaatgact 2700

ttatcatcta gtctttgtct atttttcctt tgatgttcaa gtcctagtct ataggattgg 2760

cagtttaaat gctttactcc cccttttaaa ataaatgatt aaaatgtgct ttgaaaaaaa 2820

aaaaaaaaaa aaaaaaaaaa aaaa 2844

<210> 48
<211> 510
<212> PRT
<213> Homo sapiens

<400> 48
Met Arg Pro Gly Leu Ser Phe Leu Leu Ala Leu Leu Phe Phe Leu Gly
1               5                   10                  15

Gln Ala Ala Gly Asp Leu Gly Asp Val Gly Pro Pro Ile Pro Ser Pro
            20                  25                  30

Gly Phe Ser Ser Phe Pro Gly Val Asp Ser Ser Ser Ser Phe Ser Ser
        35                  40                  45

Ser Ser Arg Ser Gly Ser Ser Ser Ser Arg Ser Leu Gly Ser Gly Gly
    50                  55                  60

Ser Val Ser Gln Leu Phe Ser Asn Phe Thr Gly Ser Val Asp Asp Arg
65                  70                  75                  80

Gly Thr Cys Gln Cys Ser Val Ser Leu Pro Asp Thr Thr Phe Pro Val
                85                  90                  95

Asp Arg Val Glu Arg Leu Glu Phe Thr Ala His Val Leu Ser Gln Lys
            100                 105                 110

Phe Glu Lys Glu Leu Ser Lys Val Arg Glu Tyr Val Gln Leu Ile Ser
        115                 120                 125

Val Tyr Glu Lys Lys Leu Leu Asn Leu Thr Val Arg Ile Asp Ile Met
    130                 135                 140

Glu Lys Asp Thr Ile Ser Tyr Thr Glu Leu Asp Phe Glu Leu Ile Lys
145                 150                 155                 160

Val Glu Val Lys Glu Met Glu Lys Leu Val Ile Gln Leu Lys Glu Ser
                165                 170                 175

Phe Gly Gly Ser Ser Glu Ile Val Asp Gln Leu Glu Val Glu Ile Arg
            180                 185                 190

Asn Met Thr Leu Leu Val Glu Lys Leu Glu Thr Leu Asp Lys Asn Asn
        195                 200                 205

Val Leu Ala Ile Arg Arg Glu Ile Val Ala Leu Lys Thr Lys Leu Lys
    210                 215                 220

Glu Cys Glu Ala Ser Lys Asp Gln Asn Thr Pro Val Val His Pro Pro
225                 230                 235                 240

Pro Thr Pro Gly Ser Cys Gly His Gly Gly Val Val Asn Ile Ser Lys
                245                 250                 255

Pro Ser Val Val Gln Leu Asn Trp Arg Gly Phe Ser Tyr Leu Tyr Gly
            260                 265                 270

Ala Trp Gly Arg Asp Tyr Ser Pro Gln His Pro Asn Lys Gly Leu Tyr
        275                 280                 285

Trp Val Ala Pro Leu Asn Thr Asp Gly Arg Leu Leu Glu Tyr Tyr Arg
    290                 295                 300

Leu Tyr Asn Thr Leu Asp Asp Leu Leu Leu Tyr Ile Asn Ala Arg Glu
305                 310                 315                 320

Leu Arg Ile Thr Tyr Gly Gln Gly Ser Gly Thr Ala Val Tyr Asn Asn
                325                 330                 335

Asn Met Tyr Val Asn Met Tyr Asn Thr Gly Asn Ile Ala Arg Val Asn
            340                 345                 350

Leu Thr Thr Asn Thr Ile Ala Val Thr Gln Thr Leu Pro Asn Ala Ala
        355                 360                 365

Tyr Asn Asn Arg Phe Ser Tyr Ala Asn Val Ala Trp Gln Asp Ile Asp
    370                 375                 380

Phe Ala Val Asp Glu Asn Gly Leu Trp Val Ile Tyr Ser Thr Glu Ala
385                 390                 395                 400

Ser Thr Gly Asn Met Val Ile Ser Lys Leu Asn Asp Thr Thr Leu Gln
                405                 410                 415

Val Leu Asn Thr Trp Tyr Thr Lys Gln Tyr Lys Pro Ser Ala Ser Asn
            420                 425                 430

Ala Phe Met Val Cys Gly Val Leu Tyr Ala Thr Arg Thr Met Asn Thr
        435                 440                 445

Arg Thr Glu Glu Ile Phe Tyr Tyr Tyr Asp Thr Asn Thr Gly Lys Glu
    450                 455                 460

Gly Lys Leu Asp Ile Val Met His Lys Met Gln Glu Lys Val Gln Ser
465                 470                 475                 480

Ile Asn Tyr Asn Pro Phe Asp Gln Lys Leu Tyr Val Tyr Asn Asp Gly
                485                 490                 495

Tyr Leu Leu Asn Tyr Asp Leu Ser Val Leu Gln Lys Pro Gln
            500                 505                 510

<210> 49
<211> 586
<212> DNA
<213> Homo sapiens

<400> 49
aaacactctg tgtggctcct cggctttgac agagtgcaag acgatgactt gcaaaatgtc      60

gcagctggaa cgcaacatag agaccatcat caacaccttc caccaatact ctgtgaagct     120

ggggcaccca gacaccctga accaggggga attcaaagag ctggtgcgaa aagatctgca     180

aaattttctc aagaaggaga ataagaatga aaaggtcata gaacacatca tggaggacct     240

ggacacaaat gcagacaagc agctgagctt cgaggagttc atcatgctga tggcgaggct     300

aacctgggcc tcccacgaga agatgcacga gggtgacgag ggccctggcc accaccataa     360

gccaggcctc ggggagggca ccccctaaga ccacagtggc caagatcaca gtggccacgg     420

ccacggccac agtcatggtg gccacggcca cagccactaa tcaggaggcc aggccaccct     480

gcctctaccc aaccagggcc ccggggcctg ttatgtcaaa ctgtcttggc tgtggggcta     540

ggggctgggg ccaaataaag tctcttcctc caagtcaaaa aaaaaa                    586


<210> 50
<211> 114
<212> PRT
<213> Homo sapiens

<400> 50
Met Thr Cys Lys Met Ser Gln Leu Glu Arg Asn Ile Glu Thr Ile Ile
1               5                   10                  15


Asn Thr Phe His Gln Tyr Ser Val Lys Leu Gly His Pro Asp Thr Leu
            20                  25                  30


Asn Gln Gly Glu Phe Lys Glu Leu Val Arg Lys Asp Leu Gln Asn Phe
        35                  40                  45


Leu Lys Lys Glu Asn Lys Asn Glu Lys Val Ile Glu His Ile Met Glu
    50                  55                  60


Asp Leu Asp Thr Asn Ala Asp Lys Gln Leu Ser Phe Glu Glu Phe Ile
65                  70                  75                  80


Met Leu Met Ala Arg Leu Thr Trp Ala Ser His Glu Lys Met His Glu
                85                  90                  95


Gly Asp Glu Gly Pro Gly His His His Lys Pro Gly Leu Gly Glu Gly
            100                 105                 110


Thr Pro

<210> 51
<211> 3096
<212> DNA
<213> Homo sapiens

<400> 51
gccagcagcc tctaagagct cctagacggc ctttggcttt ggctggcaca ggggtgaagg 60

agagccagcg gcattgagta accaggcgaa tgtgccccca ggagagttca ttccaaccct 120

cccagttcct actgctggtg ggggtcccag tggcaagtgt cctccttctg gcccaatgcc 180

ttcgatggca ctgccctaga aggctgctgg gggcctgctg gacactgaat ggtcaagagg 240

aaccagtgtc ccagcctacc ccccaactag aaaatgaggt ctcaaggcag cacctgccag 300

ccacactgcc agagatggtt gccttctacc aggagctaca cacacccact caaggccaga 360

ccatggtccg ccagttgatg cacaaactgt tggtgttttc ggctcgagag gtggatcacc 420

gcggcggttg cctgatgctc caggatacag gcatctcctt gctcatccca ccaggtgctg 480

tggctgtggg ccgccaggag cgggtgtctt tgatcctggt gtgggacctg tcggacgccc 540

catcgctgtc ccaagcccag gggctggtaa gccctgtggt ggcatgtggc ccccatgggg 600

cctccttcct gaagccttgc actctcacgt tcaaacactg tgccgagcag cccagccatg 660

ctcgcaccta cagcagcaac actaccctgc tggatgccaa ggtatggagg cccctggggc 720

ggccgggggc ccacgcctcc cgggatgagt gtcgcatcca cctctcccac ttcagcctct 780

acacctgtgt gctggaggca cctgtggggc gcgaagcccg caaatggctg cagctggccg 840

tattctgctc accgctggtg ccaggacagt cccatctgca actgcgtatc tacttcctca 900

acaacacgcc ctgcgccctg cagtgggcac tgaccaacga gcagccccat ggtgggcgcc 960

tgcgtgggcc ctgccagctc ttcgacttca atggggctag gggcgaccag tgcctgaagc 1020

tcacgtacat ctcagagggt tgggagaatg tggatgacag cagttgccag ctggttcccc 1080

atctccacat ctggcatgga aagtgcccct tccgctcctt ctgcttccgg agaaaagcag 1140

ccgatgagaa tgaggactgt tcagcactaa ccaatgagat cattgtcacc atgcacacct 1200

tccaggatgg cttggagacc aagtatatgg aaatcctcag attccaggca tcagaggagg 1260

aatcctgggc agcgccacca cctgtttccc agccgccccc atgcaatagg ctgcccccag 1320

agctctttga gcagctgcgg atgttattgg agccaaacag catcaccggc aatgactggc 1380

gcggactggc ctcccacctg ggctttgcgg gcatgaagat ccggttcctg tcctgccagc 1440

gcagccccgc agcggccatc ctggagttgt ttgaggagca gaacggcagc ctgcaggagc 1500

tgcactacct catgaccgtc atggagcggc tagactgcgc ctccgccatc cagaactacc 1560

tgagtgggac acacggcggc agcccaggcc ccgagcgcgg gggcgcccgg gataaccagg 1620

gcctggagct ggacgagaag ctctgagcgc ccagcgggca gggccggagg aggtgtaggg 1680

gcgagagggg ttgttctcct acgcctaaga ggaacacagc agctgttcct ggctgtgccc 1740

accacccttc ccagaaccct cggacggtgc cagggccgcc cgcgtcctcg ccagcctccc 1800

agcagcaggc gcgcccttcc ggccgcgagc tgtcccgcgt gcgggtcccc tcctggccgg 1860

cagagggcgc aggagcccag gagatggcct cagcgtggcc agcctgccaa actgaccagg 1920

ggacggccac ggtccccgcg tgcactttag ggccggtcag gccctaaagt attcactgag 1980

cacttaggaa gaccgggcat tgggctggtg tcatgaagaa tactggaata taggaggtag 2040

ggtctggagc cctgacaagc acacagttct tttgcaggag tcaagatgtg ctcataggag 2100

ccaagggcaa actcgaaaac agccctgccc tgtggttagg ggaggtgggt gtggctttag 2160

gagttgagag gggaagagct gagtgcgggc tggagcagcg agaatgcttc acggaggatg 2220

ttggggagag aggatttgat ttgccgagtg gtttggagat ctttaggggc gggtagggga 2280

agagggtggc tggacttaag gtaggagccg aaagtgggca tgggagccca tccccgtggg 2340

tgtgaggccg gggtaggacc aggcttcccg gctaggcacg cctggctggc tgcctctgcc 2400

tctactaact tcctttgtct ctttatcctc ctgcttaggc cgtgtcttcc tctctctctg 2460

ccattttctc tatcagaaaa cattaaagct gagtagaaac ttcaagatga tcattctggc 2520

ccagcccttt tatctcttta tctccaatgg tgtgcgtttt aagtttctct gacttggttt 2580

ttctgtccct gtctcccaca gttgttcccg ctctgcacct gtgtttcagt tactctctct 2640

ctctgtctta gatctttttg tttctgccta gtctctgtgc cctgtctttg tgtgtttctt 2700

tctctgcctg aaaccctccc tcttttccct tcaggcatat gtctctaaac caatgttatg 2760

tatcaaagac tgttgaaaga gacagggcta ctctaggaat ggatgggtgg ggatattttc 2820

cccatggatc aagatccagt ttagggttgg gaaataaagg atagggataa aagaggcacc 2880

ccattcagtc agcagatact tattcagcac ctactatgtg tttgatactg tatacaaaag 2940

acatgaatgg gctgggtcct gaacagagaa taaagggttt gactccaaaa aaaaaaaaaa 3000

aaaaaaaaaa aaaaaaaaaa aaaaaaaaaa aaaaaaaaaa aaaaaaaaaa aaaaaaaaaa 3060

aaaaaaaaaa aaaaaaaaaa aaaaaaaaaa aaaaaa 3096

<210> 52
<211> 518
<212> PRT
<213> Homo sapiens

<400> 52
Met Cys Pro Gln Glu Ser Ser Phe Gln Pro Ser Gln Phe Leu Leu Leu
1               5                   10                  15

Val Gly Val Pro Val Ala Ser Val Leu Leu Leu Ala Gln Cys Leu Arg

            20                  25                  30

Trp His Cys Pro Arg Arg Leu Leu Gly Ala Cys Trp Thr Leu Asn Gly
        35                  40                  45

Gln Glu Glu Pro Val Ser Gln Pro Thr Pro Gln Leu Glu Asn Glu Val
    50                  55                  60

Ser Arg Gln His Leu Pro Ala Thr Leu Pro Glu Met Val Ala Phe Tyr
65                  70                  75                  80

Gln Glu Leu His Thr Pro Thr Gln Gly Gln Thr Met Val Arg Gln Leu
                85                  90                  95

Met His Lys Leu Leu Val Phe Ser Ala Arg Glu Val Asp His Arg Gly
            100                 105                 110

Gly Cys Leu Met Leu Gln Asp Thr Gly Ile Ser Leu Leu Ile Pro Pro
        115                 120                 125

Gly Ala Val Ala Val Gly Arg Gln Glu Arg Val Ser Leu Ile Leu Val
    130                 135                 140

Trp Asp Leu Ser Asp Ala Pro Ser Leu Ser Gln Ala Gln Gly Leu Val
145                 150                 155                 160

Ser Pro Val Val Ala Cys Gly Pro His Gly Ala Ser Phe Leu Lys Pro
                165                 170                 175

Cys Thr Leu Thr Phe Lys His Cys Ala Glu Gln Pro Ser His Ala Arg
            180                 185                 190

Thr Tyr Ser Ser Asn Thr Thr Leu Leu Asp Ala Lys Val Trp Arg Pro
        195                 200                 205

Leu Gly Arg Pro Gly Ala His Ala Ser Arg Asp Glu Cys Arg Ile His
    210                 215                 220

Leu Ser His Phe Ser Leu Tyr Thr Cys Val Leu Glu Ala Pro Val Gly
225                 230                 235                 240

Arg Glu Ala Arg Lys Trp Leu Gln Leu Ala Val Phe Cys Ser Pro Leu
                245                 250                 255

Val Pro Gly Gln Ser His Leu Gln Leu Arg Ile Tyr Phe Leu Asn Asn
            260                 265                 270

Thr Pro Cys Ala Leu Gln Trp Ala Leu Thr Asn Glu Gln Pro His Gly
        275                 280                 285

Gly Arg Leu Arg Gly Pro Cys Gln Leu Phe Asp Phe Asn Gly Ala Arg
    290                 295                 300

Gly Asp Gln Cys Leu Lys Leu Thr Tyr Ile Ser Glu Gly Trp Glu Asn
305                 310                 315                 320

Val Asp Asp Ser Ser Cys Gln Leu Val Pro His Leu His Ile Trp His
                325                 330                 335

Gly Lys Cys Pro Phe Arg Ser Phe Cys Phe Arg Arg Lys Ala Ala Asp
            340                 345                 350

Glu Asn Glu Asp Cys Ser Ala Leu Thr Asn Glu Ile Ile Val Thr Met
        355                 360                 365

His Thr Phe Gln Asp Gly Leu Glu Thr Lys Tyr Met Glu Ile Leu Arg
    370                 375                 380

Phe Gln Ala Ser Glu Glu Glu Ser Trp Ala Ala Pro Pro Pro Val Ser
385                 390                 395                 400

Gln Pro Pro Pro Cys Asn Arg Leu Pro Pro Glu Leu Phe Glu Gln Leu
                405                 410                 415

Arg Met Leu Leu Glu Pro Asn Ser Ile Thr Gly Asn Asp Trp Arg Gly
            420                 425                 430

Leu Ala Ser His Leu Gly Leu Cys Gly Met Lys Ile Arg Phe Leu Ser
        435                 440                 445

Cys Gln Arg Ser Pro Ala Ala Ala Ile Leu Glu Leu Phe Glu Glu Gln
    450                 455                 460

Asn Gly Ser Leu Gln Glu Leu His Tyr Leu Met Thr Val Met Glu Arg
465                 470                 475                 480

Leu Asp Cys Ala Ser Ala Ile Gln Asn Tyr Leu Ser Gly Thr His Gly
                485                 490                 495

Gly Ser Pro Gly Pro Glu Arg Gly Gly Ala Arg Asp Asn Gln Gly Leu
            500                 505                 510

Glu Leu Asp Glu Lys Leu
        515

<210> 53
<211> 1437
<212> DNA
<213> Homo sapiens

<400> 53
attgctgatg gatcagtgag cctgtgttca tgccagtgag ctgctgtggc tcagatactg      60

atactttctt tccaaacagc ataagaagtg attgagccac aagtatactg aaggaagggc     120

tccctcgagt tctggtgtga agagataaat caccagtcac agactatgca cccgactgct     180

gctgttcagt ccagggaaaa tgaaagttgg agtgctgtgg ctcatttctt tcttcacctt     240

cactgacggc cacggtggct tcctggggaa aaatgatggc atcaaaacaa aaaaagaact     300

cattgtgaat aagaaaaaac atctaggccc agtcgaagaa tatcagctgc tgcttcaggt     360

gacctataga gattccaagg agaaaagaga tttgagaaat tttctgaagc tcttgaagcc     420

tccattatta tggtcacatg ggctaattag aattatcaga gcaaaggcta ccacagactg     480

caacagcctg aatggagtcc tgcagtgtac ctgtgaagac agctacacct ggtttcctcc     540

ctcatgcctt gatccccaga actgctacct tcacacggct ggagcactcc caagctgtga     600

atgtcatctc aacaacctca gccagagtgt caatttctgt gagagaacaa agatttgggg     660

cactttcaaa attaatgaaa ggtttacaaa tgaccttttg aattcatctt ctgctatata     720

ctccaaatat gcaaatggaa ttgaaattca acttaaaaaa gcatatgaaa gaattcaagg     780

ttttgagtcg gttcaggtca cccaatttcg aatgtcactc ttgtcgccca agttggagtg     840

caatggcaca atctaggctc actgcaaccc tgcaacctct gcctaccggg ttcaagagat     900

tcccctgcct cagcctccca agtagctgga attacaggca cctgccacca catccagcta     960

actttttttg tatttttact agagacaggg tttcaccatg ttggccacac tggtctcaaa    1020

ctcctgacct caggtgatcc gcctgcctcg gcccccaaag tgctgggatt acaggcatga    1080

gccaccacat ctggcctagg accttaaata ttggaaagca tcctcaaaac tgtgggtcag    1140

tgagtagaac tacaaaacaa tagcagtagg gcagaaactt gaaagaaggc aggagatcat    1200

ggtgacagtg gatgggaaaa agtgagggtt ggggataagg gttgcgggtt gtcgaagggt    1260

ggatttctcc cttcagcaac tacaggagat atgatgcctc ataattcgga gccagaagtg    1320

gggctttggg tgagatatct ttgcacagat aacatgtata catcatagtt caaaacccag    1380

tagtcattgt ttacagcaaa taaagaaata tttagtaaat taaaaaaaaa aaaaaaa       1437

<210> 54

<211> 218
<212> PRT
<213> Homo sapiens

<400> 54
Met Lys Val Gly Val Leu Trp Leu Ile Ser Phe Phe Thr Phe Thr Asp
1               5                   10                  15

Gly His Gly Gly Phe Leu Gly Lys Asn Asp Gly Ile Lys Thr Lys Lys
            20                  25                  30

Glu Leu Ile Val Asn Lys Lys Lys His Leu Gly Pro Val Glu Glu Tyr
        35                  40                  45

Gln Leu Leu Leu Gln Val Thr Tyr Arg Asp Ser Lys Glu Lys Arg Asp
    50                  55                  60

Leu Arg Asn Phe Leu Lys Leu Leu Lys Pro Pro Leu Leu Trp Ser His
65                  70                  75                  80

Gly Leu Ile Arg Ile Ile Arg Ala Lys Ala Thr Thr Asp Cys Asn Ser
                85                  90                  95

Leu Asn Gly Val Leu Gln Cys Thr Cys Glu Asp Ser Tyr Thr Trp Phe
            100                 105                 110

Pro Pro Ser Cys Leu Asp Pro Gln Asn Cys Tyr Leu His Thr Ala Gly
        115                 120                 125

Ala Leu Pro Ser Cys Glu Cys His Leu Asn Asn Leu Ser Gln Ser Val
    130                 135                 140

Asn Phe Cys Glu Arg Thr Lys Ile Trp Gly Thr Phe Lys Ile Asn Glu
145                 150                 155                 160

Arg Phe Thr Asn Asp Leu Leu Asn Ser Ser Ser Ala Ile Tyr Ser Lys
                165                 170                 175

Tyr Ala Asn Gly Ile Glu Ile Gln Leu Lys Lys Ala Tyr Glu Arg Ile
            180                 185                 190

Gln Gly Phe Glu Ser Val Gln Val Thr Gln Phe Arg Met Ser Leu Leu
        195                 200                 205

Ser Pro Lys Leu Glu Cys Asn Gly Thr Ile
    210                 215

<210> 55
<211> 1096
<212> DNA
<213> Homo sapiens

<400> 55
atggtgctca tggcgccccg aaccctcctc ctgctgctct caggggccct ggccctgacc      60

cagacctggg cgcgctccca ctccatgagg tatttctaca ccaccatgtc ccggcccggc     120

cgcggggagc cccgcttcat ctccgtcggc tacgtggacg atacgcagtt cgtgcggttc     180

gacagcgacg ccgcgagcca gaggatggag ccgcgggcgc cgtggatgga gcgggagggg     240

ccggagtatt gggaccggaa cacacagatc tgcaaggccc aggcacagac tgaacgagag     300

aacctgcgga tcgcgctccg ctactacaac cagagcgagg gcggttctca caccatgcag     360

gtgatgtatg gctgcgacgt ggggcccgac gggcgcttcc tccgcgggta tgaacagcac     420

gcctacgaca gcaaggatta catcgctctg aacgaggacc tgcgctcctg gaccgcggcg     480

gacatggcag ctcagatcac caagcgcaag tgggaggcgg cccgtcaggc ggagcagctg     540

agagcctacc tggagggcga gttcgtggag tggctccgca gatacctgga gaacggaaag     600

gagacgctgc agcgcgcgga cccccccaag acacatatga cccaccaccc catctctgac     660

catgaggcca ccctgaggtg ctgggccctg ggcttctacc ctgcggagat cacactgacc     720

tggcagcggg atggggagga ccagacccac acacggagct catggagacc aggcctgcag     780

gggatggaac cttccagaag tgggcggctg tggtggtgcc ttctggagag gagcagagat     840

acacctgcca tgtgcagcat gagggtctgc cagagcccct caccctgaga tgggagccat     900

cttcccagcc caccatcccc atcgtgggca tcgttgctgg cctggttcta cttgtagctg     960

tggtcactgg agctgtggtc gctgctgtaa tgtggaggaa gaagagctca gatagaaaag    1020

gagggagcta ctctcaggct gcaagcggca acagtgccca gggctctgat gtgtctctca    1080

cggcgtgaaa gtgtga                                                    1096


<210> 56
<211> 295
<212> PRT
<213> Homo sapiens

<400> 56
Met Val Leu Met Ala Pro Arg Thr Leu Leu Leu Leu Leu Ser Gly Ala
1               5                   10                  15


Leu Ala Leu Thr Gln Thr Trp Ala Arg Ser His Ser Met Arg Tyr Phe
            20                  25                  30


Tyr Thr Thr Met Ser Arg Pro Gly Arg Gly Glu Pro Arg Phe Ile Ser
        35                  40                  45

Val Gly Tyr Val Asp Asp Thr Gln Phe Val Arg Phe Asp Ser Asp Ala
    50                  55                  60

Ala Ser Gln Arg Met Glu Pro Arg Ala Pro Trp Met Glu Arg Glu Gly
65                  70                  75                  80

Pro Glu Tyr Trp Asp Arg Asn Thr Gln Ile Cys Lys Ala Gln Ala Gln
                85                  90                  95

Thr Glu Arg Glu Asn Leu Arg Ile Ala Leu Arg Tyr Tyr Asn Gln Ser
            100                 105                 110

Glu Gly Gly Ser His Thr Met Gln Val Met Tyr Gly Cys Asp Val Gly
        115                 120                 125

Pro Asp Gly Arg Phe Leu Arg Gly Tyr Glu Gln His Ala Tyr Asp Ser
    130                 135                 140

Lys Asp Tyr Ile Ala Leu Asn Glu Asp Leu Arg Ser Trp Thr Ala Ala
145                 150                 155                 160

Asp Met Ala Ala Gln Ile Thr Lys Arg Lys Trp Glu Ala Ala Arg Gln
                165                 170                 175

Ala Glu Gln Leu Arg Ala Tyr Leu Glu Gly Glu Phe Val Glu Trp Leu
            180                 185                 190

Arg Arg Tyr Leu Glu Asn Gly Lys Glu Thr Leu Gln Arg Ala Asp Pro
        195                 200                 205

Pro Lys Thr His Met Thr His His Pro Ile Ser Asp His Glu Ala Thr
    210                 215                 220

Leu Arg Cys Trp Ala Leu Gly Phe Tyr Pro Ala Glu Ile Thr Leu Thr
225                 230                 235                 240

Trp Gln Arg Asp Gly Glu Asp Gln Thr His Thr Arg Ser Ser Trp Arg
                245                 250                 255

Pro Gly Leu Gln Gly Met Glu Pro Ser Arg Ser Gly Arg Leu Trp Trp
            260                 265                 270

Cys Leu Leu Glu Arg Ser Arg Asp Thr Pro Ala Met Cys Ser Met Arg
        275                 280                 285

Val Cys Gln Ser Pro Ser Pro
    290                 295

<210> 57
<211> 2974
<212> DNA
<213> Homo sapiens

<400> 57
   gtgtgcgtga tggagaaaat tgggcaccag ggctgctccc gagattctca gatctgattt      60
   ccacgcttgc taccaaaata gtctgggcag gccacttttg gaagtaggcg ttatctagtg     120
   agcaggcggc cgctttcgat ttcgctttcc cctaaatggc tgagcttctc gccagcgcag     180
   gatcagcctg ttcctgggac tttccgagag ccccgccctc gttccctccc ccagccgcca     240
   gtaggggagg actcggcggt acccggagct tcaggcccca ccggggcgcg gagagtccca     300
   ggcccggccg ggaccgggac ggcgtccgag tgccaatggc tagctctagg tgtcccgctc     360
   cccgcgggtg ccgctgcctc cccggagctt ctctcgcatg gctggggaca gtactgctac     420
   ttctcgccga ctgggtgctg ctccggaccg cgctgccccg catattctcc ctgctggtgc     480
   ccaccgcgct gccactgctc cgggtctggg cggtgggcct gagccgctgg gccgtgctct     540
   ggctgggggc ctgcggggtc ctcagggcaa cggttggctc caagagcgaa aacgcaggtg     600
   cccagggctg gctggctgct ttgaagccat tagctgcggc actgggcttg gccctgccgg     660
   gacttgcctt gttccgagag ctgatctcat ggggagcccc cgggtccgcg gatagcacca     720
   ggctactgca ctggggaagt caccctaccg ccttcgttgt cagttatgca gcggcactgc     780
   ccgcagcagc cctgtggcac aaactcggga gcctctgggt gcccggcggt cagggcggct     840
   ctggaaaccc tgtgcgtcgg cttctaggct gcctgggctc ggagacgcgc cgcctctcgc     900
   tgttcctggt cctggtggtc ctctcctctc ttggggagat ggccattcca ttctttacgg     960
   gccgcctcac tgactggatt ctacaagatg gctcagccga taccttcact cgaaacttaa    1020
   ctctcatgtc cattctcacc atagccagtg cagtgctgga gttcgtgggt gacgggatct    1080
   ataacaacac catgggccac gtgcacagcc acttgcaggg agaggtgttt ggggctgtcc    1140
   tgcgccagga gacggagttt ttccaacaga accagacagg taacatcatg tctcgggtaa    1200
   cagaggacac gtccaccctg agtgattctc tgagtgagaa tctgagctta tttctgtggt    1260
   acctggtgcg aggcctatgt ctcttgggga tcatgctctg gggatcagtg tccctcacca    1320
   tggtcaccct gatcaccctg cctctgcttt tccttctgcc caagaaggtg ggaaaatggt    1380
   accagttgct ggaagtgcag gtgcgggaat ctctggcaaa gtccagccag gtggccattg    1440
   aggctctgtc ggccatgcct acagttcgaa gctttgccaa cgaggagggc gaagcccaga    1500
   agtttaggga aaagctgcaa gaaataaaga cactcaacca gaaggaggct gtggcctatg 1560
   cagtcaactc ctggaccact agtatttcag gtatgctgct gaaagtggga atcctctaca 1620
   ttggtgggca gctggtgacc agtggggctg taagcagtgg gaaccttgtc acatttgttc 1680
   tctaccagat gcagttcacc caggctgtgg aggtactgct ctccatctac cccagagtac 1740
   agaaggctgt gggctcctca gagaaaatat ttgagtacct ggaccgcacc cctcgctgcc 1800
   cacccagtgg tctgttgact cccttacact tggagggcct tgtccagttc caagatgtct 1860
   cctttgccta cccaaaccgc ccagatgtct tagtgctaca ggggctgaca ttcaccctac 1920
   gccctggcga ggtgacggcg ctggtgggac ccaatgggtc tgggaagagc acagtggctg 1980
   ccctgctgca gaatctgtac cagcccaccg ggggacagct gctgttggat gggaagcccc 2040
   ttccccaata tgagcaccgc tacctgcaca ggcaggtggc tgcagtggga caagagccac 2100
   aggtatttgg aagaagtctt caagaaaata ttgcctatgg cctgacccag aagccaacta 2160
   tggaggaaat cacagctgct gcagtaaagt ctggggccca tagtttcatc tctggactcc 2220
   ctcagggcta tgacacagag gtagacgagg ctgggagcca gctgtcaggg ggtcagcgac 2280
   aggcagtggc gttggcccga gcattgatcc ggaaaccgtg tgtacttatc ctggatgatg 2340
   ccaccagtgc cctggatgca aacagccagt tacaggtgga gcagctcctg tacgaaagcc 2400
   ctgagcggta ctcccgctca gtgcttctca tcacccagca cctcagcctg gtggagcagg 2460
   ctgaccacat cctctttctg gaaggaggcg ctatccggga gggggaaccc caccagcagc 2520
   tcatggagaa aaaggggtgc tactgggcca tggtgcaggc tcctgcagat gctccagaat 2580
   gaaagccttc tcagacctgc gcactccatc tccctccctt ttcttctctc tgtggtggag 2640
   aaccacagct gcagagtagg cagctgcctc caggatgagt tacttgaaat ttgccttgag 2700
   tgtgttacct cctttccaag ctcctcgtga taatgcagac ttcctggagt acaaacacag 2760
   gatttgtaat tccttactgt aacggagttt agagccaggg ctgatgcttt ggtgtggcca 2820
   gcactctgaa actgagaaat gttcagaatg tacggaaaga tgatcagcta ttttcaacat 2880
   aactgaaggc atatgctggc ccataaacac cctgtaggtt cttgatattt ataataaaat 2940
   tggtgttttg taaaaaaaaa aaaaaaaaaa aaaa 2974

<210> 58
<211> 808
<212> PRT
<213> Homo sapiens

<400> 58
Met Ala Glu Leu Leu Ala Ser Ala Gly Ser Ala Cys Ser Trp Asp Phe
1               5                   10                  15

Pro Arg Ala Pro Pro Ser Phe Pro Pro Pro Ala Ala Ser Arg Gly Gly
            20                  25                  30

Leu Gly Gly Thr Arg Ser Phe Arg Pro His Arg Gly Ala Glu Ser Pro
        35                  40                  45

Arg Pro Gly Arg Asp Arg Asp Gly Val Arg Val Pro Met Ala Ser Ser
    50                  55                  60

Arg Cys Pro Ala Pro Arg Gly Cys Arg Cys Leu Pro Gly Ala Ser Leu
65                  70                  75                  80

Ala Trp Leu Gly Thr Val Leu Leu Leu Leu Ala Asp Trp Val Leu Leu
                85                  90                  95

Arg Thr Ala Leu Pro Arg Ile Phe Ser Leu Leu Val Pro Thr Ala Leu
            100                 105                 110

Pro Leu Leu Arg Val Trp Ala Val Gly Leu Ser Arg Trp Ala Val Leu
        115                 120                 125

Trp Leu Gly Ala Cys Gly Val Leu Arg Ala Thr Val Gly Ser Lys Ser
    130                 135                 140

Glu Asn Ala Gly Ala Gln Gly Trp Leu Ala Ala Leu Lys Pro Leu Ala
145                 150                 155                 160

Ala Ala Leu Gly Leu Ala Leu Pro Gly Leu Ala Leu Phe Arg Glu Leu
                165                 170                 175

Ile Ser Trp Gly Ala Pro Gly Ser Ala Asp Ser Thr Arg Leu Leu His
            180                 185                 190

Trp Gly Ser His Pro Thr Ala Phe Val Val Ser Tyr Ala Ala Ala Leu
        195                 200                 205

Pro Ala Ala Ala Leu Trp His Lys Leu Gly Ser Leu Trp Val Pro Gly
    210                 215                 220

Gly Gln Gly Gly Ser Gly Asn Pro Val Arg Arg Leu Leu Gly Cys Leu
225                 230                 235                 240

Gly Ser Glu Thr Arg Arg Leu Ser Leu Phe Leu Val Leu Val Val Leu
                245                 250                 255

Ser Ser Leu Gly Glu Met Ala Ile Pro Phe Phe Thr Gly Arg Leu Thr

            260                 265                 270

Asp Trp Ile Leu Gln Asp Gly Ser Ala Asp Thr Phe Thr Arg Asn Leu
        275                 280                 285

Thr Leu Met Ser Ile Leu Thr Ile Ala Ser Ala Val Leu Glu Phe Val
    290                 295                 300

Gly Asp Gly Ile Tyr Asn Asn Thr Met Gly His Val His Ser His Leu
305                 310                 315                 320

Gln Gly Glu Val Phe Gly Ala Val Leu Arg Gln Glu Thr Glu Phe Phe
                325                 330                 335

Gln Gln Asn Gln Thr Gly Asn Ile Met Ser Arg Val Thr Glu Asp Thr
            340                 345                 350

Ser Thr Leu Ser Asp Ser Leu Ser Glu Asn Leu Ser Leu Phe Leu Trp
        355                 360                 365

Tyr Leu Val Arg Gly Leu Cys Leu Leu Gly Ile Met Leu Trp Gly Ser
    370                 375                 380

Val Ser Leu Thr Met Val Thr Leu Ile Thr Leu Pro Leu Leu Phe Leu
385                 390                 395                 400

Leu Pro Lys Lys Val Gly Lys Trp Tyr Gln Leu Leu Glu Val Gln Val
                405                 410                 415

Arg Glu Ser Leu Ala Lys Ser Ser Gln Val Ala Ile Glu Ala Leu Ser
            420                 425                 430

Ala Met Pro Thr Val Arg Ser Phe Ala Asn Glu Glu Gly Glu Ala Gln
        435                 440                 445

Lys Phe Arg Glu Lys Leu Gln Glu Ile Lys Thr Leu Asn Gln Lys Glu
    450                 455                 460

Ala Val Ala Tyr Ala Val Asn Ser Trp Thr Thr Ser Ile Ser Gly Met
465                 470                 475                 480

Leu Leu Lys Val Gly Ile Leu Tyr Ile Gly Gly Gln Leu Val Thr Ser
                485                 490                 495

Gly Ala Val Ser Ser Gly Asn Leu Val Thr Phe Val Leu Tyr Gln Met
            500                 505                 510

Gln Phe Thr Gln Ala Val Glu Val Leu Leu Ser Ile Tyr Pro Arg Val
        515                 520                 525

Gln Lys Ala Val Gly Ser Ser Glu Lys Ile Phe Glu Tyr Leu Asp Arg
    530                 535                 540

Thr Pro Arg Cys Pro Pro Ser Gly Leu Leu Thr Pro Leu His Leu Glu
545                 550                 555                 560

Gly Leu Val Gln Phe Gln Asp Val Ser Phe Ala Tyr Pro Asn Arg Pro
                565                 570                 575

Asp Val Leu Val Leu Gln Gly Leu Thr Phe Thr Leu Arg Pro Gly Glu
            580                 585                 590

Val Thr Ala Leu Val Gly Pro Asn Gly Ser Gly Lys Ser Thr Val Ala
        595                 600                 605

Ala Leu Leu Gln Asn Leu Tyr Gln Pro Thr Gly Gly Gln Leu Leu Leu
    610                 615                 620

Asp Gly Lys Pro Leu Pro Gln Tyr Glu His Arg Tyr Leu His Arg Gln
625                 630                 635                 640

Val Ala Ala Val Gly Gln Glu Pro Gln Val Phe Gly Arg Ser Leu Gln
                645                 650                 655

Glu Asn Ile Ala Tyr Gly Leu Thr Gln Lys Pro Thr Met Glu Glu Ile
            660                 665                 670

Thr Ala Ala Ala Val Lys Ser Gly Ala His Ser Phe Ile Ser Gly Leu
        675                 680                 685

Pro Gln Gly Tyr Asp Thr Glu Val Asp Glu Ala Gly Ser Gln Leu Ser
    690                 695                 700

Gly Gly Gln Arg Gln Ala Val Ala Leu Ala Arg Ala Leu Ile Arg Lys
705                 710                 715                 720

Pro Cys Val Leu Ile Leu Asp Asp Ala Thr Ser Ala Leu Asp Ala Asn
                725                 730                 735

Ser Gln Leu Gln Val Glu Gln Leu Leu Tyr Glu Ser Pro Glu Arg Tyr
            740                 745                 750

Ser Arg Ser Val Leu Leu Ile Thr Gln His Leu Ser Leu Val Glu Gln
        755                 760                 765

Ala Asp His Ile Leu Phe Leu Glu Gly Gly Ala Ile Arg Glu Gly Gly
    770                 775                 780

Thr His Gln Gln Leu Met Glu Lys Lys Gly Cys Tyr Trp Ala Met Val
785                 790                 795                 800

Gln Ala Pro Ala Asp Ala Pro Glu
                805

<210> 59
<211> 3096
<212> DNA
<213> Homo sapiens

<400> 59
cgggcgccca gagggcaaat gaggggcggc ggggtggcgg gtcgggggga gacgcggaat      60

ttcccagcgc gggggctctg gcttaccctg caaccgggca gtctctttct gtttacggag     120

agaaagggga aatggaaaag tcggggaggc ggtggctggc gtccgctgcg ccgcccctgg     180

gcaggctcag acgccgtgag tcaggggcag agcagggcgg tctgagcgtg cgggcgacgc     240

gggtctcact cgtccgctcc gctctggact gcgcgccacg ctctggggtc cggcgccctg     300

gttcctgctt ctgccgctgc cgccgccgga tcccagtggc ccggcgtgct cggctcccac     360

aggcctgcag ccagcatcgc accgaacctt cggggggccg cggctggagc gctcggccgg     420

cgtgggagcg ccaaggccgc agatgcaatc ttcttaccgc gaagaagcca ggggaatagg     480

tagccacatc ttgtttgcag ataagaaagg aagctaacgc agtatctgca aagccaggag     540

tctgactcag tacttttctc actcatgcat acaaagcagc taaaaatgac acagcttatt     600

taccatgccc ctgacactgc actgagcact ttatgagctt gaactctgtt aatcctcacg     660

accacctcat gagactctcc agaaagagca acagtaatgg agtacatgag cactggaagt     720

gacaataaag aagagattga tttattaatt aaacatttaa atgtgtctga tgtaatagac     780

attatggaaa atctttatgc aagtgaagag ccagcagttt atgaacccag tctaatgacc     840

atgtgtcaag acagtaatca aaacgatgag cgttctaagt ctctgctgct tagtggccaa     900

gaggtaccat ggttgtcatc agtcagatat ggaactgtgg aggatttgct tgcttttgca     960

aaccatatat ccaacactgc aaagcatttt tatggacaac gaccacagga atctggaatt    1020

ttattaaaca tggtcatcac tccccaaaat ggacgttacc aaatagattc cgatgttctc    1080

ctgatcccct ggaagctgac ttacaggaat attggttctg attttattcc tcggggcgcc    1140

tttggaaagg tatacttggc acaagatata aagacgaaga aaagaatggc gtgtaaactg 1200

atcccagtag atcaatttaa gccatctgat gtggaaatcc aggcttgctt ccggcacgag 1260

aacatcgcag agctgtatgg cgcagtcctg tggggtgaaa ctgtccatct ctttatggaa 1320

gcaggcgagg gagggtctgt tctggagaaa ctggagagct gtggaccaat gagagaattt 1380

gaaattattt gggtgacaaa gcatgttctc aagggacttg attttctaca ctcaaagaaa 1440

gtgatccatc atgatattaa acctagcaac attgttttca tgtccacaaa agctgttttg 1500

gtggattttg gcctaagtgt tcaaatgacc gaagatgtct attttcctaa ggacctccga 1560

ggaacagaga tttacatgag cccagaggtc atcctgtgca ggggccattc aaccaaagca 1620

gacatctaca gcctgggggc cacgctcatc cacatgcaga cgggcacccc accctgggtg 1680

aagcgctacc ctcgctcagc ctatccctcc tacctgtaca taatccacaa gcaagcacct 1740

ccactggaag acattgcaga tgactgcagt ccagggatga gagagctgat agaagcttcc 1800

ctggagagaa accccaatca ccgcccaaga gccgcagacc tactaaaaca tgaggccctg 1860

aacccgccca gagaggatca gccacgctgt cagagtctgg actctgccct cttggagcgc 1920

aagaggctgc tgagtaggaa ggagctggaa cttcctgaga acattgctga ttcttcgtgc 1980

acaggaagca ccgaggaatc tgagatgctc aagaggcaac gctctctcta catcgacctc 2040

ggcgctctgg ctggctactt caatcttgtt cggggaccac caacgcttga atatggctga 2100

aggatgccat gtttgctcta aattaagaca gcattgatct cctggaggct ggttctgctg 2160

cctctacaca ggggccctgt acagtgaatg gtgccatttt cgaaggagca gtgtgacctc 2220

ctgtgaccca tgaatgtgcc tccaagcggc cctgtgtgtt tgacatgtga agctatttga 2280

tatgcaccag gtctcaaggt tctcatttct caggtgacgt gattctaagg caggaatttg 2340

agagttcaca gaaggatcgt gtctgctgac tgtttcattc actgtgcact ttgctcaaaa 2400

ttttaaaaat accaatcaca aggataatag agtagcctaa aattactatt cttggttctt 2460

atttaagtat ggaatattca ttttactcag aatagctgtt ttgtgtatat tggtgtatat 2520

tatataactc tttgagcctt tattggtaaa ttctggtata cattgaattc attataattt 2580

gggtgactag aacaacttga agattgtagc aataagctgg actagtgtcc taaaaatggc 2640

taactgatga attagaagcc atctgacagc aggccactag tgacagtttc ttttgtgttc 2700

ctatggaaac attttatact gtacatgcta tgctgaagac attcaaaacg tgatgttttg 2760

aatgtggata aaactgtgta aaccacataa tttttgtaca tcccaaagga tgagaatgtg 2820

acctttaaga aaaatgaaaa cttttgtaaa ttattgatga ttttgtaatt cttatgacta 2880

aattttcttt taagcatttg tatattaaaa tagcatactg tgtatgtttt atatcaaatg 2940

ccttcatgaa tctttcatac atatatatat ttgtaacatt gtaaagtatg tgagtagtct 3000

tatgtaaagt atgtttttac attatgcaaa taaaacccaa tacttttgtc caatgtggtt 3060

ggtcaaatca actgaataaa ttcagtattt tgcctt 3096

<210> 60
<211> 467
<212> PRT
<213> Homo sapiens

<400> 60
Met Glu Tyr Met Ser Thr Gly Ser Asp Asn Lys Glu Glu Ile Asp Leu
1               5                   10                  15

Leu Ile Lys His Leu Asn Val Ser Asp Val Ile Asp Ile Met Glu Asn
            20                  25                  30

Leu Tyr Ala Ser Glu Glu Pro Ala Val Tyr Glu Pro Ser Leu Met Thr
        35                  40                  45

Met Cys Gln Asp Ser Asn Gln Asn Asp Glu Arg Ser Lys Ser Leu Leu
    50                  55                  60

Leu Ser Gly Gln Glu Val Pro Trp Leu Ser Ser Val Arg Tyr Gly Thr
65                  70                  75                  80

Val Glu Asp Leu Leu Ala Phe Ala Asn His Ile Ser Asn Thr Ala Lys
                85                  90                  95

His Phe Tyr Gly Gln Arg Pro Gln Glu Ser Gly Ile Leu Leu Asn Met
            100                 105                 110

Val Ile Thr Pro Gln Asn Gly Arg Tyr Gln Ile Asp Ser Asp Val Leu
        115                 120                 125

Leu Ile Pro Trp Lys Leu Thr Tyr Arg Asn Ile Gly Ser Asp Phe Ile
    130                 135                 140

Pro Arg Gly Ala Phe Gly Lys Val Tyr Leu Ala Gln Asp Ile Lys Thr
145                 150                 155                 160

Lys Lys Arg Met Ala Cys Lys Leu Ile Pro Val Asp Gln Phe Lys Pro
                165                 170                 175

Ser Asp Val Glu Ile Gln Ala Cys Phe Arg His Glu Asn Ile Ala Glu
            180                 185                 190

Leu Tyr Gly Ala Val Leu Trp Gly Glu Thr Val His Leu Phe Met Glu

195                 200                 205

Ala Gly Glu Gly Gly Ser Val Leu Glu Lys Leu Glu Ser Cys Gly Pro
    210                 215                 220

Met Arg Glu Phe Glu Ile Ile Trp Val Thr Lys His Val Leu Lys Gly
225                 230                 235                 240

Leu Asp Phe Leu His Ser Lys Lys Val Ile His His Asp Ile Lys Pro
                245                 250                 255

Ser Asn Ile Val Phe Met Ser Thr Lys Ala Val Leu Val Asp Phe Gly
            260                 265                 270

Leu Ser Val Gln Met Thr Glu Asp Val Tyr Phe Pro Lys Asp Leu Arg
        275                 280                 285

Gly Thr Glu Ile Tyr Met Ser Pro Glu Val Ile Leu Cys Arg Gly His
    290                 295                 300

Ser Thr Lys Ala Asp Ile Tyr Ser Leu Gly Ala Thr Leu Ile His Met
305                 310                 315                 320

Gln Thr Gly Thr Pro Pro Trp Val Lys Arg Tyr Pro Arg Ser Ala Tyr
                325                 330                 335

Pro Ser Tyr Leu Tyr Ile Ile His Lys Gln Ala Pro Pro Leu Glu Asp
            340                 345                 350

Ile Ala Asp Asp Cys Ser Pro Gly Met Arg Glu Leu Ile Glu Ala Ser
        355                 360                 365

Leu Glu Arg Asn Pro Asn His Arg Pro Arg Ala Ala Asp Leu Leu Lys
    370                 375                 380

His Glu Ala Leu Asn Pro Pro Arg Glu Asp Gln Pro Arg Cys Gln Ser
385                 390                 395                 400

Leu Asp Ser Ala Leu Leu Glu Arg Lys Arg Leu Leu Ser Arg Lys Glu
                405                 410                 415

Leu Glu Leu Pro Glu Asn Ile Ala Asp Ser Ser Cys Thr Gly Ser Thr
            420                 425                 430

Glu Glu Ser Glu Met Leu Lys Arg Gln Arg Ser Leu Tyr Ile Asp Leu
        435                 440                 445

Gly Ala Leu Ala Gly Tyr Phe Asn Leu Val Arg Gly Pro Pro Thr Leu
    450                 455                 460

Glu Tyr Gly
465

<210> 61
<211> 4445
<212> DNA
<213> Homo sapiens

<400> 61
cgctccccgc tcccgtggct gccgccgccc cggggaagaa gagacagggg tggggtttgg      60

gggaagcgag agaggagggg agagaccctg gccaggctgg agcctggatt cgaggggagg     120

agggacggga ggaggagaaa ggtggaggag aagggagggg ggagcgggga ggagcggccg     180

ggcctggggc cttgaggccc ggggagagcc ggggagccgg gcccgcgcgc cgagatgttg     240

ctgctgctgt tactggcgcc actcttcctc cgcccccggg gcgcgggcgg ggcgcagacc     300

cccaacgcca cctcagaagg ttgccagatc atacacccgc cctgggaagg gggcatcagg     360

taccggggcc tgactcggga ccaggtgaag gctatcaact tcctgccagt ggactatgag     420

attgagtatg tgtgccgggg ggagcgcgag gtggtggggc ccaaggtccg caagtgcctg     480

gccaacggct cctggacaga tatggacaca cccagccgct gtgtccgaat ctgctccaag     540

tcttatttga ccctggaaaa tgggaaggtt ttcctgacgg gtggggacct cccagctctg     600

gacggagccc gggtggattt ccggtgtgac cccgacttcc atctggtggg cagctcccgg     660

agcatctgta gtcagggcca gtggagcacc cccaagcccc actgccaggt gaatcgaacg     720

ccacactcag aacggcgcgc agtgtacatc ggggcactgt ttcccatgag cgggggctgg     780

ccagggggcc aggcctgcca gcccgcggtg gagatggcgc tggaggacgt gaatagccgc     840

agggacatcc tgccggacta tgagctcaag ctcatccacc acgacagcaa gtgtgatcca     900

ggccaagcca ccaagtacct atatgagctg ctctacaacg accctatcaa gatcatcctt     960

atgcctggct gcagctctgt ctccacgctg gtggctgagg ctgctaggat gtggaacctc    1020

attgtgcttt cctatggctc cagctcacca gccctgtcaa accggcagcg tttccccact    1080

ttcttccgaa cgcacccatc agccacactc cacaacccta cccgcgtgaa actctttgaa    1140

aagtggggct ggaagaagat tgctaccatc cagcagacca ctgaggtctt cacttcgact    1200

ctggacgacc tggaggaacg agtgaaggag gctggaattg agattacttt ccgccagagt    1260

ttcttctcag atccagctgt gcccgtcaaa aacctgaagc gccaggatgc ccgaatcatc    1320

gtgggacttt tctatgagac tgaagcccgg aaagtttttt gtgaggtgta caaggagcgt    1380

ctctttggga agaagtacgt ctggttcctc attgggtggt atgctgacaa ttggttcaag 1440

atctacgacc cttctatcaa ctgcacagtg gatgagatga ctgaggcggt ggagggccac 1500

atcacaactg agattgtcat gctgaatcct gccaataccc gcagcatttc caacatgaca 1560

tcccaggaat ttgtggagaa actaaccaag cgactgaaaa gacaccctga ggagacagga 1620

ggcttccagg aggcaccgct ggcctatgat gccatctggg ccttggcact ggccctgaac 1680

aagacatctg gaggaggcgg ccgttctggt gtgcgcctgg aggacttcaa ctacaacaac 1740

cagaccatta ccgaccaaat ctaccgggca atgaactctt cgtcctttga gggtgtctct 1800

ggccatgtgg tgtttgatgc cagcggctct cggatggcat ggacgcttat cgagcagctt 1860

cagggtggca gctacaagaa gattggctac tatgacagca ccaaggatga tctttcctgg 1920

tccaaaacag ataaatggat tggagggtcc cccccagctg accagaccct ggtcatcaag 1980

acattccgct tcctgtcaca gaaactcttt atctccgtct cagttctctc cagcctgggc 2040

attgtcctag ctgttgtctg tctgtccttt aacatctaca actcacatgt ccgttatatc 2100

cagaactcac agcccaacct gaacaacctg actgctgtgg gctgctcact ggctttagct 2160

gctgtcttcc ccctggggct cgatggttac cacattggga ggaaccagtt tcctttcgtc 2220

tgccaggccc gcctctggct cctgggcctg ggctttagtc tgggctacgg ttccatgttc 2280

accaagattt ggtgggtcca cacggtcttc acaaagaagg aagaaaagaa ggagtggagg 2340

aagactctgg aaccctggaa gctgtatgcc acagtgggcc tgctggtggg catggatgtc 2400

ctcactctcg ccatctggca gatcgtggac cctctgcacc ggaccattga gacatttgcc 2460

aaggaggaac ctaaggaaga tattgacgtc tctattctgc cccagctgga gcattgcagc 2520

tccaggaaga tgaatacatg gcttggcatt ttctatggtt acaaggggct gctgctgctg 2580

ctgggaatct tccttgctta tgagaccaag agtgtgtcca ctgagaagat caatgatcac 2640

cgggctgtgg gcatggctat ctacaatgtg gcagtcctgt gcctcatcac tgctcctgtc 2700

accatgattc tgtccagcca gcaggatgca gcctttgcct ttgcctctct tgccatagtt 2760

ttctcctcct atatcactct tgttgtgctc tttgtgccca agatgcgcag gctgatcacc 2820

cgaggggaat ggcagtcgga ggcgcaggac accatgaaga cagggtcatc gaccaacaac 2880

aacgaggagg agaagtcccg gctgttggag aaggagaacc gtgaactgga aaagatcatt 2940

gctgagaaag aggagcgtgt ctctgaactg cgccatcaac tccagtctcg gcagcagctc 3000

cgctcccggc gccacccacc gacaccccca gaaccctctg ggggcctgcc caggggaccc 3060

cctgagcccc ccgaccggct tagctgtgat gggagtcgag tgcatttgct ttataagtga 3120

gggtagggtg agggaggaca ggccagtagg gggagggaaa gggagagggg aagggcaggg 3180

gactcaggaa gcagggggtc cccatcccca gctgggaaga acatgctatc caatctcatc 3240

tcttgtaaat acatgtcccc ctgtgagttc tgggctgatt tgggtctctc atacctctgg 3300

gaaacagacc tttttctctc ttactgcttc atgtaatttt gtatcacctc ttcacaattt 3360

agttcgtacc tggcttgaag ctgctcactg ctcacacgct gcctcctcag cagcctcact 3420

gcatctttct cttcccatgc aacaccctct tctagttacc acggcaaccc ctgcagctcc 3480

tctgcctttg tgctctgttc ctgtccagca ggggtctccc aacaagtgct ctttccaccc 3540

caaaggggcc tctccttttc tccactgtca taatctcttt ccatcttact tgcccttcta 3600

tactttctca catgtggctc cccctgaatt ttgcttcctt tgggagctca ttcttttcgc 3660

caaggctcac atgctccttg cctctgctct gtgcactcac gctcagcaca catgcatcct 3720

cccctctcct gcgtgtgccc actgaacatg ctcatgtgta cacacgcttt tcccgtatgc 3780

tttcttcatg ttcagtcaca tgtgctctcg ggtgccctgc attcacagct acgtgtgccc 3840

ctctcatggt catgggtctg cccttgagcg tgtttgggta ggcatgtgca atttgtctag 3900

catgctgagt catgtctttc ctatttgcac acgtccatgt ttatccatgt actttccctg 3960

tgtaccctcc atgtaccttg tgtactttct tcccttaaat catggtattc ttctgacaga 4020

gccatatgta ccctaccctg cacattgtta tgcacttttc cccaattcat gtttggtggg 4080

gccatccaca ccctctcctt gtcacagaat ctccatttct gctcagattc cccccatctc 4140

cattgcattc atgtactacc ctcagtctac actcacaatc atcttctccc aagactgctc 4200

ccttttgttt tgtgtttttt tgaggggaat taaggaaaaa taagtggggg caggtttgga 4260

gagctgcttc cagtggatag ttgatgagaa tcctgaccaa aggaaggcac ccttgactgt 4320

tgggatagac agatggacct atggggtggg aggtggtgtc cctttcacac tgtggtgtct 4380

cttggggaag gatctccccg aatctcaata aaccagtgaa cagtgtgact cggcaaaaaa 4440

aaaaa 4445

<210> 62
<211> 961
<212> PRT
<213> Homo sapiens

<400> 62
Met Leu Leu Leu Leu Leu Leu Ala Pro Leu Phe Leu Arg Pro Pro Gly
1               5                   10                  15

Ala Gly Gly Ala Gln Thr Pro Asn Ala Thr Ser Glu Gly Cys Gln Ile
            20                  25                  30

Ile His Pro Pro Trp Glu Gly Gly Ile Arg Tyr Arg Gly Leu Thr Arg
        35                  40                  45

Asp Gln Val Lys Ala Ile Asn Phe Leu Pro Val Asp Tyr Glu Ile Glu
    50                  55                  60

Tyr Val Cys Arg Gly Glu Arg Glu Val Val Gly Pro Lys Val Arg Lys
65                  70                  75                  80

Cys Leu Ala Asn Gly Ser Trp Thr Asp Met Asp Thr Pro Ser Arg Cys
                85                  90                  95

Val Arg Ile Cys Ser Lys Ser Tyr Leu Thr Leu Glu Asn Gly Lys Val
            100                 105                 110

Phe Leu Thr Gly Gly Asp Leu Pro Ala Leu Asp Gly Ala Arg Val Asp
        115                 120                 125

Phe Arg Cys Asp Pro Asp Phe His Leu Val Gly Ser Ser Arg Ser Ile
    130                 135                 140

Cys Ser Gln Gly Gln Trp Ser Thr Pro Lys Pro His Cys Gln Val Asn
145                 150                 155                 160

Arg Thr Pro His Ser Glu Arg Arg Ala Val Tyr Ile Gly Ala Leu Phe
                165                 170                 175

Pro Met Ser Gly Gly Trp Pro Gly Gly Gln Ala Cys Gln Pro Ala Val
            180                 185                 190

Glu Met Ala Leu Glu Asp Val Asn Ser Arg Arg Asp Ile Leu Pro Asp
        195                 200                 205

Tyr Glu Leu Lys Leu Ile His His Asp Ser Lys Cys Asp Pro Gly Gln
    210                 215                 220

Ala Thr Lys Tyr Leu Tyr Glu Leu Leu Tyr Asn Asp Pro Ile Lys Ile
225                 230                 235                 240

Ile Leu Met Pro Gly Cys Ser Ser Val Ser Thr Leu Val Ala Glu Ala
                245                 250                 255

Ala Arg Met Trp Asn Leu Ile Val Leu Ser Tyr Gly Ser Ser Ser Pro
            260                 265                 270

Ala Leu Ser Asn Arg Gln Arg Phe Pro Thr Phe Phe Arg Thr His Pro
        275                 280                 285

Ser Ala Thr Leu His Asn Pro Thr Arg Val Lys Leu Phe Glu Lys Trp
    290                 295                 300

Gly Trp Lys Lys Ile Ala Thr Ile Gln Gln Thr Thr Glu Val Phe Thr
305                 310                 315                 320

Ser Thr Leu Asp Asp Leu Glu Glu Arg Val Lys Glu Ala Gly Ile Glu
                325                 330                 335

Ile Thr Phe Arg Gln Ser Phe Phe Ser Asp Pro Ala Val Pro Val Lys
            340                 345                 350

Asn Leu Lys Arg Gln Asp Ala Arg Ile Ile Val Gly Leu Phe Tyr Glu
        355                 360                 365

Thr Glu Ala Arg Lys Val Phe Cys Glu Val Tyr Lys Glu Arg Leu Phe
    370                 375                 380

Gly Lys Lys Tyr Val Trp Phe Leu Ile Gly Trp Tyr Ala Asp Asn Trp
385                 390                 395                 400

Phe Lys Ile Tyr Asp Pro Ser Ile Asn Cys Thr Val Asp Glu Met Thr
                405                 410                 415

Glu Ala Val Glu Gly His Ile Thr Thr Glu Ile Val Met Leu Asn Pro
            420                 425                 430

Ala Asn Thr Arg Ser Ile Ser Asn Met Thr Ser Gln Glu Phe Val Glu
        435                 440                 445

Lys Leu Thr Lys Arg Leu Lys Arg His Pro Glu Glu Thr Gly Gly Phe
    450                 455                 460

Gln Glu Ala Pro Leu Ala Tyr Asp Ala Ile Trp Ala Leu Ala Leu Ala
465                 470                 475                 480

Leu Asn Lys Thr Ser Gly Gly Gly Gly Arg Ser Gly Val Arg Leu Glu
                485                 490                 495

Asp Phe Asn Tyr Asn Asn Gln Thr Ile Thr Asp Gln Ile Tyr Arg Ala
            500                 505                 510

Met Asn Ser Ser Ser Phe Glu Gly Val Ser Gly His Val Val Phe Asp
        515                 520                 525

Ala Ser Gly Ser Arg Met Ala Trp Thr Leu Ile Glu Gln Leu Gln Gly
    530                 535                 540

Gly Ser Tyr Lys Lys Ile Gly Tyr Tyr Asp Ser Thr Lys Asp Asp Leu
545                 550                 555                 560

Ser Trp Ser Lys Thr Asp Lys Trp Ile Gly Gly Ser Pro Pro Ala Asp
                565                 570                 575

Gln Thr Leu Val Ile Lys Thr Phe Arg Phe Leu Ser Gln Lys Leu Phe
            580                 585                 590

Ile Ser Val Ser Val Leu Ser Ser Leu Gly Ile Val Leu Ala Val Val
        595                 600                 605

Cys Leu Ser Phe Asn Ile Tyr Asn Ser His Val Arg Tyr Ile Gln Asn
    610                 615                 620

Ser Gln Pro Asn Leu Asn Asn Leu Thr Ala Val Gly Cys Ser Leu Ala
625                 630                 635                 640

Leu Ala Ala Val Phe Pro Leu Gly Leu Asp Gly Tyr His Ile Gly Arg
                645                 650                 655

Asn Gln Phe Pro Phe Val Cys Gln Ala Arg Leu Trp Leu Leu Gly Leu
            660                 665                 670

Gly Phe Ser Leu Gly Tyr Gly Ser Met Phe Thr Lys Ile Trp Trp Val
        675                 680                 685

His Thr Val Phe Thr Lys Lys Glu Glu Lys Lys Glu Trp Arg Lys Thr
    690                 695                 700

Leu Glu Pro Trp Lys Leu Tyr Ala Thr Val Gly Leu Leu Val Gly Met
705                 710                 715                 720

Asp Val Leu Thr Leu Ala Ile Trp Gln Ile Val Asp Pro Leu His Arg
                725                 730                 735

Thr Ile Glu Thr Phe Ala Lys Glu Glu Pro Lys Glu Asp Ile Asp Val
            740                 745                 750

Ser Ile Leu Pro Gln Leu Glu His Cys Ser Ser Arg Lys Met Asn Thr
        755                 760                 765

Trp Leu Gly Ile Phe Tyr Gly Tyr Lys Gly Leu Leu Leu Leu Gly

770                 775                 780

Ile Phe Leu Ala Tyr Glu Thr Lys Ser Val Ser Thr Glu Lys Ile Asn
785                 790                 795                 800

Asp His Arg Ala Val Gly Met Ala Ile Tyr Asn Val Ala Val Leu Cys
                805                 810                 815

Leu Ile Thr Ala Pro Val Thr Met Ile Leu Ser Ser Gln Gln Asp Ala
            820                 825                 830

Ala Phe Ala Phe Ala Ser Leu Ala Ile Val Phe Ser Ser Tyr Ile Thr
        835                 840                 845

Leu Val Val Leu Phe Val Pro Lys Met Arg Arg Leu Ile Thr Arg Gly
    850                 855                 860

Glu Trp Gln Ser Glu Ala Gln Asp Thr Met Lys Thr Gly Ser Ser Thr
865                 870                 875                 880

Asn Asn Asn Glu Glu Glu Lys Ser Arg Leu Leu Glu Lys Glu Asn Arg
                885                 890                 895

Glu Leu Glu Lys Ile Ile Ala Glu Lys Glu Glu Arg Val Ser Glu Leu
            900                 905                 910

Arg His Gln Leu Gln Ser Arg Gln Gln Leu Arg Ser Arg Arg His Pro
        915                 920                 925

Pro Thr Pro Pro Glu Pro Ser Gly Gly Leu Pro Arg Gly Pro Pro Glu
    930                 935                 940

Pro Pro Asp Arg Leu Ser Cys Asp Gly Ser Arg Val His Leu Leu Tyr
945                 950                 955                 960

Lys

<210> 63
<211> 1872
<212> DNA
<213> Homo sapiens

<400> 63
gtacttacac aaccccagat ggtacaacct acaacacacc taggctgtat gatgtagcca      60

attgctccta gactacaaat ctatacagca tgttgctgta ctgaatactc taagcaatta     120

taacacaatg acctagcaac agtaacataa gcaaaggaga gtcacgccca aaatggaaac 180

ccaaagccaa agtacccctt cagactctac atatgacttc tgagaatcaa gagaaagtga 240

aagctcttct ccgagacctg caagaacaag atgctgatgc tggatctgaa agaggccttt 300

ctggggagga ggaagatgat gagcctgatt gctgtaacga tgagcggtac tggccagctg 360

gacaggaacc ttccctcgtt cccgacttgg atcctttgga atatgctggc ttagcctcag 420

tggagcctta tgttccagaa tttacagtct ccccatttgc agtgcaaaaa ctttccaggt 480

atggtttcaa tactgaacgc tgtcaagcgg tcctgaggat gtgtgatgga gatgtgggag 540

catcactaga gcatctcctt acccagtgtt tttcagagac atttggagag aggatgaaga 600

tctctgaggc agtcaaccag ataagcttgg atgagtgtat ggaacagcga caggaagagg 660

catttgctct caagtccatc tgtggagaaa aatttataga aagaattcag aacagagtct 720

ggaccattgg gttagaactg gagtatctga caagtagatt ccgcaaatcc aagccaaaag 780

aaagtaccaa aaatgtacaa gagaattcac ttgaaatctg taaattttac ctcaaaggaa 840

attgtaaatt tggatcaaaa tgcagattca aacatgaagt gcccccaaat caaattgttg 900

gaagaataga aagaagtgta gatgattctc atcttaatgc tattgaagat gcatcttttt 960

tatatgaact tgaaattcga ttttctaaag accacaaata tccctaccaa gctccgctcg 1020

tggcatttta ttccaccaat gagaacctac ctctggcttg tcgtttacat atttctgagt 1080

ttctttatga caaggccttg acatttgcgg aaacttcgga acctgtcgta tattctttga 1140

taaccctttt agaggaagag tcggaaatag tcaagttact aacgaatacc caccacaagt 1200

atagtgaccc tcctgtgaac tttctgccag taccctctag gaccagaata aataatcctg 1260

cctgtcataa aacagtgatt ccaaataatt cttttgtttc taatcaaatt ccagaagttg 1320

aaaaagcatc agaatctgag gagtcagatg aggatgacgg tcctgcacct gttatagtag 1380

agaatgaaag ctatgtgaac cttaagaaaa agatttccaa aagatatgac tggcaggcaa 1440

agtcagtaca tgctgaaaat ggtaaaatct gcaagcagtt ccgaatgaaa caggtgctca 1500

atagccacat gtagccaggt gttgctgtat tggacagcac aggtatagaa catttccatc 1560

aacacggaaa gttctattgg acagtactgg tatagagact cttgttggga agaactggat 1620

atgtgctaag caagagggct ttatttcacc cagaaaatct ttcggtttat tcagatgttg 1680

gaaaactaat gtacagattc cacaactcat tctggctgat tctctgagca gcattctgaa 1740

taaagtcttc tacatccaat cccaacaaat ccaacaaagg atcactcaag gccaggagtg 1800

caagaccatc cagggcaaca taacaagact ctgtctctat aattaaaaaa aaaaaaaagc 1860

tgtaatccca aa 1872

<210> 64
<211> 433
<212> PRT
<213> Homo sapiens

<400> 64
Met Thr Ser Glu Asn Gln Glu Lys Val Lys Ala Leu Leu Arg Asp Leu
1               5                   10                  15

Gln Glu Gln Asp Ala Asp Ala Gly Ser Glu Arg Gly Leu Ser Gly Glu
            20                  25                  30

Glu Glu Asp Asp Glu Pro Asp Cys Cys Asn Asp Glu Arg Tyr Trp Pro
        35                  40                  45

Ala Gly Gln Glu Pro Ser Leu Val Pro Asp Leu Asp Pro Leu Glu Tyr
    50                  55                  60

Ala Gly Leu Ala Ser Val Glu Pro Tyr Val Pro Glu Phe Thr Val Ser
65                  70                  75                  80

Pro Phe Ala Val Gln Lys Leu Ser Arg Tyr Gly Phe Asn Thr Glu Arg
                85                  90                  95

Cys Gln Ala Val Leu Arg Met Cys Asp Gly Asp Val Gly Ala Ser Leu
            100                 105                 110

Glu His Leu Leu Thr Gln Cys Phe Ser Glu Thr Phe Gly Glu Arg Met
        115                 120                 125

Lys Ile Ser Glu Ala Val Asn Gln Ile Ser Leu Asp Glu Cys Met Glu
    130                 135                 140

Gln Arg Gln Glu Glu Ala Phe Ala Leu Lys Ser Ile Cys Gly Glu Lys
145                 150                 155                 160

Phe Ile Glu Arg Ile Gln Asn Arg Val Trp Thr Ile Gly Leu Glu Leu
                165                 170                 175

Glu Tyr Leu Thr Ser Arg Phe Arg Lys Ser Lys Pro Lys Glu Ser Thr
            180                 185                 190

Lys Asn Val Gln Glu Asn Ser Leu Glu Ile Cys Lys Phe Tyr Leu Lys
        195                 200                 205

Gly Asn Cys Lys Phe Gly Ser Lys Cys Arg Phe Lys His Glu Val Pro
    210                 215                 220

Pro Asn Gln Ile Val Gly Arg Ile Glu Arg Ser Val Asp Asp Ser His
225                 230                 235                 240

Leu Asn Ala Ile Glu Asp Ala Ser Phe Leu Tyr Glu Leu Glu Ile Arg
                245                 250                 255

Phe Ser Lys Asp His Lys Tyr Pro Tyr Gln Ala Pro Leu Val Ala Phe
            260                 265                 270

Tyr Ser Thr Asn Glu Asn Leu Pro Leu Ala Cys Arg Leu His Ile Ser
        275                 280                 285

Glu Phe Leu Tyr Asp Lys Ala Leu Thr Phe Ala Glu Thr Ser Glu Pro
    290                 295                 300

Val Val Tyr Ser Leu Ile Thr Leu Leu Glu Glu Glu Ser Glu Ile Val
305                 310                 315                 320

Lys Leu Leu Thr Asn Thr His His Lys Tyr Ser Asp Pro Pro Val Asn
                325                 330                 335

Phe Leu Pro Val Pro Ser Arg Thr Arg Ile Asn Asn Pro Ala Cys His
            340                 345                 350

Lys Thr Val Ile Pro Asn Asn Ser Phe Val Ser Asn Gln Ile Pro Glu
        355                 360                 365

Val Glu Lys Ala Ser Glu Ser Glu Glu Ser Asp Glu Asp Asp Gly Pro
    370                 375                 380

Ala Pro Val Ile Val Glu Asn Glu Ser Tyr Val Asn Leu Lys Lys Lys
385                 390                 395                 400

Ile Ser Lys Arg Tyr Asp Trp Gln Ala Lys Ser Val His Ala Glu Asn
                405                 410                 415

Gly Lys Ile Cys Lys Gln Phe Arg Met Lys Gln Val Leu Asn Ser His
            420                 425                 430

Met

<210> 65
<211> 2186
<212> DNA
<213> Homo sapiens

<400> 65
   agtaaggaga accagccagg cagaacatca cagcgggagg agctgtccca ggtggcccag 60
   ctcagcaatg gcaatggggg tccccagagt cattctgctc tgcctctttg gggctgcact 120
   ctgcctgaca gggtcccaag ccctgcagtg ctacagcttt gagcacacct actttggccc 180
   ctttgacctc agggccatga agctgcccag catctcctgt cctcatgagt gctttgaggc 240
   tatcctgtct ctggacaccg ggtatcgcgc gccggtgacc ctggtgcgga agggctgctg 300
   gaccgggcct cctgcgggcc agacgcaatc gaacgcggac gcgctgccgc cagactactc 360
   ggtggtgcgc ggctgcacaa ctgacaaatg caacgcccac ctcatgactc atgacgccct 420
   ccccaacctg agccaagcac ccgacccgcc gacgctcagc ggcgccgagt gctacgcctg 480
   tatcggggtc caccaggatg actgcgctat cggcaggtcc cgacgagtcc agtgtcacca 540
   ggaccagacc gcctgcttcc agggcaatgg cagaatgaca gttggcaatt tctcagtccc 600
   tgtgtacatc agaacctgcc accggccctc ctgcaccacc gagggcagca ccagcccctg 660
   gacagccatc gacctccagg gctcctgctg tgaggggtac ctctgcaaca ggaaatccat 720
   gacccagccc ttcaccagtg cttcagccac cacccctccc cgagcactac aggtcctggc 780
   cctgctcctc ccagtcctcc tgctggtggg gctctcagca tagaccgccc ctccaggatg 840
   ctggggacag ggctcacaca cctcattctt gctgcttcag cccctatcac atagctcact 900
   ggaaaatgat gttaaagtaa gaattgcact cctgtccctc tggccttcca tctctcccgc 960
   ccttgtgccc cacaacctgg ccaacagtac tggaagaaac tggacacagt caccagcatc 1020
   cccggggagg gcaaaacagc catgtcgtgc cctgatgaag agcaattctg atcacagctg 1080
   ttactcactg agcaccagcc aggcaccagg caccccataa cacggcttcc tgtgctctcc 1140
   ctccagagcc tgtcgcagct ctaggaggga gctatacaat gatgtcttta ttagtgtcat 1200
   catgagaagc ccaataagca gtatgcccta acagttagta ggccaggctc tggagctaag 1260
   ctgcatgggt tcaaatccca gctccaccat tcagcctgca gagaccatga gcgagttact 1320
   taagccaggc tctggagcta agctgcatgg gttcaaatcc cagctccagc attcagccta 1380
   cagagaccat gggtgagtta cttaagccag gctctggagc taagctgcat gggttcaaat 1440
   cccagctcca ccattcagcc tgcagagact gtgggtgagt tacttgagct ctctgtgcca 1500
   atattttctc acctataagg tggaggtgaa aataaactct ataacatgac aagaactact 1560
   tcacagtagt tgcagtgagg attcaacgag atgaacattt agtacttggg acacagcagt 1620
   ggcccagtgt aaatgggcta cttgtcataa gccctaagtc acaggtcaac aaactgagag 1680
   gcaaaagcac ttggttgagc ttgtgtatct agtgagtatg gattcaggga ccagattccc 1740
   agccccacga actgctaagc aaccccacct cctaaacaca tgagtgccga ttaacttcac 1800
   agaaaaacac acaaggcaaa gttcagcgag gtgaaattct ccaagctata aagatcaggg 1860
   aagacttcct ggaggaattc acccttgagc aaaatcctaa aggatcaata gtagctggca 1920
   aaaagaagca ggaggaagcg cattctaggt agaggagaca gcctggacaa aggtctgagg 1980
   gaggaaggag cacaaggagt gcaggacact ttcatgagtg caggacactt tcataactgc 2040
   atgaacttca tagagatggg atcctttagc atgttctctg tgcacatgct tgaccatgtt 2100
   ctttcacatg ctttttgcca cttgatcttt ccagcaactc agtgagagaa gcaaaaaagt 2160
   aagttgcatc caaaaaaaaa aaaaaa 2186

<210> 66
<211> 208
<212> PRT
<213> Homo sapiens

<400> 66
Met Lys Leu Pro Ser Ile Ser Cys Pro His Glu Cys Phe Glu Ala Ile
1               5                   10                  15

Leu Ser Leu Asp Thr Gly Tyr Arg Ala Pro Val Thr Leu Val Arg Lys
            20                  25                  30

Gly Cys Trp Thr Gly Pro Pro Ala Gly Gln Thr Gln Ser Asn Ala Asp
        35                  40                  45

Ala Leu Pro Pro Asp Tyr Ser Val Val Arg Gly Cys Thr Thr Asp Lys
    50                  55                  60

Cys Asn Ala His Leu Met Thr His Asp Ala Leu Pro Asn Leu Ser Gln
65                  70                  75                  80

Ala Pro Asp Pro Pro Thr Leu Ser Gly Ala Glu Cys Tyr Ala Cys Ile
                85                  90                  95

Gly Val His Gln Asp Asp Cys Ala Ile Gly Arg Ser Arg Arg Val Gln
            100                 105                 110

Cys His Gln Asp Gln Thr Ala Cys Phe Gln Gly Asn Gly Arg Met Thr
        115                 120                 125

Val Gly Asn Phe Ser Val Pro Val Tyr Ile Arg Thr Cys His Arg Pro
    130                 135                 140

Ser Cys Thr Thr Glu Gly Ser Thr Ser Pro Trp Thr Ala Ile Asp Leu
145                 150                 155                 160

Gln Gly Ser Cys Cys Glu Gly Tyr Leu Cys Asn Arg Lys Ser Met Thr
                165                 170                 175

Gln Pro Phe Thr Ser Ala Ser Ala Thr Thr Pro Pro Arg Ala Leu Gln
            180                 185                 190

Val Leu Ala Leu Leu Leu Pro Val Leu Leu Leu Val Gly Leu Ser Ala
        195                 200                 205

<210> 67
<211> 3143
<212> DNA
<213> Homo sapiens

<400> 67
ggcacgaggc ccagcactag aagtcggcgg tgtttccttt cggtgatcag cactgaacac      60

agaggactcg ccatggagtt tgggctgagc tgggttttcc tcgttgctct tttaagaggt     120

gtccagtgtc aggcgcagtt ggtggagtct gggggaggcg tggtccagcc cgggagctcc     180

ctgagactct cctgtgcagc ctctggtttc aggttcagca attatggcat gcactgggtc     240

cgccaggctc caggcaaggg gctggagtgg gtggcagttt tttcatatga tgaaagtgat     300

aaatattatg cagcctccgt gaagggtcga tttaccatct ccagagacaa ctccaagaac     360

acgttgtctc tgcaaatgaa cagcctgaga gttgaggaca cggctgttta ctactgtgcg     420

aaagatcaga agccctggta cagcaacagc tggtttctaa ccaactttga ctcttggggc     480

cggggcaccc tggtcaccgt ctcctcagcc tccaccaagg gcccatcggt cttccccctg     540

gcaccctcct ccaagagcac ctctgggggc acagcggccc tgggctgcct ggtcaaggac     600

tacttccccg aaccggtgac ggtgtcgtgg aactcaggcg ccctgaccag cggcgtgcac     660

accttcccgg ctgtcctaca gtcctcagga ctctactccc tcagcagcgt ggtgaccgtg     720

ccctccagca gcttgggcac ccagacctac atctgcaacg tgaatcacaa gcccagcaac     780

accaaggtgg acaagaaagt tgagcccaaa tcttgtgaca aaactcacac atgcccaccg     840

tgcccagcac ctgaactcct ggggggaccg tcagtcttcc tcttcccccc aaaacccaag     900

gacaccctca tgatctcccg gacccctgag gtcacatgcg tggtggtgga cgtgagccac     960

gaagaccctg aggtcaagtt caactggtac gtggacggcg tggaggtgca taatgccaag    1020

acaaagccgc gggaggagca gtacaacagc acgtaccgtg tggtcagcgt cctcaccgtc    1080

ctgcaccagg actggctgaa tggcaaggag tacaagtgca aggtctccaa caaagccctc    1140

ccagccccca tcgagaaaac catctccaaa gccaaagggc agccccgaga accacaggtg    1200

tacaccctgc ccccatcccg ggatgagctg accaagaacc aggtcagcct gacctgcctg    1260

gtcaaaggct tctatcccag cgacatcgcc gtggagtggg agagcaatgg gcagccggag 1320
aacaactaca agaccacgcc tcccgtgctg gactccgacg gctccttctt cctctacagc 1380
aagctcaccg tggacaagag caggtggcag caggggaacg tcttctcatg ctccgtgatg 1440
catgaggctc tgcacaacca ctacacgcag aagagcctct ccctgtctcc ggagctgcaa 1500
ctggaggaga gctgtgcgga ggcgcaggac ggggagctgg acgggctgtg gacgaccatc 1560
accatcttca tcacactctt cctgttaagc gtgtgctaca gtgccaccgt caccttcttc 1620
aaggtgaagt ggatcttctc ctcggtggtg gacctgaagc agaccatcat ccccgactac 1680
aggaacatga tcggacaggg ggcctagggc caccctctgc ggggtgtcca gggccgccca 1740
gaccccacac accagccatg ggccatgctc agccaccacc caggccacac ctgcccccga 1800
cctcaccgcc ctcaacccca tggctctctg gcctcgcagt tgccctctga ccctgacaca 1860
cctgacacgc cccccttcca gaccctgtgc atagcaggtc taccccagac ctccgctgct 1920
tggtgcatgc agggcactgg gggccaggtg tcccctcagc aggacatcct tgccctccgg 1980
accacaaggt gctcacacaa aaggaggcag tgaccggtat cccaggcccc cacccaggca 2040
ggagctggcc ctggagccaa ccccgtccac gccagcctcc tgaacacagg cgtggtttcc 2100
agatggtgag tgggagcatc agccgccaag gtagggaagc cacagcacca tcaggccctg 2160
ttggggaggc ttccgagagc tgcgaaggct cactcagacg gccttcctcc cagcccgcag 2220
ccagccagcc tccattccgg gcactcccgt gaactcctga catgaggaat gaggttgttc 2280
tgatttcaag caaagaacgc tgctctctgg ctcctgggaa cagtctcggt gccagcacca 2340
ccccttggct gcctgcccac actgctggat tctcgggtgg aactggaccc gcagggacag 2400
ccagccccag agtccgcact ggggagagaa ggggccaggc ccaggacact gccacctccc 2460
acccactcca gtccaccgag atcactcaga gaagagcctg ggccatgtgg ccgctgcagg 2520
agccccacag tgcaagggtg aggatagccc aaggaagggc tgggcatctg cccagacagg 2580
cctcccagag aaggctggtg accaggtccc aggcgggcaa gactcagcct tggtggggcc 2640
tgaggacaga ggaggcccag gagcatcggg gagagaggtg gagggacacc gggagagcca 2700
ggagcgtgga cacagccaga actcatcaca gaggctggcg tccagtcccg ggtcacgtgc 2760
agcaggaaca agcagccact ctgggggcac caggtggaga ggcaagacga caaagagggt 2820
gcccgtgttc ttgcgaaagc ggggctgctg gccacgagtg ctggacagag gcccccacgc 2880
tctgctgccc ccatcacgcc gttccgtgac tgtcacgcag aatccgcaga caggaaggga 2940
gactcgagcg ggagtgcggc cagcgcctgc ctcggccgtc agggaggact cccgggctca 3000
ctcgaaggag gtgccaccat ttcagctttg gtagcttttc ttcttctttt aaattttcta 3060
aagctcatta attgtctttg atgtttcttt tgtgatgaca ataaaatatc ctttttaagt 3120

cttgtaaaaa aaaaaaaaaa aaa                                              3143

<210> 68
<211> 544
<212> PRT
<213> Homo sapiens

<400> 68
Met Glu Phe Gly Leu Ser Trp Val Phe Leu Val Ala Leu Leu Arg Gly
1               5                   10                  15

Val Gln Cys Gln Ala Gln Leu Val Glu Ser Gly Gly Gly Val Val Gln
            20                  25                  30

Pro Gly Ser Ser Leu Arg Leu Ser Cys Ala Ala Ser Gly Phe Arg Phe
        35                  40                  45

Ser Asn Tyr Gly Met His Trp Val Arg Gln Ala Pro Gly Lys Gly Leu
    50                  55                  60

Glu Trp Val Ala Val Phe Ser Tyr Asp Glu Ser Asp Lys Tyr Tyr Ala
65                  70                  75                  80

Ala Ser Val Lys Gly Arg Phe Thr Ile Ser Arg Asp Asn Ser Lys Asn
                85                  90                  95

Thr Leu Ser Leu Gln Met Asn Ser Leu Arg Val Glu Asp Thr Ala Val
            100                 105                 110

Tyr Tyr Cys Ala Lys Asp Gln Lys Pro Trp Tyr Ser Asn Ser Trp Phe
        115                 120                 125

Leu Thr Asn Phe Asp Ser Trp Gly Arg Gly Thr Leu Val Thr Val Ser
    130                 135                 140

Ser Ala Ser Thr Lys Gly Pro Ser Val Phe Pro Leu Ala Pro Ser Ser
145                 150                 155                 160

Lys Ser Thr Ser Gly Gly Thr Ala Ala Leu Gly Cys Leu Val Lys Asp
                165                 170                 175

Tyr Phe Pro Glu Pro Val Thr Val Ser Trp Asn Ser Gly Ala Leu Thr
            180                 185                 190

Ser Gly Val His Thr Phe Pro Ala Val Leu Gln Ser Ser Gly Leu Tyr
        195                 200                 205

Ser Leu Ser Ser Val Val Thr Val Pro Ser Ser Ser Leu Gly Thr Gln
    210                 215                 220

Thr Tyr Ile Cys Asn Val Asn His Lys Pro Ser Asn Thr Lys Val Asp
225                 230                 235                 240

Lys Lys Val Glu Pro Lys Ser Cys Asp Lys Thr His Thr Cys Pro Pro
                245                 250                 255

Cys Pro Ala Pro Glu Leu Leu Gly Gly Pro Ser Val Phe Leu Phe Pro
            260                 265                 270

Pro Lys Pro Lys Asp Thr Leu Met Ile Ser Arg Thr Pro Glu Val Thr
        275                 280                 285

Cys Val Val Val Asp Val Ser His Glu Asp Pro Glu Val Lys Phe Asn
    290                 295                 300

Trp Tyr Val Asp Gly Val Glu Val His Asn Ala Lys Thr Lys Pro Arg
305                 310                 315                 320

Glu Glu Gln Tyr Asn Ser Thr Tyr Arg Val Val Ser Val Leu Thr Val
                325                 330                 335

Leu His Gln Asp Trp Leu Asn Gly Lys Glu Tyr Lys Cys Lys Val Ser
            340                 345                 350

Asn Lys Ala Leu Pro Ala Pro Ile Glu Lys Thr Ile Ser Lys Ala Lys
        355                 360                 365

Gly Gln Pro Arg Glu Pro Gln Val Tyr Thr Leu Pro Pro Ser Arg Asp
    370                 375                 380

Glu Leu Thr Lys Asn Gln Val Ser Leu Thr Cys Leu Val Lys Gly Phe
385                 390                 395                 400

Tyr Pro Ser Asp Ile Ala Val Glu Trp Glu Ser Asn Gly Gln Pro Glu
                405                 410                 415

Asn Asn Tyr Lys Thr Thr Pro Pro Val Leu Asp Ser Asp Gly Ser Phe
            420                 425                 430

Phe Leu Tyr Ser Lys Leu Thr Val Asp Lys Ser Arg Trp Gln Gln Gly
        435                 440                 445

Asn Val Phe Ser Cys Ser Val Met His Glu Ala Leu His Asn His Tyr
    450                 455                 460

Thr Gln Lys Ser Leu Ser Leu Ser Pro Glu Leu Gln Leu Glu Glu Ser
465                 470                 475                 480

Cys Ala Glu Ala Gln Asp Gly Glu Leu Asp Gly Leu Trp Thr Thr Ile
                485                 490                 495

Thr Ile Phe Ile Thr Leu Phe Leu Leu Ser Val Cys Tyr Ser Ala Thr
            500                 505                 510

Val Thr Phe Phe Lys Val Lys Trp Ile Phe Ser Ser Val Val Asp Leu
        515                 520                 525

Lys Gln Thr Ile Ile Pro Asp Tyr Arg Asn Met Ile Gly Gln Gly Ala
    530                 535                 540

<210> 69
<211> 1124
<212> DNA
<213> Homo sapiens

<400> 69
tccgcaggcg gaccgggggc aaaggaggtg gcatgtcggt caggcacagc agggtcctgt      60

gtccgcgctg agccgcgctc tccctgctcc agcaaggacc atgagggcgc tggaggggcc     120

aggcctgtcg ctgctgtgcc tggtgttggc gctgcctgcc ctgctgccgg tgccggctgt     180

acgcggagtg gcagaaacac ccacctaccc ctggcgggac gcagagacag gggagcggct     240

ggtgtgcgcc cagtgccccc caggcacctt tgtgcagcgg ccgtgccgcc gagacagccc     300

cacgacgtgt ggcccgtgtc caccgcgcca ctacacgcag ttctggaact acctggagcg     360

ctgccgctac tgcaacgtcc tctgcgggga gcgtgaggag gaggcacggg cttgccacgc     420

cacccacaac cgtgcctgcc gctgccgcac cggcttcttc gcgcacgctg gtttctgctt     480

ggagcacgca tcgtgtccac ctggtgccgg cgtgattgcc ccgggcaccc ccagccagaa     540

cacgcagtgc cagccgtgcc ccccaggcac cttctcagcc agcagctcca gctcagagca     600

gtgccagccc caccgcaact gcacggccct gggcctggcc ctcaatgtgc caggctcttc     660

ctcccatgac accctgtgca ccagctgcac tggcttcccc ctcagcacca gggtaccagg     720

agctgaggag tgtgagcgtg ccgtcatcga ctttgtggct ttccaggaca tctccatcaa     780

gaggctgcag cggctgctgc aggccctcga ggccccggag ggctggggtc cgacaccaag     840

ggcgggccgc gcggccttgc agctgaagct gcgtcggcgg ctcacggagc tcctgggggc     900

gcaggacggg gcgctgctgg tgcggctgct gcaggcgctg cgcgtggcca ggatgcccgg     960

gctggagcgg agcgtccgtg agcgcttcct ccctgtgcac tgatcctggc cccctcttat   1020

ttattctaca tccttggcac cccacttgca ctgaaagagg ctttttttta aatagaagaa   1080

atgaggtttc ttaaagctta tttttataaa gctttttcat aaaa                    1124

<210> 70
<211> 300
<212> PRT
<213> Homo sapiens

<400> 70
Met Arg Ala Leu Glu Gly Pro Gly Leu Ser Leu Leu Cys Leu Val Leu
1               5                   10                  15

Ala Leu Pro Ala Leu Leu Pro Val Pro Ala Val Arg Gly Val Ala Glu
            20                  25                  30

Thr Pro Thr Tyr Pro Trp Arg Asp Ala Glu Thr Gly Glu Arg Leu Val
        35                  40                  45

Cys Ala Gln Cys Pro Pro Gly Thr Phe Val Gln Arg Pro Cys Arg Arg
    50                  55                  60

Asp Ser Pro Thr Thr Cys Gly Pro Cys Pro Pro Arg His Tyr Thr Gln
65                  70                  75                  80

Phe Trp Asn Tyr Leu Glu Arg Cys Arg Tyr Cys Asn Val Leu Cys Gly
                85                  90                  95

Glu Arg Glu Glu Glu Ala Arg Ala Cys His Ala Thr His Asn Arg Ala
            100                 105                 110

Cys Arg Cys Arg Thr Gly Phe Phe Ala His Ala Gly Phe Cys Leu Glu
        115                 120                 125

His Ala Ser Cys Pro Pro Gly Ala Gly Val Ile Ala Pro Gly Thr Pro
    130                 135                 140

Ser Gln Asn Thr Gln Cys Gln Pro Cys Pro Pro Gly Thr Phe Ser Ala
145                 150                 155                 160

Ser Ser Ser Ser Ser Glu Gln Cys Gln Pro His Arg Asn Cys Thr Ala
                165                 170                 175

Leu Gly Leu Ala Leu Asn Val Pro Gly Ser Ser Ser His Asp Thr Leu
            180                 185                 190

Cys Thr Ser Cys Thr Gly Phe Pro Leu Ser Thr Arg Val Pro Gly Ala
        195                 200                 205

Glu Glu Cys Glu Arg Ala Val Ile Asp Phe Val Ala Phe Gln Asp Ile
    210                 215                 220

Ser Ile Lys Arg Leu Gln Arg Leu Leu Gln Ala Leu Glu Ala Pro Glu
225                 230                 235                 240

Gly Trp Gly Pro Thr Pro Arg Ala Gly Arg Ala Ala Leu Gln Leu Lys
                245                 250                 255

Leu Arg Arg Arg Leu Thr Glu Leu Leu Gly Ala Gln Asp Gly Ala Leu
            260                 265                 270

Leu Val Arg Leu Leu Gln Ala Leu Arg Val Ala Arg Met Pro Gly Leu
        275                 280                 285

Glu Arg Ser Val Arg Glu Arg Phe Leu Pro Val His
    290                 295                 300

<210> 71
<211> 716
<212> DNA
<213> Homo sapiens

<400> 71
aggctcagta taaatagcag ccaccgctcc ctggcaggca gggacccgca gctcagctac      60

agcacagatc aggtgaggag cacaccaagg agtgattttt aaaacttact ctgttttctc     120

tttcccaaca agattatcat ttcctttaaa aaaaatagtt atcctggggc atacagccat     180

accattctga aggtgtctta tctcctctga tctagagagc accatgaagc ttctcacggg     240

cctggttttc tgctccttgg tcctgggtgt cagcagccga agcttctttt cgttccttgg     300

cgaggctttt gatggggctc gggacatgtg gagagcctac tctgacatga gagaagccaa     360

ttacatcggc tcagacaaat acttccatgc tcgggggaac tatgatgctg ccaaaagggg     420

acctgggggt gcctgggctg cagaagtgat cagcgatgcc agagagaata tccagagatt     480

ctttggccat ggtgcggagg actcgctggc tgatcaggct gccaatgaat ggggcaggag     540

tggcaaagac cccaatcact tccgacctgc tggcctgcct gagaaatact gagcttcctc     600

ttcactctgc tctcaggaga tctggctgtg aggccctcag ggcagggata caaagcgggg     660

agagggtaca caatgggtat ctaataaata cttaagaggt ggaatttgtg gaaact         716

<210> 72

<211> 122
<212> PRT
<213> Homo sapiens

<400> 72
Met Lys Leu Leu Thr Gly Leu Val Phe Cys Ser Leu Val Leu Gly Val
1               5                   10                  15

Ser Ser Arg Ser Phe Phe Ser Phe Leu Gly Glu Ala Phe Asp Gly Ala
            20                  25                  30

Arg Asp Met Trp Arg Ala Tyr Ser Asp Met Arg Glu Ala Asn Tyr Ile
        35                  40                  45

Gly Ser Asp Lys Tyr Phe His Ala Arg Gly Asn Tyr Asp Ala Ala Lys
    50                  55                  60

Arg Gly Pro Gly Gly Ala Trp Ala Ala Glu Val Ile Ser Asp Ala Arg
65                  70                  75                  80

Glu Asn Ile Gln Arg Phe Phe Gly His Gly Ala Glu Asp Ser Leu Ala
                85                  90                  95

Asp Gln Ala Ala Asn Glu Trp Gly Arg Ser Gly Lys Asp Pro Asn His
            100                 105                 110

Phe Arg Pro Ala Gly Leu Pro Glu Lys Tyr
        115                 120


<210> 73
<211> 1100
<212> DNA
<213> Homo sapiens

<400> 73
ctaaagctgg gttggtagct cctacctact gtgtggcaag aaggtatggg tcatgaacag      60

aaccaaggag ctgcgctgct acagatgtta ccacttctgt ggctgctacc ccactcctgg     120

gccgtccctg aagctcctac tccaatgtgg ccagatgacc tgcaaaacca cacattcctg     180

cacacagtgt actgccagga tgggagtccc agtgtgggac tctctgaggc ctacgacgag     240

gaccagcttt tcttcttcga cttttcccag aacactcggg tgcctcgcct gcccgaattt     300

gctgactggg ctcaggaaca gggagatgct cctgccattt tatttgacaa agagttctgc     360

gagtggatga tccagcaaat agggccaaaa cttgatggga aaatcccggt gtccagaggg     420

tttcctatcg ctgaagtgtt cacgctgaag cccctggagt ttggcaagcc caacactttg     480

gtctgttttg tcagtaatct cttcccaccc atgctgacag tgaactggca gcatcattcc     540

gtccctgtgg aaggatttgg gcctactttt gtctcagctg tcgatggact cagcttccag 600

gccttttctt acttaaactt cacaccagaa ccttctgaca ttttctcctg cattgtgact 660

cacgaaattg accgctacac agcaattgcc tattgggtac cccggaacgc actgccctca 720

gatctgctgg agaatgtgct gtgtggcgtg gcctttggcc tgggtgtgct gggcatcatc 780

gtgggcattg ttctcatcat ctacttccgg aagccttgct caggtgactg attcttccag 840

accagagttt gatgccagca gcttcggcca tccaaacaga ggatgctcag atttctcaca 900

tcctgcccag gatctcctct tagggtagaa gaagtctctg ggacatccct ggggtgtgtg 960

tgtagatttc ccacctgggg actctgctgt ccctgggctt gcatcccagg gatcccagag 1020

tggcctgcct atcacaacca catcccttcc ccccacaagg caataaatct catttcttta 1080

aaaaaaaaaa aaaaaaaaaa 1100

<210> 74
<211> 261
<212> PRT
<213> Homo sapiens

<400> 74
Met Gly His Glu Gln Asn Gln Gly Ala Ala Leu Leu Gln Met Leu Pro
1               5                   10                  15

Leu Leu Trp Leu Leu Pro His Ser Trp Ala Val Pro Glu Ala Pro Thr
            20                  25                  30

Pro Met Trp Pro Asp Asp Leu Gln Asn His Thr Phe Leu His Thr Val
        35                  40                  45

Tyr Cys Gln Asp Gly Ser Pro Ser Val Gly Leu Ser Glu Ala Tyr Asp
    50                  55                  60

Glu Asp Gln Leu Phe Phe Phe Asp Phe Ser Gln Asn Thr Arg Val Pro
65                  70                  75                  80

Arg Leu Pro Glu Phe Ala Asp Trp Ala Gln Glu Gln Gly Asp Ala Pro
                85                  90                  95

Ala Ile Leu Phe Asp Lys Glu Phe Cys Glu Trp Met Ile Gln Gln Ile
            100                 105                 110

Gly Pro Lys Leu Asp Gly Lys Ile Pro Val Ser Arg Gly Phe Pro Ile
        115                 120                 125

Ala Glu Val Phe Thr Leu Lys Pro Leu Glu Phe Gly Lys Pro Asn Thr
    130                 135                 140

Leu Val Cys Phe Val Ser Asn Leu Phe Pro Pro Met Leu Thr Val Asn
145                 150                 155                 160

Trp Gln His His Ser Val Pro Val Glu Gly Phe Gly Pro Thr Phe Val
                165                 170                 175

Ser Ala Val Asp Gly Leu Ser Phe Gln Ala Phe Ser Tyr Leu Asn Phe
            180                 185                 190

Thr Pro Glu Pro Ser Asp Ile Phe Ser Cys Ile Val Thr His Glu Ile
        195                 200                 205

Asp Arg Tyr Thr Ala Ile Ala Tyr Trp Val Pro Arg Asn Ala Leu Pro
    210                 215                 220

Ser Asp Leu Leu Glu Asn Val Leu Cys Gly Val Ala Phe Gly Leu Gly
225                 230                 235                 240

Val Leu Gly Ile Ile Val Gly Ile Val Leu Ile Ile Tyr Phe Arg Lys
                245                 250                 255

Pro Cys Ser Gly Asp
            260

<210> 75
<211> 4927
<212> DNA
<213> Homo sapiens

<400> 75
ggcaggtgca gctgccacag tgagacgggc accccgaccc gggcatggag gggggcaagg      60

ggcccaggct cagagacttc ctgagtggga gtctggctac ctgggcgctg ggactggccg     120

ggctggtcgg ggaggcggag gactcggagg gggaagaaga ggaagaggag gaagagccgc     180

ccctttggtt ggagaagaga ttcctgcgcc tcagcgatgg ggccctgctc ctccgggtgc     240

tgggcatcat tgcccccagc tcccgagggg gacctcggat gctcagaggc cttgacggac     300

ctgctgcctg gcgagtgtgg aacctgaacc acctgtgggg ccgactgagg gacttctacc     360

aggaggagct gcagctgctg atcctgtcgc cacccccaga cctccagaca ttgggatttg     420

accctctctc agaagaagcg gtggagcagc tggaaggcgt tcttcggcta ctgttgggag     480

cgtcagtaca gtgtgagcac cgggaactct tcatccgcca catccagggc ctcagtctcg     540

aggtccagag cgagctggcc gctgccatcc aggaggtgac ccagccgggg gccggcgtgg     600

tgctggcact gtctgggcca gatcctgggg agctggcacc tgccgagctg gagatgctgt     660

cccggagcct gatggggaca ctgtcgaagc tggcacggga gcgtgacctg ggggcccagc 720
ggctggctga actgctgctg gagcgagaac ccctctgctt gaggcctgag gctccctcta 780
gggctcccgc cgagggcccc tcgcaccatc tggccctgca gctggccaac gccaaggctc 840
agctgcggcg tctgcggcag gagctggagg agaaggccga gctgctgcta gactcccagg 900
ccgaggtgca gggtttggag gccgaaataa gaaggctccg ccaggaggcc caggcgctgt 960
cgggacaggc caagcgggcc gagctgtacc gcgaggaggc agaggcgctg cgggagcggg 1020
ccggccgcct gccccgcctg caggaggagc tgaggcgctg ccgcgagcgg ctgcaggcgg 1080
ctgaggccta caagagtcag ctggaggagg agcgggtgct ctcgggggtg ctggaggcgt 1140
ccaaggcgct gctggaagag cagctggagg ctgcccgaga gcgctgcgcc cggctgcacg 1200
agacccagcg cgagaacctg ctgctgcgaa cccggctggg cgaggcccat gcggagctgg 1260
actctctgcg gcatcaggtg gaccagctgg ctgaggagaa tgtggagctg gagctggagc 1320
ttcagcggag cttggagcca cctccaggat cccctgggga ggcaccccta gcaggagcgg 1380
ccccctcgct gcaagatgag gtgagggagg cagaggctgg gcggcttcgg acccttgaga 1440
gggagaaccg ggagcttcgg gggctgcttc aggtgcttca ggggcagcca gggggccagc 1500
accccctgct ggaggcaccg agagaggacc ctgttcttcc agtgctggag gaggctcccc 1560
agactcctgt ggccttcgac cacagccctc agggcttggt tcagaaggca agggatggag 1620
gcccccaggc cttggacttg gctcccccgg cattagactc agtgctcgag gcatcagctg 1680
agtgtcccca ggcacctgat tcagacccac aggaggcaga gagtcccctt caggcagctg 1740
ccatggaccc ccaggcctca gactggtccc cgcaagagtc aggctctcct gtggagacac 1800
aggagtcccc ggagaaggct ggccgtagat cctctctcca gagccctgcc tctgtggccc 1860
cacctcaggg tccagggacc aaaattcagg ccccgcagtt gctgggagga gagacagagg 1920
gaagagaggc tccccaaggc gagttggtgc ctgaggcctg ggggttgaga caggaggccc 1980
ctgagcacaa gccagggcct tcggagccca gctctgtgca gctggaggag caggaggccc 2040
caaaccaggg cctggacctg gccacgggac aagcagaggc cagagagcat gaccagaggc 2100
tggaagggac ggtcagggac ccagcctggc aaaaaccaca gcagaagtca gaaggggctc 2160
ttgaggtcca ggtctgggaa ggcccaatcc caggggagag cctggccagt ggtgtcgcag 2220
agcaggaggc cctcagggag gaggtggcac agttgaggag aaaggctgag gcccttggag 2280
atgagctgga agcccaggcc cgcaagctgg aggcccaaaa cacggaggct gcccgcctct 2340
ccaaggagct ggcccaagcg cgaagggcag aggccgaggc ccaccgggag gcagaggccc 2400
aggcctggga gcaagcccgg ctgcgggagg cagtggaggc tgctggccag gagctggagt 2460

ctgcgtccca ggaacgggag gcgctggtgg aggcgctggc agcagcgggc cgggagcgga 2520

ggcagtggga gcgtgagggg tccaggctgc gggcccagtc ggaggccgcc gaggaacgga 2580

tgcaggtgct ggagagcgag ggccgccagc acttggagga ggctgagagg gagcgccggg 2640

agaaggaggc cctccaggcg gagctggaga aagctgtggt gcggggcaag gagttggggg 2700

cccggctgga gcatttgcag cgtgagctgg agcaggcggc tctcgagcgc caggaatttc 2760

tgcgagaaaa ggaaagccag caccagaggt accagggctt ggagcagcgg ctggaagctg 2820

agctgcaggc ggcggcgacc agcaaggagg aggcgctgat ggagctcaag accagggccc 2880

tgcagctgga agaggagctg ttccagctgc gccagggccc cgcggggctg ggcccaaaaa 2940

agcgtgcgga gcctcagctg gtggagaccc agaatgtgcg gcttattgag gtggagcgca 3000

gtaatgcgat gctggtggca gagaaggcag ctttgcaggg gcagctgcag cacctggagg 3060

ggcagctggg gagcctgcag ggccgtgccc aggagctgct gctgcagagc cagcgggcgc 3120

aggagcacag cagccgcctg caggccgaga agtctgtgct ggagattcag ggccaggagc 3180

tgcaccggaa gctggaggtg ctggaggagg aggtgcgggc ggcacggcag tcccaggagg 3240

agacccgcgg gcagcagcag gccctgcttc gggaccacaa ggccctggca cagctgcagc 3300

ggcggcagga ggccgagcta gagggactgc tggtgcggca ccgagacctc aaggccaaca 3360

tgcgggcact ggagctggcc caccgggagc tgcagggccg gcacgagcag ctgcaggccc 3420

agcgggccag cgtggaggca caggaggtgg ccctgctggc agagcgtgaa cgcctgatgc 3480

aagatgggca tcggcagcgg ggcctggagg aggagctgcg gaggcttcag agcgagcacg 3540

acagggctca gatgctgctg gcagagttgt ctcgggagcg gggtgagctg cagggtgaac 3600

gcggggagct acggggccgg ctggcgcggc tggagctgga gcgggcacag ctggagatgc 3660

agagccagca gctgcgcgag tccaaccagc agctggacct gagcgcctgc cggctgacca 3720

cgcagtgtga gctattgaca cagctgcgaa gtgcccagga agaggagaac cggcagctgc 3780

tggctgaagt tcaggccctg agccgggaga acagggagct cctggagcgc agcctggaga 3840

gtcgggacca cctgcaccgc gaacagcggg agtacctgga ccagcttaat gccctgcgcc 3900

gcgagaagca gaagctcgtg gagaagatca tggaccaata ccgcgtgctg gagcctgtgc 3960

ccctgccccg gaccaagaag ggcagctggc tggcagacaa ggtgaagagg ctgatgcggc 4020

cccggcggga gggggccccc cctgggggcc tgcgcctggg ggccgatggg gctggcagca 4080

ccgagagcct gggggccccc ccggagacgg agcttcctga gggcagggag gcagatggga 4140

cagggtcccc ttccccggca cccatgcgcc gggcccagag ctccctctgc ctgcgggatg 4200

agaccttggc aggcgggcag cggcggaaac tcagctcaag gttcccggtg gggcgaagct 4260

ctgagtcatt cagccctggg gacaccccta ggcaacgatt ccgacagcgc catccaggcc 4320

ccctgggggc gcccgtctcc cacagcaaag gacctggtgt gggatgggag aactccgctg 4380

agaccctgca ggaacacgaa acagatgcca accgagaggg ccctgaggta caggaaccgg 4440

agaaacgtcc cctcacccca tccctcagcc agtgacaccg tgggaacagc aggcttggga 4500

gtgcagcctt ctcggcactg gagtgtcagc ggaggcccca ggcagcccaa gagctcaggg 4560

agccagggac cccaagggga gtccttggac aaggaggcct gggccctgag atcctccacg 4620

gtcagcgccg gggcccggag atggagctgg gacgagtgtg tggacagggg ggatggctgg 4680

cccccacgag cagctccagg ctggagttct ggttcttcca ggtggctccc gctgaggcag 4740

cggtctctgg gggatccccc agctgaagga ggctggcagg agttggcaag agaaccccct 4800

gccctgtcca ggtgggaagc tgagtcccag tgctggggga ctgtggcctg ggctgatctt 4860

gagccttaac tggacatgag gggcatgaga ataaagctga actgcagcct cctgaaaaaa 4920

aaaaaaa 4927

<210> 76
<211> 1476
<212> PRT
<213> Homo sapiens

<400> 76
Met Glu Gly Gly Lys Gly Pro Arg Leu Arg Asp Phe Leu Ser Gly Ser
1               5                   10                  15

Leu Ala Thr Trp Ala Leu Gly Leu Ala Gly Leu Val Gly Glu Ala Glu
            20                  25                  30

Asp Ser Glu Gly Glu Glu Glu Glu Glu Glu Glu Glu Pro Pro Leu Trp
        35                  40                  45

Leu Glu Lys Arg Phe Leu Arg Leu Ser Asp Gly Ala Leu Leu Leu Arg
    50                  55                  60

Val Leu Gly Ile Ile Ala Pro Ser Ser Arg Gly Gly Pro Arg Met Leu
65                  70                  75                  80

Arg Gly Leu Asp Gly Pro Ala Ala Trp Arg Val Trp Asn Leu Asn His
                85                  90                  95

Leu Trp Gly Arg Leu Arg Asp Phe Tyr Gln Glu Glu Leu Gln Leu Leu
            100                 105                 110

Ile Leu Ser Pro Pro Pro Asp Leu Gln Thr Leu Gly Phe Asp Pro Leu
        115                 120                 125

Ser Glu Glu Ala Val Glu Gln Leu Glu Gly Val Leu Arg Leu Leu Leu
    130                 135                 140

Gly Ala Ser Val Gln Cys Glu His Arg Glu Leu Phe Ile Arg His Ile
145                 150                 155                 160

Gln Gly Leu Ser Leu Glu Val Gln Ser Glu Leu Ala Ala Ala Ile Gln
                165                 170                 175

Glu Val Thr Gln Pro Gly Ala Gly Val Val Leu Ala Leu Ser Gly Pro
            180                 185                 190

Asp Pro Gly Glu Leu Ala Pro Ala Glu Leu Glu Met Leu Ser Arg Ser
        195                 200                 205

Leu Met Gly Thr Leu Ser Lys Leu Ala Arg Glu Arg Asp Leu Gly Ala
    210                 215                 220

Gln Arg Leu Ala Glu Leu Leu Leu Glu Arg Glu Pro Leu Cys Leu Arg
225                 230                 235                 240

Pro Glu Ala Pro Ser Arg Ala Pro Ala Glu Gly Pro Ser His His Leu
                245                 250                 255

Ala Leu Gln Leu Ala Asn Ala Lys Ala Gln Leu Arg Arg Leu Arg Gln
            260                 265                 270

Glu Leu Glu Glu Lys Ala Glu Leu Leu Leu Asp Ser Gln Ala Glu Val
        275                 280                 285

Gln Gly Leu Glu Ala Glu Ile Arg Arg Leu Arg Gln Glu Ala Gln Ala
    290                 295                 300

Leu Ser Gly Gln Ala Lys Arg Ala Glu Leu Tyr Arg Glu Glu Ala Glu
305                 310                 315                 320

Ala Leu Arg Glu Arg Ala Gly Arg Leu Pro Arg Leu Gln Glu Glu Leu
                325                 330                 335

Arg Arg Cys Arg Glu Arg Leu Gln Ala Ala Glu Ala Tyr Lys Ser Gln
            340                 345                 350

Leu Glu Glu Glu Arg Val Leu Ser Gly Val Leu Glu Ala Ser Lys Ala
        355                 360                 365

Leu Leu Glu Glu Gln Leu Glu Ala Ala Arg Glu Arg Cys Ala Arg Leu
    370                 375                 380

His Glu Thr Gln Arg Glu Asn Leu Leu Leu Arg Thr Arg Leu Gly Glu
385                 390                 395                 400

Ala His Ala Glu Leu Asp Ser Leu Arg His Gln Val Asp Gln Leu Ala
                405                 410                 415

Glu Glu Asn Val Glu Leu Glu Leu Glu Leu Gln Arg Ser Leu Glu Pro
            420                 425                 430

Pro Pro Gly Ser Pro Gly Glu Ala Pro Leu Ala Gly Ala Ala Pro Ser
        435                 440                 445

Leu Gln Asp Glu Val Arg Glu Ala Glu Ala Gly Arg Leu Arg Thr Leu
    450                 455                 460

Glu Arg Glu Asn Arg Glu Leu Arg Gly Leu Leu Gln Val Leu Gln Gly
465                 470                 475                 480

Gln Pro Gly Gly Gln His Pro Leu Leu Glu Ala Pro Arg Glu Asp Pro
                485                 490                 495

Val Leu Pro Val Leu Glu Glu Ala Pro Gln Thr Pro Val Ala Phe Asp
            500                 505                 510

His Ser Pro Gln Gly Leu Val Gln Lys Ala Arg Asp Gly Gly Pro Gln
        515                 520                 525

Ala Leu Asp Leu Ala Pro Pro Ala Leu Asp Ser Val Leu Glu Ala Ser
    530                 535                 540

Ala Glu Cys Pro Gln Ala Pro Asp Ser Asp Pro Gln Glu Ala Glu Ser
545                 550                 555                 560

Pro Leu Gln Ala Ala Ala Met Asp Pro Gln Ala Ser Asp Trp Ser Pro
                565                 570                 575

Gln Glu Ser Gly Ser Pro Val Glu Thr Gln Glu Ser Pro Glu Lys Ala
            580                 585                 590

Gly Arg Arg Ser Ser Leu Gln Ser Pro Ala Ser Val Ala Pro Pro Gln
        595                 600                 605

Gly Pro Gly Thr Lys Ile Gln Ala Pro Gln Leu Leu Gly Gly Glu Thr

610                 615                 620

Glu Gly Arg Glu Ala Pro Gln Gly Glu Leu Val Pro Glu Ala Trp Gly
625                 630                 635                 640

Leu Arg Gln Glu Gly Pro Glu His Lys Pro Gly Pro Ser Glu Pro Ser
                645                 650                 655

Ser Val Gln Leu Glu Glu Gln Glu Gly Pro Asn Gln Gly Leu Asp Leu
            660                 665                 670

Ala Thr Gly Gln Ala Glu Ala Arg Glu His Asp Gln Arg Leu Glu Gly
        675                 680                 685

Thr Val Arg Asp Pro Ala Trp Gln Lys Pro Gln Gln Lys Ser Glu Gly
    690                 695                 700

Ala Leu Glu Val Gln Val Trp Glu Gly Pro Ile Pro Gly Glu Ser Leu
705                 710                 715                 720

Ala Ser Gly Val Ala Glu Gln Glu Ala Leu Arg Glu Glu Val Ala Gln
                725                 730                 735

Leu Arg Arg Lys Ala Glu Ala Leu Gly Asp Glu Leu Glu Ala Gln Ala
            740                 745                 750

Arg Lys Leu Glu Ala Gln Asn Thr Glu Ala Ala Arg Leu Ser Lys Glu
        755                 760                 765

Leu Ala Gln Ala Arg Arg Ala Glu Ala Glu Ala His Arg Glu Ala Glu
    770                 775                 780

Ala Gln Ala Trp Glu Gln Ala Arg Leu Arg Glu Ala Val Glu Ala Ala
785                 790                 795                 800

Gly Gln Glu Leu Glu Ser Ala Ser Gln Glu Arg Glu Ala Leu Val Glu
                805                 810                 815

Ala Leu Ala Ala Ala Gly Arg Glu Arg Arg Gln Trp Glu Arg Glu Gly
            820                 825                 830

Ser Arg Leu Arg Ala Gln Ser Glu Ala Ala Glu Glu Arg Met Gln Val
        835                 840                 845

Leu Glu Ser Glu Gly Arg Gln His Leu Glu Glu Ala Glu Arg Glu Arg
    850                 855                 860

Arg Glu Lys Glu Ala Leu Gln Ala Glu Leu Glu Lys Ala Val Val Arg
865                 870                 875                 880

Gly Lys Glu Leu Gly Ala Arg Leu Glu His Leu Gln Arg Glu Leu Glu
                885                 890                 895

Gln Ala Ala Leu Glu Arg Gln Glu Phe Leu Arg Glu Lys Glu Ser Gln
            900                 905                 910

His Gln Arg Tyr Gln Gly Leu Glu Gln Arg Leu Glu Ala Glu Leu Gln
        915                 920                 925

Ala Ala Ala Thr Ser Lys Glu Glu Ala Leu Met Glu Leu Lys Thr Arg
    930                 935                 940

Ala Leu Gln Leu Glu Glu Glu Leu Phe Gln Leu Arg Gln Gly Pro Ala
945                 950                 955                 960

Gly Leu Gly Pro Lys Lys Arg Ala Glu Pro Gln Leu Val Glu Thr Gln
                965                 970                 975

Asn Val Arg Leu Ile Glu Val Glu Arg Ser Asn Ala Met Leu Val Ala
            980                 985                 990

Glu Lys Ala Ala Leu Gln Gly Gln  Leu Gln His Leu Glu  Gly Gln Leu
        995                 1000                1005

Gly Ser  Leu Gln Gly Arg Ala  Gln Glu Leu Leu Leu  Gln Ser Gln
    1010                1015                1020

Arg Ala  Gln Glu His Ser Ser  Arg Leu Gln Ala Glu  Lys Ser Val
    1025                1030                1035

Leu Glu  Ile Gln Gly Gln Glu  Leu His Arg Lys Leu  Glu Val Leu
    1040                1045                1050

Glu Glu  Glu Val Arg Ala Ala  Arg Gln Ser Gln Glu  Glu Thr Arg
    1055                1060                1065

Gly Gln  Gln Gln Ala Leu Leu  Arg Asp His Lys Ala  Leu Ala Gln
    1070                1075                1080

Leu Gln  Arg Arg Gln Glu Ala  Glu Leu Glu Gly Leu  Leu Val Arg
    1085                1090                1095

His Arg Asp Leu Lys Ala Asn Met Arg Ala Leu Glu Leu Ala His
    1100                1105                1110

Arg Glu Leu Gln Gly Arg His Glu Gln Leu Gln Ala Gln Arg Ala
    1115                1120                1125

Ser Val Glu Ala Gln Glu Val Ala Leu Leu Ala Glu Arg Glu Arg
    1130                1135                1140

Leu Met Gln Asp Gly His Arg Gln Arg Gly Leu Glu Glu Glu Leu
    1145                1150                1155

Arg Arg Leu Gln Ser Glu His Asp Arg Ala Gln Met Leu Leu Ala
    1160                1165                1170

Glu Leu Ser Arg Glu Arg Gly Glu Leu Gln Gly Glu Arg Gly Glu
    1175                1180                1185

Leu Arg Gly Arg Leu Ala Arg Leu Glu Leu Glu Arg Ala Gln Leu
    1190                1195                1200

Glu Met Gln Ser Gln Gln Leu Arg Glu Ser Asn Gln Gln Leu Asp
    1205                1210                1215

Leu Ser Ala Cys Arg Leu Thr Thr Gln Cys Glu Leu Leu Thr Gln
    1220                1225                1230

Leu Arg Ser Ala Gln Glu Glu Glu Asn Arg Gln Leu Leu Ala Glu
    1235                1240                1245

Val Gln Ala Leu Ser Arg Glu Asn Arg Glu Leu Leu Glu Arg Ser
    1250                1255                1260

Leu Glu Ser Arg Asp His Leu His Arg Glu Gln Arg Glu Tyr Leu
    1265                1270                1275

Asp Gln Leu Asn Ala Leu Arg Arg Glu Lys Gln Lys Leu Val Glu
    1280                1285                1290

Lys Ile Met Asp Gln Tyr Arg Val Leu Glu Pro Val Pro Leu Pro
    1295                1300                1305

Arg Thr Lys Lys Gly Ser Trp Leu Ala Asp Lys Val Lys Arg Leu
    1310                1315                1320

Met Arg Pro Arg Arg Glu Gly Gly Pro Pro Gly Gly Leu Arg Leu
    1325                1330                1335

Gly Ala Asp Gly Ala Gly Ser Thr Glu Ser Leu Gly Gly Pro Pro
    1340                1345                1350

Glu Thr Glu Leu Pro Glu Gly Arg Glu Ala Asp Gly Thr Gly Ser
    1355                1360                1365

Pro Ser Pro Ala Pro Met Arg Arg Ala Gln Ser Ser Leu Cys Leu
    1370                1375                1380

Arg Asp Glu Thr Leu Ala Gly Gly Gln Arg Arg Lys Leu Ser Ser
    1385                1390                1395

Arg Phe Pro Val Gly Arg Ser Ser Glu Ser Phe Ser Pro Gly Asp
    1400                1405                1410

Thr Pro Arg Gln Arg Phe Arg Gln Arg His Pro Gly Pro Leu Gly
    1415                1420                1425

Ala Pro Val Ser His Ser Lys Gly Pro Gly Val Gly Trp Glu Asn
    1430                1435                1440

Ser Ala Glu Thr Leu Gln Glu His Glu Thr Asp Ala Asn Arg Glu
    1445                1450                1455

Gly Pro Glu Val Gln Glu Pro Glu Lys Arg Pro Leu Thr Pro Ser
    1460                1465                1470

Leu Ser Gln
    1475

<210> 77
<211> 840
<212> DNA
<213> Homo sapiens

<400> 77
actcgccacc tcctcttcca cccctgccag gcccagcagc caccacagcg cctgcttcct      60

cggccctgaa atcatgcccc taggtctcct gtggctgggc ctagccctgt tgggggctct     120

gcatgcccag gcccaggact ccacctcaga cctgatccca gccccacctc tgagcaaggt     180

ccctctgcag cagaacttcc aggacaacca attccagggg aagtggtatg tggtaggcct     240

ggcagggaat gcaattctca gagaagacaa agacccgcaa aagatgtatg ccaccatcta     300

tgagctgaaa gaagacaaga gctacaatgt cacctccgtc ctgtttagga aaaagaagtg     360

tgactactgg atcaggactt ttgttccagg ttgccagccc ggcgagttca cgctgggcaa 420

cattaagagt taccctggat taacgagtta cctcgtccga gtggtgagca ccaactacaa 480

ccagcatgct atggtgttct tcaagaaagt ttctcaaaac agggagtact tcaagatcac 540

cctctacggg agaaccaagg agctgacttc ggaactaaag gagaacttca tccgcttctc 600

caaatctctg ggcctccctg aaaaccacat cgtcttccct gtcccaatcg accagtgtat 660

cgacggctga gtgcacaggt gccgccagct gccgcaccag cccgaacacc attgagggag 720

ctgggagacc ctccccacag tgccacccat gcagctgctc cccaggccac cccgctgatg 780

gagccccacc ttgtctgcta aataaacatg tgccctcagg ccaaaaaaaa aaaaaaaaaa 840

<210> 78
<211> 198
<212> PRT
<213> Homo sapiens

<400> 78
Met Pro Leu Gly Leu Leu Trp Leu Gly Leu Ala Leu Leu Gly Ala Leu
1               5                   10                  15

His Ala Gln Ala Gln Asp Ser Thr Ser Asp Leu Ile Pro Ala Pro Pro
            20                  25                  30

Leu Ser Lys Val Pro Leu Gln Gln Asn Phe Gln Asp Asn Gln Phe Gln
        35                  40                  45

Gly Lys Trp Tyr Val Val Gly Leu Ala Gly Asn Ala Ile Leu Arg Glu
    50                  55                  60

Asp Lys Asp Pro Gln Lys Met Tyr Ala Thr Ile Tyr Glu Leu Lys Glu
65                  70                  75                  80

Asp Lys Ser Tyr Asn Val Thr Ser Val Leu Phe Arg Lys Lys Lys Cys
                85                  90                  95

Asp Tyr Trp Ile Arg Thr Phe Val Pro Gly Cys Gln Pro Gly Glu Phe
            100                 105                 110

Thr Leu Gly Asn Ile Lys Ser Tyr Pro Gly Leu Thr Ser Tyr Leu Val
        115                 120                 125

Arg Val Val Ser Thr Asn Tyr Asn Gln His Ala Met Val Phe Phe Lys
    130                 135                 140

Lys Val Ser Gln Asn Arg Glu Tyr Phe Lys Ile Thr Leu Tyr Gly Arg

145                 150                 155                 160

Thr Lys Glu Leu Thr Ser Glu Leu Lys Glu Asn Phe Ile Arg Phe Ser
                165                 170                 175

Lys Ser Leu Gly Leu Pro Glu Asn His Ile Val Phe Pro Val Pro Ile
            180                 185                 190

Asp Gln Cys Ile Asp Gly
        195

<210> 79
<211> 2224
<212> DNA
<213> Homo sapiens

<400> 79
gctgacacct gcccagtgga agctggcatc cctccccttg tgggttcaga gctgcaagaa      60

gcaccaggct cggccacttc agaagcccca gcctcgacct agcccaccct ctcagggcca     120

cagtgcagaa gcctgcacac ctgccaagtc tctccgactc cttgcagctg ctgtcagcat     180

ggcccaggct cctgctgacc cgggcagaga aggccacctt gaacaaagaa tcctgcaggt     240

gctgacagag gctggctccc cggtgaaact tgcccagctg gtgaaggaat gccaagcacc     300

caagagggag ctcaaccaag tcctctaccg aatgaaaaag gagttgaaag tctccctcac     360

atcccctgcc acctggtgct tgggcgggac tgatcctgaa ggcgagggtc ctgcagagct     420

ggccttgtcc agccctgccg agaggcccca gcaacatgca gctacaattc cagagacccc     480

tggccctcag ttcagccaac aacggaggga agacatctac aggtttctca aagacaatgg     540

tccccagagg gccctggtca tcgcccaagc actgggaatg aggacagcaa aagatgtgaa     600

ccgagacttg tacaggatga agagcaggca ccttctggac atggatgagc agtccaaagc     660

atggacgatt taccgcccag aagattctgg aagaagagca aagtcagcct caattattta     720

ccagcacaat ccaatcaaca tgatctgcca gaatggaccc aacagctgga tttccattgc     780

aaactccgaa gccatccaga ttggacacgg gaacatcatt acaagacaga cagtctccag     840

ggaggacggt tccgccggtc cacgccacct cccttcaatg gcaccaggtg attcctcaac     900

ttgggggacc ctagttgatc cctgggggcc ccaggacatc cacatggagc ggtccatact     960

gagacgggtg cagctgggac acagcaatga gatgaggctc cacggcgtcc cgtccgaggg    1020

ccctgcccac atcccccctg gcagcccccc agtctctgcc actgctgccg gcccagaagc    1080

ttcgtttgaa gcaagaattc ccagtccagg aactcaccct gagggggaag ccgcccagag    1140

aatccacatg aaatcgtgct ttctcgagga cgccaccatc ggcaacagca acaaaatgtc    1200

tatcagccca ggggtggctg gcccaggagg agtcgcaggg tctggagagg gggagccagg 1260

ggaggacgca ggtcgtcgtc ccgcagacac acaatccaga agtcactttc ctcgagacat 1320

tggtcagccc atcactccca gccactcgaa gctcaccccc aagctggaaa ctatgactct 1380

tggaaacagg agtcacaaag ctgcagaagg cagccactat gtggatgaag cctcacacga 1440

ggggagctgg tggggaggtg ggatttagtg cacagcctca cgtggggctt ggacacaggc 1500

tgggggtggg cgcatgctag ggagactagc ctgctgctct ctgcattcct tagcgtcttg 1560

tttgacctgc ttgcttccag acataacctg catgaatcag ttttggggga atggacctgg 1620

catggggatg ggttcaggcc aggtcttttg atggccagga gtagatgaca gggagttgcc 1680

ttggggaacc tttggtgtgc caagaggagg tgggtagatg ggagtggggc tcggtccccc 1740

aggcccaggg gactctctcc actctttcct gggctcgggg catctgcctg gagttacctt 1800

ccatcatggc tacctgctgt ggtttgaatg tttgagtccc aacaaaattc atatcaaaac 1860

ataatcccaa ctgggtgcag tggctcacgc ctgtaatccc agcactttgg gaggccgagg 1920

cgggcggatc aataggtcag gaaatccaga ccgtcctggc taacatggtg aaaccccgtc 1980

tctactaaaa aaaaaaatac aaaaaattag ccgggcgttg tggcgggcac ctggagtccc 2040

agctactccg gaggctgagg gaggagaatg gtgtgaaccc gggaggtgga gcttccagtg 2100

agccgagatc gcgccactgc actccaggct gggcgacaga gcgagactcc gtctcaaaaa 2160

aataaataca taaataaaaa ataaaccacc cataaaaaaa aaaaaaaaaa aaaaaaaaaa 2220

aaaa 2224

<210> 80
<211> 429
<212> PRT
<213> Homo sapiens

<400> 80
Met Ala Gln Ala Pro Ala Asp Pro Gly Arg Glu Gly His Leu Glu Gln
1               5                   10                  15

Arg Ile Leu Gln Val Leu Thr Glu Ala Gly Ser Pro Val Lys Leu Ala
            20                  25                  30

Gln Leu Val Lys Glu Cys Gln Ala Pro Lys Arg Glu Leu Asn Gln Val
        35                  40                  45

Leu Tyr Arg Met Lys Lys Glu Leu Lys Val Ser Leu Thr Ser Pro Ala
    50                  55                  60

Thr Trp Cys Leu Gly Gly Thr Asp Pro Glu Gly Glu Gly Pro Ala Glu
65                  70                  75                  80

Leu Ala Leu Ser Ser Pro Ala Glu Arg Pro Gln Gln His Ala Ala Thr
                85                  90                  95

Ile Pro Glu Thr Pro Gly Pro Gln Phe Ser Gln Gln Arg Glu Glu Asp
            100                 105                 110

Ile Tyr Arg Phe Leu Lys Asp Asn Gly Pro Gln Arg Ala Leu Val Ile
        115                 120                 125

Ala Gln Ala Leu Gly Met Arg Thr Ala Lys Asp Val Asn Arg Asp Leu
    130                 135                 140

Tyr Arg Met Lys Ser Arg His Leu Leu Asp Met Asp Glu Gln Ser Lys
145                 150                 155                 160

Ala Trp Thr Ile Tyr Arg Pro Glu Asp Ser Gly Arg Arg Ala Lys Ser
                165                 170                 175

Ala Ser Ile Ile Tyr Gln His Asn Pro Ile Asn Met Ile Cys Gln Asn
            180                 185                 190

Gly Pro Asn Ser Trp Ile Ser Ile Ala Asn Ser Glu Ala Ile Gln Ile
        195                 200                 205

Gly His Gly Asn Ile Ile Thr Arg Gln Thr Val Ser Arg Glu Asp Gly
    210                 215                 220

Ser Ala Gly Pro Arg His Leu Pro Ser Met Ala Pro Gly Asp Ser Ser
225                 230                 235                 240

Thr Trp Gly Thr Leu Val Asp Pro Trp Gly Pro Gln Asp Ile His Met
                245                 250                 255

Glu Arg Ser Ile Leu Arg Arg Val Gln Leu Gly His Ser Asn Glu Met
            260                 265                 270

Arg Leu His Gly Val Pro Ser Glu Gly Pro Ala His Ile Pro Pro Gly
        275                 280                 285

Ser Pro Pro Val Ser Ala Thr Ala Ala Gly Pro Glu Ala Ser Phe Glu
    290                 295                 300

Ala Arg Ile Pro Ser Pro Gly Thr His Pro Glu Gly Glu Ala Ala Gln
305                 310                 315                 320

Arg Ile His Met Lys Ser Cys Phe Leu Glu Asp Ala Thr Ile Gly Asn
                325                 330                 335

Ser Asn Lys Met Ser Ile Ser Pro Gly Val Ala Gly Pro Gly Gly Val
            340                 345                 350

Ala Gly Ser Gly Glu Gly Glu Pro Gly Glu Asp Ala Gly Arg Arg Pro
        355                 360                 365

Ala Asp Thr Gln Ser Arg Ser His Phe Pro Arg Asp Ile Gly Gln Pro
    370                 375                 380

Ile Thr Pro Ser His Ser Lys Leu Thr Pro Lys Leu Glu Thr Met Thr
385                 390                 395                 400

Leu Gly Asn Arg Ser His Lys Ala Ala Glu Gly Ser His Tyr Val Asp
                405                 410                 415

Glu Ala Ser His Glu Gly Ser Trp Trp Gly Gly Gly Ile
            420                 425

<210> 81
<211> 2425
<212> DNA
<213> Homo sapiens

<400> 81
acgcggggat tctgtgtaat tcctatccac agtcacttct gttcattttt aatgaaaacc      60

tattgctgtt attttctatt atgagttaac ggtgatgtgt gttttttaaa ttttgtatac     120

ttgctctcac cctaaaatat accttaagag acttttcccc tgtttttctt tctgctttcc     180

aagactccag gaaaaacagc ttccatggca cattttgtac agggcacatc tagaatgatt     240

gccgcagaaa gttctacgga gcataaagag tgtgctgaac catcaacaag aaagaacttg     300

atgaattctc ttgaacaaaa gataaggtgt ttggaaaaac aaagaaaaga gctcctggaa     360

gttaaccagc aatgggatca gcaatttaga agtatgaaag agttatatga aagaaaggta     420

gcagagctga agacgaaact ggacgccgcg gaaagattcc tcagcacgcg ggagaaggat     480

ccgcatcaga ggcagagaaa ggacgacagg cagagagagg acgacaggca gcgcgacctg     540

acccgggacc ggctgcagcg ggaggagaag gaaaaggaac gcctaaatga agaattacat     600

gaattgaaag aagagaataa acttttaaag ggaaaaaata ctcttgcgaa caaggaaaag     660

gaacattacg aatgtgaaat aaaacgcctc aataaggctc ttcaggatgc cttgaatatc     720

aagtgttcat tttccgagga ctgtttgagg aagtctcgag tggaattctg ccatgaggag     780

atgagaacag aaatggaagt tctgaagcag caggtgcaaa tatacgaaga agacttcaaa 840

aaggaacgat cggatcgaga gagacttaat caagagaaag aggagctaca gcaaattaat 900

gaaacttccc aatcccagtt gaacaggctg aattcccaga taaaagcttg tcagatggag 960

aaagaaaaac tagaaaagca attaaaacag atgtattgcc caccctgtaa ctgcggcttg 1020

gttttccacc tgcaagatcc atgggtacca acaggccctg gagctgtgca gaagcaacgg 1080

gagcacccac cagactgtca gtggtatgct cttgaccagc ttccgccaga tgtacaacac 1140

aaggcaaatg gtttatcctc agtaaagaaa gtccatccgt agaagtacac actaacagag 1200

agagacaaca ggcaacgagg gagcgtggct gaggaccctc ttctttgttt atactgaggc 1260

ttgtttctgc cacaaaggca catcatctac cctgttattt gtgcctggtc atgttttcct 1320

ttggtatgaa tacaggaaaa ggcttctgac agctaagatc ctgatcaaat tggaggagcc 1380

agttattata caaccaaata caacttccag tttccttgaa atgagacttg gcagatggat 1440

gatgcacgac tccctgaaaa tctcttcagc atattgctgt tggatgcaca aaaatgattc 1500

tatgatttag gcaactcagt gtttgctgca gggatattgt atgttgattt aaagtgcata 1560

tgtgtgtgtg tgtgtgtgtg tgtgtgtgtg tttcctgggg taaaaatttc tgcagttgta 1620

aacgtttaac attgctccca ttcttcttcc acagggcctt agttctttag ctatttgact 1680

acatattgtt gacctcagtg atcctaaacg tagaattgaa gaggtgtata tacctcgtta 1740

aattaaataa gtggttcaat ctgaataaga tggttaattg gaagctattc cttagacaga 1800

tcatttcttt aagatggtga gaaattgtgc gcctgcatca caagacttga ttatgaaaga 1860

taaagtcttg ggctgggtgc agtggctcat gcctataatc ccagcacttt gggaggctga 1920

ggcaggtgga ttgctttgag gccaggagtt ctagaccagc ctggccagca tggtgaaacc 1980

ccatctctac tataaataca aaaattagcc aggctcgtgg cacacaccta taaacccagc 2040

tactcaggag gcttgaggcg ggaggatcac ttgagcccag ggaggcagag gttgctgtaa 2100

gttgagatcg ccactgcact ccagcctggg aaatagagtg agcaagactc agtgccccct 2160

acccacccac tccacaccac caaaaaaaaa aaaaaaaaaa aaaggaagac aaagtcctga 2220

tgggatgatg atggctatat ggctatgctg tgtccatata tacttacaga aatatttcag 2280

acatatggat ttatagttaa atctgtgttt ttccttccta tggatttgat taattttaat 2340

gaaaaactta attctgactc ctcaagccaa ttaaaatgaa aatggaattt atttaaaaaa 2400

aaaaaaaaaa aaaaaaaaaa aaaaa 2425

<210> 82
<211> 325
<212> PRT
<213> Homo sapiens

<400> 82
Met Ala His Phe Val Gln Gly Thr Ser Arg Met Ile Ala Ala Glu Ser
1               5                   10                  15

Ser Thr Glu His Lys Glu Cys Ala Glu Pro Ser Thr Arg Lys Asn Leu
            20                  25                  30

Met Asn Ser Leu Glu Gln Lys Ile Arg Cys Leu Glu Lys Gln Arg Lys
        35                  40                  45

Glu Leu Leu Glu Val Asn Gln Gln Trp Asp Gln Gln Phe Arg Ser Met
    50                  55                  60

Lys Glu Leu Tyr Glu Arg Lys Val Ala Glu Leu Lys Thr Lys Leu Asp
65                  70                  75                  80

Ala Ala Glu Arg Phe Leu Ser Thr Arg Glu Lys Asp Pro His Gln Arg
                85                  90                  95

Gln Arg Lys Asp Asp Arg Gln Arg Glu Asp Asp Arg Gln Arg Asp Leu
            100                 105                 110

Thr Arg Asp Arg Leu Gln Arg Glu Glu Lys Glu Lys Glu Arg Leu Asn
        115                 120                 125

Glu Glu Leu His Glu Leu Lys Glu Glu Asn Lys Leu Leu Lys Gly Lys
    130                 135                 140

Asn Thr Leu Ala Asn Lys Glu Lys Glu His Tyr Glu Cys Glu Ile Lys
145                 150                 155                 160

Arg Leu Asn Lys Ala Leu Gln Asp Ala Leu Asn Ile Lys Cys Ser Phe
                165                 170                 175

Ser Glu Asp Cys Leu Arg Lys Ser Arg Val Glu Phe Cys His Glu Glu
            180                 185                 190

Met Arg Thr Glu Met Glu Val Leu Lys Gln Gln Val Gln Ile Tyr Glu
        195                 200                 205

Glu Asp Phe Lys Lys Glu Arg Ser Asp Arg Glu Arg Leu Asn Gln Glu
    210                 215                 220

Lys Glu Glu Leu Gln Gln Ile Asn Glu Thr Ser Gln Ser Gln Leu Asn
225                 230                 235                 240

Arg Leu Asn Ser Gln Ile Lys Ala Cys Gln Met Glu Lys Glu Lys Leu
                245                 250                 255

Glu Lys Gln Leu Lys Gln Met Tyr Cys Pro Pro Cys Asn Cys Gly Leu
            260                 265                 270

Val Phe His Leu Gln Asp Pro Trp Val Pro Thr Gly Pro Gly Ala Val
        275                 280                 285

Gln Lys Gln Arg Glu His Pro Pro Asp Cys Gln Trp Tyr Ala Leu Asp
    290                 295                 300

Gln Leu Pro Pro Asp Val Gln His Lys Ala Asn Gly Leu Ser Ser Val
305                 310                 315                 320

Lys Lys Val His Pro
                325

<210> 83
<211> 2737
<212> DNA
<213> Homo sapiens

<400> 83
agtgcctcac caccacctgc ccgcgcgccg ccccatataa aggcgccgcc gtccttaccc      60

ccaggactcg gccccatgga gacgccgccg gccgccgctg gcgcatggcg ggtaggagct     120

gtggcgcggg gccttccagg agtctgagct atgagtggcc cctgtggaga gaagcctgtc     180

ctggaagcca gccccaccat gagtctgtgg gaatttgagg acagccacag ccgtcagggc     240

accccaaggc cgggtcaaga gctggccgct gaggaggcct cggccctgga actgcagatg     300

aaggtggact tcttccggaa gctgggctat tcatccacgg agatccacag cgtcctgcag     360

aagctgggcg tccaggcaga caccaacacg gtgctgggtg agctggtgaa acacgggaca     420

gccaccgagc gggagcgcca gacctcaccg gacccctgcc ctcagctccc tctagtcccg     480

cggggtggtg gcacccctaa ggctcccaac ctggagcctc cactcccaga agaggaaaag     540

gagggcagcg acctgagacc agtggtcatc gatgggagca acgtggccat gagccatggg     600

aacaaggagg tcttctcctg ccggggcatc ctgctggcag tgaactggtt tctggagcgg     660

ggccacacag acatcacagt gtttgtgcca tcctggagga aggagcagcc tcggcccgac     720

gtgcccatca cagaccagca catcctgcgg gaactggaga agaagaagat cctggtgttc     780

acaccatcac gacgcgtggg tggcaagcgg gtggtgtgct atgacgacag attcattgtg     840

aagctggcct acgagtctga cgggatcgtg gtttccaacg acacataccg tgacctccaa     900

ggcgagcggc aggagtggaa gcgcttcatc gaggagcggc tgctcatgta ctccttcgtc 960
aatgacaagt ttatgccccc tgatgaccca ctgggccggc acgggcccag cctggacaac 1020
ttcctgcgta agaagccact cactttggag cacaggaagc agccgtgtcc ctatggaagg 1080
aaatgcacct atgggatcaa gtgccgattc ttccacccag agcggccaag ctgcccccag 1140
cgctctgtgg cagatgagct ccgtgccaat gctctcctct caccccccag agccccaagc 1200
aaggacaaaa atggccggcg gccttcacct tcatcccagt ccagctctct gctaacagag 1260
agtgagcagt gcagcctgga tgggaagaag ctgggggccc aggcatcccc agggtcccgc 1320
caagagggtc taacacagac ctatgcccca tcaggcagga gcctcgcacc tagcgggggc 1380
agtggcagca gctttgggcc cacagactgg ctcccacaga cgctggactc actcccgtac 1440
gtctcccagg attgcctgga ctcgggcatt ggctccctgg agagccagat gtcggaactt 1500
tggggggttc gaggaggagg ccctggtgag ccgggcccac cccgagcccc ttacacgggc 1560
tacagtccct atggatctga gctcccagcc accgcagcct tctctgcctt tggccgggcc 1620
atgggtgctg gccacttcag tgtccctgcc gactacccac ccgcgccccc tgcctttcca 1680
cctcgagagt actggtctga accataccca ctgcccccac ccacatcagt ccttcaggag 1740
cccccagtgc agagcccagg ggctggcagg agcccgtggg gcagggcagg cagcctggcc 1800
aaggagcagg ccagcgtgta tactaagctg tgtggtgtgt ttcccccgca cctggtggag 1860
gctgtgatgg ggcgcttccc acagctcctg gacccccagc agctggctgc cgagatcctc 1920
tcctacaagt cccagcaccc cagtgagtaa gctgcctgtg gctggcaagg gcagcacccc 1980
cagcctccaa gggccgtcag gctgggcttt gggccattga gcagcccatt cccagccctg 2040
aggcccaccc cagaggctgg acagagggag gattcaagtc gggaaggaaa cccacaaacc 2100
aaagatactg taggattggt tctggcccat gcagcacctc tagctgtctg cctcagtggg 2160
tcagaagcga tcaccctgtt gatacacatt gtatctctgt agtttaagga gacgctgccg 2220
gtaacggcgt cggtccgtgg ctgaggccca aaccgtcttt tctctcagag ggtggggagg 2280
gaggtggggg cagcagaggc ctgggctggg tgccctgtgc acgccacccc acttccgccc 2340
tacccctggg acgttggcct tggctggcta gttgggcacc gtgtgcctgc cctccaaggg 2400
cctcctctac gccaatgagg cctcatctgt gctctcgctg ggcacgtggc ttcatgtcag 2460
taagcaagat gcttcttaat aacccacctt ctgccccact ctattcctta tcctgctgcc 2520
cctgtagggg tcaagggccc tccgtctaca ccctcttctt ctcctccatc ctttattcag 2580
agtcatctcg cccttcccca tgggtggggg aacctgtgtt tgtttgtgtg cacatgtaaa 2640
ttttaaatat tttaagcaga aagtccttac ctcctgtaac acatcaataa agtacaatca 2700
ttgtgagccc tttcaaaaaa aaaaaaaaaa aaaaaaa 2737

<210> 84
<211> 599
<212> PRT
<213> Homo sapiens

<400> 84
Met Ser Gly Pro Cys Gly Glu Lys Pro Val Leu Glu Ala Ser Pro Thr
1               5                   10                  15

Met Ser Leu Trp Glu Phe Glu Asp Ser His Ser Arg Gln Gly Thr Pro
            20                  25                  30

Arg Pro Gly Gln Glu Leu Ala Ala Glu Glu Ala Ser Ala Leu Glu Leu
        35                  40                  45

Gln Met Lys Val Asp Phe Phe Arg Lys Leu Gly Tyr Ser Ser Thr Glu
    50                  55                  60

Ile His Ser Val Leu Gln Lys Leu Gly Val Gln Ala Asp Thr Asn Thr
65                  70                  75                  80

Val Leu Gly Glu Leu Val Lys His Gly Thr Ala Thr Glu Arg Glu Arg
                85                  90                  95

Gln Thr Ser Pro Asp Pro Cys Pro Gln Leu Pro Leu Val Pro Arg Gly
            100                 105                 110

Gly Gly Thr Pro Lys Ala Pro Asn Leu Glu Pro Pro Leu Pro Glu Glu
        115                 120                 125

Glu Lys Glu Gly Ser Asp Leu Arg Pro Val Val Ile Asp Gly Ser Asn
    130                 135                 140

Val Ala Met Ser His Gly Asn Lys Glu Val Phe Ser Cys Arg Gly Ile
145                 150                 155                 160

Leu Leu Ala Val Asn Trp Phe Leu Glu Arg Gly His Thr Asp Ile Thr
                165                 170                 175

Val Phe Val Pro Ser Trp Arg Lys Glu Gln Pro Arg Pro Asp Val Pro
            180                 185                 190

Ile Thr Asp Gln His Ile Leu Arg Glu Leu Glu Lys Lys Lys Ile Leu
        195                 200                 205

Val Phe Thr Pro Ser Arg Arg Val Gly Gly Lys Arg Val Val Cys Tyr

210                 215                 220

Asp Asp Arg Phe Ile Val Lys Leu Ala Tyr Glu Ser Asp Gly Ile Val
225                 230                 235                 240

Val Ser Asn Asp Thr Tyr Arg Asp Leu Gln Gly Glu Arg Gln Glu Trp
                245                 250                 255

Lys Arg Phe Ile Glu Glu Arg Leu Leu Met Tyr Ser Phe Val Asn Asp
            260                 265                 270

Lys Phe Met Pro Pro Asp Asp Pro Leu Gly Arg His Gly Pro Ser Leu
        275                 280                 285

Asp Asn Phe Leu Arg Lys Lys Pro Leu Thr Leu Glu His Arg Lys Gln
    290                 295                 300

Pro Cys Pro Tyr Gly Arg Lys Cys Thr Tyr Gly Ile Lys Cys Arg Phe
305                 310                 315                 320

Phe His Pro Glu Arg Pro Ser Cys Pro Gln Arg Ser Val Ala Asp Glu
                325                 330                 335

Leu Arg Ala Asn Ala Leu Leu Ser Pro Pro Arg Ala Pro Ser Lys Asp
            340                 345                 350

Lys Asn Gly Arg Arg Pro Ser Pro Ser Ser Gln Ser Ser Ser Leu Leu
        355                 360                 365

Thr Glu Ser Glu Gln Cys Ser Leu Asp Gly Lys Lys Leu Gly Ala Gln
    370                 375                 380

Ala Ser Pro Gly Ser Arg Gln Glu Gly Leu Thr Gln Thr Tyr Ala Pro
385                 390                 395                 400

Ser Gly Arg Ser Leu Ala Pro Ser Gly Gly Ser Gly Ser Ser Phe Gly
                405                 410                 415

Pro Thr Asp Trp Leu Pro Gln Thr Leu Asp Ser Leu Pro Tyr Val Ser
            420                 425                 430

Gln Asp Cys Leu Asp Ser Gly Ile Gly Ser Leu Glu Ser Gln Met Ser
        435                 440                 445

Glu Leu Trp Gly Val Arg Gly Gly Gly Pro Gly Glu Pro Gly Pro Pro
    450                 455                 460

Arg Ala Pro Tyr Thr Gly Tyr Ser Pro Tyr Gly Ser Glu Leu Pro Ala
465                 470                 475                 480

Thr Ala Ala Phe Ser Ala Phe Gly Arg Ala Met Gly Ala Gly His Phe
                485                 490                 495

Ser Val Pro Ala Asp Tyr Pro Pro Ala Pro Pro Ala Phe Pro Pro Arg
            500                 505                 510

Glu Tyr Trp Ser Glu Pro Tyr Pro Leu Pro Pro Pro Thr Ser Val Leu
        515                 520                 525

Gln Glu Pro Pro Val Gln Ser Pro Gly Ala Gly Arg Ser Pro Trp Gly
    530                 535                 540

Arg Ala Gly Ser Leu Ala Lys Glu Gln Ala Ser Val Tyr Thr Lys Leu
545                 550                 555                 560

Cys Gly Val Phe Pro Pro His Leu Val Glu Ala Val Met Gly Arg Phe
                565                 570                 575

Pro Gln Leu Leu Asp Pro Gln Gln Leu Ala Ala Glu Ile Leu Ser Tyr
            580                 585                 590

Lys Ser Gln His Pro Ser Glu
        595

<210> 85
<211> 1867
<212> DNA
<213> Homo sapiens

<400> 85
cacatagctc agttcccata aaagggctgg tttgccgcgt cggggagtgg agtgggacag      60

gtatataaag gaagtacagg gcctggggaa gaggccctgt ctaggtagct ggcaccagga     120

gccgtgggca agggaagagg ccacaccctg ccctgctctg ctgcagccag aatgggtgtg     180

aaggcgtctc aaacaggctt tgtggtcctg gtgctgctcc agtgctgctc tgcatacaaa     240

ctggtctgct actacaccag ctggtcccag taccgggaag gcgatgggag ctgcttccca     300

gatgcccttg accgcttcct ctgtacccac atcatctaca gctttgccaa tataagcaac     360

gatcacatcg acacctggga gtggaatgat gtgacgctct acggcatgct caacacactc     420

aagaacagga accccaacct gaagactctc ttgtctgtcg gaggatggaa ctttgggtct     480

caaagatttt ccaagatagc ctccaacacc cagagtcgcc ggactttcat caagtcagta     540

ccgccatttc tgcgcaccca tggctttgat gggctggacc ttgcctggct ctaccctgga 600

cggagagaca aacagcattt taccacccta atcaaggaaa tgaaggccga atttataaag 660

gaagcccagc cagggaaaaa gcagctcctg ctcagcgcag cactgtctgc ggggaaggtc 720

accattgaca gcagctatga cattgccaag atatcccaac acctggattt cattagcatc 780

atgacctacg attttcatgg agcctggcgt gggaccacag gccatcacag tcccctgttc 840

cgaggtcagg aggatgcaag tcctgacaga ttcagcaaca ctgactatgc tgtggggtac 900

atgttgaggc tgggggctcc tgccagtaag ctggtgatgg gcatccccac cttcgggagg 960

agcttcactc tggcttcttc tgagactggt gttggagccc caatctcagg accgggaatt 1020

ccaggccggt tcaccaagga ggcagggacc cttgcctact atgagatctg tgacttcctc 1080

cgcggagcca cagtccatag aatcctcggc cagcaggtcc cctatgccac caagggcaac 1140

cagtgggtag gatacgacga ccaggaaagc gtcaaaagca aggtgcagta cctgaaggac 1200

aggcagctgg cgggcgccat ggtatgggcc ctggacctgg atgacttcca gggctccttc 1260

tgcggccagg atctgcgctt ccctctcacc aatgccatca aggatgcact cgctgcaacg 1320

tagccctctg ttctgcacac agcacggggg ccaaggatgc cccgtccccc tctggctcca 1380

gctggccggg agcctgatca cctgccctgc tgagtcccag gctgagcctc agtctccctc 1440

ccttggggcc tatgcagagg tccacaacac acagatttga gctcagccct ggtgggcaga 1500

gaggtaggga tggggctgtg gggatagtga ggcatcgcaa tgtaagactc gggattagta 1560

cacacttgtt gattaatgga aatgtttaca gatccccaag cctggcaagg gaatttcttc 1620

aactccctgc cccccagccc tccttatcaa aggacaccat tttggcaagc tctatcacca 1680

aggagccaaa catcctacaa gacacagtga ccatactaat tataccccct gcaaagccca 1740

gcttgaaacc ttcacttagg aacgtaatcg tgtcccctat cctacttccc cttcctaatt 1800

ccacagctgc tcaataaagt acaagagctt aacagtgaaa aaaaaaaaaa aaaaaaaaaa 1860

aaaaaaa 1867

<210> 86
<211> 383
<212> PRT
<213> Homo sapiens

<400> 86
Met Gly Val Lys Ala Ser Gln Thr Gly Phe Val Val Leu Val Leu Leu
1               5                   10                  15

Gln Cys Cys Ser Ala Tyr Lys Leu Val Cys Tyr Tyr Thr Ser Trp Ser
            20                  25                  30

Gln Tyr Arg Glu Gly Asp Gly Ser Cys Phe Pro Asp Ala Leu Asp Arg
        35                  40                  45

Phe Leu Cys Thr His Ile Ile Tyr Ser Phe Ala Asn Ile Ser Asn Asp
    50                  55                  60

His Ile Asp Thr Trp Glu Trp Asn Asp Val Thr Leu Tyr Gly Met Leu
65                  70                  75                  80

Asn Thr Leu Lys Asn Arg Asn Pro Asn Leu Lys Thr Leu Leu Ser Val
                85                  90                  95

Gly Gly Trp Asn Phe Gly Ser Gln Arg Phe Ser Lys Ile Ala Ser Asn
            100                 105                 110

Thr Gln Ser Arg Arg Thr Phe Ile Lys Ser Val Pro Pro Phe Leu Arg
        115                 120                 125

Thr His Gly Phe Asp Gly Leu Asp Leu Ala Trp Leu Tyr Pro Gly Arg
    130                 135                 140

Arg Asp Lys Gln His Phe Thr Thr Leu Ile Lys Glu Met Lys Ala Glu
145                 150                 155                 160

Phe Ile Lys Glu Ala Gln Pro Gly Lys Lys Gln Leu Leu Leu Ser Ala
                165                 170                 175

Ala Leu Ser Ala Gly Lys Val Thr Ile Asp Ser Ser Tyr Asp Ile Ala
            180                 185                 190

Lys Ile Ser Gln His Leu Asp Phe Ile Ser Ile Met Thr Tyr Asp Phe
        195                 200                 205

His Gly Ala Trp Arg Gly Thr Thr Gly His His Ser Pro Leu Phe Arg
    210                 215                 220

Gly Gln Glu Asp Ala Ser Pro Asp Arg Phe Ser Asn Thr Asp Tyr Ala
225                 230                 235                 240

Val Gly Tyr Met Leu Arg Leu Gly Ala Pro Ala Ser Lys Leu Val Met
                245                 250                 255

Gly Ile Pro Thr Phe Gly Arg Ser Phe Thr Leu Ala Ser Ser Glu Thr
            260                 265                 270

Gly Val Gly Ala Pro Ile Ser Gly Pro Gly Ile Pro Gly Arg Phe Thr
        275                 280                 285

Lys Glu Ala Gly Thr Leu Ala Tyr Tyr Glu Ile Cys Asp Phe Leu Arg
    290                 295                 300

Gly Ala Thr Val His Arg Ile Leu Gly Gln Gln Val Pro Tyr Ala Thr
305                 310                 315                 320

Lys Gly Asn Gln Trp Val Gly Tyr Asp Asp Gln Glu Ser Val Lys Ser
                325                 330                 335

Lys Val Gln Tyr Leu Lys Asp Arg Gln Leu Ala Gly Ala Met Val Trp
            340                 345                 350

Ala Leu Asp Leu Asp Asp Phe Gln Gly Ser Phe Cys Gly Gln Asp Leu
        355                 360                 365

Arg Phe Pro Leu Thr Asn Ala Ile Lys Asp Ala Leu Ala Ala Thr
    370                 375                 380

<210> 87
<211> 2406
<212> DNA
<213> Homo sapiens

<400> 87
gattctgtgt gtgtcctcag atgctcagcc acagaccttt gagggagtaa agggggcaga      60

cccacccacc ttgcctccag gctctttcct tcctggtcct gttctatggt ggggctccct     120

tgccagactt cagactgaga agtcagatga agtttcaaga aaaggaaatt ggtgggtgac     180

agagatgggt ggaggggctg gggaaaggct gtttacttcc tcctgtctag tcggtttggt     240

ccctttaggg ctccggatat ctttggtgac ttgtccactc cagtgtggca tcatgtggca     300

gctgctcctc ccaactgctc tgctacttct agtttcagct ggcatgcgga ctgaagatct     360

cccaaaggct gtggtgttcc tggagcctca atggtacagg gtgctcgaga aggacagtgt     420

gactctgaag tgccagggag cctactcccc tgaggacaat tccacacagt ggtttcacaa     480

tgagagcctc atctcaagcc aggcctcgag ctacttcatt gacgctgcca cagtcgacga     540

cagtggagag tacaggtgcc agacaaacct ctccaccctc agtgacccgg tgcagctaga     600

agtccatatc ggctggctgt tgctccaggc ccctcggtgg gtgttcaagg aggaagaccc     660

tattcacctg aggtgtcaca gctggaagaa cactgctctg cataaggtca catatttaca     720

gaatggcaaa ggcaggaagt attttcatca taattctgac ttctacattc caaaagccac     780

actcaaagac agcggctcct acttctgcag ggggcttttt gggagtaaaa atgtgtcttc     840

agagactgtg aacatcacca tcactcaagg tttggcagtg tcaaccatct catcattctt 900

tccacctggg taccaagtct ctttctgctt ggtgatggta ctcctttttg cagtggacac 960

aggactatat ttctctgtga agacaaacat tcgaagctca acaagagact ggaaggacca 1020

taaatttaaa tggagaaagg accctcaaga caaatgaccc ccatcccatg ggggtaataa 1080

gagcagtagc agcagcatct ctgaacattt ctctggattt gcaaccccat catcctcagg 1140

cctctctaca agcagcagga aacatagaac tcagagccag atcccttatc caactctcga 1200

cttttccttg gtctccagtg gaagggaaaa gcccatgatc ttcaagcagg gaagccccag 1260

tgagtagctg cattcctaga aattgaagtt tcagagctac acaaacactt tttctgtccc 1320

aaccgttccc tcacagcaaa gcaacaatac aggctaggga tggtaatcct ttaaacatac 1380

aaaaattgct cgtgttataa attacccagt ttagagggga aaaaaaaaca attattccta 1440

aataaatgga taagtagaat taatggttga ggcaggacca tacagagtgt gggaactgct 1500

ggggatctag ggaattcagt gggaccaatg aaagcatggc tgagaaatag caggtagtcc 1560

aggatagtct aagggaggtg ttcccatctg agcccagaga taagggtgtc ttcctagaac 1620

attagccgta gtggaattaa caggaaatca tgagggtgac gtagaattga gtcttccagg 1680

ggactctatc agaactggac catctccaag tatataacga tgagtcctct taatgctagg 1740

agtagaaaat ggtcctagga aggggactga ggattgcggt ggggggtggg gtggaaaaga 1800

aagtacagaa caaaccctgt gtcactgtcc caagttgcta agtgaacaga actatctcag 1860

catcagaatg agaaagcctg agaagaaaga accaaccaca agcacacagg aaggaaagcg 1920

caggaggtga aaatgctttc ttggccaggg tagtaagaat tagaggttaa tgcagggact 1980

gtaaaaccac cttttctgct tcaatatcta attcctgtgt agctttgttc attgcattta 2040

ttaaacaaat gttgtataac caatactaaa tgtactactg agcttcgctg agttaagtta 2100

tgaaactttc aaatccttca tcatgtcagt tccaatgagg tggggatgga gaagacaatt 2160

gttgcttatg aaagaaagct ttagctgtct ctgttttgta agctttaagc gcaacatttc 2220

ttggttccaa taaagcattt tacaagatct tgcatgctac tcttagatag aagatgggaa 2280

aaccatggta ataaaatatg aatgataaaa aaaaaaaaaa aaaaaaaaaa aaaaaaaaaa 2340

aaaaaaaaaa aaaaaaaaaa aaaaaaaaaa aaaaaaaaaa aaaaaaaaaa aaaaaaaaaa 2400

aaaaaa 2406

<210> 88
<211> 290
<212> PRT
<213> Homo sapiens

<400> 88
Met Gly Gly Gly Ala Gly Glu Arg Leu Phe Thr Ser Ser Cys Leu Val
1               5                   10                  15

Gly Leu Val Pro Leu Gly Leu Arg Ile Ser Leu Val Thr Cys Pro Leu
            20                  25                  30

Gln Cys Gly Ile Met Trp Gln Leu Leu Leu Pro Thr Ala Leu Leu Leu
        35                  40                  45

Leu Val Ser Ala Gly Met Arg Thr Glu Asp Leu Pro Lys Ala Val Val
    50                  55                  60

Phe Leu Glu Pro Gln Trp Tyr Arg Val Leu Glu Lys Asp Ser Val Thr
65                  70                  75                  80

Leu Lys Cys Gln Gly Ala Tyr Ser Pro Glu Asp Asn Ser Thr Gln Trp
                85                  90                  95

Phe His Asn Glu Ser Leu Ile Ser Ser Gln Ala Ser Ser Tyr Phe Ile
            100                 105                 110

Asp Ala Ala Thr Val Asp Asp Ser Gly Glu Tyr Arg Cys Gln Thr Asn
        115                 120                 125

Leu Ser Thr Leu Ser Asp Pro Val Gln Leu Glu Val His Ile Gly Trp
    130                 135                 140

Leu Leu Leu Gln Ala Pro Arg Trp Val Phe Lys Glu Glu Asp Pro Ile
145                 150                 155                 160

His Leu Arg Cys His Ser Trp Lys Asn Thr Ala Leu His Lys Val Thr
                165                 170                 175

Tyr Leu Gln Asn Gly Lys Gly Arg Lys Tyr Phe His His Asn Ser Asp
            180                 185                 190

Phe Tyr Ile Pro Lys Ala Thr Leu Lys Asp Ser Gly Ser Tyr Phe Cys
        195                 200                 205

Arg Gly Leu Phe Gly Ser Lys Asn Val Ser Ser Glu Thr Val Asn Ile
    210                 215                 220

Thr Ile Thr Gln Gly Leu Ala Val Ser Thr Ile Ser Ser Phe Phe Pro
225                 230                 235                 240

Pro Gly Tyr Gln Val Ser Phe Cys Leu Val Met Val Leu Leu Phe Ala
                245                 250                 255

Val Asp Thr Gly Leu Tyr Phe Ser Val Lys Thr Asn Ile Arg Ser Ser
            260                 265                 270

Thr Arg Asp Trp Lys Asp His Lys Phe Lys Trp Arg Lys Asp Pro Gln
        275                 280                 285

Asp Lys
    290

<210> 89
<211> 7133
<212> DNA
<213> Homo sapiens

<400> 89
aaagtcagag tactgggaga acagaagact tcacaattta atgcctcagt ttttaaaaaa      60

ggatccttac acttcatgtc tcctagccat cagaagagga atgagacagc aaaagttcaa     120

atggcctgtt tcaagtttct gatataaaac gatgacattt tcaggaaaat cctgcatttc     180

cagagagaga ctggctggtt aaatttctga aagaggacac cagctaaaag aaggtattgc     240

atctcacccg agcagactgt gtctgtggaa agtgtaagcc ccttgccaga agagcagctt     300

cccagcaaag gcagagggtg aaaacagcaa aggtcttaag acactgggga cctagagtca     360

aaagggacct cctccaggga aaacgctgtg tgagaaatgg cctcattcgg tgactgtgag     420

tgacacagca gaaagttggg tcattccggc tgcttttttg agaagtccct gaagagatca     480

ataacagcaa gagggaacct ggcaaggaag ctattcctat aatccaggaa agagatgagg     540

aaggcttgga ccaggtggta gtggtgtcag gtagtcaaat gctgggtata ttttgaagat     600

acaccccata ggatttgctc cacattgaat gtggaatgct ggaagagaga taaagtgtac     660

ctgtcacata ctttttgagt tttatttatt ttcttagaag taagtacaca aagagatgct     720

acctaggaga agggtattct tttcactatt ctttcaaatt ttctgtatgt tcgaacattt     780

tcatagtaga aagttggggg gaaaatctgt ttcataaaca tttcctcagc agcagtccag     840

tctattgcat tttaattggt tgtgatatca ttgttttatg caatacgttc tcaacaagta     900

tatcctccgg caaactgaac aaggaccaag tctgttctgc ctacagctct gcttcctcat     960

agctgctttc cagaacgtga ctcttgcaaa ttatcaagaa aggggaacta atctaaggga    1020

tccagatcaa acagcctcat gaagacttat tttatgtttc taatataaag atagaagttt    1080

tcagaaaagc cctgctacac agaggatcag agcaggggtg ggcctgctgg gctgcagctg    1140

ggattctgag catcctttcc cggaggcacg gaaagtgagt gagtgagccc agtgaggaag    1200

aagttgaagc tttgatatga gtaaacaagt atctctacct gaaatgatta aagactggac 1260

caaagagcat gtgaaaaaat gggtaaatga agaccttaag attaatgagc aatacgggca 1320

aattctgctc agtgaagaag taacaggatt agtcctgcag gaattaactg agaaggacct 1380

tgtagaaatg gggctaccat ggggtccagc acttttgata aaacgttcat acaacaaatt 1440

gaatagtaag tcccctgaaa gtgacaatca tgatccggga caattagata attcaaaacc 1500

gtccaaaaca gaacaccaga aaaatccaaa acacaccaaa aaggaagaag aaaattcaat 1560

gtcatctaat attgattatg atcccagaga gatcagagat atcaaacaag aagaatcaat 1620

tcttatgaaa gaaaatgtgt tagatgaagt agcaaatgct aaacacaaga aaaagggtaa 1680

gctaaaacct gaacaattga cttgtatgcc atatcctttt gatcagttcc atgacagcca 1740

tcgctacata gaacattata ctctacaacc tgaaacagga gcactcaatc tcattgatcc 1800

aatacatgag ttcaaagctc tcacaaacac agaaacagcc acggaagtgg acattaagat 1860

gaaattcagc aatgaagtct tccgatttgc atcagcttgt atgaattcac gcaccaatgg 1920

caccatccat tttggagtca aggacaaacc ccatggagaa attgttggtg tgaaaatcac 1980

cagtaaggct gccttcattg accacttcaa tgtaatgatc aaaaagtatt ttgaagaaag 2040

tgagatcaat gaagccaaga agtgtattcg ggagccaagg tttgtggaag tccttctgca 2100

gaacaataca ccatctgaca gatttgtcat tgaagttgat actattccaa aacactctat 2160

atgtaatgat aagtatttct acattcagat gcaaatttgt aaagataaaa tatggaaaca 2220

aaaccaaaat ctttcactgt ttgtaagaga aggggctagc tctagggata tcctggccaa 2280

ttccaagcaa cgggatgtag atttcaaggc atttttacaa aatttaaagt cactggtagc 2340

atctagaaaa gaggctgaag aagagtatgg aatgaaggca atgaagaagg agagtgaagg 2400

actaaagctg gttaaacttc tcataggaaa ccgagactca ctggataatt catactatga 2460

ctggtacatt cttgtaacaa ataaatgcca tccaaaccaa ataaagcact tagatttttt 2520

aaaagaaatt aaatggtttg ctgtgttgga gtttgatcct gaatctatga tcaatggagt 2580

ggtcaaagct tacaaagaaa gtcgggtggc aaaccttcac tttccaaatc aatatgaaga 2640

caagacaact aacatgtggg agaagatttc tactcttaat ctttaccaac agcccagctg 2700

gattttctgc aacggcagat cagacctgaa aagcgagaca tataaacctc tagaaccaca 2760

tttatggcag agagaaagag cttcagaagt caggaaacta attttatttc tcacagatga 2820

aaatataatg acaagaggaa aatttttggt agtgtttcta ttactctctt cagtggaaag 2880

cccaggagat ccactcattg aaactttctg ggctttctat caagctctca aaggaatgga 2940

aaatatgttg tgtatctctg taaactcaca tatttatcaa cgatggaaag atctactaca 3000

aacaagaatg aagatggaag atgaactaac aaaccacagt atttccactt taaatataga 3060

actggtaaac agcactatcc ttaaactaaa atcggtgact cggtcatcaa gaaggttttt 3120

gcccgcccgt ggatcttctt cagttatcct agagaaaaag aaagaggatg tcttgactgc 3180

actggaaatc ctctgtgaaa atgagtgtac agagacagac atcgagaaag acaaatctaa 3240

attcctggag tttaagaaat caaaagaaga acacttttat cgaggtggca aagtatcctg 3300

gtggaacttc tatttttctt ctgaaaacta ttcttcagat tttgttaaaa gggacagtta 3360

tgaaaagctt aaagatttaa tacactgctg ggcagagtct cctaaaccaa tatttgcaaa 3420

aatcatcaat ctttatcatc atccaggctg tggaggtacc acactggcta tgcatgttct 3480

ctgggactta aagaaaaact tcagatgtgc tgtgttaaaa aacaagacaa ctgattttgc 3540

agaaattgca gagcaagtga tcaatctggt cacctatagg gcaaagagcc atcaggatta 3600

cattcctgtg cttctccttg tggatgattt tgaagaacaa gaaaatgtct actttctaca 3660

aaatgccatc cattccgttt tagcagaaaa ggatttgcga tatgaaaaaa cattggtaat 3720

tatcttaaac tgcatgagat cccggaatcc agatgaaagt gcaaaattgg cagacagtat 3780

tgcactaaat taccaacttt cttccaagga acaaagagct tttggtgcca aactgaagga 3840

aattgaaaag cagcacaaga actgtgaaaa cttttattcc ttcatgatca tgaaaagcaa 3900

ttttgatgaa acatatatag aaaatgtagt caggaatatc ctaaaaggac aggatgttga 3960

cagcaaggaa gcacaactca tttccttcct ggctttactc agctcttatg ttactgactc 4020

tacaatttca gtttcacagt gtgaaatatt tttgggaatc atatacacta gtacaccctg 4080

ggaacctgaa agcttagaag acaagatggg aacttattct acacttctaa taaaaacaga 4140

agttgcagaa tatgggagat acacaggtgt gcgtatcatt caccctctga ttgccctgta 4200

ctgtctaaaa gaactggaaa gaagctatca cttggataaa tgtcaaattg cattgaatat 4260

attagaagag aatttattct atgattctgg aataggaaga gacaaatttc aacatgatgt 4320

tcaaactctt ctgcttacaa gacagcgcaa ggtgtatgga gatgaaacag acactctgtt 4380

ttccccatta atggaagctt tacagaataa agacattgaa aaggtcttga gtgcaggaag 4440

tagacgattc ccacaaaatg cattcatttg tcaagcctta gcaagacatt tctacattaa 4500

agagaaggac tttaacacag ctctggactg ggcacgtcag gccaaaatga aagcacctaa 4560

aaattcctat atttcagata cactaggtca agtctacaaa agtgaaatca aatggtggtt 4620

ggatgggaac aaaaactgta ggagcattac tgttaatgac ctaacacatc tcctagaagc 4680

tgcggaaaaa gcctcaagag ctttcaaaga atcccaaagg caaactgata gtaaaaacta 4740

tgaaaccgag aactggtcac cacagaagtc ccagagacga tatgacatgt ataacacagc 4800

ttgtttcttg ggtgaaatag aagttggtct ttacactatc cagattcttc agctcactcc 4860

ctttttccac aaagaaaatg aattatccaa aaaacatatg gtgcaatttt tatcaggaaa 4920

gtggaccatt cctcctgatc ccagaaatga atgttatttg gctcttagca agttcacatc 4980

ccacctaaaa aatttacaat cagatctgaa aaggtgcttt gactttttta ttgattatat 5040

ggttcttctg aaaatgaggt atacccaaaa agaaattgca gaaatcatgt taagcaagaa 5100

agtcagtcgt tgtttcagga aatacacaga acttttctgt catttggatc catgtctatt 5160

acaaagtaaa gagagtcaat tactccagga ggagaattgc aggaaaaagc tagaagctct 5220

gagagcagat aggtttgctg gactcttgga atatcttaat ccaaactaca aagatgctac 5280

caccatggaa agtatagtga atgaatatgc cttcctactg cagcaaaact caaaaaagcc 5340

catgacaaat gagaaacaaa attccatttt ggccaacatt attctgagtt gtctaaagcc 5400

caactccaag ttaattcaac cacttaccac gctaaaaaaa caactccgag aggtcttgca 5460

atttgtagga ctaagtcatc aatatccagg tccttatttc ttggcctgcc tcctgttctg 5520

gccagaaaat caagagctag atcaagattc caaactaata gaaaagtatg tttcatcctt 5580

aaatagatcc ttcaggggac agtacaagcg catgtgcagg tccaagcagg caagcacact 5640

tttctatctg ggcaaaagga agggtctaaa cagtattgtt cacaaggcca aaatagagca 5700

gtactttgat aaagcacaaa atacaaattc cctctggcac agtggggatg tgtggaaaaa 5760

aaatgaagtc aaagacctcc tgcgtcgtct aactggtcag gctgaaggca agctaatctc 5820

tgtagaatat ggaacagagg aaaaaataaa aataccagta atatctgttt attcaggtcc 5880

actcagaagt ggtaggaaca tagaaagagt gtctttctac ctaggatttt ccattgaagg 5940

ccctctggca tatgatatag aagtaattta agacaataca tcacctgtag ttcaaatacg 6000

tttatttata tctttatgat tttattctct ctctctattc tcatggcact ttcataacat 6060

tatggctaac ctctaattac agattttgct tttgcctccc tgaatgaatt acaagccttt 6120

ttaagatatg aaatatgcct acccgcagag cttggcacaa agtggagtca atcttttaat 6180

gttttaaata tgcattttca gactcaaata attaagaagt ttcattgata tccactggtc 6240

acatcataac tgtctatagg gcaataaaat ctgtgttaaa ctcaattgct tttataagtt 6300

ttctaaatta tttcttcact gtgacagcaa agatttaaat aagatgaatg taaaagagaa 6360

agcttattgg actcaaaccc acagatccac accagagttc tatttacctc atcttggtat 6420

caataaaaac ttatgtggaa ggtaaatata ttgttcccca tccaccacat aacactctcc 6480

ccaacacaca cacacacaca cacacacaca cacacacaca cacacactcc ttgtacccct 6540

tgcccttctc ccagctcatt gctccaggag agagaagagt tcaaaaaata aagtaatcat 6600

aaacttgaac tctctccatt ctcttgttcc catttacagg tgaatctctt cctttaagcc 6660

atttttgtct cctgtgaata cagccttatc tccacctgtt tcttagatcc catctcccct   6720

ggcttatttt ttccattcat taccctcttt gttcccttta cttctcaacc tgtgctatat   6780

acatgctgtt ctctctgttg agattgcctt atttccatct aacattctct ctcctgctat   6840

tctgatttgt cattcacaac tgatttcaag agtcaccttc accaggaagt cttccttgac   6900

caccatcatt cctgcctgat tagagggctt cctcatggta atatgtgttc tcaagttttc   6960

agtgtcaagg aatgccatcc cagaagctca ttctcagatg cacaacagcc agaacagtct   7020

caagcagcat tctagagctt ggaatttaag aactacgcat tgcctataaa gtgaaacata   7080

ggctaatata gattaaattg aatattgaat aaaaaatata tttatttatc cac          7133

<210> 90
<211> 1584
<212> PRT
<213> Homo sapiens

<400> 90

<210> 91
<211> 2387
<212> DNA
<213> Homo sapiens

<400> 91
agacacctct gccctcacca tgagcctctg gcagcccctg gtcctggtgc tcctggtgct     60

gggctgctgc tttgctgccc ccagacagcg ccagtccacc cttgtgctct tccctggaga    120

cctgagaacc aatctcaccg acaggcagct ggcagaggaa tacctgtacc gctatggtta    180

cactcgggtg gcagagatgc gtggagagtc gaaatctctg gggcctgcgc tgctgcttct    240

ccagaagcaa ctgtccctgc ccgagaccgg tgagctggat agcgccacgc tgaaggccat    300

gcgaacccca cggtgcgggg tcccagacct gggcagattc caaacctttg agggcgacct    360

caagtggcac caccacaaca tcacctattg gatccaaaac tactcggaag acttgccgcg    420

ggcggtgatt gacgacgcct ttgcccgcgc cttcgcactg tggagcgcgg tgacgccgct    480

caccttcact cgcgtgtaca gccgggacgc agacatcgtc atccagtttg gtgtcgcgga    540

gcacggagac gggtatccct tcgacgggaa ggacgggctc ctggcacacg cctttcctcc    600

tggccccggc attcagggag acgcccattt cgacgatgac gagttgtggt ccctgggcaa    660

gggcgtcgtg gttccaactc ggtttggaaa cgcagatggc gcggcctgcc acttcccctt    720

catcttcgag ggccgctcct actctgcctg caccaccgac ggtcgctccg acggcttgcc    780

ctggtgcagt accacggcca actacgacac cgacgaccgg tttggcttct gccccagcga    840

gagactctac acccaggacg gcaatgctga tgggaaaccc tgccagtttc cattcatctt    900

ccaaggccaa tcctactccg cctgcaccac ggacggtcgc tccgacggct accgctggtg    960

cgccaccacc gccaactacg accgggacaa gctcttcggc ttctgcccga cccgagctga   1020

ctcgacggtg atggggggca actcggcggg ggagctgtgc gtcttcccct tcactttcct   1080

gggtaaggag tactcgacct gtaccagcga gggccgcgga gatgggcgcc tctggtgcgc   1140

taccacctcg aactttgaca gcgacaagaa gtggggcttc tgcccggacc aaggatacag   1200

tttgttcctc gtggcggcgc atgagttcgg ccacgcgctg ggcttagatc attcctcagt   1260

gccggaggcg ctcatgtacc ctatgtaccg cttcactgag gggcccccct tgcataagga   1320

cgacgtgaat ggcatccggc acctctatgg tcctcgccct gaacctgagc cacggcctcc   1380

aaccaccacc acaccgcagc ccacggctcc cccgacggtc tgccccaccg gaccccccac   1440

tgtccacccc tcagagcgcc ccacagctgg ccccacaggt cccccctcag ctggccccac   1500

aggtcccccc actgctggcc cttctacggc cactactgtg cctttgagtc cggtggacga   1560

tgcctgcaac gtgaacatct tcgacgccat cgcggagatt gggaaccagc tgtatttgtt    1620

caaggatggg aagtactggc gattctctga gggcaggggg agccggccgc agggcccctt    1680

ccttatcgcc gacaagtggc ccgcgctgcc ccgcaagctg gactcggtct ttgaggagcg    1740

gctctccaag aagcttttct tcttctctgg gcgccaggtg tgggtgtaca caggcgcgtc    1800

ggtgctgggc ccgaggcgtc tggacaagct gggcctggga gccgacgtgg cccaggtgac    1860

cggggccctc cggagtggca gggggaagat gctgctgttc agcgggcggc gcctctggag    1920

gttcgacgtg aaggcgcaga tggtggatcc ccggagcgcc agcgaggtgg accggatgtt    1980

ccccggggtg cctttggaca cgcacgacgt cttccagtac cgagagaaag cctatttctg    2040

ccaggaccgc ttctactggc gcgtgagttc ccggagtgag ttgaaccagg tggaccaagt    2100

gggctacgtg acctatgaca tcctgcagtg ccctgaggac tagggctccc gtcctgcttt    2160

ggcagtgcca tgtaaatccc cactgggacc aaccctgggg aaggagccag tttgccggat    2220

acaaactggt attctgttct ggaggaaagg gaggagtgga ggtgggctgg gccctctctt    2280

ctcacctttg ttttttgttg gagtgtttct aataaacttg gattctctaa cctttaaaaa    2340

aaaaaaaaaa aaaaaaaaaa aaaaaaaaaa aaaaaaaaaa aaaaaaa                2387

<210> 92
<211> 707
<212> PRT
<213> Homo sapiens

<400> 92

<210> 93
<211> 1147
<212> DNA
<213> Homo sapiens

<400> 93
accaaatcaa ccataggtcc aagaacaatt gtctctggac ggcagctatg cgactcaccg      60

tgctgtgtgc tgtgtgcctg ctgcctggca gcctggccct gccgctgcct caggaggcgg     120

gaggcatgag tgagctacag tgggaacagg ctcaggacta tctcaagaga ttttatctct     180

atgactcaga aacaaaaaat gccaacagtt tagaagccaa actcaaggag atgcaaaaat     240

tctttggcct acctataact ggaatgttaa actcccgcgt catagaaata atgcagaagc     300

ccagatgtgg agtgccagat gttgcagaat actcactatt tccaaatagc ccaaaatgga     360

cttccaaagt ggtcacctac aggatcgtat catatactcg agacttaccg catattacag     420

tggatcgatt agtgtcaaag gctttaaaca tgtggggcaa agagatcccc ctgcatttca     480

ggaaagttgt atggggaact gctgacatca tgattggctt tgcgcgagga gctcatgggg     540

actcctaccc atttgatggg ccaggaaaca cgctggctca tgcctttgcg cctgggacag     600

gtctcggagg agatgctcac ttcgatgagg atgaacgctg gacggatggt agcagtctag     660

ggattaactt cctgtatgct gcaactcatg aacttggcca ttctttgggt atgggacatt 720

cctctgatcc taatgcagtg atgtatccaa cctatggaaa tggagatccc caaaatttta 780

aactttccca ggatgatatt aaaggcattc agaaactata tggaaagaga agtaattcaa 840

gaaagaaata gaaacttcag gcagaacatc cattcattca ttcattggat tgtatatcat 900

tgttgcacaa tcagaattga taagcactgt tcctccactc catttagcaa ttatgtcacc 960

cttttttatt gcagttggtt tttgaatgtc tttcactcct tttaaggata aactccttta 1020

tggtgtgact gtgtcttatt catctatact tgcagtgggt agatgtcaat aaatgttaca 1080

tacacaaata aataaaatgt ttattccatg gtaaatttaa aaaaaaaaaa aaaaaaaaaa 1140

aaaaaaa 1147

<210> 94
<211> 267
<212> PRT
<213> Homo sapiens

<400> 94
Met Arg Leu Thr Val Leu Cys Ala Val Cys Leu Leu Pro Gly Ser Leu
1               5                   10                  15

Ala Leu Pro Leu Pro Gln Glu Ala Gly Gly Met Ser Glu Leu Gln Trp
            20                  25                  30

Glu Gln Ala Gln Asp Tyr Leu Lys Arg Phe Tyr Leu Tyr Asp Ser Glu
        35                  40                  45

Thr Lys Asn Ala Asn Ser Leu Glu Ala Lys Leu Lys Glu Met Gln Lys
    50                  55                  60

Phe Phe Gly Leu Pro Ile Thr Gly Met Leu Asn Ser Arg Val Ile Glu
65                  70                  75                  80

Ile Met Gln Lys Pro Arg Cys Gly Val Pro Asp Val Ala Glu Tyr Ser
                85                  90                  95

Leu Phe Pro Asn Ser Pro Lys Trp Thr Ser Lys Val Val Thr Tyr Arg
            100                 105                 110

Ile Val Ser Tyr Thr Arg Asp Leu Pro His Ile Thr Val Asp Arg Leu
        115                 120                 125

Val Ser Lys Ala Leu Asn Met Trp Gly Lys Glu Ile Pro Leu His Phe
    130                 135                 140

Arg Lys Val Val Trp Gly Thr Ala Asp Ile Met Ile Gly Phe Ala Arg
145                 150                 155                 160

Gly Ala His Gly Asp Ser Tyr Pro Phe Asp Gly Pro Gly Asn Thr Leu
                165                 170                 175

Ala His Ala Phe Ala Pro Gly Thr Gly Leu Gly Gly Asp Ala His Phe
            180                 185                 190

Asp Glu Asp Glu Arg Trp Thr Asp Gly Ser Ser Leu Gly Ile Asn Phe
        195                 200                 205

Leu Tyr Ala Ala Thr His Glu Leu Gly His Ser Leu Gly Met Gly His
    210                 215                 220

Ser Ser Asp Pro Asn Ala Val Met Tyr Pro Thr Tyr Gly Asn Gly Asp
225                 230                 235                 240

Pro Gln Asn Phe Lys Leu Ser Gln Asp Asp Ile Lys Gly Ile Gln Lys
                245                 250                 255

Leu Tyr Gly Lys Arg Ser Asn Ser Arg Lys Lys
            260                 265

<210> 95
<211> 2574
<212> DNA
<213> Homo sapiens

<400> 95
ttggatgttc cgggaaagtg atgtgggtag gacaggcggg gcgagccgca ggtgccagaa      60

cacagattgt ataaaaggct gggggctggt ggggagcagg ggaagggaat gtgaccaggt     120

ctaggtctgg agtttcagct tggacactga gccaagcaga caagcaaagc aagccaggac     180

acaccatcct gccccaggcc cagcttctct cctgccttcc aacgccatgg ggagcaatct     240

cagcccccaa ctctgcctga tgccctttat cttgggcctc ttgtctggag gtgtgaccac     300

cactccatgg tctttggccc ggccccaggg atcctgctct ctggaggggg tagagatcaa     360

aggcggctcc ttccgacttc tccaagaggg ccaggcactg gagtacgtgt gtccttctgg     420

cttctacccg taccctgtgc agacacgtac ctgcagatct acggggtcct ggagcaccct     480

gaagactcaa gaccaaaaga ctgtcaggaa ggcagagtgc agagcaatcc actgtccaag     540

accacacgac ttcgagaacg gggaatactg gccccggtct ccctactaca atgtgagtga     600

tgagatctct ttccactgct atgacggtta cactctccgg ggctctgcca atcgcacctg     660

ccaagtgaat ggccgatgga gtgggcagac agcgatctgt gacaacggag cggggtactg     720

ctccaacccg ggcatcccca ttggcacaag gaaggtgggc agccagtacc gccttgaaga 780

cagcgtcacc taccactgca gccgggggct taccctgcgt ggctcccagc ggcgaacgtg 840

tcaggaaggt ggctcttgga gcgggacgga gccttcctgc caagactcct tcatgtacga 900

cacccctcaa gaggtggccg aagctttcct gtcttccctg acagagacca tagaaggagt 960

cgatgctgag gatgggcacg gcccagggga acaacagaag cggaagatcg tcctggaccc 1020

ttcaggctcc atgaacatct acctggtgct agatggatca gacagcattg gggccagcaa 1080

cttcacagga gccaaaaagt gtctagtcaa cttaattgag aaggtggcaa gttatggtgt 1140

gaagccaaga tatggtctag tgacatatgc cacatacccc aaaatttggg tcaaagtgtc 1200

tgaagcagac agcagtaatg cagactgggt cacgaagcag ctcaatgaaa tcaattatga 1260

agaccacaag ttgaagtcag ggactaacac caagaaggcc ctccaggcag tgtacagcat 1320

gatgagctgg ccagatgacg tccctcctga aggctggaac cgcacccgcc atgtcatcat 1380

cctcatgact gatggattgc acaacatggg cggggaccca attactgtca ttgatgagat 1440

ccgggacttg ctatacattg gcaaggatcg caaaaaccca agggaggatt atctggatgt 1500

ctatgtgttt ggggtcgggc ctttggtgaa ccaagtgaac atcaatgctt tggcttccaa 1560

gaaagacaat gagcaacatg tgttcaaagt caaggatatg gaaaacctgg aagatgtttt 1620

ctaccaaatg atcgatgaaa gccagtctct gagtctctgt ggcatggttt gggaacacag 1680

gaagggtacc gattaccaca agcaaccatg gcaggccaag atctcagtca ttcgcccttc 1740

aaagggacac gagagctgta tgggggctgt ggtgtctgag tactttgtgc tgacagcagc 1800

acattgtttc actgtggatg acaaggaaca ctcaatcaag gtcagcgtag gaggggagaa 1860

gcgggacctg gagatagaag tagtcctatt tcaccccaac tacaacatta atgggaaaaa 1920

agaagcagga attcctgaat tttatgacta tgacgttgcc ctgatcaagc tcaagaataa 1980

gctgaaatat ggccagacta tcaggcccat ttgtctcccc tgcaccgagg gaacaactcg 2040

agctttgagg cttcctccaa ctaccacttg ccagcaacaa aaggaagagc tgctccctgc 2100

acaggatatc aaagctctgt ttgtgtctga ggaggagaaa aagctgactc ggaaggaggt 2160

ctacatcaag aatggggata agaaaggcag ctgtgagaga gatgctcaat atgccccagg 2220

ctatgacaaa gtcaaggaca tctcagaggt ggtcacccct cggttccttt gtactggagg 2280

agtgagtccc tatgctgacc ccaatacttg cagaggtgat tctggcggcc ccttgatagt 2340

tcacaagaga agtcgtttca ttcaagttgg tgtaatcagc tggggagtag tggatgtctg 2400

caaaaaccag aagcggcaaa agcaggtacc tgctcacgcc cgagactttc acatcaacct 2460

ctttcaagtg ctgccctggc tgaaggagaa actccaagat gaggatttgg gttttctata 2520

aggggtttcc tgctggacag gggcgtggga ttgaattaaa acagctgcga caac        2574

<210> 96
<211> 764
<212> PRT
<213> Homo sapiens

<400> 96
Met Gly Ser Asn Leu Ser Pro Gln Leu Cys Leu Met Pro Phe Ile Leu
1               5                   10                  15

Gly Leu Leu Ser Gly Gly Val Thr Thr Thr Pro Trp Ser Leu Ala Arg
            20                  25                  30

Pro Gln Gly Ser Cys Ser Leu Glu Gly Val Glu Ile Lys Gly Gly Ser
        35                  40                  45

Phe Arg Leu Leu Gln Glu Gly Gln Ala Leu Glu Tyr Val Cys Pro Ser
    50                  55                  60

Gly Phe Tyr Pro Tyr Pro Val Gln Thr Arg Thr Cys Arg Ser Thr Gly
65                  70                  75                  80

Ser Trp Ser Thr Leu Lys Thr Gln Asp Gln Lys Thr Val Arg Lys Ala
                85                  90                  95

Glu Cys Arg Ala Ile His Cys Pro Arg Pro His Asp Phe Glu Asn Gly
            100                 105                 110

Glu Tyr Trp Pro Arg Ser Pro Tyr Tyr Asn Val Ser Asp Glu Ile Ser
        115                 120                 125

Phe His Cys Tyr Asp Gly Tyr Thr Leu Arg Gly Ser Ala Asn Arg Thr
    130                 135                 140

Cys Gln Val Asn Gly Arg Trp Ser Gly Gln Thr Ala Ile Cys Asp Asn
145                 150                 155                 160

Gly Ala Gly Tyr Cys Ser Asn Pro Gly Ile Pro Ile Gly Thr Arg Lys
                165                 170                 175

Val Gly Ser Gln Tyr Arg Leu Glu Asp Ser Val Thr Tyr His Cys Ser
            180                 185                 190

Arg Gly Leu Thr Leu Arg Gly Ser Gln Arg Arg Thr Cys Gln Glu Gly
        195                 200                 205

Gly Ser Trp Ser Gly Thr Glu Pro Ser Cys Gln Asp Ser Phe Met Tyr
    210                 215                 220

Asp Thr Pro Gln Glu Val Ala Glu Ala Phe Leu Ser Ser Leu Thr Glu
225                 230                 235                 240

Thr Ile Glu Gly Val Asp Ala Glu Asp Gly His Gly Pro Gly Glu Gln
                245                 250                 255

Gln Lys Arg Lys Ile Val Leu Asp Pro Ser Gly Ser Met Asn Ile Tyr
            260                 265                 270

Leu Val Leu Asp Gly Ser Asp Ser Ile Gly Ala Ser Asn Phe Thr Gly
        275                 280                 285

Ala Lys Lys Cys Leu Val Asn Leu Ile Glu Lys Val Ala Ser Tyr Gly
    290                 295                 300

Val Lys Pro Arg Tyr Gly Leu Val Thr Tyr Ala Thr Tyr Pro Lys Ile
305                 310                 315                 320

Trp Val Lys Val Ser Glu Ala Asp Ser Ser Asn Ala Asp Trp Val Thr
                325                 330                 335

Lys Gln Leu Asn Glu Ile Asn Tyr Glu Asp His Lys Leu Lys Ser Gly
            340                 345                 350

Thr Asn Thr Lys Lys Ala Leu Gln Ala Val Tyr Ser Met Met Ser Trp
        355                 360                 365

Pro Asp Asp Val Pro Pro Glu Gly Trp Asn Arg Thr Arg His Val Ile
    370                 375                 380

Ile Leu Met Thr Asp Gly Leu His Asn Met Gly Gly Asp Pro Ile Thr
385                 390                 395                 400

Val Ile Asp Glu Ile Arg Asp Leu Leu Tyr Ile Gly Lys Asp Arg Lys
                405                 410                 415

Asn Pro Arg Glu Asp Tyr Leu Asp Val Tyr Val Phe Gly Val Gly Pro
            420                 425                 430

Leu Val Asn Gln Val Asn Ile Asn Ala Leu Ala Ser Lys Lys Asp Asn
        435                 440                 445

Glu Gln His Val Phe Lys Val Lys Asp Met Glu Asn Leu Glu Asp Val

450                 455                 460

Phe Tyr Gln Met Ile Asp Glu Ser Gln Ser Leu Ser Leu Cys Gly Met
465                 470                 475                 480

Val Trp Glu His Arg Lys Gly Thr Asp Tyr His Lys Gln Pro Trp Gln
                485                 490                 495

Ala Lys Ile Ser Val Ile Arg Pro Ser Lys Gly His Glu Ser Cys Met
            500                 505                 510

Gly Ala Val Val Ser Glu Tyr Phe Val Leu Thr Ala Ala His Cys Phe
        515                 520                 525

Thr Val Asp Asp Lys Glu His Ser Ile Lys Val Ser Val Gly Gly Glu
    530                 535                 540

Lys Arg Asp Leu Glu Ile Glu Val Val Leu Phe His Pro Asn Tyr Asn
545                 550                 555                 560

Ile Asn Gly Lys Lys Glu Ala Gly Ile Pro Glu Phe Tyr Asp Tyr Asp
                565                 570                 575

Val Ala Leu Ile Lys Leu Lys Asn Lys Leu Lys Tyr Gly Gln Thr Ile
            580                 585                 590

Arg Pro Ile Cys Leu Pro Cys Thr Glu Gly Thr Thr Arg Ala Leu Arg
        595                 600                 605

Leu Pro Pro Thr Thr Thr Cys Gln Gln Gln Lys Glu Glu Leu Leu Pro
    610                 615                 620

Ala Gln Asp Ile Lys Ala Leu Phe Val Ser Glu Glu Glu Lys Lys Leu
625                 630                 635                 640

Thr Arg Lys Glu Val Tyr Ile Lys Asn Gly Asp Lys Lys Gly Ser Cys
                645                 650                 655

Glu Arg Asp Ala Gln Tyr Ala Pro Gly Tyr Asp Lys Val Lys Asp Ile
            660                 665                 670

Ser Glu Val Val Thr Pro Arg Phe Leu Cys Thr Gly Gly Val Ser Pro
        675                 680                 685

Tyr Ala Asp Pro Asn Thr Cys Arg Gly Asp Ser Gly Gly Pro Leu Ile
    690                 695                 700

Val His Lys Arg Ser Arg Phe Ile Gln Val Gly Val Ile Ser Trp Gly
705                 710                 715                 720

Val Val Asp Val Cys Lys Asn Gln Lys Arg Gln Lys Gln Val Pro Ala
                725                 730                 735

His Ala Arg Asp Phe His Ile Asn Leu Phe Gln Val Leu Pro Trp Leu
            740                 745                 750

Lys Glu Lys Leu Gln Asp Glu Asp Leu Gly Phe Leu
        755                 760

<210> 97
<211> 510
<212> DNA
<213> Homo sapiens

<400> 97
tgaggctgcc ttataaagca ccaagaggct gccagtggga cattttctcg gccctgccag      60

cccccaggag gaaggtgggt ctgaatctag caccatgacg gaactagaga cagccatggg     120

catgatcata gacgtctttt cccgatattc gggcagcgag ggcagcacgc agaccctgac     180

caagggggag ctcaaggtgc tgatggagaa ggagctacca ggcttcctgc agagtggaaa     240

agacaaggat gccgtggata aattgctcaa ggacctggac gccaatggag atgcccaggt     300

ggacttcagt gagttcatcg tgttcgtggc tgcaatcacg tctgcctgtc acaagtactt     360

tgagaaggca ggactcaaat gatgccctgg agatgtcaca gattcctggc agagccatgg     420

tcccaggctt cccaaaagtg tttgttggca attattcccc taggctgagc ctgctcatgt     480

acctctgatt aataaatgct tatgaaatga                                      510

<210> 98
<211> 95
<212> PRT
<213> Homo sapiens

<400> 98
Met Thr Glu Leu Glu Thr Ala Met Gly Met Ile Ile Asp Val Phe Ser
1               5                   10                  15

Arg Tyr Ser Gly Ser Glu Gly Ser Thr Gln Thr Leu Thr Lys Gly Glu
            20                  25                  30

Leu Lys Val Leu Met Glu Lys Glu Leu Pro Gly Phe Leu Gln Ser Gly
        35                  40                  45

Lys Asp Lys Asp Ala Val Asp Lys Leu Leu Lys Asp Leu Asp Ala Asn
    50                  55                  60

Gly Asp Ala Gln Val Asp Phe Ser Glu Phe Ile Val Phe Val Ala Ala
65                  70                  75                  80

Ile Thr Ser Ala Cys His Lys Tyr Phe Glu Lys Ala Gly Leu Lys
                85                  90                  95

<210> 99
<211> 1103
<212> DNA
<213> Homo sapiens

<400> 99
cacagagccc gggccgcagg cacctcctcg ccagctcttc cgctcctctc acagccgcca      60

gacccgcctg ctgagcccca tggcccgcgc tgctctctcc gccgccccca gcaatccccg     120

gctcctgcga gtggcactgc tgctcctgct cctggtagcc gctggccggc gcgcagcagg     180

agcgtccgtg gccactgaac tgcgctgcca gtgcttgcag accctgcagg gaattcaccc     240

caagaacatc caaagtgtga acgtgaagtc ccccggaccc cactgcgccc aaaccgaagt     300

catagccaca ctcaagaatg ggcggaaagc ttgcctcaat cctgcatccc ccatagttaa     360

gaaaatcatc gaaaagatgc tgaacagtga caaatccaac tgaccagaag ggaggaggaa     420

gctcactggt ggctgttcct gaaggaggcc ctgcccttat aggaacagaa gaggaaagag     480

agacacagct gcagaggcca cctggattgt gcctaatgtg tttgagcatc gcttaggaga     540

agtcttctat ttatttattt attcattagt tttgaagatt ctatgttaat attttaggtg     600

taaaataatt aagggtatga ttaactctac ctgcacactg tcctattata ttcattcttt     660

ttgaaatgtc aaccccaagt tagttcaatc tggattcata tttaatttga aggtagaatg     720

ttttcaaatg ttctccagtc attatgttaa tatttctgag gagcctgcaa catgccagcc     780

actgtgatag aggctggcgg atccaagcaa atggccaatg agatcattgt gaaggcaggg     840

gaatgtatgt gcacatctgt tttgtaactg tttagatgaa tgtcagttgt tatttattga     900

aatgatttca cagtgtgtgg tcaacatttc tcatgttgaa actttaagaa ctaaaatgtt     960

ctaaatatcc cttggacatt ttatgtcttt cttgtaaggc atactgcctt gtttaatggt    1020

agttttacag tgtttctggc ttagaacaaa ggggcttaat tattgatgtt ttcatagaga    1080

atataaaaat aaagcactta tag                                            1103

<210> 100
<211> 107
<212> PRT
<213> Homo sapiens

<400> 100
Met Ala Arg Ala Ala Leu Ser Ala Ala Pro Ser Asn Pro Arg Leu Leu
1               5                   10                  15

Arg Val Ala Leu Leu Leu Leu Leu Leu Val Ala Ala Gly Arg Arg Ala
            20                  25                  30

Ala Gly Ala Ser Val Ala Thr Glu Leu Arg Cys Gln Cys Leu Gln Thr
        35                  40                  45

Leu Gln Gly Ile His Pro Lys Asn Ile Gln Ser Val Asn Val Lys Ser
    50                  55                  60

Pro Gly Pro His Cys Ala Gln Thr Glu Val Ile Ala Thr Leu Lys Asn
65                  70                  75                  80

Gly Arg Lys Ala Cys Leu Asn Pro Ala Ser Pro Ile Val Lys Lys Ile
                85                  90                  95

Ile Glu Lys Met Leu Asn Ser Asp Lys Ser Asn
            100                 105

<210> 101
<211> 1572
<212> DNA
<213> Homo sapiens

<400> 101
aatttctcac tgcccctgtg ataaactgtg gtcactggct gtggcagcaa ctattataag      60

atgctctgaa aactcttcag acactgaggg gcaccagagg agcagactac aagaatggca     120

cacgctatgg aaaactcctg gacaatcagt aaagagtacc atattgatga agaagtgggc     180

tttgctctgc caaatccaca ggaaaatcta cctgattttt ataatgactg gatgttcatt     240

gctaaacatc tgcctgatct catagagtct ggccagcttc gagaaagagt tgagaagtta     300

aacatgctca gcattgatca tctcacagac cacaagtcac agcgccttgc acgtctagtt     360

ctgggatgca tcaccatggc atatgtgtgg ggcaaaggtc atggagatgt ccgtaaggtc     420

ttgccaagaa atattgctgt tccttactgc caactctcca agaaactgga actgcctcct     480

attttggttt atgcagactg tgtcttggca aactggaaga aaaaggatcc taataagccc     540

ctgacttatg agaacatgga cgttttgttc tcatttcgtg atggagactg cagtaaagga     600

ttcttcctgg tctctctatt ggtggaaata gcagctgctt ctgcaatcaa agtaattcct     660

actgtattca aggcaatgca aatgcaagaa cgggacactt tgctaaaggc gctgttggaa     720

atagcttctt gcttggagaa agcccttcaa gtgtttcacc aaatccacga tcatgtgaac     780

ccaaaagcat ttttcagtgt tcttcgcata tatttgtctg gctggaaagg caacccccag 840

ctatcagacg gtctggtgta tgaagggttc tgggaagacc caaaggagtt tgcagggggc 900

agtgcaggcc aaagcagcgt ctttcagtgc tttgacgtcc tgctgggcat ccagcagact 960

gctggtggag gacatgctgc tcagttcctc caggacatga gaagatatat gccaccagct 1020

cacaggaact tcctgtgctc attagagtca aatccctcag tccgtgagtt tgtcctttca 1080

aaaggtgatg ctggcctgcg ggaagcttat gacgcctgtg tgaaagctct ggtctccctg 1140

aggagctacc atctgcaaat cgtgactaag tacatcctga ttcctgcaag ccagcagcca 1200

aaggagaata agacctctga agacccttca aaactggaag ccaaaggaac tggaggcact 1260

gatttaatga atttcctgaa gactgtaaga agtacaactg agaaatccct tttgaaggaa 1320

ggttaatgta acccaacaag agcacatttt atcatagcag agacatctgt atgcattcct 1380

gtcattaccc attgtaacag agccacaaac taatactatg caatgtttta ccaataatgc 1440

aatacaaaag acctcaaaat acctgtgcat ttcttgtagg aaaacaacaa aaggtaatta 1500

tgtgtaatta tactagaagt tttgtaatct gtatcttatc attggaataa aatgacattc 1560

aataaataaa aa 1572

<210> 102
<211> 403
<212> PRT
<213> Homo sapiens

<400> 102

<210> 103
<211> 3015
<212> DNA
<213> Homo sapiens

<400> 103
cagggtttat tgttatgtaa gttcttgttc agcttccttt gttttctttc acttctgaga      60

atttaacttt cgtttctcac tcagctcctg tggggaaact catttgtgga gaccagccct     120

ctggcttggt gagtgaatct ggtttacacc ggctcctgcc ctgccttcac tcttctcccc     180

tgattcaaga ctcctctgct ttggactgaa gcactgcagg agtttgtgac caagaacttc     240

aagagtcaag acagaaggaa gccaagggag cagtgcaatg gatttctcag taaaggtaga     300

catagagaag gaggtgacct gccccatctg cctggagctc ctgacagaac ctctgagcct     360

agattgtggc cacagcttct gccaagcctg catcactgca aagatcaagg agtcagtgat     420

catctcaaga ggggaaagca gctgtcctgt gtgtcagacc agattccagc ctgggaacct     480

ccgacctaat cggcatctgg ccaacatagt tgagagagtc aaagaggtca agatgagccc     540

acaggagggg cagaagagag atgtctgtga gcaccatgga aaaaaactcc agatcttctg     600

taaggaggat ggaaaagtca tttgctgggt ttgtgaactg tctcaggaac accaaggtca     660

ccaaacattc cgcataaacg aggtggtcaa ggaatgtcag gaaaagctgc aggtagccct     720

gcagaggctg ataaaggagg atcaagaggc tgagaagctg gaagatgaca tcagacaaga     780

gagaaccgcc tggaagaatt atatccagat cgagagacag aagattctga aagggttcaa     840

tgaaatgaga gtcatcttgg acaatgagga gcagagagag ctgcaaaagc tggaggaagg     900

tgaggtgaat gtgctggata acctggcagc agctacagac cagctggtcc agcagaggca     960

ggatgccagc acgctcatct cagatctcca gcggaggttg aggggatcgt cagtagagat    1020

gctgcaggat gtgattgacg tcatgaaaag gagtgaaagc tggacattga agaagccaaa    1080

atctgtttcc aagaaactaa agagtgtatt ccgagtacca gatctgagtg ggatgctgca    1140

agttcttaaa gagctgacag atgtccagta ctactgggtg gacgtgatgc tgaatccagg 1200

cagtgccact tcgaatgttg ctatttctgt ggatcagaga caagtgaaaa ctgtacgcac 1260

ctgcacattt aagaattcaa atccatgtga tttttctgct tttggtgtct tcggctgcca 1320

atatttctct tcggggaaat attactggga agtagatgtg tctggaaaga ttgcctggat 1380

cctgggcgta cacagtaaaa taagtagtct gaataaaagg aagagctctg ggtttgcttt 1440

tgatccaagt gtaaattatt caaaagttta ctccagatat agacctcaat atggctactg 1500

ggttatagga ttacagaata catgtgaata taatgctttt gaggactcct cctcttctga 1560

tcccaaggtt ttgactctct ttatggctgt gcctccctgt cgtattgggg ttttcctaga 1620

ctatgaggca ggcattgtct catttttcaa tgtcacaaac cacggagcac tcatctacaa 1680

gttctctgga tgtcgctttt ctcgacctgc ttatccgtat ttcaatcctt ggaactgcct 1740

agtccccatg actgtgtgcc caccgagctc ctgagtgttc tcattccttt acccacttct 1800

gcatagtagc ccttgtgctg agactcagat tctgcacctg agttcatctc tactgagacc 1860

atctcttcct ttctttcccc ttcttttact tagaatgtct ttgtattcat ttgctagggc 1920

ttccatagca aagcatcata gattgctgat ttaaactgta attgtattgc cgtactgtgg 1980

gctggaaatc ccaaatctag attccagcag agttggttct ttctgaggtc tgcaaggaag 2040

ggctctgttc catgcctctc tccttggctt gtagaaggca tcttgtccct atgactcttc 2100

acattgtctt tatgtacatc tctgtgccca agttttccct ttttattaag acaccagtca 2160

tactggctca gggcccaccg ctaatgcctt aatgaaatca ttttaacatt atattctcta 2220

caaagacctt atttccaaat aagataatat ttggaggtat tgggaataaa aactccaaca 2280

tataaatttg aggaaggcac gatttcactc ataacaatct taccctttct tgcaagagat 2340

gcttgtacat tattttccta ataccttggt ttcactagta gtaaacatta ttattttttt 2400

tatatttgca aaggaaacat atctaatcct tcctatagaa agaacagtat tgctgtaatt 2460

ccttttcttt tcttcctcat ttcctctgcc ccttaaaaga ttgaagaaag agaaacttgt 2520

caactcatat ccacgttatc tagcaaagta cataagaatc tatcactaag taatgtatcc 2580

ttcagaatgt gttggtttac cagtgacacc ccatattcat cacaaaatta aagcaagaag 2640

tccatagtaa tttatttgct aatagtggat ttttaatgct cagagtttct gaggtcaaat 2700

tttatctttt cacttacaag ctctatgatc ttaaataatt tacttaatgt attttggtgt 2760

attttcctca aattaatatt ggtgttcaag actatatcta attcctctga tcactttgag 2820

aaacaaactt ttattaaatg taaggcactt ttctatgaat tttaaatata aaaataaata 2880

ttgttctgat tattactgaa aagatgtcag ccatttcaat gtcttgggaa acaatttttt 2940

gtttttgttc tgttttcttt ttgcttcaat aaaacaatag ctggctctaa aaaaaaaaaa    3000

aaaaaaaaaa aaaaa                                                      3015

<210> 104
<211> 498
<212> PRT
<213> Homo sapiens

<400> 104
Met Asp Phe Ser Val Lys Val Asp Ile Glu Lys Glu Val Thr Cys Pro
1               5                   10                  15

Ile Cys Leu Glu Leu Leu Thr Glu Pro Leu Ser Leu Asp Cys Gly His
            20                  25                  30

Ser Phe Cys Gln Ala Cys Ile Thr Ala Lys Ile Lys Glu Ser Val Ile
        35                  40                  45

Ile Ser Arg Gly Glu Ser Ser Cys Pro Val Cys Gln Thr Arg Phe Gln
    50                  55                  60

Pro Gly Asn Leu Arg Pro Asn Arg His Leu Ala Asn Ile Val Glu Arg
65                  70                  75                  80

Val Lys Glu Val Lys Met Ser Pro Gln Glu Gly Gln Lys Arg Asp Val
                85                  90                  95

Cys Glu His His Gly Lys Lys Leu Gln Ile Phe Cys Lys Glu Asp Gly
            100                 105                 110

Lys Val Ile Cys Trp Val Cys Glu Leu Ser Gln Glu His Gln Gly His
        115                 120                 125

Gln Thr Phe Arg Ile Asn Glu Val Val Lys Glu Cys Gln Glu Lys Leu
    130                 135                 140

Gln Val Ala Leu Gln Arg Leu Ile Lys Glu Asp Gln Glu Ala Glu Lys
145                 150                 155                 160

Leu Glu Asp Asp Ile Arg Gln Glu Arg Thr Ala Trp Lys Asn Tyr Ile
                165                 170                 175

Gln Ile Glu Arg Gln Lys Ile Leu Lys Gly Phe Asn Glu Met Arg Val
            180                 185                 190

Ile Leu Asp Asn Glu Glu Gln Arg Glu Leu Gln Lys Leu Glu Glu Gly
        195                 200                 205

Glu Val Asn Val Leu Asp Asn Leu Ala Ala Ala Thr Asp Gln Leu Val
    210                 215                 220

Gln Gln Arg Gln Asp Ala Ser Thr Leu Ile Ser Asp Leu Gln Arg Arg
225                 230                 235                 240

Leu Arg Gly Ser Ser Val Glu Met Leu Gln Asp Val Ile Asp Val Met
                245                 250                 255

Lys Arg Ser Glu Ser Trp Thr Leu Lys Lys Pro Lys Ser Val Ser Lys
            260                 265                 270

Lys Leu Lys Ser Val Phe Arg Val Pro Asp Leu Ser Gly Met Leu Gln
        275                 280                 285

Val Leu Lys Glu Leu Thr Asp Val Gln Tyr Tyr Trp Val Asp Val Met
    290                 295                 300

Leu Asn Pro Gly Ser Ala Thr Ser Asn Val Ala Ile Ser Val Asp Gln
305                 310                 315                 320

Arg Gln Val Lys Thr Val Arg Thr Cys Thr Phe Lys Asn Ser Asn Pro
                325                 330                 335

Cys Asp Phe Ser Ala Phe Gly Val Phe Gly Cys Gln Tyr Phe Ser Ser
            340                 345                 350

Gly Lys Tyr Tyr Trp Glu Val Asp Val Ser Gly Lys Ile Ala Trp Ile
        355                 360                 365

Leu Gly Val His Ser Lys Ile Ser Ser Leu Asn Lys Arg Lys Ser Ser
    370                 375                 380

Gly Phe Ala Phe Asp Pro Ser Val Asn Tyr Ser Lys Val Tyr Ser Arg
385                 390                 395                 400

Tyr Arg Pro Gln Tyr Gly Tyr Trp Val Ile Gly Leu Gln Asn Thr Cys
                405                 410                 415

Glu Tyr Asn Ala Phe Glu Asp Ser Ser Ser Ser Asp Pro Lys Val Leu
            420                 425                 430

Thr Leu Phe Met Ala Val Pro Pro Cys Arg Ile Gly Val Phe Leu Asp
        435                 440                 445

Tyr Glu Ala Gly Ile Val Ser Phe Phe Asn Val Thr Asn His Gly Ala
    450                 455                 460

Leu Ile Tyr Lys Phe Ser Gly Cys Arg Phe Ser Arg Pro Ala Tyr Pro
465                 470                 475                 480

Tyr Phe Asn Pro Trp Asn Cys Leu Val Pro Met Thr Val Cys Pro Pro
                485                 490                 495

Ser Ser

<210> 105
<211> 716
<212> DNA
<213> Homo sapiens

<400> 105
aggctcagta taaatagcag ccaccgctcc ctggcaggca gggacccgca gctcagctac      60

agcacagatc aggtgaggag cacaccaagg agtgattttt aaaacttact ctgttttctc     120

tttcccaaca agattatcat ttcctttaaa aaaaatagtt atcctggggc atacagccat     180

accattctga aggtgtctta tctcctctga tctagagagc accatgaagc ttctcacggg     240

cctggttttc tgctccttgg tcctgggtgt cagcagccga agcttctttt cgttccttgg     300

cgaggctttt gatggggctc gggacatgtg gagagcctac tctgacatga gagaagccaa     360

ttacatcggc tcagacaaat acttccatgc tcgggggaac tatgatgctg ccaaaagggg     420

acctgggggt gcctgggctg cagaagtgat cagcgatgcc agagagaata tccagagatt     480

ctttggccat ggtgcggagg actcgctggc tgatcaggct gccaatgaat ggggcaggag     540

tggcaaagac cccaatcact tccgacctgc tggcctgcct gagaaatact gagcttcctc     600

ttcactctgc tctcaggaga tctggctgtg aggccctcag ggcagggata caaagcgggg     660

agagggtaca caatgggtat ctaataaata cttaagaggt ggaatttgtg gaaact         716

<210> 106
<211> 122
<212> PRT
<213> Homo sapiens

<400> 106
Met Lys Leu Leu Thr Gly Leu Val Phe Cys Ser Leu Val Leu Gly Val
1               5                   10                  15

Ser Ser Arg Ser Phe Phe Ser Phe Leu Gly Glu Ala Phe Asp Gly Ala
            20                  25                  30

Arg Asp Met Trp Arg Ala Tyr Ser Asp Met Arg Glu Ala Asn Tyr Ile
        35                  40                  45

Gly Ser Asp Lys Tyr Phe His Ala Arg Gly Asn Tyr Asp Ala Ala Lys
    50                  55                  60

Arg Gly Pro Gly Gly Ala Trp Ala Ala Glu Val Ile Ser Asp Ala Arg
65                  70                  75                  80

Glu Asn Ile Gln Arg Phe Phe Gly His Gly Ala Glu Asp Ser Leu Ala
                85                  90                  95

Asp Gln Ala Ala Asn Glu Trp Gly Arg Ser Gly Lys Asp Pro Asn His
            100                 105                 110

Phe Arg Pro Ala Gly Leu Pro Glu Lys Tyr
        115                 120

<210> 107
<211> 1449
<212> DNA
<213> Homo sapiens

<400> 107
tcacctgccc cacagccgca ccctgcctgt gcctgcaccc tggggagccc agagccggca      60

ggggccgagg cggtgggacc tcgggggagc tcaagcctcg actgtcccct cgctggaggc     120

cagaggccaa gatgatgtcc atgctgggcg gcttgcagag atacttccgg gtcatcctcc     180

tcctcctcct ggccctgact ctgctcctgc tggccggatt cctgcactcg gacttagagc     240

tggacacacc cctgtttggg ggccaggctg aggggccgcc ggtcaccaac atcatgttcc     300

tgaagacgca caagacggcc agcagcacgg tgctcaacat cctctaccgc ttcgccgaga     360

cccacaacct gtccgtggcg ctgcccgccg gctcacgcgt ccacctgggc tacccctggc     420

tcttcctggc gcgctacgtg gaaggcgtgg ggtcgcagca gcgcttcaac atcatgtgca     480

accacctgag gttcaacctg cctcaggtgc agaaagtcat gcccaacgac accttctact     540

tctccatcct gaggaacccc gtgttccagc tggagtcctc cttcatctac tacaaaacct     600

acgcccccgc cttccggggc gccccgagcc tggacgcgtt cctggcctcg ccgcggacgt     660

tctacaacga cagccgccac ctcaggaacg tctacgccaa gaacaacatg tggttcgact     720

tcggcttcga ccccaacgcg cagtgcgagg agggctacgt gcgcgcgcgc atcgccgagg     780

tggagcggcg cttccggctg gtgctcatcg ccgagcacct ggacgagtcc ctggtgctgc     840

tgcggcgccg gctgcgctgg gcgctggacg acgtggtggc cttcaggctc aactcccgca     900

gcgcgcgctc cgtggcccgc ctgtcgcccg agacccggga gcgcgcgcgg agctggtgcg 960

cgctggactg gcgcctgtac gagcatttca accgcaccct ctgggcgcag ctgcgcgccg 1020

agctggggcc gcggcggctg cgcggggagg tggagcggct gcgcgcccgg aggcgcgaac 1080

tcgcgagcct gtgcctgcag gacggcggcg cgctcaagaa ccacacgcag atcagagacc 1140

cgcgcctgcg cccctaccag tccggcaagg ccgacatcct gggttacaac ctccggccgg 1200

gcctggacaa ccagacgctg ggcgtgtgcc agaggcttgt gatgcctgag ctccagtaca 1260

tggcccgcct gtacgccctg cagttcccgg agaagcccct caagaacatc ccgttcctgg 1320

gggcgtagag gggccgggcc ggggacgagg cctcctgcgg acaccagctc ctctctccgc 1380

cgtcaccggg gaggccgggg atccttgcag ggcttctggg gcgttgggaa acccaggccc 1440

gccggccac 1449

<210> 108
<211> 398
<212> PRT
<213> Homo sapiens

<400> 108

<210> 109
<211> 5392
<212> DNA
<213> Homo sapiens

<400> 109
aattcactaa tgcattctgc tctttttgag agcacagctt ctcagatgtg ctccttggag      60

ctggtgtgca gtgtcctgac tgtaagatca agtccaaacc tgttttggaa ttgaggaaac     120

ttctcttttg atctcagccc ttggtggtcc aggtcttcat gctgctgtgg gtgatattac     180

tggtcctggc tcctgtcagt ggacagtttg caaggacacc caggcccatt attttcctcc     240

agcctccatg gaccacagtc ttccaaggag agagagtgac cctcacttgc aagggatttc     300

gcttctactc accacagaaa acaaaatggt accatcggta cctcgggaaa gaaatactaa     360

gagaaacccc agacaatatc cttgaggttc aggaatctgg agagtacaga tgccaggccc     420

agggctcccc tctcagtagc cctgtgcact tggatttttc ttcagcttcg ctgatcctgc     480

aagctccact ttctgtgttt gaaggagact ctgtggttct gaggtgccgg gcaaaggcgg     540

aagtaacact gaataatact atttacaaga atgataatgt cctggcattc cttaataaaa     600

gaactgactt ccatattcct catgcatgtc tcaaggacaa tggtgcatat cgctgtactg     660

gatataagga aagttgttgc cctgtttctt ccaatacagt caaaatccaa gtccaagagc     720

catttacacg tccagtgctg agagccagct ccttccagcc catcagcggg aacccagtga     780

ccctgacctg tgagacccag ctctctctag agaggtcaga tgtcccgctc cggttccgct     840

tcttcagaga tgaccagacc ctgggattag gctggagtct ctccccgaat ttccagatta     900

ctgccatgtg gagtaaagat tcagggttct actggtgtaa ggcagcaaca atgcctcaca     960

gcgtcatatc tgacagcccg agatcctgga tacaggtgca gatccctgca tctcatcctg    1020

tcctcactct cagccctgaa aaggctctga attttgaggg aaccaaggtg acacttcact    1080

gtgaaaccca ggaagattct ctgcgcactt tgtacaggtt ttatcatgag ggtgtccccc    1140

tgaggcacaa gtcagtccgc tgtgaaaggg gagcatccat cagcttctca ctgactacag    1200

agaattcagg gaactactac tgcacagctg acaatggcct tggcgccaag cccagtaagg    1260

ctgtgagcct ctcagtcact gttcccgtgt ctcatcctgt cctcaacctc agctctcctg    1320

aggacctgat tttgagggga gccaaggtga cacttcactg tgaagcccag agaggttcac    1380

tccccatcct gtaccagttt catcatgagg atgctgccct ggagcgtagg tcggccaact    1440

ctgcaggagg agtggccatc agcttctctc tgactgcaga gcattcaggg aactactact    1500

gcacagctga caatggcttt ggcccccagc gcagtaaggc ggtgagcctc tccatcactg 1560
tccctgtgtc tcatcctgtc ctcaccctca gctctgctga ggccctgact tttgaaggag 1620
ccactgtgac acttcactgt gaagtccaga gaggttcccc acaaatccta taccagtttt 1680
atcatgagga catgcccctg tggagcagct caacaccctc tgtgggaaga gtgtccttca 1740
gcttctctct gactgaagga cattcaggga attactactg cacagctgac aatggctttg 1800
gtccccagcg cagtgaagtg gtgagccttt ttgtcactgt tccagtgtct cgccccatcc 1860
tcaccctcag ggttcccagg gcccaggctg tggtggggga cctgctggag cttcactgtg 1920
aggccccgag aggctctccc ccaatcctgt actggtttta tcatgaggat gtcaccctgg 1980
ggagcagctc agccccctct ggaggagaag cttctttcaa cctctctctg actgcagaac 2040
attctggaaa ctactcatgt gaggccaaca atggcctagt ggcccagcac agtgacacaa 2100
tatcactcag tgttatagtt ccagtatctc gtcccatcct caccttcagg gctcccaggg 2160
cccaggctgt ggtgggggac ctgctggagc ttcactgtga ggccctgaga ggctcctccc 2220
caatcctgta ctggttttat catgaagatg tcaccctggg taagatctca gccccctctg 2280
gaggaggggc ctccttcaac ctctctctga ctacagaaca ttctggaatc tactcctgtg 2340
aggcagacaa tggtccggag gcccagcgca gtgagatggt gacactgaaa gttgcagttc 2400
cggtgtctcg cccggtcctc accctcaggg ctcccgggac ccatgctgcg gtgggggacc 2460
tgctggagct tcactgtgag gccctgagag gctctcccct gatcctgtac cggttttttc 2520
atgaggatgt caccctagga aataggtcgt ccccctctgg aggagcgtcc ttaaacctct 2580
ctctgactgc agagcactct ggaaactact cctgtgaggc cgacaatggc ctcggggccc 2640
agcgcagtga gacagtgaca ctttatatca cagggctgac cgcgaacaga agtggccctt 2700
ttgccacagg agtcgccggg ggcctgctca gcatagcagg ccttgctgcg ggggcactgc 2760
tgctctactg ctggctctcg agaaaagcag ggagaaagcc tgcctctgac cccgccagga 2820
gccctccaga ctcggactcc caagagccca cctatcacaa tgtaccagcc tgggaagagc 2880
tgcaaccagt gtacactaat gcaaatccta gaggagaaaa tgtggtttac tcagaagtac 2940
ggatcatcca agagaaaaag aaacatgcag tggcctctga ccccaggcat ctcaggaaca 3000
agggttcccc tatcatctac tctgaagtta aggtggcgtc aaccccggtt tccggatccc 3060
tgttcttggc ttcctcagct cctcacagat gagtccacac gtctctccaa ctgctgtttc 3120
agcctctgca ccccaaagtt ccccttgggg gagaagcagc attgaagtgg gaagatttag 3180
gctgccccag accatatcta ctggcctttg tttcacatgt cctcattctc agtctgacca 3240
gaatgcaggg ccctgctgga ctgtcacctg tttcccagtt aaagccctga ctggcaggtt 3300
ttttaatcca gtggcaaggt gctcccactc cagggcccag cacatctcct ggattcctta 3360

gtgggcttca gctgtgattg ctgttctgag tactgctctc atcacacccc cacagagggg 3420

gtcttaccac acaaagggag agtgggcctt caggagatgc cgggctggcc taacagctca 3480

ggtgctccta aactccgaca cagagttcct gctttgggtg gatgcatttc tcaattgtca 3540

tcagcctggt ggggctactg cagtgtgctg ccaaatggga cagcacacag cctgtgcaca 3600

tgggacatgt gatgggtctc ccccacgggg ctgcatttca cactcctcca cctgtctcaa 3660

actctaaggt cggcacttga caccaaggta acttctctcc tgctcatgtg tcagtgtcta 3720

cctgcccaag taagtggctt tcatacacca agtcccaagt tcttcccatc ctaacagaag 3780

taacccagca agtcaaggcc aggaggacca ggggtgcaga cagaacacat actggaacac 3840

aggaggtgct caattactat ttgactgact gactgaatga atgaatgaat gaggaagaaa 3900

actgtgggta atcaaactgg cataaaatcc agtgcactcc ctaggaaatc cgggaggtat 3960

tctggcttcc ctaagaaaca acggaagaga aggagcttgg atgaggaaac tgttcagcaa 4020

gaggaagggc ttctcacact ttcatgtgct tgtggatcac ctgaggatcc tgtgaaaata 4080

cagatactga ttcagtgggt ctgtgtagag cctgagactg ccattctaac atgttcccag 4140

gggatgctga tgctgctggc cctgggactg cactgcatgc atgtgaagcc ctataggtct 4200

cagcagaggc ccatggagag ggaatgtgtg gctctggctg cccagggccc aactcggttc 4260

acacggatcg tgctgctccc tggccagcct ttggccacag caccaccagc tgctgttgct 4320

gagagagctt cttctctgtg acatgttggc tttcatcagc caccctggga agcggaaagt 4380

agctgccact atctttgttt ccccacctca ggcctcacac tttcccatga aaagggtgaa 4440

tgtatataac ctgagccctc tccattcaga gttgttctcc catctctgag caatgggatg 4500

ttctgttccg cttttatgat atccatcaca tcttatcttg atctttgctc ccagtggatt 4560

gtacagtgat gacttttaag ccccacggcc ctgaaataaa atccttccaa gggcattgga 4620

agctctctcc acctgaacca tggcttttca tgcttccaag tgtcagggcc ttgcccagat 4680

agacagggct gactctgctg ccccaacctt tcaaggagga aaccagacac ctgagacagg 4740

agcctgtatg cagcccagtg cagccttgca gaggacaagg ctggaggcat ttgtcatcac 4800

tacagatatg caactaaaat agacgtggag caagagaaat gcattcccac cgaggccgct 4860

tttttaggcc tagttgaaag tcaagaagga cagcagcaag cataggctca ggattaaaga 4920

aaaaaatctg ctcacagttt gttctggagg tcacatcacc aacaaagctc acgccctatg 4980

cagttctgag aaggtggagg caccaggctc aaaagaggaa atttagaatt tctcattggg 5040

agagtaaggt acccccatcc cagaatgata actgcacagt ggcagaacaa actccaccct 5100

aatgtgggtg gaccccatcc agtctgttga aggcctgagt gtaacaaaag ggcttattct 5160

tcctcaagta agggggaact cctgctttgg gctgggacat aagtttttct gctttcagac   5220

gcaaactgaa aaatggctct tcttgggtct tgagcttgct ggcatatgga ctgaaagaaa   5280

ctatgctatt ggatctcctg gatctccagc ttgctgactg cagatcttga gatatgtcag   5340

cctctacagt cacaagagct aattcattct aataaaccaa tctttctgta aa           5392

<210> 110
<211> 977
<212> PRT
<213> Homo sapiens

<400> 110
Met Leu Leu Trp Val Ile Leu Leu Val Leu Ala Pro Val Ser Gly Gln
1               5                   10                  15

Phe Ala Arg Thr Pro Arg Pro Ile Ile Phe Leu Gln Pro Pro Trp Thr
            20                  25                  30

Thr Val Phe Gln Gly Glu Arg Val Thr Leu Thr Cys Lys Gly Phe Arg
        35                  40                  45

Phe Tyr Ser Pro Gln Lys Thr Lys Trp Tyr His Arg Tyr Leu Gly Lys
    50                  55                  60

Glu Ile Leu Arg Glu Thr Pro Asp Asn Ile Leu Glu Val Gln Glu Ser
65                  70                  75                  80

Gly Glu Tyr Arg Cys Gln Ala Gln Gly Ser Pro Leu Ser Ser Pro Val
                85                  90                  95

His Leu Asp Phe Ser Ser Ala Ser Leu Ile Leu Gln Ala Pro Leu Ser
            100                 105                 110

Val Phe Glu Gly Asp Ser Val Val Leu Arg Cys Arg Ala Lys Ala Glu
        115                 120                 125

Val Thr Leu Asn Asn Thr Ile Tyr Lys Asn Asp Asn Val Leu Ala Phe
    130                 135                 140

Leu Asn Lys Arg Thr Asp Phe His Ile Pro His Ala Cys Leu Lys Asp
145                 150                 155                 160

Asn Gly Ala Tyr Arg Cys Thr Gly Tyr Lys Glu Ser Cys Cys Pro Val
                165                 170                 175

Ser Ser Asn Thr Val Lys Ile Gln Val Gln Glu Pro Phe Thr Arg Pro
            180                 185                 190

Val Leu Arg Ala Ser Ser Phe Gln Pro Ile Ser Gly Asn Pro Val Thr
        195                 200                 205

Leu Thr Cys Glu Thr Gln Leu Ser Leu Glu Arg Ser Asp Val Pro Leu
    210                 215                 220

Arg Phe Arg Phe Phe Arg Asp Asp Gln Thr Leu Gly Leu Gly Trp Ser
225                 230                 235                 240

Leu Ser Pro Asn Phe Gln Ile Thr Ala Met Trp Ser Lys Asp Ser Gly
                245                 250                 255

Phe Tyr Trp Cys Lys Ala Ala Thr Met Pro His Ser Val Ile Ser Asp
            260                 265                 270

Ser Pro Arg Ser Trp Ile Gln Val Gln Ile Pro Ala Ser His Pro Val
        275                 280                 285

Leu Thr Leu Ser Pro Glu Lys Ala Leu Asn Phe Glu Gly Thr Lys Val
    290                 295                 300

Thr Leu His Cys Glu Thr Gln Glu Asp Ser Leu Arg Thr Leu Tyr Arg
305                 310                 315                 320

Phe Tyr His Glu Gly Val Pro Leu Arg His Lys Ser Val Arg Cys Glu
                325                 330                 335

Arg Gly Ala Ser Ile Ser Phe Ser Leu Thr Thr Glu Asn Ser Gly Asn
            340                 345                 350

Tyr Tyr Cys Thr Ala Asp Asn Gly Leu Gly Ala Lys Pro Ser Lys Ala
        355                 360                 365

Val Ser Leu Ser Val Thr Val Pro Val Ser His Pro Val Leu Asn Leu
    370                 375                 380

Ser Ser Pro Glu Asp Leu Ile Phe Glu Gly Ala Lys Val Thr Leu His
385                 390                 395                 400

Cys Glu Ala Gln Arg Gly Ser Leu Pro Ile Leu Tyr Gln Phe His His
                405                 410                 415

Glu Asp Ala Ala Leu Glu Arg Arg Ser Ala Asn Ser Ala Gly Gly Val
            420                 425                 430

Ala Ile Ser Phe Ser Leu Thr Ala Glu His Ser Gly Asn Tyr Tyr Cys
        435                 440                 445

Thr Ala Asp Asn Gly Phe Gly Pro Gln Arg Ser Lys Ala Val Ser Leu
    450                 455                 460

Ser Ile Thr Val Pro Val Ser His Pro Val Leu Thr Leu Ser Ser Ala
465                 470                 475                 480

Glu Ala Leu Thr Phe Glu Gly Ala Thr Val Thr Leu His Cys Glu Val
                485                 490                 495

Gln Arg Gly Ser Pro Gln Ile Leu Tyr Gln Phe Tyr His Glu Asp Met
            500                 505                 510

Pro Leu Trp Ser Ser Ser Thr Pro Ser Val Gly Arg Val Ser Phe Ser
        515                 520                 525

Phe Ser Leu Thr Glu Gly His Ser Gly Asn Tyr Tyr Cys Thr Ala Asp
    530                 535                 540

Asn Gly Phe Gly Pro Gln Arg Ser Glu Val Val Ser Leu Phe Val Thr
545                 550                 555                 560

Val Pro Val Ser Arg Pro Ile Leu Thr Leu Arg Val Pro Arg Ala Gln
                565                 570                 575

Ala Val Val Gly Asp Leu Leu Glu Leu His Cys Glu Ala Pro Arg Gly
            580                 585                 590

Ser Pro Pro Ile Leu Tyr Trp Phe Tyr His Glu Asp Val Thr Leu Gly
        595                 600                 605

Ser Ser Ser Ala Pro Ser Gly Gly Glu Ala Ser Phe Asn Leu Ser Leu
    610                 615                 620

Thr Ala Glu His Ser Gly Asn Tyr Ser Cys Glu Ala Asn Asn Gly Leu
625                 630                 635                 640

Val Ala Gln His Ser Asp Thr Ile Ser Leu Ser Val Ile Val Pro Val
                645                 650                 655

Ser Arg Pro Ile Leu Thr Phe Arg Ala Pro Arg Ala Gln Ala Val Val
            660                 665                 670

Gly Asp Leu Leu Glu Leu His Cys Glu Ala Leu Arg Gly Ser Ser Pro
        675                 680                 685

Ile Leu Tyr Trp Phe Tyr His Glu Asp Val Thr Leu Gly Lys Ile Ser
    690                 695                 700

Ala Pro Ser Gly Gly Gly Ala Ser Phe Asn Leu Ser Leu Thr Thr Glu
705                 710                 715                 720

His Ser Gly Ile Tyr Ser Cys Glu Ala Asp Asn Gly Pro Glu Ala Gln
                725                 730                 735

Arg Ser Glu Met Val Thr Leu Lys Val Ala Val Pro Val Ser Arg Pro
            740                 745                 750

Val Leu Thr Leu Arg Ala Pro Gly Thr His Ala Ala Val Gly Asp Leu
        755                 760                 765

Leu Glu Leu His Cys Glu Ala Leu Arg Gly Ser Pro Leu Ile Leu Tyr
    770                 775                 780

Arg Phe Phe His Glu Asp Val Thr Leu Gly Asn Arg Ser Ser Pro Ser
785                 790                 795                 800

Gly Gly Ala Ser Leu Asn Leu Ser Leu Thr Ala Glu His Ser Gly Asn
                805                 810                 815

Tyr Ser Cys Glu Ala Asp Asn Gly Leu Gly Ala Gln Arg Ser Glu Thr
            820                 825                 830

Val Thr Leu Tyr Ile Thr Gly Leu Thr Ala Asn Arg Ser Gly Pro Phe
        835                 840                 845

Ala Thr Gly Val Ala Gly Gly Leu Leu Ser Ile Ala Gly Leu Ala Ala
    850                 855                 860

Gly Ala Leu Leu Leu Tyr Cys Trp Leu Ser Arg Lys Ala Gly Arg Lys
865                 870                 875                 880

Pro Ala Ser Asp Pro Ala Arg Ser Pro Pro Asp Ser Asp Ser Gln Glu
                885                 890                 895

Pro Thr Tyr His Asn Val Pro Ala Trp Glu Glu Leu Gln Pro Val Tyr
            900                 905                 910

Thr Asn Ala Asn Pro Arg Gly Glu Asn Val Val Tyr Ser Glu Val Arg

915                 920                 925

Ile Ile Gln Glu Lys Lys Lys His Ala Val Ala Ser Asp Pro Arg His
    930                 935                 940

Leu Arg Asn Lys Gly Ser Pro Ile Ile Tyr Ser Glu Val Lys Val Ala
945                 950                 955                 960

Ser Thr Pro Val Ser Gly Ser Leu Phe Leu Ala Ser Ser Ala Pro His
                965                 970                 975

Arg

<210> 111
<211> 3143
<212> DNA
<213> Homo sapiens

<400> 111
ggcacgaggc ccagcactag aagtcggcgg tgtttccttt cggtgatcag cactgaacac      60

agaggactcg ccatggagtt tgggctgagc tgggttttcc tcgttgctct tttaagaggt     120

gtccagtgtc aggcgcagtt ggtggagtct gggggaggcg tggtccagcc cgggagctcc     180

ctgagactct cctgtgcagc ctctggtttc aggttcagca attatggcat gcactgggtc     240

cgccaggctc caggcaaggg gctggagtgg gtggcagttt tttcatatga tgaaagtgat     300

aaatattatg cagcctccgt gaagggtcga tttaccatct ccagagacaa ctccaagaac     360

acgttgtctc tgcaaatgaa cagcctgaga gttgaggaca cggctgttta ctactgtgcg     420

aaagatcaga agccctggta cagcaacagc tggtttctaa ccaactttga ctcttggggc     480

cggggcaccc tggtcaccgt ctcctcagcc tccaccaagg gcccatcggt cttccccctg     540

gcaccctcct ccaagagcac ctctgggggc acagcggccc tgggctgcct ggtcaaggac     600

tacttccccg aaccggtgac ggtgtcgtgg aactcaggcg ccctgaccag cggcgtgcac     660

accttcccgg ctgtcctaca gtcctcagga ctctactccc tcagcagcgt ggtgaccgtg     720

ccctccagca gcttgggcac ccagacctac atctgcaacg tgaatcacaa gcccagcaac     780

accaaggtgg acaagaaagt tgagcccaaa tcttgtgaca aaactcacac atgcccaccg     840

tgcccagcac ctgaactcct ggggggaccg tcagtcttcc tcttcccccc aaaacccaag     900

gacaccctca tgatctcccg gacccctgag gtcacatgcg tggtggtgga cgtgagccac     960

gaagaccctg aggtcaagtt caactggtac gtggacggcg tggaggtgca taatgccaag    1020

acaaagccgc gggaggagca gtacaacagc acgtaccgtg tggtcagcgt cctcaccgtc    1080

ctgcaccagg actggctgaa tggcaaggag tacaagtgca aggtctccaa caaagccctc 1140

ccagccccca tcgagaaaac catctccaaa gccaaagggc agccccgaga accacaggtg 1200

tacaccctgc ccccatcccg ggatgagctg accaagaacc aggtcagcct gacctgcctg 1260

gtcaaaggct tctatcccag cgacatcgcc gtggagtggg agagcaatgg gcagccggag 1320

aacaactaca agaccacgcc tcccgtgctg gactccgacg gctccttctt cctctacagc 1380

aagctcaccg tggacaagag caggtggcag caggggaacg tcttctcatg ctccgtgatg 1440

catgaggctc tgcacaacca ctacacgcag aagagcctct ccctgtctcc ggagctgcaa 1500

ctggaggaga gctgtgcgga ggcgcaggac ggggagctgg acgggctgtg gacgaccatc 1560

accatcttca tcacactctt cctgttaagc gtgtgctaca gtgccaccgt caccttcttc 1620

aaggtgaagt ggatcttctc ctcggtggtg gacctgaagc agaccatcat ccccgactac 1680

aggaacatga tcggacaggg ggcctagggc caccctctgc ggggtgtcca gggccgccca 1740

gaccccacac accagccatg ggccatgctc agccaccacc caggccacac ctgcccccga 1800

cctcaccgcc ctcaacccca tggctctctg gcctcgcagt tgccctctga ccctgacaca 1860

cctgacacgc cccccttcca gaccctgtgc atagcaggtc taccccagac ctccgctgct 1920

tggtgcatgc agggcactgg gggccaggtg tcccctcagc aggacatcct tgccctccgg 1980

accacaaggt gctcacacaa aaggaggcag tgaccggtat cccaggcccc cacccaggca 2040

ggagctggcc ctggagccaa ccccgtccac gccagcctcc tgaacacagg cgtggtttcc 2100

agatggtgag tgggagcatc agccgccaag gtagggaagc cacagcacca tcaggccctg 2160

ttggggaggc ttccgagagc tgcgaaggct cactcagacg gccttcctcc cagcccgcag 2220

ccagccagcc tccattccgg gcactcccgt gaactcctga catgaggaat gaggttgttc 2280

tgatttcaag caaagaacgc tgctctctgg ctcctgggaa cagtctcggt gccagcacca 2340

ccccttggct gcctgcccac actgctggat tctcgggtgg aactggaccc gcagggacag 2400

ccagccccag agtccgcact ggggagagaa ggggccaggc ccaggacact gccacctccc 2460

acccactcca gtccaccgag atcactcaga gaagagcctg ggccatgtgg ccgctgcagg 2520

agccccacag tgcaagggtg aggatagccc aaggaagggc tgggcatctg cccagacagg 2580

cctcccagag aaggctggtg accaggtccc aggcgggcaa gactcagcct tggtggggcc 2640

tgaggacaga ggaggcccag gagcatcggg gagagaggtg gagggacacc gggagagcca 2700

ggagcgtgga cacagccaga actcatcaca gaggctggcg tccagtcccg ggtcacgtgc 2760

agcaggaaca agcagccact ctgggggcac caggtggaga ggcaagacga caaagagggt 2820

gcccgtgttc ttgcgaaagc ggggctgctg gccacgagtg ctggacagag gcccccacgc 2880

tctgctgccc ccatcacgcc gttccgtgac tgtcacgcag aatccgcaga caggaaggga 2940

gactcgagcg ggagtgcggc cagcgcctgc ctcggccgtc agggaggact cccgggctca 3000

ctcgaaggag gtgccaccat ttcagctttg gtagcttttc ttcttctttt aaattttcta 3060

aagctcatta attgtctttg atgtttcttt tgtgatgaca ataaaatatc ctttttaagt 3120

cttgtaaaaa aaaaaaaaaa aaa 3143

<210> 112
<211> 544
<212> PRT
<213> Homo sapiens

<400> 112
Met Glu Phe Gly Leu Ser Trp Val Phe Leu Val Ala Leu Leu Arg Gly
1               5                   10                  15

Val Gln Cys Gln Ala Gln Leu Val Glu Ser Gly Gly Gly Val Val Gln
            20                  25                  30

Pro Gly Ser Ser Leu Arg Leu Ser Cys Ala Ala Ser Gly Phe Arg Phe
        35                  40                  45

Ser Asn Tyr Gly Met His Trp Val Arg Gln Ala Pro Gly Lys Gly Leu
    50                  55                  60

Glu Trp Val Ala Val Phe Ser Tyr Asp Glu Ser Asp Lys Tyr Tyr Ala
65                  70                  75                  80

Ala Ser Val Lys Gly Arg Phe Thr Ile Ser Arg Asp Asn Ser Lys Asn
                85                  90                  95

Thr Leu Ser Leu Gln Met Asn Ser Leu Arg Val Glu Asp Thr Ala Val
            100                 105                 110

Tyr Tyr Cys Ala Lys Asp Gln Lys Pro Trp Tyr Ser Asn Ser Trp Phe
        115                 120                 125

Leu Thr Asn Phe Asp Ser Trp Gly Arg Gly Thr Leu Val Thr Val Ser
    130                 135                 140

Ser Ala Ser Thr Lys Gly Pro Ser Val Phe Pro Leu Ala Pro Ser Ser
145                 150                 155                 160

Lys Ser Thr Ser Gly Gly Thr Ala Ala Leu Gly Cys Leu Val Lys Asp
                165                 170                 175

Tyr Phe Pro Glu Pro Val Thr Val Ser Trp Asn Ser Gly Ala Leu Thr

180                 185                 190

Ser Gly Val His Thr Phe Pro Ala Val Leu Gln Ser Ser Gly Leu Tyr
        195                 200                 205

Ser Leu Ser Ser Val Val Thr Val Pro Ser Ser Ser Leu Gly Thr Gln
    210                 215                 220

Thr Tyr Ile Cys Asn Val Asn His Lys Pro Ser Asn Thr Lys Val Asp
225                 230                 235                 240

Lys Lys Val Glu Pro Lys Ser Cys Asp Lys Thr His Thr Cys Pro Pro
                245                 250                 255

Cys Pro Ala Pro Glu Leu Leu Gly Gly Pro Ser Val Phe Leu Phe Pro
            260                 265                 270

Pro Lys Pro Lys Asp Thr Leu Met Ile Ser Arg Thr Pro Glu Val Thr
        275                 280                 285

Cys Val Val Val Asp Val Ser His Glu Asp Pro Glu Val Lys Phe Asn
    290                 295                 300

Trp Tyr Val Asp Gly Val Glu Val His Asn Ala Lys Thr Lys Pro Arg
305                 310                 315                 320

Glu Glu Gln Tyr Asn Ser Thr Tyr Arg Val Val Ser Val Leu Thr Val
                325                 330                 335

Leu His Gln Asp Trp Leu Asn Gly Lys Glu Tyr Lys Cys Lys Val Ser
            340                 345                 350

Asn Lys Ala Leu Pro Ala Pro Ile Glu Lys Thr Ile Ser Lys Ala Lys
        355                 360                 365

Gly Gln Pro Arg Glu Pro Gln Val Tyr Thr Leu Pro Pro Ser Arg Asp
    370                 375                 380

Glu Leu Thr Lys Asn Gln Val Ser Leu Thr Cys Leu Val Lys Gly Phe
385                 390                 395                 400

Tyr Pro Ser Asp Ile Ala Val Glu Trp Glu Ser Asn Gly Gln Pro Glu
                405                 410                 415

Asn Asn Tyr Lys Thr Thr Pro Pro Val Leu Asp Ser Asp Gly Ser Phe
            420                 425                 430

Phe Leu Tyr Ser Lys Leu Thr Val Asp Lys Ser Arg Trp Gln Gln Gly
        435                 440                 445

Asn Val Phe Ser Cys Ser Val Met His Glu Ala Leu His Asn His Tyr
    450                 455                 460

Thr Gln Lys Ser Leu Ser Leu Ser Pro Glu Leu Gln Leu Glu Glu Ser
465                 470                 475                 480

Cys Ala Glu Ala Gln Asp Gly Glu Leu Asp Gly Leu Trp Thr Thr Ile
                485                 490                 495

Thr Ile Phe Ile Thr Leu Phe Leu Leu Ser Val Cys Tyr Ser Ala Thr
            500                 505                 510

Val Thr Phe Phe Lys Val Lys Trp Ile Phe Ser Ser Val Val Asp Leu
        515                 520                 525

Lys Gln Thr Ile Ile Pro Asp Tyr Arg Asn Met Ile Gly Gln Gly Ala
    530                 535                 540

<210> 113
<211> 2213
<212> DNA
<213> Homo sapiens

<400> 113
gctctagaac tagtggatcc cccgggctgc aggaattctc taaagaagcc cctgggagca      60

cagctcatca ccatggactg gacctggagg ttcctctttg tggtggcagc agctacaggt     120

gtccagtccc aggtgcagct ggtgcagtct ggggctgagg tgaagaagcc tgggtcctcg     180

gtgaaggtct cctgcaaggc ttctggaggc accttcagca gctatgctat cagctgggtg     240

cgacaggccc ctggacaagg gcttgagtgg atgggaggga tcatccctat ctttggtaca     300

gcaaactacg cacagaagtt ccagggcaga gtcacgatta ccgcggacga atccacgagc     360

acagcctaca tggagctgag cagcctgaga tctgaggaca cggccgtgta ttactgtgcg     420

aaaaccggga tcctggggcc gtatagcagt ggctggtacc cgaactcgga ctactactac     480

tacggtatgg acgtctgggg ccaagggacc acggtcaccg tctcctcagg gagtgcatcc     540

gccccaaccc ttttccccct cgtctcctgt gagaattccc cgtcggatac gagcagcgtg     600

gccgttggct gcctcgcaca ggacttcctt cccgactcca tcactttctc ctggaaatac     660

aagaacaact ctgacatcag cagcacccgg ggcttcccat cagtcctgag agggggcaag     720

tacgcagcca cctcacaggt gctgctgcct tccaaggacg tcatgcaggg cacagacgaa     780

cacgtggtgt gcaaagtcca gcaccccaac ggcaacaaag aaaagaacgt gcctcttcca     840

gtgattgctg agctgcctcc caaagtgagc gtcttcgtcc caccccgcga cggcttcttc     900

ggcaaccccc gcagcaagtc caagctcatc tgccaggcca cgggtttcag tccccggcag     960

attcaggtgt cctggctgcg cgaggggaag caggtggggt ctggcgtcac cacggaccag    1020

gtgcaggctg aggccaaaga gtctgggccc acgacctaca aggtgaccag cacactgacc    1080

atcaaagaga gcgactggct cagccagagc atgttcacct gccgcgtgga tcacaggggc    1140

ctgaccttcc agcagaatgc gtcctccatg tgtgtccccg atcaagacac agccatccgg    1200

gtcttcgcca tccccccatc ctttgccagc atcttcctca ccaagtccac caagttgacc    1260

tgcctggtca cagacctgac cacctatgac agcgtgacca tctcctggac ccgccagaat    1320

ggcgaagctg tgaaaaccca caccaacatc tccgagagcc accccaatgc cactttcagc    1380

gccgtgggtg aggccagcat ctgcgaggat gactggaatt ccggggagag gttcacgtgc    1440

accgtgaccc acacagacct gccctcgcca ctgaagcaga ccatctcccg gcccaagggg    1500

gtggccctgc acaggcccga tgtctacttg ctgccaccag cccgggagca gctgaacctg    1560

cgggagtcgg ccaccatcac gtgcctggtg acgggcttct ctcccgcgga cgtcttcgtg    1620

cagtggatgc agagggggca gcccttgtcc ccggagaagt atgtgaccag cgccccaatg    1680

cctgagcccc aggccccagg ccggtacttc gcccacagca tcctgaccgt gtccgaagag    1740

gaatggaaca cgggggagac ctacacctgc gtggtggccc atgaggccct gcccaacagg    1800

gtcaccgaga ggaccgtgga caagtccacc gagggggagg tgagcgccga cgaggagggc    1860

tttgagaacc tgtgggccac cgcctccacc ttcatcgtcc tcttcctcct gagcctcttc    1920

tacagtacca ccgtcacctt gttcaaggtg aaatgatccc aacagaagaa catcggagac    1980

cagagagagg aactcaaagg ggcgctgcct ccgggtctgg ggtcctggcc tgcgtggcct    2040

gttggcacgt gtttctcttc ccgcccggcc tccagttgtg tgctctcaca caggcttcct    2100

tctcgaccgg caggggctgg ctggcttgca ggccacgagg tgggctctac cccacactgc    2160

tttgctgtgt atacgcttgt tgccctgaaa taaatatgca cattttatcc atg           2213

<210> 114
<211> 627
<212> PRT
<213> Homo sapiens

<400> 114

<210> 115
<211> 2387
<212> DNA
<213> Homo sapiens

<400> 115
agacacctct gccctcacca tgagcctctg gcagcccctg gtcctggtgc tcctggtgct      60

gggctgctgc tttgctgccc ccagacagcg ccagtccacc cttgtgctct tccctggaga     120

cctgagaacc aatctcaccg acaggcagct ggcagaggaa tacctgtacc gctatggtta     180

cactcgggtg gcagagatgc gtggagagtc gaaatctctg gggcctgcgc tgctgcttct     240

ccagaagcaa ctgtccctgc ccgagaccgg tgagctggat agcgccacgc tgaaggccat     300

gcgaacccca cggtgcgggg tcccagacct gggcagattc caaacctttg agggcgacct     360

caagtggcac caccacaaca tcacctattg gatccaaaac tactcggaag acttgccgcg     420

ggcggtgatt gacgacgcct ttgcccgcgc cttcgcactg tggagcgcgg tgacgccgct     480

caccttcact cgcgtgtaca gccgggacgc agacatcgtc atccagtttg gtgtcgcgga     540

gcacggagac gggtatccct tcgacgggaa ggacgggctc ctggcacacg cctttcctcc     600

tggccccggc attcagggag acgcccattt cgacgatgac gagttgtggt ccctgggcaa     660

gggcgtcgtg gttccaactc ggtttggaaa cgcagatggc gcggcctgcc acttcccctt 720

catcttcgag ggccgctcct actctgcctg caccaccgac ggtcgctccg acggcttgcc 780

ctggtgcagt accacggcca actacgacac cgacgaccgg tttggcttct gccccagcga 840

gagactctac acccaggacg gcaatgctga tgggaaaccc tgccagtttc cattcatctt 900

ccaaggccaa tcctactccg cctgcaccac ggacggtcgc tccgacggct accgctggtg 960

cgccaccacc gccaactacg accgggacaa gctcttcggc ttctgcccga cccgagctga 1020

ctcgacggtg atggggggca actcggcggg ggagctgtgc gtcttcccct tcactttcct 1080

gggtaaggag tactcgacct gtaccagcga gggccgcgga gatgggcgcc tctggtgcgc 1140

taccacctcg aactttgaca gcgacaagaa gtggggcttc tgcccggacc aaggatacag 1200

tttgttcctc gtggcggcgc atgagttcgg ccacgcgctg ggcttagatc attcctcagt 1260

gccggaggcg ctcatgtacc ctatgtaccg cttcactgag gggcccccct tgcataagga 1320

cgacgtgaat ggcatccggc acctctatgg tcctcgccct gaacctgagc cacggcctcc 1380

aaccaccacc acaccgcagc ccacggctcc cccgacggtc tgccccaccg gaccccccac 1440

tgtccacccc tcagagcgcc ccacagctgg ccccacaggt cccccctcag ctggccccac 1500

aggtcccccc actgctggcc cttctacggc cactactgtg cctttgagtc cggtggacga 1560

tgcctgcaac gtgaacatct tcgacgccat cgcggagatt gggaaccagc tgtatttgtt 1620

caaggatggg aagtactggc gattctctga gggcaggggg agccggccgc agggcccctt 1680

ccttatcgcc gacaagtggc ccgcgctgcc ccgcaagctg gactcggtct ttgaggagcg 1740

gctctccaag aagcttttct tcttctctgg gcgccaggtg tgggtgtaca caggcgcgtc 1800

ggtgctgggc ccgaggcgtc tggacaagct gggcctggga gccgacgtgg cccaggtgac 1860

cggggccctc cggagtggca gggggaagat gctgctgttc agcgggcggc gcctctggag 1920

gttcgacgtg aaggcgcaga tggtggatcc ccggagcgcc agcgaggtgg accggatgtt 1980

ccccggggtg cctttggaca cgcacgacgt cttccagtac cgagagaaag cctatttctg 2040

ccaggaccgc ttctactggc gcgtgagttc ccggagtgag ttgaaccagg tggaccaagt 2100

gggctacgtg acctatgaca tcctgcagtg ccctgaggac tagggctccc gtcctgcttt 2160

ggcagtgcca tgtaaatccc cactgggacc aaccctgggg aaggagccag tttgccggat 2220

acaaactggt attctgttct ggaggaaagg gaggagtgga ggtgggctgg gccctctctt 2280

ctcacctttg ttttttgttg gagtgtttct aataaacttg gattctctaa cctttaaaaa 2340

aaaaaaaaaa aaaaaaaaaa aaaaaaaaaa aaaaaaaaaa aaaaaaa 2387

<210> 116
<211> 707

<212> PRT
<213> Homo sapiens

<400> 116

<210> 117
<211> 1103
<212> DNA
<213> Homo sapiens

<400> 117
cacagagccc gggccgcagg cacctcctcg ccagctcttc cgctcctctc acagccgcca 60

gacccgcctg ctgagcccca tggcccgcgc tgctctctcc gccgccccca gcaatccccg 120

gctcctgcga gtggcactgc tgctcctgct cctggtagcc gctggccggc gcgcagcagg 180

agcgtccgtg gccactgaac tgcgctgcca gtgcttgcag accctgcagg gaattcaccc 240

caagaacatc caaagtgtga acgtgaagtc ccccggaccc cactgcgccc aaaccgaagt 300

catagccaca ctcaagaatg ggcggaaagc ttgcctcaat cctgcatccc ccatagttaa 360

gaaaatcatc gaaaagatgc tgaacagtga caaatccaac tgaccagaag ggaggaggaa 420

gctcactggt ggctgttcct gaaggaggcc ctgcccttat aggaacagaa gaggaaagag 480

agacacagct gcagaggcca cctggattgt gcctaatgtg tttgagcatc gcttaggaga 540

agtcttctat ttatttattt attcattagt tttgaagatt ctatgttaat attttaggtg 600

taaaataatt aagggtatga ttaactctac ctgcacactg tcctattata ttcattcttt 660

ttgaaatgtc aaccccaagt tagttcaatc tggattcata tttaatttga aggtagaatg 720

ttttcaaatg ttctccagtc attatgttaa tatttctgag gagcctgcaa catgccagcc 780

actgtgatag aggctggcgg atccaagcaa atggccaatg agatcattgt gaaggcaggg 840

gaatgtatgt gcacatctgt tttgtaactg tttagatgaa tgtcagttgt tatttattga 900

aatgatttca cagtgtgtgg tcaacatttc tcatgttgaa actttaagaa ctaaaatgtt 960

ctaaatatcc cttggacatt ttatgtcttt cttgtaaggc atactgcctt gtttaatggt 1020

agttttacag tgtttctggc ttagaacaaa ggggcttaat tattgatgtt ttcatagaga 1080

atataaaaat aaagcactta tag 1103

<210> 118
<211> 107
<212> PRT
<213> Homo sapiens

<400> 118
Met Ala Arg Ala Ala Leu Ser Ala Ala Pro Ser Asn Pro Arg Leu Leu
1               5                   10                  15

Arg Val Ala Leu Leu Leu Leu Leu Leu Val Ala Ala Gly Arg Arg Ala
            20                  25                  30

Ala Gly Ala Ser Val Ala Thr Glu Leu Arg Cys Gln Cys Leu Gln Thr

        35                  40                  45

Leu Gln Gly Ile His Pro Lys Asn Ile Gln Ser Val Asn Val Lys Ser
    50                  55                  60

Pro Gly Pro His Cys Ala Gln Thr Glu Val Ile Ala Thr Leu Lys Asn
65                  70                  75                  80

Gly Arg Lys Ala Cys Leu Asn Pro Ala Ser Pro Ile Val Lys Lys Ile
                85                  90                  95

Ile Glu Lys Met Leu Asn Ser Asp Lys Ser Asn
            100                 105

<210> 119
<211> 1123
<212> DNA
<213> Homo sapiens

<400> 119
ctatagaaga gctatgacgt cgcatgcacg cgtacgtaag ctcggaattc ggctcgaggc 60

ttctcgcaca gcttcccgac gcgtctgctg agccccatgg cccacgccac gctctccgcc 120

gcccccagca atccccggct cctgcgggtg gcgctgctgc tcctgctcct ggtggccgcc 180

agccggcgcg cagcaggagc gtccgtggtc actgaactgc gctgccagtg cttgcagaca 240

ctgcagggaa ttcacctcaa gaacatccaa agtgtgaatg ttaggtcccc cggaccccac 300

tgcgcccaaa ccgaagtcat agccacactc aagaatggga agaaagcttg tctcaacccc 360

gcatccccca tggttcagaa aatcatcgaa aagatactga acaaggggag caccaactga 420

caggagagaa gtaagaagct tatcagcgta tcattgacac ttcctgcagg gtggtccctg 480

cccttaccag agctgaaaat gaaaaagaga acagcagctt tctagggaca gctggaaagg 540

acttaatgtg tttgactatt tcttacgagg gttctactta tttatgtatt tatttttgaa 600

agcttgtatt ttaatatttt acatgctgtt atttaaagat gtgagtgtgt ttcatcaaac 660

atagctcagt cctgattatt taattggaat atgatgggtt ttaaatgtgt cattaaacta 720

atatttagtg ggagaccata atgtgtcagc caccttgata aatgacaggg tggggaactg 780

gagggtgggg ggattgaaat gcaagcaatt agtggatcac tgttagggta agggaatgta 840

tgtacacatc tattttttat actttttttt taaaaaaaaa atgtcagttg ttatttattc 900

aaattatctc acattatgtg ttcaacattt ttatgctgaa gtttccctta gacattttat 960

gtcttgcttg tagggcataa tgccttgttt aatgtccatt ctgcagcgtt tctctttccc 1020

ttggaaaaga gaatttatca ttactgttac atttgtacaa atgacatgat aataaaagtt 1080

ttatgaaaaa aaaaaaaaaa aaaaaaaaaa aaaaaaaaaa agg                    1123

<210> 120
<211> 107
<212> PRT
<213> Homo sapiens

<400> 120
Met Ala His Ala Thr Leu Ser Ala Ala Pro Ser Asn Pro Arg Leu Leu
1               5                   10                  15

Arg Val Ala Leu Leu Leu Leu Leu Leu Val Ala Ala Ser Arg Arg Ala
            20                  25                  30

Ala Gly Ala Ser Val Val Thr Glu Leu Arg Cys Gln Cys Leu Gln Thr
        35                  40                  45

Leu Gln Gly Ile His Leu Lys Asn Ile Gln Ser Val Asn Val Arg Ser
    50                  55                  60

Pro Gly Pro His Cys Ala Gln Thr Glu Val Ile Ala Thr Leu Lys Asn
65                  70                  75                  80

Gly Lys Lys Ala Cys Leu Asn Pro Ala Ser Pro Met Val Gln Lys Ile
                85                  90                  95

Ile Glu Lys Ile Leu Asn Lys Gly Ser Thr Asn
            100                 105

<210> 121
<211> 1498
<212> DNA
<213> Homo sapiens

<400> 121
accaaacctc ttcgaggcac aaggcacaac aggctgctct gggattctct tcagccaatc      60

ttcattgctc aagtgtctga agcagccatg gcagaagtac ctgagctcgc cagtgaaatg     120

atggcttatt acagtggcaa tgaggatgac ttgttctttg aagctgatgg ccctaaacag     180

atgaagtgct ccttccagga cctggacctc tgccctctgg atggcggcat ccagctacga     240

atctccgacc accactacag caagggcttc aggcaggccg cgtcagttgt tgtggccatg     300

gacaagctga ggaagatgct ggttccctgc ccacagacct tccaggagaa tgacctgagc     360

accttctttc ccttcatctt tgaagaagaa cctatcttct tcgacacatg ggataacgag     420

gcttatgtgc acgatgcacc tgtacgatca ctgaactgca cgctccggga ctcacagcaa     480

aaaagcttgg tgatgtctgg tccatatgaa ctgaaagctc tccacctcca gggacaggat     540

atggagcaac aagtggtgtt ctccatgtcc tttgtacaag gagaagaaag taatgacaaa    600

atacctgtgg ccttgggcct caaggaaaag aatctgtacc tgtcctgcgt gttgaaagat    660

gataagccca ctctacagct ggagagtgta gatcccaaaa attacccaaa gaagaagatg    720

gaaaagcgat ttgtcttcaa caagatagaa atcaataaca agctggaatt tgagtctgcc    780

cagttcccca actggtacat cagcacctct caagcagaaa acatgcccgt cttcctggga    840

gggaccaaag gcggccagga tataactgac ttcaccatgc aatttgtgtc ttcctaaaga    900

gagctgtacc cagagagtcc tgtgctgaat gtggactcaa tccctagggc tggcagaaag    960

ggaacagaaa ggtttttgag tacggctata gcctggactt tcctgttgtc tacaccaatg   1020

cccaactgcc tgccttaggg tagtgctaag aggatctcct gtccatcagc caggacagtc   1080

agctctctcc tttcagggcc aatccccagc ccttttgttg agccaggcct ctctcacctc   1140

tcctactcac ttaaagcccg cctgacagaa accacggcca catttggttc taagaaaccc   1200

tctgtcattc gctcccacat tctgatgagc aaccgcttcc ctatttattt atttatttgt   1260

ttgtttgttt tattcattgg tctaatttat tcaaaggggg caagaagtag cagtgtctgt   1320

aaaagagcct agtttttaat agctatggaa tcaattcaat ttggactggt gtgctctctt   1380

taaatcaagt cctttaatta agactgaaaa tatataagct cagattattt aaatgggaat   1440

atttataaat gagcaaatat catactgttc aatggttctg aaataaactt cactgaag     1498

<210> 122
<211> 269
<212> PRT
<213> Homo sapiens

<400> 122
Met Ala Glu Val Pro Glu Leu Ala Ser Glu Met Met Ala Tyr Tyr Ser
1               5                   10                  15

Gly Asn Glu Asp Asp Leu Phe Phe Glu Ala Asp Gly Pro Lys Gln Met
            20                  25                  30

Lys Cys Ser Phe Gln Asp Leu Asp Leu Cys Pro Leu Asp Gly Gly Ile
        35                  40                  45

Gln Leu Arg Ile Ser Asp His His Tyr Ser Lys Gly Phe Arg Gln Ala
    50                  55                  60

Ala Ser Val Val Val Ala Met Asp Lys Leu Arg Lys Met Leu Val Pro
65                  70                  75                  80

Cys Pro Gln Thr Phe Gln Glu Asn Asp Leu Ser Thr Phe Phe Pro Phe
                85                  90                  95

Ile Phe Glu Glu Glu Pro Ile Phe Phe Asp Thr Trp Asp Asn Glu Ala
            100                 105                 110

Tyr Val His Asp Ala Pro Val Arg Ser Leu Asn Cys Thr Leu Arg Asp
        115                 120                 125

Ser Gln Gln Lys Ser Leu Val Met Ser Gly Pro Tyr Glu Leu Lys Ala
    130                 135                 140

Leu His Leu Gln Gly Gln Asp Met Glu Gln Gln Val Val Phe Ser Met
145                 150                 155                 160

Ser Phe Val Gln Gly Glu Glu Ser Asn Asp Lys Ile Pro Val Ala Leu
                165                 170                 175

Gly Leu Lys Glu Lys Asn Leu Tyr Leu Ser Cys Val Leu Lys Asp Asp
            180                 185                 190

Lys Pro Thr Leu Gln Leu Glu Ser Val Asp Pro Lys Asn Tyr Pro Lys
        195                 200                 205

Lys Lys Met Glu Lys Arg Phe Val Phe Asn Lys Ile Glu Ile Asn Asn
    210                 215                 220

Lys Leu Glu Phe Glu Ser Ala Gln Phe Pro Asn Trp Tyr Ile Ser Thr
225                 230                 235                 240

Ser Gln Ala Glu Asn Met Pro Val Phe Leu Gly Gly Thr Lys Gly Gly
                245                 250                 255

Gln Asp Ile Thr Asp Phe Thr Met Gln Phe Val Ser Ser
            260                 265

<210> 123
<211> 1726
<212> DNA
<213> Homo sapiens

<400> 123
gtcaggttca tggttacgaa gctgctgacc ccaggatccc agcccgtggg agagaagggg      60

gtctctgaca gcccccaccc ctccccactg ccagatcctt attgggtctg agtttcaggg     120

gtggggcccc agctggaggt tataaaacag ctcaatcggg gagtacaacc ttcggtttct     180

cttcggggaa agctgctttc agcgcacacg ggaagatatc agaaacatcc taggatcagg     240

acaccccaga tcttctcaac tggaaccacg aaggctgttt cttccacaca gcactttgat     300

ctccatttaa gcaggcacct ctgtcctgcg ttccggagct gcgttcccga tggtcctcct 360

ttggctcacg ctgctcctga tcgccctgcc ctgtctcctg caaacgaagg aagatccaaa 420

cccaccaatc acgaacctaa ggatgaaagc aaaggctcag cagttgacct gggaccttaa 480

cagaaatgtg accgatatcg agtgtgttaa agacgccgac tattctatgc cggcagtgaa 540

caatagctat tgccagtttg gagcaatttc cttatgtgaa gtgaccaact acaccgtccg 600

agtggccaac ccaccattct ccacgtggat cctcttccct gagaacagtg ggaagccttg 660

ggcaggtgcg gagaatctga cctgctggat tcatgacgtg gatttcttga gctgcagctg 720

ggcggtaggc ccgggggccc ccgcggacgt ccagtacgac ctgtacttga acgttgccaa 780

caggcgtcaa cagtacgagt gtcttcacta caaaacggat gctcagggaa cacgtatcgg 840

gtgtcgtttc gatgacatct ctcgactctc cagcggttct caaagttccc acatcctggt 900

gcggggcagg agcgcagcct tcggtatccc ctgcacagat aagtttgtcg tcttttcaca 960

gattgagata ttaactccac ccaacatgac tgcaaagtgt aataagacac attcctttat 1020

gcactggaaa atgagaagtc atttcaatcg caaatttcgc tatgagcttc agatacaaaa 1080

gagaatgcag cctgtaatca cagaacaggt cagagacaga acctccttcc agctactcaa 1140

tcctggaacg tacacagtac aaataagagc ccgggaaaga gtgtatgaat tcttgagcgc 1200

ctggagcacc ccccagcgct tcgagtgcga ccaggaggag ggcgcaaaca cacgtgcctg 1260

gcggacgtcg ctgctgatcg cgctggggac gctgctggcc ctggtctgtg tcttcgtgat 1320

ctgcagaagg tatctggtga tgcagagact ctttccccgc atccctcaca tgaaagaccc 1380

catcggtgac agcttccaaa acgacaagct ggtggtctgg gaggcgggca aagccggcct 1440

ggaggagtgt ctggtgactg aagtacaggt cgtgcagaaa acttgagact ggggttcagg 1500

gcttgtgggg gtctgcctca atctccctgg ccgggccagg cgcctgcaca gactggctgc 1560

tggacctgcg cacgcagccc aggaatggac attcctaacg ggtggtgggc atgggagatg 1620

cctgtgtaat ttcgtccgaa gctgccagga agaagaacag aactttgtgt gtttatttca 1680

tgataaagtg attttttttt ttttaaccca aaaaaaaaaa aaaaaa 1726

<210> 124
<211> 378
<212> PRT
<213> Homo sapiens

<400> 124
Met Val Leu Leu Trp Leu Thr Leu Leu Leu Ile Ala Leu Pro Cys Leu
1               5                   10                  15

Leu Gln Thr Lys Glu Asp Pro Asn Pro Pro Ile Thr Asn Leu Arg Met

20                  25                  30

Lys Ala Lys Ala Gln Gln Leu Thr Trp Asp Leu Asn Arg Asn Val Thr
        35                  40                  45

Asp Ile Glu Cys Val Lys Asp Ala Asp Tyr Ser Met Pro Ala Val Asn
    50                  55                  60

Asn Ser Tyr Cys Gln Phe Gly Ala Ile Ser Leu Cys Glu Val Thr Asn
65                  70                  75                  80

Tyr Thr Val Arg Val Ala Asn Pro Pro Phe Ser Thr Trp Ile Leu Phe
                85                  90                  95

Pro Glu Asn Ser Gly Lys Pro Trp Ala Gly Ala Glu Asn Leu Thr Cys
            100                 105                 110

Trp Ile His Asp Val Asp Phe Leu Ser Cys Ser Trp Ala Val Gly Pro
        115                 120                 125

Gly Ala Pro Ala Asp Val Gln Tyr Asp Leu Tyr Leu Asn Val Ala Asn
    130                 135                 140

Arg Arg Gln Gln Tyr Glu Cys Leu His Tyr Lys Thr Asp Ala Gln Gly
145                 150                 155                 160

Thr Arg Ile Gly Cys Arg Phe Asp Asp Ile Ser Arg Leu Ser Ser Gly
                165                 170                 175

Ser Gln Ser Ser His Ile Leu Val Arg Gly Arg Ser Ala Ala Phe Gly
            180                 185                 190

Ile Pro Cys Thr Asp Lys Phe Val Val Phe Ser Gln Ile Glu Ile Leu
        195                 200                 205

Thr Pro Pro Asn Met Thr Ala Lys Cys Asn Lys Thr His Ser Phe Met
    210                 215                 220

His Trp Lys Met Arg Ser His Phe Asn Arg Lys Phe Arg Tyr Glu Leu
225                 230                 235                 240

Gln Ile Gln Lys Arg Met Gln Pro Val Ile Thr Glu Gln Val Arg Asp
                245                 250                 255

Arg Thr Ser Phe Gln Leu Leu Asn Pro Gly Thr Tyr Thr Val Gln Ile
            260                 265                 270

Arg Ala Arg Glu Arg Val Tyr Glu Phe Leu Ser Ala Trp Ser Thr Pro
        275                 280                 285

Gln Arg Phe Glu Cys Asp Gln Glu Glu Gly Ala Asn Thr Arg Ala Trp
    290                 295                 300

Arg Thr Ser Leu Leu Ile Ala Leu Gly Thr Leu Leu Ala Leu Val Cys
305                 310                 315                 320

Val Phe Val Ile Cys Arg Arg Tyr Leu Val Met Gln Arg Leu Phe Pro
                325                 330                 335

Arg Ile Pro His Met Lys Asp Pro Ile Gly Asp Ser Phe Gln Asn Asp
            340                 345                 350

Lys Leu Val Val Trp Glu Ala Gly Lys Ala Gly Leu Glu Glu Cys Leu
        355                 360                 365

Val Thr Glu Val Gln Val Val Gln Lys Thr
    370                 375

<210> 125
<211> 1301
<212> DNA
<213> Homo sapiens

<400> 125
gggaggagag aaaagccatg gccgacaagg tcctgaagga gaagagaaag ctgtttatcc      60

gttccatggg tgaaggtaca ataaatggct tactggatga attattacag acaagggtgc     120

tgaacaagga agagatggag aaagtaaaac gtgaaaatgc tacagttatg gataagaccc     180

gagctttgat tgactccgtt attccgaaag gggcacaggc atgccaaatt tgcatcacat     240

acatttgtga agaagacagt tacctggcag ggacgctggg actctcagca gctcctcagg     300

cagtgcagga caacccagct atgcccacat cctcaggctc agaagggaat gtcaagcttt     360

gctccctaga agaagctcaa aggatatgga aacaaaagtc ggcagagatt tatccaataa     420

tggacaagtc aagccgcaca cgtcttgctc tcattatctg caatgaagaa tttgacagta     480

ttcctagaag aactggagct gaggttgaca tcacaggcat gacaatgctg ctacaaaatc     540

tggggtacag cgtagatgtg aaaaaaaatc tcactgcttc ggacatgact acagagctgg     600

aggcatttgc acaccgccca gagcacaaga cctctgacag cacgttcctg gtgttcatgt     660

ctcatggtat tcgggaaggc atttgtggga agaaacactc tgagcaagtc ccagatatac     720

tacaactcaa tgcaatcttt aacatgttga ataccaagaa ctgcccaagt ttgaaggaca     780

aaccgaaggt gatcatcatc caggcctgcc gtggtgacag ccctggtgtg gtgtggttta 840

aagattcagt aggagtttct ggaaacctat ctttaccaac tacagaagag tttgaggatg 900

atgctattaa gaaagcccac atagagaagg attttatcgc tttctgctct tccacaccag 960

ataatgtttc ttggagacat cccacaatgg gctctgtttt tattggaaga ctcattgaac 1020

atatgcaaga atatgcctgt tcctgtgatg tggaggaaat tttccgcaag gttcgatttt 1080

catttgagca gccagatggt agagcgcaga tgcccaccac tgaaagagtg actttgacaa 1140

gatgtttcta cctcttccca ggacattaaa ataaggaaac tgtatgaatg tctgtgggca 1200

ggaagtgaag agatccttct gtaaaggttt ttggaattat gtctgctgaa taataaactt 1260

ttttgaaata ataaatctgg tagaaaaatg aaaaaaaaaa a 1301

<210> 126
<211> 383
<212> PRT
<213> Homo sapiens

<400> 126
Met Ala Asp Lys Val Leu Lys Glu Lys Arg Lys Leu Phe Ile Arg Ser
1               5                   10                  15

Met Gly Glu Gly Thr Ile Asn Gly Leu Leu Asp Glu Leu Leu Gln Thr
            20                  25                  30

Arg Val Leu Asn Lys Glu Glu Met Glu Lys Val Lys Arg Glu Asn Ala
        35                  40                  45

Thr Val Met Asp Lys Thr Arg Ala Leu Ile Asp Ser Val Ile Pro Lys
    50                  55                  60

Gly Ala Gln Ala Cys Gln Ile Cys Ile Thr Tyr Ile Cys Glu Glu Asp
65                  70                  75                  80

Ser Tyr Leu Ala Gly Thr Leu Gly Leu Ser Ala Ala Pro Gln Ala Val
                85                  90                  95

Gln Asp Asn Pro Ala Met Pro Thr Ser Ser Gly Ser Glu Gly Asn Val
            100                 105                 110

Lys Leu Cys Ser Leu Glu Glu Ala Gln Arg Ile Trp Lys Gln Lys Ser
        115                 120                 125

Ala Glu Ile Tyr Pro Ile Met Asp Lys Ser Ser Arg Thr Arg Leu Ala
    130                 135                 140

Leu Ile Ile Cys Asn Glu Glu Phe Asp Ser Ile Pro Arg Arg Thr Gly
145                 150                 155                 160

Ala Glu Val Asp Ile Thr Gly Met Thr Met Leu Leu Gln Asn Leu Gly
                165                 170                 175

Tyr Ser Val Asp Val Lys Lys Asn Leu Thr Ala Ser Asp Met Thr Thr
            180                 185                 190

Glu Leu Glu Ala Phe Ala His Arg Pro Glu His Lys Thr Ser Asp Ser
        195                 200                 205

Thr Phe Leu Val Phe Met Ser His Gly Ile Arg Glu Gly Ile Cys Gly
    210                 215                 220

Lys Lys His Ser Glu Gln Val Pro Asp Ile Leu Gln Leu Asn Ala Ile
225                 230                 235                 240

Phe Asn Met Leu Asn Thr Lys Asn Cys Pro Ser Leu Lys Asp Lys Pro
                245                 250                 255

Lys Val Ile Ile Ile Gln Ala Cys Arg Gly Asp Ser Pro Gly Val Val
            260                 265                 270

Trp Phe Lys Asp Ser Val Gly Val Ser Gly Asn Leu Ser Leu Pro Thr
        275                 280                 285

Thr Glu Glu Phe Glu Asp Asp Ala Ile Lys Lys Ala His Ile Glu Lys
    290                 295                 300

Asp Phe Ile Ala Phe Cys Ser Ser Thr Pro Asp Asn Val Ser Trp Arg
305                 310                 315                 320

His Pro Thr Met Gly Ser Val Phe Ile Gly Arg Leu Ile Glu His Met
                325                 330                 335

Gln Glu Tyr Ala Cys Ser Cys Asp Val Glu Glu Ile Phe Arg Lys Val
            340                 345                 350

Arg Phe Ser Phe Glu Gln Pro Asp Gly Arg Ala Gln Met Pro Thr Thr
        355                 360                 365

Glu Arg Val Thr Leu Thr Arg Cys Phe Tyr Leu Phe Pro Gly His
    370                 375                 380

<210> 127
<211> 1406
<212> DNA
<213> Homo sapiens

<400> 127
gagggcgcca tgaggagcct gtgctgcgcc ccactcctgc tcctcttgct gctgccgccg      60

ctgctgctca cgccccgcgc tggggacgcc gccgtgatca ccggggcttg tgacaaggac     120

tcccaatgtg gtggaggcat gtgctgtgct gtcagtatct gggtcaagag cataaggatt     180

tgcacaccta tgggcaaact gggagacagc tgccatccac tgactcgtaa agttccattt     240

tttgggcgga ggatgcatca cacttgccca tgtctgccag gcttggcctg tttacggact     300

tcatttaacc gatttatttg tttagcccaa aagtaatcgc tctggagtag aaaccaaatg     360

tgaatagcca catcttacct gtaaagtctt acttgtgatt gtgccaaaca aaaaatgtgc     420

cagaaagaaa tgctcttgct tcctcaactt tccaagtaac atttttatct ttgatttgta     480

aatgattttt tttttttttt ttatcgaaag agaattttac ttttggatag aaatatgaag     540

tgtaaggcat tatggaactg gttcttattt ccctgtttgt gttttggttt gatttggctt     600

ttttcttaaa tgtcaaaaac gtacccattt tcacaaaaat gaggaaaata agaatttgat     660

attttgttag aaaaactttt tttttttttt ctcaccaccc caagccccat ttgtgccctg     720

ccgcacaaat acacctacag cttttggtcc cttgcctctt ccacctcaaa gaatttcaag     780

gctcttacct tactttattt ttgtccattt ctcttccctc ctcttgcatt ttaaagtgga     840

gggtttgtct ctttgagttt gatggcagaa tcactgatgg gaatccagct ttttgctggc     900

atttaaatag tgaaaagagt gtatatgtga acttgacact ccaaactcct gtcatggcac     960

ggaagctagg agtgctgctg gacccttcct aaacctgtca ctcaagagga cttcagctct    1020

gctgttgggc tggtgtgtgg acagaaggaa tggaaagcca aattaattta gtccagattt    1080

ctaggtttgg gtttttctaa aaataaaaga ttacatttac ttcttttact ttttataaag    1140

ttttttttcc ttagtctcct acttagagat attctagaaa atgtcacttg aagaggaagt    1200

atttatttta atctggcaca acactaatta ccatttttaa agcggtatta agttgtaatt    1260

taaaccttgt ttgtaactga aaggtcgatt gtaatggatt gccgtttgta cctgtatcag    1320

tattgctgtg taaaaattct gtatcagaat aataacagta ctgtatatca tttgatttat    1380

tttaatatta tatccttatt tttgtc                                          1406


<210> 128
<211> 108
<212> PRT
<213> Homo sapiens

<400> 128

Met Arg Ser Leu Cys Cys Ala Pro Leu Leu Leu Leu Leu Leu Leu Pro
1               5                   10                  15

Pro Leu Leu Leu Thr Pro Arg Ala Gly Asp Ala Ala Val Ile Thr Gly
            20                  25                  30

Ala Cys Asp Lys Asp Ser Gln Cys Gly Gly Gly Met Cys Cys Ala Val
        35                  40                  45

Ser Ile Trp Val Lys Ser Ile Arg Ile Cys Thr Pro Met Gly Lys Leu
    50                  55                  60

Gly Asp Ser Cys His Pro Leu Thr Arg Lys Val Pro Phe Phe Gly Arg
65                  70                  75                  80

Arg Met His His Thr Cys Pro Cys Leu Pro Gly Leu Ala Cys Leu Arg
                85                  90                  95

Thr Ser Phe Asn Arg Phe Ile Cys Leu Ala Gln Lys
            100                 105

<210> 129
<211> 4206
<212> DNA
<213> Homo sapiens

<400> 129
ataataccta ccttgcagga ccacgacagg attaagtgag gaaaaacccc catgagagtg      60

ttttgccatt gtcaagtgag cctgagggag gctgaggggg gatcaggctg tatcatgccc     120

ccgaggacaa actttccagt ttaccctgct ccctctctct gtccctaggc tgccccaggc     180

cctgtgcaga cacaccaggc cctcagccgc agcccatgga cctgcgggtg ggccagcggc     240

ccccagtgga gcccccacca gagcccacat tgctggccct gcagcgtccc cagcgcctgc     300

accaccacct cttcctagca ggcctgcagc agcagcgctc ggtggagccc atgaggctct     360

ccatggacac gccgatgccc gagttgcagg tgggacccca ggaacaagag ctgcggcagc     420

ttctccacaa ggacaagagc aagcgaagtg ctgtagccag cagcgtggtc aagcagaagc     480

tagcggaggt gattctgaaa aaacagcagg cggccctaga aagaacagtc catcccaaca     540

gccccggcat tccctacaga accctggagc ccctggagac ggaaggagcc acccgctcca     600

tgctcagcag ctttttgcct cctgttccca gcctgcccag tgacccccca gagcacttcc     660

ctctgcgcaa gacagtctct gagcccaacc tgaagctgcg ctataagccc aagaagtccc     720

tggagcggag gaagaatcca ctgctccgaa aggagagtgc gccccccagc ctccggcggc     780

ggcccgcaga gaccctcgga gactcctccc caagtagtag cagcacgccc gcatcagggt     840

gcagctcccc caatgacagc gagcacggcc ccaatcccat cctgggctcg gaggcgctct 900

tgggccagcg gctgcggctg caggagactt ctgtggcccc gttcgccttg ccgacagtgt 960

ccttgctgcc cgcaatcact ctggggctgc ccgcccctgc cagggctgac agtgaccgca 1020

ggacccatcc gactctgggc cctcgggggc caatcctggg gagcccccac actcccctct 1080

tcctgcccca tggcttggag cccgaggctg ggggcacctt gccctctcgc ctgcagccca 1140

ttctcctcct ggacccctca ggctctcatg ccccgctgct gactgtgccc gggcttgggc 1200

ccttgccctt ccactttgcc cagtccttaa tgaccaccga gcggctctct gggtcaggcc 1260

tccactggcc actgagccgg actcgctcag agcccctgcc ccccagtgcc accgctcccc 1320

caccgccggg ccccatgcag ccccgcctgg agcagctcaa aactcacgtc caggtgatca 1380

agaggtcagc caagccgagt gagaagcccc ggctgcggca gataccctcg gctgaagacc 1440

tggagacaga tggcggggga ccgggccagg tggtggacga tggcctggag cacagggagc 1500

tgggccatgg gcagcctgag gccagaggcc ccgctcctct ccagcagcac cctcaggtgt 1560

tgctctggga acagcagcga ctggctgggc ggctcccccg gggcagcacc ggggacactg 1620

tgctgcttcc tctggcccag ggtgggcacc ggcctctgtc ccgggctcag tcttccccag 1680

ccgcacctgc ctcactgtca gccccagagc ctgccagcca ggcccgagtc ctctccagct 1740

cagagacccc tgccaggacc ctgcccttca ccacagggct gatctatgac tcggtcatgc 1800

tgaagcacca gtgctcctgc ggtgacaaca gcaggcaccc ggagcacgcc ggccgcatcc 1860

agagcatctg gtcccggctg caggagcggg ggctccggag ccagtgtgag tgtctccgag 1920

gccggaaggc ctccctggaa gagctgcagt cggtccactc tgagcggcac gtgctcctct 1980

acggcaccaa cccgctcagc cgcctcaaac tggacaacgg gaagctggca gggctcctgg 2040

cacagcggat gtttgtgatg ctgccctgtg gtggggttgg ggtggacact gacaccatct 2100

ggaatgagct tcattcctcc aatgcagccc gctgggccgc tggcagtgtc actgacctcg 2160

ccttcaaagt ggcttctcgt gagctaaaga atggtttcgc tgtggtgcgg cccccaggac 2220

accatgcaga tcattcaaca gccatgggct tctgcttctt caactcagtg gccatcgcct 2280

gccggcagct gcaacagcag agcaaggcca gcaagatcct cattgtagac tgggacgtgc 2340

accatggcaa cggcacccag caaaccttct accaagaccc cagtgtgctc tacatctccc 2400

tgcatcgcca tgacgacggc aacttcttcc cggggagtgg ggctgtggat gaggtagggg 2460

ctggcagcgg tgagggcttc aatgtcaatg tggcctgggc tggaggtctg gaccccccca 2520

tgggggatcc tgagtacctg gctgctttca ggatagtcgt gatgcccatc gcccgagagt 2580

tctctccaga cctagtcctg gtgtctgctg gatttgatgc tgctgagggt cacccggccc 2640

cactgggtgg ctaccatgtt tctgccaaat gttttggata catgacgcag caactgatga 2700

acctggcagg aggcgcagtg gtgctggcct tggagggtgg ccatgacctc acagccatct 2760

gtgacgcctc tgaggcctgt gtggctgctc ttctgggtaa cagggtggat cccctttcag 2820

aagaaggctg gaaacagaaa cccaacctca atgccatccg ctctctggag gccgtgatcc 2880

gggtgcacag taaatactgg ggctgcatgc agcgcctggc ctcctgtcca gactcctggg 2940

tgcctagagt gccaggggct gacaaagaag aagtggaggc agtgaccgca ctggcgtccc 3000

tctctgtggg catcctggct gaagataggc cctcggagca gctggtggag gaggaagaac 3060

ctatgaatct ctaaggctct ggaaccatct gcccgcccac catgcccttg ggacctggtt 3120

ctcttctaac ccctggcaat agcccccatt cctgggtctt tagagatcct gtgggcaagt 3180

agttggaacc agagaacagc ctgcctgctt tgacagttat cccagggagc gtgagaaaat 3240

ccctgggtct agaatgggaa ctggagagga ccctgagagg agacgggctg ggcggcgacc 3300

cccacagggc tctcgagaac agattctccc ctccagtatg ggccctggct gtggccccca 3360

ttcctcagga ctgcacagag gaggactggc tccggctccg tcgggctcac ccttaaccac 3420

tattcctggc tctgcaaacc ccagactttg cacacagccc caggctccac acagaaatgt 3480

gaacttggcc tcagacaggc tggcccttcc taggctctag gggctagggg ggagtgggga 3540

gccaagaggt cccatattcc tgagtgcagg ggtagtccct ctcacctgct tcctcagacg 3600

actctggaag cttccctcta ccaccgggca ctgagacgaa gctccctgac agccgagact 3660

ggcagccctc catctggtcc gtaccctcgc cagaggcccc cctacatcaa cctcctggcg 3720

atgccctggt ggagcagatg ggtgctctgg gagtcctgtg cttcctgatc caatggtgcc 3780

aaacccttca tctcccccag aagcgcagca tacccctggg acccctcggc cactgcccac 3840

tcggggagcc ttctctgttt ctggggcctc ccccaccata gctctgattc ccaccccaca 3900

taggaatagc ctgactgagg gggaaggggt gggagagaag atacagacat ggaggagggg 3960

aggctgctct ggcaaagtct tcaaggcttt tgggggtcca ggcctggggt caagaaggaa 4020

aatgtgtgtg agcatgtgtg tgagtgaggc gtgtgtgtga gcgtgtgtgt gagtgaggcg 4080

tgtgtgtgtg tctttcctag gacccaccat accctgtgta tgtatgcatg tttttgtaaa 4140

aaggaagaaa atggaaaaaa atctgaacaa taaatgtttt atttgcttta aaaaaaaaaa 4200

aaaaaa 4206

<210> 130
<211> 952
<212> PRT
<213> Homo sapiens

<400> 130

Met Asp Leu Arg Val Gly Gln Arg Pro Pro Val Glu Pro Pro Pro Glu
1               5                   10                  15

Pro Thr Leu Leu Ala Leu Gln Arg Pro Gln Arg Leu His His His Leu
            20                  25                  30

Phe Leu Ala Gly Leu Gln Gln Gln Arg Ser Val Glu Pro Met Arg Leu
        35                  40                  45

Ser Met Asp Thr Pro Met Pro Glu Leu Gln Val Gly Pro Gln Glu Gln
    50                  55                  60

Glu Leu Arg Gln Leu Leu His Lys Asp Lys Ser Lys Arg Ser Ala Val
65                  70                  75                  80

Ala Ser Ser Val Val Lys Gln Lys Leu Ala Glu Val Ile Leu Lys Lys
                85                  90                  95

Gln Gln Ala Ala Leu Glu Arg Thr Val His Pro Asn Ser Pro Gly Ile
            100                 105                 110

Pro Tyr Arg Thr Leu Glu Pro Leu Glu Thr Glu Gly Ala Thr Arg Ser
        115                 120                 125

Met Leu Ser Ser Phe Leu Pro Pro Val Pro Ser Leu Pro Ser Asp Pro
    130                 135                 140

Pro Glu His Phe Pro Leu Arg Lys Thr Val Ser Glu Pro Asn Leu Lys
145                 150                 155                 160

Leu Arg Tyr Lys Pro Lys Lys Ser Leu Glu Arg Arg Lys Asn Pro Leu
                165                 170                 175

Leu Arg Lys Glu Ser Ala Pro Pro Ser Leu Arg Arg Arg Pro Ala Glu
            180                 185                 190

Thr Leu Gly Asp Ser Ser Pro Ser Ser Ser Ser Thr Pro Ala Ser Gly
        195                 200                 205

Cys Ser Ser Pro Asn Asp Ser Glu His Gly Pro Asn Pro Ile Leu Gly
    210                 215                 220

Ser Glu Ala Leu Leu Gly Gln Arg Leu Arg Leu Gln Glu Thr Ser Val
225                 230                 235                 240

Ala Pro Phe Ala Leu Pro Thr Val Ser Leu Leu Pro Ala Ile Thr Leu

245                 250                 255

Gly Leu Pro Ala Pro Ala Arg Ala Asp Ser Asp Arg Arg Thr His Pro
            260                 265                 270

Thr Leu Gly Pro Arg Gly Pro Ile Leu Gly Ser Pro His Thr Pro Leu
        275                 280                 285

Phe Leu Pro His Gly Leu Glu Pro Glu Ala Gly Gly Thr Leu Pro Ser
    290                 295                 300

Arg Leu Gln Pro Ile Leu Leu Leu Asp Pro Ser Gly Ser His Ala Pro
305                 310                 315                 320

Leu Leu Thr Val Pro Gly Leu Gly Pro Leu Pro Phe His Phe Ala Gln
                325                 330                 335

Ser Leu Met Thr Thr Glu Arg Leu Ser Gly Ser Gly Leu His Trp Pro
            340                 345                 350

Leu Ser Arg Thr Arg Ser Glu Pro Leu Pro Pro Ser Ala Thr Ala Pro
        355                 360                 365

Pro Pro Pro Gly Pro Met Gln Pro Arg Leu Glu Gln Leu Lys Thr His
    370                 375                 380

Val Gln Val Ile Lys Arg Ser Ala Lys Pro Ser Glu Lys Pro Arg Leu
385                 390                 395                 400

Arg Gln Ile Pro Ser Ala Glu Asp Leu Glu Thr Asp Gly Gly Gly Pro
                405                 410                 415

Gly Gln Val Val Asp Asp Gly Leu Glu His Arg Glu Leu Gly His Gly
            420                 425                 430

Gln Pro Glu Ala Arg Gly Pro Ala Pro Leu Gln Gln His Pro Gln Val
        435                 440                 445

Leu Leu Trp Glu Gln Gln Arg Leu Ala Gly Arg Leu Pro Arg Gly Ser
    450                 455                 460

Thr Gly Asp Thr Val Leu Leu Pro Leu Ala Gln Gly Gly His Arg Pro
465                 470                 475                 480

Leu Ser Arg Ala Gln Ser Ser Pro Ala Ala Pro Ala Ser Leu Ser Ala
                485                 490                 495

Pro Glu Pro Ala Ser Gln Ala Arg Val Leu Ser Ser Ser Glu Thr Pro
            500                 505                 510

Ala Arg Thr Leu Pro Phe Thr Thr Gly Leu Ile Tyr Asp Ser Val Met
        515                 520                 525

Leu Lys His Gln Cys Ser Cys Gly Asp Asn Ser Arg His Pro Glu His
    530                 535                 540

Ala Gly Arg Ile Gln Ser Ile Trp Ser Arg Leu Gln Glu Arg Gly Leu
545                 550                 555                 560

Arg Ser Gln Cys Glu Cys Leu Arg Gly Arg Lys Ala Ser Leu Glu Glu
                565                 570                 575

Leu Gln Ser Val His Ser Glu Arg His Val Leu Leu Tyr Gly Thr Asn
            580                 585                 590

Pro Leu Ser Arg Leu Lys Leu Asp Asn Gly Lys Leu Ala Gly Leu Leu
        595                 600                 605

Ala Gln Arg Met Phe Val Met Leu Pro Cys Gly Gly Val Gly Val Asp
    610                 615                 620

Thr Asp Thr Ile Trp Asn Glu Leu His Ser Ser Asn Ala Ala Arg Trp
625                 630                 635                 640

Ala Ala Gly Ser Val Thr Asp Leu Ala Phe Lys Val Ala Ser Arg Glu
                645                 650                 655

Leu Lys Asn Gly Phe Ala Val Val Arg Pro Pro Gly His His Ala Asp
            660                 665                 670

His Ser Thr Ala Met Gly Phe Cys Phe Phe Asn Ser Val Ala Ile Ala
        675                 680                 685

Cys Arg Gln Leu Gln Gln Gln Ser Lys Ala Ser Lys Ile Leu Ile Val
    690                 695                 700

Asp Trp Asp Val His His Gly Asn Gly Thr Gln Gln Thr Phe Tyr Gln
705                 710                 715                 720

Asp Pro Ser Val Leu Tyr Ile Ser Leu His Arg His Asp Asp Gly Asn
                725                 730                 735

Phe Phe Pro Gly Ser Gly Ala Val Asp Glu Val Gly Ala Gly Ser Gly
            740                 745                 750

Glu Gly Phe Asn Val Asn Val Ala Trp Ala Gly Gly Leu Asp Pro Pro
        755                 760                 765

Met Gly Asp Pro Glu Tyr Leu Ala Ala Phe Arg Ile Val Val Met Pro
    770                 775                 780

Ile Ala Arg Glu Phe Ser Pro Asp Leu Val Leu Val Ser Ala Gly Phe
785                 790                 795                 800

Asp Ala Ala Glu Gly His Pro Ala Pro Leu Gly Gly Tyr His Val Ser
                805                 810                 815

Ala Lys Cys Phe Gly Tyr Met Thr Gln Gln Leu Met Asn Leu Ala Gly
            820                 825                 830

Gly Ala Val Val Leu Ala Leu Glu Gly Gly His Asp Leu Thr Ala Ile
        835                 840                 845

Cys Asp Ala Ser Glu Ala Cys Val Ala Ala Leu Leu Gly Asn Arg Val
    850                 855                 860

Asp Pro Leu Ser Glu Glu Gly Trp Lys Gln Lys Pro Asn Leu Asn Ala
865                 870                 875                 880

Ile Arg Ser Leu Glu Ala Val Ile Arg Val His Ser Lys Tyr Trp Gly
                885                 890                 895

Cys Met Gln Arg Leu Ala Ser Cys Pro Asp Ser Trp Val Pro Arg Val
            900                 905                 910

Pro Gly Ala Asp Lys Glu Glu Val Glu Ala Val Thr Ala Leu Ala Ser
        915                 920                 925

Leu Ser Val Gly Ile Leu Ala Glu Asp Arg Pro Ser Glu Gln Leu Val
    930                 935                 940

Glu Glu Glu Glu Pro Met Asn Leu
945                 950

<210> 131
<211> 1970
<212> DNA
<213> Homo sapiens

<400> 131
aggaaacggt ttattaggag ggagtggtgg agctgggcca ggcaggaaga cgctggaata      60

agaaacattt ttgctccagc ccccatccca gtcccgggag gctgccgcgc cagctgcgcc     120

gagcgagccc ctccccggct ccagcccggt ccggggccgc gccggacccc agcccgccgt     180

ccagcgctgg cggtgcaact gcggccgcgc ggtggagggg aggtggcccc ggtccgccga     240

aggctagcgc cccgccaccc gcagagcggg cccagaggga ccatgacctt gggctccccc     300

aggaaaggcc ttctgatgct gctgatggcc ttggtgaccc agggagaccc tgtgaagccg     360

tctcggggcc cgctggtgac ctgcacgtgt gagagcccac attgcaaggg gcctacctgc     420

cggggggcct ggtgcacagt agtgctggtg cgggaggagg ggaggcaccc ccaggaacat     480

cggggctgcg ggaacttgca cagggagctc tgcagggggc gccccaccga gttcgtcaac     540

cactactgct gcgacagcca cctctgcaac cacaacgtgt ccctggtgct ggaggccacc     600

caacctcctt cggagcagcc gggaacagat ggccagctgg ccctgatcct gggccccgtg     660

ctggccttgc tggccctggt ggccctgggt gtcctgggcc tgtggcatgt ccgacggagg     720

caggagaagc agcgtggcct gcacagcgag ctgggagagt ccagtctcat cctgaaagca     780

tctgagcagg gcgacacgat gttgggggac ctcctggaca gtgactgcac cacagggagt     840

ggctcagggc tccccttcct ggtgcagagg acagtggcac ggcaggttgc cttggtggag     900

tgtgtgggaa aaggccgcta tggcgaagtg tggcggggct tgtggcacgg tgagagtgtg     960

gccgtcaaga tcttctcctc gagggatgaa cagtcctggt tccgggagac tgagatctat    1020

aacacagtat tgctcagaca cgacaacatc ctaggcttca tcgcctcaga catgacctcc    1080

cgcaactcga gcacgcagct gtggctcatc acgcactacc acgagcacgg ctccctctac    1140

gactttctgc agagacagac gctggagccc catctggctc tgaggctagc tgtgtccgcg    1200

gcatgcggcc tggcgcacct gcacgtggag atcttcggta cacagggcaa accagccatt    1260

gcccaccgcg acttcaagag ccgcaatgtg ctggtcaaga gcaacctgca gtgttgcatc    1320

gccgacctgg gcctggctgt gatgcactca cagggcagcg attacctgga catcggcaac    1380

aacccgagag tgggcaccaa gcggtacatg gcacccgagg tgctggacga gcagatccgc    1440

acggactgct ttgagtccta caagtggact gacatctggg cctttggcct ggtgctgtgg    1500

gagattgccc gccggaccat cgtgaatggc atcgtggagg actatagacc acccttctat    1560

gatgtggtgc ccaatgaccc cagctttgag gacatgaaga aggtggtgtg tgtggatcag    1620

cagaccccca ccatccctaa ccggctggct gcagacccgg tcctctcagg cctagctcag    1680

atgatgcggg agtgctggta cccaaacccc tctgcccgac tcaccgcgct gcggatcaag    1740

aagacactac aaaaaattag caacagtcca gagaagccta aagtgattca atagcccagg    1800

agcacctgat tcctttctgc ctgcaggggg ctgggggggt ggggggcagt ggatggtgcc 1860

ctatctgggt agaggtagtg tgagtgtggt gtgtgctggg gatgggcagc tgcgcctgcc 1920

tgctcggccc ccagcccacc cagccaaaaa tacagctggg ctgaaacctg 1970

<210> 132
<211> 503
<212> PRT
<213> Homo sapiens

<400> 132
Met Thr Leu Gly Ser Pro Arg Lys Gly Leu Leu Met Leu Leu Met Ala
1               5                   10                  15

Leu Val Thr Gln Gly Asp Pro Val Lys Pro Ser Arg Gly Pro Leu Val
            20                  25                  30

Thr Cys Thr Cys Glu Ser Pro His Cys Lys Gly Pro Thr Cys Arg Gly
        35                  40                  45

Ala Trp Cys Thr Val Val Leu Val Arg Glu Glu Gly Arg His Pro Gln
    50                  55                  60

Glu His Arg Gly Cys Gly Asn Leu His Arg Glu Leu Cys Arg Gly Arg
65                  70                  75                  80

Pro Thr Glu Phe Val Asn His Tyr Cys Cys Asp Ser His Leu Cys Asn
                85                  90                  95

His Asn Val Ser Leu Val Leu Glu Ala Thr Gln Pro Pro Ser Glu Gln
            100                 105                 110

Pro Gly Thr Asp Gly Gln Leu Ala Leu Ile Leu Gly Pro Val Leu Ala
        115                 120                 125

Leu Leu Ala Leu Val Ala Leu Gly Val Leu Gly Leu Trp His Val Arg
    130                 135                 140

Arg Arg Gln Glu Lys Gln Arg Gly Leu His Ser Glu Leu Gly Glu Ser
145                 150                 155                 160

Ser Leu Ile Leu Lys Ala Ser Glu Gln Gly Asp Thr Met Leu Gly Asp
                165                 170                 175

Leu Leu Asp Ser Asp Cys Thr Thr Gly Ser Gly Ser Gly Leu Pro Phe
            180                 185                 190

Leu Val Gln Arg Thr Val Ala Arg Gln Val Ala Leu Val Glu Cys Val
        195                 200                 205

Gly Lys Gly Arg Tyr Gly Glu Val Trp Arg Gly Leu Trp His Gly Glu
    210                 215                 220

Ser Val Ala Val Lys Ile Phe Ser Ser Arg Asp Glu Gln Ser Trp Phe
225                 230                 235                 240

Arg Glu Thr Glu Ile Tyr Asn Thr Val Leu Leu Arg His Asp Asn Ile
                245                 250                 255

Leu Gly Phe Ile Ala Ser Asp Met Thr Ser Arg Asn Ser Ser Thr Gln
            260                 265                 270

Leu Trp Leu Ile Thr His Tyr His Glu His Gly Ser Leu Tyr Asp Phe
        275                 280                 285

Leu Gln Arg Gln Thr Leu Glu Pro His Leu Ala Leu Arg Leu Ala Val
    290                 295                 300

Ser Ala Ala Cys Gly Leu Ala His Leu His Val Glu Ile Phe Gly Thr
305                 310                 315                 320

Gln Gly Lys Pro Ala Ile Ala His Arg Asp Phe Lys Ser Arg Asn Val
                325                 330                 335

Leu Val Lys Ser Asn Leu Gln Cys Cys Ile Ala Asp Leu Gly Leu Ala
            340                 345                 350

Val Met His Ser Gln Gly Ser Asp Tyr Leu Asp Ile Gly Asn Asn Pro
        355                 360                 365

Arg Val Gly Thr Lys Arg Tyr Met Ala Pro Glu Val Leu Asp Glu Gln
    370                 375                 380

Ile Arg Thr Asp Cys Phe Glu Ser Tyr Lys Trp Thr Asp Ile Trp Ala
385                 390                 395                 400

Phe Gly Leu Val Leu Trp Glu Ile Ala Arg Arg Thr Ile Val Asn Gly
                405                 410                 415

Ile Val Glu Asp Tyr Arg Pro Pro Phe Tyr Asp Val Val Pro Asn Asp
            420                 425                 430

Pro Ser Phe Glu Asp Met Lys Lys Val Val Cys Val Asp Gln Gln Thr
        435                 440                 445

Pro Thr Ile Pro Asn Arg Leu Ala Ala Asp Pro Val Leu Ser Gly Leu
    450                 455                 460

Ala Gln Met Met Arg Glu Cys Trp Tyr Pro Asn Pro Ser Ala Arg Leu
465                 470                 475                 480

Thr Ala Leu Arg Ile Lys Lys Thr Leu Gln Lys Ile Ser Asn Ser Pro
                485                 490                 495

Glu Lys Pro Lys Val Ile Gln
            500

<210> 133
<211> 2464
<212> DNA
<213> Homo sapiens

<400> 133
cggaaacttg ggggagtgca cagaagaact tcgggagcgc acgcgggacc agggaccagg      60

ctgagactcg gggcgccagt ccgggcaggg gcagcgggac gcggccggag atgccctgta     120

tccaagccca atatgggaca ccagcaccga gtccgggacc ccgtgaccac ctggcaagcg     180

accccctgac ccctgagttc atcaagccca ccatggacct ggccagcccc gaggcagccc     240

ccgctgcccc cactgccctg cccagcttca gcacgttcat ggacggctac acaggagagt     300

ttgacacctt cctctaccag ctgccaggaa cagtccagcc atgctcctca gcctcctcct     360

cggcctcctc cacatcctcg tcctcagcca cctcccctgc ctctgcctcc ttcaagttcg     420

aggacttcca ggtgtacggc tgctaccccg gccccctgag cggcccagtg gatgaggccc     480

tgtcctccag tggctctgac tactatggca gcccctgctc ggccccgtcg ccctccacgc     540

ccagcttcca gccgccccag ctctctccct gggatggctc cttcggccac ttctcgccca     600

gccagactta cgaaggcctg cgggcatgga cagagcagct gcccaaagcc tctgggcccc     660

cacagcctcc agccttcttt tccttcagtc ctcccactgg ccccagcccc agcctggccc     720

agagccccct gaagttgttc ccctcacagg ccacccacca gctgggggag ggagagagct     780

attccatgcc tacggccttc ccaggtttgg cacccacttc tccacacctt gagggctcgg     840

ggatactgga tacacccgtg acctcaacca aggcccggag cggggcccca ggtccaagtg     900

aaggccgctg tgctgtgtgt ggggacaacg cttcatgcca gcattatggt gtccgcacat     960

gtgagggctg caagggcttc ttcaagcgca cagtgcagaa aaacgccaag tacatctgcc    1020

tggctaacaa ggactgccct gtggacaaga ggcggcgaaa ccgctgccag ttctgccgct    1080

tccagaagtg cctggcggtg ggcatggtga aggaagttgt ccgaacagac agcctgaagg 1140

ggcggcgggg ccggctacct tcaaaaccca agcagccccc agatgcctcc cctgccaatc 1200

tcctcacttc cctggtcctt gcacacctgg attcagggcc cagcactgcc aaactggact 1260

actccaagtt ccaggagctg gtgctgcccc actttgggaa ggaagatgct ggggatgtac 1320

agcagttcta cgacctgctc tccggttctc tggaggtcat ccgaaagtgg gcggagaaga 1380

tccctggctt tgctgagctg tcaccggctg accaggacct gttgctggag tcggccttcc 1440

tggagctctt catcctccgc ctggcgtaca ggtctaagcc aggcgagggc aagctcatct 1500

tctgctcagg cctggtgcta caccggctgc agtgtgcccg tggcttcggg gactggattg 1560

acagtatcct ggccttctca aggtccctgc acagcttgct tgtcgatgtc cctgccttcg 1620

cctgcctctc tgcccttgtc ctcatcaccg accggcatgg gctgcaggag ccgcggcggg 1680

tggaggagct gcagaaccgc atcgccagct gcctgaagga gcacgtggca gctgtggcgg 1740

gcgagcccca gccagccagc tgcctgtcac gtctgttggg caaactgccc gagctgcgga 1800

ccctgtgcac ccagggcctg cagcgcatct tctacctcaa gctggaggac ttggtgcccc 1860

ctccacccat cattgacaag atcttcatgg acacgctgcc cttctgaccc ctgcctggga 1920

acacgtgtgc acatgcgcac tctcatatgc caccccatgt gcctttagtc cacggacccc 1980

cagagcaccc ccaagcctgg gcttgagctg cagaatgact ccaccttctc acctgctcca 2040

ggaggtttgc agggagctca agcccttggg gagggggatg ccttcatggg ggtgacccca 2100

cgatttgtct tatccccccc agcctggccc cggcctttat gttttttgta agataaaccg 2160

tttttaacac atagcgccgt gctgtaaata agcccagtgc tgctgtaaat acaggaagaa 2220

agagcttgag gtgggagcgg ggctgggagg aagggatggg ccccgccttc ctgggcagcc 2280

tttccagcct cctgcctggc tctctcttcc taccctcctt ccacatgtac ataaactgtc 2340

actctaggaa gaagacaaat gacagattct gacatttata tttgtgtatt ttcctggatt 2400

tatagtatgt gacttttctg attaatatat ttaatatatt gaataaaaaa tagacatgta 2460

gttg 2464

<210> 134
<211> 598
<212> PRT
<213> Homo sapiens

<400> 134
Met Pro Cys Ile Gln Ala Gln Tyr Gly Thr Pro Ala Pro Ser Pro Gly
1               5                   10                  15

Pro Arg Asp His Leu Ala Ser Asp Pro Leu Thr Pro Glu Phe Ile Lys

            20                  25                  30

Pro Thr Met Asp Leu Ala Ser Pro Glu Ala Ala Pro Ala Ala Pro Thr
        35                  40                  45

Ala Leu Pro Ser Phe Ser Thr Phe Met Asp Gly Tyr Thr Gly Glu Phe
    50                  55                  60

Asp Thr Phe Leu Tyr Gln Leu Pro Gly Thr Val Gln Pro Cys Ser Ser
65                  70                  75                  80

Ala Ser Ser Ser Ala Ser Ser Thr Ser Ser Ser Ser Ala Thr Ser Pro
                85                  90                  95

Ala Ser Ala Ser Phe Lys Phe Glu Asp Phe Gln Val Tyr Gly Cys Tyr
            100                 105                 110

Pro Gly Pro Leu Ser Gly Pro Val Asp Glu Ala Leu Ser Ser Ser Gly
        115                 120                 125

Ser Asp Tyr Tyr Gly Ser Pro Cys Ser Ala Pro Ser Pro Ser Thr Pro
    130                 135                 140

Ser Phe Gln Pro Pro Gln Leu Ser Pro Trp Asp Gly Ser Phe Gly His
145                 150                 155                 160

Phe Ser Pro Ser Gln Thr Tyr Glu Gly Leu Arg Ala Trp Thr Glu Gln
                165                 170                 175

Leu Pro Lys Ala Ser Gly Pro Pro Gln Pro Pro Ala Phe Phe Ser Phe
            180                 185                 190

Ser Pro Pro Thr Gly Pro Ser Pro Ser Leu Ala Gln Ser Pro Leu Lys
        195                 200                 205

Leu Phe Pro Ser Gln Ala Thr His Gln Leu Gly Glu Gly Glu Ser Tyr
    210                 215                 220

Ser Met Pro Thr Ala Phe Pro Gly Leu Ala Pro Thr Ser Pro His Leu
225                 230                 235                 240

Glu Gly Ser Gly Ile Leu Asp Thr Pro Val Thr Ser Thr Lys Ala Arg
                245                 250                 255

Ser Gly Ala Pro Gly Pro Ser Glu Gly Arg Cys Ala Val Cys Gly Asp
            260                 265                 270

Asn Ala Ser Cys Gln His Tyr Gly Val Arg Thr Cys Glu Gly Cys Lys
        275                 280                 285

Gly Phe Phe Lys Arg Thr Val Gln Lys Asn Ala Lys Tyr Ile Cys Leu
    290                 295                 300

Ala Asn Lys Asp Cys Pro Val Asp Lys Arg Arg Arg Asn Arg Cys Gln
305                 310                 315                 320

Phe Cys Arg Phe Gln Lys Cys Leu Ala Val Gly Met Val Lys Glu Val
                325                 330                 335

Val Arg Thr Asp Ser Leu Lys Gly Arg Arg Gly Arg Leu Pro Ser Lys
            340                 345                 350

Pro Lys Gln Pro Pro Asp Ala Ser Pro Ala Asn Leu Leu Thr Ser Leu
        355                 360                 365

Val Leu Ala His Leu Asp Ser Gly Pro Ser Thr Ala Lys Leu Asp Tyr
    370                 375                 380

Ser Lys Phe Gln Glu Leu Val Leu Pro His Phe Gly Lys Glu Asp Ala
385                 390                 395                 400

Gly Asp Val Gln Gln Phe Tyr Asp Leu Leu Ser Gly Ser Leu Glu Val
                405                 410                 415

Ile Arg Lys Trp Ala Glu Lys Ile Pro Gly Phe Ala Glu Leu Ser Pro
            420                 425                 430

Ala Asp Gln Asp Leu Leu Leu Glu Ser Ala Phe Leu Glu Leu Phe Ile
        435                 440                 445

Leu Arg Leu Ala Tyr Arg Ser Lys Pro Gly Glu Gly Lys Leu Ile Phe
    450                 455                 460

Cys Ser Gly Leu Val Leu His Arg Leu Gln Cys Ala Arg Gly Phe Gly
465                 470                 475                 480

Asp Trp Ile Asp Ser Ile Leu Ala Phe Ser Arg Ser Leu His Ser Leu
                485                 490                 495

Leu Val Asp Val Pro Ala Phe Ala Cys Leu Ser Ala Leu Val Leu Ile
            500                 505                 510

Thr Asp Arg His Gly Leu Gln Glu Pro Arg Arg Val Glu Glu Leu Gln
        515                 520                 525

Asn Arg Ile Ala Ser Cys Leu Lys Glu His Val Ala Ala Val Ala Gly
    530                 535                 540

Glu Pro Gln Pro Ala Ser Cys Leu Ser Arg Leu Leu Gly Lys Leu Pro
545                 550                 555                 560

Glu Leu Arg Thr Leu Cys Thr Gln Gly Leu Gln Arg Ile Phe Tyr Leu
                565                 570                 575

Lys Leu Glu Asp Leu Val Pro Pro Pro Pro Ile Ile Asp Lys Ile Phe
            580                 585                 590

Met Asp Thr Leu Pro Phe
        595

<210> 135
<211> 1978
<212> DNA
<213> Homo sapiens

<400> 135
agaaactcac acaccttgac aatctgtctg tccgtctgca gctgcgtgac tgtctgtctc      60

tgccatgtct ctctccccat gccgggccca gaggggcttc agcgctcgct cagcctgttc     120

tgctcgctca aggggccgca gcaggggagg cttcagcagc aggggcggct tcagcagcag     180

gagccttaat tcctttgggg ggtgcctgga aggctctcgt gggagtacct gggggtcagg     240

gggtaggctg ggggtgcggt ttggggagtg gagtggtggg cctgggctct ccctgtgccc     300

tccggggggc atccaagaag tgaccatcaa ccagaatccg ctgaccccac tgaagattga     360

gatcgatccc cagttccagg tggtgcggac gcaggagacc caggagatca gaaccctcaa     420

caaccagttt gcttccttca ttgacaaggt gcggttcctg gagcagcaga acaaggtcct     480

ggagacgaag tggcatctgc tgcagcaaca ggggttgagt ggcagccagc agggcctgga     540

gcctgtcttt gaggcctgcc tggatcagct caggaagcag ctggagcagc tccagggaga     600

acgaggggct ctggatgctg agttgaaggc ctgccgggac caggaggagg agtataagtc     660

caagtatgag gaggaggccc acaggcgtgc cacacttgag aacgactttg tggtcctcaa     720

gaaggatgtg gatggggttt tcctgagcaa gatggagttg gagggcaagc tggaggctct     780

gagagagtac ctctacttct tgaagcatct gaatgaagaa gagctgggcc agctccaggc     840

ccaggccagc gacacgtctg tggtgctgtc catggacaac aaccgctacc tggacttcag     900

cagcatcatc actgaggtcc gcgcccggta cgaggagatc gcccggagca gcaaggctga 960

ggctgaggcc ttgtaccaga ccaagtacca ggaacttcag gtgtctgccc agcttcatgg 1020

ggacaggatg caggaaacga aagtccagat ctctcagcta caccaagaga ttcagaggct 1080

gcagagtcag actgagaacc tcaagaagca gaacgccagc ctgcaggccg ccatcactga 1140

tgctgagcag cgtggggagc tggccctcaa ggacgctcag gccaaggtgg acgagctgga 1200

ggctgctctg aggatggcca agcagaacct ggcccggctg ctgtgcgagt accaggagct 1260

gacgagcacg aagctttccc tggatgtgga gattgccact taccgcaggc tgctggaggg 1320

cgaggagtgc aggatgtctg gggagtgcac cagccaggtc actatctcct cggtgggagg 1380

cagcgctgtc atgtctggag gagttggtgg aggcttgggg agcacttgtg gactcggtag 1440

tgggaaaggc agccctgggt cctgctgcac cagcattgtg actggaggct ccaacatcat 1500

tctgggctct gggaaggacc ctgttttgga ttcctgctct gtgtctggct ccagcgctgg 1560

ctccagctgc cacaccatcc tgaagaagac agttgagtcg agtctgaaga catccatcac 1620

ctactgagcg acccagcagc cacctccttc ctgaacacat ttggcccact ccccccatca 1680

gccggctctg caaggccaac tccgtgtccg ctgcccacag cccaagccag cccacagcgg 1740

atgctgcaaa aatcaataaa gtctcccctc ctgctgttct gaatgctcta agtgcttgca 1800

cacctcaccc agcaaaacaa aagctgtgtg gctccccagc ttcctctcct cggaaggcaa 1860

ttgtggcata tctgtgtcca gacgtgttct attggtatct cactccacat cccagccact 1920

ctatcaagga aactaaattc atccaaggta gttcaattaa attgactttt tgaaaagt 1978

<210> 136
<211> 520
<212> PRT
<213> Homo sapiens

<400> 136
Met Ser Leu Ser Pro Cys Arg Ala Gln Arg Gly Phe Ser Ala Arg Ser
1               5                   10                  15

Ala Cys Ser Ala Arg Ser Arg Gly Arg Ser Arg Gly Gly Phe Ser Ser
            20                  25                  30

Arg Gly Gly Phe Ser Ser Arg Ser Leu Asn Ser Phe Gly Gly Cys Leu
        35                  40                  45

Glu Gly Ser Arg Gly Ser Thr Trp Gly Ser Gly Gly Arg Leu Gly Val
    50                  55                  60

Arg Phe Gly Glu Trp Ser Gly Gly Pro Gly Leu Ser Leu Cys Pro Pro
65                  70                  75                  80

Gly Gly Ile Gln Glu Val Thr Ile Asn Gln Asn Pro Leu Thr Pro Leu
                85                  90                  95

Lys Ile Glu Ile Asp Pro Gln Phe Gln Val Val Arg Thr Gln Glu Thr
            100                 105                 110

Gln Glu Ile Arg Thr Leu Asn Asn Gln Phe Ala Ser Phe Ile Asp Lys
        115                 120                 125

Val Arg Phe Leu Glu Gln Gln Asn Lys Val Leu Glu Thr Lys Trp His
    130                 135                 140

Leu Leu Gln Gln Gln Gly Leu Ser Gly Ser Gln Gln Gly Leu Glu Pro
145                 150                 155                 160

Val Phe Glu Ala Cys Leu Asp Gln Leu Arg Lys Gln Leu Glu Gln Leu
                165                 170                 175

Gln Gly Glu Arg Gly Ala Leu Asp Ala Glu Leu Lys Ala Cys Arg Asp
            180                 185                 190

Gln Glu Glu Glu Tyr Lys Ser Lys Tyr Glu Glu Glu Ala His Arg Arg
        195                 200                 205

Ala Thr Leu Glu Asn Asp Phe Val Val Leu Lys Lys Asp Val Asp Gly
    210                 215                 220

Val Phe Leu Ser Lys Met Glu Leu Glu Gly Lys Leu Glu Ala Leu Arg
225                 230                 235                 240

Glu Tyr Leu Tyr Phe Leu Lys His Leu Asn Glu Glu Glu Leu Gly Gln
                245                 250                 255

Leu Gln Ala Gln Ala Ser Asp Thr Ser Val Val Leu Ser Met Asp Asn
            260                 265                 270

Asn Arg Tyr Leu Asp Phe Ser Ser Ile Ile Thr Glu Val Arg Ala Arg
        275                 280                 285

Tyr Glu Glu Ile Ala Arg Ser Ser Lys Ala Glu Ala Glu Ala Leu Tyr
    290                 295                 300

Gln Thr Lys Tyr Gln Glu Leu Gln Val Ser Ala Gln Leu His Gly Asp
305                 310                 315                 320

Arg Met Gln Glu Thr Lys Val Gln Ile Ser Gln Leu His Gln Glu Ile
                325                 330                 335

Gln Arg Leu Gln Ser Gln Thr Glu Asn Leu Lys Lys Gln Asn Ala Ser
            340                 345                 350

Leu Gln Ala Ala Ile Thr Asp Ala Glu Gln Arg Gly Glu Leu Ala Leu
        355                 360                 365

Lys Asp Ala Gln Ala Lys Val Asp Glu Leu Glu Ala Ala Leu Arg Met
    370                 375                 380

Ala Lys Gln Asn Leu Ala Arg Leu Leu Cys Glu Tyr Gln Glu Leu Thr
385                 390                 395                 400

Ser Thr Lys Leu Ser Leu Asp Val Glu Ile Ala Thr Tyr Arg Arg Leu
                405                 410                 415

Leu Glu Gly Glu Glu Cys Arg Met Ser Gly Glu Cys Thr Ser Gln Val
            420                 425                 430

Thr Ile Ser Ser Val Gly Gly Ser Ala Val Met Ser Gly Gly Val Gly
        435                 440                 445

Gly Gly Leu Gly Ser Thr Cys Gly Leu Gly Ser Gly Lys Gly Ser Pro
    450                 455                 460

Gly Ser Cys Cys Thr Ser Ile Val Thr Gly Gly Ser Asn Ile Ile Leu
465                 470                 475                 480

Gly Ser Gly Lys Asp Pro Val Leu Asp Ser Cys Ser Val Ser Gly Ser
                485                 490                 495

Ser Ala Gly Ser Ser Cys His Thr Ile Leu Lys Lys Thr Val Glu Ser
            500                 505                 510

Ser Leu Lys Thr Ser Ile Thr Tyr
        515                 520

<210> 137
<211> 2109
<212> DNA
<213> Homo sapiens

<400> 137
gagggaagaa cacaatggat ctggtgctaa aaagatgcct tcttcatttg gctgtgatag      60

gtgctttgct ggctgtgggg gctacaaaag tacccagaaa ccaggactgg cttggtgtct 120
caaggcaact cagaaccaaa gcctggaaca ggcagctgta tccagagtgg acagaagccc 180
agagacttga ctgctggaga ggtggtcaag tgtccctcaa ggtcagtaat gatgggccta 240
cactgattgg tgcaaatgcc tccttctcta ttgccttgaa cttccctgga agccaaaagg 300
tattgccaga tgggcaggtt atctgggtca acaataccat catcaatggg agccaggtgt 360
ggggaggaca gccagtgtat ccccaggaaa ctgacgatgc ctgcatcttc cctgatggtg 420
gaccttgccc atctggctct tggtctcaga agagaagctt tgtttatgtc tggaagacct 480
ggggccaata ctggcaagtt ctagggggcc cagtgtctgg gctgagcatt gggacaggca 540
gggcaatgct gggcacacac accatggaag tgactgtcta ccatcgccgg ggatcccgga 600
gctatgtgcc tcttgctcat tccagctcag ccttcaccat tactgaccag gtgcctttct 660
ccgtgagcgt gtcccagttg cgggccttgg atggagggaa caagcacttc ctgagaaatc 720
agcctctgac ctttgccctc cagctccatg accccagtgg ctatctggct gaagctgacc 780
tctcctacac ctgggacttt ggagacagta gtggaaccct gatctctcgg gcacttgtgg 840
tcactcatac ttacctggag cctggcccag tcactgccca ggtggtcctg caggctgcca 900
ttcctctcac ctcctgtggc tcctccccag ttccaggcac cacagatggg cacaggccaa 960
ctgcagaggc ccctaacacc acagctggcc aagtgcctac tacagaagtt gtgggtacta 1020
cacctggtca ggcgccaact gcagagccct ctggaaccac atctgtgcag gtgccaacca 1080
ctgaagtcat aagcactgca cctgtgcaga tgccaactgc agagagcaca ggtatgacac 1140
ctgagaaggt gccagtttca gaggtcatgg gtaccacact ggcagagatg tcaactccag 1200
aggctacagg tatgacacct gcagaggtat caattgtggt gctttctgga accacagctg 1260
cacaggtaac aactacagag tgggtggaga ccacagctag agagctacct atccctgagc 1320
ctgaaggtcc agatgccagc tcaatcatgt ctacggaaag tattacaggt tccctgggcc 1380
ccctgctgga tggtacagcc accttaaggc tggtgaagag acaagtcccc ctggattgtg 1440
ttctgtatcg atatggttcc ttttccgtca ccctggacat tgtccagggt attgaaagtg 1500
ccgagatcct gcaggctgtg ccgtccggtg agggggatgc atttgagctg actgtgtcct 1560
gccaaggcgg gctgcccaag gaagcctgca tggagatctc atcgccaggg tgccagcccc 1620
ctgcccagcg gctgtgccag cctgtgctac ccagcccagc ctgccagctg gttctgcacc 1680
agatactgaa gggtggctcg gggacatact gcctcaatgt gtctctggct gataccaaca 1740
gcctggcagt ggtcagcacc cagcttatca tgcctggtca agaagcaggc cttgggcagg 1800
ttccgctgat cgtgggcatc ttgctggtgt tgatggctgt ggtccttgca tctctgatat 1860
ataggcgcag acttatgaag caagacttct ccgtacccca gttgccacat agcagcagtc 1920

actggctgcg tctaccccgc atcttctgct cttgtcccat tggtgagaac agccccctcc 1980

tcagtgggca gcaggtctga gtactctcat atgatgctgt gattttcctg gagttgacag 2040

aaacacctat atttccccca gtcttccctg ggagactact attaactgaa ataaatactc 2100

agagcctga 2109

<210> 138
<211> 661
<212> PRT
<213> Homo sapiens

<400> 138
Met Asp Leu Val Leu Lys Arg Cys Leu Leu His Leu Ala Val Ile Gly
1               5                   10                  15

Ala Leu Leu Ala Val Gly Ala Thr Lys Val Pro Arg Asn Gln Asp Trp
            20                  25                  30

Leu Gly Val Ser Arg Gln Leu Arg Thr Lys Ala Trp Asn Arg Gln Leu
        35                  40                  45

Tyr Pro Glu Trp Thr Glu Ala Gln Arg Leu Asp Cys Trp Arg Gly Gly
    50                  55                  60

Gln Val Ser Leu Lys Val Ser Asn Asp Gly Pro Thr Leu Ile Gly Ala
65                  70                  75                  80

Asn Ala Ser Phe Ser Ile Ala Leu Asn Phe Pro Gly Ser Gln Lys Val
                85                  90                  95

Leu Pro Asp Gly Gln Val Ile Trp Val Asn Asn Thr Ile Ile Asn Gly
            100                 105                 110

Ser Gln Val Trp Gly Gly Gln Pro Val Tyr Pro Gln Glu Thr Asp Asp
        115                 120                 125

Ala Cys Ile Phe Pro Asp Gly Gly Pro Cys Pro Ser Gly Ser Trp Ser
    130                 135                 140

Gln Lys Arg Ser Phe Val Tyr Val Trp Lys Thr Trp Gly Gln Tyr Trp
145                 150                 155                 160

Gln Val Leu Gly Gly Pro Val Ser Gly Leu Ser Ile Gly Thr Gly Arg
                165                 170                 175

Ala Met Leu Gly Thr His Thr Met Glu Val Thr Val Tyr His Arg Arg

180                 185                 190

Gly Ser Arg Ser Tyr Val Pro Leu Ala His Ser Ser Ser Ala Phe Thr
        195                 200                 205

Ile Thr Asp Gln Val Pro Phe Ser Val Ser Val Ser Gln Leu Arg Ala
    210                 215                 220

Leu Asp Gly Gly Asn Lys His Phe Leu Arg Asn Gln Pro Leu Thr Phe
225                 230                 235                 240

Ala Leu Gln Leu His Asp Pro Ser Gly Tyr Leu Ala Glu Ala Asp Leu
                245                 250                 255

Ser Tyr Thr Trp Asp Phe Gly Asp Ser Ser Gly Thr Leu Ile Ser Arg
            260                 265                 270

Ala Leu Val Val Thr His Thr Tyr Leu Glu Pro Gly Pro Val Thr Ala
        275                 280                 285

Gln Val Val Leu Gln Ala Ala Ile Pro Leu Thr Ser Cys Gly Ser Ser
    290                 295                 300

Pro Val Pro Gly Thr Thr Asp Gly His Arg Pro Thr Ala Glu Ala Pro
305                 310                 315                 320

Asn Thr Thr Ala Gly Gln Val Pro Thr Thr Glu Val Val Gly Thr Thr
                325                 330                 335

Pro Gly Gln Ala Pro Thr Ala Glu Pro Ser Gly Thr Thr Ser Val Gln
            340                 345                 350

Val Pro Thr Thr Glu Val Ile Ser Thr Ala Pro Val Gln Met Pro Thr
        355                 360                 365

Ala Glu Ser Thr Gly Met Thr Pro Glu Lys Val Pro Val Ser Glu Val
    370                 375                 380

Met Gly Thr Thr Leu Ala Glu Met Ser Thr Pro Glu Ala Thr Gly Met
385                 390                 395                 400

Thr Pro Ala Glu Val Ser Ile Val Val Leu Ser Gly Thr Thr Ala Ala
                405                 410                 415

Gln Val Thr Thr Thr Glu Trp Val Glu Thr Thr Ala Arg Glu Leu Pro
            420                 425                 430

Ile Pro Glu Pro Glu Gly Pro Asp Ala Ser Ser Ile Met Ser Thr Glu
        435                 440                 445

Ser Ile Thr Gly Ser Leu Gly Pro Leu Leu Asp Gly Thr Ala Thr Leu
    450                 455                 460

Arg Leu Val Lys Arg Gln Val Pro Leu Asp Cys Val Leu Tyr Arg Tyr
465                 470                 475                 480

Gly Ser Phe Ser Val Thr Leu Asp Ile Val Gln Gly Ile Glu Ser Ala
                485                 490                 495

Glu Ile Leu Gln Ala Val Pro Ser Gly Glu Gly Asp Ala Phe Glu Leu
            500                 505                 510

Thr Val Ser Cys Gln Gly Gly Leu Pro Lys Glu Ala Cys Met Glu Ile
        515                 520                 525

Ser Ser Pro Gly Cys Gln Pro Pro Ala Gln Arg Leu Cys Gln Pro Val
    530                 535                 540

Leu Pro Ser Pro Ala Cys Gln Leu Val Leu His Gln Ile Leu Lys Gly
545                 550                 555                 560

Gly Ser Gly Thr Tyr Cys Leu Asn Val Ser Leu Ala Asp Thr Asn Ser
                565                 570                 575

Leu Ala Val Val Ser Thr Gln Leu Ile Met Pro Gly Gln Glu Ala Gly
            580                 585                 590

Leu Gly Gln Val Pro Leu Ile Val Gly Ile Leu Leu Val Leu Met Ala
        595                 600                 605

Val Val Leu Ala Ser Leu Ile Tyr Arg Arg Arg Leu Met Lys Gln Asp
    610                 615                 620

Phe Ser Val Pro Gln Leu Pro His Ser Ser Ser His Trp Leu Arg Leu
625                 630                 635                 640

Pro Arg Ile Phe Cys Ser Cys Pro Ile Gly Glu Asn Ser Pro Leu Leu
                645                 650                 655

Ser Gly Gln Gln Val
            660

<210> 139
<211> 2288
<212> DNA
<213> Homo sapiens

<400> 139
gcctgtcgca ggcgtgcagg gacctggact ccgcctcgtc cccggggctc gggcagccga 60

gccatggcgg ggaactgtgg ggcccgcggc gcgctgtcgg cgcacacgct gctgttcgac 120

ctgccgcccg cgctgctcgg agagctctgc gctgttctgg acagctgcga cggcgcgctg 180

ggctggcgcg gcctggcaga gagactttca agcagctggc tggatgttcg tcatattgaa 240

aagtatgtag accaaggtaa aagtggaaca agagaattac tttggtcctg ggcacagaaa 300

aacaagacca tcggtgacct tttacaggtc ctccaggaga tgggacatcg tcgagctatt 360

catttaatta caaactatgg agcagtgttg agtccttcag agaagagtta tcaggaaggt 420

ggatttccaa atatattatt caaggaaaca gccaatgtca ccgtggataa tgttcttatt 480

cctgaacata atgaaaaagg agtactgctt aaatcttcca tcagctttca aaatatcata 540

gaaggaacta gaaatttcca caaagacttc ctaattggag aaggagagat ttttgaggta 600

tacagagtgg agattcaaaa cctaacatat gctgtcaaat tatttaaaca ggagaaaaaa 660

atgcagtgta agaagcattg gaagaggttt ttatctgagc ttgaagtttt actactgttt 720

catcacccaa acatactaga gttggctgca tattttacag agactgagaa gttctgtctg 780

atttatccat acatgagaaa tggaacactt tttgacagat tgcagtgtgt aggtgacacg 840

gccccactcc cttggcacat tcgaatcggt atattaatag gaatatccaa agccattcac 900

tacctgcaca acgttcaacc atgctcggtc atctgtggca gtatatcaag tgcaaacatc 960

cttttggatg atcagtttca acccaaacta actgattttg ccatggcaca cttccggtcc 1020

cacctagaac atcagagttg taccataaat atgaccagca gcagcagtaa acatctgtgg 1080

tacatgccag aagagtacat cagacagggg aaactttcca ttaaaacaga tgtctacagc 1140

tttggaattg taataatgga agttctaaca ggatgtagag tagtgttaga tgatccaaaa 1200

catatccagc tgcgggatct ccttagagaa ttgatggaga agagaggcct ggattcatgt 1260

ctctcatttc tagataagaa agtgcctccc tgccctcgga atttctctgc caagctcttc 1320

tgtttggcag gccggtgtgc tgcaacgcgg gcaaagttaa gaccatcaat ggatgaagtt 1380

ttaaatactc ttgaaagtac tcaagccagc ttgtattttg ctgaagatcc tcccacatca 1440

ctaaagtcct tcaggtgtcc ttctcctcta ttcctggaga atgtaccaag tattccagtg 1500

gaagatgatg aaagccagaa taacaattta ctaccttctg atgaaggcct gaggatagac 1560

agaatgactc agaaaactcc ttttgaatgc agccagtctg aggttatgtt tctgagcttg 1620

gacaaaaagc cagagagcaa gagaaatgag gaagcttgca acatgcccag ttcttcttgt   1680

gaagaaagtt ggttcccaaa gtatatagtt ccatcccagg acttaaggcc ctataaggta   1740

aatatagatc cttcttcaga agctccaggg cattcttgca ggagcaggcc agtggagagc   1800

agctgttcct ccaaattttc ctgggatgaa tatgaacagt acaaaaaaga ataaattcta   1860

ccagaagata aagaaaaaag caagtattgc ataggcacct gagcataggt atgaccttgg   1920

gaagacattg gctccataag caatgccaag agaatgatca atagtgagtt tgggtgatgc   1980

agataaacaa tctggataat tccatttctt ttttcccaaa ccctcaaaca gagtgcctta   2040

aaaaattgtt ttatcaggat aattgtctca tgaccaaatc cacgctcaat tagagccatt   2100

caaaattcct taagatcatg ggttctgact tcagccaaac aaaacaatca aaacctacca   2160

aaaagggact ggattgtaat gtcctctcca tcatcctcag tgtgagtcct cagagcctcc   2220

atctgccaag aacattcagt tggattccat cgtttggttt agcttgcttg cacgggttgt   2280

aggaaatg                                                            2288

<210> 140
<211> 596
<212> PRT
<213> Homo sapiens

<400> 140
Met Ala Gly Asn Cys Gly Ala Arg Gly Ala Leu Ser Ala His Thr Leu
1               5                   10                  15

Leu Phe Asp Leu Pro Pro Ala Leu Leu Gly Glu Leu Cys Ala Val Leu
            20                  25                  30

Asp Ser Cys Asp Gly Ala Leu Gly Trp Arg Gly Leu Ala Glu Arg Leu
        35                  40                  45

Ser Ser Ser Trp Leu Asp Val Arg His Ile Glu Lys Tyr Val Asp Gln
    50                  55                  60

Gly Lys Ser Gly Thr Arg Glu Leu Leu Trp Ser Trp Ala Gln Lys Asn
65                  70                  75                  80

Lys Thr Ile Gly Asp Leu Leu Gln Val Leu Gln Glu Met Gly His Arg
                85                  90                  95

Arg Ala Ile His Leu Ile Thr Asn Tyr Gly Ala Val Leu Ser Pro Ser
            100                 105                 110

Glu Lys Ser Tyr Gln Glu Gly Gly Phe Pro Asn Ile Leu Phe Lys Glu
        115                 120                 125

Thr Ala Asn Val Thr Val Asp Asn Val Leu Ile Pro Glu His Asn Glu
    130                 135                 140

Lys Gly Val Leu Leu Lys Ser Ser Ile Ser Phe Gln Asn Ile Ile Glu
145                 150                 155                 160

Gly Thr Arg Asn Phe His Lys Asp Phe Leu Ile Gly Glu Gly Glu Ile
                165                 170                 175

Phe Glu Val Tyr Arg Val Glu Ile Gln Asn Leu Thr Tyr Ala Val Lys
            180                 185                 190

Leu Phe Lys Gln Glu Lys Lys Met Gln Cys Lys Lys His Trp Lys Arg
        195                 200                 205

Phe Leu Ser Glu Leu Glu Val Leu Leu Leu Phe His His Pro Asn Ile
    210                 215                 220

Leu Glu Leu Ala Ala Tyr Phe Thr Glu Thr Glu Lys Phe Cys Leu Ile
225                 230                 235                 240

Tyr Pro Tyr Met Arg Asn Gly Thr Leu Phe Asp Arg Leu Gln Cys Val
                245                 250                 255

Gly Asp Thr Ala Pro Leu Pro Trp His Ile Arg Ile Gly Ile Leu Ile
            260                 265                 270

Gly Ile Ser Lys Ala Ile His Tyr Leu His Asn Val Gln Pro Cys Ser
        275                 280                 285

Val Ile Cys Gly Ser Ile Ser Ser Ala Asn Ile Leu Leu Asp Asp Gln
    290                 295                 300

Phe Gln Pro Lys Leu Thr Asp Phe Ala Met Ala His Phe Arg Ser His
305                 310                 315                 320

Leu Glu His Gln Ser Cys Thr Ile Asn Met Thr Ser Ser Ser Ser Lys
                325                 330                 335

His Leu Trp Tyr Met Pro Glu Glu Tyr Ile Arg Gln Gly Lys Leu Ser
            340                 345                 350

Ile Lys Thr Asp Val Tyr Ser Phe Gly Ile Val Ile Met Glu Val Leu
        355                 360                 365

Thr Gly Cys Arg Val Val Leu Asp Asp Pro Lys His Ile Gln Leu Arg
370                 375                 380

Asp Leu Leu Arg Glu Leu Met Glu Lys Arg Gly Leu Asp Ser Cys Leu
385                 390                 395                 400

Ser Phe Leu Asp Lys Lys Val Pro Pro Cys Pro Arg Asn Phe Ser Ala
                405                 410                 415

Lys Leu Phe Cys Leu Ala Gly Arg Cys Ala Ala Thr Arg Ala Lys Leu
            420                 425                 430

Arg Pro Ser Met Asp Glu Val Leu Asn Thr Leu Glu Ser Thr Gln Ala
        435                 440                 445

Ser Leu Tyr Phe Ala Glu Asp Pro Pro Thr Ser Leu Lys Ser Phe Arg
    450                 455                 460

Cys Pro Ser Pro Leu Phe Leu Glu Asn Val Pro Ser Ile Pro Val Glu
465                 470                 475                 480

Asp Asp Glu Ser Gln Asn Asn Asn Leu Leu Pro Ser Asp Glu Gly Leu
                485                 490                 495

Arg Ile Asp Arg Met Thr Gln Lys Thr Pro Phe Glu Cys Ser Gln Ser
            500                 505                 510

Glu Val Met Phe Leu Ser Leu Asp Lys Lys Pro Glu Ser Lys Arg Asn
        515                 520                 525

Glu Glu Ala Cys Asn Met Pro Ser Ser Ser Cys Glu Glu Ser Trp Phe
    530                 535                 540

Pro Lys Tyr Ile Val Pro Ser Gln Asp Leu Arg Pro Tyr Lys Val Asn
545                 550                 555                 560

Ile Asp Pro Ser Ser Glu Ala Pro Gly His Ser Cys Arg Ser Arg Pro
                565                 570                 575

Val Glu Ser Ser Cys Ser Ser Lys Phe Ser Trp Asp Glu Tyr Glu Gln
            580                 585                 590

Tyr Lys Lys Glu
        595

<210> 141
<211> 601
<212> DNA
<213> Homo sapiens

<400> 141
ctgataaact aattgcctca cattgtcact gcaaatcgac acctattaat gggtctcacc     60

tcccaactgc ttccccctct gttcttcctg ctagcatgtg ccggcaactt tgtccacgga    120

cacaagtgcg atatcacctt acaggagatc atcaaaactt tgaacagcct cacagagcag    180

aagactctgt gcaccgagtt gaccgtaaca gacatctttg ctgcctccaa gaacacaact    240

gagaaggaaa ccttctgcag ggctgcgact gtgctccggc agttctacag ccaccatgag    300

aaggacactc gctgcctggg tgcgactgca cagcagttcc acaggcacaa gcagctgatc    360

cgattcctga aacggctcga caggaacctc tggggcctgg cgggcttgaa ttcctgtcct    420

gtgaaggaag ccaaccagag tacgttggaa aacttcttgg aaaggctaaa gacgatcatg    480

agagagaaat attcaaagtg ttcgagctga atattttaat ttatgagttt ttgatagctt    540

tattttttaa gtatttatat atttataact catcataaaa taaagtatat atagaatcta    600

a                                                                   601


<210> 142
<211> 153
<212> PRT
<213> Homo sapiens

<400> 142

<210> 143
<211> 1282
<212> DNA
<213> Homo sapiens

<400> 143
aagccaccca gcctatgcat ccgctcctca atcctctcct gttggcactg ggcctcatgg      60

cgcttttgtt gaccacggtc attgctctca cttgccttgg cggctttgcc tccccaggcc     120

ctgtgcctcc ctctacagcc ctcagggagc tcattgagga gctggtcaac atcacccaga     180

accagaaggc tccgctctgc aatggcagca tggtatggag catcaacctg acagctggca     240

tgtactgtgc agccctggaa tccctgatca acgtgtcagg ctgcagtgcc atcgagaaga     300

cccagaggat gctgagcgga ttctgcccgc acaaggtctc agctgggcag ttttccagct     360

tgcatgtccg agacaccaaa atcgaggtgg cccagtttgt aaaggacctg ctcttacatt     420

taaagaaact ttttcgcgag ggacggttca actgaaactt cgaaagcatc attatttgca     480

gagacaggac ctgactattg aagttgcaga ttcatttttc tttctgatgt caaaaatgtc     540

ttgggtaggc gggaaggagg gttagggagg ggtaaaattc cttagcttag acctcagcct     600

gtgctgcccg tcttcagcct agccgacctc agccttcccc ttgcccaggg ctcagcctgg     660

tgggcctcct ctgtccaggg ccctgagctc ggtggaccca gggatgacat gtccctacac     720

ccctcccctg ccctagagca cactgtagca ttacagtggg tgcccccctt gccagacatg     780

tggtgggaca gggacccact tcacacacag gcaactgagg cagacagcag ctcaggcaca     840

cttcttcttg gtcttattta ttattgtgtg ttatttaaat gagtgtgttt gtcaccgttg     900

gggattgggg aagactgtgg ctgctggcac ttggagccaa gggttcagag actcagggcc     960

ccagcactaa agcagtggac cccaggagtc cctggtaata agtactgtgt acagaattct    1020

gctacctcac tggggtcctg gggcctcgga gcctcatccg aggcagggtc aggagagggg    1080

cagaacagcc gctcctgtct gccagccagc agccagctct cagccaacga gtaatttatt    1140

gtttttcctc gtatttaaat attaaatatg ttagcaaaga gttaatatat agaagggtac    1200

cttgaacact gggggagggg acattgaaca agttgtttca ttgactatca aactgaagcc    1260

agaaataaag ttggtgacag at                                              1282

<210> 144
<211> 132
<212> PRT
<213> Homo sapiens

<400> 144
Met Ala Leu Leu Leu Thr Thr Val Ile Ala Leu Thr Cys Leu Gly Gly
1               5                   10                  15

Phe Ala Ser Pro Gly Pro Val Pro Pro Ser Thr Ala Leu Arg Glu Leu
            20                  25                  30

Ile Glu Glu Leu Val Asn Ile Thr Gln Asn Gln Lys Ala Pro Leu Cys
        35                  40                  45

Asn Gly Ser Met Val Trp Ser Ile Asn Leu Thr Ala Gly Met Tyr Cys
    50                  55                  60

Ala Ala Leu Glu Ser Leu Ile Asn Val Ser Gly Cys Ser Ala Ile Glu
65                  70                  75                  80

Lys Thr Gln Arg Met Leu Ser Gly Phe Cys Pro His Lys Val Ser Ala
                85                  90                  95

Gly Gln Phe Ser Ser Leu His Val Arg Asp Thr Lys Ile Glu Val Ala
            100                 105                 110

Gln Phe Val Lys Asp Leu Leu Leu His Leu Lys Lys Leu Phe Arg Glu
        115                 120                 125

Gly Arg Phe Asn
    130

<210> 145
<211> 4123
<212> DNA
<213> Homo sapiens

<400> 145
tgcggagttt tggaatagag gacaaagata agcacgccca gaatgctcat cttttcggtc      60

cgggacatgt ttgcaaagat gagcattctg ggcgtgcgga gttttggaat agaggacaaa     120

gataagcaaa ttatcacttt cttcagcccc cttacaattt tggttggacc caatggggcg     180

ggaaagacga ccatcattga atgtctaaaa tatatttgta ctggagattt ccctcctgga     240

accaaaggaa atacatttgt acacgatccc aaggttgctc aagaaacaga tgtgagagcc     300

cagattcgtc tgcaatttcg tgatgtcaat ggagaactta tagctgtgca aagatctatg 360

gtgtgtactc agaaaagcaa aaagacagaa tttaaaaccc tggaaggagt cattactaga 420

acaaagcatg gtgaaaaggt cagtctgagc tctaagtgtg cagaaattga ccgagaaatg 480

atcagttctc ttggggtttc caaggctgtg ctaaataatg tcattttctg tcatcaagaa 540

gattctaatt ggcctttaag tgagggaaag gctttgaagc aaaagtttga tgagattttt 600

tcagcaacaa gatacattaa agccctagaa acacttcggc aggtacgtca gacacaaggt 660

cagaaagtag aagaatatca aatggaacta aaatatctga agcaatataa ggaaaaagct 720

tgtgagattc gtgatcagat tacaagtaag gaagcccagt taacatcttc aaaggaaatt 780

gtcaaatcct atgagaatga acttgatcca ttgaagaatc gtctaaaaga aattgaacat 840

aatctctcta aaataatgaa acttgacaat gaaattaaag ccttggatag ccgaaagaag 900

caaatggaga aagataatag tgaactggaa gagaaaatgg aaaaggtttt tcaagggact 960

gatgagcaac taaatgactt atatcacaat caccagagaa cagtaaggga gaaagaaagg 1020

aaattggtag actgtcatcg tgaactggaa aaactaaata aagaatctag gcttctcaat 1080

caggaaaaat cagaactgct tgttgaacag ggtcgtctac agctgcaagc agatcgccat 1140

caagaacata tccgagctag agattcatta attcagtctt tggcaacaca gctagaattg 1200

gatggctttg agcgtggacc attcagtgaa agacagatta aaaattttca caaacttgtg 1260

agagagagac aagaagggga agcaaaaact gccaaccaac tgatgaatga ctttgcagaa 1320

aaagagactc tgaaacaaaa acagatagat gagataagag ataagaaaac tggactggga 1380

agaataattg agttaaaatc agaaatccta agtaagaagc agaatgagct gaaaaatgtg 1440

aagtatgaat tacagcagtt ggaaggatct tcagacagga ttcttgaact ggaccaggag 1500

ctcataaaag ctgaacgtga gttaagcaag gctgagaaaa acagcaatgt agaaacctta 1560

aaaatggaag taataagtct ccaaaatgaa aaagcagact tagacaggac cctgcgtaaa 1620

cttgaccagg agatggagca gttaaaccat catacaacaa cacgtaccca aatggagatg 1680

ctgaccaaag acaaagctga caaagatgaa caaatcagaa aaataaaatc taggcacagt 1740

gatgaattaa cctcactgtt gggatatttt cccaacaaaa aacagcttga agactggcta 1800

catagtaaat caaaagaaat taatcagacc agggacagac ttgccaaatt gaacaaggaa 1860

ctagcttcat ctgagcagaa taaaaatcat ataaataatg aactagaaag aaaggaagag 1920

cagttgtcca gttacgaaga caagctgttt gatgtttgtg gtagccagga ttttgaaagt 1980

gatttagaca ggcttaaaga ggaaattgaa aaatcatcaa aacagcgagc catgctggct 2040

ggagccacag cagtttactc ccagttcatt actcagctaa cagacgaaaa ccagtcatgt 2100

tgccccgttt gtcagagagt ttttcagaca gaggctgagt tacaagaagc catcagtgat 2160

ttgcagtcta aactgcgact tgctccagat aaactcaagt caacagaatc agagctaaaa 2220

aaaaaggaaa agcggcgtga tgaaatgctg ggacttgcgc ccatgaggca aagcataatt 2280

gatttgaagg agaaggaaat accagaatta agaaacaaac tgcagaatgt caatagagac 2340

atacagcgcc taaagaacga catagaagaa caagaaacac tcttgggtac aataatgcct 2400

gaagaagaaa gtgccaaagt atgcctgaca gatgttacaa ttatggagag gttccagatg 2460

gaacttaaag atgttgaaag aaaaattgca caacaagcag ctaagctaca aggaatagac 2520

ttagatcgaa ctgtccaaca agtcaaccag gagaaacaag agaaacagca caagttagac 2580

acagtttcta gtaagattga attgaatcgt aagcttatac aggaccagca ggaacagatt 2640

caacatctaa aaagtacaac aaatgagcta aaatctgaga aacttcagat atccactaat 2700

ttgcaacgtc gtcagcaact ggaggagcag actgtggaat tatccactga agttcagtct 2760

ttgtacagag agataaagga tgctaaagag caggtaagcc ctttggaaac aacattggaa 2820

aagttccagc aagaaaaaga agaattaatc aacaaaaaaa atacaagcaa caaaatagca 2880

caggataaac tgaatgatat taaagagaag gttaaaaata ttcatggcta tatgaaagac 2940

attgagaatc atattcaaga tgggaaagac gactatatga agcaaaaaga aactgaactt 3000

aataaagtaa tagctcaact aagtgaatgc gagaaacaca aagaaaagat aaatgaagat 3060

atgagactca tgagacaaga tattgataca cagaagatac aagaaaggtg gctacaagat 3120

aaccttactt taagaaaaag aaatgaggaa ctaaaagaag ttgaagaaga aggaaaacaa 3180

catttgaagg aaatgggtca aatgcaggtt ttgcaaatga aaagtgaaca tcagaagttg 3240

gaagagaaca tagacaatat aaaaagaaat cataatttgg cattagggcg acagaaaggt 3300

tatgaagaag aaattattca ttttaagaaa gaacttcgag aaccacaatt tcgggatgct 3360

gaggaaaagt atagagaaat gatgattgtt atgaggacaa cagaacttgt gaacaaggat 3420

ctggatattt attataagac tcttgaccaa gcaataatga aatttcacag tatgaagatg 3480

gaagaaatca ataaaattat acgtgacctg tggcgaagta cctatcgtgg acaagatatt 3540

gaatacatag aaatacggtc tgatgccgat gaaaatgtat cagcttctga taaaaggcgg 3600

aattataact accgagtggt gatgctgaag ggagacacag ccttggatat gcgaggacga 3660

tgcagtgctg gacaaaaggt attagcctca ctcatcattc gcctggccct ggctgaaacg 3720

ttctgcctca actgtggcat cattgccttg gatgagccaa caacaaatct tgaccgagaa 3780

aacattgaat ctcttgcaca tgctctggtt gagataataa aaagtcgctc acagcagcgt 3840

aacttccagc ttctggtaat cactcatgat gaagattttg tggagctttt aggacgttct 3900

gaatatgtgg agaaattcta caggattaaa aagaacatcg atcagtgctc agagattgtg 3960

aaatgcagtg ttagctccct gggattcaat gttcattaaa aatatccaag atttaaatgc 4020

catagaaatg taggtcctca gaaagtgtat aataagaaac ttatttctca tatcaactta 4080

gtcaataaga aaatatattc tttcaaagga aaaaaaaaaa aaa 4123

<210> 146
<211> 1318
<212> PRT
<213> Homo sapiens

<400> 146
Met Leu Ile Phe Ser Val Arg Asp Met Phe Ala Lys Met Ser Ile Leu
1               5                   10                  15

Gly Val Arg Ser Phe Gly Ile Glu Asp Lys Asp Lys Gln Ile Ile Thr
            20                  25                  30

Phe Phe Ser Pro Leu Thr Ile Leu Val Gly Pro Asn Gly Ala Gly Lys
        35                  40                  45

Thr Thr Ile Ile Glu Cys Leu Lys Tyr Ile Cys Thr Gly Asp Phe Pro
    50                  55                  60

Pro Gly Thr Lys Gly Asn Thr Phe Val His Asp Pro Lys Val Ala Gln
65                  70                  75                  80

Glu Thr Asp Val Arg Ala Gln Ile Arg Leu Gln Phe Arg Asp Val Asn
                85                  90                  95

Gly Glu Leu Ile Ala Val Gln Arg Ser Met Val Cys Thr Gln Lys Ser
            100                 105                 110

Lys Lys Thr Glu Phe Lys Thr Leu Glu Gly Val Ile Thr Arg Thr Lys
        115                 120                 125

His Gly Glu Lys Val Ser Leu Ser Ser Lys Cys Ala Glu Ile Asp Arg
    130                 135                 140

Glu Met Ile Ser Ser Leu Gly Val Ser Lys Ala Val Leu Asn Asn Val
145                 150                 155                 160

Ile Phe Cys His Gln Glu Asp Ser Asn Trp Pro Leu Ser Glu Gly Lys
                165                 170                 175

Ala Leu Lys Gln Lys Phe Asp Glu Ile Phe Ser Ala Thr Arg Tyr Ile
            180                 185                 190

Lys Ala Leu Glu Thr Leu Arg Gln Val Arg Gln Thr Gln Gly Gln Lys
        195                 200                 205

Val Glu Glu Tyr Gln Met Glu Leu Lys Tyr Leu Lys Gln Tyr Lys Glu
    210                 215                 220

Lys Ala Cys Glu Ile Arg Asp Gln Ile Thr Ser Lys Glu Ala Gln Leu
225                 230                 235                 240

Thr Ser Ser Lys Glu Ile Val Lys Ser Tyr Glu Asn Glu Leu Asp Pro
                245                 250                 255

Leu Lys Asn Arg Leu Lys Glu Ile Glu His Asn Leu Ser Lys Ile Met
            260                 265                 270

Lys Leu Asp Asn Glu Ile Lys Ala Leu Asp Ser Arg Lys Lys Gln Met
        275                 280                 285

Glu Lys Asp Asn Ser Glu Leu Glu Glu Lys Met Glu Lys Val Phe Gln
    290                 295                 300

Gly Thr Asp Glu Gln Leu Asn Asp Leu Tyr His Asn His Gln Arg Thr
305                 310                 315                 320

Val Arg Glu Lys Glu Arg Lys Leu Val Asp Cys His Arg Glu Leu Glu
                325                 330                 335

Lys Leu Asn Lys Glu Ser Arg Leu Leu Asn Gln Glu Lys Ser Glu Leu
            340                 345                 350

Leu Val Glu Gln Gly Arg Leu Gln Leu Gln Ala Asp Arg His Gln Glu
        355                 360                 365

His Ile Arg Ala Arg Asp Ser Leu Ile Gln Ser Leu Ala Thr Gln Leu
    370                 375                 380

Glu Leu Asp Gly Phe Glu Arg Gly Pro Phe Ser Glu Arg Gln Ile Lys
385                 390                 395                 400

Asn Phe His Lys Leu Val Arg Glu Arg Gln Glu Gly Glu Ala Lys Thr
                405                 410                 415

Ala Asn Gln Leu Met Asn Asp Phe Ala Glu Lys Glu Thr Leu Lys Gln
            420                 425                 430

Lys Gln Ile Asp Glu Ile Arg Asp Lys Lys Thr Gly Leu Gly Arg Ile
        435                 440                 445

Ile Glu Leu Lys Ser Glu Ile Leu Ser Lys Lys Gln Asn Glu Leu Lys
    450                 455                 460

Asn Val Lys Tyr Glu Leu Gln Gln Leu Glu Gly Ser Ser Asp Arg Ile
465                 470                 475                 480

Leu Glu Leu Asp Gln Glu Leu Ile Lys Ala Glu Arg Glu Leu Ser Lys
                485                 490                 495

Ala Glu Lys Asn Ser Asn Val Glu Thr Leu Lys Met Glu Val Ile Ser
            500                 505                 510

Leu Gln Asn Glu Lys Ala Asp Leu Asp Arg Thr Leu Arg Lys Leu Asp
        515                 520                 525

Gln Glu Met Glu Gln Leu Asn His His Thr Thr Thr Arg Thr Gln Met
    530                 535                 540

Glu Met Leu Thr Lys Asp Lys Ala Asp Lys Asp Glu Gln Ile Arg Lys
545                 550                 555                 560

Ile Lys Ser Arg His Ser Asp Glu Leu Thr Ser Leu Leu Gly Tyr Phe
                565                 570                 575

Pro Asn Lys Lys Gln Leu Glu Asp Trp Leu His Ser Lys Ser Lys Glu
            580                 585                 590

Ile Asn Gln Thr Arg Asp Arg Leu Ala Lys Leu Asn Lys Glu Leu Ala
        595                 600                 605

Ser Ser Glu Gln Asn Lys Asn His Ile Asn Asn Glu Leu Glu Arg Lys
    610                 615                 620

Glu Glu Gln Leu Ser Ser Tyr Glu Asp Lys Leu Phe Asp Val Cys Gly
625                 630                 635                 640

Ser Gln Asp Phe Glu Ser Asp Leu Asp Arg Leu Lys Glu Glu Ile Glu
                645                 650                 655

Lys Ser Ser Lys Gln Arg Ala Met Leu Ala Gly Ala Thr Ala Val Tyr
            660                 665                 670

Ser Gln Phe Ile Thr Gln Leu Thr Asp Glu Asn Gln Ser Cys Cys Pro

        675                 680                 685

Val Cys Gln Arg Val Phe Gln Thr Glu Ala Glu Leu Gln Glu Ala Ile
    690                 695                 700

Ser Asp Leu Gln Ser Lys Leu Arg Leu Ala Pro Asp Lys Leu Lys Ser
705                 710                 715                 720

Thr Glu Ser Glu Leu Lys Lys Lys Glu Lys Arg Arg Asp Glu Met Leu
                725                 730                 735

Gly Leu Ala Pro Met Arg Gln Ser Ile Ile Asp Leu Lys Glu Lys Glu
            740                 745                 750

Ile Pro Glu Leu Arg Asn Lys Leu Gln Asn Val Asn Arg Asp Ile Gln
        755                 760                 765

Arg Leu Lys Asn Asp Ile Glu Glu Gln Glu Thr Leu Leu Gly Thr Ile
    770                 775                 780

Met Pro Glu Glu Glu Ser Ala Lys Val Cys Leu Thr Asp Val Thr Ile
785                 790                 795                 800

Met Glu Arg Phe Gln Met Glu Leu Lys Asp Val Glu Arg Lys Ile Ala
                805                 810                 815

Gln Gln Ala Ala Lys Leu Gln Gly Ile Asp Leu Asp Arg Thr Val Gln
            820                 825                 830

Gln Val Asn Gln Glu Lys Gln Glu Lys Gln His Lys Leu Asp Thr Val
        835                 840                 845

Ser Ser Lys Ile Glu Leu Asn Arg Lys Leu Ile Gln Asp Gln Gln Glu
    850                 855                 860

Gln Ile Gln His Leu Lys Ser Thr Thr Asn Glu Leu Lys Ser Glu Lys
865                 870                 875                 880

Leu Gln Ile Ser Thr Asn Leu Gln Arg Arg Gln Gln Leu Glu Glu Gln
                885                 890                 895

Thr Val Glu Leu Ser Thr Glu Val Gln Ser Leu Tyr Arg Glu Ile Lys
            900                 905                 910

Asp Ala Lys Glu Gln Val Ser Pro Leu Glu Thr Thr Leu Glu Lys Phe
        915                 920                 925

Gln Gln Glu Lys Glu Glu Leu Ile Asn Lys Lys Asn Thr Ser Asn Lys
    930                 935                 940

Ile Ala Gln Asp Lys Leu Asn Asp Ile Lys Glu Lys Val Lys Asn Ile
945                 950                 955                 960

His Gly Tyr Met Lys Asp Ile Glu Asn His Ile Gln Asp Gly Lys Asp
                965                 970                 975

Asp Tyr Met Lys Gln Lys Glu Thr Glu Leu Asn Lys Val Ile Ala Gln
            980                 985                 990

Leu Ser Glu Cys Glu Lys His Lys  Glu Lys Ile Asn Glu  Asp Met Arg
        995                 1000                1005

Leu Met  Arg Gln Asp Ile Asp  Thr Gln Lys Ile Gln  Glu Arg Trp
    1010                1015                1020

Leu Gln  Asp Asn Leu Thr Leu  Arg Lys Arg Asn Glu  Glu Leu Lys
    1025                1030                1035

Glu Val  Glu Glu Glu Gly Lys  Gln His Leu Lys Glu  Met Gly Gln
    1040                1045                1050

Met Gln  Val Leu Gln Met Lys  Ser Glu His Gln Lys  Leu Glu Glu
    1055                1060                1065

Asn Ile  Asp Asn Ile Lys Arg  Asn His Asn Leu Ala  Leu Gly Arg
    1070                1075                1080

Gln Lys  Gly Tyr Glu Glu Glu  Ile Ile His Phe Lys  Lys Glu Leu
    1085                1090                1095

Arg Glu  Pro Gln Phe Arg Asp  Ala Glu Glu Lys Tyr  Arg Glu Met
    1100                1105                1110

Met Ile  Val Met Arg Thr Thr  Glu Leu Val Asn Lys  Asp Leu Asp
    1115                1120                1125

Ile Tyr  Tyr Lys Thr Leu Asp  Gln Ala Ile Met Lys  Phe His Ser
    1130                1135                1140

Met Lys  Met Glu Glu Ile Asn  Lys Ile Ile Arg Asp  Leu Trp Arg
    1145                1150                1155

Ser Thr Tyr Arg Gly Gln Asp Ile Glu Tyr Ile Glu Ile Arg Ser
    1160                1165                1170

Asp Ala Asp Glu Asn Val Ser Ala Ser Asp Lys Arg Arg Asn Tyr
    1175                1180                1185

Asn Tyr Arg Val Val Met Leu Lys Gly Asp Thr Ala Leu Asp Met
    1190                1195                1200

Arg Gly Arg Cys Ser Ala Gly Gln Lys Val Leu Ala Ser Leu Ile
    1205                1210                1215

Ile Arg Leu Ala Leu Ala Glu Thr Phe Cys Leu Asn Cys Gly Ile
    1220                1225                1230

Ile Ala Leu Asp Glu Pro Thr Thr Asn Leu Asp Arg Glu Asn Ile
    1235                1240                1245

Glu Ser Leu Ala His Ala Leu Val Glu Ile Ile Lys Ser Arg Ser
    1250                1255                1260

Gln Gln Arg Asn Phe Gln Leu Leu Val Ile Thr His Asp Glu Asp
    1265                1270                1275

Phe Val Glu Leu Leu Gly Arg Ser Glu Tyr Val Glu Lys Phe Tyr
    1280                1285                1290

Arg Ile Lys Lys Asn Ile Asp Gln Cys Ser Glu Ile Val Lys Cys
    1295                1300                1305

Ser Val Ser Ser Leu Gly Phe Asn Val His
    1310                1315

<210> 147
<211> 816
<212> DNA
<213> Homo sapiens

<400> 147
atgcactttc tttgccaaag gcaaacgcag aacgtttcag agccatgagg atgcttctgc      60

atttgagttt gctagctctt ggagctgcct acgtgtatgc catccccaca gaaattccca     120

caagtgcatt ggtgaaagag accttggcac tgctttctac tcatcgaact ctgctgatag     180

ccaatgagac tctgaggatt cctgttcctg tacataaaaa tcaccaactg tgcactgaag     240

aaatctttca gggaataggc acactggaga gtcaaactgt gcaagggggt actgtggaaa     300

gactattcaa aaacttgtcc ttaataaaga aatacattga cggccaaaaa aaaaagtgtg    360

gagaagaaag acggagagta aaccaattcc tagactacct gcaagagttt cttggtgtaa    420

tgaacaccga gtggataata gaaagttgag actaaactgg tttgttgcag ccaaagattt    480

tggaggagaa ggacatttta ctgcagtgag aatgagggcc aagaaagagt caggccttaa    540

ttttcaatat aatttaactt cagagggaaa gtaaatattt caggcatact gacactttgc    600

cagaaagcat aaaattctta aaatatattt cagatatcag aatcattgaa gtattttcct    660

ccaggcaaaa ttgatatact tttttcttat ttaacttaac attctgtaaa atgtctgtta    720

acttaatagt atttatgaaa tggttaagaa tttggtaaat tagtatttat ttaatgttat    780

gttgtgttct aataaaacaa aaatagacaa ctgttc                              816

<210> 148
<211> 134
<212> PRT
<213> Homo sapiens

<400> 148
Met Arg Met Leu Leu His Leu Ser Leu Leu Ala Leu Gly Ala Ala Tyr
1               5                   10                  15

Val Tyr Ala Ile Pro Thr Glu Ile Pro Thr Ser Ala Leu Val Lys Glu
            20                  25                  30

Thr Leu Ala Leu Leu Ser Thr His Arg Thr Leu Leu Ile Ala Asn Glu
        35                  40                  45

Thr Leu Arg Ile Pro Val Pro Val His Lys Asn His Gln Leu Cys Thr
    50                  55                  60

Glu Glu Ile Phe Gln Gly Ile Gly Thr Leu Glu Ser Gln Thr Val Gln
65                  70                  75                  80

Gly Gly Thr Val Glu Arg Leu Phe Lys Asn Leu Ser Leu Ile Lys Lys
                85                  90                  95

Tyr Ile Asp Gly Gln Lys Lys Lys Cys Gly Glu Glu Arg Arg Arg Val
            100                 105                 110

Asn Gln Phe Leu Asp Tyr Leu Gln Glu Phe Leu Gly Val Met Asn Thr
        115                 120                 125

Glu Trp Ile Ile Glu Ser
    130

<210> 149
<211> 2035
<212> DNA
<213> Homo sapiens

<400> 149
cgagccccgc cgaaccgagg ccacccggag ccgtgcccag tccacgccgg ccgtgcccgg 60

cggccttaag aaccaggcaa cctctgcctt cttccctctt ccactcggag tcgcgctccg 120

cgcgccctca ctgcagcccc tgcgtcgccg ggaccctcgc gcgcgaccag ccgaatcgct 180

cctgcagcag agccaacatg cccatcactc ggatgcgcat gagaccctgg ctagagatgc 240

agattaattc caaccaaatc ccggggctca tctggattaa taaagaggag atgatcttcc 300

agatcccatg gaagcatgct gccaagcatg gctgggacat caacaaggat gcctgtttgt 360

tccggagctg ggccattcac acaggccgat acaaagcagg ggaaaaggag ccagatccca 420

agacgtggaa ggccaacttt cgctgtgcca tgaactccct gccagatatc gaggaggtga 480

aagaccagag caggaacaag ggcagctcag ctgtgcgagt gtaccggatg cttccacctc 540

tcaccaagaa ccagagaaaa gaaagaaagt cgaagtccag ccgagatgct aagagcaagg 600

ccaagaggaa gtcatgtggg gattccagcc ctgatacctt ctctgatgga ctcagcagct 660

ccactctgcc tgatgaccac agcagctaca cagttccagg ctacatgcag gacttggagg 720

tggagcaggc cctgactcca gcactgtcgc catgtgctgt cagcagcact ctccccgact 780

ggcacatccc agtggaagtt gtgccggaca gcaccagtga tctgtacaac ttccaggtgt 840

cacccatgcc ctccacctct gaagctacaa cagatgagga tgaggaaggg aaattacctg 900

aggacatcat gaagctcttg gagcagtcgg agtggcagcc aacaaacgtg gatgggaagg 960

ggtacctact caatgaacct ggagtccagc ccacctctgt ctatggagac tttagctgta 1020

aggaggagcc agaaattgac agcccagggg gggatattgg gctgagtcta cagcgtgtct 1080

tcacagatct gaagaacatg gatgccacct ggctggacag cctgctgacc ccagtccggt 1140

tgccctccat ccaggccatt ccctgtgcac cgtagcaggg cccctgggcc cctcttattc 1200

ctctaggcaa gcaggacctg gcatcatggt ggatatggtg cagagaagct ggacttctgt 1260

gggcccctca acagccaagt gtgaccccac tgccaagtgg ggatgggcct ccctccttgg 1320

gtcattgacc tctcagggcc tggcaggcca gtgtctgggt ttttcttgtg gtgtaaagct 1380

ggccctgcct cctgggaaga tgaggttctg agaccagtgt atcaggtcag ggacttggac 1440

aggagtcagt gtctggcttt ttcctctgag cccagctgcc tggagagggt ctcgctgtca 1500

ctggctggct cctaggggaa cagaccagtg accccagaaa agcataacac caatcccagg 1560

gctggctctg cactaagcga aaattgcact aaatgaatct cgttccaaag aactacccct 1620

tttcagctga gccctgggga ctgttccaaa gccagtgaat gtgaaggaaa ctcccctcct 1680

tcggggcaat gctccctcag cctcagagga gctctaccct gctccctgct ttggctgagg 1740

ggcttgggaa aaaaacttgg cactttttcg tgtggatctt gccacatttc tgatcagagg 1800

tgtacactaa catttccccc gagctcttgg cctttgcatt tatttataca gtgccttgct 1860

cggggcccac caccccctca agccccagca gccctcaaca ggcccaggga gggaagtgtg 1920

agcgccttgg tatgacttaa aattggaaat gtcatctaac cattaagtca tgtgtgaaca 1980

cataaggacg tgtgtaaata tgtacatttg tctttttata aaaagtaaaa ttgtt 2035

<210> 150
<211> 325
<212> PRT
<213> Homo sapiens

<400> 150
Met Pro Ile Thr Arg Met Arg Met Arg Pro Trp Leu Glu Met Gln Ile
1               5                   10                  15

Asn Ser Asn Gln Ile Pro Gly Leu Ile Trp Ile Asn Lys Glu Glu Met
            20                  25                  30

Ile Phe Gln Ile Pro Trp Lys His Ala Ala Lys His Gly Trp Asp Ile
        35                  40                  45

Asn Lys Asp Ala Cys Leu Phe Arg Ser Trp Ala Ile His Thr Gly Arg
    50                  55                  60

Tyr Lys Ala Gly Glu Lys Glu Pro Asp Pro Lys Thr Trp Lys Ala Asn
65                  70                  75                  80

Phe Arg Cys Ala Met Asn Ser Leu Pro Asp Ile Glu Glu Val Lys Asp
                85                  90                  95

Gln Ser Arg Asn Lys Gly Ser Ser Ala Val Arg Val Tyr Arg Met Leu
            100                 105                 110

Pro Pro Leu Thr Lys Asn Gln Arg Lys Glu Arg Lys Ser Lys Ser Ser
        115                 120                 125

Arg Asp Ala Lys Ser Lys Ala Lys Arg Lys Ser Cys Gly Asp Ser Ser
    130                 135                 140

Pro Asp Thr Phe Ser Asp Gly Leu Ser Ser Ser Thr Leu Pro Asp Asp
145                 150                 155                 160

His Ser Ser Tyr Thr Val Pro Gly Tyr Met Gln Asp Leu Glu Val Glu

165                 170                 175

Gln Ala Leu Thr Pro Ala Leu Ser Pro Cys Ala Val Ser Ser Thr Leu
            180                 185                 190

Pro Asp Trp His Ile Pro Val Glu Val Val Pro Asp Ser Thr Ser Asp
        195                 200                 205

Leu Tyr Asn Phe Gln Val Ser Pro Met Pro Ser Thr Ser Glu Ala Thr
    210                 215                 220

Thr Asp Glu Asp Glu Glu Gly Lys Leu Pro Glu Asp Ile Met Lys Leu
225                 230                 235                 240

Leu Glu Gln Ser Glu Trp Gln Pro Thr Asn Val Asp Gly Lys Gly Tyr
                245                 250                 255

Leu Leu Asn Glu Pro Gly Val Gln Pro Thr Ser Val Tyr Gly Asp Phe
            260                 265                 270

Ser Cys Lys Glu Glu Pro Glu Ile Asp Ser Pro Gly Gly Asp Ile Gly
        275                 280                 285

Leu Ser Leu Gln Arg Val Phe Thr Asp Leu Lys Asn Met Asp Ala Thr
    290                 295                 300

Trp Leu Asp Ser Leu Leu Thr Pro Val Arg Leu Pro Ser Ile Gln Ala
305                 310                 315                 320

Ile Pro Cys Ala Pro
                325

<210> 151
<211> 2256
<212> DNA
<213> Homo sapiens

<400> 151
tcagagtccg gctcaggctc cggctgcggc tccagcccgc gatgccccat tccgtgaccc      60

tgcgcgggcc ttcgccctgg ggcttccgcc tggtgggcgg ccgggacttc agcgcgcccc     120

tcaccatctc acgggtccat gctggcagca aggctgcatt ggctgccctg tgcccaggag     180

acctgatcca ggccatcaat ggtgagagca cagagctcat gacacacctg gaggcacaga     240

accgcatcaa gggctgccac gatcacctca cactgtctgt gagcaggcct gagggcagga     300

gctggcccag tgcccctgat gacagcaagg ctcaggcaca caggatccac atcgatcctg     360

agatccagga cggcagccca acaaccagca ggcggccctc aggcaccggg actgggccag 420
aagatggcag accaagcctg ggatctccat atggacaacc ccctcgcttt ccagtccctc 480
acaatggcag cagcgaggcc accctgccag cccagatgag caccctgcat gtgtctccac 540
cccccagcgc tgacccagcc agaggcctcc cgcggagccg ggactgcaga gtcgacctgg 600
gctccgaggt gtacaggatg ctgcgggagc cagccgagcc cgtggccgcg gagcccaagc 660
agtcaggctc cttccgctac ttgcagggca tgctagaggc cggcgagggc ggggattggc 720
ccgggcctgg cggcccccgg aacctcaagc ccacggccag caagctgggc gctccgctga 780
gcggcctgca ggggctgccc gagtgcacgc gctgcggcca cggcatcgtg ggcaccatcg 840
tcaaggcacg ggacaagctc taccatcccg agtgcttcat gtgcagtgac tgcggcctga 900
acctcaagca gcgtggttac ttctttctgg acgagcggct ctactgtgag agccacgcca 960
aggcgcgcgt gaagccgccc gagggctacg acgtggtggc ggtgtacccc aatgccaagg 1020
tggaactcgt ctgagctggg accctgctcc cacgcctgct tcttaaggtc cctgctcggc 1080
cggtgtaaat atgtttcacc ctgtccctct aataaagctc ctctgctcca ccttgaacct 1140
gtcacctggc ctgccaccct cctgcgcagg ccatgcatgg ctcccgagta cagtagtatc 1200
tgcttaggtg ccaggcatgt cctaggctct gggtgcagta gtgagcagga cgggtaccat 1260
gctgccctga aggggccaca gcctgggtgg aaggcagacc tgaatcacaa cgggccagct 1320
ctagtaatac gaaggtgagg tctgggatgc tgcgtgcggc gcgacgacag gaggtatgac 1380
ctgggtgggg tcagggaaag tcttagctga gaactaagag atgagggagg cacaaactct 1440
gcacggggga actgtgtgtg caaaggtgag ctgggggcga gaaaggcctc tgtggccgtc 1500
atcggcacta gcgggttggg ggtggtggtg gggtgctggc agcctcagga gcgatcgctc 1560
ccctggagag ggtgattgag tgtggagatc accggggccg ccgcgggccg tctgcacttc 1620
tgttccaggt ttttctggca ttttctgttc caggtttctt ccacccacga gcactttatt 1680
ctcctccgag accccctttt cttccccttc ctctctccca ccagggggcg cgcttaaatt 1740
tccagacaga gaccagaagg aaggtttaga aagaaaagaa cacttgccca gggcagcttt 1800
gccctccaag aggctccctc ctgtctctaa gtcaattcca cccctcctcg aagtcccggc 1860
ttccacccct ctcctcagag tcccggcgtt tacctcgtgg tgtgtttgcc ttgggccttt 1920
catgccccgg ctgcacacat cgtctgtgaa ttgtggctgt tcactcccga ggatgtgtcc 1980
tagactccgg gtcgcgtgga tctaccctct agtttacttg ctcgggagaa gaaactgact 2040
cgttttattt agtgcctatt tagcgagccc agagtaacgt acatttgtgc tgttttcaat 2100
tttgtgctat cgcaaatcac aaaaaaactg ttatcaattc acattcatca tcagcacgag 2160
acccatttcc ttgtgctcct gccagctcag gatattatgt ttcttttttc atttttgcca 2220

gtctgttaag taaaatgtca tatgcgtttg ttttaa                                    2256

<210> 152
<211> 330
<212> PRT
<213> Homo sapiens

<400> 152
Met Pro His Ser Val Thr Leu Arg Gly Pro Ser Pro Trp Gly Phe Arg
1               5                   10                  15

Leu Val Gly Gly Arg Asp Phe Ser Ala Pro Leu Thr Ile Ser Arg Val
            20                  25                  30

His Ala Gly Ser Lys Ala Ala Leu Ala Ala Leu Cys Pro Gly Asp Leu
        35                  40                  45

Ile Gln Ala Ile Asn Gly Glu Ser Thr Glu Leu Met Thr His Leu Glu
    50                  55                  60

Ala Gln Asn Arg Ile Lys Gly Cys His Asp His Leu Thr Leu Ser Val
65                  70                  75                  80

Ser Arg Pro Glu Gly Arg Ser Trp Pro Ser Ala Pro Asp Asp Ser Lys
                85                  90                  95

Ala Gln Ala His Arg Ile His Ile Asp Pro Glu Ile Gln Asp Gly Ser
            100                 105                 110

Pro Thr Thr Ser Arg Arg Pro Ser Gly Thr Gly Thr Gly Pro Glu Asp
        115                 120                 125

Gly Arg Pro Ser Leu Gly Ser Pro Tyr Gly Gln Pro Pro Arg Phe Pro
    130                 135                 140

Val Pro His Asn Gly Ser Ser Glu Ala Thr Leu Pro Ala Gln Met Ser
145                 150                 155                 160

Thr Leu His Val Ser Pro Pro Pro Ser Ala Asp Pro Ala Arg Gly Leu
                165                 170                 175

Pro Arg Ser Arg Asp Cys Arg Val Asp Leu Gly Ser Glu Val Tyr Arg
            180                 185                 190

Met Leu Arg Glu Pro Ala Glu Pro Val Ala Ala Glu Pro Lys Gln Ser
        195                 200                 205

Gly Ser Phe Arg Tyr Leu Gln Gly Met Leu Glu Ala Gly Glu Gly Gly
    210                 215                 220

Asp Trp Pro Gly Pro Gly Gly Pro Arg Asn Leu Lys Pro Thr Ala Ser
225                 230                 235                 240

Lys Leu Gly Ala Pro Leu Ser Gly Leu Gln Gly Leu Pro Glu Cys Thr
                245                 250                 255

Arg Cys Gly His Gly Ile Val Gly Thr Ile Val Lys Ala Arg Asp Lys
            260                 265                 270

Leu Tyr His Pro Glu Cys Phe Met Cys Ser Asp Cys Gly Leu Asn Leu
        275                 280                 285

Lys Gln Arg Gly Tyr Phe Phe Leu Asp Glu Arg Leu Tyr Cys Glu Ser
    290                 295                 300

His Ala Lys Ala Arg Val Lys Pro Pro Glu Gly Tyr Asp Val Val Ala
305                 310                 315                 320

Val Tyr Pro Asn Ala Lys Val Glu Leu Val
                325                 330

<210> 153
<211> 778
<212> DNA
<213> Homo sapiens

<400> 153
aggaaaggct aaagttctct ggaggatgtg gctgcagagc ctgctgctct tgggcactgt      60

ggcctgcagc atctctgcac ccgcccgctc gcccagcccc agcacgcagc cctgggagca     120

tgtgaatgcc atccaggagg cccggcgtct cctgaacctg agtagagaca ctgctgctga     180

gatgaatgaa acagtagaag tcatctcaga aatgtttgac ctccaggagc cgacctgcct     240

acagacccgc ctggagctgt acaagcaggg cctgcggggc agcctcacca agctcaaggg     300

ccccttgacc atgatggcca gccactacaa gcagcactgc cctccaaccc cggaaacttc     360

ctgtgcaatc cagactatca cctttgaaag tttcaaagag aacctgaagg actttctgct     420

tgtcatcccc tttgactgct gggagccagt ccaggagtga gaccggccag atgaggctgg     480

ccaagccggg gagctgctct ctcatgaaac aagagctaga aactcaggat ggtcatcttg     540

gagggaccaa ggggtgggcc acagccatgg tgggagtggc ctggacctgc cctgggccac     600

actgaccctg atacaggcat ggcagaagaa tgggaatatt ttatactgac agaaatcagt     660

aatatttata tatttatatt tttaaaatat ttatttattt atttatttaa gttcatattc 720

catatttatt caagatgttt taccgtaata attattatta aaaatatgct tctactta 778

<210> 154
<211> 144
<212> PRT
<213> Homo sapiens

<400> 154
Met Trp Leu Gln Ser Leu Leu Leu Leu Gly Thr Val Ala Cys Ser Ile
1               5                   10                  15

Ser Ala Pro Ala Arg Ser Pro Ser Pro Ser Thr Gln Pro Trp Glu His
            20                  25                  30

Val Asn Ala Ile Gln Glu Ala Arg Arg Leu Leu Asn Leu Ser Arg Asp
        35                  40                  45

Thr Ala Ala Glu Met Asn Glu Thr Val Glu Val Ile Ser Glu Met Phe
    50                  55                  60

Asp Leu Gln Glu Pro Thr Cys Leu Gln Thr Arg Leu Glu Leu Tyr Lys
65                  70                  75                  80

Gln Gly Leu Arg Gly Ser Leu Thr Lys Leu Lys Gly Pro Leu Thr Met
                85                  90                  95

Met Ala Ser His Tyr Lys Gln His Cys Pro Pro Thr Pro Glu Thr Ser
            100                 105                 110

Cys Ala Ile Gln Thr Ile Thr Phe Glu Ser Phe Lys Glu Asn Leu Lys
        115                 120                 125

Asp Phe Leu Leu Val Ile Pro Phe Asp Cys Trp Glu Pro Val Gln Glu
    130                 135                 140

<210> 155
<211> 985
<212> DNA
<213> Homo sapiens

<400> 155
cccggggttg tgggcacctt gctgctgcac atataaggcg ggaggttgtt gccaactctt 60

cagagcccca cgaaggacca gaacaagaca gagtgcctcc tgccgatcca aacatgagcc 120

gcctgcccgt cctgctcctg ctccaactcc tggtccgccc cggactccaa gctcccatga 180

cccagacaac gtccttgaag acaagctggg ttaactgctc taacatgatc gatgaaatta 240

taacacactt aaagcagcca cctttgcctt tgctggactt caacaacctc aatggggaag    300

accaagacat tctgatggaa aataaccttc gaaggccaaa cctggaggca ttcaacaggg    360

ctgtcaagag tttacagaac gcatcagcaa ttgagagcat tcttaaaaat ctcctgccat    420

gtctgcccct ggccacggcc gcacccacgc gacatccaat ccatatcaag gacggtgact    480

ggaatgaatt ccggaggaaa ctgacgttct atctgaaaac ccttgagaat gcgcaggctc    540

aacagacgac tttgagcctc gcgatctttt gagtccaacg tccagctcgt tctctgggcc    600

ttctcaccac agagcctcgg gacatcaaaa acagcagaac ttctgaaacc tctgggtcat    660

ctctcacaca ttccaggacc agaagcattt caccttttcc tgcggcatca gatgaattgt    720

taattatcta atttctgaaa tgtgcagctc ccatttggcc ttgtgcggtt gtgttctcat    780

ttttatccca ttgagactat ttatttatgt atgtatgtat ttatttattt attgcctgga    840

gtgtgaactg tatttatttt agcagaggag ccatgtcctg ctgcttctgc aaaaaactca    900

gagtggggtg gggagcatgt tcatttgtac ctcgagtttt aaactggttc ctagggatgt    960

gtgagaataa actagactct gaaca                                          985

<210> 156
<211> 152
<212> PRT
<213> Homo sapiens

<400> 156
Met Ser Arg Leu Pro Val Leu Leu Leu Leu Gln Leu Leu Val Arg Pro
1               5                   10                  15

Gly Leu Gln Ala Pro Met Thr Gln Thr Thr Ser Leu Lys Thr Ser Trp
            20                  25                  30

Val Asn Cys Ser Asn Met Ile Asp Glu Ile Ile Thr His Leu Lys Gln
        35                  40                  45

Pro Pro Leu Pro Leu Leu Asp Phe Asn Asn Leu Asn Gly Glu Asp Gln
    50                  55                  60

Asp Ile Leu Met Glu Asn Asn Leu Arg Arg Pro Asn Leu Glu Ala Phe
65                  70                  75                  80

Asn Arg Ala Val Lys Ser Leu Gln Asn Ala Ser Ala Ile Glu Ser Ile
                85                  90                  95

Leu Lys Asn Leu Leu Pro Cys Leu Pro Leu Ala Thr Ala Ala Pro Thr
            100                 105                 110

Arg His Pro Ile His Ile Lys Asp Gly Asp Trp Asn Glu Phe Arg Arg
        115                 120                 125

Lys Leu Thr Phe Tyr Leu Lys Thr Leu Glu Asn Ala Gln Ala Gln Gln
    130                 135                 140

Thr Thr Leu Ser Leu Ala Ile Phe
145                 150

<210> 157
<211> 1821
<212> DNA
<213> Homo sapiens

<400> 157
acaaggaggc aggcaagaca gcaaggcata gagacaacat agagctaagt aaagccagtg      60

gaaatgaaga gtcttccaat cctactgttg ctgtgcgtgg cagtttgctc agcctatcca     120

ttggatggag ctgcaagggg tgaggacacc agcatgaacc ttgttcagaa atatctagaa     180

aactactacg acctcaaaaa agatgtgaaa cagtttgtta ggagaaagga cagtggtcct     240

gttgttaaaa aaatccgaga aatgcagaag ttccttggat tggaggtgac ggggaagctg     300

gactccgaca ctctggaggt gatgcgcaag cccaggtgtg gagttcctga tgttggtcac     360

ttcagaacct ttcctggcat cccgaagtgg aggaaaaccc accttacata caggattgtg     420

aattatacac cagatttgcc aaaagatgct gttgattctg ctgttgagaa agctctgaaa     480

gtctgggaag aggtgactcc actcacattc tccaggctgt atgaaggaga ggctgatata     540

atgatctctt ttgcagttag agaacatgga gacttttacc cttttgatgg acctggaaat     600

gttttggccc atgcctatgc ccctgggcca gggattaatg gagatgccca ctttgatgat     660

gatgaacaat ggacaaagga tacaacaggg accaatttat ttctcgttgc tgctcatgaa     720

attggccact ccctgggtct ctttcactca gccaacactg aagctttgat gtacccactc     780

tatcactcac tcacagacct gactcggttc cgcctgtctc aagatgatat aaatggcatt     840

cagtccctct atggacctcc ccctgactcc cctgagaccc ccctggtacc cacggaacct     900

gtccctccag aacctgggac gccagccaac tgtgatcctg ctttgtcctt tgatgctgtc     960

agcactctga ggggagaaat cctgatcttt aaagacaggc acttttggcg caaatccctc    1020

aggaagcttg aacctgaatt gcatttgatc tcttcatttt ggccatctct tccttcaggc    1080

gtggatgccg catatgaagt tactagcaag gacctcgttt tcatttttaa aggaaatcaa    1140

ttctgggcca tcagaggaaa tgaggtacga gctggatacc caagaggcat ccacacccta    1200

ggtttccctc caaccgtgag gaaaatcgat gcagccattt ctgataagga aaagaacaaa    1260

acatatttct ttgtagagga caaatactgg agatttgatg agaagagaaa ttccatggag    1320

ccaggctttc ccaagcaaat agctgaagac tttccaggga ttgactcaaa gattgatgct 1380

gtttttgaag aatttgggtt cttttatttc tttactggat cttcacagtt ggagtttgac 1440

ccaaatgcaa agaaagtgac acacactttg aagagtaaca gctggcttaa ttgttgaaag 1500

agatatgtag aaggcacaat atgggcactt taaatgaagc taataattct tcacctaagt 1560

ctctgtgaat tgaaatgttc gttttctcct gcctgtgctg tgactcgagt cacactcaag 1620

ggaacttgag cgtgaatctg tatcttgccg gtcattttta tgttattaca gggcattcaa 1680

atgggctgct gcttagcttg caccttgtca catagagtga tctttcccaa gagaagggga 1740

agcactcgtg tgcaacagac aagtgactgt atctgtgtag actatttgct tatttaataa 1800

agacgatttg tcagttgttt t 1821

<210> 158
<211> 477
<212> PRT
<213> Homo sapiens

<400> 158
Met Lys Ser Leu Pro Ile Leu Leu Leu Leu Cys Val Ala Val Cys Ser
1               5                   10                  15

Ala Tyr Pro Leu Asp Gly Ala Ala Arg Gly Glu Asp Thr Ser Met Asn
            20                  25                  30

Leu Val Gln Lys Tyr Leu Glu Asn Tyr Tyr Asp Leu Lys Lys Asp Val
        35                  40                  45

Lys Gln Phe Val Arg Arg Lys Asp Ser Gly Pro Val Val Lys Lys Ile
    50                  55                  60

Arg Glu Met Gln Lys Phe Leu Gly Leu Glu Val Thr Gly Lys Leu Asp
65                  70                  75                  80

Ser Asp Thr Leu Glu Val Met Arg Lys Pro Arg Cys Gly Val Pro Asp
                85                  90                  95

Val Gly His Phe Arg Thr Phe Pro Gly Ile Pro Lys Trp Arg Lys Thr
            100                 105                 110

His Leu Thr Tyr Arg Ile Val Asn Tyr Thr Pro Asp Leu Pro Lys Asp
        115                 120                 125

Ala Val Asp Ser Ala Val Glu Lys Ala Leu Lys Val Trp Glu Glu Val
    130                 135                 140

Thr Pro Leu Thr Phe Ser Arg Leu Tyr Glu Gly Glu Ala Asp Ile Met
145                 150                 155                 160

Ile Ser Phe Ala Val Arg Glu His Gly Asp Phe Tyr Pro Phe Asp Gly
                165                 170                 175

Pro Gly Asn Val Leu Ala His Ala Tyr Ala Pro Gly Pro Gly Ile Asn
            180                 185                 190

Gly Asp Ala His Phe Asp Asp Asp Glu Gln Trp Thr Lys Asp Thr Thr
        195                 200                 205

Gly Thr Asn Leu Phe Leu Val Ala Ala His Glu Ile Gly His Ser Leu
    210                 215                 220

Gly Leu Phe His Ser Ala Asn Thr Glu Ala Leu Met Tyr Pro Leu Tyr
225                 230                 235                 240

His Ser Leu Thr Asp Leu Thr Arg Phe Arg Leu Ser Gln Asp Asp Ile
                245                 250                 255

Asn Gly Ile Gln Ser Leu Tyr Gly Pro Pro Pro Asp Ser Pro Glu Thr
            260                 265                 270

Pro Leu Val Pro Thr Glu Pro Val Pro Pro Glu Pro Gly Thr Pro Ala
        275                 280                 285

Asn Cys Asp Pro Ala Leu Ser Phe Asp Ala Val Ser Thr Leu Arg Gly
    290                 295                 300

Glu Ile Leu Ile Phe Lys Asp Arg His Phe Trp Arg Lys Ser Leu Arg
305                 310                 315                 320

Lys Leu Glu Pro Glu Leu His Leu Ile Ser Ser Phe Trp Pro Ser Leu
                325                 330                 335

Pro Ser Gly Val Asp Ala Ala Tyr Glu Val Thr Ser Lys Asp Leu Val
            340                 345                 350

Phe Ile Phe Lys Gly Asn Gln Phe Trp Ala Ile Arg Gly Asn Glu Val
        355                 360                 365

Arg Ala Gly Tyr Pro Arg Gly Ile His Thr Leu Gly Phe Pro Pro Thr
    370                 375                 380

Val Arg Lys Ile Asp Ala Ala Ile Ser Asp Lys Glu Lys Asn Lys Thr
385                 390                 395                 400

Tyr Phe Phe Val Glu Asp Lys Tyr Trp Arg Phe Asp Glu Lys Arg Asn
                405                 410                 415

Ser Met Glu Pro Gly Phe Pro Lys Gln Ile Ala Glu Asp Phe Pro Gly
            420                 425                 430

Ile Asp Ser Lys Ile Asp Ala Val Phe Glu Glu Phe Gly Phe Phe Tyr
        435                 440                 445

Phe Phe Thr Gly Ser Ser Gln Leu Glu Phe Asp Pro Asn Ala Lys Lys
    450                 455                 460

Val Thr His Thr Leu Lys Ser Asn Ser Trp Leu Asn Cys
465                 470                 475

<210> 159
<211> 1627
<212> DNA
<213> Homo sapiens

<400> 159
gaagaaacca ccggaaggaa ccatctcact gtgtgtaaac atgacttcca agctggccgt      60

ggctctcttg gcagccttcc tgatttctgc agctctgtgt gaaggtgcag ttttgccaag     120

gagtgctaaa gaacttagat gtcagtgcat aaagacatac tccaaacctt tccaccccaa     180

atttatcaaa gaactgagag tgattgagag tggaccacac tgcgccaaca cagaaattat     240

tgtaaagctt tctgatggaa gagagctctg tctggacccc aaggaaaact gggtgcagag     300

ggttgtggag aagtttttga agagggctga gaattcataa aaaaattcat tctctgtggt     360

atccaagaat cagtgaagat gccagtgaaa cttcaagcaa atctacttca acacttcatg     420

tattgtgtgg gtctgttgta gggttgccag atgcaataca agattcctgg ttaaatttga     480

atttcagtaa acaatgaata gtttttcatt gtaccatgaa atatccagaa catacttata     540

tgtaaagtat tatttatttg aatctacaaa aaacaacaaa taatttttaa atataaggat     600

tttcctagat attgcacggg agaatataca aatagcaaaa ttgaggccaa gggccaagag     660

aatatccgaa ctttaatttc aggaattgaa tgggtttgct agaatgtgat atttgaagca     720

tcacataaaa atgatgggac aataaatttt gccataaagt caaatttagc tggaaatcct     780

ggattttttt ctgttaaatc tggcaaccct agtctgctag ccaggatcca caagtccttg     840

ttccactgtg ccttggtttc tcctttattt ctaagtggaa aaagtattag ccaccatctt     900

acctcacagt gatgttgtga ggacatgtgg aagcacttta agttttttca tcataacata     960

aattattttc aagtgtaact tattaaccta tttattattt atgtatttat ttaagcatca   1020

aatatttgtg caagaatttg gaaaaataga agatgaatca ttgattgaat agttataaag   1080

atgttatagt aaatttattt tattttagat attaaatgat gttttattag ataaatttca   1140

atcagggttt ttagattaaa caaacaaaca attgggtacc cagttaaatt ttcatttcag   1200

atatacaaca aataattttt tagtataagt acattattgt ttatctgaaa ttttaattga   1260

actaacaatc ctagtttgat actcccagtc ttgtcattgc cagctgtgtt ggtagtgctg   1320

tgttgaatta cggaataatg agttagaact attaaaacag ccaaaactcc acagtcaata   1380

ttagtaattt cttgctggtt gaaacttgtt tattatgtac aaatagattc ttataatatt   1440

atttaaatga ctgcattttt aaatacaagg ctttatattt ttaactttaa gatgttttta   1500

tgtgctctcc aaattttttt tactgtttct gattgtatgg aaatataaaa gtaaatatga   1560

aacatttaaa atataatttg ttgtcaaagt aaaaaaaaaa aaaaaaaaaa aaaaaaaaaa   1620

aaaaaaa                                                             1627

<210> 160
<211> 99
<212> PRT
<213> Homo sapiens

<400> 160
Met Thr Ser Lys Leu Ala Val Ala Leu Leu Ala Ala Phe Leu Ile Ser
1               5                   10                  15

Ala Ala Leu Cys Glu Gly Ala Val Leu Pro Arg Ser Ala Lys Glu Leu
            20                  25                  30

Arg Cys Gln Cys Ile Lys Thr Tyr Ser Lys Pro Phe His Pro Lys Phe
        35                  40                  45

Ile Lys Glu Leu Arg Val Ile Glu Ser Gly Pro His Cys Ala Asn Thr
    50                  55                  60

Glu Ile Ile Val Lys Leu Ser Asp Gly Arg Glu Leu Cys Leu Asp Pro
65                  70                  75                  80

Lys Glu Asn Trp Val Gln Arg Val Val Glu Lys Phe Leu Lys Arg Ala
                85                  90                  95

Glu Asn Ser

<210> 161

<211> 3934
<212> DNA
<213> Homo sapiens

<400> 161
cctctcaccc tttagcccag aactgctttg aatacaccaa ttgctgtggg gcggctcgag      60

gaagagaaga caccagtgcc tcagaaactg ctcggtcaga cggtgatagc gagccacgca     120

ttcacagggc cactgctgct cacagaagca gtgaggatga tgccaggatg atgtctgcct     180

cgcgcctggc tgggactctg atcccagcca tggccttcct ctcctgcgtg agaccagaaa     240

gctgggagcc ctgcgtggag acttggccct aaaccacaca gaagagctgg catgaaaccc     300

agagctttca gactccggag cctcagccct tcaccccgat tccattgctt cttgctaaat     360

gctgccgttt tatcacggag gtggttccta atattactta tcaatgcatg gagctgaatt     420

tctacaaaat ccccgacaac ctccccttct caaccaagaa cctggacctg agctttaatc     480

ccctgaggca tttaggcagc tatagcttct tcagtttccc agaactgcag gtgctggatt     540

tatccaggtg tgaaatccag acaattgaag atggggcata tcagagccta agccacctct     600

ctaccttaat attgacagga aaccccatcc agagtttagc cctgggagcc ttttctggac     660

tatcaagttt acagaagctg gtggctgtgg agacaaatct agcatctcta gagaacttcc     720

ccattggaca tctcaaaact ttgaaagaac ttaatgtggc tcacaatctt atccaatctt     780

tcaaattacc tgagtatttt tctaatctga ccaatctaga gcacttggac ctttccagca     840

acaagattca aagtatttat tgcacagact tgcgggttct acatcaaatg cccctactca     900

atctctcttt agacctgtcc ctgaacccta tgaactttat ccaaccaggt gcatttaaag     960

aaattaggct tcataagctg actttaagaa ataattttga tagtttaaat gtaatgaaaa    1020

cttgtattca aggtctggct ggtttagaag tccatcgttt ggttctggga gaatttagaa    1080

atgaaggaaa cttggaaaag tttgacaaat ctgctctaga gggcctgtgc aatttgacca    1140

ttgaagaatt ccgattagca tacttagact actacctcga tgatattatt gacttattta    1200

attgtttgac aaatgtttct tcattttccc tggtgagtgt gactattgaa agggtaaaag    1260

acttttctta taatttcgga tggcaacatt tagaattagt taactgtaaa tttggacagt    1320

ttcccacatt gaaactcaaa tctctcaaaa ggcttacttt cacttccaac aaaggtggga    1380

atgctttttc agaagttgat ctaccaagcc ttgagtttct agatctcagt agaaatggct    1440

tgagtttcaa aggttgctgt tctcaaagtg attttgggac aaccagccta aagtatttag    1500

atctgagctt caatggtgtt attaccatga gttcaaactt cttgggctta gaacaactag    1560

aacatctgga tttccagcat tccaatttga aacaaatgag tgagttttca gtattcctat    1620

cactcagaaa cctcatttac cttgacattt ctcatactca caccagagtt gctttcaatg    1680

gcatcttcaa tggcttgtcc agtctcgaag tcttgaaaat ggctggcaat tctttccagg 1740

aaaacttcct tccagatatc ttcacagagc tgagaaactt gaccttcctg gacctctctc 1800

agtgtcaact ggagcagttg tctccaacag catttaactc actctccagt cttcaggtac 1860

taaatatgag ccacaacaac ttcttttcat tggatacgtt tccttataag tgtctgaact 1920

ccctccaggt tcttgattac agtctcaatc acataatgac ttccaaaaaa caggaactac 1980

agcattttcc aagtagtcta gctttcttaa atcttactca gaatgacttt gcttgtactt 2040

gtgaacacca gagtttcctg caatggatca aggaccagag gcagctcttg gtggaagttg 2100

aacgaatgga atgtgcaaca ccttcagata agcagggcat gcctgtgctg agtttgaata 2160

tcacctgtca gatgaataag accatcattg gtgtgtcggt cctcagtgtg cttgtagtat 2220

ctgttgtagc agttctggtc tataagttct attttcacct gatgcttctt gctggctgca 2280

taaagtatgg tagaggtgaa aacatctatg atgcctttgt tatctactca agccaggatg 2340

aggactgggt aaggaatgag ctagtaaaga atttagaaga aggggtgcct ccatttcagc 2400

tctgccttca ctacagagac tttattcccg gtgtggccat tgctgccaac atcatccatg 2460

aaggtttcca taaaagccga aaggtgattg ttgtggtgtc ccagcacttc atccagagcc 2520

gctggtgtat ctttgaatat gagattgctc agacctggca gtttctgagc agtcgtgctg 2580

gtatcatctt cattgtcctg cagaaggtgg agaagaccct gctcaggcag caggtggagc 2640

tgtaccgcct tctcagcagg aacacttacc tggagtggga ggacagtgtc ctggggcggc 2700

acatcttctg gagacgactc agaaaagccc tgctggatgg taaatcatgg aatccagaag 2760

gaacagtggg tacaggatgc aattggcagg aagcaacatc tatctgaaga ggaaaaataa 2820

aaacctcctg aggcatttct tgcccagctg ggtccaacac ttgttcagtt aataagtatt 2880

aaatgctgcc acatgtcagg ccttatgcta agggtgagta attccatggt gcactagata 2940

tgcagggctg ctaatctcaa ggagcttcca gtgcagaggg aataaatgct agactaaaat 3000

acagagtctt ccaggtgggc atttcaacca actcagtcaa ggaacccatg acaaagaaag 3060

tcatttcaac tcttacctca tcaagttgaa taaagacaga gaaaacagaa agagacattg 3120

ttcttttcct gagtcttttg aatggaaatt gtattatgtt atagccatca taaaaccatt 3180

ttggtagttt tgactgaact gggtgttcac tttttccttt ttgattgaat acaatttaaa 3240

ttctacttga tgactgcagt cgtcaagggg ctcctgatgc aagatgcccc ttccatttta 3300

agtctgtctc cttacagagg ttaaagtcta gtggctaatt cctaaggaaa cctgattaac 3360

acatgctcac aaccatcctg gtcattctcg agcatgttct attttttaac taatcacccc 3420

tgatatattt ttatttttat atatccagtt ttcatttttt tacgtcttgc ctataagcta 3480

atatcataaa taaggttgtt taagacgtgc ttcaaatatc catattaacc actatttttc 3540

aaggaagtat ggaaaagtac actctgtcac tttgtcactc gatgtcattc caaagttatt 3600

gcctactaag taatgactgt catgaaagca gcattgaaat aatttgttta aagggggcac 3660

tcttttaaac gggaagaaaa tttccgcttc ctggtcttat catggacaat ttgggctaga 3720

ggcaggaagg aagtgggatg acctcaggag gtcacctttt cttgattcca gaaacatatg 3780

ggctgataaa cccggggtga cctcatgaaa tgagttgcag cagaagttta tttttttcag 3840

aacaagtgat gtttgatgga cctctgaatc tctttaggga gacacagatg gctgggatcc 3900

ctcccctgta cccttctcac tgccaggaga acta 3934

<210> 162
<211> 799
<212> PRT
<213> Homo sapiens

<400> 162
Met Glu Leu Asn Phe Tyr Lys Ile Pro Asp Asn Leu Pro Phe Ser Thr
1               5                   10                  15

Lys Asn Leu Asp Leu Ser Phe Asn Pro Leu Arg His Leu Gly Ser Tyr
            20                  25                  30

Ser Phe Phe Ser Phe Pro Glu Leu Gln Val Leu Asp Leu Ser Arg Cys
        35                  40                  45

Glu Ile Gln Thr Ile Glu Asp Gly Ala Tyr Gln Ser Leu Ser His Leu
    50                  55                  60

Ser Thr Leu Ile Leu Thr Gly Asn Pro Ile Gln Ser Leu Ala Leu Gly
65                  70                  75                  80

Ala Phe Ser Gly Leu Ser Ser Leu Gln Lys Leu Val Ala Val Glu Thr
                85                  90                  95

Asn Leu Ala Ser Leu Glu Asn Phe Pro Ile Gly His Leu Lys Thr Leu
            100                 105                 110

Lys Glu Leu Asn Val Ala His Asn Leu Ile Gln Ser Phe Lys Leu Pro
        115                 120                 125

Glu Tyr Phe Ser Asn Leu Thr Asn Leu Glu His Leu Asp Leu Ser Ser
    130                 135                 140

Asn Lys Ile Gln Ser Ile Tyr Cys Thr Asp Leu Arg Val Leu His Gln
145                 150                 155                 160

Met Pro Leu Leu Asn Leu Ser Leu Asp Leu Ser Leu Asn Pro Met Asn
                165                 170                 175

Phe Ile Gln Pro Gly Ala Phe Lys Glu Ile Arg Leu His Lys Leu Thr
            180                 185                 190

Leu Arg Asn Asn Phe Asp Ser Leu Asn Val Met Lys Thr Cys Ile Gln
        195                 200                 205

Gly Leu Ala Gly Leu Glu Val His Arg Leu Val Leu Gly Glu Phe Arg
    210                 215                 220

Asn Glu Gly Asn Leu Glu Lys Phe Asp Lys Ser Ala Leu Glu Gly Leu
225                 230                 235                 240

Cys Asn Leu Thr Ile Glu Glu Phe Arg Leu Ala Tyr Leu Asp Tyr Tyr
                245                 250                 255

Leu Asp Asp Ile Ile Asp Leu Phe Asn Cys Leu Thr Asn Val Ser Ser
            260                 265                 270

Phe Ser Leu Val Ser Val Thr Ile Glu Arg Val Lys Asp Phe Ser Tyr
        275                 280                 285

Asn Phe Gly Trp Gln His Leu Glu Leu Val Asn Cys Lys Phe Gly Gln
    290                 295                 300

Phe Pro Thr Leu Lys Leu Lys Ser Leu Lys Arg Leu Thr Phe Thr Ser
305                 310                 315                 320

Asn Lys Gly Gly Asn Ala Phe Ser Glu Val Asp Leu Pro Ser Leu Glu
                325                 330                 335

Phe Leu Asp Leu Ser Arg Asn Gly Leu Ser Phe Lys Gly Cys Cys Ser
            340                 345                 350

Gln Ser Asp Phe Gly Thr Thr Ser Leu Lys Tyr Leu Asp Leu Ser Phe
        355                 360                 365

Asn Gly Val Ile Thr Met Ser Ser Asn Phe Leu Gly Leu Glu Gln Leu
    370                 375                 380

Glu His Leu Asp Phe Gln His Ser Asn Leu Lys Gln Met Ser Glu Phe
385                 390                 395                 400

Ser Val Phe Leu Ser Leu Arg Asn Leu Ile Tyr Leu Asp Ile Ser His
                405                 410                 415

Thr His Thr Arg Val Ala Phe Asn Gly Ile Phe Asn Gly Leu Ser Ser
            420                 425                 430

Leu Glu Val Leu Lys Met Ala Gly Asn Ser Phe Gln Glu Asn Phe Leu
        435                 440                 445

Pro Asp Ile Phe Thr Glu Leu Arg Asn Leu Thr Phe Leu Asp Leu Ser
    450                 455                 460

Gln Cys Gln Leu Glu Gln Leu Ser Pro Thr Ala Phe Asn Ser Leu Ser
465                 470                 475                 480

Ser Leu Gln Val Leu Asn Met Ser His Asn Asn Phe Phe Ser Leu Asp
                485                 490                 495

Thr Phe Pro Tyr Lys Cys Leu Asn Ser Leu Gln Val Leu Asp Tyr Ser
            500                 505                 510

Leu Asn His Ile Met Thr Ser Lys Lys Gln Glu Leu Gln His Phe Pro
        515                 520                 525

Ser Ser Leu Ala Phe Leu Asn Leu Thr Gln Asn Asp Phe Ala Cys Thr
    530                 535                 540

Cys Glu His Gln Ser Phe Leu Gln Trp Ile Lys Asp Gln Arg Gln Leu
545                 550                 555                 560

Leu Val Glu Val Glu Arg Met Glu Cys Ala Thr Pro Ser Asp Lys Gln
                565                 570                 575

Gly Met Pro Val Leu Ser Leu Asn Ile Thr Cys Gln Met Asn Lys Thr
            580                 585                 590

Ile Ile Gly Val Ser Val Leu Ser Val Leu Val Val Ser Val Val Ala
        595                 600                 605

Val Leu Val Tyr Lys Phe Tyr Phe His Leu Met Leu Leu Ala Gly Cys
    610                 615                 620

Ile Lys Tyr Gly Arg Gly Glu Asn Ile Tyr Asp Ala Phe Val Ile Tyr
625                 630                 635                 640

Ser Ser Gln Asp Glu Asp Trp Val Arg Asn Glu Leu Val Lys Asn Leu

645                 650                 655

Glu Glu Gly Val Pro Pro Phe Gln Leu Cys Leu His Tyr Arg Asp Phe
            660                 665                 670

Ile Pro Gly Val Ala Ile Ala Ala Asn Ile Ile His Glu Gly Phe His
        675                 680                 685

Lys Ser Arg Lys Val Ile Val Val Val Ser Gln His Phe Ile Gln Ser
    690                 695                 700

Arg Trp Cys Ile Phe Glu Tyr Glu Ile Ala Gln Thr Trp Gln Phe Leu
705                 710                 715                 720

Ser Ser Arg Ala Gly Ile Ile Phe Ile Val Leu Gln Lys Val Glu Lys
                725                 730                 735

Thr Leu Leu Arg Gln Gln Val Glu Leu Tyr Arg Leu Leu Ser Arg Asn
            740                 745                 750

Thr Tyr Leu Glu Trp Glu Asp Ser Val Leu Gly Arg His Ile Phe Trp
        755                 760                 765

Arg Arg Leu Arg Lys Ala Leu Leu Asp Gly Lys Ser Trp Asn Pro Glu
    770                 775                 780

Gly Thr Val Gly Thr Gly Cys Asn Trp Gln Glu Ala Thr Ser Ile
785                 790                 795

<210> 163
<211> 1182
<212> DNA
<213> Homo sapiens

<400> 163
tagttctccc tgagtgagac ttgcctgctt ctctggcccc tggtcctgtc ctgttctcca      60

gcatggtgtg tctgaagctc cctggaggct cctgcatgac agcgctgaca gtgacactga     120

tggtgctgag ctccccactg gctttggctg gggacacccg accacgtttc ttgtggcagc     180

ttaagtttga atgtcatttc ttcaatggga cggagcgggt gcggttgctg gaaagatgca     240

tctataacca agaggagtcc gtgcgcttcg acagcgacgt gggggagtac cgggcggtga     300

cggagctggg gcggcctgat gccgagtact ggaacagcca gaaggacctc ctggagcaga     360

ggcgggccgc ggtggacacc tactgcagac acaactacgg ggttggtgag agcttcacag     420

tgcagcggcg agttgagcct aaggtgactg tgtatccttc aaagacccag cccctgcagc     480

accacaacct cctggtctgc tctgtgagtg gtttctatcc aggcagcatt gaagtcaggt 540

ggttccggaa cggccaggaa gagaaggctg gggtggtgtc cacaggcctg atccagaatg 600

gagattggac cttccagacc ctggtgatgc tggaaacagt tcctcggagt ggagaggttt 660

acacctgcca agtggagcac ccaagtgtga cgagccctct cacagtggaa tggagagcac 720

ggtctgaatc tgcacagagc aagatgctga gtggagtcgg gggcttcgtg ctgggcctgc 780

tcttccttgg ggccgggctg ttcatctact tcaggaatca gaaaggacac tctggacttc 840

agccaacagg attcctgagc tgaaatgcag atgaccacat tcaaggaaga accttctgtc 900

ccagctttgc agaatgaaaa gctttcctgc ttggcagtta ttcttccaca agagagggct 960

ttctcaggac ctggttgcta ctggttcggc aactgcagaa aatgtcctcc cttgtggctt 1020

cctcagctcc tgcccttggc ctgaagtccc agcattgatg acagcgcctc atcttcaact 1080

tttgtgctcc cctttgccta aaccgtatgg cctcccgtgc atctgtactc accctgtacg 1140

acaaacacat tacattatta aatgtttctc aaagatggag tt 1182

<210> 164
<211> 266
<212> PRT
<213> Homo sapiens

<400> 164
Met Val Cys Leu Lys Leu Pro Gly Gly Ser Cys Met Thr Ala Leu Thr
1               5                   10                  15

Val Thr Leu Met Val Leu Ser Ser Pro Leu Ala Leu Ala Gly Asp Thr
            20                  25                  30

Arg Pro Arg Phe Leu Trp Gln Leu Lys Phe Glu Cys His Phe Phe Asn
        35                  40                  45

Gly Thr Glu Arg Val Arg Leu Leu Glu Arg Cys Ile Tyr Asn Gln Glu
    50                  55                  60

Glu Ser Val Arg Phe Asp Ser Asp Val Gly Glu Tyr Arg Ala Val Thr
65                  70                  75                  80

Glu Leu Gly Arg Pro Asp Ala Glu Tyr Trp Asn Ser Gln Lys Asp Leu
                85                  90                  95

Leu Glu Gln Arg Arg Ala Ala Val Asp Thr Tyr Cys Arg His Asn Tyr
            100                 105                 110

Gly Val Gly Glu Ser Phe Thr Val Gln Arg Arg Val Glu Pro Lys Val
        115                 120                 125

Thr Val Tyr Pro Ser Lys Thr Gln Pro Leu Gln His His Asn Leu Leu
    130                 135                 140

Val Cys Ser Val Ser Gly Phe Tyr Pro Gly Ser Ile Glu Val Arg Trp
145                 150                 155                 160

Phe Arg Asn Gly Gln Glu Glu Lys Ala Gly Val Val Ser Thr Gly Leu
                165                 170                 175

Ile Gln Asn Gly Asp Trp Thr Phe Gln Thr Leu Val Met Leu Glu Thr
            180                 185                 190

Val Pro Arg Ser Gly Glu Val Tyr Thr Cys Gln Val Glu His Pro Ser
        195                 200                 205

Val Thr Ser Pro Leu Thr Val Glu Trp Arg Ala Arg Ser Glu Ser Ala
    210                 215                 220

Gln Ser Lys Met Leu Ser Gly Val Gly Gly Phe Val Leu Gly Leu Leu
225                 230                 235                 240

Phe Leu Gly Ala Gly Leu Phe Ile Tyr Phe Arg Asn Gln Lys Gly His
                245                 250                 255

Ser Gly Leu Gln Pro Thr Gly Phe Leu Ser
            260                 265

<210> 165
<211> 681
<212> DNA
<213> Homo sapiens

<220>
<221> modified_base
<222> (67)..(67)
<223> a, c, t, g, unknown or other

<400> 165
acgtcactgt cttgaagcag cagtagcctg ggaagtgagg caggaggaat tgagaggcag      60

gaagggngct ggagacacag ctgagcctgg aaatgagagt gggcatcgcc gtggtcatca     120

tgactcctct gcggcgtggt caccatgttg gttcactgtg ttgggctctt attgacgggt     180

ctcctgctag gcctgacctt gggtgccgga gccctgctgg cttctgagcc tatctaccaa     240

ccaccttcag cctgggtgcc agctgggggg ctggtggggc tggcgctgct gggagccctg     300

ctcacacttc ggtggccacg tccattcaca gttctgggca caaccctgct gggttctgca     360

gtgcttgtgg cctgtgttga ctacttcctg gaggggctgg cactggggag ttggctgggc 420

caacgcctgc agacacttcc agccttgcct tctctctgct gatatagctg ggtcttactg 480

gggatctggc cagccttggg ggcccttgga gccctggccc agtggaagct cgtgcctgag 540

gaacatggag gccacgctaa tgggtctgtt cctggtttcc cagatgcata aaggaagaca 600

tatccctccc ctgggcagca aggctacaat gggagggagg gagaacatgg gagcatgtga 660

ataaaatggc attaaatact g 681

<210> 166
<211> 105
<212> PRT
<213> Homo sapiens

<400> 166
Met Leu Val His Cys Val Gly Leu Leu Leu Thr Gly Leu Leu Leu Gly
1               5                   10                  15

Leu Thr Leu Gly Ala Gly Ala Leu Leu Ala Ser Glu Pro Ile Tyr Gln
            20                  25                  30

Pro Pro Ser Ala Trp Val Pro Ala Gly Gly Leu Val Gly Leu Ala Leu
        35                  40                  45

Leu Gly Ala Leu Leu Thr Leu Arg Trp Pro Arg Pro Phe Thr Val Leu
    50                  55                  60

Gly Thr Thr Leu Leu Gly Ser Ala Val Leu Val Ala Cys Val Asp Tyr
65                  70                  75                  80

Phe Leu Glu Gly Leu Ala Leu Gly Ser Trp Leu Gly Gln Arg Leu Gln
                85                  90                  95

Thr Leu Pro Ala Leu Pro Ser Leu Cys
            100                 105

<210> 167
<211> 946
<212> DNA
<213> Homo sapiens

<400> 167
ccgggtcgac tactactact actaaattcg cggccgcgtc gaccgcccct tggcttctgc 60

actgatggtg ggtggatgag taatgcatcc aggaagcctg gaggcctgtg gtttccgcac 120

ccgctgccac ccccgcccct agcgtggaca tttatcctct agcgctcagg ccctgccgcc 180

atcgccgcag atccagcgcc cagagagaca ccagagaacc caccatggcc ccctttgagc 240

ccctggcttc tggcatcctg ttgttgctgt ggctgatagc ccccagcagg gcctgcacct 300

gtgtcccacc ccacccacag acggccttct gcaattccga cctcgtcatc agggccaagt 360

tcgtggggac accagaagtc aaccagacca ccttatacca gcgttatgag atcaagatga 420

ccaagatgta taaagggttc caagccttag gggatgccgc tgacatccgg ttcgtctaca 480

cccccgccat ggagagtgtc tgcggatact tccacaggtc ccacaaccgc agcgaggagt 540

ttctcattgc tggaaaactg caggatggac tcttgcacat cactacctgc agtttcgtgg 600

ctccctggaa cagcctgagc ttagctcagc gccggggctt caccaagacc tacactgttg 660

gctgtgagga atgcacagtg tttccctgtt tatccatccc ctgcaaactg cagagtggca 720

ctcattgctt gtggacggac cagctcctcc aaggctctga aaagggcttc cagtcccgtc 780

accttgcctg cctgcctcgg gagccagggc tgtgcacctg gcagtccctg cggtcccaga 840

tagcctgaat cctgcccgga gtggaagctg aagcctgcac agtgtccacc ctgttcccac 900

tcccatcttt cttccggaca atgaaataaa gagttaccac ccagca 946

<210> 168
<211> 207
<212> PRT
<213> Homo sapiens

<400> 168
Met Ala Pro Phe Glu Pro Leu Ala Ser Gly Ile Leu Leu Leu Leu Trp
1               5                   10                  15

Leu Ile Ala Pro Ser Arg Ala Cys Thr Cys Val Pro Pro His Pro Gln
            20                  25                  30

Thr Ala Phe Cys Asn Ser Asp Leu Val Ile Arg Ala Lys Phe Val Gly
        35                  40                  45

Thr Pro Glu Val Asn Gln Thr Thr Leu Tyr Gln Arg Tyr Glu Ile Lys
    50                  55                  60

Met Thr Lys Met Tyr Lys Gly Phe Gln Ala Leu Gly Asp Ala Ala Asp
65                  70                  75                  80

Ile Arg Phe Val Tyr Thr Pro Ala Met Glu Ser Val Cys Gly Tyr Phe
                85                  90                  95

His Arg Ser His Asn Arg Ser Glu Glu Phe Leu Ile Ala Gly Lys Leu
            100                 105                 110

Gln Asp Gly Leu Leu His Ile Thr Thr Cys Ser Phe Val Ala Pro Trp

115                 120                 125

Asn Ser Leu Ser Leu Ala Gln Arg Arg Gly Phe Thr Lys Thr Tyr Thr
    130                 135                 140

Val Gly Cys Glu Glu Cys Thr Val Phe Pro Cys Leu Ser Ile Pro Cys
145                 150                 155                 160

Lys Leu Gln Ser Gly Thr His Cys Leu Trp Thr Asp Gln Leu Leu Gln
                165                 170                 175

Gly Ser Glu Lys Gly Phe Gln Ser Arg His Leu Ala Cys Leu Pro Arg
            180                 185                 190

Glu Pro Gly Leu Cys Thr Trp Gln Ser Leu Arg Ser Gln Ile Ala
        195                 200                 205

<210> 169
<211> 1462
<212> DNA
<213> Homo sapiens

<400> 169
gcccgcgccg cgccgctctt tctccgacag ctgccggggg tgccctgcaa gctgttccgc      60

gcgtcctgcc cgtctgtccc cgcgggtcgt cgcccgccac agccgcgcca tgaccaccca     120

gcagatagac ctccagggcc cggggccgtg gggcttccgc ctcgtgggcg gcaaggactt     180

cgagcagcct ctcgccattt cccgggtcac tcctggaagc aaggcggctc tagctaattt     240

atgtattgga gatgtaatca cagccattga tggggaaaat actagcaata tgacacactt     300

ggaagctcag aacagaatca aaggctgcac agacaacttg actctcactg tagccagatc     360

tgaacataaa gtctggtctc ctctggtgac ggaggaaggg aagcgtcatc catacaagat     420

gaatttagcc tctgaacccc aggaggtcct gcacatagga agcgcccaca accgaagtgc     480

catgcccttt accgcctcgc ctgcctccag cactactgcc agggtcatca caaaccagta     540

caacaaccca gctggcctct actcttctga aaatatctcc aacttcaaca atgccctgga     600

gtcaaagact gctgccagcg gggtggaggc gaacagcaga cccttagacc atgctcagcc     660

tccaagcagc cttgtcatcg acaaagaatc tgaagtttac aagatgcttc aggagaaaca     720

ggagttgaat gagcccccga aacagtccac gtctttcttg gttttgcagg aaatcctgga     780

gtctgaagaa aaaggggatc ccaacaagcc ctcaggattc agaagtgtta aagctcctgt     840

cactaaagtg gctgcgtcga ttggaaatgc tcagaagttg cctatgtgtg acaaatgtgg     900

cactgggatt gttggtgtgt ttgtgaagct gcgggaccgt caccgccacc ctgagtgtta     960

tgtgtgcact gactgtggca ccaacctgaa acagaagggc catttctttg tggaggatca 1020

aatctactgt gagaagcatg cccgggagcg agtcacacca cctgagggtt atgaagtggt 1080

cactgtgttc cccaagtgag ccagcagatc tgaccactgt tctccagcag gcctctgctg 1140

cagctttttc tctcagtgtt ctggccctct cctctcttga aagttctctg cttactttgg 1200

ttttccctct gcttgtaaaa cattgagtcc cctccctgcc ttggttaatt gactcacacc 1260

agctgtgcga tgcccgcttt tacaattaaa ggaaaactgt tttgttcagt gtcaccttgt 1320

cagcaacact gtgtcccttc gccccaccgt tcttctctgc tgcatttgga catcagccaa 1380

atttgaaccc aatcaaatat aacgtgtctg acactgattt tgtttttact caataaatgt 1440

atagactaca aaaaaaaaaa aa 1462

<210> 170
<211> 329
<212> PRT
<213> Homo sapiens

<400> 170
Met Thr Thr Gln Gln Ile Asp Leu Gln Gly Pro Gly Pro Trp Gly Phe
1               5                   10                  15

Arg Leu Val Gly Gly Lys Asp Phe Glu Gln Pro Leu Ala Ile Ser Arg
            20                  25                  30

Val Thr Pro Gly Ser Lys Ala Ala Leu Ala Asn Leu Cys Ile Gly Asp
        35                  40                  45

Val Ile Thr Ala Ile Asp Gly Glu Asn Thr Ser Asn Met Thr His Leu
    50                  55                  60

Glu Ala Gln Asn Arg Ile Lys Gly Cys Thr Asp Asn Leu Thr Leu Thr
65                  70                  75                  80

Val Ala Arg Ser Glu His Lys Val Trp Ser Pro Leu Val Thr Glu Glu
                85                  90                  95

Gly Lys Arg His Pro Tyr Lys Met Asn Leu Ala Ser Glu Pro Gln Glu
            100                 105                 110

Val Leu His Ile Gly Ser Ala His Asn Arg Ser Ala Met Pro Phe Thr
        115                 120                 125

Ala Ser Pro Ala Ser Ser Thr Thr Ala Arg Val Ile Thr Asn Gln Tyr
    130                 135                 140

Asn Asn Pro Ala Gly Leu Tyr Ser Ser Glu Asn Ile Ser Asn Phe Asn
145                 150                 155                 160

Asn Ala Leu Glu Ser Lys Thr Ala Ala Ser Gly Val Glu Ala Asn Ser
                165                 170                 175

Arg Pro Leu Asp His Ala Gln Pro Pro Ser Ser Leu Val Ile Asp Lys
            180                 185                 190

Glu Ser Glu Val Tyr Lys Met Leu Gln Glu Lys Gln Glu Leu Asn Glu
        195                 200                 205

Pro Pro Lys Gln Ser Thr Ser Phe Leu Val Leu Gln Glu Ile Leu Glu
    210                 215                 220

Ser Glu Glu Lys Gly Asp Pro Asn Lys Pro Ser Gly Phe Arg Ser Val
225                 230                 235                 240

Lys Ala Pro Val Thr Lys Val Ala Ala Ser Ile Gly Asn Ala Gln Lys
                245                 250                 255

Leu Pro Met Cys Asp Lys Cys Gly Thr Gly Ile Val Gly Val Phe Val
            260                 265                 270

Lys Leu Arg Asp Arg His Arg His Pro Glu Cys Tyr Val Cys Thr Asp
        275                 280                 285

Cys Gly Thr Asn Leu Lys Gln Lys Gly His Phe Phe Val Glu Asp Gln
    290                 295                 300

Ile Tyr Cys Glu Lys His Ala Arg Glu Arg Val Thr Pro Pro Glu Gly
305                 310                 315                 320

Tyr Glu Val Val Thr Val Phe Pro Lys
                325

<210> 171
<211> 1103
<212> DNA
<213> Homo sapiens

<400> 171
cacagagccc gggccgcagg cacctcctcg ccagctcttc cgctcctctc acagccgcca      60

gacccgcctg ctgagcccca tggcccgcgc tgctctctcc gccgccccca gcaatccccg     120

gctcctgcga gtggcactgc tgctcctgct cctggtagcc gctggccggc gcgcagcagg     180

agcgtccgtg gccactgaac tgcgctgcca gtgcttgcag accctgcagg gaattcaccc     240

caagaacatc caaagtgtga acgtgaagtc ccccggaccc cactgcgccc aaaccgaagt 300

catagccaca ctcaagaatg ggcggaaagc ttgcctcaat cctgcatccc ccatagttaa 360

gaaaatcatc gaaaagatgc tgaacagtga caaatccaac tgaccagaag ggaggaggaa 420

gctcactggt ggctgttcct gaaggaggcc ctgcccttat aggaacagaa gaggaaagag 480

agacacagct gcagaggcca cctggattgt gcctaatgtg tttgagcatc gcttaggaga 540

agtcttctat ttatttattt attcattagt tttgaagatt ctatgttaat attttaggtg 600

taaaataatt aagggtatga ttaactctac ctgcacactg tcctattata ttcattcttt 660

ttgaaatgtc aaccccaagt tagttcaatc tggattcata tttaatttga aggtagaatg 720

ttttcaaatg ttctccagtc attatgttaa tatttctgag gagcctgcaa catgccagcc 780

actgtgatag aggctggcgg atccaagcaa atggccaatg agatcattgt gaaggcaggg 840

gaatgtatgt gcacatctgt tttgtaactg tttagatgaa tgtcagttgt tatttattga 900

aatgatttca cagtgtgtgg tcaacatttc tcatgttgaa actttaagaa ctaaaatgtt 960

ctaaatatcc cttggacatt ttatgtcttt cttgtaaggc atactgcctt gtttaatggt 1020

agttttacag tgtttctggc ttagaacaaa ggggcttaat tattgatgtt ttcatagaga 1080

atataaaaat aaagcactta tag 1103

<210> 172
<211> 107
<212> PRT
<213> Homo sapiens

<400> 172
Met Ala Arg Ala Ala Leu Ser Ala Ala Pro Ser Asn Pro Arg Leu Leu
1               5                   10                  15

Arg Val Ala Leu Leu Leu Leu Leu Leu Val Ala Ala Gly Arg Arg Ala
            20                  25                  30

Ala Gly Ala Ser Val Ala Thr Glu Leu Arg Cys Gln Cys Leu Gln Thr
        35                  40                  45

Leu Gln Gly Ile His Pro Lys Asn Ile Gln Ser Val Asn Val Lys Ser
    50                  55                  60

Pro Gly Pro His Cys Ala Gln Thr Glu Val Ile Ala Thr Leu Lys Asn
65                  70                  75                  80

Gly Arg Lys Ala Cys Leu Asn Pro Ala Ser Pro Ile Val Lys Lys Ile
                85                  90                  95

Ile Glu Lys Met Leu Asn Ser Asp Lys Ser Asn
            100                 105

<210> 173
<211> 1825
<212> DNA
<213> Homo sapiens

<400> 173
tgcaattaaa ggagtcgggt ctctaactgt tgatctgttt ttttcccttc tgagcaatgg      60

agcttaccat ctttatcctg agactggcca tttacatcct gacatttccc ttgtacctgc     120

tgaactttct gggcttgtgg agctggatat gcaaaaaatg gttcccctac ttcttggtga     180

ggttcactgt gatatacaac gaacagatgg caagcaagaa gcgggagctc ttcagtaacc     240

tgcaggagtt tgcgggcccc tccgggaaac tctccctgct ggaagtgggc tgtggcacgg     300

gggccaactt caagttctac ccacctgggt gcagggtgac ctgtattgac cccaacccca     360

actttgagaa gtttttgatc aagagcattg cagagaaccg acacctgcag tttgagcgct     420

ttgtggtagc tgccggggag aacatgcacc aggtggctga tggctctgtg gatgtggtgg     480

tctgcaccct ggtgctgtgc tctgtgaaga accaggagcg gattctccgc gaggtgtgca     540

gagtgctgag accgggaggg gctttctatt tcatggagca tgtggcagct gagtgttcga     600

cttggaatta cttctggcaa caagtcctgg atcctgcctg gcaccttctg tttgatgggt     660

gcaacctgac cagagagagc tggaaggccc tggagcgggc cagcttctct aagctgaagc     720

tgcagcacat ccaggcccca ctgtcctggg agttggtgcg ccctcatatc tatggatatg     780

ctgtgaaata gtgtgagctg gcagttaaga gctgaatggc tcaaagaatt taaagcttca     840

gttttacatt taaaatgcta agtgggagaa gagaaacctt tttttggggg ggcggttttt     900

ttggtttgtt gttggttttt tttttttttt tggcaggaga atctcttgaa cccagaaggc     960

gaaggttgca gtgaaccgag atcatgccat tgtactctag cctgggtgac aagagcaaga    1020

ctccgtctca aaaaaaaaaa aaaaaaaaaa aagaagtaga gacagggaga cggggtctca    1080

ctgtgttgcc taggccggtc ttgaactcct gggctcaagt gattctccca ccttgacctc    1140

ctaaattgtt gggattacag gtgtgagaca gtgcacctgg ccgaaatagc tcaagtttct    1200

gaaaaacaaa tctgaatcta tttgttattc ttagcgtcac tggtctggct ttcagaatta    1260

acatacaagg ttgccacacc tagttctgcc cagctttatg tcttttattc cagtattcca    1320

ccaaagtttg ttttcctgca ttccagttct caagtcttaa gataaagatt gtacttgaca    1380

gtttagtata tccataaaac tatttgaggt ggttaaggtt cttgggttca ttttccttaa    1440

tactttgctg aatattgtag attgtaggca atgaaaaagt ctactaaatt aggaaaacct    1500

tgaataatta ggtatcctag gtaagagccc ctaaacatca agcaatctgt gagtctgtaa 1560

agaaataaat attttttgga ttattcttat ctaattccac ccctgttgga agatgatttc 1620

tttgttcttt gcaactatgg aagctgtgaa aatcatcaca agtgcctctg aaagcgagtg 1680

ttaggttggt tagagggttt aatattttct gcaatggttt gtaggaattt taataaatgt 1740

agtatatttt ctgagatgat tttgtaaaag tactatttta aatatcaaat caaccaataa 1800

attcacattt gtgttaggaa caaaa 1825

<210> 174
<211> 244
<212> PRT
<213> Homo sapiens

<400> 174
Met Glu Leu Thr Ile Phe Ile Leu Arg Leu Ala Ile Tyr Ile Leu Thr
1               5                   10                  15

Phe Pro Leu Tyr Leu Leu Asn Phe Leu Gly Leu Trp Ser Trp Ile Cys
            20                  25                  30

Lys Lys Trp Phe Pro Tyr Phe Leu Val Arg Phe Thr Val Ile Tyr Asn
        35                  40                  45

Glu Gln Met Ala Ser Lys Lys Arg Glu Leu Phe Ser Asn Leu Gln Glu
    50                  55                  60

Phe Ala Gly Pro Ser Gly Lys Leu Ser Leu Leu Glu Val Gly Cys Gly
65                  70                  75                  80

Thr Gly Ala Asn Phe Lys Phe Tyr Pro Pro Gly Cys Arg Val Thr Cys
                85                  90                  95

Ile Asp Pro Asn Pro Asn Phe Glu Lys Phe Leu Ile Lys Ser Ile Ala
            100                 105                 110

Glu Asn Arg His Leu Gln Phe Glu Arg Phe Val Val Ala Ala Gly Glu
        115                 120                 125

Asn Met His Gln Val Ala Asp Gly Ser Val Asp Val Val Val Cys Thr
    130                 135                 140

Leu Val Leu Cys Ser Val Lys Asn Gln Glu Arg Ile Leu Arg Glu Val
145                 150                 155                 160

Cys Arg Val Leu Arg Pro Gly Gly Ala Phe Tyr Phe Met Glu His Val
                165                 170                 175

Ala Ala Glu Cys Ser Thr Trp Asn Tyr Phe Trp Gln Gln Val Leu Asp
            180                 185                 190

Pro Ala Trp His Leu Leu Phe Asp Gly Cys Asn Leu Thr Arg Glu Ser
        195                 200                 205

Trp Lys Ala Leu Glu Arg Ala Ser Phe Ser Lys Leu Lys Leu Gln His
    210                 215                 220

Ile Gln Ala Pro Leu Ser Trp Glu Leu Val Arg Pro His Ile Tyr Gly
225                 230                 235                 240

Tyr Ala Val Lys

<210> 175
<211> 8042
<212> DNA
<213> Homo sapiens

<400> 175
gtctcctgct atcaggtaat gccaacctca accccctgga ccagtcaccc cgatcagact      60

ttgcttgcct gattttaggc caggtcagac atcagaggag agcccagatc cttgtttcct     120

ctccccccgc agggcttccc ctttcttggt catcgatccc tagagctctg gctctttctc     180

ttcttggggt gagaaactgc taagaacagt ttatttccca tgcatgcatc tgggcttgcc     240

actgatctgc cttttccccc ctcctcccca ggttagggac acctgggtcc aaccttccca     300

aggaatatgg ggatgggggt tggagatgaa gtttagtgct agactgtaag gtaggtgggg     360

tggaagtaca tgagggaaag ggctaaaatg gtactaatgg tgttaaggat agggtgaatt     420

atttaagagg aaaaagacta ggcccaggtt ctccttagcc atgtggcttt cccagggcag     480

agggtggccc ctccctaccc aggggcccag cagagactga cttttgatcc ctaccccaga     540

gaccagtgaa ccattaactc ctctcttatg tgagccttca cacactggcc ccacccctga     600

ccccgcccca aggcaggtgg gctaggaggg ggtgcatgtc catctcagcc ttctatgtga     660

cgtggcctcc gatccacctg gacacctgga ggctaagcct ggattccccc ttccctgact     720

caggaactgc ttaacgtcta cagcaaggcc taatagggga cctgagggca cagtcctcag     780

gatgtttcgg ggagaatagg agccagaacc tgagccccta agctattccc ctcaccaatg     840

atggggtccc cagtgagtca tctgctggcc ggcttctgtg tgtgggtcgt cttgggctgg     900

gtagggggct cagtccccaa cctgggccct gctgagcagg agcagaacca ttacctggcc     960

cagctgtttg gcctgtacgg cgagaatggg acgctgactg cagggggctt ggcgcggctt    1020

ctccacagcc tggggctagg ccgagttcag gggcttcgcc tgggacagca tgggcctctg 1080

actggacggg ctgcatcccc agctgcagac aattccacac acaggccaca gaaccctgag 1140

ctgagtgtgg atgtctgggc agggatgcct ctgggtccct cagggtgggg tgacctggaa 1200

gagtcaaagg cccctcacct accccgtggg ccagccccct cgggcctgga cctccttcac 1260

aggcttctgt tgctggacca ctcattggct gaccacctga atgaggattg tctgaacggc 1320

tcccagctgc tggtcaattt tggcttgagc cccgctgctc ctctgacccc tcgtcagttt 1380

gctctgctgt gcccagccct gctttatcag atcgacagcc gcgtctgcat cggcgctccg 1440

gcccctgcac ccccagggga tctactatct gccctgcttc agagtgccct ggcagtcctg 1500

ttgctcagcc tcccttctcc cctatccctg ctgctgctgc ggctcctggg acctcgtcta 1560

ctacggccct tgctgggctt cctgggggcc ctggcggtgg gcactctttg tggggatgca 1620

ctgctacatc tgctaccgca tgcacaagaa gggcggcacg caggacctgg cggactacca 1680

gagaaggacc tgggcccggg gctgtcagtg ctcggaggcc tcttcctgct ctttgtgctg 1740

gagaacatgc tggggctttt gcggcaccga gggctcaggc caagatgctg caggcgaaaa 1800

cgaaggaatc tcgaaacacg caacttggat ccggagaatg gcagtgggat ggcccttcag 1860

cccctacagg cagctccaga gccaggggct cagggccaga gggagaagaa cagccagcac 1920

ccaccagctc tggcccctcc tgggcaccaa ggccacagtc atgggcacca gggtggcact 1980

gatatcacgt ggatggtcct cctgggagat ggtctacaca acctcactga tgggctggcc 2040

ataggtgctg ccttctctga tggcttctcc agcggcctca gtaccacctt agcggtcttc 2100

tgccatgagc tgccccacga actgggtgac tttgccatgc tgctccagtc agggctgtcc 2160

tttcggcggc tgctgctgct gagcctcgtg tctggagccc tgggattggg gggtgcagtc 2220

ctgggggtgg ggctcagcct gggccctgtc cccctcactc cctgggtgtt tggggtcact 2280

gctggggtct tcctctatgt ggcccttgtg gacatgctac cagccctgct tcgtcctccg 2340

gagcccctgc ctacgcccca tgtgctcctg caggggctgg ggctgctgct ggggggcggc 2400

ctcatgcttg ccataaccct gctggaggag cggctactgc ccgtgaccac tgagggctga 2460

tggggccagt ggaaaggggt cgggttgccc ttccttcccc ccaaccacag gaatggaggc 2520

gggacacagg gccagtagga gcaataggat tttaataaac agaacccatc ccaaagccat 2580

gactacgaca gttgtacttg caccaaaaca gcatagaaaa ccagagtgtg gtgggaggac 2640

ccgaagccgg ttgggggagg atgtgagtag gggcctggag ggtgcagggt cattaatctg 2700

cggggagaac attgtgcttt agcccaggga ggggaggggt ggggcaaatg caccgaggtc 2760

cccacttttt cctgctgccc tcggcaccct ggggatgcag gcatctgggc acatctgccc 2820

cttattgctg cccaccagcg ttaaacgccc ccgatcccaa cactagcacc acaggtggtt 2880
ccggggcagg gagaggcagg aatgggaaaa ttgcttagag aaagattcca ctagaatcca 2940
gtgaattgtg ctcagttctc tttacttcct acaaccgagt acatgggtca cagggtggag 3000
ggtgcaacag gacatggaac atgcccctcc gtgcccccca acacacacct gcacacagga 3060
tggtggtgtc tgcagcatca caggtcatgc agggcatggg gaaggggagg ttcacacaca 3120
catagatgcc cacagcgggt accagacgga gaacacccct gaatatacat agctgtacat 3180
ggggaacccc caggtcccca ccccaaccct ctcccctgtc ttgctgtccc ccgcagggga 3240
actatattgc tttgagagag ccaccccagg ggctgctctg ccaggcaccc tcccctccca 3300
cccaccccca ttttggcaca tctgcaagac acacagcagc gagagtaggc accctccctt 3360
cccaggcttc tgtggcctgg agctggagaa gggggtagga gacttcatcc tccatcctcc 3420
cctaaccctt cccaaacccc tgccaaaccc actcaagcca gaacccaccc ccacccccca 3480
aacacacata caaagctgag ctatccagga acacaaggga aacaaggaga ttgtccaggg 3540
tgggagcgga ggcagcgggg gaagaagact ggaagcagag acctcccccc ttgtgggggg 3600
cagactggca caacagctac tttagtgcaa ttggagaggg tgcccagagt gagaggtgga 3660
gaagggaggg aaggcggtcc ccaacttccc tgggggcaaa gtcaggcttc cagattcccc 3720
agggaaaggg cctagcagga gtgggtgagg gccaaggtgg atcctctggt tacccgccac 3780
cctctgccct cccaaatgca gtgacagtgt ccccctcaca cctaagtggg caacagcagc 3840
cttggagtca gtaccttcaa gtaattcaaa gagcagaccc tccccacccc agcttcaccc 3900
catctctggg atttggtcgc ttctctaggg gttgggttgg gaggagggag cccccaaggc 3960
agacccttcc ctctctacct cccgattccc agaccactgg gcttggtcct caaagattcc 4020
tcacctccgc ccttgcccaa cctgggtcaa ggctgcagaa ggctggagcc accacaatta 4080
gaggggaagg ggctgctttg ttccttatcc ctccttctta aaaggtaggg ttcaaactag 4140
gcgggatggg ggcccatact ggtttgcccc aggagtaggg tttctgggct agggtctgta 4200
aggctatttt cctttgcggt gggaagggga ggtaggggat gaacactggg tatgggaagt 4260
gggtgagaaa tggctgagag ggaaggagga aggggcctcc ccgctggagc agtcactgga 4320
gtcatttaga caaaaacact catgtgcata agatacacag tgcgcaaact cagccctgcc 4380
agcccggccc caatcccacc tctcaggact ccttccaaga ccctggagga ggttctgggg 4440
atacagctgt agaaccgttc actctggccc catccacccc acctccagcc tcttctcccc 4500
ttctaggtcc agggagtaag aaggtgctcg ggtgggcaga cagtggtgga aacagtattg 4560
agttttcctt tggttacata ttgaaggcaa aggtgagctg gacttacagt caaaacggat 4620
aggggtgagg aaggaagagg ggccatggct ggggttggag agggaggtag gccctcctca 4680

gcccctccac cccaagaaac acatctacgt ggggtggcag tccctcagtc ttttctctcc 4740

cacgccccac agcaccaaac aaaaggagaa gccccctccc ccaggggctg ctccccaccc 4800

caacctgggc tgtacattca gtggttttca gcagtcctat ggctcagggg gccacggggc 4860

aggggaggtg gcccgtcagt ctcagtggac agtggcagtg acccgggtct gctggtcctt 4920

cctgggaccc acggtttgtg ccattcgttt ttctgaagga tagtatatga ccctaccaga 4980

gccaccgccc aatcagaaat agctccccca aaatcagaaa ctaaaaactg gctctttcca 5040

tctcaccctc tgattcacac gtctgtttcc ctgtctccta tgtcgccctc gctctgtttc 5100

ctttgctggg acctggggct gctcccaggg tcctcgctta aaataggcct tttcacaaaa 5160

gtgcttatta cttgaagcaa gtgctttagg gctgcggtgg ggagaggggg gagggggagg 5220

gcaaagggaa ggcgctgttc cctccccgcc ctcccccatg aagactgggg ggcattctgc 5280

ctgttttact tctccacacc cgtttaagtc aaagaggttt aaaaaaaatc ttaacattaa 5340

aataaatatg cagtggccat gagaatggtc aaaatgcttc aaaaaacact agggctgggg 5400

agcccagggt ggggcgtggg gtttcctttg gcataaggag aggaggctgc ttgtccccca 5460

ggcttcctca cagccacata tggaggacag gaggactccc caccacctcc cagcactgct 5520

gacagtgcgc tgagggcagc agggcgcaga cagcccccag aaatctcatc tagcaagaca 5580

acggggctct gacactcaga cgcttcccgc catcaccaaa catcacgaaa ggagaacacg 5640

actcacaccc ggcaagttct ctctggagga cccatcttct cactcccctt gcccccaccc 5700

acctaattcc cagcaccaag atgagaggga gggggtggaa tgggaagatt tcaggagaga 5760

gaattgaagg gaaggcaagc tttgcagagc cagcagggtt ggggatgggg gctgcagccc 5820

ttaagagagg tcacattgat tgtcatatag gggaggacac ggtgtggagg gaggtgggca 5880

agaggggcgt cacaaggccc tttggctttg aaaataaaaa taaaaactaa aagctgcaca 5940

atccttctca tcaataatgg tcatttggaa gccttgaaat gatttaggaa ctgagggttg 6000

ggagaagcaa aacacagaga gacaggggat caaaagggac catgaaaaga taaggacttg 6060

gacaacaccc caggcttctg gaaaccagca tgaggtatgg ttaaaggtat ccgttgggga 6120

gggagggggt ccctgggacg cccccctccc ctcagcccac ccttgacaag ggtggcaaga 6180

cattcagggt atacaactaa aagatgggta agactgtgaa taaagcaaac ttgagtaggg 6240

gagggaggct gaaggatccc ccctcagggg agaccctgta ctgtgtaaac gaagcctgtc 6300

cagttctcag gggacaatac tgatggcagc caaactgggc aaggatgcag tgtgggggcg 6360

gagggggcat gacctctatt caagttctgt gtcttggccc ctggctgagg tattgagtgt 6420

gaggaaggga acactgggct gcaggagtgg gtccaactgt ccaggaggct gcactaggag 6480

atgggtggac agagcactta aagggaccag ggccaaattc aatgctacat ttagagagaa 6540

aactgccccc tcttcactcc agcccagttt ggcttttggg gtgcgacttt agaaatctta 6600

tcagtgcagc ccccagctca atcccatctt tcctccctgc tgggaaggca gggaccaagc 6660

cccagtacct attcatctct ctctaggtca gagcttcttc ctcacactgg aggggaagcc 6720

tgggtcccga gtagccacgg tcccttgtcg gccctgcccc tcctaccatc tttggcttca 6780

gatcaggagt gactggggca gtgggtactc ttggacataa cggaaggtgg aggcagatag 6840

ggagcacaga taaactatgg gcttggcggc acaggaggaa gaacatggtc cctccctcca 6900

tatacatgca aacacatgcc tgaaacacag tggacatact agggctgtgg ttttgaaatg 6960

ggcaggaaat taatttgttt cttcccctaa aggataaaac gcttacttaa aaattcatga 7020

caagcctcaa agttcaaggg aaggagagcc agggcaggaa tgaccacagg ctctgccctc 7080

ccaaaagaag ataaaagaaa gaagcaaggt taaagtgcgt ggttaggggc caggctagga 7140

gtgggaggga atggggaggg ggcaggctgt gggggcagaa ccagcccttg atttgcatat 7200

gaggagccag gggagagtgc aggattgggg cccaggtaca gtagttgctg cttcaagtgt 7260

tttgcatttg aactttaggg gtgatagggt acagaagtgg ggaaaaagct ccaggcccca 7320

gtgctaaata tcctcccccc tcatcctcta gctgcccctg aaggggggag ggggacaccc 7380

ccagagtgct cacacagtac cagaaattaa ggcttctcac tgctgcgggg atggagggac 7440

cggccgagca gggaggcagt gatgggtatg gaagaagagg ggatctgcct ggcagtaggg 7500

gcaggggaga agggggacac agagaggagt ccccagggaa gagtcggggg agctggcagc 7560

agaaagaggg aaccaagaga tggagtcaaa gagtggggga gccaggcatt tagcaatcat 7620

tttgggacac ttggcagaag gggtcgccct gggggtacca agggcctggg gtgctccctg 7680

tcagtcccag acccctgcca ggccaagatg gtgtggcaaa ggggtgagca gctgctcccc 7740

agggcttcct tcccctccgc cccctccacc cagtcgtgtt ctcctttaaa gtgccctaaa 7800

tgtttagact ttttctaaat aaatagaatt agatatcaag ccaccggcgg gggagggaca 7860

ctggaggggg ctactcagag tagcagccat ctaacccaat ggcgccgggc cgctcctggc 7920

tgtaggggca ggaggcccca tggggcaggg cgccgcagcc acccaccgtc ttggcggctg 7980

caatgctgca gttcttgagc aggctgaagc tgaaaggact gacggcgtcc ttgggtggga 8040

aa 8042

<210> 176
<211> 540
<212> PRT
<213> Homo sapiens

<400> 176

Met Met Gly Ser Pro Val Ser His Leu Leu Ala Gly Phe Cys Val Trp
1               5                   10                  15

Val Val Leu Gly Trp Val Gly Gly Ser Val Pro Asn Leu Gly Pro Ala
            20                  25                  30

Glu Gln Glu Gln Asn His Tyr Leu Ala Gln Leu Phe Gly Leu Tyr Gly
        35                  40                  45

Glu Asn Gly Thr Leu Thr Ala Gly Gly Leu Ala Arg Leu Leu His Ser
    50                  55                  60

Leu Gly Leu Gly Arg Val Gln Gly Leu Arg Leu Gly Gln His Gly Pro
65                  70                  75                  80

Leu Thr Gly Arg Ala Ala Ser Pro Ala Ala Asp Asn Ser Thr His Arg
                85                  90                  95

Pro Gln Asn Pro Glu Leu Ser Val Asp Val Trp Ala Gly Met Pro Leu
            100                 105                 110

Gly Pro Ser Gly Trp Gly Asp Leu Glu Glu Ser Lys Ala Pro His Leu
        115                 120                 125

Pro Arg Gly Pro Ala Pro Ser Gly Leu Asp Leu Leu His Arg Leu Leu
    130                 135                 140

Leu Leu Asp His Ser Leu Ala Asp His Leu Asn Glu Asp Cys Leu Asn
145                 150                 155                 160

Gly Ser Gln Leu Leu Val Asn Phe Gly Leu Ser Pro Ala Ala Pro Leu
                165                 170                 175

Thr Pro Arg Gln Phe Ala Leu Leu Cys Pro Ala Leu Leu Tyr Gln Ile
            180                 185                 190

Asp Ser Arg Val Cys Ile Gly Ala Pro Ala Pro Ala Pro Pro Gly Asp
        195                 200                 205

Leu Leu Ser Ala Leu Leu Gln Ser Ala Leu Ala Val Leu Leu Leu Ser
    210                 215                 220

Leu Pro Ser Pro Leu Ser Leu Leu Leu Leu Arg Leu Leu Gly Pro Arg
225                 230                 235                 240

Leu Leu Arg Pro Leu Leu Gly Phe Leu Gly Ala Leu Ala Val Gly Thr

245                 250                 255

Leu Cys Gly Asp Ala Leu Leu His Leu Leu Pro His Ala Gln Glu Gly
            260                 265                 270

Arg His Ala Gly Pro Gly Gly Leu Pro Glu Lys Asp Leu Gly Pro Gly
        275                 280                 285

Leu Ser Val Leu Gly Gly Leu Phe Leu Leu Phe Val Leu Glu Asn Met
    290                 295                 300

Leu Gly Leu Leu Arg His Arg Gly Leu Arg Pro Arg Cys Cys Arg Arg
305                 310                 315                 320

Lys Arg Arg Asn Leu Glu Thr Arg Asn Leu Asp Pro Glu Asn Gly Ser
                325                 330                 335

Gly Met Ala Leu Gln Pro Leu Gln Ala Ala Pro Glu Pro Gly Ala Gln
            340                 345                 350

Gly Gln Arg Glu Lys Asn Ser Gln His Pro Pro Ala Leu Ala Pro Pro
        355                 360                 365

Gly His Gln Gly His Ser His Gly His Gln Gly Gly Thr Asp Ile Thr
    370                 375                 380

Trp Met Val Leu Leu Gly Asp Gly Leu His Asn Leu Thr Asp Gly Leu
385                 390                 395                 400

Ala Ile Gly Ala Ala Phe Ser Asp Gly Phe Ser Ser Gly Leu Ser Thr
                405                 410                 415

Thr Leu Ala Val Phe Cys His Glu Leu Pro His Glu Leu Gly Asp Phe
            420                 425                 430

Ala Met Leu Leu Gln Ser Gly Leu Ser Phe Arg Arg Leu Leu Leu Leu
        435                 440                 445

Ser Leu Val Ser Gly Ala Leu Gly Leu Gly Gly Ala Val Leu Gly Val
    450                 455                 460

Gly Leu Ser Leu Gly Pro Val Pro Leu Thr Pro Trp Val Phe Gly Val
465                 470                 475                 480

Thr Ala Gly Val Phe Leu Tyr Val Ala Leu Val Asp Met Leu Pro Ala
                485                 490                 495

Leu Leu Arg Pro Pro Glu Pro Leu Pro Thr Pro His Val Leu Leu Gln
            500                 505                 510

Gly Leu Gly Leu Leu Leu Gly Gly Gly Leu Met Leu Ala Ile Thr Leu
        515                 520                 525

Leu Glu Glu Arg Leu Leu Pro Val Thr Thr Glu Gly
    530                 535                 540

<210> 177
<211> 3600
<212> DNA
<213> Homo sapiens

<400> 177
   cccagactcc agccctggac cgcgcatccc gagcccagcg cccagacaga gtgtccccac      60
   accctcctct gagacgccat gttcaactcg atgaccccac caccaatcag tagctatggc     120
   gagccctgct gtctccggcc cctccccagt cagggggccc ccagtgtggg gacagaagga     180
   ctgtctggcc cgcccttctg ccaccaagct aacctcatgt ccggccccca cagttatggg     240
   ccagccagag agaccaacag ctgcaccgag ggcccactct tttcttctcc ccggagtgca     300
   gtcaagttga ccaagaagcg ggcactgtcc atctcacctc tgtcggatgc cagcctggac     360
   ctgcagacgg ttatccgcac ctcacccagc tccctcgtag ctttcatcaa ctcgcgatgc     420
   acatctccag gaggctccta cggtcatctc tccattggca ccatgagccc atctctggga     480
   ttcccagccc agatgaatca ccaaaaaggg ccctcgcctt cctttggggt ccagccttgt     540
   ggtccccatg actctgcccg gggtgggatg atcccacatc ctcagtcccg gggacccttc     600
   ccaacttgcc agctgaagtc tgagctggac atgctggttg gcaagtgccg ggaggaaccc     660
   ttggaaggtg atatgtccag ccccaactcc acaggcatac aggatcccct gttggggatg     720
   ctggatgggc gggaggacct cgagagagag gagaagcgtg agcctgaatc tgtgtatgaa     780
   actgactgcc gttgggatgg ctgcagccag gaatttgact cccaagagca gctggtgcac     840
   cacatcaaca gcgagcacat ccacggggag cggaaggagt tcgtgtgcca ctgggggggc     900
   tgctccaggg agctgaggcc cttcaaagcc cagtacatgc tggtggttca catgcgcaga     960
   cacactggcg agaagccaca caagtgcacg tttgaagggt gccggaagtc atactcacgc    1020
   ctcgaaaacc tgaagacgca cctgcggtca cacacgggtg agaagccata catgtgtgag    1080
   cacgagggct gcagtaaagc cttcagcaat gccagtgacc gagccaagca ccagaatcgg    1140
   acccattcca atgagaagcc gtatgtatgt aagctccctg gctgcaccaa acgctataca    1200
   gatcctagct cgctgcgaaa acatgtcaag acagtgcatg gtcctgacgc ccatgtgacc    1260
   aaacggcacc gtggggatgg ccccctgcct cgggcaccat ccatttctac agtggagccc 1320
   aagagggagc gggaaggagg tcccatcagg gaggaaagca gactgactgt gccagagggt 1380
   gccatgaagc cacagccaag ccctggggcc cagtcatcct gcagcagtga ccactccccg 1440
   gcagggagtg cagccaatac agacagtggt gtggaaatga ctggcaatgc agggggcagc 1500
   actgaagacc tctccagctt ggacgaggga ccttgcattg ctggcactgg tctgtccact 1560
   cttcgccgcc ttgagaacct caggctggac cagctacatc aactccggcc aatagggacc 1620
   cggggtctca aactgcccag cttgtcccac accggtacca ctgtgtcccg ccgcgtgggc 1680
   cccccagtct ctcttgaacg ccgcagcagc agctccagca gcatcagctc tgcctatact 1740
   gtcagccgcc gctcctccct ggcctctcct ttcccccctg gctccccacc agagaatgga 1800
   gcatcctccc tgcctggcct tatgcctgcc cagcactacc tgcttcgggc aagatatgct 1860
   tcagccagag ggggtggtac ttcgcccact gcagcatcca gcctggatcg gataggtggt 1920
   cttcccatgc ctccttggag aagccgagcc gagtatccag gatacaaccc caatgcaggg 1980
   gtcacccgga gggccagtga cccagcccag gctgctgacc gtcctgctcc agctagagtc 2040
   cagaggttca agagcctggg ctgtgtccat accccaccca ctgtggcagg gggaggacag 2100
   aactttgatc cttacctccc aacctctgtc tactcaccac agccccccag catcactgag 2160
   aatgctgcca tggatgctag agggctacag gaagagccag aagttgggac ctccatggtg 2220
   ggcagtggtc tgaaccccta tatggacttc ccacctactg atactctggg atatggggga 2280
   cctgaagggg cagcagctga gccttatgga gcgaggggtc caggctctct gcctcttggg 2340
   cctggtccac ccaccaacta tggccccaac ccctgtcccc agcaggcctc atatcctgac 2400
   cccacccaag aaacatgggg tgagttccct tcccactctg ggctgtaccc aggccccaag 2460
   gctctaggtg gaacctacag ccagtgtcct cgacttgaac attatggaca agtgcaagtc 2520
   aagccagaac aggggtgccc agtggggtct gactccacag gactggcacc ctgcctcaat 2580
   gcccacccca gtgaggggcc cccacatcca cagcctctct tttcccatta cccccagccc 2640
   tctcctcccc aatatctcca gtcaggcccc tatacccagc caccccctga ttatcttcct 2700
   tcagaaccca ggccttgcct ggactttgat tcccccaccc attccacagg gcagctcaag 2760
   gctcagcttg tgtgtaatta tgttcaatct caacaggagc tactgtggga gggtgggggc 2820
   agggaagatg cccccgccca ggaaccttcc taccagagtc ccaagtttct ggggggttcc 2880
   caggttagcc caagccgtgc taaagctcca gtgaacacat atggacctgg ctttggaccc 2940
   aacttgccca atcacaagtc aggttcctat cccacccctt caccatgcca tgaaaatttt 3000
   gtagtggggg caaatagggc ttcacatagg gcagcagcac cacctcgact tctgccccca 3060
   ttgcccactt gctatgggcc tctcaaagtg ggaggcacaa accccagctg tggtcatcct 3120
   gaggtgggca ggctaggagg gggtcctgcc ttgtaccctc ctcccgaagg acaggtatgt 3180
   aaccccctgg actctcttga tcttgacaac actcagctgg actttgtggc tattctggat 3240
   gagccccagg ggctgagtcc tcctccttcc catgatcagc ggggcagctc tggacatacc 3300
   ccacctccct ctgggccccc caacatggct gtgggcaaca tgagtgtctt actgagatcc 3360
   ctacctgggg aaacagaatt cctcaactct agtgcctaaa gagtagggaa tctcatccat 3420
   cacagatcgc atttcctaag gggtttctat ccttccagaa aaattggggg agctgcagtc 3480
   ccctgcacaa gatgccccag ggatgggagg tatgggctgg gggctatgta tagtctgtat 3540
   acgttttgag gagaaatttg ataatgacac tgtttcctga taataaagga actgcatcag 3600

<210> 178
<211> 1106
<212> PRT
<213> Homo sapiens

<400> 178
Met Phe Asn Ser Met Thr Pro Pro Pro Ile Ser Ser Tyr Gly Glu Pro
1               5                   10                  15

Cys Cys Leu Arg Pro Leu Pro Ser Gln Gly Ala Pro Ser Val Gly Thr
            20                  25                  30

Glu Gly Leu Ser Gly Pro Pro Phe Cys His Gln Ala Asn Leu Met Ser
        35                  40                  45

Gly Pro His Ser Tyr Gly Pro Ala Arg Glu Thr Asn Ser Cys Thr Glu
    50                  55                  60

Gly Pro Leu Phe Ser Ser Pro Arg Ser Ala Val Lys Leu Thr Lys Lys
65                  70                  75                  80

Arg Ala Leu Ser Ile Ser Pro Leu Ser Asp Ala Ser Leu Asp Leu Gln
                85                  90                  95

Thr Val Ile Arg Thr Ser Pro Ser Ser Leu Val Ala Phe Ile Asn Ser
            100                 105                 110

Arg Cys Thr Ser Pro Gly Gly Ser Tyr Gly His Leu Ser Ile Gly Thr
        115                 120                 125

Met Ser Pro Ser Leu Gly Phe Pro Ala Gln Met Asn His Gln Lys Gly
    130                 135                 140

Pro Ser Pro Ser Phe Gly Val Gln Pro Cys Gly Pro His Asp Ser Ala
145                 150                 155                 160

Arg Gly Gly Met Ile Pro His Pro Gln Ser Arg Gly Pro Phe Pro Thr
                165                 170                 175

Cys Gln Leu Lys Ser Glu Leu Asp Met Leu Val Gly Lys Cys Arg Glu
            180                 185                 190

Glu Pro Leu Glu Gly Asp Met Ser Ser Pro Asn Ser Thr Gly Ile Gln
        195                 200                 205

Asp Pro Leu Leu Gly Met Leu Asp Gly Arg Glu Asp Leu Glu Arg Glu
    210                 215                 220

Glu Lys Arg Glu Pro Glu Ser Val Tyr Glu Thr Asp Cys Arg Trp Asp
225                 230                 235                 240

Gly Cys Ser Gln Glu Phe Asp Ser Gln Glu Gln Leu Val His His Ile
                245                 250                 255

Asn Ser Glu His Ile His Gly Glu Arg Lys Glu Phe Val Cys His Trp
            260                 265                 270

Gly Gly Cys Ser Arg Glu Leu Arg Pro Phe Lys Ala Gln Tyr Met Leu
        275                 280                 285

Val Val His Met Arg Arg His Thr Gly Glu Lys Pro His Lys Cys Thr
    290                 295                 300

Phe Glu Gly Cys Arg Lys Ser Tyr Ser Arg Leu Glu Asn Leu Lys Thr
305                 310                 315                 320

His Leu Arg Ser His Thr Gly Glu Lys Pro Tyr Met Cys Glu His Glu
                325                 330                 335

Gly Cys Ser Lys Ala Phe Ser Asn Ala Ser Asp Arg Ala Lys His Gln
            340                 345                 350

Asn Arg Thr His Ser Asn Glu Lys Pro Tyr Val Cys Lys Leu Pro Gly
        355                 360                 365

Cys Thr Lys Arg Tyr Thr Asp Pro Ser Ser Leu Arg Lys His Val Lys
    370                 375                 380

Thr Val His Gly Pro Asp Ala His Val Thr Lys Arg His Arg Gly Asp

385                 390                 395                 400

Gly Pro Leu Pro Arg Ala Pro Ser Ile Ser Thr Val Glu Pro Lys Arg
                405                 410                 415

Glu Arg Glu Gly Gly Pro Ile Arg Glu Glu Ser Arg Leu Thr Val Pro
            420                 425                 430

Glu Gly Ala Met Lys Pro Gln Pro Ser Pro Gly Ala Gln Ser Ser Cys
        435                 440                 445

Ser Ser Asp His Ser Pro Ala Gly Ser Ala Ala Asn Thr Asp Ser Gly
    450                 455                 460

Val Glu Met Thr Gly Asn Ala Gly Gly Ser Thr Glu Asp Leu Ser Ser
465                 470                 475                 480

Leu Asp Glu Gly Pro Cys Ile Ala Gly Thr Gly Leu Ser Thr Leu Arg
                485                 490                 495

Arg Leu Glu Asn Leu Arg Leu Asp Gln Leu His Gln Leu Arg Pro Ile
            500                 505                 510

Gly Thr Arg Gly Leu Lys Leu Pro Ser Leu Ser His Thr Gly Thr Thr
        515                 520                 525

Val Ser Arg Arg Val Gly Pro Pro Val Ser Leu Glu Arg Arg Ser Ser
    530                 535                 540

Ser Ser Ser Ser Ile Ser Ser Ala Tyr Thr Val Ser Arg Arg Ser Ser
545                 550                 555                 560

Leu Ala Ser Pro Phe Pro Pro Gly Ser Pro Pro Glu Asn Gly Ala Ser
                565                 570                 575

Ser Leu Pro Gly Leu Met Pro Ala Gln His Tyr Leu Leu Arg Ala Arg
            580                 585                 590

Tyr Ala Ser Ala Arg Gly Gly Gly Thr Ser Pro Thr Ala Ala Ser Ser
        595                 600                 605

Leu Asp Arg Ile Gly Gly Leu Pro Met Pro Pro Trp Arg Ser Arg Ala
    610                 615                 620

Glu Tyr Pro Gly Tyr Asn Pro Asn Ala Gly Val Thr Arg Arg Ala Ser
625                 630                 635                 640

Asp Pro Ala Gln Ala Ala Asp Arg Pro Ala Pro Ala Arg Val Gln Arg
                645                 650                 655

Phe Lys Ser Leu Gly Cys Val His Thr Pro Pro Thr Val Ala Gly Gly
            660                 665                 670

Gly Gln Asn Phe Asp Pro Tyr Leu Pro Thr Ser Val Tyr Ser Pro Gln
        675                 680                 685

Pro Pro Ser Ile Thr Glu Asn Ala Ala Met Asp Ala Arg Gly Leu Gln
    690                 695                 700

Glu Glu Pro Glu Val Gly Thr Ser Met Val Gly Ser Gly Leu Asn Pro
705                 710                 715                 720

Tyr Met Asp Phe Pro Pro Thr Asp Thr Leu Gly Tyr Gly Gly Pro Glu
                725                 730                 735

Gly Ala Ala Ala Glu Pro Tyr Gly Ala Arg Gly Pro Gly Ser Leu Pro
            740                 745                 750

Leu Gly Pro Gly Pro Pro Thr Asn Tyr Gly Pro Asn Pro Cys Pro Gln
        755                 760                 765

Gln Ala Ser Tyr Pro Asp Pro Thr Gln Glu Thr Trp Gly Glu Phe Pro
    770                 775                 780

Ser His Ser Gly Leu Tyr Pro Gly Pro Lys Ala Leu Gly Gly Thr Tyr
785                 790                 795                 800

Ser Gln Cys Pro Arg Leu Glu His Tyr Gly Gln Val Gln Val Lys Pro
                805                 810                 815

Glu Gln Gly Cys Pro Val Gly Ser Asp Ser Thr Gly Leu Ala Pro Cys
            820                 825                 830

Leu Asn Ala His Pro Ser Glu Gly Pro Pro His Pro Gln Pro Leu Phe
        835                 840                 845

Ser His Tyr Pro Gln Pro Ser Pro Pro Gln Tyr Leu Gln Ser Gly Pro
    850                 855                 860

Tyr Thr Gln Pro Pro Pro Asp Tyr Leu Pro Ser Glu Pro Arg Pro Cys
865                 870                 875                 880

Leu Asp Phe Asp Ser Pro Thr His Ser Thr Gly Gln Leu Lys Ala Gln
                885                 890                 895

Leu Val Cys Asn Tyr Val Gln Ser Gln Gln Glu Leu Leu Trp Glu Gly
            900                 905                 910

Gly Gly Arg Glu Asp Ala Pro Ala Gln Glu Pro Ser Tyr Gln Ser Pro
        915                 920                 925

Lys Phe Leu Gly Gly Ser Gln Val Ser Pro Ser Arg Ala Lys Ala Pro
    930                 935                 940

Val Asn Thr Tyr Gly Pro Gly Phe Gly Pro Asn Leu Pro Asn His Lys
945                 950                 955                 960

Ser Gly Ser Tyr Pro Thr Pro Ser Pro Cys His Glu Asn Phe Val Val
                965                 970                 975

Gly Ala Asn Arg Ala Ser His Arg Ala Ala Ala Pro Pro Arg Leu Leu
            980                 985                 990

Pro Pro Leu Pro Thr Cys Tyr Gly  Pro Leu Lys Val Gly  Gly Thr Asn
        995                 1000                1005

Pro Ser  Cys Gly His Pro Glu  Val Gly Arg Leu Gly  Gly Gly Pro
    1010                1015                1020

Ala Leu  Tyr Pro Pro Pro Glu  Gly Gln Val Cys Asn  Pro Leu Asp
    1025                1030                1035

Ser Leu  Asp Leu Asp Asn Thr  Gln Leu Asp Phe Val  Ala Ile Leu
    1040                1045                1050

Asp Glu  Pro Gln Gly Leu Ser  Pro Pro Pro Ser His  Asp Gln Arg
    1055                1060                1065

Gly Ser  Ser Gly His Thr Pro  Pro Pro Ser Gly Pro  Pro Asn Met
    1070                1075                1080

Ala Val  Gly Asn Met Ser Val  Leu Leu Arg Ser Leu  Pro Gly Glu
    1085                1090                1095

Thr Glu  Phe Leu Asn Ser Ser  Ala
    1100                1105

<210> 179
<211> 4111
<212> DNA
<213> Homo sapiens

<400> 179
acacaccaca cacactcaca ctcacacaca ctcacacaca ctcatcccct tgaatcttgg 60

ggcaggaact cagaaaactt ccagcccggg cagcgcgcgc ttggtgcaag actcaggagc 120

tagcagcccg tccccctccg actctccggt gccgccgctg cctgctcccg ccaccctagg 180

aggcgcggtg ccacccacta ctctgtcctc tgcctgtgct ccgtgcccga ccctatcccg 240

gcggagtctc cccatcctcc tttgctttcc gactgcccaa ggcactttca atctcaatct 300

cttctctctc tctctctctc tctctgtctc tctctctctc tctctctctc tctctctcgc 360

agggtggggg gaagaggagg aggaattctt tccccgccta acatttcaag ggacacaatt 420

cactccaagt ctcttccctt tccaagccgc ttccgaagtg ctcccggtgc ccgcaactcc 480

tgatcccaac ccgcgagagg agcctctgcg acctcaaagc ctctcttcct tctccctcgc 540

ttccctcctc ctcttgctac ctccacctcc accgccacct ccacctccgg cacccaccca 600

ccgccgccgc cgccaccggc agcgcctcct cctctcctcc tcctcctccc ctcttctctt 660

tttggcagcc gctggacgtc cggtgttgat ggtggcagcg gcggcagcct aagcaacagc 720

agccctcgca gcccgccagc tcgcgctcgc cccgccggcg tccccagccc tatcacctca 780

tctcccgaaa ggtgctgggc agctccgggg cggtcgaggc gaagcggctg cagcggcggt 840

agcggcggcg ggaggcagga tgagcgcacg cggtgagggc gcggggcagc cgtccacttc 900

agcccaggga caacctgccg ccccagcgcc tcagaagaga ggacgcggcc gccccaggaa 960

gcagcagcaa gaaccaaccg gtgagccctc tcctaagaga cccaggggaa gacccaaagg 1020

cagcaaaaac aagagtccct ctaaagcagc tcaaaagaaa gcagaagcca ctggagaaaa 1080

acggccaaga ggcagaccta ggaaatggcc acaacaagtt gttcagaaga agcctgctca 1140

ggaggaaact gaagagacat cctcacaaga gtctgccgaa gaggactagg gggcgcaacg 1200

ttcgatttct acctcagcag cagttggatc ttttgaaggg agaagacact gcagtgacca 1260

cttattctgt attgccatgg tctttccact ttcatctggg gtggggtggg gtggggtggg 1320

ggaggggggg gtggggtggg gagaaatcac ataaccttaa aaaggactat attaatcacc 1380

ttctttgtaa tcccttcaca gtcccaggtt tagtgaaaaa ctgctgtaaa cacaggggac 1440

acagcttaac aatgcaactt ttaattactg ttttcttttt tcttaaccta ctaatagttt 1500

gttgatctga taagcaagag tgggcgggtg agaaaaaccg aattgggttt agtcaatcac 1560

tgcactgcat gcaaacaaga aacgtgtcac acttgtgacg tcgggcattc atataggaag 1620

aacgcggtgt gtaacactgt gtacacctca aataccaccc caacccactc cctgtagtga 1680

atcctctgtt tagaacacca aagataagga ctagatacta ctttctcttt ttcgtataat 1740

cttgtagaca cttacttgat gatttttaac tttttatttc taaatgagac gaaatgctga 1800

tgtatccttt cattcagcta acaaactaga aaaggttatg ttcatttttc aaaaagggaa 1860

gtaagcaaac aaatattgcc aactcttcta tttatggata tcacacatat cagcaggagt 1920

aataaattta ctcacagcac ttgttttcag gacaacactt cattttcagg aaatctactt 1980

cctacagagc caaaatgcca tttagcaata aataacactt gtcagcctca gagcatttaa 2040

ggaaactaga caagtaaaat tatcctcttt gtaatttaat gaaaaggtac aacagaataa 2100

tgcatgatga actcacctaa ttatgaggtg ggaggagcga aatctaaatt tcttttgcta 2160

tagttataca tcaatttaaa aagcaaaaaa aaaaaggggg gggcaatctc tctctgtgtc 2220

tttctctctc tctctccctc tccctctctc ttttcatgtg tatcagtttc catgaaagac 2280

ctgaatacca cttacctcaa attaagcata tgtgttactt caagtaatac gttttgacat 2340

aagatggttg accaaggtgc ttttcttcgg cttgagttca ccatctcttc attcaaactg 2400

cacttttagc cagagatgca atatatcccc actactcaat actacctctg aatgttacaa 2460

cgaatttaca gtctagtact tattacatgc tgctatacac aagcaatgca agaaaaaaac 2520

ttactgggta ggtgattcta atcatctgca gttctttttg tacacttaat tacagttaaa 2580

gaagcaatct ccttactgtg tttcagcatg actatgtatt tttctatgtt tttttaatta 2640

aaaattttta aaatacttgt ttcagcttct ctgctagatt tctacattaa cttgaaaatt 2700

ttttaaccaa gtcgctccta ggttcttaag gataattttc ctcaatcaca ctacacatca 2760

cacaagattt gactgtaata tttaaatatt accctccaag tctgtacctc aaatgaattc 2820

tttaaggaga tggactaatt gacttgcaaa gacctacctc cagacttcaa aaggaatgaa 2880

cttgttactt gcagcattca tttgtttttt caatgtttga aatagttcaa actgcagcta 2940

accctagtca aaactatttt tgtaaaagac atttgataga aaggaacacg tttttacata 3000

cttttgcaaa ataagtaaat aataaataaa ataaagccaa ccttcaaaga acttgaagct 3060

ttgtaggtga gatgcaacaa gccctgcttt tgcataatgc aatcaaaaat atgtgttttt 3120

aagattagtt gaatataaga aaatgcttga caaatatttt catgtatttt acacaaatgt 3180

gatttttgta atatgtctca accagattta ttttaaacgc ttcttatgta gagtttttat 3240

gcctttctct cctagtgagt gtgctgactt tttaacatgg tattatcaac tgggccagga 3300

ggtagtttct catgacggct tttgtcagta tggcttttag tactgaagcc aaatgaaact 3360

caaaaccatc tctcttccag ctgcttcagg gaggtagttt caaaggccac atacctctct 3420

gagactggca gatcgctcac tgttgtgaat caccaaagga gctatggaga gaattaaaac 3480

tcaacattac tgttaactgt gcgttaaata agcaaataaa cagtggctca taaaaataaa 3540

agtcgcattc catatctttg gatgggcctt ttagaaacct cattggccag ctcataaaat 3600

ggaagcaatt gctcatgttg gccaaacatg gtgcaccgag tgatttccat ctctggtaaa 3660

gttacacttt tatttcctgt atgttgtaca atcaaaacac actactacct cttaagtccc 3720

agtatacctc atttttcata ctgaaaaaaa aagcttgtgg ccaatggaac agtaagaaca 3780

tcataaaatt tttatatata tagtttattt ttgtgggaga taaattttat aggactgttc 3840

tttgctgttg ttggtcgcag ctacataaga ctggacattt aacttttcta ccatttctgc 3900

aagttaggta tgtttgcagg agaaaagtat caagacgttt aactgcagtt gactttctcc 3960

ctgttccttt gagtgtcttc taactttatt ctttgttctt tatgtagaat tgctgtctat 4020

gattgtactt tgaatcgctt gcttgttgaa aatatttctc tagtgtatta tcactgtctg 4080

ttctgcacaa taaacataac agcctctgtg a 4111

<210> 180
<211> 109
<212> PRT
<213> Homo sapiens

<400> 180
Met Ser Ala Arg Gly Glu Gly Ala Gly Gln Pro Ser Thr Ser Ala Gln
1               5                   10                  15

Gly Gln Pro Ala Ala Pro Ala Pro Gln Lys Arg Gly Arg Gly Arg Pro
            20                  25                  30

Arg Lys Gln Gln Gln Glu Pro Thr Gly Glu Pro Ser Pro Lys Arg Pro
        35                  40                  45

Arg Gly Arg Pro Lys Gly Ser Lys Asn Lys Ser Pro Ser Lys Ala Ala
    50                  55                  60

Gln Lys Lys Ala Glu Ala Thr Gly Glu Lys Arg Pro Arg Gly Arg Pro
65                  70                  75                  80

Arg Lys Trp Pro Gln Gln Val Val Gln Lys Lys Pro Ala Gln Glu Glu
                85                  90                  95

Thr Glu Glu Thr Ser Ser Gln Glu Ser Ala Glu Glu Asp
            100                 105

<210> 181
<211> 3252
<212> DNA
<213> Homo sapiens

<400> 181
gcggcccagg cccggaacct tccctggtcg tgcgccctat gtaaggccag ccgcggcagg 60
accaaggcgg cggtgtcagc tcgcgagcct accctccgcg gacggtcttg ggtcgcctgc 120
tgcctggctt gcctggtcgg cggcgggtgc cccgcgcgca cgcgcaaagc ccgccgcgtt 180
ccccgacccc aggccgcgct ctgtgggcct ctgagggcgg catgcgggac tacgacgagg 240
tgaccgcctt cctgggcgag tgggggccct tccagcgcct catcttcttc ctgctcagcg 300
ccagcatcat ccccaatggc ttcaccggcc tgtcctccgt gttcctgata gcgaccccgg 360
agcaccgctg ccgggtgccg gacgccgcga acctgagcag cgcctggcgc aaccacactg 420
tcccactgcg gctgcgggac ggccgcgagg tgccccacag ctgccgccgc taccggctcg 480
ccaccatcgc caacttctcg gcgctcgggc tggagccggg gcgcgacgtg gacctggggc 540
agctggagca ggagagctgt ctggatggct gggagttcag tcaggacgtc tacctgtcca 600
ccattgtgac cgagtggaac ctggtgtgtg aggacgactg gaaggcccca ctcacaatct 660
ccttgttctt cgtgggtgtg ctgttgggct ccttcatttc agggcagctg tcagacaggt 720
ttggccggaa gaatgtgctg ttcgtgacca tgggcatgca gacaggcttc agcttcctgc 780
agatcttctc gaagaatttt gagatgtttg tcgtgctgtt tgtccttgta ggcatgggcc 840
agatctccaa ctatgtggca gcatttgtcc tggggacaga aattcttggc aagtcagttc 900
gtataatatt ctctacgtta ggagtgtgca tattttatgc atttggctac atggtgctgc 960
cactgtttgc ttacttcatc cgagactggc ggatgctgct ggtggcgctg acgatgccgg 1020
gggtgctgtg cgtggcactc tggtggttca tccctgagtc cccccgatgg ctcatctctc 1080
agggacgatt tgaagaggca gaggtgatca tccgcaaggc tgccaaagcc aatgggattg 1140
ttgtgccttc cactatcttt gacccgagtg agttacaaga cctaagttcc aagaagcaac 1200
agtcccacaa cattctggat ctgcttcgaa cctggaatat ccggatggtc accatcatgt 1260
ccataatgct gtggatgacc atatcagtgg gctattttgg gctttcgctt gatactccta 1320
acttgcatgg ggacatcttt gtgaactgct tcctttcagc gatggttgaa gtcccagcat 1380
atgtgttggc ctggctgctg ctgcaatatt tgccccggcg ctattccatg gccactgccc 1440
tcttcctggg tggcagtgtc cttctcttca tgcagctggt acccccagac ttgtattatt 1500
tggctacagt cctggtgatg gtgggcaagt ttggagtcac ggctgccttt tccatggtct 1560
acgtgtacac agccgagctg tatcccacag tggtgagaaa catgggtgtg ggagtcagct 1620
ccacagcatc ccgcctgggc agcatcctgt ctccctactt cgtttacctt ggtgcctacg 1680
accgcttcct gccctacatt ctcatgggaa gtctgaccat cctgacagcc atcctcacct 1740
tgtttctccc agagagcttc ggtaccccac tcccagacac cattgaccag atgctaagag 1800

tcaaaggaat gaaacacaga aaaactccaa gtcacacaag gatgttaaaa gatggtcaag 1860

aaaggcccac aatccttaaa agcacagcct tctaacatcg cttccagtaa gggagaaact 1920

gaagaggaaa gactgtcttg ctagaaatgg ccagcttgtg cagactccga gtccttcagt 1980

gacaaaggcc tttgctgttt gtcctcttga cctgtgtctg acttgctcct ggatgggcac 2040

ccacactcag aggctacata tggccctaga gcaccacctt cctctaggga cactggggct 2100

acctacagac aacttcatct aagtcctaac tattacaatg atggactcag cacctccaaa 2160

gcagttaatt tttcactaga accagtgaga tctggaggaa tgtgagaagc atatgctaaa 2220

tgtacatttt aattttagac tacttgaaaa ggcccctaat aaggctagag gtctaagtcc 2280

cccacccctt tccccactcc cctctagtgg tgaactttag aggaaaagga agtaattgca 2340

caaggagttt gattcttacc ttttctcagt tacagaggac attaactgga tcattgcttc 2400

cccagggcag gagagcgcag agctagggaa agtgaaaggt aatgaagatg gagcagaatg 2460

agcagatgca gatcaccagc aaagtgcact gatgtgtgag ctcttaagac cactcagcat 2520

gacgactgag tagacttgtt tacatctgat caaagcactg ggcttgtcca ggctcataat 2580

aaatgctcca ttgaatctac tattcttgtt ttccactgct gtggaaacct ccttgctact 2640

atagcgtctt atgtatggtt taaaggaaat ttatcaggtg agagagatga gcaacgttgt 2700

cttttctctc aaagctgtaa tgtgggtttt gttttactgt ttatttgttt gttgttgtat 2760

ccttttctcc ttgttatttg cccttcagaa tgcacttggg aaaggctggt tccttagcct 2820

cctggtttgt gtcttttttt tttttttttt aaacacagaa tcactctggc aattgtctgc 2880

agctgccact ggtgcaaggc cttaccagcc ctagcctcta gcacttctct aagtgccaaa 2940

aacagtgtca ttgtgtgtgt tcctttcttg atacttagtc atgggaggat attacaaaaa 3000

agaaatttaa attgtgttca tagtctttca gagtagctca ctttagtcct gtaactttat 3060

tgggtgatat tttgtgttca gtgtaattgt cttctctttg ctgattatgt taccatggta 3120

ctcctaaagc atatgcctca cctggttaaa aaagaacaaa catgtttttg tgaaagctac 3180

tgaagtgcct tgggaaatga gaaagtttta ataagtaaaa tgatttttta aatatcaaaa 3240

aaaaaaaaaa aa 3252

<210> 182
<211> 557
<212> PRT
<213> Homo sapiens

<400> 182

<210> 183
<211> 2135
<212> DNA
<213> Homo sapiens

<400> 183
ccccggcttc gcgccccaat ttctaacagc ctgcctgtcc cccgggaacg ttctaacatc      60

cttggggagc gccccagcta caagacactg tcctgagaac gctgtcatca cccgtagttg     120

caagtttcgg agcggcagtg ggaagcatgc gggactacga cgaggtgatc gccttcctgg     180

gcgagtgggg gcccttccag cgcctcatct tcttcctgct cagcgccagc atcatcccca     240

atggcttcaa tggtatgtca gtcgtgttcc tggcggggac cccggagcac cgctgtcgag     300

tgccggacgc cgcgaacctg agcagcgcct ggcgcaacaa cagtgtcccg ctgcggctgc     360

gggacggccg cgaggtgccc cacagctgca gccgctaccg gctcgccacc atcgccaact     420

tctcggcgct cgggctggag ccggggcgcg acgtggacct ggggcagctg gagcaggaga     480

gctgcctgga tggctgggag ttcagccagg acgtctacct gtccaccgtc gtgaccgagt     540

ggaatctggt gtgtgaggac aactggaagg tgcccctcac cacctccctg ttcttcgtag     600

gcgtgctcct cggctccttc gtgtccgggc agctgtcaga caggtttggc aggaagaacg     660

ttctcttcgc aaccatggct gtacagactg gcttcagctt cctgcagatt ttctccatca     720

gctgggagat gttcactgtg ttatttgtca tcgtgggcat gggccagatc tccaactatg     780

tggtagcctt catactagga acagaaattc ttggcaagtc agttcgtatt atattctcta     840

cattaggagt gtgcacattt tttgcagttg gctatatgct gctgccactg tttgcttact     900

tcatcagaga ctggcggatg ctgctgctgg cgctgacggt gccgggagtg ctgtgtgtcc     960

cgctgtggtg gttcattcct gaatctcccc gatggctgat atcccagaga agatttagag    1020

aggctgaaga tatcatccaa aaagctgcaa aaatgaacaa cacagctgta ccagcagtga    1080

tatttgattc tgtggaggag ctaaatcccc tgaagcagca gaaagctttc attctggacc    1140

tgttcaggac tcggaatatt gccataatga ccattatgtc tttgctgcta tggatgctga    1200

cctcagtggg ttactttgct ctgtctctgg atgctcctaa tttacatgga gatgcctacc 1260

tgaactgttt cctctctgcc ttgattgaaa ttccagctta cattacagcc tggctgctat 1320

tgcgaacgct gcccaggcgt tatatcatag ctgcagtact gttctgggga ggaggtgtgc 1380

ttctcttcat tcaactggta cctgtggatt attacttctt atccattggt ctggtcatgc 1440

tgggaaaatt tgggatcacc tctgctttct ccatgctgta tgtcttcact gctgagctct 1500

acccaaccct ggtcaggaac atggcggtgg gggtcacatc cacggcctcc agagtgggca 1560

gcatcattgc cccctacttt gtttacctcg gtgcttacaa cagaatgctg ccctacatcg 1620

tcatgggtag tctgactgtc ctgattggaa tcttcaccct ttttttccct gaaagtttgg 1680

gaatgactct tccagaaacc ttagagcaga tgcagaaagt gaaatggttc agatctggga 1740

aaaaaacaag agactcaatg gagacagaag aaaatcccaa ggttctaata actgcattct 1800

gaaaaaatat ctaccccatt tggtgaagtg aaaaacagaa aaataagacc ctgtggagaa 1860

attcgttgtt cccactgaaa tggactgact gtaacgattg acaccaaaat gaaccttgct 1920

atcaagaaat gctcgtcata cagtaaactc tggatgattc ttccagataa tgtccttgct 1980

ttacaaacca accatttcta gagagtctcc ttactcatta attcaatgaa atggattggt 2040

aagatgtctt gaaaacatgt tagtcaagga ctggtaaaat acatataaag attaacactc 2100

atttccaatc atacaaatac tatccaaata aaaat 2135

<210> 184
<211> 551
<212> PRT
<213> Homo sapiens

<400> 184
Met Arg Asp Tyr Asp Glu Val Ile Ala Phe Leu Gly Glu Trp Gly Pro
1               5                   10                  15

Phe Gln Arg Leu Ile Phe Phe Leu Leu Ser Ala Ser Ile Ile Pro Asn
            20                  25                  30

Gly Phe Asn Gly Met Ser Val Val Phe Leu Ala Gly Thr Pro Glu His
        35                  40                  45

Arg Cys Arg Val Pro Asp Ala Ala Asn Leu Ser Ser Ala Trp Arg Asn
    50                  55                  60

Asn Ser Val Pro Leu Arg Leu Arg Asp Gly Arg Glu Val Pro His Ser
65                  70                  75                  80

Cys Ser Arg Tyr Arg Leu Ala Thr Ile Ala Asn Phe Ser Ala Leu Gly
                85                  90                  95

Leu Glu Pro Gly Arg Asp Val Asp Leu Gly Gln Leu Glu Gln Glu Ser
            100                 105                 110

Cys Leu Asp Gly Trp Glu Phe Ser Gln Asp Val Tyr Leu Ser Thr Val
        115                 120                 125

Val Thr Glu Trp Asn Leu Val Cys Glu Asp Asn Trp Lys Val Pro Leu
    130                 135                 140

Thr Thr Ser Leu Phe Phe Val Gly Val Leu Leu Gly Ser Phe Val Ser
145                 150                 155                 160

Gly Gln Leu Ser Asp Arg Phe Gly Arg Lys Asn Val Leu Phe Ala Thr
                165                 170                 175

Met Ala Val Gln Thr Gly Phe Ser Phe Leu Gln Ile Phe Ser Ile Ser
            180                 185                 190

Trp Glu Met Phe Thr Val Leu Phe Val Ile Val Gly Met Gly Gln Ile
        195                 200                 205

Ser Asn Tyr Val Val Ala Phe Ile Leu Gly Thr Glu Ile Leu Gly Lys
    210                 215                 220

Ser Val Arg Ile Ile Phe Ser Thr Leu Gly Val Cys Thr Phe Phe Ala
225                 230                 235                 240

Val Gly Tyr Met Leu Leu Pro Leu Phe Ala Tyr Phe Ile Arg Asp Trp
                245                 250                 255

Arg Met Leu Leu Leu Ala Leu Thr Val Pro Gly Val Leu Cys Val Pro
            260                 265                 270

Leu Trp Trp Phe Ile Pro Glu Ser Pro Arg Trp Leu Ile Ser Gln Arg
        275                 280                 285

Arg Phe Arg Glu Ala Glu Asp Ile Ile Gln Lys Ala Ala Lys Met Asn
    290                 295                 300

Asn Thr Ala Val Pro Ala Val Ile Phe Asp Ser Val Glu Glu Leu Asn
305                 310                 315                 320

Pro Leu Lys Gln Gln Lys Ala Phe Ile Leu Asp Leu Phe Arg Thr Arg
                325                 330                 335

Asn Ile Ala Ile Met Thr Ile Met Ser Leu Leu Leu Trp Met Leu Thr
            340                 345                 350

Ser Val Gly Tyr Phe Ala Leu Ser Leu Asp Ala Pro Asn Leu His Gly
        355                 360                 365

Asp Ala Tyr Leu Asn Cys Phe Leu Ser Ala Leu Ile Glu Ile Pro Ala
    370                 375                 380

Tyr Ile Thr Ala Trp Leu Leu Leu Arg Thr Leu Pro Arg Arg Tyr Ile
385                 390                 395                 400

Ile Ala Ala Val Leu Phe Trp Gly Gly Gly Val Leu Leu Phe Ile Gln
                405                 410                 415

Leu Val Pro Val Asp Tyr Tyr Phe Leu Ser Ile Gly Leu Val Met Leu
            420                 425                 430

Gly Lys Phe Gly Ile Thr Ser Ala Phe Ser Met Leu Tyr Val Phe Thr
        435                 440                 445

Ala Glu Leu Tyr Pro Thr Leu Val Arg Asn Met Ala Val Gly Val Thr
    450                 455                 460

Ser Thr Ala Ser Arg Val Gly Ser Ile Ile Ala Pro Tyr Phe Val Tyr
465                 470                 475                 480

Leu Gly Ala Tyr Asn Arg Met Leu Pro Tyr Ile Val Met Gly Ser Leu
                485                 490                 495

Thr Val Leu Ile Gly Ile Phe Thr Leu Phe Phe Pro Glu Ser Leu Gly
            500                 505                 510

Met Thr Leu Pro Glu Thr Leu Glu Gln Met Gln Lys Val Lys Trp Phe
        515                 520                 525

Arg Ser Gly Lys Lys Thr Arg Asp Ser Met Glu Thr Glu Glu Asn Pro
    530                 535                 540

Lys Val Leu Ile Thr Ala Phe
545                 550

<210> 185
<211> 2168
<212> DNA
<213> Homo sapiens

<400> 185
gcgagtgtcc agctgcggag acccgtgata attcgttaac taattcaaca aacgggaccc 60
ttctgtgtgc cagaaaccgc aagcagttgc taacccagtg ggacaggcgg attggaagag 120
cgggaaggtc ctggcccaga gcagtgtgac acttccctct gtgaccatga aactctgggt 180
gtctgcattg ctgatggcct ggtttggtgt cctgagctgt gtgcaggccg aattcttcac 240
ctctattggg cacatgactg acctgattta tgcagagaaa gagctggtgc agtctctgaa 300
agagtacatc cttgtggagg aagccaagct ttccaagatt aagagctggg ccaacaaaat 360
ggaagccttg actagcaagt cagctgctga tgctgagggc tacctggctc accctgtgaa 420
tgcctacaaa ctggtgaagc ggctaaacac agactggcct gcgctggagg accttgtcct 480
gcaggactca gctgcaggtt ttatcgccaa cctctctgtg cagcggcagt tcttccccac 540
tgatgaggac gagataggag ctgccaaagc cctgatgaga cttcaggaca catacaggct 600
ggacccaggc acaatttcca gaggggaact tccaggaacc aagtaccagg caatgctgag 660
tgtggatgac tgctttggga tgggccgctc ggcctacaat gaaggggact attatcatac 720
ggtgttgtgg atggagcagg tgctaaagca gcttgatgcc ggggaggagg ccaccacaac 780
caagtcacag gtgctggact acctcagcta tgctgtcttc cagttgggtg atctgcaccg 840
tgccctggag ctcacccgcc gcctgctctc ccttgaccca agccacgaac gagctggagg 900
gaatctgcgg tactttgagc agttattgga ggaagagaga gaaaaaacgt taacaaatca 960
gacagaagct gagctagcaa ccccagaagg catctatgag aggcctgtgg actacctgcc 1020
tgagagggat gtttacgaga gcctctgtcg tggggagggt gtcaaactga caccccgtag 1080
acagaagagg cttttctgta ggtaccacca tggcaacagg gccccacagc tgctcattgc 1140
ccccttcaaa gaggaggacg agtgggacag cccgcacatc gtcaggtact acgatgtcat 1200
gtctgatgag gaaatcgaga ggatcaagga gatcgcaaaa cctaaacttg cacgagccac 1260
cgttcgtgat cccaagacag gagtcctcac tgtcgccagc taccgggttt ccaaaagctc 1320
ctggctagag gaagatgatg accctgttgt ggcccgagta aatcgtcgga tgcagcatat 1380
cacagggtta acagtaaaga ctgcagaatt gttacaggtt gcaaattatg gagtgggagg 1440
acagtatgaa ccgcacttcg acttctctag gcgacctttt gacagcggcc tcaaaacaga 1500
ggggaatagg ttagcgacgt ttcttaacta catgagtgat gtagaagctg gtggtgccac 1560
cgtcttccct gatctggggg ctgcaatttg gcctaagaag ggtacagctg tgttctggta 1620
caacctcttg cggagcgggg aaggtgacta ccgaacaaga catgctgcct gccctgtgct 1680
tgtgggctgc aagtgggtct ccaataagtg gttccatgaa cgaggacagg agttcttgag 1740
accttgtgga tcaacagaag ttgactgaca tccttttctg tccttcccct tcctggtcct 1800

tcagcccatg tcaacgtgac agacaccttt gtatgttcct ttgtatgttc ctatcaggct 1860

gatttttgga gaaatgaatg tttgtctgga gcagagggag accatactag ggcgactcct 1920

gtgtgactga agtcccagcc cttccattca gcctgtgcca tccctggccc caaggctagg 1980

atcaaagtgg ctgcagcaga gttagctgtc tagcgcctag caaggtgcct ttgtacctca 2040

ggtgttttag gtgtgagatg tttcagtgaa ccaaagttct gataccttgt ttacatgttt 2100

gtttttatgg catttctatc tattgtggct ttaccaaaaa ataaaatgtc cctaccagaa 2160

aaaaaaaa 2168

<210> 186
<211> 533
<212> PRT
<213> Homo sapiens

<400> 186
Met Lys Leu Trp Val Ser Ala Leu Leu Met Ala Trp Phe Gly Val Leu
1               5                   10                  15

Ser Cys Val Gln Ala Glu Phe Phe Thr Ser Ile Gly His Met Thr Asp
            20                  25                  30

Leu Ile Tyr Ala Glu Lys Glu Leu Val Gln Ser Leu Lys Glu Tyr Ile
        35                  40                  45

Leu Val Glu Glu Ala Lys Leu Ser Lys Ile Lys Ser Trp Ala Asn Lys
    50                  55                  60

Met Glu Ala Leu Thr Ser Lys Ser Ala Ala Asp Ala Glu Gly Tyr Leu
65                  70                  75                  80

Ala His Pro Val Asn Ala Tyr Lys Leu Val Lys Arg Leu Asn Thr Asp
                85                  90                  95

Trp Pro Ala Leu Glu Asp Leu Val Leu Gln Asp Ser Ala Ala Gly Phe
            100                 105                 110

Ile Ala Asn Leu Ser Val Gln Arg Gln Phe Phe Pro Thr Asp Glu Asp
        115                 120                 125

Glu Ile Gly Ala Ala Lys Ala Leu Met Arg Leu Gln Asp Thr Tyr Arg
    130                 135                 140

Leu Asp Pro Gly Thr Ile Ser Arg Gly Glu Leu Pro Gly Thr Lys Tyr
145                 150                 155                 160

Gln Ala Met Leu Ser Val Asp Asp Cys Phe Gly Met Gly Arg Ser Ala
                165                 170                 175

Tyr Asn Glu Gly Asp Tyr Tyr His Thr Val Leu Trp Met Glu Gln Val
            180                 185                 190

Leu Lys Gln Leu Asp Ala Gly Glu Glu Ala Thr Thr Thr Lys Ser Gln
        195                 200                 205

Val Leu Asp Tyr Leu Ser Tyr Ala Val Phe Gln Leu Gly Asp Leu His
    210                 215                 220

Arg Ala Leu Glu Leu Thr Arg Arg Leu Leu Ser Leu Asp Pro Ser His
225                 230                 235                 240

Glu Arg Ala Gly Gly Asn Leu Arg Tyr Phe Glu Gln Leu Leu Glu Glu
                245                 250                 255

Glu Arg Glu Lys Thr Leu Thr Asn Gln Thr Glu Ala Glu Leu Ala Thr
            260                 265                 270

Pro Glu Gly Ile Tyr Glu Arg Pro Val Asp Tyr Leu Pro Glu Arg Asp
        275                 280                 285

Val Tyr Glu Ser Leu Cys Arg Gly Glu Gly Val Lys Leu Thr Pro Arg
    290                 295                 300

Arg Gln Lys Arg Leu Phe Cys Arg Tyr His His Gly Asn Arg Ala Pro
305                 310                 315                 320

Gln Leu Leu Ile Ala Pro Phe Lys Glu Glu Asp Glu Trp Asp Ser Pro
                325                 330                 335

His Ile Val Arg Tyr Tyr Asp Val Met Ser Asp Glu Glu Ile Glu Arg
            340                 345                 350

Ile Lys Glu Ile Ala Lys Pro Lys Leu Ala Arg Ala Thr Val Arg Asp
        355                 360                 365

Pro Lys Thr Gly Val Leu Thr Val Ala Ser Tyr Arg Val Ser Lys Ser
    370                 375                 380

Ser Trp Leu Glu Glu Asp Asp Asp Pro Val Val Ala Arg Val Asn Arg
385                 390                 395                 400

Arg Met Gln His Ile Thr Gly Leu Thr Val Lys Thr Ala Glu Leu Leu
                405                 410                 415

Gln Val Ala Asn Tyr Gly Val Gly Gly Gln Tyr Glu Pro His Phe Asp
            420                 425                 430

Phe Ser Arg Arg Pro Phe Asp Ser Gly Leu Lys Thr Glu Gly Asn Arg
        435                 440                 445

Leu Ala Thr Phe Leu Asn Tyr Met Ser Asp Val Glu Ala Gly Gly Ala
    450                 455                 460

Thr Val Phe Pro Asp Leu Gly Ala Ala Ile Trp Pro Lys Lys Gly Thr
465                 470                 475                 480

Ala Val Phe Trp Tyr Asn Leu Leu Arg Ser Gly Glu Gly Asp Tyr Arg
                485                 490                 495

Thr Arg His Ala Ala Cys Pro Val Leu Val Gly Cys Lys Trp Val Ser
            500                 505                 510

Asn Lys Trp Phe His Glu Arg Gly Gln Glu Phe Leu Arg Pro Cys Gly
        515                 520                 525

Ser Thr Glu Val Asp
    530

<210> 187
<211> 735
<212> DNA
<213> Homo sapiens

<400> 187
gcagccagaa agctctggag catcagggag actccaactt aaggcaacag catgggtgaa      60

taagggcttc ctgtggactg gcaatgagag gcaaaacctg gtgcttgagc actggcccct     120

aaggcaggcc ttacagatct cttacactcg tggtgggaag agtttagtgt gaaactgggg     180

tggaattggg tgtccacgta tgttcccttt tgccttacta tatgttctgt cagtttcttt     240

caggaaaatc ttcatcttac aacttgtagg gctggtgtta acttacgact tcactaactg     300

tgactttgag aagattaaag cagcctatct cagtactatt tctaaagacc tgattacata     360

tatgagtggg accaaaagta ccgagttcaa caacaccgtc tcttgtagca atcggccaca     420

ttgccttact gaaatccaga gcctaacctt caatcccacc gccggctgcg cgtcgctcgc     480

caaagaaatg ttcgccatga aaactaaggc tgccttagct atctggtgcc caggctattc     540

ggaaactcag ataaatgcta ctcaggcaat gaagaagagg agaaaaagga aagtcacaac     600

caataaatgt ctggaacaag tgtcacaatt acaaggattg tggcgtcgct tcaatcgacc 660

tttactgaaa caacagtaaa ccatctttat tatggtcata tttcacagca ccaaaataaa 720

tcatctttat taagt 735

<210> 188
<211> 159
<212> PRT
<213> Homo sapiens

<400> 188
Met Phe Pro Phe Ala Leu Leu Tyr Val Leu Ser Val Ser Phe Arg Lys
1               5                   10                  15

Ile Phe Ile Leu Gln Leu Val Gly Leu Val Leu Thr Tyr Asp Phe Thr
            20                  25                  30

Asn Cys Asp Phe Glu Lys Ile Lys Ala Ala Tyr Leu Ser Thr Ile Ser
        35                  40                  45

Lys Asp Leu Ile Thr Tyr Met Ser Gly Thr Lys Ser Thr Glu Phe Asn
    50                  55                  60

Asn Thr Val Ser Cys Ser Asn Arg Pro His Cys Leu Thr Glu Ile Gln
65                  70                  75                  80

Ser Leu Thr Phe Asn Pro Thr Ala Gly Cys Ala Ser Leu Ala Lys Glu
                85                  90                  95

Met Phe Ala Met Lys Thr Lys Ala Ala Leu Ala Ile Trp Cys Pro Gly
            100                 105                 110

Tyr Ser Glu Thr Gln Ile Asn Ala Thr Gln Ala Met Lys Lys Arg Arg
        115                 120                 125

Lys Arg Lys Val Thr Thr Asn Lys Cys Leu Glu Gln Val Ser Gln Leu
    130                 135                 140

Gln Gly Leu Trp Arg Arg Phe Asn Arg Pro Leu Leu Lys Gln Gln
145                 150                 155

<210> 189
<211> 1677
<212> DNA
<213> Homo sapiens

<400> 189
aggttctctt acatcgaccg cctaagagtc gcgctgtaag aagcaacaac ctctcctctt 60

cgtctccgcc atcagctcgg cagtcgcgaa gcagcaacca tgcgtgagtg catctccatc    120

cacgttggcc aggctggtgt ccagattggc aatgcctgct gggagctcta ctgcctggaa    180

cacggcatcc agcccgatgg ccagatgcca agtgacaaga ccattggggg aggagatgat    240

tccttcaaca ccttcttcag tgagacgggg gctggcaagc atgtgccccg ggcagtgttt    300

gtagacttgg aacccacagt cattgatgaa gttcgcactg gcacctaccg ccagctcttc    360

caccctgagc aacttatcac aggcaaagaa gatgctgcca ataactatgc ccgagggcac    420

tacaccattg gcaaggagat cattgacctc gtgttggacc gaattcgcaa gctggccgac    480

cagtgcacgg gtctccaggg cttcttggtt ttccacagct ttggtggggg aactggttct    540

gggttcacct cgctgctcat ggaacgtctc tcagttgatt atggcaagaa gtccaagctg    600

gagttctcta tttacccggc gccccaggtt tccacagctg tagttgagcc ctacaactcc    660

atcctcacca cccacaccac cctggagcac tctgattgtg ccttcatggt agacaatgag    720

gccatctatg acatctgtcg tagaaacctc gatattgagc gtccaaccta tactaacctg    780

aataggttaa taggtcaaat tgtgtcctcc atcactgctt ccctgagatt tgatggagcc    840

ctgaatgttg acctgacaga attccagacc aacctggtgc cctatccccg catccacttc    900

cctctggcca catatgcccc tgtcatctct gctgagaaag cctaccatga acagctttct    960

gtagcagaga tcaccaatgc ttgctttgag ccagccaacc agatggtgaa atgtgaccct   1020

cgccatggta aatacatggc ttgctgcctg ttgtaccgtg gtgacgtggt tcccaaagat   1080

gtcaatgctg ccattgccac catcaagacc aagcgtacca tccagtttgt ggattggtgc   1140

cccactggct tcaaggttgg catcaactac cagcctccca ctgtggtgcc tggtggagac   1200

ctggccaagg tacagagagc tgtgtgcatg ctgagcaaca ccacagccat tgctgaggcc   1260

tgggctcgcc tggaccacaa gtttgacctg atgtatgcca aacgtgcctt tgttcactgg   1320

tacgttgggg aggggatgga ggaaggtgag ttttcagagg cccgtgagga catggctgcc   1380

cttgagaagg attatgagga ggttggtgtg gattctgttg aaggagaggg tgaggaagaa   1440

ggagaggaat actaaagtta aaacgtcaca aaggtgctgc ttttacaggg aagcttattc   1500

tgttttaaac attgaaaagt tgtggtctga tcagttaatt tgtatgtagc agtgtatgct   1560

ctcatataca attactgacc tatgctctaa aacatgaatg ctttgttaca gacccaagct   1620

gtccatttct gtgatgggtt ttgaataaag tattccctgt cttaaaaaaa aaaaaaa      1677

<210> 190
<211> 451
<212> PRT
<213> Homo sapiens

<400> 190

<210> 191
<211> 1558
<212> DNA
<213> Homo sapiens

<400> 191
cttggtagtc tgttagtggg agatccttgt tgccgtccct tcgcctcctt caccgccgca      60

gaccccttca agttctagtc atgcgtgagt gcatctccat ccacgttggc caggctggtg     120

tccagattgg caatgcctgc tgggagctct actgcctgga acacggcatc cagcccgatg     180

gccagatgcc aagtgacaag accattgggg gaggagatga ttccttcaac accttcttca     240

gtgaaacggg tgctggcaag catgtgcccc gggcagtgtt tgtagacttg gaacccacag     300

tcattgatga agttcgcact ggcacctacc gccagctctt ccaccctgag cagctcatca     360

caggcaagga agatgctgcc aataactatg cccgagggca ctacaccatt ggcaaggaga     420

tcattgacct cgtgttggac cgaattcgca agctggctga ccagtgcacc ggtcttcagg     480

gcttcttggt tttccacagc tttggtgggg gaactggttc tgggttcacc tcgctgctca     540

tggaacgtct ctcagttgat tatggcaaga agtccaagct ggagttctct atttacccgg     600

cgccccaggt ttccacagct gtagttgagc cctacaactc catcctcacc acccacacca     660

ccctggagca ctctgattgt gccttcatgg tagacaatga ggccatctat gacatctgtc     720

gtagaaacct cgatatcgag cgcccaacct acactaacct taaccgcctt attagccaga     780

ttgtgtcctc catcactgct tccctgagat ttgatggagc cctgaatgtt gacctgacag     840

aattccagac caacctggtg ccctaccccc gcatccactt ccctctggcc acatatgccc     900

ctgtcatctc tgctgagaaa gcctaccatg aacagcttac tgtagcagag atcaccaatg     960

cttgctttga gccagccaac cagatggtga aatgtgaccc tcgccatggt aaatacatgg    1020

cttgctgcct gttataccgt ggtgacgtgg ttcccaaaga tgtcaatgct gccattgcca    1080

ccatcaaaac caagcgtacc atccagtttg tggattggtg ccccactggc ttcaaggttg    1140

gcattaatta ccagcctccc actgtggtgc ctggcggaga cctggccaag gtacagagag    1200

ctgtgtgcat gctgagcaat accacagctg ttgctgaggc ctgggctcgc ctggaccaca    1260

agtttgacct gatgtatgcc aagcgtgcct ttgttcactg gtacgtgggt gaggggatgg    1320

aggaaggcga gttttcagag gcccgtgagg acatggctgc ccttgagaag gattatgagg    1380

aggttggagc agatagtgct gacggagagg atgagggtga agagtattaa cctgtgtgct    1440

gtacttttac actcctttgt cttggaactg tcttattttt gttctgtaaa tgtctattgc    1500

cgtaaattgt taataaaatt gatgtttcca ttttaaaaaa aaaaaaaaaa aaaaaaaa      1558


<210> 192
<211> 449
<212> PRT
<213> Homo sapiens

<400> 192
Met Arg Glu Cys Ile Ser Ile His Val Gly Gln Ala Gly Val Gln Ile

1               5                   10                  15

Gly Asn Ala Cys Trp Glu Leu Tyr Cys Leu Glu His Gly Ile Gln Pro
            20                  25                  30

Asp Gly Gln Met Pro Ser Asp Lys Thr Ile Gly Gly Gly Asp Asp Ser
        35                  40                  45

Phe Asn Thr Phe Phe Ser Glu Thr Gly Ala Gly Lys His Val Pro Arg
    50                  55                  60

Ala Val Phe Val Asp Leu Glu Pro Thr Val Ile Asp Glu Val Arg Thr
65                  70                  75                  80

Gly Thr Tyr Arg Gln Leu Phe His Pro Glu Gln Leu Ile Thr Gly Lys
                85                  90                  95

Glu Asp Ala Ala Asn Asn Tyr Ala Arg Gly His Tyr Thr Ile Gly Lys
            100                 105                 110

Glu Ile Ile Asp Leu Val Leu Asp Arg Ile Arg Lys Leu Ala Asp Gln
        115                 120                 125

Cys Thr Gly Leu Gln Gly Phe Leu Val Phe His Ser Phe Gly Gly Gly
    130                 135                 140

Thr Gly Ser Gly Phe Thr Ser Leu Leu Met Glu Arg Leu Ser Val Asp
145                 150                 155                 160

Tyr Gly Lys Lys Ser Lys Leu Glu Phe Ser Ile Tyr Pro Ala Pro Gln
                165                 170                 175

Val Ser Thr Ala Val Val Glu Pro Tyr Asn Ser Ile Leu Thr Thr His
            180                 185                 190

Thr Thr Leu Glu His Ser Asp Cys Ala Phe Met Val Asp Asn Glu Ala
        195                 200                 205

Ile Tyr Asp Ile Cys Arg Arg Asn Leu Asp Ile Glu Arg Pro Thr Tyr
    210                 215                 220

Thr Asn Leu Asn Arg Leu Ile Ser Gln Ile Val Ser Ser Ile Thr Ala
225                 230                 235                 240

Ser Leu Arg Phe Asp Gly Ala Leu Asn Val Asp Leu Thr Glu Phe Gln
                245                 250                 255

Thr Asn Leu Val Pro Tyr Pro Arg Ile His Phe Pro Leu Ala Thr Tyr
            260                 265                 270

Ala Pro Val Ile Ser Ala Glu Lys Ala Tyr His Glu Gln Leu Thr Val
        275                 280                 285

Ala Glu Ile Thr Asn Ala Cys Phe Glu Pro Ala Asn Gln Met Val Lys
    290                 295                 300

Cys Asp Pro Arg His Gly Lys Tyr Met Ala Cys Cys Leu Leu Tyr Arg
305                 310                 315                 320

Gly Asp Val Val Pro Lys Asp Val Asn Ala Ala Ile Ala Thr Ile Lys
                325                 330                 335

Thr Lys Arg Thr Ile Gln Phe Val Asp Trp Cys Pro Thr Gly Phe Lys
            340                 345                 350

Val Gly Ile Asn Tyr Gln Pro Pro Thr Val Val Pro Gly Gly Asp Leu
        355                 360                 365

Ala Lys Val Gln Arg Ala Val Cys Met Leu Ser Asn Thr Thr Ala Val
    370                 375                 380

Ala Glu Ala Trp Ala Arg Leu Asp His Lys Phe Asp Leu Met Tyr Ala
385                 390                 395                 400

Lys Arg Ala Phe Val His Trp Tyr Val Gly Glu Gly Met Glu Glu Gly
                405                 410                 415

Glu Phe Ser Glu Ala Arg Glu Asp Met Ala Ala Leu Glu Lys Asp Tyr
            420                 425                 430

Glu Glu Val Gly Ala Asp Ser Ala Asp Gly Glu Asp Glu Gly Glu Glu
        435                 440                 445

Tyr

<210> 193
<211> 1143
<212> DNA
<213> Homo sapiens

<400> 193
gaatgtctct ctacccccca accccccatg tctgtggtga agtcgatcga attagtgctg      60

cccgaggata gaatctacct ggctggctcc agcataaaag ggcaggtgat cttaaccctg 120

aacagcaccc tggtggaccc catagtgaag gtggagctcg tgggaagggg ttacgtcgaa 180

tggagtgaag aagccggggc atcctgtgat tatagcagaa atgttatttg caacaacaag 240

gcagactacg tgcataagac aaagacattc ccagtggagg ataattggtt aagtgcaggc 300

agccacacct ttgacttcca tttcaactta cctcccaggc ttccttctac cttcaccagc 360

aaatttggcc atgtcttcta tttcgtacaa gcttcctgca tgggcaggga acacatttta 420

gccaagaaga ggatgtactt attggttcaa ggaacttcca ccttccacaa agaaacccca 480

ttccaggtac cgatggggga aaggtggagg tgggaacaat ctccaagctc caaggaggaa 540

gagggaagga gaggaggggg agctccagct gctcctgaaa aggctgaaga gaagccagag 600

gcagaaaagt gggagaaccc cttgttcgtg gaggctgagg agaaagtctc ctacaactgc 660

tgccgccagg gcactgtctg tttgcaaatc cagatggaaa ggaacacctt cacgccagga 720

gagaaggtcg tcttcacaac agagatcaac aaccagacca gcaaatgcat caagacggtc 780

gtattcgccc tgtatgccca catacagtac gagggcttca cgcccagtgc agagcggcgg 840

tctcggctgg acagcagcga gcttctgagg caggaggcca acacccccgt gacccgcttc 900

aacaccacca aggttgtcag caccttcaac ctgccgttgc tgctgtccgt gagcagcagc 960

acgcaggacg gtgagatcat gcacactcgc tacgagctgg tcaccaccgt gcacctgccc 1020

tggtccctga ccagcctcaa ggccaaagtt cccatcatca tcaccagcgc ctcagtggac 1080

tctgccatct gccagctgtc agaggacgga gtgttacccg tgaacccaga tcaccagaat 1140

taa 1143

<210> 194
<211> 342
<212> PRT
<213> Homo sapiens

<400> 194
Met Gly Asp Arg Glu Glu Cys Leu Ser Thr Pro Gln Pro Pro Met Ser
1               5                   10                  15

Val Val Lys Ser Ile Glu Leu Val Leu Pro Glu Asp Arg Ile Tyr Leu
            20                  25                  30

Ala Gly Ser Ser Ile Lys Gly Gln Val Ile Leu Thr Leu Asn Ser Thr
        35                  40                  45

Leu Val Asp Pro Ile Val Lys Val Glu Leu Val Gly Arg Gly Tyr Val
    50                  55                  60

Glu Trp Ser Glu Glu Ala Gly Ala Ser Cys Asp Tyr Ser Arg Asn Val
65                  70                  75                  80

Ile Cys Asn Asn Lys Ala Asp Tyr Val His Lys Thr Lys Thr Phe Pro
                85                  90                  95

Val Glu Asp Asn Trp Leu Ser Ala Gly Ser His Thr Phe Asp Phe His
            100                 105                 110

Phe Asn Leu Pro Pro Arg Leu Pro Ser Thr Phe Thr Ser Lys Phe Gly
        115                 120                 125

His Val Phe Tyr Phe Val Gln Ala Ser Cys Met Gly Arg Glu His Ile
    130                 135                 140

Leu Ala Lys Lys Arg Met Tyr Leu Leu Val Gln Gly Thr Ser Thr Phe
145                 150                 155                 160

His Lys Glu Thr Pro Phe Gln Asn Pro Leu Phe Val Glu Ala Glu Glu
                165                 170                 175

Lys Val Ser Tyr Asn Cys Cys Arg Gln Gly Thr Val Cys Leu Gln Ile
            180                 185                 190

Gln Met Glu Arg Asn Thr Phe Thr Pro Gly Glu Lys Val Val Phe Thr
        195                 200                 205

Thr Glu Ile Asn Asn Gln Thr Ser Lys Cys Ile Lys Thr Val Val Phe
    210                 215                 220

Ala Leu Tyr Ala His Ile Gln Tyr Glu Gly Phe Thr Pro Ser Ala Glu
225                 230                 235                 240

Arg Arg Ser Arg Leu Asp Ser Ser Glu Leu Leu Arg Gln Glu Ala Asn
                245                 250                 255

Thr Pro Val Thr Arg Phe Asn Thr Thr Lys Val Val Ser Thr Phe Asn
            260                 265                 270

Leu Pro Leu Leu Leu Ser Val Ser Ser Ser Thr Gln Asp Gly Glu Ile
        275                 280                 285

Met His Thr Arg Tyr Glu Leu Val Thr Thr Val His Leu Pro Trp Ser
    290                 295                 300

Leu Thr Ser Leu Lys Ala Lys Val Pro Ile Ile Ile Thr Ser Ala Ser
305                 310                 315                 320

Val Asp Ser Ala Ile Cys Gln Leu Ser Glu Asp Gly Val Leu Pro Val
                325                 330                 335

Asn Pro Asp His Gln Asn
            340

<210> 195
<211> 1068
<212> DNA
<213> Homo sapiens

<400> 195
atggatttca tctttcatga gaaacaggaa ggtttcctgt gtgctcagca ctgtctgaac      60

aatctattgc aaggagaata ttttagccct gtggaattag cctcaattgc acatcagcta     120

gatgaagaag agaggatgag aatggcagaa ggaggagtca ccagtgaaga gtatcttgca     180

tttttacagc agccttcaga aaacatggat gataccggtt tcttctccat tcaggtaata     240

agcaatgcct tgaagttctg gggtttagag atcatccatt tcaataatcc tgaatatcag     300

aagctcggca ttgatcctat aaatgaaaga tcttttatat gtaattataa acaacactgg     360

tttactatta gaaaatttgg aaaacactgg tttaacttga attctctctt ggcgggtcca     420

gaattaatat cagatacatg ccttgcaaat ttcttggctc gattacaaca acaagcatat     480

tctgtatttg ttgtcaaggg tgatctgcca gactgtgaag ctgaccaact cctgcagatc     540

atcagtgtcg aagagatgga tacaccaaaa cttaatggaa aaaaattagt aaaacaaaaa     600

gagcatagag tctataaaac agtccttgaa aaagtatcag aagaaagtga tgagtctgga     660

acatcagacc aagatgagga ggattttcag agggcccttg aactaagccg ccaagaaacc     720

aatagagaag atgaacatct ccgcagtact attgagttaa gcatgcaagg tagttccgga     780

aacacatcgc aagatcttcc aaagacatca tgtgtaactc ctgcttcaga acagccgaag     840

aaaataaaag aagactattt tgaaaagcat cagcaggaac agaagcagca gcaacaacag     900

tcagatctgc cgggccacag ttcataccta cacgaaaggc caacaacaag ttcgagagca     960

attgagagtg atctcagtga tgacatcagt gaaggcacag tacaggccgc tgtcgacacc    1020

attttagaaa ttatgagaaa gaatttgaaa atcaaagggg aaaaataa                 1068

<210> 196
<211> 2393
<212> DNA
<213> Homo sapiens

<400> 196

cgctgagatc tgtggaggtt tttctctgca aatgcagaaa gaaatcaggt ggatggatgc 60
ataattatgg ccctgctcct ggtctctttg ctggcattcc tgagcttggg ctcaggatgt 120
catcatcgga tctgtcactg ctctaacagg gtttttctct gccaagagag caaggtgaca 180
gagattcctt ctgacctccc gaggaatgcc attgaactga ggtttgtcct caccaagctt 240
cgagtcatcc aaaaaggtgc attttcagga tttggggacc tggagaaaat agagatctct 300
cagaatgatg tcttggaggt gatagaggca gatgtgttct ccaaccttcc caaattacat 360
gaaattagaa ttgaaaaggc caacaacctg ctctacatca cccctgaggc cttccagaac 420
cttcccaacc ttcaatatct gttaatatcc aacacaggta ttaagcacct tccagatgtt 480
cacaagattc attctctcca aaaggtttta cttgacattc aagataacat aaacatccac 540
acaattgaaa gaaattcttt cgtggggctg agctttgaaa gtgtgattct atggctgaat 600
aagaatggga ttcaagaaat acacaactgt gcattcaatg gaacccaact agatgcagtg 660
aatctaagcg ataataataa tttagaagaa ttgcctaatg atgttttcca cggagcctct 720
ggaccagtca ttctagatat ttcaagaaca aggatccatt ccctgcctag ctatggctta 780
gaaaatctta agaagctgag ggccaggtcg acttacaact taaaaaagct gcctactctg 840
gaaaagcttg tcgccctcat ggaagccagc ctcacctatc ccagccattg ctgtgccttt 900
gcaaactgga gacggcaaat ctctgagctt catccaattt gcaacaaatc tattttaagg 960
caagaagttg attatatgac tcaggctagg ggtcagagat cctctctggc agaagacaat 1020
gagtccagct acagcagagg atttgacatg acgtacactg agtttgacta tgacttatgc 1080
aatgaagtgg ttgacgtgac ctgctcccct aagccagatg cattcaaccc atgtgaagat 1140
atcatggggt acaacatcct cagagtcctg atatggttta tcagcatcct ggccatcact 1200
gggaacatca tagtgctagt gatcctaact accagccaat ataaactcac agtccccagg 1260
ttccttatgt gcaacctggc ctttgctgat ctctgcattg gaatctacct gctgctcatt 1320
gcatcagttg atatccatac caagagccaa tatcacaact atgccattga ctggcaaact 1380
ggggcaggct gtgatgctgc tggctttttc actgtctttg ccagtgagct gtcagtctac 1440
actctgacag ctatcacctt ggaaagatgg cataccatca cgcatgccat gcagctggac 1500
tgcaaggtgc agctccgcca tgctgccagt gtcatggtga tgggctggat ttttgctttt 1560
gcagctgccc tctttcccat ctttggcatc agcagctaca tgaaggtgag catctgcctg 1620
cccatggata ttgacagccc tttgtcacag ctgtatgtca tgtccctcct tgtgctcaat 1680
gtcctggcct ttgtggtcat ctgtggctgc tatatccaca tctacctcac agtgcggaac 1740
cccaacatcg tgtcctcctc tagtgacacc aggatcgcca agcgcatggc catgctcatc 1800
ttcactgact tcctctgcat ggcacccatt tctttctttg ccatttctgc ctccctcaag 1860

gtgcccctca tcactgtgtc caaagcaaag attctgctgg ttctgtttca ccccatcaac   1920

tcctgtgcca accccttcct ctatgccatc tttaccaaaa actttcgcag agatttcttc   1980

attctgctga gcaagtgtgg ctgctatgaa atgcaagccc aaatttatag gacagaaact   2040

tcatccactg tccacaacac ccatccaagg aatggccact gctcttcagc tcccagagtc   2100

accagtggtt ccacttacat acttgtccct ctaagtcatt tagcccaaaa ctaaaacaca   2160

atgtgaaaat gtatctgagt attgaatgat aattcagtcc ttgcctttga agggtatgtc   2220

acaaggagct gacagtgctt ctacacattt catctaattt aatattcctg gcataccttt   2280

aaggtaaatt ggtcaggaac tattaattcc atgtgataca ttaggaagct gaattattag   2340

taacaacaat aataattaaa gaatgcaata ctgtaaaaaa gcggccgcga att          2393

<210> 197
<211> 695
<212> PRT
<213> Homo sapiens

<400> 197

<210> 198
<211> 6378
<212> DNA
<213> Homo sapiens

<400> 198
cccattactg ttggagctac agggagagaa acaggaggag actgcaagag atcatttggg      60

aaggccgtgg gcacgctctt tactccatgt gtgggacatt cattgcggaa taacatcgga     120

ggagaagttt cccagagcta tggggacttc ccatccggcg ttcctggtct taggctgtct     180

tctcacaggg ctgagcctaa tcctctgcca gctttcatta ccctctatcc ttccaaatga     240

aaatgaaaag gttgtgcagc tgaattcatc cttttctctg agatgctttg gggagagtga     300

agtgagctgg cagtacccca tgtctgaaga agagagctcc gatgtggaaa tcagaaatga     360

agaaaacaac agcggccttt ttgtgacggt cttggaagtg agcagtgcct cggcggccca     420

cacagggttg tacacttgct attacaacca cactcagaca gaagagaatg agcttgaagg     480

caggcacatt tacatctatg tgccagaccc agatgtagcc tttgtacctc taggaatgac     540

ggattattta gtcatcgtgg aggatgatga ttctgccatt ataccttgtc gcacaactga     600

tcccgagact cctgtaacct tacacaacag tgagggggtg gtacctgcct cctacgacag     660

cagacagggc tttaatggga ccttcactgt agggccctat atctgtgagg ccaccgtcaa     720

aggaaagaag ttccagacca tcccatttaa tgtttatgct ttaaaagcaa catcagagct     780

ggatctagaa atggaagctc ttaaaaccgt gtataagtca ggggaaacga ttgtggtcac     840

ctgtgctgtt tttaacaatg aggtggttga ccttcaatgg acttaccctg gagaagtgaa     900

aggcaaaggc atcacaatgc tggaagaaat caaagtccca tccatcaaat tggtgtacac     960

tttgacggtc cccgaggcca cggtgaaaga cagtggagat tacgaatgtg ctgcccgcca    1020

ggctaccagg gaggtcaaag aaatgaagaa agtcactatt tctgtccatg agaaaggttt    1080

cattgaaatc aaacccacct tcagccagtt ggaagctgtc aacctgcatg aagtcaaaca 1140

ttttgttgta gaggtgcggg cctacccacc tcccaggata tcctggctga aaaacaatct 1200

gactctgatt gaaaatctca ctgagatcac cactgatgtg gaaaagattc aggaaataag 1260

gtatcgaagc aaattaaagc tgatccgtgc taaggaagaa gacagtggcc attatactat 1320

tgtagctcaa aatgaagatg ctgtgaagag ctatactttt gaactgttaa ctcaagttcc 1380

ttcatccatt ctggacttgg tcgatgatca ccatggctca actgggggac agacggtgag 1440

gtgcacagct gaaggcacgc cgcttcctga tattgagtgg atgatatgca aagatattaa 1500

gaaatgtaat aatgaaactt cctggactat tttggccaac aatgtctcaa acatcatcac 1560

ggagatccac tcccgagaca ggagtaccgt ggagggccgt gtgactttcg ccaaagtgga 1620

ggagaccatc gccgtgcgat gcctggctaa gaatctcctt ggagctgaga accgagagct 1680

gaagctggtg gctcccaccc tgcgttctga actcacggtg gctgctgcag tcctggtgct 1740

gttggtgatt gtgatcatct cacttattgt cctggttgtc atttggaaac agaaaccgag 1800

gtatgaaatt cgctggaggg tcattgaatc aatcagcccg gatggacatg aatatattta 1860

tgtggacccg atgcagctgc cttatgactc aagatgggag tttccaagag atggactagt 1920

gcttggtcgg gtcttggggt ctggagcgtt tgggaaggtg gttgaaggaa cagcctatgg 1980

attaagccgg tcccaacctg tcatgaaagt tgcagtgaag atgctaaaac ccacggccag 2040

atccagtgaa aaacaagctc tcatgtctga actgaagata atgactcacc tggggccaca 2100

tttgaacatt gtaaacttgc tgggagcctg caccaagtca ggccccattt acatcatcac 2160

agagtattgc ttctatggag atttggtcaa ctatttgcat aagaataggg atagcttcct 2220

gagccaccac ccagagaagc caaagaaaga gctggatatc tttggattga accctgctga 2280

tgaaagcaca cggagctatg ttattttatc ttttgaaaac aatggtgact acatggacat 2340

gaagcaggct gatactacac agtatgtccc catgctagaa aggaaagagg tttctaaata 2400

ttccgacatc cagagatcac tctatgatcg tccagcctca tataagaaga aatctatgtt 2460

agactcagaa gtcaaaaacc tcctttcaga tgataactca gaaggcctta ctttattgga 2520

tttgttgagc ttcacctatc aagttgcccg aggaatggag tttttggctt caaaaaattg 2580

tgtccaccgt gatctggctg ctcgcaacgt cctcctggca caaggaaaaa ttgtgaagat 2640

ctgtgacttt ggcctggcca gagacatcat gcatgattcg aactatgtgt cgaaaggcag 2700

tacctttctg cccgtgaagt ggatggctcc tgagagcatc tttgacaacc tctacaccac 2760

actgagtgat gtctggtctt atggcattct gctctgggag atcttttccc ttggtggcac 2820

cccttacccc ggcatgatgg tggattctac tttctacaat aagatcaaga gtgggtaccg 2880

gatggccaag cctgaccacg ctaccagtga agtctacgag atcatggtga aatgctggaa 2940

cagtgagccg gagaagagac cctcctttta ccacctgagt gagattgtgg agaatctgct 3000
gcctggacaa tataaaaaga gttatgaaaa aattcacctg gacttcctga agagtgacca 3060
tcctgctgtg gcacgcatgc gtgtggactc agacaatgca tacattggtg tcacctacaa 3120
aaacgaggaa gacaagctga aggactggga gggtggtctg gatgagcaga gactgagcgc 3180
tgacagtggc tacatcattc ctctgcctga cattgaccct gtccctgagg aggaggacct 3240
gggcaagagg aacagacaca gctcgcagac ctctgaagag agtgccattg agacgggttc 3300
cagcagttcc accttcatca agagagagga cgagaccatt gaagacatcg acatgatgga 3360
cgacatcggc atagactctt cagacctggt ggaagacagc ttcctgtaac tggcggattc 3420
gaggggttcc ttccacttct ggggccacct ctggatcccg ttcagaaaac cactttattg 3480
caatgcggag gttgagagga ggacttggtt gatgtttaaa gagaagttcc cagccaaggg 3540
cctcggggag cgttctaaat atgaatgaat gggatatttt gaaatgaact ttgtcagtgt 3600
tgcctctcgc aatgcctcag tagcatctca gtggtgtgtg aagtttggag atagatggat 3660
aagggaataa taggccacag aaggtgaact ttgtgcttca aggacattgg tgagagtcca 3720
acagacacaa tttatactgc gacagaactt cagcattgta attatgtaaa taactctaac 3780
caaggctgtg tttagattgt attaactatc ttctttggac ttctgaagag accactcaat 3840
ccatccatgt acttccctct tgaaacctga tgtcagctgc tgttgaactt tttaaagaag 3900
tgcatgaaaa accatttttg aaccttaaaa ggtactggta ctatagcatt ttgctatctt 3960
ttttagtgtt aagagataaa gaataataat taaccaacct tgtttaatag atttgggtca 4020
tttagaagcc tgacaactca ttttcatatt gtaatctatg tttataatac tactactgtt 4080
atcagtaatg ctaaatgtgt aataatgtaa catgatttcc ctccagagaa agcacaattt 4140
aaaacaatcc ttactaagta ggtgatgagt ttgacagttt ttgacattta tattaaataa 4200
catgtttctc tataaagtat ggtaatagct ttagtgaatt aaatttagtt gagcatagag 4260
aacaaagtaa aagtagtgtt gtccaggaag tcagaatttt taactgtact gaataggttc 4320
cccaatccat cgtattaaaa aacaattaac tgccctctga aataatggga ttagaaacaa 4380
acaaaactct taagtcctaa aagttctcaa tgtagaggca taaacctgtg ctgaacataa 4440
cttctcatgt atattaccca atggaaaata taatgatcag caaaaagact ggatttgcag 4500
aagttttttt tttttttctt catgcctgat gaaagctttg gcaaccccaa tatatgtatt 4560
ttttgaatct atgaacctga aaagggtcag aaggatgccc agacatcagc ctccttcttt 4620
caccccttac cccaaagaga aagagtttga aactcgagac cataaagata ttctttagtg 4680
gaggctggat gtgcattagc ctggatcctc agttctcaaa tgtgtgtggc agccaggatg 4740

actagatcct gggtttccat ccttgagatt ctgaagtatg aagtctgagg gaaaccagag 4800

tctgtatttt tctaaactcc ctggctgttc tgatcggcca gttttcggaa acactgactt 4860

aggtttcagg aagttgccat gggaaacaaa taatttgaac tttggaacag ggttggaatt 4920

caaccacgca ggaagcctac tatttaaatc cttggcttca ggttagtgac atttaatgcc 4980

atctagctag caattgcgac cttaatttaa ctttccagtc ttagctgagg ctgagaaagc 5040

taaagtttgg ttttgacagg ttttccaaaa gtaaagatgc tacttcccac tgtatggggg 5100

agattgaact ttccccgtct cccgtcttct gcctcccact ccataccccg ccaaggaaag 5160

gcatgtacaa aaattatgca attcagtgtt ccaagtctct gtgtaaccag ctcagtgttt 5220

tggtggaaaa aacattttaa gttttactga taatttgagg ttagatggga ggatgaattg 5280

tcacatctat ccacactgtc aaacaggttg gtgtgggttc attggcattc tttgcaatac 5340

tgcttaattg ctgataccat atgaatgaaa catgggctgt gattactgca atcactgtgc 5400

tatcggcaga tgatgctttg gaagatgcag aagcaataat aaagtacttg actacctact 5460

ggtgtaatct caatgcaagc cccaactttc ttatccaact ttttcatagt aagtgcgaag 5520

actgagccag attggccaat taaaaacgaa aacctgacta ggttctgtag agccaattag 5580

acttgaaata cgtttgtgtt tctagaatca cagctcaagc attctgttta tcgctcactc 5640

tcccttgtac agccttattt tgttggtgct ttgcattttg atattgctgt gagccttgca 5700

tgacatcatg aggccggatg aaacttctca gtccagcagt ttccagtcct aacaaatgct 5760

cccacctgaa tttgtatatg actgcatttg tgggtgtgtg tgtgttttca gcaaattcca 5820

gatttgtttc cttttggcct cctgcaaagt ctccagaaga aaatttgcca atctttccta 5880

ctttctattt ttatgatgac aatcaaagcc ggcctgagaa acactatttg tgacttttta 5940

aacgattagt gatgtcctta aaatgtggtc tgccaatctg tacaaaatgg tcctattttt 6000

gtgaagaggg acataagata aaatgatgtt atacatcaat atgtatatat gtatttctat 6060

atagacttgg agaatactgc caaaacattt atgacaagct gtatcactgc cttcgtttat 6120

atttttttaa ctgtgataat ccccacaggc acattaactg ttgcactttt gaatgtccaa 6180

aatttatatt ttagaaataa taaaaagaaa gatacttaca tgttcccaaa acaatggtgt 6240

ggtgaatgtg tgagaaaaac taacttgata gggtctacca atacaaaatg tattacgaat 6300

gcccctgttc atgtttttgt tttaaaacgt gtaaatgaag atctttatat ttcaataaat 6360

gatatataat ttaaagtt 6378

<210> 199
<211> 1089
<212> PRT
<213> Homo sapiens

<400> 199

<210> 200
<211> 2807
<212> DNA
<213> Homo sapiens

<400> 200
tgattttggg acccagccat ggtccttctg cttcttcttt aaaataccca ctttctcccc      60

atcgccaagc ggcgtttggc aatatcagat atccactcta tttattttta cctaaggaaa     120

aactccagct cccttcccac tcccagctgc cttgccaccc ctcccagccc tctgcttgcc     180

ctccacctgg cctgctggga gtcagagccc agcaaaacct gtttagacac atggacaaga     240

atcccagcgc tacaaggcac acagtccgct tcttcgtcct cagggttgcc agcgcttcct     300

ggaagtcctg aagctctcgc agtgcagtga gttcatgcac cttcttgcca agcctcagtc     360

tttgggatct ggggaggccg cctggttttc ctccctcctt ctgcacgtct gctggggtct     420

cttcctctcc aggccttgcc gtccccctgg cctctcttcc cagctcacac atgaagatgc     480

acttgcaaag ggctctggtg gtcctggccc tgctgaactt tgccacggtc agcctctctc     540

tgtccacttg caccaccttg gacttcggcc acatcaagaa gaagagggtg gaagccatta     600

ggggacagat cttgagcaag ctcaggctca ccagcccccc tgagccaacg gtgatgaccc     660

acgtccccta tcaggtcctg gccctttaca acagcacccg ggagctgctg gaggagatgc 720

atggggagag ggaggaaggc tgcacccagg aaaacaccga gtcggaatac tatgccaaag 780

aaatccataa attcgacatg atccaggggc tggcggagca caacgaactg gctgtctgcc 840

ctaaaggaat tacctccaag gttttccgct tcaatgtgtc ctcagtggag aaaaatagaa 900

ccaacctatt ccgagcagaa ttccgggtct tgcgggtgcc caaccccagc tctaagcgga 960

atgagcagag gatcgagctc ttccagatcc ttcggccaga tgagcacatt gccaaacagc 1020

gctatatcgg tggcaagaat ctgcccacac ggggcactgc cgagtggctg tcctttgatg 1080

tcactgacac tgtgcgtgag tggctgttga gaagagagtc caacttaggt ctagaaatca 1140

gcattcactg tccatgtcac acctttcagc ccaatggaga tatcctggaa aacattcacg 1200

aggtgatgga aatcaaattc aaaggcgtgg acaatgagga tgaccatggc cgtggagatc 1260

tggggcgcct caagaagcag aaggatcacc acaaccctca tctaatcctc atgatgattc 1320

ccccacaccg gctcgacaac ccgggccagg ggggtcagag gaagaagcgg gctttggaca 1380

ccaattactg cttccgcaac ttggaggaga actgctgtgt gcgccccctc tacattgact 1440

tccgacagga tctgggctgg aagtgggtcc atgaacctaa gggctactat gccaacttct 1500

gctcaggccc ttgcccatac ctccgcagtg cagacacaac ccacagcacg gtgctgggac 1560

tgtacaacac tctgaaccct gaagcatctg cctcgccttg ctgcgtgccc caggacctgg 1620

agcccctgac catcctgtac tatgttggga ggacccccaa agtggagcag ctctccaaca 1680

tggtggtgaa gtcttgtaaa tgtagctgag accccacgtg cgacagagag aggggagaga 1740

gaaccaccac tgcctgactg cccgctcctc gggaaacaca caagcaacaa acctcactga 1800

gaggcctgga gcccacaacc ttcggctccg ggcaaatggc tgagatggag gtttcctttt 1860

ggaacatttc tttcttgctg gctctgagaa tcacggtggt aaagaaagtg tgggtttggt 1920

tagaggaagg ctgaactctt cagaacacac agactttctg tgacgcagac agaggggatg 1980

gggatagagg aaagggatgg taagttgaga tgttgtgtgg caatgggatt tgggctaccc 2040

taaagggaga aggaagggca gagaatggct gggtcagggc cagactggaa gacacttcag 2100

atctgaggtt ggatttgctc attgctgtac cacatctgct ctagggaatc tggattatgt 2160

tatacaaggc aagcattttt tttttttttt taaagacagg ttacgaagac aaagtcccag 2220

aattgtatct catactgtct gggattaagg gcaaatctat tacttttgca aactgtcctc 2280

tacatcaatt aacatcgtgg gtcactacag ggagaaaatc caggtcatgc agttcctggc 2340

ccatcaactg tattgggcct tttggatatg ctgaacgcag aagaaagggt ggaaatcaac 2400

cctctcctgt ctgccctctg ggtccctcct ctcacctctc cctcgatcat atttcccctt 2460

ggacacttgg ttagacgcct tccaggtcag gatgcacatt tctggattgt ggttccatgc 2520

agccttgggg cattatgggt tcttccccca cttcccctcc aagaccctgt gttcatttgg 2580

tgttcctgga agcaggtgct acaacatgtg aggcattcgg ggaagctgca catgtgccac 2640

acagtgactt ggccccagac gcatagactg aggtataaag acaagtatga atattactct 2700

caaaatcttt gtataaataa atatttttgg ggcatcctgg atgatttcat cttctggaat 2760

attgtttcta gaacagtaaa agccttattc taaaaaaaaa aaaaaaa 2807

<210> 201
<211> 412
<212> PRT
<213> Homo sapiens

<400> 201

<210> 202
<211> 2240
<212> DNA
<213> Homo sapiens

<400> 202
acttccccgc cctcgcccca aaggagcagc agctccttct tgcctctcca ttgccgccgc      60

cgcaccggcg gagctcctct ctcgcgcgtc tctcctccga tggagctcgg gcgccgccga     120

cgccgccgct gccccgaacc ctgagcgggg ccgccccggt cggaggaacg cgccgcccag     180

tccgagggcg cagagcgcca ggagcacgcg gagggctggg gcgcgggctc cgggaacgag     240

aaagtgcagc tctctcgggt cactgggccg gcggcggggg gactatggct ctgaaggaca     300

cgggcagcgg cggcagcacc atcctgccca ttagcgagat ggtttcctcg tccagctcgc     360

ccggcgcgtc ggccgccgcc gccccggggc cctgcgcacc ctcgcccttc cctgaagtag     420

tggagctgaa cgtaggcggc caggtttatg tgaccaagca ctcgacgctg ctcagcgtcc     480

cggacagtac tttggccagc atgttctcgc cctctagtcc ccgtggcggc gcccggcgcc     540

ggggcgagct gcccagggac agccgggcgc gcttcttcat cgaccgggac ggcttccttt     600

tcaggtacgt gctggattat ctgcgggaca agcaactcgc gctgccggag cacttccccg     660

agaaggagcg gctgctgcgc gaggccgagt atttccagct caccgacttg gtcaagctgc     720

tgtcgcccaa ggtcaccaag cagaactctc tcaacgacga gggctgccag agcgacctgg     780

aggacaacgt ctcgcagggt agcagcgacg cgctgctgct gcgcggggcg gcggccgccg     840

tgccctcggg cccgggagcg cacggtggtg gcggcggcgg cggcgcgcag gacaagcgct     900

cgggcttcct cacgctgggc taccggggct cctacaccac cgtgcgcgac aaccaggccg     960

acgccaaatt ccggcgtgtg gcgcgcatca tggtgtgcgg gcgcatcgcg ctggccaagg    1020

aggtcttcgg ggacacgctc aacgagagcc gcgaccccga ccggcagccg gagaagtaca    1080

cgtcccgctt ctacctcaag ttcacctact tggagcaggc ctttgatcgc ctgtccgagg    1140

ccggcttcca catggtggcg tgtaactcct cgggcaccgc cgccttcgtc aaccagtacc    1200

gcgacgacaa gatctggagc agctacaccg agtacatttt cttccgacca cctcagaaaa    1260

tagtatcacc taaacaagaa catgaagata ggaaacatga caaagtcact gataaaggaa    1320

gtgaaagtgg gacttcctgt aatgagctct ccacttccag ttgtgacagc cattcagagg    1380

caagcactcc ccaggacaac ccatccagtg cccagcaggc aacagctcac caacctaaca    1440

ctttaacatt ggatcgcccc tctaaaaaag cacctgtaca atggataccc ccaccagaca    1500

aacgcagaaa cagtgaactc tttcagaccc tcatcagcaa gtcccgggaa acaaatctgt    1560

ccaaaaagaa agtctgtgag aagctaagtg tggaagaaga aatgaaaaag tgtattcagg    1620

attttaaaaa aatccacatt ccagattatt ttccagagcg caaacgccaa tggcaatctg 1680

aactgttgca gaagtatggg ttatagtaat tgtcacattc ctgcagtatt ttgatgacat 1740

tcaatgttta ctacagtgtc accacctgac tgatgtccta acaatggtca gtgtgattct 1800

tgctgctctt ccttgttgtg aacagtggat gtgggacagt attttctttt atgttttagt 1860

tgttgttctt tttagaaaca tgattaaaaa ggaaaaaata ttaaatcaat aagtgttaaa 1920

tcaaaatgga atatctgatt caaaccattt tacaagaatg aaagtaaaat gtgcatgatc 1980

aagcttagta tcttggtttt tgaactctgg tcaactggat atgtttgtca ttttgtaact 2040

taccaaaaac aaaccatcat atcataccaa ctaaaatgat atatggatga agcaacatca 2100

agtaaaattt tagacgatgg ctataggacc caaatctaaa gctgtctaaa tgttaattca 2160

atgaaacaag tattattttt gcatgaatac aatgttacaa ataaatcaca agaaataggg 2220

aagatctgtt tgttgcttgg 2240

<210> 203
<211> 473
<212> PRT
<213> Homo sapiens

<400> 203
Met Ala Leu Lys Asp Thr Gly Ser Gly Gly Ser Thr Ile Leu Pro Ile
1               5                   10                  15

Ser Glu Met Val Ser Ser Ser Ser Ser Pro Gly Ala Ser Ala Ala Ala
            20                  25                  30

Ala Pro Gly Pro Cys Ala Pro Ser Pro Phe Pro Glu Val Val Glu Leu
        35                  40                  45

Asn Val Gly Gly Gln Val Tyr Val Thr Lys His Ser Thr Leu Leu Ser
    50                  55                  60

Val Pro Asp Ser Thr Leu Ala Ser Met Phe Ser Pro Ser Ser Pro Arg
65                  70                  75                  80

Gly Gly Ala Arg Arg Arg Gly Glu Leu Pro Arg Asp Ser Arg Ala Arg
                85                  90                  95

Phe Phe Ile Asp Arg Asp Gly Phe Leu Phe Arg Tyr Val Leu Asp Tyr
            100                 105                 110

Leu Arg Asp Lys Gln Leu Ala Leu Pro Glu His Phe Pro Glu Lys Glu
        115                 120                 125

Arg Leu Leu Arg Glu Ala Glu Tyr Phe Gln Leu Thr Asp Leu Val Lys
    130                 135                 140

Leu Leu Ser Pro Lys Val Thr Lys Gln Asn Ser Leu Asn Asp Glu Gly
145                 150                 155                 160

Cys Gln Ser Asp Leu Glu Asp Asn Val Ser Gln Gly Ser Ser Asp Ala
                165                 170                 175

Leu Leu Leu Arg Gly Ala Ala Ala Ala Val Pro Ser Gly Pro Gly Ala
            180                 185                 190

His Gly Gly Gly Gly Gly Gly Gly Ala Gln Asp Lys Arg Ser Gly Phe
        195                 200                 205

Leu Thr Leu Gly Tyr Arg Gly Ser Tyr Thr Thr Val Arg Asp Asn Gln
    210                 215                 220

Ala Asp Ala Lys Phe Arg Arg Val Ala Arg Ile Met Val Cys Gly Arg
225                 230                 235                 240

Ile Ala Leu Ala Lys Glu Val Phe Gly Asp Thr Leu Asn Glu Ser Arg
                245                 250                 255

Asp Pro Asp Arg Gln Pro Glu Lys Tyr Thr Ser Arg Phe Tyr Leu Lys
            260                 265                 270

Phe Thr Tyr Leu Glu Gln Ala Phe Asp Arg Leu Ser Glu Ala Gly Phe
        275                 280                 285

His Met Val Ala Cys Asn Ser Ser Gly Thr Ala Ala Phe Val Asn Gln
    290                 295                 300

Tyr Arg Asp Asp Lys Ile Trp Ser Ser Tyr Thr Glu Tyr Ile Phe Phe
305                 310                 315                 320

Arg Pro Pro Gln Lys Ile Val Ser Pro Lys Gln Glu His Glu Asp Arg
                325                 330                 335

Lys His Asp Lys Val Thr Asp Lys Gly Ser Glu Ser Gly Thr Ser Cys
            340                 345                 350

Asn Glu Leu Ser Thr Ser Ser Cys Asp Ser His Ser Glu Ala Ser Thr
        355                 360                 365

Pro Gln Asp Asn Pro Ser Ser Ala Gln Gln Ala Thr Ala His Gln Pro

370                 375                 380

Asn Thr Leu Thr Leu Asp Arg Pro Ser Lys Lys Ala Pro Val Gln Trp
385                 390                 395                 400

Ile Pro Pro Pro Asp Lys Arg Arg Asn Ser Glu Leu Phe Gln Thr Leu
                405                 410                 415

Ile Ser Lys Ser Arg Glu Thr Asn Leu Ser Lys Lys Lys Val Cys Glu
            420                 425                 430

Lys Leu Ser Val Glu Glu Glu Met Lys Lys Cys Ile Gln Asp Phe Lys
        435                 440                 445

Lys Ile His Ile Pro Asp Tyr Phe Pro Glu Arg Lys Arg Gln Trp Gln
    450                 455                 460

Ser Glu Leu Leu Gln Lys Tyr Gly Leu
465                 470

<210> 204
<211> 2983
<212> DNA
<213> Homo sapiens

<400> 204
agagatagag tcttccctgg cattgcagga gagaatctga agggatgatg gatgcatcaa      60

aagagctgca agttctccac attgacttct tgaatcagga caacgccgtt tctcaccaca     120

catgggagtt ccaaacgagc agtcctgtgt tccggcgagg acaggtgttt cacctgcggc     180

tggtgctgaa ccagccccta caatcctacc accaactgaa actggaattc agcacagggc     240

cgaatcctag catcgccaaa cacaccctgg tggtgctcga cccgaggacg ccctcagacc     300

actacaactg gcaggcaacc cttcaaaatg agtctggcaa agaggtcaca gtggctgtca     360

ccagttcccc caatgccatc ctgggcaagt accaactaaa cgtgaaaact ggaaaccaca     420

tccttaagtc tgaagaaaac atcctatacc ttctcttcaa cccatggtgt aaagaggaca     480

tggttttcat gcctgatgag gacgagcgca aagagtacat cctcaatgac acgggctgcc     540

attacgtggg ggctgccaga agtatcaaat gcaaaccctg gaactttggt cagtttgaga     600

aaaatgtcct ggactgctgc atttccctgc tgactgagag ctccctcaag cccacagata     660

ggagggaccc cgtgctggtg tgcagggcca tgtgtgctat gatgagcttt gagaaaggcc     720

agggcgtgct cattgggaat tggactgggg actatgaagg tggcacagcc ccatacaagt     780

ggacaggcag tgccccgatc ctgcagcagt actacaacac gaagcaggct gtgtgctttg     840

gccagtgctg ggtgtttgct gggatcctga ctacagtgct gagagcgttg ggcatcccag 900

cacgcagtgt gacaggcttc gattcagctc acgacacaga aaggaacctc acggtggaca 960

cctatgtgaa tgagaatggc aagaaaatca ccagtatgac ccacgactct gtctggaatt 1020

tccatgtgtg gacggatgcc tggatgaagc gaccggatct gcccaagggc tacgacggct 1080

ggcaggctgt ggacgcaacg ccgcaggagc gaagccaggg tgtcttctgc tgtgggccat 1140

caccactgac cgccatccgc aaaggtgaca tctttattgt ctatgacacc agattcgtct 1200

tctcagaagt gaatggtgac aggctcatct ggttggtgaa gatggtgaat gggcaggagg 1260

agttacacgt aatttcaatg gagaccacaa gcatcgggaa aaacatcagc accaaggcag 1320

tgggccaaga caggcggaga gatatcacct atgagtacaa gtatccagaa ggctcctctg 1380

aggagaggca ggtcatggat catgccttcc tccttctcag ttctgagagg gagcacagac 1440

gacctgtaaa agagaacttt cttcacatgt cggtacaatc agatgatgtg ctgctgggaa 1500

actctgttaa tttcaccgtg attcttaaaa ggaagaccgc tgccctacag aatgtcaaca 1560

tcttgggctc ctttgaacta cagttgtaca ctggcaagaa gatggcaaaa ctgtgtgacc 1620

tcaataagac ctcgcagatc caaggtcaag tatcagaagt gactctgacc ttggactcca 1680

agacctacat caacagcctg gctatattag atgatgagcc agttatcaga ggtttcatca 1740

ttgcggaaat tgtggagtct aaggaaatca tggcctctga agtattcacg tctttccagt 1800

accctgagtt ctctatagag ttgcctaaca caggcagaat tggccagcta cttgtctgca 1860

attgtatctt caagaatacc ctggccatcc ctttgactga cgtcaagttc tctttggaaa 1920

gcctgggcat ctcctcacta cagacctctg accatgggac ggtgcagcct ggtgagacca 1980

tccaatccca aataaaatgc accccaataa aaactggacc caagaaattt atcgtcaagt 2040

taagttccaa acaagtgaaa gagattaatg ctcagaagat tgttctcatc accaagtagc 2100

cttgtctgat gctgtggagc cttagttgag atttcagcat ttcctacctt gtgcttagct 2160

ttcagattat ggatgattaa atttgatgac ttatatgagg gcagattcaa gagccagcag 2220

gtcaaaaagg ccaacacaac cataagcagc cagacccaca aggccaggtc ctgtgctatc 2280

acagggtcac ctcttttaca gttagaaaca ccagccgagg ccacagaatc ccatcccttt 2340

cctgagtcat ggcctcaaaa atcagggcca ccattgtctc aattcaaatc catagatttc 2400

gaagccacag agtctctccc tggagcagca gactatgggc agcccagtgc tgccacctgc 2460

tgacgaccct tgagaagctg ccatatcttc aggccatggg ttcaccagcc ctgaaggcac 2520

ctgtcaactg gagtgctctc tcagcactgg gatgggcctg atagaagtgc attctcctcc 2580

tattgcctcc attctcctct ctctatccct gaaatccagg aagtccctct cctggtgctc 2640

caagcagttt gaagcccaat ctgcaaggac atttctcaag ggccatgtgg ttttgcagac 2700

aaccctgtcc tcaggcctga actcaccata gagacccatg tcagcaaacg gtgaccagca 2760

aatcctcttc ccttattcta aagctgcccc ttgggagact ccagggagaa ggcattgctt 2820

cctccctggt gtgaactctt tctttggtat tccatccact atcctggcaa ctcaaggctg 2880

cttctgttaa ctgaagcctg ctccttcttg ttctgccctc cagagatttg ctcaaatgat 2940

caataagctt taaattaaac tctacttcaa gaaaaaaaaa ccg 2983

<210> 205
<211> 684
<212> PRT
<213> Homo sapiens

<400> 205
Met Met Asp Ala Ser Lys Glu Leu Gln Val Leu His Ile Asp Phe Leu
1               5                   10                  15

Asn Gln Asp Asn Ala Val Ser His His Thr Trp Glu Phe Gln Thr Ser
            20                  25                  30

Ser Pro Val Phe Arg Arg Gly Gln Val Phe His Leu Arg Leu Val Leu
        35                  40                  45

Asn Gln Pro Leu Gln Ser Tyr His Gln Leu Lys Leu Glu Phe Ser Thr
    50                  55                  60

Gly Pro Asn Pro Ser Ile Ala Lys His Thr Leu Val Val Leu Asp Pro
65                  70                  75                  80

Arg Thr Pro Ser Asp His Tyr Asn Trp Gln Ala Thr Leu Gln Asn Glu
                85                  90                  95

Ser Gly Lys Glu Val Thr Val Ala Val Thr Ser Ser Pro Asn Ala Ile
            100                 105                 110

Leu Gly Lys Tyr Gln Leu Asn Val Lys Thr Gly Asn His Ile Leu Lys
        115                 120                 125

Ser Glu Glu Asn Ile Leu Tyr Leu Leu Phe Asn Pro Trp Cys Lys Glu
    130                 135                 140

Asp Met Val Phe Met Pro Asp Glu Asp Glu Arg Lys Glu Tyr Ile Leu
145                 150                 155                 160

Asn Asp Thr Gly Cys His Tyr Val Gly Ala Ala Arg Ser Ile Lys Cys
                165                 170                 175

Lys Pro Trp Asn Phe Gly Gln Phe Glu Lys Asn Val Leu Asp Cys Cys
            180                 185                 190

Ile Ser Leu Leu Thr Glu Ser Ser Leu Lys Pro Thr Asp Arg Arg Asp
        195                 200                 205

Pro Val Leu Val Cys Arg Ala Met Cys Ala Met Met Ser Phe Glu Lys
    210                 215                 220

Gly Gln Gly Val Leu Ile Gly Asn Trp Thr Gly Asp Tyr Glu Gly Gly
225                 230                 235                 240

Thr Ala Pro Tyr Lys Trp Thr Gly Ser Ala Pro Ile Leu Gln Gln Tyr
                245                 250                 255

Tyr Asn Thr Lys Gln Ala Val Cys Phe Gly Gln Cys Trp Val Phe Ala
            260                 265                 270

Gly Ile Leu Thr Thr Val Leu Arg Ala Leu Gly Ile Pro Ala Arg Ser
        275                 280                 285

Val Thr Gly Phe Asp Ser Ala His Asp Thr Glu Arg Asn Leu Thr Val
    290                 295                 300

Asp Thr Tyr Val Asn Glu Asn Gly Glu Lys Ile Thr Ser Met Thr His
305                 310                 315                 320

Asp Ser Val Trp Asn Phe His Val Trp Thr Asp Ala Trp Met Lys Arg
                325                 330                 335

Pro Asp Leu Pro Lys Gly Tyr Asp Gly Trp Gln Ala Val Asp Ala Thr
            340                 345                 350

Pro Gln Glu Arg Ser Gln Gly Val Phe Cys Cys Gly Pro Ser Pro Leu
        355                 360                 365

Thr Ala Ile Arg Lys Gly Asp Ile Phe Ile Val Tyr Asp Thr Arg Phe
    370                 375                 380

Val Phe Ser Glu Val Asn Gly Asp Arg Leu Ile Trp Leu Val Lys Met
385                 390                 395                 400

Val Asn Gly Gln Glu Glu Leu His Val Ile Ser Met Glu Thr Thr Ser
                405                 410                 415

Ile Gly Lys Asn Ile Ser Thr Lys Ala Val Gly Gln Asp Arg Arg Arg
            420                 425                 430

Asp Ile Thr Tyr Glu Tyr Lys Tyr Pro Glu Gly Ser Ser Glu Glu Arg
        435                 440                 445

Gln Val Met Asp His Ala Phe Leu Leu Leu Ser Ser Glu Arg Glu His
    450                 455                 460

Arg Arg Pro Val Lys Glu Asn Phe Leu His Met Ser Val Gln Ser Asp
465                 470                 475                 480

Asp Val Leu Leu Gly Asn Ser Val Asn Phe Thr Val Ile Leu Lys Arg
                485                 490                 495

Lys Thr Ala Ala Leu Gln Asn Val Asn Ile Leu Gly Ser Phe Glu Leu
            500                 505                 510

Gln Leu Tyr Thr Gly Lys Lys Met Ala Lys Leu Cys Asp Leu Asn Lys
        515                 520                 525

Thr Ser Gln Ile Gln Gly Gln Val Ser Glu Val Thr Leu Thr Leu Asp
    530                 535                 540

Ser Lys Thr Tyr Ile Asn Ser Leu Ala Ile Leu Asp Asp Glu Pro Val
545                 550                 555                 560

Ile Arg Gly Phe Ile Ile Ala Glu Ile Val Glu Ser Lys Glu Ile Met
                565                 570                 575

Ala Ser Glu Val Phe Thr Ser Phe Gln Tyr Pro Glu Phe Ser Ile Glu
            580                 585                 590

Leu Pro Asn Thr Gly Arg Ile Gly Gln Leu Leu Val Cys Asn Cys Ile
        595                 600                 605

Phe Lys Asn Thr Leu Ala Ile Pro Leu Thr Asp Val Lys Phe Ser Leu
    610                 615                 620

Glu Ser Leu Gly Ile Ser Ser Leu Gln Thr Ser Asp His Gly Thr Val
625                 630                 635                 640

Gln Pro Gly Glu Thr Ile Gln Ser Gln Ile Lys Cys Thr Pro Ile Lys
                645                 650                 655

Thr Gly Pro Lys Lys Phe Ile Val Lys Leu Ser Ser Lys Gln Val Lys

660                 665                 670

Glu Ile Asn Ala Gln Lys Ile Val Leu Ile Thr Lys
        675                 680

<210> 206
<211> 1172
<212> DNA
<213> Homo sapiens

<400> 206
agtgaacacc acgagctagc gtgtctactt cacaggccag gtcgtcaact caactgtcaa      60

ggtgtccatt gtaccagctg ggccatggat ccctcccgcc tgaaatctga ctccacctgc     120

caagagtctg agcctgcagg cctagatttc gactctgctg gccaagatta tttctctgca     180

gcccaataat tcgactcttt ctaccaagaa ttggacctgg actctcttaa cgaagatctt     240

ctttcccagt ccatgccaca tgccaggaca gagacctctg tgggcacata tgaatcccac     300

tcgacttctg aactggagga tctgacagag cccgagcaaa gagagctcaa aaccaaactc     360

actaaattgg aggctgaaat tgtaacccta cgccacgtac tagcagccaa agagagacgc     420

tgtggggaac tcaagaggaa gttaggcctc accgccttgg tagggctgag acagaatctg     480

tccaagagct ggcttgatgt tcaggtctcc aacacctatg tgaaacagaa gacatcagct     540

gctctgtcca ccatgggcac tctcatctgc aggaagcttg gaggcgtgaa gaagtcggcc     600

acattcagat cttttgaagg aaaccctaaa ggagaaggaa gcagaatata accctctgga     660

gagctcaaga cagtaggaac ctggacggtg cttgtttgaa ttttctgata tctgattttc     720

acacgaatat actttttgtt tgttttcctt ttccattttc aaaatgccaa gatctgggag     780

cctctttcac tcctctagac acactgcctg agatagaagt ttgcatctta ttggctgtgg     840

gcagagacta ggacccttat gggtacattt cagtaatctc tcactgccta aaatggtaat     900

ccctccagcc taagaggatg attctgtttt gttgactctt gaatgctccc ccaacattcc     960

tttggttttc ctataagcaa aaccaaatac cactataaat acaaaagcat ctggtaaact    1020

gtaaagttac tcatgtatcc caaccctcca cagatttctg aagacaaagt tttgcttgga    1080

ttaaaacaag atgttttact attttgcatg ttcgattttt aatttgttgt gtttatcaac    1140

caaaatggct ttgtatgctg gagaacaaga gg                                  1172

<210> 207
<211> 132
<212> PRT
<213> Homo sapiens

<400> 207
Met Pro His Ala Arg Thr Glu Thr Ser Val Gly Thr Tyr Glu Ser His

1               5                   10                  15

Ser Thr Ser Glu Leu Glu Asp Leu Thr Glu Pro Glu Gln Arg Glu Leu
            20                  25                  30

Lys Thr Lys Leu Thr Lys Leu Glu Ala Glu Ile Val Thr Leu Arg His
        35                  40                  45

Val Leu Ala Ala Lys Glu Arg Arg Cys Gly Glu Leu Lys Arg Lys Leu
    50                  55                  60

Gly Leu Thr Ala Leu Val Gly Leu Arg Gln Asn Leu Ser Lys Ser Trp
65                  70                  75                  80

Leu Asp Val Gln Val Ser Asn Thr Tyr Val Lys Gln Lys Thr Ser Ala
                85                  90                  95

Ala Leu Ser Thr Met Gly Thr Leu Ile Cys Arg Lys Leu Gly Gly Val
            100                 105                 110

Lys Lys Ser Ala Thr Leu Arg Ser Phe Glu Gly Asn Pro Lys Gly Glu
        115                 120                 125

Gly Ser Arg Ile
    130

<210> 208
<211> 2051
<212> DNA
<213> Homo sapiens

<400> 208
agttcatcct gagccagctg ctgaggcggc ggctggggct ggggcggctg tggcggcgcc      60

tggcaggctc cgccagcccc ctgggagcgg gggatgggct ccgggcgcgg agaaactccc     120

tcaacatcca tctccatctt cccattcact ggagggcaag acaaaagcgg agtggagact     180

tggcagcggc gcccggcagc tactcagctg cccgtggcac gcatggctcg ccgaggaggg     240

gccgcctccc gcgcgccctc ccttctgcca cccttcgccc ccgctccccg cagctccgct     300

cgcctgcttg gctccggccc tggcggcgtc tgggccgggg tgcgcgagga gcgggtggcc     360

gcgtgtcctt ggagcgctgg ccttcaggcg cccccggcag tcccgctaag cagagcgcat     420

cccggcggcg gcggcggcgg cggcgcctcc cagccgcgct cccagcctca gggccgcttg     480

ggtacttgct ctccagcccg gcccacgctc ctagccgctt tccagcgtct cttggggccc     540

cttggcgttt ctcctcccta ccaaccttct gaacaaagtt gctggaagaa ccaacctggt     600

cgccgggctt tgcccggcga agctggcaaa ttccgggcaa cagagggtcg atggggtagg 660

aggaaggaga agcgagaagt cgctgccagg catttactgc aatgtgcccg gggcgggcgc 720

cagctgggcg ccgtctcttt ttggtggtga gttctgtgct ggaagagcgt cctcagcagg 780

gcagggctcc accaggaggc ttcggtggtg accccagggc aggcagtgcg ttgcgtctat 840

aaatttaaat agagatggcg agggtagcct tggaaattct taaatcaggg ttcaccacta 900

gtcaggtgaa caggatgact tctcaattta acaccacttg atcctgggta gtagcttaca 960

acttcatggg aactgccact gcaactctgc acgtccagga gggactttca agcagctctg 1020

gagagaaatg gcgggttatc aagaacctgt tcaatattaa atagagccca ttattctggg 1080

ctttgaattc cataatgctc ccaaatggat cagcagatta atgaactatt catgcgtttc 1140

ttggcagagg atggcttttt tcccctagca tgtatcacag atcacacttg cttgatgaaa 1200

ctggaaaatt gcattgctca tcccatgaat gctttgcatt aacttaaaca caacttttac 1260

atatcaagac acactgtccc attgatgcag ttacacaaga acacatcatt aggttgtatc 1320

cctcaatgct atcttttaat ctgactctgc atgtgtgaca gaaattaatt gccactttca 1380

tcagtgtaga caaccaatct gggtaatgca atgtgaacaa gtttttgaaa aagaagaaaa 1440

agtttaggga ccaaattacc cattctcttt ttcttcagat tttctaaaaa tcagatttca 1500

agttcgtttt ctaaattggg aattgttgaa aatagagtga gctcctcccc agtacctggt 1560

aataaaccaa caatcattat agaacgttta taatggtaaa ttagattata tgtgtaatgt 1620

gcttagcaga gtgtctttgt gcatatcgag cacctaacat tgttataccg agaacagtaa 1680

gatgatatat gtccacactg agtgttcatc tgaagacctt gatttacaaa atagaagcta 1740

ccggtttaaa tcacctttat ttaaatttcc taaaagatat ttggtatctt tagagaatga 1800

agccccttct gaagacagga agaaagtcaa taatattttg tgcaataaag gtttgaattt 1860

gtcaggtttt tgtttttttt tttttcccta cagtgtagca ataaaaatca catgtcagtt 1920

cagacagcag caggaatagc tgactggtaa gttattattc ttccatgctt gctacttcca 1980

aaatacgttt gtgaacttct ttcaaggctt aaatgaagat taaatcaaaa taaagataca 2040

atacttcaat t 2051

<210> 209
<211> 3556
<212> DNA
<213> Homo sapiens

<400> 209
cgcctcctcc tccaccagca ccactccctc cggggcaccc cggtccctgc agagtccctg 60

gccggcgccg cctccgctgc ggccccctga atcccgagcc tgcctcgccc aagctggtag 120

gacagaagga cagacagatt cctctagcct agcgctccgc cgctgctgcc ttacgcggcc 180
ccgcgtcggg agaactggga tcgccccaag agcaccgcga ggggtcatct caggctggct 240
gcatgcctca gctgaagatc ccagctcctg tcaatgccac ctctctgctt gactgtctcc 300
ttccagattc gagcaggtat gagctgggaa gaatgaaggc agggcatgcc cgtgtgccag 360
ctctgcacag ctggatagct gaggaaagat gtggaggaga agccggggat tgtgtggaag 420
tctaagggtg ttgtttgccc tttgggttcc agaagatgca tgccaggacc ctgggtggca 480
ctgccaggaa gcaacagaga ggagataaaa ctcacagcag acagacttgc cttaacaaca 540
actcccttga attaaaacac gcttttcaag aaaacaaatt atcagttcga tcagcaaaca 600
gcagagaagt ttctctcata atggcaaaga agggccgggt tgctgaccag tgaaagagct 660
tcagaaaagg agaggggaga tgagatggcc agaaggagca agagcaccgt acatccctgg 720
acaacctcat tctaatgggt caggggctgg gacgtgcatt ttggagtgca ggagaagtgg 780
caactcacaa atgctagatt ttcttctaga gatgaccaag ctgtagttct taaagcagtg 840
gcactagggc agaaaactct cacactttga tgtgcacaca cagcccctgg ggatcttgtt 900
gacatgtaga ttctgattcc gtaagtcggg ctgagattct gcatttccaa caagctccta 960
gatgaggtcc attttgctgg tccatgaaac acacttagaa taagtagcaa ggtataggag 1020
gatactgact ttgctcagtg atgcttgggc ttccgtccaa actaaaataa aacaaaagca 1080
gacataaatg gcccaattca acagcctgag aagtttggtg ataatgaccc aagccctggc 1140
ctggtgacca agtggctgct cagagagctc tatctccaaa ctccttccct tcctgcctcc 1200
cctccaagag tgagtaatgt gccagagttc attggggtct gctgtcccca gggaaaggtg 1260
gcccagttgc caagtgcaag tgtatagaac ggcttacctg tgcaaagcta tcagccctgg 1320
acctatgggc ctagaaacat tacttgccac tcactcactc actatcattc attcatttct 1380
gcaacaaaca cttcctgagc atatgctata caccaggccc tgagctcagc tctggggata 1440
caggatgaat ggcagattgt gccatcagga agccgactgc acagtcagcc cgctggagca 1500
catatgaaaa acaatacgga tgttttgatt tattatcata ttgatcatta tgatggtagc 1560
gtatactttt ggaaggcttg ccatacatta ggcattgtgc taagcacatt atatggttaa 1620
tctcatcaat cttcacaacc tatgtggcag gtactattat taaacccact tcaaagagga 1680
gggaactgaa cctcagagag ggtaagtgac ttgcacaagg acacaagcta gtgaggagag 1740
gaactgagag gaatggccag acaggccatc agcctctgtc cccacatgct gggtctgagt 1800
gtctgacggt ggacacattc ccttcctgga gggagagctc ctagggagct gatggcagca 1860
ctgagtctgg aatgtgatgg gagtgcatta gatgacgtgg gtgatctgat gagtacagag 1920
gcgtcacact gcaatgactt catgcacata tctccccttg aatgcacaga gattcagcag 1980

aggccaggag ctcaagtttc tggcccagtg tccccctctt cccctgggga aagccaggga 2040

cacactgcct ggctttgcta cacctttcta ggcaaaggca gtatgctctc cctctccttg 2100

gcctccctgg ctgtccacac atccacaccc actcacacac acatgcacat gatcatacac 2160

attacacaag cccacatgcc cacagacatg ctcacactca cactcacatg catacactgc 2220

catacacaac acacatacat acattcacac acacttgcac acacacacac acacacagtt 2280

ctgatggcca atcttttggg gctatgttga cactgtgaag caggtgaaac aaagtctttg 2340

ccactccctg ctttgggcct attatgtttg ttttatagtt taactctcaa aagacaactt 2400

tattttccaa aattattcag gttcccagcc aggtgcagtg gctcacacct gtaatcccag 2460

tactttggga ggccaaggcg ggcagatcac ctgaggtcag gagtttgaga ccagcttggc 2520

caacatggtg aaacccagtc tctactaaaa atacaaaaat tagccagaca tggtggcaca 2580

cacatgtaat cccagctatt cgggaggctg aggcaggaga atcgcttgaa cccgggaggc 2640

ggaggttgca gtgggccaag atcgtgccac tgcactccag cctgggcaac agagcaagac 2700

tccatctcaa aaaacaaaaa caaaatcatt caggttctgt aggggaccct cactgtgtcc 2760

tggattccat tccactttta aagtcccaaa cagcttgtat ggccacttcc cttgacaccc 2820

aaaccagata ggagtcttaa atgacacaga gtattgatgg tttctttaaa aggaaatcca 2880

ctctcaatgg gaagctggat aacttcgcat ccagatttca tccatcttgg gctttggtgg 2940

ctgctgtttg tttttgtgca ggagaatctc tcccagccaa cgtcgatttc actaacacag 3000

tggttaacct ttgctctcta ctcctgcatc aatcttccgc ctaccgcatg ttgagccatg 3060

ctgctttctg gcattccttt tggattttca gtttactacc caactaccag tcctaataac 3120

atcagataaa agggcttccg ctgtgctgca cttgttttca aaaactataa atgccatcga 3180

cccagagtta agtccagcct cagagagaca gaatataaat acccaagtgg ccctgggcca 3240

ctgagttcct cagacattat gtgctatggg agttccagaa agggggattg cctggttttg 3300

agtgcagaag gcttcctgga ggaggcgaga tttctgatgg gcctctgtta tgagctaaat 3360

tgagtccacc ccaaaattca gatgttcaaa tcctaacctg tagtacctaa gcatgtggcc 3420

ttatttggaa gcagaagcat aacagaggta attcgttaag atgaggtcac agtggaagag 3480

ggtggacccc aatctgatac aactcgtgtc cttataaaaa gcagaaattt gaagacagac 3540

acacaagaag atgaag 3556

<210> 210
<211> 3132
<212> DNA
<213> Homo sapiens

<400> 210
cccgcatctc ctgcacatct ccacccctgc gcaggaggag atccccaggc tgctctctcc      60
atctctccta cagctccctg caaacgaggg ggaagctgct gagagtccct atcactgctg     120
gccttttaat gttgtatgca aggaggaaga gggcgaggga taacttggtg ctggacaact     180
gacctgcggc ccgaagggcc tctggggagg gggtgcaaaa gaggagcggc tgggctgggg     240
gactccatgc gggggcgatg gacagggcgg cgctcctggg actggcccgc ttgtgcgcgc     300
tgtgggcagc cctgctcgtg ctgttcccct acggagccca aggaaactgg atgtggttgg     360
gcattgcctc cttcggggtt ccagagaagc tgggctgcgc caatttgccg ctgaacagcc     420
gccagaagga gctgtgcaag aggaaaccgt acctgctgcc gagcatccga gagggcgccc     480
ggctgggcat tcaggagtgc gggagccagt tcagacacga gagatggaac tgcatgatca     540
ccgccgccgc cactaccgcc ccgatgggcg ccagccccct ctttggctac gagctgagca     600
gcggcaccaa agagacagca tttatttatg ctgtgatggc tgcaggcctg gtgcattctg     660
tgaccaggtc atgcagtgca ggcaacatga cagagtgttc ctgtgacacc accttgcaga     720
acggcggctc agcaagtgaa ggctggcact gggggggctg ctccgatgat gtccagtatg     780
gcatgtggtt cagcagaaag ttcctagatt tccccatcgg aaacaccacg ggcaaagaaa     840
acaaagtact attagcaatg aacctacata acaatgaagc tggaaggcag gctgtcgcca     900
agttgatgtc agtagactgc cgctgccacg gagtttccgg ctcctgtgct gtgaaaacat     960
gctggaaaac catgtcttct tttgaaaaga ttggccattt gttgaaggat aaatatgaaa    1020
acagtatcca gatatcagac aaaacaaaga ggaaaatgcg caggagagaa aaagatcaga    1080
ggaaaatacc aatccataag gatgatctgc tctatgttaa taagtctccc aactactgtg    1140
tagaagataa gaaactggga atcccaggga cacaaggcag agaatgcaac cgtacatcag    1200
agggtgcaga tggctgcaac ctcctctgct gtggccgagg ttacaacacc catgtggtca    1260
ggcacgtgga gaggtgtgag tgtaagttca tctggtgctg ctatgtccgt tgcaggaggt    1320
gtgaaagcat gactgatgtc cacacttgca agtaaccact ccatccagcc ttgggcaaga    1380
tgcctcagca atatacaatg gcattgcaac cagagaggtg cccatccctg tgcagcgcta    1440
gtaaagttga ctcttgcagt ggaatcccta gaaccttgga cctgagagtt tcccttacct    1500
gatcgacata ttttccttta tctgatcaac ccatcaatca tgtggatttc ttgggattct    1560
aatgttgaaa aggtttatat tcaccttttg atgatttggg gaatatatat tgacatacaa    1620
ggaagataat ctgtttccta agcaagaaat aacaggaaag atcccttatg ccaggaggcc    1680
tgccatactc aggataagat ccttgaatat ggaacttagt tacaggactc aataatggtg    1740
ggtgaacatt agtcattttt aaaagacacc tcttatagca ataaggagac attaacatga    1800

atctcattta ttctctcagt attttaactg aagaaattat actgtttgtg tgtggataga 1860

agatgttgaa aagttaacat aagcattggg tgctgactta ccctttcatg tacttccaaa 1920

gaaaggtaat caaaaagaat cttcttaagt gatataatat ccctaaaaaa atgatcatta 1980

cagatgttta gtgacaaaga atcaatatgt aaaaagtata atgaatgatt tagattttaa 2040

gtgccttttc actgggagaa tctggaaaaa cctccataag gtatatagca atctttgatc 2100

tttagattca tacttttatc acagatcagt ttcaactgtt aaaaacccac ctctgagata 2160

ctggggggag gatcctgaaa catgcgggaa aaggagaggt aaacagtgga ggtaaaaata 2220

taatttcata cattgtaaag aaaagcaccc tttaaatgtg taaagacagt gttttgtaaa 2280

gaattttgtt taaaaagttt ctattttgta aatacagtac ttaagttata tgatttatat 2340

taaaacattt attgacaaag cctaagagct aaggcagtaa aattatctca taaataatat 2400

tagcttattt tttttcatac tattaatgct atttttttgg acatcgaaga gaatttaact 2460

tagcagttag ttatatggat gtgtatttct tgctaaaatg acagttttat atgttataga 2520

ttaaaatatg ttgcaaaata tcaaaaattt gtgttatttc agcagtaaga ttaattgaat 2580

tctcttttca cattagttat gcttaactca taaggttatt ataataaatt atattagtaa 2640

aagtcttaac tggaaaaaag aatctaaatc agaatagtga tcaatttgtg gatttgatat 2700

cctggatatt tattatattt tatgtaatgc tgcatttcta tttgaatgtt aagtggtctt 2760

tcttgttttt aatattcatg catgtatatt catcatattt tacaaggttc ctggtaaaaa 2820

ttacagggct ctatttaagg atgtatttta atgtaaatgc ttatgttttt tatgaattgt 2880

taaatatttc agtattatat agaaaaaaat agatttttaa aattcagaat ggacaaagag 2940

aatattcatt ttcttattaa taagataaag aaatgtttcc ctgccccaca gtcttcattc 3000

tatttctctt taattttatt cactgaggca gagaaacaat ttttgaaaaa gagcaaaccc 3060

atggaaaatg tctcagatct aatattaaaa tcaagactaa gcatttaact gtgaaaaaaa 3120

aaaaaaaaaa aa 3132

<210> 211
<211> 365
<212> PRT
<213> Homo sapiens

<400> 211
Met Asp Arg Ala Ala Leu Leu Gly Leu Ala Arg Leu Cys Ala Leu Trp
1               5                   10                  15

Ala Ala Leu Leu Val Leu Phe Pro Tyr Gly Ala Gln Gly Asn Trp Met
            20                  25                  30

Trp Leu Gly Ile Ala Ser Phe Gly Val Pro Glu Lys Leu Gly Cys Ala
        35                  40                  45

Asn Leu Pro Leu Asn Ser Arg Gln Lys Glu Leu Cys Lys Arg Lys Pro
    50                  55                  60

Tyr Leu Leu Pro Ser Ile Arg Glu Gly Ala Arg Leu Gly Ile Gln Glu
65                  70                  75                  80

Cys Gly Ser Gln Phe Arg His Glu Arg Trp Asn Cys Met Ile Thr Ala
                85                  90                  95

Ala Ala Thr Thr Ala Pro Met Gly Ala Ser Pro Leu Phe Gly Tyr Glu
            100                 105                 110

Leu Ser Ser Gly Thr Lys Glu Thr Ala Phe Ile Tyr Ala Val Met Ala
        115                 120                 125

Ala Gly Leu Val His Ser Val Thr Arg Ser Cys Ser Ala Gly Asn Met
    130                 135                 140

Thr Glu Cys Ser Cys Asp Thr Thr Leu Gln Asn Gly Gly Ser Ala Ser
145                 150                 155                 160

Glu Gly Trp His Trp Gly Gly Cys Ser Asp Asp Val Gln Tyr Gly Met
                165                 170                 175

Trp Phe Ser Arg Lys Phe Leu Asp Phe Pro Ile Gly Asn Thr Thr Gly
            180                 185                 190

Lys Glu Asn Lys Val Leu Leu Ala Met Asn Leu His Asn Asn Glu Ala
        195                 200                 205

Gly Arg Gln Ala Val Ala Lys Leu Met Ser Val Asp Cys Arg Cys His
    210                 215                 220

Gly Val Ser Gly Ser Cys Ala Val Lys Thr Cys Trp Lys Thr Met Ser
225                 230                 235                 240

Ser Phe Glu Lys Ile Gly His Leu Leu Lys Asp Lys Tyr Glu Asn Ser
                245                 250                 255

Ile Gln Ile Ser Asp Lys Thr Lys Arg Lys Met Arg Arg Arg Glu Lys
            260                 265                 270

Asp Gln Arg Lys Ile Pro Ile His Lys Asp Asp Leu Leu Tyr Val Asn

275                 280                 285

Lys Ser Pro Asn Tyr Cys Val Glu Asp Lys Lys Leu Gly Ile Pro Gly
    290                 295                 300

Thr Gln Gly Arg Glu Cys Asn Arg Thr Ser Glu Gly Ala Asp Gly Cys
305                 310                 315                 320

Asn Leu Leu Cys Cys Gly Arg Gly Tyr Asn Thr His Val Val Arg His
                325                 330                 335

Val Glu Arg Cys Glu Cys Lys Phe Ile Trp Cys Cys Tyr Val Arg Cys
            340                 345                 350

Arg Arg Cys Glu Ser Met Thr Asp Val His Thr Cys Lys
        355                 360                 365

<210> 212
<211> 4182
<212> DNA
<213> Homo sapiens

<220>
<221> modified_base
<222> (4174)..(4174)
<223> a, c, t, g, unknown or other

<400> 212
ccagtttcca cttttgagaa gcaggatgtt gaatgtgagt aatgacacaa caggcctata      60

aaatgcggac taaccctcgg atgactcatc cggtacaatg cagctactat ttgtcaagga     120

gactgaactc atactatggg ggaaaccact tctcaggcag ccagctggtt ctcaggtcag     180

caagagacga tccattttta tcggaagctt tatagtagca ataatgctaa tacccagcac     240

tcgggctcca caatgtagag gaaatggcat cgcctggcag tgacagctat attgtgcgtg     300

tcaaggctgt ggttatgacc agagatgact ccagcggggg atggttccca caggaaggag     360

gcgggatcag tcgcgtcggg gtctgtaagg tcatgcaccc cgaaggcaat ggacgaagcg     420

gctttctcat ccatggtgaa cgacagaaag acaaactggt ggtattggaa tgctatgtaa     480

gaaaggactt ggtctacacc aaagccaatc caacgtttca tcactggaag gtcgataata     540

ggaagtttgg acttactttc caaagccctg ctgatgcccg agcctttgac aggggagtaa     600

ggaaagcaat cgaagacctt atagaaggtt caacaacgtc atcttccacc atccataatg     660

aagctgagct tggcgatgat gacgttttta caacagctac agacagttct tctaattcct     720

ctcagaagag agagcaacct actcggacaa tctcctctcc cacatcctgt gagcaccgga     780

ggatttatac cctgggccac ctccacgact cataccccac agaccactat cacctcgatc 840

agccgatgcc aaggccctac cgccaggtga gcttcccgga cgacgacgag gagatcgtgc 900

gcatcaaccc ccgggagaag atctggatga cggggtacga ggattaccgg cacgcacccg 960

tcaggggcaa gtacccggac ccctcggagg acgcggactc ctcctacgtg cgcttcgcca 1020

agggcgaggt ccccaagcat gactacaact acccctacgt ggactcctca gactttggcc 1080

taggcgagga ccccaaaggc cgcgggggca gcgtgatcaa gacgcagccc tcccggggca 1140

agtcgcggcg gcggaaggag gacggagagc gctcgcggtg cgtgtactgc agggacatgt 1200

tcaaccacga ggagaaccgc cggggccact gccaggacgc gcccgactcc gtgagaactt 1260

gcatccgccg ggtgagctgc atgtggtgcg cggacagcat gctctatcac tgtatgtcgg 1320

accccgaggg agactataca gacccttgct cgtgcgatac tagcgacgag aagttttgcc 1380

tccggtggat ggctcttatt gccttgtctt tcctggcccc ctgtatgtgc tgttacctgc 1440

cccttcgggc ctgctaccac tgcggagtga tgtgcaggtg ctgtggcggg aagcacaaag 1500

cggccgcgtg actcagtttc cctcccttct ccctccatcc gcagccacag gggaactcgt 1560

ctcttacata ctctcatctt ctcccccgct cccttccact ccaaggagcg aggagggcaa 1620

gcggcctccc agctccctgg tacctcgagg caccattcca gccagggacg ctgccgggta 1680

gactctccac tccccctgcc gcccacactg cagcagccac atccatacac acacgctcgc 1740

acagtgttct gaggaaggaa ccttcgccac agactcctgt actattaaca atctgtaacc 1800

aagctaactg tctcatccat gtgttgattt cctgtttcct cctcccccgc ctcttccagt 1860

tcaaaggagt ctgcaattgg aactgctgat tttcggtggg ttttgtagtt gatttttcca 1920

agagcgtcga agactctctt tctcttggtt caccttgcct gtcgctagca agcatctggt 1980

tcagcggaaa tgggatgtga gaatgatgaa acccgacaga agtatctcag cctgcagtca 2040

gttattatgt ataggaggtg agctagttaa caaacttgta ccacaaaaca atatcgcttt 2100

aacttttcta aagccaaatt tcccatgtaa gctgcagttt ctatctttag cccatcatca 2160

ttttctgccc ccccaaaatc tgttgaaatg attcactgat gcaaaacatt cacccgtaat 2220

agactgagaa ttgagcctta acttcagatt aacttgtgag aatcaggaaa attccttcaa 2280

ctgcattgca tcctcttgga ccagggctag aatggggatt tcaggtttct atgagcctcc 2340

ccattacccc taaagtagaa cattttttaa attgtgttgc caccacttct aaaaatttag 2400

caatattaga ataggatact agtgaagtaa gaaattttgc ttgttgtttt gcaaaccaaa 2460

tagtttcctc aaacacaaat cagtgttccc acgaacaagt tccagttgag aaacactaag 2520

gttatggtga ataaaaccat agggagcttc ttccccccaa cccctggcta ttttatatta 2580

atggggagag gggattttta aatgtcataa atttgaagag tggtgggttg cattttcttc 2640

atgggtttat gttttcgttt cattttggac tcaatttcac atcaccaaat tcctcattta   2700

tacttgggga aaaaacaagg ccatatgtaa aaacccttcc aatgcctaag tgtctttctc   2760

ctgcaactcc aaacccagac tcgccacttt gggtgcacag gtggttaggt cagccaactg   2820

gttctgcctg tcgccttgcc acggaggagg cttctaatta gctggaaaag agtatttttc   2880

taatacgttg caaggattag ccaaatcttc ttattgaaga aagaagaaaa gtgaagagtg   2940

gttacctata cctagcatag tacaatcaga acctcgtgga gaccaccggg gacaggcttg   3000

cggacgccgg ctgttcttcc cgccacgatc tttctgtggt agcggccagc agaagacatg   3060

gcctgctccc cactcccttt ccccactcct tctttattgc acacaggaca ccagtcttca   3120

aggaaaggga cttttttcca gtctgccaat catattggga aagtgctagc tgtgctcacc   3180

ttcatggggc tgtttccagc tcgtccacaa gctcatcgat tttctttagt agattaccgg   3240

tgtaaatacc cagtgtgctt atgagtcagt tagtagacgt cttcattcat tggagtaact   3300

ggtttaggct ttccagtttg gaaaaggagc agagagctgt ccatctggat tgatggaaga   3360

aaagagaacc tcatccatgc ctggagaaca tctagaaaac cttcagccag cctccagtgc   3420

tgtcgagaga ccaccttccc ccgacccgga ggcacttcct tggggtcttt ctctagggtc   3480

tcttctctac aaagcacaac actaatgttc gtttccttag acctcagttc aagtgcccct   3540

atttatttca ataagaacgc acatatccca gctgtttttt gtttgtcacc tctatttagt   3600

tgttacctgt ttctctcttc tttcacccct tgtccttttc caccctttta agagttacgc   3660

tagcagatct tactccacgt atactttttg gtttgtgaag gcatcggtta agggcacaaa   3720

gacagccatg ggacatttta tgtaaatacg tctctaattg ccacactgca gctgaacagt   3780

gtgtagtatt ttcccagtca gctttgccat actgacgtca atcatttgag agaaattatt   3840

cagattttat ttttgtatct gtggtaacaa aacattaacc aaaagatttt ctgtccagaa   3900

gcctccccga ccccccaagc tatttgctca cattaacaaa ttaaagtgcc tgaagcataa   3960

ttcattcttt acctgtatac taaaaaccct gttgtattga tttttttata ataagccttt   4020

ttacctctgt gtaaaaaata tatatacaag tgtatgatgt acattttagt tcttaacttt   4080

tttttatggt ttctaatatg tatgaccaat gtagccattg ctttaaaatg taccgtgtaa   4140

atataaacac atcctatcag aaaagaaaaa atcnagaacg aa                     4182

<210> 213
<211> 418
<212> PRT
<213> Homo sapiens

<400> 213
Met Thr Glu Glu Thr His Pro Asp Asp Asp Ser Tyr Ile Val Arg Val

1               5                   10                  15

Lys Ala Val Val Met Thr Arg Asp Asp Ser Ser Gly Gly Trp Phe Pro
            20                  25                  30

Gln Glu Gly Gly Gly Ile Ser Arg Val Gly Val Cys Lys Val Met His
        35                  40                  45

Pro Glu Gly Asn Gly Arg Ser Gly Phe Leu Ile His Gly Glu Arg Gln
    50                  55                  60

Lys Asp Lys Leu Val Val Leu Glu Cys Tyr Val Arg Lys Asp Leu Val
65                  70                  75                  80

Tyr Thr Lys Ala Asn Pro Thr Phe His His Trp Lys Val Asp Asn Arg
                85                  90                  95

Lys Phe Gly Leu Thr Phe Gln Ser Pro Ala Asp Ala Arg Ala Phe Asp
            100                 105                 110

Arg Gly Val Arg Lys Ala Ile Glu Asp Leu Ile Glu Gly Ser Thr Thr
        115                 120                 125

Ser Ser Ser Thr Ile His Asn Glu Ala Glu Leu Gly Asp Asp Asp Val
    130                 135                 140

Phe Thr Thr Ala Thr Asp Ser Ser Ser Asn Ser Ser Gln Lys Arg Glu
145                 150                 155                 160

Gln Pro Thr Arg Thr Ile Ser Ser Pro Thr Ser Cys Glu His Arg Arg
                165                 170                 175

Ile Tyr Thr Leu Gly His Leu His Asp Ser Tyr Pro Thr Asp His Tyr
            180                 185                 190

His Leu Asp Gln Pro Met Pro Arg Pro Cys Arg Gln Val Ser Phe Pro
        195                 200                 205

Asp Asp Asp Glu Glu Ile Val Arg Ile Asn Pro Arg Glu Lys Ile Trp
    210                 215                 220

Met Thr Gly Tyr Glu Asp Tyr Arg His Ala Pro Val Arg Gly Lys Tyr
225                 230                 235                 240

Pro Asp Pro Ser Glu Asp Ala Asp Ser Ser Tyr Val Arg Phe Ala Lys
                245                 250                 255

Gly Glu Val Pro Lys His Asp Tyr Asn Tyr Pro Tyr Val Asp Ser Ser
            260                 265                 270

Asp Phe Gly Leu Gly Glu Asp Pro Lys Gly Arg Gly Gly Ser Val Ile
        275                 280                 285

Lys Thr Gln Pro Ser Arg Gly Lys Ser Arg Arg Arg Lys Glu Asp Gly
    290                 295                 300

Glu Arg Ser Arg Cys Val Tyr Cys Arg Asp Met Phe Asn His Glu Glu
305                 310                 315                 320

Asn Arg Arg Gly His Cys Gln Asp Ala Pro Asp Ser Val Arg Thr Cys
                325                 330                 335

Ile Arg Arg Val Ser Cys Met Trp Cys Ala Asp Ser Met Leu Tyr His
            340                 345                 350

Cys Met Ser Asp Pro Glu Gly Asp Tyr Thr Asp Pro Cys Ser Cys Asp
        355                 360                 365

Thr Ser Asp Glu Lys Phe Cys Leu Arg Trp Met Ala Leu Ile Ala Leu
    370                 375                 380

Ser Phe Leu Ala Pro Cys Met Cys Cys Tyr Leu Pro Leu Arg Ala Cys
385                 390                 395                 400

Tyr His Cys Gly Val Met Cys Arg Cys Cys Gly Gly Lys His Lys Ala
                405                 410                 415

Ala Ala

<210> 214
<211> 814
<212> DNA
<213> Homo sapiens

<220>
<221> modified_base
<222> (63)..(63)
<223> a, c, t, g, unknown or other

<220>
<221> modified_base
<222> (126)..(126)
<223> a, c, t, g, unknown or other

<220>
<221> modified_base
<222> (128)..(128)
<223> a, c, t, g, unknown or other

<220>
<221> modified_base
<222> (144)..(144)
<223> a, c, t, g, unknown or other

<220>
<221> modified_base
<222> (184)..(184)
<223> a, c, t, g, unknown or other

<220>
<221> modified_base
<222> (204)..(204)
<223> a, c, t, g, unknown or other

<220>
<221> modified_base
<222> (297)..(297)
<223> a, c, t, g, unknown or other

<400> 214
atttcagcac accaaagaaa attgagctgg caaaagatga atctttcaca agcagtgatg     60

atnatgaaaa tgtagattta gataaaagac ttcaatatta tagatatccg tggtcaactg    120

tgcatnancc tgcaaggaga ccantatcca tctccagaga ctggcatgga tataagaaga    180

aganccatac tattagtgta gganaagaca ttaattgtga cgtgatgatt cacagagacg    240

acaagtatag tatagtgagg gccccttctc catgctggat aatggtgaag caagacncat    300

gtaagctctt cctcctctac ctcctctacc tcagatgcat tttggctggg agattgtgcc    360

caagttgtat agagggtaaa gcccaactgg tatcaaaggt tggttagcta atctgtggtc    420

atatgagcat ttatcttgca gacacccaag ttttgtgcct caccaggcac aagtttgctg    480

tacttatcaa cggtctgtct gtagactcac caattctctt ctcttatgac tgcgttataa    540

agcctttaga gatgttcttc aacaggatta tctaaagact tccttgggtt cttgcaggcc    600

tcacaaatct tattttcaga ataagaccct cctttttgag aagaatttct ttcttttaga    660

aaatgccgta gagaaatcca atatcagaat gtctgaacat agtagagaat gtcactttat    720

gtaaacacta catttttctt taaatattta gtttctctct tttttttggt aaacttcaag    780

tactataatt aaaataacta agagacataa aaaa                                814


<210> 215
<211> 202
<212> PRT
<213> Homo sapiens

<400> 215
Met Ala Glu Gly Glu Glu Glu Gly Gly Asp Val Leu Ile Ser Val Gly
1               5                   10                  15

His Ala Asn Val Leu Gly Tyr Thr Leu Arg Glu Phe Leu Gln Leu Leu
            20                  25                  30

Gln His Ile Thr Ile Gly Thr Val Leu Gln Ile Lys Val Tyr Arg Asp
        35                  40                  45

Phe Ile Asn Ile Pro Glu Glu Trp Gln Glu Ile Tyr Asp Leu Ile Pro
    50                  55                  60

Glu Ala Lys Phe Pro Val Thr Ser Thr Pro Lys Lys Ile Glu Leu Ala
65                  70                  75                  80

Lys Asp Glu Ser Phe Thr Ser Ser Asp Asp Asn Glu Asn Val Asp Leu
                85                  90                  95

Asp Lys Arg Leu Gln Tyr Tyr Arg Tyr Pro Trp Ser Thr Val His His
            100                 105                 110

Pro Ala Arg Arg Pro Ile Ser Ile Ser Arg Asp Trp His Gly Tyr Lys
        115                 120                 125

Lys Lys Asn His Thr Ile Ser Val Gly Lys Asp Ile Asn Cys Asp Val
    130                 135                 140

Met Ile His Arg Asp Asp Lys Lys Glu Val Arg Ala Pro Ser Pro Tyr
145                 150                 155                 160

Trp Ile Met Val Lys Gln Asp Asn Glu Ser Ser Ser Ser Ser Thr Ser
                165                 170                 175

Ser Thr Ser Asp Ala Phe Trp Leu Glu Asp Cys Ala Gln Val Glu Glu
            180                 185                 190

Gly Lys Ala Gln Leu Val Ser Lys Val Gly
        195                 200

<210> 216
<211> 2337
<212> DNA
<213> Homo sapiens

<400> 216
agcggaggcg gcgcggggac gggcgctggg cggccgcgga gctccgggtg ccgccgcgtc      60

cccagcgccc cggccggccc ctctgggcgg cctgcggggg cggcgcagtt gcgaaactga 120
gtgcctgtgt agatcatcct agaatacctg tgtggtgctg tccttcctca agactacctc 180
attctcactg gctcggagac tcctgcctgc tgagctgact acacagactt agtcttctcc 240
actccgtgtt cctgcagcta gagacatgac ctaacaccct gatgaccact ctcagggacc 300
ttgagtgact ggccggtgca ccatggaact taaagtatgg gtggatggag ttcagaggat 360
tgtttgtgga gtcactgaag tcacaacttg ccaggaggtt gtcatagcct tagctcaagc 420
aataggtcga actggaaggt acacccttat agagaaatgg agagatactg aaagacactt 480
agcacctcat gaaaatccta tcatatcctt aaacaaatgg gggcagtatg ctagtgatgt 540
gcagctcatt ctacgacgaa ctgggccgtc tctcagtgag cgacccactt cagacagtgt 600
ggctcgaatt cctgaaagaa ctttatacag gcagagtctg ccccccttag ctaaactgag 660
gcctcagatt gacaaatcaa tcaaaaggag ggaaccgaaa aggaaatcac tgacatttac 720
aggaggtgcc aaaggattaa tggacatttt tggaaaaggt aaagaaactg agtttaagca 780
aaaggtgctg aataactgca aaacaacagc agatgagttg aagaagctaa tccgtctgca 840
gacagagaag cttcaatcca ttgagaaaca gctggaatct aatgaaatag aaataagatt 900
ttgggagcaa aagtataatt ccaaccttga agaggaaatt gtccgtctag agcaaaagat 960
caaaagaaac gatgtagaaa ttgaggagga agaattctgg gaaaatgaat tacagattga 1020
acaggaaaat gaaaaacagc tgaaggatca acttcaagaa ataagacaga aaataacaga 1080
atgtgaaaac aaattaaagg actatttggc acagatccgg actatggaaa gtggtcttga 1140
agcagaaaaa ttgcaacggg aagttcaaga ggcacaggtc aatgaggaag aggttaaagg 1200
aaagatcggt aaggtcaaag gggagattga cattcaaggc cagcagagtc tgaggttgga 1260
aaatggcatc aaagctgtgg aaagatctct tggacaagcc accaaacgct tacaggacaa 1320
agaacaggaa ctggagcagt tgactaagga gttgcggcaa gtcaatctcc agcagttcat 1380
ccagcagaca gggacaaaag ttaccgtttt gccagcggag cccattgaaa tagaggcctc 1440
acatgcagac attgaaaggg ggatcatcat tctttctgat aagcaggagt gtaaagatta 1500
gatatcacac caaaagctca ggcaagaaaa gcaaaaatga acaagtggga ctacattaaa 1560
cccaaaactc ctgcacaaca aaggaaacga tcaccaaaat gaaaaggtgg actgtagatt 1620
gggagaaaat atttgcaaac cgttatctgg taggggttta atatccaaac tatataagga 1680
actcacagaa cttaaagaaa acaaataatc caattaaaaa tggtcaaagg ggctgggcat 1740
ggtggctcac gcctgtaatc tcagcacttt gggaggccga ggtgggagaa tcacaaggtc 1800
aggagactga gaccatcctg gctaacacca tgatgggcta acacccatct ctactaaaaa 1860
tacgaaacat tagccaggtg tggtggcacg cgcctgtaat cccagctact cgggaggctg 1920

aggcagaagg attgcttgaa cccgggaggt ggaggttgca gtgagccgag atcgcattac 1980

tgcactccag cctgggtaac agagtgagac tccatctcaa aaaaaaaaaa aaaatagata 2040

tttttccaaa gaagacataa aaacagccaa caggtatatg aaaaagtgct caacatcact 2100

gatcagcagg gaaatgcaaa tcaaagccac aatgagatac cacctcacac ctgttaggtt 2160

acatctgcta ttgtcaaaaa gataagagat agcaagtgtc agcaagggtc tggagaaaag 2220

ggaatttttg tatactgttg gtgggaatgt aaatcagtac ggctattgtg gaaaacagtg 2280

tagaggtttc tcaaaaaatt aaaactatca tacaacccag caaaaaaaaa aaaaaaa 2337

<210> 217
<211> 392
<212> PRT
<213> Homo sapiens

<400> 217
Met Glu Leu Lys Val Trp Val Asp Gly Val Gln Arg Ile Val Cys Gly
1               5                   10                  15

Val Thr Glu Val Thr Thr Cys Gln Glu Val Val Ile Ala Leu Ala Gln
            20                  25                  30

Ala Ile Gly Arg Thr Gly Arg Tyr Thr Leu Ile Glu Lys Trp Arg Asp
        35                  40                  45

Thr Glu Arg His Leu Ala Pro His Glu Asn Pro Ile Ile Ser Leu Asn
    50                  55                  60

Lys Trp Gly Gln Tyr Ala Ser Asp Val Gln Leu Ile Leu Arg Arg Thr
65                  70                  75                  80

Gly Pro Ser Leu Ser Glu Arg Pro Thr Ser Asp Ser Val Ala Arg Ile
                85                  90                  95

Pro Glu Arg Thr Leu Tyr Arg Gln Ser Leu Pro Pro Leu Ala Lys Leu
            100                 105                 110

Arg Pro Gln Ile Asp Lys Ser Ile Lys Arg Arg Glu Pro Lys Arg Lys
        115                 120                 125

Ser Leu Thr Phe Thr Gly Gly Ala Lys Gly Leu Met Asp Ile Phe Gly
    130                 135                 140

Lys Gly Lys Glu Thr Glu Phe Lys Gln Lys Val Leu Asn Asn Cys Lys
145                 150                 155                 160

Thr Thr Ala Asp Glu Leu Lys Lys Leu Ile Arg Leu Gln Thr Glu Lys
                165                 170                 175

Leu Gln Ser Ile Glu Lys Gln Leu Glu Ser Asn Glu Ile Glu Ile Arg
            180                 185                 190

Phe Trp Glu Gln Lys Tyr Asn Ser Asn Leu Glu Glu Glu Ile Val Arg
        195                 200                 205

Leu Glu Gln Lys Ile Lys Arg Asn Asp Val Glu Ile Glu Glu Glu Glu
    210                 215                 220

Phe Trp Glu Asn Glu Leu Gln Ile Glu Gln Glu Asn Glu Lys Gln Leu
225                 230                 235                 240

Lys Asp Gln Leu Gln Glu Ile Arg Gln Lys Ile Thr Glu Cys Glu Asn
                245                 250                 255

Lys Leu Lys Asp Tyr Leu Ala Gln Ile Arg Thr Met Glu Ser Gly Leu
            260                 265                 270

Glu Ala Glu Lys Leu Gln Arg Glu Val Gln Glu Ala Gln Val Asn Glu
        275                 280                 285

Glu Glu Val Lys Gly Lys Ile Gly Lys Val Lys Gly Glu Ile Asp Ile
    290                 295                 300

Gln Gly Gln Gln Ser Leu Arg Leu Glu Asn Gly Ile Lys Ala Val Glu
305                 310                 315                 320

Arg Ser Leu Gly Gln Ala Thr Lys Arg Leu Gln Asp Lys Glu Gln Glu
                325                 330                 335

Leu Glu Gln Leu Thr Lys Glu Leu Arg Gln Val Asn Leu Gln Gln Phe
            340                 345                 350

Ile Gln Gln Thr Gly Thr Lys Val Thr Val Leu Pro Ala Glu Pro Ile
        355                 360                 365

Glu Ile Glu Ala Ser His Ala Asp Ile Glu Arg Gly Ile Ile Ile Leu
    370                 375                 380

Ser Asp Lys Gln Glu Cys Lys Asp
385                 390

<210> 218
<211> 7604
<212> DNA
<213> Homo sapiens

<400> 218
cgtgagtgtc tgtgcgtttt gatcccgaag ctcctcgccg ggtcgttact tcagaagaga 60

agacagtgag cgtgggcgag gcgaggcatc cagaggcccc ggcgacccct gctccccagt 120

cctccgcccc ctcgcgtcgc ggccctgggc gggcgccgcg agccggagtt ccattttaat 180

tgctgttaat catttcagag aagaacactg aactttgaaa aaaatgttgg aagccattga 240

caaaaatcgg gccctgcatg cagcagagcg cttgcaaacc aagctgcgag aacgtgggga 300

tgtagcaaat gaagacaaac tgagccttct gaagtcagtc ctgcagagcc ctctcttcag 360

tcagattctg agccttcaga cttctgtaca gcagctgaaa gaccaggtaa atattgcaac 420

ttcagcaact tcaaatattg aatatgccca cgttcctcat ctcagcccag ctgtgattcc 480

tactctgcaa aatgaatcgt ttttattatc cccaaacaat gggaatctgg aagcacttac 540

aggacctggt attccacaca ttaatgggaa acctgcttgt gatgaatttg atcagcttat 600

caaaaatatg gcccagggtc gccatgtaga agtttttgag ctcctcaaac ctccatctgg 660

aggccttggg tttagtgttg tgggactaag aagtgaaaac agaggagagc tgggaatatt 720

tgttcaagag atacaagagg gcagtgtggc ccatagagat ggaagattga aagaaactga 780

tcaaattctt gctatcaatg gacaggctct tgatcagaca attacacatc agcaggctat 840

cagcatcctg cagaaagcca aagatactgt ccagctagtt attgccagag gctcattgcc 900

tcagcttgtc agccccatag tttcccgttc tccatctgca gccagcacaa tttcagctca 960

ctctaatccg gttcactggc aacacatgga aacgattgaa ttggtgaatg atggatctgg 1020

tttgggattt ggcatcatag gaggaaaagc aactggtgtg atagtaaaaa ccattctgcc 1080

tggaggagta gctgatcagc atgggcgttt atgcagtgga gaccacattc taaagattgg 1140

tgacacagat ctagcaggaa tgagcagtga gcaagtagca caagtcctta ggcaatgtgg 1200

aaatagagtt aagttgatga ttgcaagagg tgccatagaa gaacgtacag cacccactgc 1260

tttgggcatc accctctcct catccccaac ttcaacacca gagttgcggg ttgatgcttc 1320

tactcagaaa ggtgaagaaa gtgagacatt tgatgtagaa ctcactaaaa atgtccaagg 1380

attaggaatt accattgctg gctacattgg agataaaaaa ttggaacctt caggaatctt 1440

tgtaaagagc attacaaaaa gcagtgccgt tgagcatgat ggaagaatcc aaattggaga 1500

ccaaattata gcagtagatg gcacaaacct tcagggtttt actaatcagc aagcagtaga 1560

ggtattgcga catacaggac aaactgtgct cctgacacta atgaggagag gaatgaagca 1620

ggaagccgag ctcatgtcaa gggaagacgt cacaaaagat gcagatttgt ctcctgttaa 1680

tgccagcata atcaaagaaa attatgaaaa agatgaagat tttttatctt cgacgagaaa 1740

caccaacata ttaccaactg aagaagaagg gtatccatta ctgtcagctg agatagaaga 1800

aatagaagat gcacaaaaac aagaagctgc tctgctgaca aaatggcaaa ggattatggg 1860

aattaactat gaaatagtgg tggcccatgt gagcaagttt agtgagaaca gtggattggg 1920

gataagcctg gaagcgacag tgggacatca ttttatccga tctgttctac cagagggtcc 1980

tgttggacac agcgggaagc tcttcagtgg agacgagcta ttggaagtaa atggcataac 2040

tttacttggg gaaaatcacc aagatgtggt gaatatctta aaagaactgc ctatagaagt 2100

gacaatggtg tgctgtcgtc gaactgtgcc acccaccacc caatcagaat tggatagcct 2160

ggacttatgt gatattgagc taacagaaaa gcctcacgta gatctaggtg agttcatcgg 2220

gtcatcagag acagaggatc cagtgctggc gatgactgat gcgggtcaga gtacagaaga 2280

ggttcaagca cctttggcca tgtgggaggc tggcattcag cacatagagc tggagaaagg 2340

gagcaaagga cttggtttta gcattttaga ttatcaggat ccaattgatc cagcaagcac 2400

tgtgattata attcgttctt tggtgcctgg cggcattgct gaaaaggatg gacgacttct 2460

tcctggtgac cgactcatgt ttgtaaacga tgttaacttg gaaaacagca gtcttgagga 2520

agctgtagaa gcactgaagg gagcaccgtc agggactgtg agaataggag ttgctaagcc 2580

tttacccctt tcaccagaag aaggttatgt ttctgctaag gaggattcct ttctctaccc 2640

accacactcc tgtgaggaag cagggctggc tgacaaaccc ctcttcaggg ctgacttggc 2700

tctggtgggc acaaatgatg ctgacttagt agatgaatcc acatttgagt ctccatactc 2760

tcctgaaaat gacagcatct actctactca agcctctatt ttatctcttc atggcagttc 2820

ttgtggtgat ggcctgaact atggttcttc ccttccatca tctcctccta aggatgttat 2880

tgaaaattct tgtgatccag tacttgatct gcatatgtct ctggaggaac tatataccca 2940

gaatctcctg caaagacagg atgagaatac accttcggtg gacataagta tggggcctgc 3000

ttctggcttt actataaatg actacacacc tgcaaatgct attgaacaac aatatgaatg 3060

tgaaaacaca atagtgtgga ctgaatctca tttaccaagt gaagttatat caagtgcaga 3120

acttccttct gtgctacccg attcagctgg aaagggctct gagtacctgc ttgaacagag 3180

ctccctggcc tgtaatgctg agtgtgtcat gcttcaaaat gtatctaaag aatcttttga 3240

aaggactatt aatatagcaa aaggcaattc tagcctagga atgacagtta gtgctaataa 3300

agatggcttg gggatgatcg ttcgaagcat tattcatgga ggtgccatta gtcgagatgg 3360

ccggattgcc attggggact gcatcttgtc cattaatgaa gagtctacca tcagtgtaac 3420

caatgcccag gcacgagcta tgttgagaag acattctctc attggccctg acataaaaat 3480

tacttatgtg cctgcagaac atttggaaga gttcaaaata agcttgggac aacaatctgg 3540

aagagtaatg gcactggata ttttttcttc atacactggc agagacattc cagaattacc 3600

agagcgagaa gagggagagg gtgaagaaag cgaacttcaa aacacagcat atagcaattg 3660

gaatcagccc aggcgggtgg aactctggag agaaccaagc aaatccttag gcatcagcat 3720

tgttggtgga cgagggatgg ggagtcggct aagcaatgga gaagtgatga ggggcatttt 3780

catcaaacat gttctggaag atagtccagc tggcaaaaat ggaaccttga aacctggaga 3840

tagaatcgta gaggtggatg gaatggacct cagagatgca agccatgaac aagctgtgga 3900

agccattcgg aaagcaggca accctgtagt ctttatggta cagagcatta taaacagacc 3960

aaggaaatcc cctttgcctt ccttgctgca caacctttac cctaagtaca acttcagcag 4020

cactaaccca tttgctgact ctctacaaat caacgccgac aaggcaccca gtcagtcaga 4080

gtcagagcca gagaaggctc cattgtgcag tgtgccccca cccccctcct cagcctttgc 4140

cgaaatgggt agtgatcaca cacagtcatc tgcaagcaaa atctcacaag atgtggacaa 4200

agaggatgag tttggttaca gctggaaaaa tatcagagag cgttatggaa ccctaacagg 4260

cgagctgcat atgattgaac tggagaaagg tcatagtggt ttgggcctaa gtcttgctgg 4320

gaacaaagac cgatccagga tgagtgtctt catagtgggg attgatccaa atggagctgc 4380

aggaaaagat ggtcgattgc aaattgcaga tgagcttcta gagatcaatg gtcagatttt 4440

atatggaaga agtcatcaga atgcctcatc aatcattaaa tgtgcccctt ctaaagtgaa 4500

aataattttt atcagaaata aagatgcagt gaatcagatg gccgtatgtc ctggaaatgc 4560

agtagaacct ttgccttcta actcagaaaa tcttcaaaat aaggagacag agccaactgt 4620

tactacttct gatgcagctg tggacctcag ttcatttaaa aatgtgcaac atctggagct 4680

tcccaaggat caggggggtt tgggtattgc tatcagcgaa gaagatacac tcagtggagt 4740

catcataaag agcttaacag agcatggggt agcagccacg gatggacgac tcaaagtcgg 4800

agatcagata ctggctgtag atgatgaaat tgttgttggt taccctattg aaaagtttat 4860

tagccttctg aagacagcaa agatgacagt aaaacttacc atccatgctg agaatccaga 4920

ttcccaggct gttccttcag cagctggtgc agccagtgga gaaaaaaaga acagctccca 4980

gtctctgatg gtcccacagt ctggctcccc agaaccggag tccatccgaa atacaagcag 5040

atcatcaaca ccagcaattt ttgcttctga tcctgcaacc tgccccatta tccctggctg 5100

cgaaacaacc atcgagattt ccaaagggcg aacagggctg ggcctgagca tcgttggggg 5160

ttcagacacg ctgctgggtg ccattattat ccatgaagtt tatgaagaag gagcagcatg 5220

taaagatgga agactctggg ctggagatca gatcttagag gtgaatggaa ttgacttgag 5280

aaaggccaca catgatgaag caatcaatgt cctgagacag acgccacaga gagtgcgcct 5340

gacactctac agagatgagg ccccatacaa agaggaggaa gtgtgtgaca ccctcactat 5400

tgagctgcag aagaagccgg gaaaaggcct aggattaagt attgttggta aaagaaacga 5460

tactggagta tttgtgtcag acattgtcaa aggaggaatt gcagatgccg atggaagact 5520

gatgcaggga gaccagatat taatggtgaa tggggaagac gttcgtaatg ccacccaaga 5580

agcggttgcc gctttgctaa aggtgagtga aggcagcctg tcatctttca cttttccact 5640

ctctggatcc agtacatctg agtcactgga aagtagctca aagaagaatg cattggcatc 5700

tgaaatacag ggattaagaa cagtcgaaat gaaaaagggc cctactgact cactgggaat 5760

cagcatcgct ggaggagtag gcagcccact tggtgatgtg cctatattta ttgcaatgat 5820

gcacccaact ggagttgcag cacagaccca aaaactcaga gttggggata ggattgtcac 5880

catctgtggc acatccactg agggcatgac tcacacccaa gcagttaacc tactgaaaaa 5940

tgcatctggc tccattgaaa tgcaggtggt tgctggagga gacgtgagtg tggtcacagg 6000

tcatcagcag gagcctgcaa gttccagtct ttctttcact gggctgacgt caagcagtat 6060

atttcaggat gatttaggac ctcctcaatg taagtctatt acactagagc gaggaccaga 6120

tggcttaggc ttcagtatag ttggaggata tggcagccct catggagact tacccattta 6180

tgttaaaaca gtgtttgcaa agggagcagc ctctgaagac ggacgtctga aaaggggcga 6240

tcagatcatt gctgtcaatg ggcagagtct agaaggagtc acccatgaag aagctgttgc 6300

catccttaaa cggacaaaag gcactgtcac tttgatggtt ctctcttgaa ttggctgcca 6360

gaattgaacc aacccaaccc ctagctcacc tcctactgta aagagaatgc actggtcctg 6420

acaattttta tgctgtgttc agccgggtct tcaaaactgt aggggggaaa taacacttaa 6480

gtttcttttt ctcatctaga aatgctttcc ttactgacaa cctaacatca tttttctttt 6540

cttcttgcat tttgtgaact taaagagaag gaatatttgt gtaggtgaat ctcgttttta 6600

tttgtggaga tatctaatgt tttgtagtca catgggcaag aattattaca tgctaagctg 6660

gttagtataa agaaagataa ttctaaagct aaccaaagaa aatggcttca gtaagttagg 6720

atgaaaaatg aaaatataaa ataaagaaga aaatctcggg gagtttaaaa aaaatgcctc 6780

aatttggcaa tctacctcct ctccccaccc caaactaaaa aaagaaaaaa aggttttcta 6840

atgaaaatct ttaaaaatac tgtcagtatt ttaaaatttt caacagtatt ataaaaacat 6900

tgcatctccc cacctctaat atgcatatat atttttcctg ctaaaattgg tttctacaat 6960

tgagtaaatg gcaaatacat gaagcaatgt ccctaaattt tataaagaaa ttatatttaa 7020

tgcacatttc aattttcatt cttatttttg accttttgta aaatattttc atgttgctat 7080

aagtaaatga tgatgccacc ccatgttgac tatggttttt ctagaaagca actatgctgc 7140

taaccataga ggaacataga agggttccag aatctttagt gctggtttta acaaccgatg 7200

caacattaaa aatgtgttag tgtgctgtgc aattggtttt caattcatat taatcttaat 7260

gacagagaac aatgtgttac taattatttt ggttgtatgc cattagtaaa ttgatagaaa 7320

aattaagggg attaacataa cttcatttca ttgccttata ttaacatctt ataatacaat 7380

agtttaagac taagggaaac agatggagct gtttattgag acaactggtg aggaattatc 7440

atgtgttcat tcccatttta gagcgtgaaa ctcctacatt agaatatata aagtcacttt 7500

aaatatctat atttgtaaca gaagtagtgt acagatattt tattacagca tttttgtgta 7560

aatgcagaat taaagtgaat aaataagaat tttcagtggt gcac 7604

<210> 219
<211> 2045
<212> PRT
<213> Homo sapiens

<400> 219
Thr Leu Lys Lys Met Leu Glu Ala Ile Asp Lys Asn Arg Ala Leu His
1               5                   10                  15

Ala Ala Glu Arg Leu Gln Thr Lys Leu Arg Glu Arg Gly Asp Val Ala
            20                  25                  30

Asn Glu Asp Lys Leu Ser Leu Leu Lys Ser Val Leu Gln Ser Pro Leu
        35                  40                  45

Phe Ser Gln Ile Leu Ser Leu Gln Thr Ser Val Gln Gln Leu Lys Asp
    50                  55                  60

Gln Val Asn Ile Ala Thr Ser Ala Thr Ser Asn Ile Glu Tyr Ala His
65                  70                  75                  80

Val Pro His Leu Ser Pro Ala Val Ile Pro Thr Leu Gln Asn Glu Ser
                85                  90                  95

Phe Leu Leu Ser Pro Asn Asn Gly Asn Leu Glu Ala Leu Thr Gly Pro
            100                 105                 110

Gly Ile Pro His Ile Asn Gly Lys Pro Ala Cys Asp Glu Phe Asp Gln
        115                 120                 125

Leu Ile Lys Asn Met Ala Gln Gly Arg His Val Glu Val Phe Glu Leu
    130                 135                 140

Leu Lys Pro Pro Ser Gly Gly Leu Gly Phe Ser Val Val Gly Leu Arg
145                 150                 155                 160

Ser Glu Asn Arg Gly Glu Leu Gly Ile Phe Val Gln Glu Ile Gln Glu
                165                 170                 175

Gly Ser Val Ala His Arg Asp Gly Arg Leu Lys Glu Thr Asp Gln Ile
            180                 185                 190

Leu Ala Ile Asn Gly Gln Ala Leu Asp Gln Thr Ile Thr His Gln Gln
        195                 200                 205

Ala Ile Ser Ile Leu Gln Lys Ala Lys Asp Thr Val Gln Leu Val Ile
    210                 215                 220

Ala Arg Gly Ser Leu Pro Gln Leu Val Ser Pro Ile Val Ser Arg Ser
225                 230                 235                 240

Pro Ser Ala Ala Ser Thr Ile Ser Ala His Ser Asn Pro Val His Trp
                245                 250                 255

Gln His Met Glu Thr Ile Glu Leu Val Asn Asp Gly Ser Gly Leu Gly
            260                 265                 270

Phe Gly Ile Ile Gly Gly Lys Ala Thr Gly Val Ile Val Lys Thr Ile
        275                 280                 285

Leu Pro Gly Gly Val Ala Asp Gln His Gly Arg Leu Cys Ser Gly Asp
    290                 295                 300

His Ile Leu Lys Ile Gly Asp Thr Asp Leu Ala Gly Met Ser Ser Glu
305                 310                 315                 320

Gln Val Ala Gln Val Leu Arg Gln Cys Gly Asn Arg Val Lys Leu Met
                325                 330                 335

Ile Ala Arg Gly Ala Ile Glu Glu Arg Thr Ala Pro Thr Ala Leu Gly
            340                 345                 350

Ile Thr Leu Ser Ser Ser Pro Thr Ser Thr Pro Glu Leu Arg Val Asp
        355                 360                 365

Ala Ser Thr Gln Lys Gly Glu Glu Ser Glu Thr Phe Asp Val Glu Leu
    370                 375                 380

Thr Lys Asn Val Gln Gly Leu Gly Ile Thr Ile Ala Gly Tyr Ile Gly
385                 390                 395                 400

Asp Lys Lys Leu Glu Pro Ser Gly Ile Phe Val Lys Ser Ile Thr Lys
                405                 410                 415

Ser Ser Ala Val Glu His Asp Gly Arg Ile Gln Ile Gly Asp Gln Ile
            420                 425                 430

Ile Ala Val Asp Gly Thr Asn Leu Gln Gly Phe Thr Asn Gln Gln Ala
        435                 440                 445

Val Glu Val Leu Arg His Thr Gly Gln Thr Val Leu Leu Thr Leu Met
    450                 455                 460

Arg Arg Gly Met Lys Gln Glu Ala Glu Leu Met Ser Arg Glu Asp Val
465                 470                 475                 480

Thr Lys Asp Ala Asp Leu Ser Pro Val Asn Ala Ser Ile Ile Lys Glu
                485                 490                 495

Asn Tyr Glu Lys Asp Glu Asp Phe Leu Ser Ser Thr Arg Asn Thr Asn
            500                 505                 510

Ile Leu Pro Thr Glu Glu Glu Gly Tyr Pro Leu Leu Ser Ala Glu Ile
        515                 520                 525

Glu Glu Ile Glu Asp Ala Gln Lys Gln Glu Ala Ala Leu Leu Thr Lys
    530                 535                 540

Trp Gln Arg Ile Met Gly Ile Asn Tyr Glu Ile Val Val Ala His Val
545                 550                 555                 560

Ser Lys Phe Ser Glu Asn Ser Gly Leu Gly Ile Ser Leu Glu Ala Thr
                565                 570                 575

Val Gly His His Phe Ile Arg Ser Val Leu Pro Glu Gly Pro Val Gly
            580                 585                 590

His Ser Gly Lys Leu Phe Ser Gly Asp Glu Leu Leu Glu Val Asn Gly
        595                 600                 605

Ile Thr Leu Leu Gly Glu Asn His Gln Asp Val Val Asn Ile Leu Lys
    610                 615                 620

Glu Leu Pro Ile Glu Val Thr Met Val Cys Cys Arg Arg Thr Val Pro
625                 630                 635                 640

Pro Thr Thr Gln Ser Glu Leu Asp Ser Leu Asp Leu Cys Asp Ile Glu
                645                 650                 655

Leu Thr Glu Lys Pro His Val Asp Leu Gly Glu Phe Ile Gly Ser Ser
            660                 665                 670

Glu Thr Glu Asp Pro Val Leu Ala Met Thr Asp Ala Gly Gln Ser Thr
        675                 680                 685

Glu Glu Val Gln Ala Pro Leu Ala Met Trp Glu Ala Gly Ile Gln His
    690                 695                 700

Ile Glu Leu Glu Lys Gly Ser Lys Gly Leu Gly Phe Ser Ile Leu Asp
705                 710                 715                 720

Tyr Gln Asp Pro Ile Asp Pro Ala Ser Thr Val Ile Ile Ile Arg Ser
                725                 730                 735

Leu Val Pro Gly Gly Ile Ala Glu Lys Asp Gly Arg Leu Leu Pro Gly
            740                 745                 750

Asp Arg Leu Met Phe Val Asn Asp Val Asn Leu Glu Asn Ser Ser Leu
        755                 760                 765

Glu Glu Ala Val Glu Ala Leu Lys Gly Ala Pro Ser Gly Thr Val Arg
    770                 775                 780

Ile Gly Val Ala Lys Pro Leu Pro Leu Ser Pro Glu Glu Gly Tyr Val
785                 790                 795                 800

Ser Ala Lys Glu Asp Ser Phe Leu Tyr Pro Pro His Ser Cys Glu Glu
                805                 810                 815

Ala Gly Leu Ala Asp Lys Pro Leu Phe Arg Ala Asp Leu Ala Leu Val
            820                 825                 830

Gly Thr Asn Asp Ala Asp Leu Val Asp Glu Ser Thr Phe Glu Ser Pro
        835                 840                 845

Tyr Ser Pro Glu Asn Asp Ser Ile Tyr Ser Thr Gln Ala Ser Ile Leu
    850                 855                 860

Ser Leu His Gly Ser Ser Cys Gly Asp Gly Leu Asn Tyr Gly Ser Ser
865                 870                 875                 880

Leu Pro Ser Ser Pro Pro Lys Asp Val Ile Glu Asn Ser Cys Asp Pro

885                 890                 895

Val Leu Asp Leu His Met Ser Leu Glu Glu Leu Tyr Thr Gln Asn Leu
            900                 905                 910

Leu Gln Arg Gln Asp Glu Asn Thr Pro Ser Val Asp Ile Ser Met Gly
        915                 920                 925

Pro Ala Ser Gly Phe Thr Ile Asn Asp Tyr Thr Pro Ala Asn Ala Ile
    930                 935                 940

Glu Gln Gln Tyr Glu Cys Glu Asn Thr Ile Val Trp Thr Glu Ser His
945                 950                 955                 960

Leu Pro Ser Glu Val Ile Ser Ser Ala Glu Leu Pro Ser Val Leu Pro
                965                 970                 975

Asp Ser Ala Gly Lys Gly Ser Glu Tyr Leu Leu Glu Gln Ser Ser Leu
            980                 985                 990

Ala Cys Asn Ala Glu Cys Val Met  Leu Gln Asn Val Ser  Lys Glu Ser
        995                 1000                1005

Phe Glu  Arg Thr Ile Asn Ile  Ala Lys Gly Asn Ser  Ser Leu Gly
    1010                1015                1020

Met Thr  Val Ser Ala Asn Lys  Asp Gly Leu Gly Met  Ile Val Arg
    1025                1030                1035

Ser Ile  Ile His Gly Gly Ala  Ile Ser Arg Asp Gly  Arg Ile Ala
    1040                1045                1050

Ile Gly  Asp Cys Ile Leu Ser  Ile Asn Glu Glu Ser  Thr Ile Ser
    1055                1060                1065

Val Thr  Asn Ala Gln Ala Arg  Ala Met Leu Arg Arg  His Ser Leu
    1070                1075                1080

Ile Gly  Pro Asp Ile Lys Ile  Thr Tyr Val Pro Ala  Glu His Leu
    1085                1090                1095

Glu Glu  Phe Lys Ile Ser Leu  Gly Gln Gln Ser Gly  Arg Val Met
    1100                1105                1110

Ala Leu  Asp Ile Phe Ser Ser  Tyr Thr Gly Arg Asp  Ile Pro Glu
    1115                1120                1125

Leu Pro Glu Arg Glu Glu Gly Glu Gly Glu Glu Ser Glu Leu Gln
    1130                1135                1140

Asn Thr Ala Tyr Ser Asn Trp Asn Gln Pro Arg Arg Val Glu Leu
    1145                1150                1155

Trp Arg Glu Pro Ser Lys Ser Leu Gly Ile Ser Ile Val Gly Gly
    1160                1165                1170

Arg Gly Met Gly Ser Arg Leu Ser Asn Gly Glu Val Met Arg Gly
    1175                1180                1185

Ile Phe Ile Lys His Val Leu Glu Asp Ser Pro Ala Gly Lys Asn
    1190                1195                1200

Gly Thr Leu Lys Pro Gly Asp Arg Ile Val Glu Val Asp Gly Met
    1205                1210                1215

Asp Leu Arg Asp Ala Ser His Glu Gln Ala Val Glu Ala Ile Arg
    1220                1225                1230

Lys Ala Gly Asn Pro Val Val Phe Met Val Gln Ser Ile Ile Asn
    1235                1240                1245

Arg Pro Arg Lys Ser Pro Leu Pro Ser Leu Leu His Asn Leu Tyr
    1250                1255                1260

Pro Lys Tyr Asn Phe Ser Ser Thr Asn Pro Phe Ala Asp Ser Leu
    1265                1270                1275

Gln Ile Asn Ala Asp Lys Ala Pro Ser Gln Ser Glu Ser Glu Pro
    1280                1285                1290

Glu Lys Ala Pro Leu Cys Ser Val Pro Pro Pro Pro Pro Ser Ala
    1295                1300                1305

Phe Ala Glu Met Gly Ser Asp His Thr Gln Ser Ser Ala Ser Lys
    1310                1315                1320

Ile Ser Gln Asp Val Asp Lys Glu Asp Glu Phe Gly Tyr Ser Trp
    1325                1330                1335

Lys Asn Ile Arg Glu Arg Tyr Gly Thr Leu Thr Gly Glu Leu His
    1340                1345                1350

Met Ile Glu Leu Glu Lys Gly His Ser Gly Leu Gly Leu Ser Leu
    1355                1360                1365

Ala Gly Asn Lys Asp Arg Ser Arg Met Ser Val Phe Ile Val Gly
    1370                1375                1380

Ile Asp Pro Asn Gly Ala Ala Gly Lys Asp Gly Arg Leu Gln Ile
    1385                1390                1395

Ala Asp Glu Leu Leu Glu Ile Asn Gly Gln Ile Leu Tyr Gly Arg
    1400                1405                1410

Ser His Gln Asn Ala Ser Ser Ile Ile Lys Cys Ala Pro Ser Lys
    1415                1420                1425

Val Lys Ile Ile Phe Ile Arg Asn Lys Asp Ala Val Asn Gln Met
    1430                1435                1440

Ala Val Cys Pro Gly Asn Ala Val Glu Pro Leu Pro Ser Asn Ser
    1445                1450                1455

Glu Asn Leu Gln Asn Lys Glu Thr Glu Pro Thr Val Thr Thr Ser
    1460                1465                1470

Asp Ala Ala Val Asp Leu Ser Ser Phe Lys Asn Val Gln His Leu
    1475                1480                1485

Glu Leu Pro Lys Asp Gln Gly Gly Leu Gly Ile Ala Ile Ser Glu
    1490                1495                1500

Glu Asp Thr Leu Ser Gly Val Ile Ile Lys Ser Leu Thr Glu His
    1505                1510                1515

Gly Val Ala Ala Thr Asp Gly Arg Leu Lys Val Gly Asp Gln Ile
    1520                1525                1530

Leu Ala Val Asp Asp Glu Ile Val Val Gly Tyr Pro Ile Glu Lys
    1535                1540                1545

Phe Ile Ser Leu Leu Lys Thr Ala Lys Met Thr Val Lys Leu Thr
    1550                1555                1560

Ile His Ala Glu Asn Pro Asp Ser Gln Ala Val Pro Ser Ala Ala
    1565                1570                1575

Gly Ala Ala Ser Gly Glu Lys Lys Asn Ser Ser Gln Ser Leu Met
    1580                1585                1590

Val Pro Gln Ser Gly Ser Pro Glu Pro Glu Ser Ile Arg Asn Thr
    1595                1600                1605

Ser Arg Ser Ser Thr Pro Ala Ile Phe Ala Ser Asp Pro Ala Thr
    1610                1615                1620

Cys Pro Ile Ile Pro Gly Cys Glu Thr Thr Ile Glu Ile Ser Lys
    1625                1630                1635

Gly Arg Thr Gly Leu Gly Leu Ser Ile Val Gly Gly Ser Asp Thr
    1640                1645                1650

Leu Leu Gly Ala Ile Ile Ile His Glu Val Tyr Glu Glu Gly Ala
    1655                1660                1665

Ala Cys Lys Asp Gly Arg Leu Trp Ala Gly Asp Gln Ile Leu Glu
    1670                1675                1680

Val Asn Gly Ile Asp Leu Arg Lys Ala Thr His Asp Glu Ala Ile
    1685                1690                1695

Asn Val Leu Arg Gln Thr Pro Gln Arg Val Arg Leu Thr Leu Tyr
    1700                1705                1710

Arg Asp Glu Ala Pro Tyr Lys Glu Glu Glu Val Cys Asp Thr Leu
    1715                1720                1725

Thr Ile Glu Leu Gln Lys Lys Pro Gly Lys Gly Leu Gly Leu Ser
    1730                1735                1740

Ile Val Gly Lys Arg Asn Asp Thr Gly Val Phe Val Ser Asp Ile
    1745                1750                1755

Val Lys Gly Gly Ile Ala Asp Ala Asp Gly Arg Leu Met Gln Gly
    1760                1765                1770

Asp Gln Ile Leu Met Val Asn Gly Glu Asp Val Arg Asn Ala Thr
    1775                1780                1785

Gln Glu Ala Val Ala Ala Leu Leu Lys Val Ser Glu Gly Ser Leu
    1790                1795                1800

Ser Ser Phe Thr Phe Pro Leu Ser Gly Ser Ser Thr Ser Glu Ser

1805                1810                1815

Leu Glu Ser Ser Ser Lys Lys Asn Ala Leu Ala Ser Glu Ile Gln
    1820                1825                1830

Gly Leu Arg Thr Val Glu Met Lys Lys Gly Pro Thr Asp Ser Leu
    1835                1840                1845

Gly Ile Ser Ile Ala Gly Gly Val Gly Ser Pro Leu Gly Asp Val
    1850                1855                1860

Pro Ile Phe Ile Ala Met Met His Pro Thr Gly Val Ala Ala Gln
    1865                1870                1875

Thr Gln Lys Leu Arg Val Gly Asp Arg Ile Val Thr Ile Cys Gly
    1880                1885                1890

Thr Ser Thr Glu Gly Met Thr His Thr Gln Ala Val Asn Leu Leu
    1895                1900                1905

Lys Asn Ala Ser Gly Ser Ile Glu Met Gln Val Val Ala Gly Gly
    1910                1915                1920

Asp Val Ser Val Val Thr Gly His Gln Gln Glu Pro Ala Ser Ser
    1925                1930                1935

Ser Leu Ser Phe Thr Gly Leu Thr Ser Ser Ser Ile Phe Gln Asp
    1940                1945                1950

Asp Leu Gly Pro Pro Gln Cys Lys Ser Ile Thr Leu Glu Arg Gly
    1955                1960                1965

Pro Asp Gly Leu Gly Phe Ser Ile Val Gly Gly Tyr Gly Ser Pro
    1970                1975                1980

His Gly Asp Leu Pro Ile Tyr Val Lys Thr Val Phe Ala Lys Gly
    1985                1990                1995

Ala Ala Ser Glu Asp Gly Arg Leu Lys Arg Gly Asp Gln Ile Ile
    2000                2005                2010

Ala Val Asn Gly Gln Ser Leu Glu Gly Val Thr His Glu Glu Ala
    2015                2020                2025

Val Ala Ile Leu Lys Arg Thr Lys Gly Thr Val Thr Leu Met Val
    2030                2035                2040

Leu Ser
    2045

<210> 220
<211> 1542
<212> DNA
<213> Homo sapiens

<400> 220
ggaatttcta caatggtggg ctcagctctc aggagaggtg cccatgcata tgtctacctg      60

gtgagtaagg ccagtcacat ctccagaggc catcagcacc aggcctgggg atcgaggcct     120

cctgcagcag agtgtgccac ccaaagagct ccaggcagtg tggtggagct gctgggcaaa     180

tcctaccctc aggacgacca cagcaacctc acccggaagg tcctcaccag agttggcagg     240

aacctgcaca accagcagca tcaccctctg tggctgatca aggagagggt gaaggagcac     300

ttctacaagc agtatgtggg ccgctttggg accccgttgt tctcggtcta cgacaacctt     360

tctccagtgg tcacgacctg gcagaacttt gacagcctgc tcatcccagc tgatcacccc     420

agcaggaaga agggggacaa ctattacctg aatcggactc acatgctgag agcgcacacg     480

tctgcacacc agtgggactt gctgcacgcg ggactggatg ccttcctggt ggtgggtgat     540

gtctacaggc gtgaccagat cgactcccag cactacccta ttttccacca gctggaggcc     600

gtgcggctct tctccaagca tgagttattt gctggtataa aggatggaga aagcctgcag     660

ctctttgaac aaagttctcg ctctgcgcat aaacaagaga cacacaccat ggaggccgtg     720

aagcttgtag agtttgatct taagcaaacg cttaccaggc tcatggcaca tctttttgga     780

gatgagctgg agataagatg ggtagactgc tacttccctt ttacacatcc ttcctttgag     840

atggagatca actttcatgg agaatggctg gaagttcttg gctgcggggt gatggaacaa     900

caactggtca attcagctgg tgctcaagac cgaatcggct gggcttttgg cctaggatta     960

gaaaggctag ccatgatcct ctacgacatc cctgatatcc gtctcttctg gtgtgaggac    1020

gagcgcttcc tgaagcagtt ctgtgtatcc aacattaatc agaaggtgaa gtttcagcct    1080

cttagcaaat atccggctgt gatcaatgat atttcattct ggttgccctc tgagaattac    1140

gcagaaaatg atttctatga cttagtccga acaattggag gagacctggt ggaaaaggtt    1200

gatctcatag acaagtttgt acatccaaag acgcacaaga ccagccactg ctaccgcatc    1260

acgtaccgcc acatggaacg gactctgtcc cagagagagg tcaggcacat ccaccaggcc    1320

ttgcaggagg ctgcagtcca gctgttgggt gtggagggca ggttctgatg tcaccacttc    1380

actcaggctg cagccctctt ggggctccag gatttgctga aagagaaaaa gatatggttt    1440

gtgaactggg gcatcaatca tcttttgata aatggatcag ttttaggact ttcagaaaat    1500

aaaagatcgc tcttgaaaaa aaaaaaaaaa aaaaaaaaaa aa                    1542

<210> 221
<211> 451
<212> PRT
<213> Homo sapiens

<400> 221

<210> 222
<211> 1883
<212> DNA
<213> Homo sapiens

<400> 222
tgaaggctgg ttgttcagac tgagcttcct gcctgcctgt accccgccaa cagcttcaga      60

agaaggtgac tggtggctgc ctgaggaata ccagtgggca agagaattag catttctgga     120

gcatctgctg tctgagcagc ccctgggtgc gtccactttc tgggcacgtg aggttgggcc     180

ttggccgcct gagcccttga gttggtcact tgaaccttgg gaatattgag attatattct     240

cctgcctttt aaaaagatgg acttcagcag aaatctttat gatattgggg aacaactgga     300

cagtgaagat ctggcctccc tcaagttcct gagcctggac tacattccgc aaaggaagca     360

agaacccatc aaggatgcct tgatgttatt ccagagactc caggaaaaga gaatgttgga     420

ggaaagcaat ctgtccttcc tgaaggagct gctcttccga attaatagac tggatttgct     480

gattacctac ctaaacacta gaaaggagga gatggaaagg gaacttcaga caccaggcag     540

ggctcaaatt tctgcctaca ggttccactt ctgccgcatg agctgggctg aagcaaacag     600

ccagtgccag acacagtctg tacctttctg gcggagggtc gatcatctat taataagggt     660

catgctctat cagatttcag aagaagtgag cagatcagaa ttgaggtctt ttaagtttct     720

tttgcaagag gaaatctcca aatgcaaact ggatgatgac atgaacctgc tggatatttt     780

catagagatg gagaagaggg tcatcctggg agaaggaaag ttggacatcc tgaaaagagt     840

ctgtgcccaa atcaacaaga gcctgctgaa gataatcaac gactatgaag aattcagcaa     900

aggggaggag ttgtgtgggg taatgacaat ctcggactct ccaagagaac aggatagtga     960

atcacagact ttggacaaag tttaccaaat gaaaagcaaa cctcggggat actgtctgat    1020

catcaacaat cacaattttg caaaagcacg ggagaaagtg cccaaacttc acagcattag    1080

ggacaggaat ggaacacact tggatgcagg ggctttgacc acgacctttg aagagcttca    1140

ttttgagatc aagccccacc atgactgcac agtagagcaa atctatgaga ttttgaaaat    1200

ctaccaactc atggaccaca gtaacatgga ctgcttcatc tgctgtatcc tctcccatgg    1260

agacaagggc atcatctatg gcactgatgg acaggaggcc cccatctatg agctgacatc    1320

tcagttcact ggtttgaagt gcccttccct tgctggaaaa cccaaagtgt tttttattca    1380

ggcttgtcag ggggataact accagaaagg tatacctgtt gagactgatt cagaggagca    1440

accctattta gaaatggatt tatcatcacc tcaaacgaga tatatcccgg atgaggctga    1500

ctttctgctg gggatggcca ctgtgaataa ctgtgtttcc taccgaaacc ctgcagaggg    1560

aacctggtac atccagtcac tttgccagag cctgagagag cgatgtcctc gaggcgatga 1620

tattctcacc atcctgactg aagtgaacta tgaagtaagc aacaaggatg acaagaaaaa 1680

catggggaaa cagatgcctc agcctacttt cacactaaga aaaaaacttg tcttcccttc 1740

tgattgatgg tgctattttg tttgttttgt tttgttttgt ttttttgaga cagaatctcg 1800

ctctgtcgcc caggctggag tgcagtggcg tgatctcggc tcaccgcaag ctccgcctcc 1860

cgggttcagg ccattctcct gct 1883

<210> 223
<211> 496
<212> PRT
<213> Homo sapiens

<400> 223

<210> 224
<211> 3547
<212> DNA
<213> Homo sapiens

<400> 224
gcactcgccc ggctcgcccg ctttcgcacc cagttcacgc gccacagcta tgtgtccccg      60

agccgcgcgg gcgcccgcga cgctactcct cgccctgggc gcggtgctgt ggcctgcggc     120

tggcgcctgg gagcttacga ttttgcacac caacgacgtg cacagccggc tggagcagac     180

cagcgaggac tccagcaagt gcgtcaacgc cagccgctgc atgggtggcg tggctcggct     240

cttcaccaag gttcagcaga tccgccgcgc cgaacccaac gtgctgctgc tggacgccgg     300

cgaccagtac cagggcacta tctggttcac cgtgtacaag ggcgccgagg tggcgcactt     360

catgaacgcc ctgcgctacg atgccatggc actgggaaat catgaatttg ataatggtgt     420

ggaaggactg atcgagccac tcctcaaaga ggccaaattt ccaattctga gtgcaaacat     480

taaagcaaag gggccactag catctcaaat atcaggactt tatttgccat ataaagttct     540

tcctgttggt gatgaagttg tgggaatcgt tggatacact tccaaagaaa ccccttttct     600

ctcaaatcca gggacaaatt tagtgtttga agatgaaatc actgcattac aacctgaagt     660

agataagtta aaaactctaa atgtgaacaa aattattgca ctgggacatt cgggttttga     720

aatggataaa ctcatcgctc agaaagtgag gggtgtggac gtcgtggtgg gaggacactc     780

caacacattt ctttacacag gcaatccacc ttccaaagag gtgcctgctg ggaagtaccc     840

attcatagtc acttctgatg atgggcggaa ggttcctgta gtccaggcct atgcttttgg     900

caaataccta ggctatctga agatcgagtt tgatgaaaga ggaaacgtca tctcttccca     960

tggaaatccc attcttctaa acagcagcat tcctgaagat ccaagcataa aagcagacat    1020

taacaaatgg aggataaaat tggataatta ttctacccag gaattaggga aaacaattgt 1080

ctatctggat ggctcctctc aatcatgccg ctttagagaa tgcaacatgg gcaacctgat 1140

ttgtgatgca atgattaaca acaacctgag acacacggat gaaatgttct ggaaccacgt 1200

atccatgtgc attttaaatg gaggtggtat ccggtcgccc attgatgaac gcaacaatgg 1260

cacaattacc tgggagaacc tggctgctgt attgcccttt ggaggcacat ttgacctagt 1320

ccagttaaaa ggttccaccc tgaagaaggc ctttgagcat agcgtgcacc gctacggcca 1380

gtccactgga gagttcctgc aggtgggcgg aatccatgtg gtgtatgatc tttcccgaaa 1440

acctggagac agagtagtca aattagatgt tctttgcacc aagtgtcgag tgcccagtta 1500

tgaccctctc aaaatggacg aggtatataa ggtgatcctc ccaaacttcc tggccaatgg 1560

tggagatggg ttccagatga taaaagatga attattaaga catgactctg gtgaccaaga 1620

tatcaacgtg gtttctacat atatctccaa aatgaaagta atttatccag cagttgaagg 1680

tcggatcaag ttttccacag gaagtcactg ccatggaagc ttttctttaa tatttctttc 1740

actttgggca gtgatctttg ttttatacca atagccaaaa attctccttg cctttaatgt 1800

gtgaaactgc attttttcaa gtgagattca aatctgcctt ttaggacctg gctttgtgac 1860

agcaaaaacc atctttacag gctcctagaa gctgaaggtt agagcattat aaaatgaaga 1920

gacagacatg attactcagg gtcagcaacc tagtgagtta gaaaaaaaat taacataggg 1980

ccctataagg agaaagccaa ctatgttaag tttacgtgtc caaattttaa tgaaatttta 2040

ctaacaattt taaaccatat ttttcttctt catatccatt tctaatccat caaacagctt 2100

atgtttacat aaaattttat cattcacaag gaagttttaa gcacactgtc tcatttgata 2160

tccacaactt atttttggta ggaaagagag atgtttttcc cacctgtcag atgaaaaaac 2220

tgaagctcaa aaaggttgga cttgaccata cagctaatgc tgacagatcc aagacctaga 2280

cctaggtctt ttgaactcaa gtccagcatt ctcaactata tcaagttact gttcagaata 2340

cttaatatct cctctcttca taattatcaa tagccccaag ctcatggatg acaaatctct 2400

gctttatttc ttgtctctat tttttcactt tatagctcct gttataatag caagtttaat 2460

ggtataaaca caggatacca tcctctcttg caacacccat gtgcctttga tgagtcaggt 2520

agcaagctgt agtagataat gagaaaggcc agaggctgca aagacagtca aaggacacga 2580

gagaaaggaa ggggaagaac aggactccag gactgtttta tattatagaa aagcaagagc 2640

taaagagcat ttacacatgt taaacagata cttgttaagc atagtgcctg acacacggca 2700

ttagctgtta ttttatgaga ttccatcagc tctgcctctg tcctctttct tctaacatga 2760

aggtatcatg agaagagaac cttctaacat aagctgtaat tctaaacctg cacttgtccc 2820

tctccagcaa gaggctagca ctgaattcat tctactcata ctacacaccc agttatggaa 2880

tgtccagagt tctcgaagaa aataaatgac tttaggaaga ggtatacatt ttttaagtcg 2940

ctctgcctcc aaatctgaac agtcactgta aatcattctt aagcccagat atgagaactt 3000

ctgctggaaa gtgggaccct ctgagtgggt ggtcagaaaa tacccatgct gatgaaatga 3060

cctatgccca aagaacaaat acttaacgtg ggagtggaac cacatgagcc tgctcagctc 3120

tgcataagta attcaagaaa tgggaggctt caccttaaaa acagtgtgca aatggcagct 3180

agaggttttg ataggaagta tgtttgtttc ttagtgttta caaatattaa gtactcttga 3240

tacaaaatat acttttaaac ttcataacct ttttataaaa gttgttgcag caaaataata 3300

gcctcggttc tatgcatata tggattgcta taaaaaatgt caataagatt gtacaaggaa 3360

aattagagaa agtcacattt agggtttatt ttttacactt ggccagtaaa atagggtaaa 3420

tcctattaga aattttttaa agaacttttt ttaagtttcc taaatctgtg tgtgtattgt 3480

gaagtggtat aagaaatgac tttgaaccac tttgcaattg tagattccca acaataaaat 3540

tgaagat 3547

<210> 225
<211> 574
<212> PRT
<213> Homo sapiens

<400> 225
Met Cys Pro Arg Ala Ala Arg Ala Pro Ala Thr Leu Leu Leu Ala Leu
1               5                   10                  15

Gly Ala Val Leu Trp Pro Ala Ala Gly Ala Trp Glu Leu Thr Ile Leu
            20                  25                  30

His Thr Asn Asp Val His Ser Arg Leu Glu Gln Thr Ser Glu Asp Ser
        35                  40                  45

Ser Lys Cys Val Asn Ala Ser Arg Cys Met Gly Gly Val Ala Arg Leu
    50                  55                  60

Phe Thr Lys Val Gln Gln Ile Arg Arg Ala Glu Pro Asn Val Leu Leu
65                  70                  75                  80

Leu Asp Ala Gly Asp Gln Tyr Gln Gly Thr Ile Trp Phe Thr Val Tyr
                85                  90                  95

Lys Gly Ala Glu Val Ala His Phe Met Asn Ala Leu Arg Tyr Asp Ala
            100                 105                 110

Met Ala Leu Gly Asn His Glu Phe Asp Asn Gly Val Glu Gly Leu Ile
        115                 120                 125

Glu Pro Leu Leu Lys Glu Ala Lys Phe Pro Ile Leu Ser Ala Asn Ile
    130                 135                 140

Lys Ala Lys Gly Pro Leu Ala Ser Gln Ile Ser Gly Leu Tyr Leu Pro
145                 150                 155                 160

Tyr Lys Val Leu Pro Val Gly Asp Glu Val Val Gly Ile Val Gly Tyr
                165                 170                 175

Thr Ser Lys Glu Thr Pro Phe Leu Ser Asn Pro Gly Thr Asn Leu Val
            180                 185                 190

Phe Glu Asp Glu Ile Thr Ala Leu Gln Pro Glu Val Asp Lys Leu Lys
        195                 200                 205

Thr Leu Asn Val Asn Lys Ile Ile Ala Leu Gly His Ser Gly Phe Glu
    210                 215                 220

Met Asp Lys Leu Ile Ala Gln Lys Val Arg Gly Val Asp Val Val Val
225                 230                 235                 240

Gly Gly His Ser Asn Thr Phe Leu Tyr Thr Gly Asn Pro Pro Ser Lys
                245                 250                 255

Glu Val Pro Ala Gly Lys Tyr Pro Phe Ile Val Thr Ser Asp Asp Gly
            260                 265                 270

Arg Lys Val Pro Val Val Gln Ala Tyr Ala Phe Gly Lys Tyr Leu Gly
        275                 280                 285

Tyr Leu Lys Ile Glu Phe Asp Glu Arg Gly Asn Val Ile Ser Ser His
    290                 295                 300

Gly Asn Pro Ile Leu Leu Asn Ser Ser Ile Pro Glu Asp Pro Ser Ile
305                 310                 315                 320

Lys Ala Asp Ile Asn Lys Trp Arg Ile Lys Leu Asp Asn Tyr Ser Thr
                325                 330                 335

Gln Glu Leu Gly Lys Thr Ile Val Tyr Leu Asp Gly Ser Ser Gln Ser
            340                 345                 350

Cys Arg Phe Arg Glu Cys Asn Met Gly Asn Leu Ile Cys Asp Ala Met

355                 360                 365

Ile Asn Asn Asn Leu Arg His Thr Asp Glu Met Phe Trp Asn His Val
    370                 375                 380

Ser Met Cys Ile Leu Asn Gly Gly Gly Ile Arg Ser Pro Ile Asp Glu
385                 390                 395                 400

Arg Asn Asn Gly Thr Ile Thr Trp Glu Asn Leu Ala Ala Val Leu Pro
                405                 410                 415

Phe Gly Gly Thr Phe Asp Leu Val Gln Leu Lys Gly Ser Thr Leu Lys
            420                 425                 430

Lys Ala Phe Glu His Ser Val His Arg Tyr Gly Gln Ser Thr Gly Glu
        435                 440                 445

Phe Leu Gln Val Gly Gly Ile His Val Val Tyr Asp Leu Ser Arg Lys
    450                 455                 460

Pro Gly Asp Arg Val Val Lys Leu Asp Val Leu Cys Thr Lys Cys Arg
465                 470                 475                 480

Val Pro Ser Tyr Asp Pro Leu Lys Met Asp Glu Val Tyr Lys Val Ile
                485                 490                 495

Leu Pro Asn Phe Leu Ala Asn Gly Gly Asp Gly Phe Gln Met Ile Lys
            500                 505                 510

Asp Glu Leu Leu Arg His Asp Ser Gly Asp Gln Asp Ile Asn Val Val
        515                 520                 525

Ser Thr Tyr Ile Ser Lys Met Lys Val Ile Tyr Pro Ala Val Glu Gly
    530                 535                 540

Arg Ile Lys Phe Ser Thr Gly Ser His Cys His Gly Ser Phe Ser Leu
545                 550                 555                 560

Ile Phe Leu Ser Leu Trp Ala Val Ile Phe Val Leu Tyr Gln
                565                 570

<210> 226
<211> 2688
<212> DNA
<213> Homo sapiens

<220>
<221> modified_base
<222> (2675)..(2676)
<223> a, c, t, g, unknown or other

<400> 226
aagcttgcgc gccatgtaag gtaaagtgac tgattctata gcaatccaat tgttcctttg 60

tctgcccgtt tacatataac aatgttgtca atgtttgatt gaaaatacct agcaggcgac 120

acacacacac ctagctcctc aggcggagag cacccctttc ttggccaccc gggtatcccc 180

cagggagtac ggggctcaaa acaccctttt ggagaacaag gtggaagcaa atttcaggaa 240

gtaaaacttc ctgaaataaa ataaaatatc gaatgccttg agacccatac attttcaggt 300

tttcctaatt aaagcaatta ctttccacca cccctccaac ctggaatcac caacttggtt 360

agagaaactg attttctttt tttctttttt tttcccaaaa gagtacatct gatcatttta 420

gcctgcaact aatgatagag atattagggc tagttaacca cagttttaca agactcctct 480

cccgcgtgtg ggccattgtc atgctgtcgg tcccgcccac ctgaaaggtc tccccgcccc 540

gactggggtt tgttgttgaa gaaggagaat ccccggaaag gctgagtctc cagctcaagg 600

tcaaaacgtc caaggccgaa agccctccag tttcccctgg acaccttgct cctgcttctg 660

ctacgacctt ctgggaacgc gaatttctca ttttcttctt aaattgccat tttcgcttta 720

ggagatgaat gttttccttt ggctgttttg gcaatgactc tgaattaaag cgatgctaac 780

gcctcttttc cccctaattg ttaaaagcta tggactgcag gaagatggtc cgcttctctt 840

acagtgtgat ttggatcatg gccatttcta aagcctttga actgggatta gttgccgggc 900

tgggccatca ggaatttgct cgtccatctc ggggagacct ggccttcaga gatgacagca 960

tttggcccca ggaggagcct gcaattcggc ctcggtcttc ccagcgtgtg ctgcccatgg 1020

gaatacagca cagtaaggag ctaaacagaa cctgctgcct gaatggggga acctgcatgc 1080

tggagtcctt ttgtgcctgc cctccctcct tctacggacg gaactgtgag cacgatgtgc 1140

gcaaagagaa ctgtgggtct gtgccccatg acacctggct gcccaagaag tgttccctgt 1200

gtaaatgctg gcacggtcag ctccgctgct ttcctcaggc atttctaccc ggctgtgatg 1260

gccttgtgat ggatgagcac ctcgtggctt ccaggactcc agaactacca ccgtctgcac 1320

gtactaccac ttttatgcta gctggcatct gcctttctat acaaagctac tattaatcga 1380

cattgaccta tttccagaaa tacaatttta gatattatgc aaatttcatg acccgtaaag 1440

gctgctgcta caatgtccta actgaaagat gatcatttgt agttgcctta aaataatgaa 1500

tacaatttcc aaaacggtct ctaacatttc cttacagaac taactacttc ttacctcttt 1560

gccctgccct ctcccaaaaa actacttctt ttttcaaaag aaagtcagcc atatctccat 1620

tgtgcccaag tccagtgttt cttttttttt tttgagacgg agtctcactc tgtcacccag 1680

gctggactgc aatgacgcga tctcggttca ctgcaacctc cgcatccggg gttcaagcca 1740

ttctcctgcc tcagcctccc aagtagctgg gattacaggc atgtgtcacc atgccggcta 1800

atttttttgt attttagtag agacgggggt ttcaccatat tggccagctg gtctcgaact 1860

ctgaccttgt gatccatcgc tcgcctctcg agtgctgaga ttacacacgt gagcaactgt 1920

gcaaggcctg gtgtttcttg atacatgtaa ttctaccaag gtcttcttaa tatgttcttt 1980

taaatgattg aattatacac tcagattatt ggagactaag tctaatgtgg accttagaat 2040

acagttttga gtagagttga tcaaaatcaa ttaaaatagt ctctttaaaa ggaaagaaaa 2100

catctttaag gggaggaacc agagtgctga aggaatggaa gtccatctgc gtgtgtgcag 2160

ggagactggg taggaaagag gaagcaaata gaagagagag gttgaaaaac aaaatgggtt 2220

acttgattgg tgattaggtg gtggtagaga agcaagtaaa aaggctaaat ggaagggcaa 2280

gtttccatca tctatagaaa gctatgtaag acaaggactc cccttttttt cccaaaggca 2340

ttgtaaaaag aatgaagtct ccttagaaaa aaaattatac ctcaatgtcc ccaacaagat 2400

tgcttaataa attgtgtttc ctccaagcta ttcaattctt ttaactgttg tagaagagaa 2460

aatgttcaca atatatttag ttgtaaacca agtgatcaaa ctacatattg taaagcccat 2520

ttttaaaata cattgtatat atgtgtatgc acagtaaaaa tggaaactat attgacctaa 2580

aaaaaaaaaa aggaaaccac ccttaggcag gcaggacatg ctcttcagaa ctctgctctt 2640

cagagttcca aagaagggat aaaacatctt ttatnnccat caaatagc 2688

<210> 227
<211> 2403
<212> DNA
<213> Homo sapiens

<400> 227
cggctcgagc ggctcgagtg aagagcctct ccacggctcc tgcgcctgag acagctggcc 60

tgacctccaa atcatccatc cacccctgct gtcatctgtt ttcatagtgt gagatcaacc 120

cacaggaata tccatggctt ttgtgctcat tttggttctc agtttctacg agctggtgtc 180

aggacagtgg caagtcactg gaccgggcaa gtttgtccag gccttggtgg gggaggacgc 240

cgtgttctcc tgctccctct ttcctgagac cagtgcagag gctatggaag tgcggttctt 300

caggaatcag ttccatgctg tggtccacct ctacagagat ggggaagact gggaatctaa 360

gcagatgcca cagtatcgag ggagaactga gtttgtgaag gactccattg caggggggcg 420

tgtctctcta aggctaaaaa acatcactcc ctcggacatc ggcctgtatg ggtgctggtt 480

cagttcccag atttacgatg aggaggccac ctgggagctg cgggtggcag cactgggctc 540

acttcctctc atttccatcg tgggatatgt tgacggaggt atccagttac tctgcctgtc 600

ctcaggctgg ttcccccagc ccacagccaa gtggaaaggt ccacaaggac aggatttgtc 660

ttcagactcc agagcaaatg cagatgggta cagcctgtat gatgtggaga tctccattat 720

agtccaggaa aatgctggga gcatattgtg ttccatccac cttgctgagc agagtcatga 780

ggtggaatcc aaggtattga taggagagac gtttttccag ccctcacctt ggcgcctggc 840

ttctatttta ctcgggttac tctgtggtgc cctgtgtggt gttgtcatgg ggatgataat 900

tgttttcttc aaatccaaag ggaaaatcca ggcggaactg gactggagaa gaaagcacgg 960

acaggcagaa ttgagagacg cccggaaaca cgcagtggag gtgactctgg atccagagac 1020

ggctcacccg aagctctgcg tttctgatct gaaaactgta acccatagaa aagctcccca 1080

ggaggtgcct cactctgaga agagatttac aaggaagagt gtggtggctt ctcagggttt 1140

ccaagcaggg agacattact gggaggtgga cgtgggacaa aatgtagggt ggtatgtggg 1200

agtgtgtcgg gatgacgtag acagggggaa gaacaatgtg actttgtctc ccaacaatgg 1260

gtattgggtc ctcagactga caacagaaca tttgtatttc acattcaatc cccattttat 1320

cagcctcccc cccagcaccc ctcctacacg agtaggggtc ttcctggact atgagggtgg 1380

gaccatctcc ttcttcaata caaatgacca gtcccttatt tataccctgc tgacatgtca 1440

gtttgaaggc ttgttgagac cctatatcca gcatgcgatg tatgacgagg aaaaggggac 1500

tcccatattc atatgtccag tgtcctgggg atgagacaga gaagaccctg cttaaagggc 1560

cccacaccac agacccagac acagccaagg gagagtgctc ccgacaggtg gccccagctt 1620

cctctccgga gcctgcgcac agagagtcac gccccccact ctcctttagg gagctgaggt 1680

tcttctgccc tgagccctgc agcagcggca gtcacagctt ccagatgagg ggggattggc 1740

ctgaccctgt gggagtcaga agccatggct gccctgaagt ggggacggaa tagactcaca 1800

ttaggtttag tttgtgaaaa ctccatccag ctaagcgatc ttgaacaagt cacaacctcc 1860

caggctcctc atttgctagt cacggacagt gattcctgcc tcacaggtga agattaaaga 1920

gacaacgaat gtgaatcatg cttgcaggtt tgagggcaca gtgtttgcta atgatgtgtt 1980

tttatattat acattttccc accataaact ctgtttgctt attccacatt aatttacttt 2040

tctctatacc aaatcaccca tggaatagtt attgaacacc tgctttgtga ggctcaaaga 2100

ataaagagga ggtaggattt ttcactgatt ctataagccc agcattacct gataccaaaa 2160

ccaggcaaag aaaacagaag aagaggaagg aaaactacag gtccatatcc ctcattaaca 2220

cagacacaaa aattctaaat aaaattttaa caaattaaac taaacaatat atttaaagat 2280

gatatataac tactcagtgt ggtttgtccc acaaatgcag agttggttta atatttaaat 2340

atcaaccagt gtaattcagc acattaataa agtaaaaaag aaaaccataa aaaaaaaaaa 2400

aaa                                                          2403

<210> 228
<211> 466
<212> PRT
<213> Homo sapiens

<400> 228
Met Ala Phe Val Leu Ile Leu Val Leu Ser Phe Tyr Glu Leu Val Ser
1               5                   10                  15

Gly Gln Trp Gln Val Thr Gly Pro Gly Lys Phe Val Gln Ala Leu Val
            20                  25                  30

Gly Glu Asp Ala Val Phe Ser Cys Ser Leu Phe Pro Glu Thr Ser Ala
        35                  40                  45

Glu Ala Met Glu Val Arg Phe Phe Arg Asn Gln Phe His Ala Val Val
    50                  55                  60

His Leu Tyr Arg Asp Gly Glu Asp Trp Glu Ser Lys Gln Met Pro Gln
65                  70                  75                  80

Tyr Arg Gly Arg Thr Glu Phe Val Lys Asp Ser Ile Ala Gly Gly Arg
                85                  90                  95

Val Ser Leu Arg Leu Lys Asn Ile Thr Pro Ser Asp Ile Gly Leu Tyr
            100                 105                 110

Gly Cys Trp Phe Ser Ser Gln Ile Tyr Asp Glu Glu Ala Thr Trp Glu
        115                 120                 125

Leu Arg Val Ala Ala Leu Gly Ser Leu Pro Leu Ile Ser Ile Val Gly
    130                 135                 140

Tyr Val Asp Gly Gly Ile Gln Leu Leu Cys Leu Ser Ser Gly Trp Phe
145                 150                 155                 160

Pro Gln Pro Thr Ala Lys Trp Lys Gly Pro Gln Gly Gln Asp Leu Ser
                165                 170                 175

Ser Asp Ser Arg Ala Asn Ala Asp Gly Tyr Ser Leu Tyr Asp Val Glu
            180                 185                 190

Ile Ser Ile Ile Val Gln Glu Asn Ala Gly Ser Ile Leu Cys Ser Ile
        195                 200                 205

His Leu Ala Glu Gln Ser His Glu Val Glu Ser Lys Val Leu Ile Gly
    210                 215                 220

Glu Thr Phe Phe Gln Pro Ser Pro Trp Arg Leu Ala Ser Ile Leu Leu
225                 230                 235                 240

Gly Leu Leu Cys Gly Ala Leu Cys Gly Val Val Met Gly Met Ile Ile
                245                 250                 255

Val Phe Phe Lys Ser Lys Gly Lys Ile Gln Ala Glu Leu Asp Trp Arg
            260                 265                 270

Arg Lys His Gly Gln Ala Glu Leu Arg Asp Ala Arg Lys His Ala Val
        275                 280                 285

Glu Val Thr Leu Asp Pro Glu Thr Ala His Pro Lys Leu Cys Val Ser
    290                 295                 300

Asp Leu Lys Thr Val Thr His Arg Lys Ala Pro Gln Glu Val Pro His
305                 310                 315                 320

Ser Glu Lys Arg Phe Thr Arg Lys Ser Val Val Ala Ser Gln Gly Phe
                325                 330                 335

Gln Ala Gly Lys His Tyr Trp Glu Val Asp Val Gly Gln Asn Val Gly
            340                 345                 350

Trp Tyr Val Gly Val Cys Arg Asp Asp Val Asp Arg Gly Lys Asn Asn
        355                 360                 365

Val Thr Leu Ser Pro Asn Asn Gly Tyr Trp Val Leu Arg Leu Thr Thr
    370                 375                 380

Glu His Leu Tyr Phe Thr Phe Asn Pro His Phe Ile Ser Leu Pro Pro
385                 390                 395                 400

Ser Thr Pro Pro Thr Arg Val Gly Val Phe Leu Asp Tyr Glu Gly Gly
                405                 410                 415

Thr Ile Ser Phe Phe Asn Thr Asn Asp Gln Ser Leu Ile Tyr Thr Leu
            420                 425                 430

Leu Thr Cys Gln Phe Glu Gly Leu Leu Arg Pro Tyr Ile Gln His Ala
        435                 440                 445

Met Tyr Asp Glu Glu Lys Gly Thr Pro Ile Phe Ile Cys Pro Val Ser

450                 455                 460

Trp Gly
465

<210> 229
<211> 210
<212> DNA
<213> Homo sapiens

<400> 229
atgaggatct tttactatct ccattttctg tgttatgtga ccttcattct accagccaca      60

tgtaccttgg tgaatgctga tcgttgcacc aaacgttacg gtcgttgtaa aagagactgt     120

cttgagagtg aaaagcaaat agacatatgt tccttaccaa gaaaaatttg ctgcactgag     180

aaattgtatg aagaagatga tatgttttga                                      210

<210> 230
<211> 69
<212> PRT
<213> Homo sapiens

<400> 230
Met Arg Ile Phe Tyr Tyr Leu His Phe Leu Cys Tyr Val Thr Phe Ile
1               5                   10                  15

Leu Pro Ala Thr Cys Thr Leu Val Asn Ala Asp Arg Cys Thr Lys Arg
            20                  25                  30

Tyr Gly Arg Cys Lys Arg Asp Cys Leu Glu Ser Glu Lys Gln Ile Asp
        35                  40                  45

Ile Cys Ser Leu Pro Arg Lys Ile Cys Cys Thr Glu Lys Leu Tyr Glu
    50                  55                  60

Glu Asp Asp Met Phe
65

<210> 231
<211> 420
<212> DNA
<213> Homo sapiens

<220>
<221> modified_base
<222> (36)..(36)
<223> a, c, t, g, unknown or other

<400> 231
ctcttgtcag ctgtctttca gaagacctgg tggggnaagt ccgtgggcat catgttgacc      60

gagctggaga aagccttgaa ctctatcatc gacgtctacc acaagtactc cctgataaag    120

gggaatttcc atgccgtcta cagggatgac ctgaagaaat tgctagagac cgagtgtcct    180

cagtatatca ggaaaaaggg tgcagacgtc tggttcaaag agttggatat caacactgat    240

ggtgcagtta acttccagga gttcctcatt ctggtgataa agatgggctg gcagcccaca    300

aaaaaagcca tgaagaaagc cacaaagagt agctgagtta ctgcccagag gctgggcccc    360

tgacatgtac ctgcagaata ataaagtcat caatacctca aaaaaaaaaa aaaaaaaaaa    420

<210> 232
<211> 94
<212> PRT
<213> Homo sapiens

<400> 232
Met Leu Thr Glu Leu Glu Lys Ala Leu Asn Ser Ile Ile Asp Val Tyr
1               5                   10                  15

His Lys Tyr Ser Leu Ile Lys Gly Asn Phe His Ala Val Tyr Arg Asp
            20                  25                  30

Asp Leu Lys Lys Leu Leu Glu Thr Glu Cys Pro Gln Tyr Ile Arg Lys
        35                  40                  45

Lys Gly Ala Asp Val Trp Phe Lys Glu Leu Asp Ile Asn Thr Asp Gly
    50                  55                  60

Ala Val Asn Phe Gln Glu Phe Leu Ile Leu Val Ile Lys Met Gly Trp
65                  70                  75                  80

Gln Pro Thr Lys Lys Ala Met Lys Lys Ala Thr Lys Ser Ser
                85                  90

<210> 233
<211> 832
<212> DNA
<213> Homo sapiens

<400> 233
gcactcccaa agaactgggt actcaacact gagcagatct gttctttgag ctaaaaacca     60

tgtgctgtac caagagtttg ctcctggctg ctttgatgtc agtgctgcta ctccacctct    120

gcggcgaatc agaagcagca agcaactttg actgctgtct tggatacaca gaccgtattc    180

ttcatcctaa atttattgtg ggcttcacac ggcagctggc caatgaaggc tgtgacatca    240

atgctatcat ctttcacaca aagaaaaagt tgtctgtgtg cgcaaatcca aaacagactt    300

gggtgaaata tattgtgcgt ctcctcagta aaaaagtcaa gaacatgtaa aaactgtggc    360

ttttctggaa tggaattgga catagcccaa gaacagaaag aaccttgctg gggttggagg 420

tttcacttgc acatcatgga gggtttagtg cttatctaat ttgtgcctca ctggacttgt 480

ccaattaatg aagttgattc atattgcatc atagtttgct ttgtttaagc atcacattaa 540

agttaaactg tattttatgt tatttatagc tgtaggtttt ctgtgtttag ctatttaata 600

ctaattttcc ataagctatt ttggtttagt gcaaagtata aaattatatt tgggggggaa 660

taagattata tggactttct tgcaagcaac aagctatttt ttaaaaaaaa ctatttaaca 720

ttcttttgtt tatattgttt tgtctcctaa attgttgtaa ttgcattata aaataagaaa 780

aatattaata agacaaatat tgaaaataaa gaaacaaaaa gttcttctgt ta 832

<210> 234
<211> 96
<212> PRT
<213> Homo sapiens

<400> 234
Met Cys Cys Thr Lys Ser Leu Leu Leu Ala Ala Leu Met Ser Val Leu
1               5                   10                  15

Leu Leu His Leu Cys Gly Glu Ser Glu Ala Ala Ser Asn Phe Asp Cys
            20                  25                  30

Cys Leu Gly Tyr Thr Asp Arg Ile Leu His Pro Lys Phe Ile Val Gly
        35                  40                  45

Phe Thr Arg Gln Leu Ala Asn Glu Gly Cys Asp Ile Asn Ala Ile Ile
    50                  55                  60

Phe His Thr Lys Lys Lys Leu Ser Val Cys Ala Asn Pro Lys Gln Thr
65                  70                  75                  80

Trp Val Lys Tyr Ile Val Arg Leu Leu Ser Lys Lys Val Lys Asn Met
                85                  90                  95

<210> 235
<211> 20
<212> DNA
<213> Artificial sequence

<220>
<221> source
<223> /note="Description of artificial sequence: Synthetic
probe"

<400> 235
ctttggccat ggtgcggagg 20

<210> 236
<211> 29
<212> DNA
<213> Artificial sequence

<220>
<221> source
<223> /note="Description of artificial sequence: Synthetic
      probe"

<400> 236
tgtgttggta gtgctgtgtt gaattacgg                                          29


<210> 237
<211> 22
<212> DNA
<213> Artificial sequence

<220>
<221> source
<223> /note="Description of artificial sequence: Synthetic
      probe"

<400> 237
tgtgtgaaat cagtggccgc ct                                                 22


<210> 238
<211> 24
<212> DNA
<213> Artificial sequence

<220>
<221> source
<223> /note="Description of artificial sequence: Synthetic
      probe"

<400> 238
cacttgccat tgcagaaggt cctg                                               24


<210> 239
<211> 21
<212> DNA
<213> Artificial sequence

<220>
<221> source
<223> /note="Description of artificial sequence: Synthetic
      probe"

<400> 239
ggcattcaaa tgggctgctg c                                                  21


<210> 240
<211> 26
<212> DNA
<213> Artificial sequence

<220>
<221> source
<223> /note="Description of artificial sequence: Synthetic
      probe"

<400> 240
ctggacggat ggtagcagtc taggga                                          26


<210> 241
<211> 26
<212> DNA
<213> Artificial sequence

<220>
<221> source
<223> /note="Description of artificial sequence: Synthetic
      probe"

<400> 241
tccctgataa aggggaattt ccatgc                                          26


<210> 242
<211> 23
<212> DNA
<213> Artificial sequence

<220>
<221> source
<223> /note="Description of artificial sequence: Synthetic
      probe"

<400> 242
tggcagtttc tgagcagtcg tgc                                             23


<210> 243
<211> 19
<212> DNA
<213> Artificial sequence

<220>
<221> source
<223> /note="Description of artificial sequence: Synthetic
      primer"

<400> 243
catcccttgc tgccattct                                                  19


<210> 244
<211> 20
<212> DNA
<213> Artificial sequence

<220>
<221> source
<223> /note="Description of artificial sequence: Synthetic
      primer"

<400> 244
gaccttgaac tgaatcttgc                                                    20


<210> 245
<211> 46
<212> PRT
<213> Homo sapiens

<400> 245
Pro Pro Pro Ser His Asp Gln Arg Gly Ser Ser Gly His Thr Pro Pro
1               5                   10                  15


Pro Ser Gly Pro Pro Asn Met Ala Val Gly Asn Met Ser Val Leu Leu
            20                  25                  30


Arg Ser Leu Pro Gly Glu Thr Gln Phe Leu Asn Ser Ser Ala
        35                  40                  45


<210> 246
<211> 46
<212> PRT
<213> Mucaca mulatta

<400> 246
Pro Pro Pro Ser His Asp Gln Gly Gly Ser Ser Gly His Thr Pro Pro
1               5                   10                  15


Pro Ser Gly Pro Pro Asn Met Ala Val Gly Asn Met Ser Val Leu Leu
            20                  25                  30


Arg Ser Leu Pro Gly Glu Thr Gln Phe Leu Asn Ser Ser Ala
        35                  40                  45


<210> 247
<211> 46
<212> PRT
<213> Mus musculus

<400> 247
Pro Pro Leu Ser His Glu Gln Gly Asp Ser Ser Lys Asn Thr Pro Ser
1               5                   10                  15


Pro Ser Gly Pro Pro Asn Met Ala Val Gly Asn Met Ser Val Leu Leu
            20                  25                  30


Gly Ser Leu Pro Gly Glu Thr Gln Phe Leu Asn Ser Ser Ala
        35                  40                  45


<210> 248
<211> 46

<212> PRT
<213> Rattus norvegicus

<400> 248
Pro Pro Pro Ser His Glu Gln Gly Asp Ser Ser Lys Asn Thr Pro Pro
1               5                   10                  15

Pro Ser Gly Pro Pro Asn Met Ala Val Gly Asn Met Ser Val Leu Leu
            20                  25                  30

Gly Ser Leu Pro Gly Glu Thr Gln Phe Leu Asn Ser Ser Ala
        35                  40                  45

<210> 249
<211> 46
<212> PRT
<213> Canis familiaris

<400> 249
Pro Pro Pro Ser His Asp Gln Gly Asp Ser Ser Glu His Thr Pro Pro
1               5                   10                  15

Pro Ser Gly Pro Pro Asn Met Ala Val Gly Asn Met Ser Ile Leu Leu
            20                  25                  30

Gly Ser Leu Pro Gly Glu Thr Gln Phe Leu Asn Ser Ser Ala
        35                  40                  45

<210> 250
<211> 46
<212> PRT
<213> Bos taurus

<400> 250
Pro Pro Pro Ser His Asn Gln Arg Asp Ser Ser Glu His Thr Pro Pro
1               5                   10                  15

Pro Ser Gly Thr Pro Asn Met Ala Val Gly Asn Met Ser Val Leu Leu
            20                  25                  30

Gly Ser Leu Pro Gly Glu Thr Gln Phe Leu Asn Ser Ser Ala
        35                  40                  45

<210> 251
<211> 4
<212> PRT
<213> Artificial sequence

<220>
<221> source
<223> /note="Description of artificial sequence: Synthetic
      peptide"

<400> 251
Asp Glu Ala His
1


<210> 252
<211> 19
<212> DNA
<213> Artificial sequence

<220>
<221> source
<223> /note="Description of artificial sequence: Synthetic
      probe"

<400> 252
agcgatgcca gagagaata                                                    19


<210> 253
<211> 20
<212> DNA
<213> Artificial sequence

<220>
<221> source
<223> /note="Description of artificial sequence: Synthetic
      probe"

<400> 253
ggaagtgatt ggggtctttg                                                   20


<210> 254
<211> 20
<212> DNA
<213> Artificial sequence

<220>
<221> source
<223> /note="Description of artificial sequence: Synthetic
      probe"

<400> 254
actcccagtc ttgtcattgc                                                   20


<210> 255
<211> 20
<212> DNA
<213> Artificial sequence

<220>
<221> source
<223> /note="Description of artificial sequence: Synthetic
      probe"

<400> 255
caagtttcaa ccagcaagaa                                                   20

<210> 256
<211> 19
<212> DNA
<213> Artificial sequence

<220>
<221> source
<223> /note="Description of artificial sequence: Synthetic
      probe"

<400> 256
gctacccgtg agtccctct                                                    19


<210> 257
<211> 20
<212> DNA
<213> Artificial sequence

<220>
<221> source
<223> /note="Description of artificial sequence: Synthetic
      probe"

<400> 257
tcttgcactg ctttggtttc                                                   20


<210> 258
<211> 20
<212> DNA
<213> Artificial sequence

<220>
<221> source
<223> /note="Description of artificial sequence: Synthetic
      probe"

<400> 258
agagctttgg gctcaacaag                                                   20


<210> 259
<211> 20
<212> DNA
<213> Artificial sequence

<220>
<221> source
<223> /note="Description of artificial sequence: Synthetic
      probe"

<400> 259
atgacagtgc aggtcccata                                                   20


<210> 260
<211> 20
<212> DNA
<213> Artificial sequence

<220>
<221> source
<223> /note="Description of artificial sequence: Synthetic
      probe"

<400> 260
aagggaactt gagcgtgaat                                                   20


<210> 261
<211> 20
<212> DNA
<213> Artificial sequence

<220>
<221> source
<223> /note="Description of artificial sequence: Synthetic
      probe"

<400> 261
gagtgcttcc ccttctcttg                                                   20


<210> 262
<211> 20
<212> DNA
<213> Artificial sequence

<220>
<221> source
<223> /note="Description of artificial sequence: Synthetic
      probe"

<400> 262
cacttcgatg aggatgaacg                                                   20


<210> 263
<211> 20
<212> DNA
<213> Artificial sequence

<220>
<221> source
<223> /note="Description of artificial sequence: Synthetic
      probe"

<400> 263
gtcccatacc caaagaatgg                                                   20


<210> 264
<211> 19
<212> DNA
<213> Artificial sequence

<220>
<221> source
<223> /note="Description of artificial sequence: Synthetic
      probe"

<400> 264
ttgaccgagc tggagaaag                                                    19


<210> 265
<211> 20
<212> DNA
<213> Artificial sequence

<220>
<221> source
<223> /note="Description of artificial sequence: Synthetic
      probe"

<400> 265
tcaggtcatc cctgtagacg                                                   20


<210> 266
<211> 20
<212> DNA
<213> Artificial sequence

<220>
<221> source
<223> /note="Description of artificial sequence: Synthetic
      probe"

<400> 266
agagccgctg gtgtatcttt                                                   20


<210> 267
<211> 20
<212> DNA
<213> Artificial sequence

<220>
<221> source
<223> /note="Description of artificial sequence: Synthetic
      probe"

<400> 267
ccttctgcag gacaatgaag                                                   20


<210> 268
<211> 1257
<212> DNA
<213> Homo sapiens

<400> 268
atgaccgaag aaacacaccc agacgatgac agctatattg tgcgtgtcaa ggctgtggtt      60

atgaccagag atgactccag cgggggatgg ttcccacagg aaggaggcgg gatcagtcgc     120

gtcggggtct gtaaggtcat gcaccccgaa ggcaatggac gaagcggctt tctcatccat     180

ggtgaacgac agaaagacaa actggtggta ttggaatgct atgtaagaaa ggacttggtc     240

tacaccaaag ccaatccaac gtttcatcac tggaaggtcg ataataggaa gtttggactt 300

actttccaaa gccctgctga tgcccgagcc tttgacaggg gagtaaggaa agcaatcgaa 360

gaccttatag aaggttcaac aacgtcatct tccaccatcc ataatgaagc tgagcttggc 420

gatgatgacg tttttacaac agctacagac agttcttcta attcctctca gaagagagag 480

caacctactc ggacaatctc ctctcccaca tcctgtgagc accggaggat ttataccctg 540

ggccacctcc acgactcata ccccacagac cactatcacc tcgatcagcc gatgccaagg 600

ccctaccgcc aggtgagctt cccggacgac gacgaggaga tcgtgcgcat caacccccgg 660

gagaagatct ggatgacggg gtacgaggat taccggcacg cacccgtcag gggcaagtac 720

ccggacccct cggaggacgc ggactcctcc tacgtgcgct tcgccaaggg cgaggtcccc 780

aagcatgact acaactaccc ctacgtggac tcctcagact ttggcctagg cgaggacccc 840

aaaggccgcg ggggcagcgt gatcaagacg cagccctccc ggggcaagtc gcggcggcgg 900

aaggaggacg gagagcgctc gcggtgcgtg tactgcaggg acatgttcaa ccacgaggag 960

aaccgccggg gccactgcca ggacgcgccc gactccgtga gaacttgcat ccgccgggtg 1020

agctgcatgt ggtgcgcgga cagcatgctc tatcactgta tgtcggaccc cgagggagac 1080

tatacagacc cttgctcgtg cgatactagc gacgagaagt tttgcctccg gtggatggct 1140

cttattgcct tgtctttcct ggccccctgt atgtgctgtt acctgcccct tcgggcctgc 1200

taccactgcg gagtgatgtg caggtgctgt ggcgggaagc acaaagcggc cgcgtga 1257

<210> 269
<211> 609
<212> DNA
<213> Homo sapiens

<400> 269
atggcagaag gtgaagagga aggaggtgat gttctgatta gtgttggcca tgccaatgtg 60

ttaggatata ctcttcgaga atttttacag cttttgcaac atatcactat tggaacagtg 120

ctacaaatca aggtttaccg agattttatt aacattcctg aagaatggca agaaatatat 180

gatttaatcc ctgaggccaa attcccagta acaagcacac caaagaaaat tgagctggca 240

aaagatgaat ctttcacaag cagtgatgat aatgaaaatg tagatttaga taaaagactt 300

caatattata gatatccgtg gtcaactgtg catcaccctg caaggagacc aatatccatc 360

tccagagact ggcatggata taagaagaag aaccatacta ttagtgtagg aaaagacatt 420

aattgtgacg tgatgattca cagagacgac aagaaagaag tgagggcccc ttctccatac 480

tggataatgg tgaagcaaga caatgaaagc tcttcctcct ctacctcctc tacctcagat 540

gcattttggc tggaagattg tgcccaagtt gaagagggta aagcccaact ggtatcaaag 600

gttggttag 609

## Description

### Background of the Invention

### Cross-reference to Related Applications

This application claims priority under Section 119(e) and the benefit of United States Provisional Application Serial No. 60/991,203 filed November 29, 2007, United States Provisional Application Serial No. 61/192,268 filed September 17, 2008, and International Patent Application Serial No. PCT/US08/64562 filed May 22, 2008 the entire disclosures of which are incorporated herein by reference in their entirety.

### Field of the Invention

The present invention relates to gene expression profiles in inflammatory bowel disease pathogenesis, including use in the detection and diagnosis of inflammatory bowel disease.

### Description of Related Art

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases, non-immune-mediated inflammatory diseases, infectious diseases, immunodeficiency diseases, neoplasia, etc.

The term inflammatory bowel disorder ("IBD") describes a group of chronic inflammatory disorders of unknown causes in which the intestine (bowel) becomes inflamed, often causing recurring cramps or diarrhea. The prevalence of IBD in the US is estimated to be about 200 per 100,000 population. Patients with IBD can be divided into two major groups, those with ulcerative colitis ("UC") and those with Crohn's disease ("CD"). Both UC and CD are chronic relapsing diseases and are complex clinical entities that occur in genetically susceptible individuals who are exposed to as yet poorly defined environmental stimuli. (Bonen and Cho, Gastroenterology. 2003; 124:521-536; Gaya et al. Lancet. 2006;367:1271-1284).

Although the cause of IBD remains unknown, several factors such as genetic, infectious and immunologic susceptibility have been implicated. IBD is much more common in Caucasians, especially those of Jewish descent. The chronic inflammatory nature of the condition has prompted an intense search for a possible infectious cause. Although agents have been found which stimulate acute inflammation, none has been found to cause the chronic inflammation associated with IBD. The hypothesis that IBD is an autoimmune disease is supported by the previously mentioned extraintestinal manifestation of IBD as joint arthritis, and the known positive response to IBD by treatment with therapeutic agents such as adrenal glucocorticoids, cyclosporine and azathioprine, which are known to suppress immune response. In addition, the GI tract, more than any other organ of the body, is continuously exposed to potential antigenic substances such as proteins from food, bacterial byproducts (LPS), etc.

There is sufficient overlap in the diagnostic criteria for UC and CD that it is sometimes impossible to say which a given patient has; however, the type of lesion typically seen is different, as is the localization. UC mostly appears in the colon, proximal to the rectum, and the characteristic lesion is a superficial ulcer of the mucosa; CD can appear anywhere in the bowel, with occasional involvement of stomach, esophagus and duodenum, and the lesions are usually described as extensive linear fissures.

The current therapy of IBD usually involves the administration of antiinflammatory or immunosuppressive agents, such as sulfasalazine, corticosteroids, 6-mercaptopurine/azathioprine, or cyclosporine, which usually bring only partial results. If anti-inflammatory/immunosuppressive therapies fail, colectomies are the last line of defense. The typical operation for CD not involving the rectum is resection (removal of a diseased segment of bowel) and anastomosis (reconnection) without an ostomy. Sections of the small or large intestine may be removed. About 30% of CD patients will need surgery within the first year after diagnosis. In the subsequent years, the rate is about 5% per year. Unfortunately, CD is characterized by a high rate of recurrence; about 5% of patients need a second surgery each year after initial surgery.

Refining a diagnosis of inflammatory bowel disease involves evaluating the progression status of the diseases using standard classification criteria. The classification systems used in IBD include the Truelove and Witts Index (Truelove S.C. and Witts, L.J. Br Med J. 1955;2:1041-1048), which classifies colitis as mild, moderate, or severe, as well as Lennard-Jones. (Lennard-Jones JE. Scand J Gastroenterol Suppl 1989; 170:2-6) and the simple clinical colitis activity index (SCCAI). (Walmsley et. al. Gut. 1998;43:29-32) These systems track such variables as daily bowel movements, rectal bleeding, temperature, heart rate, hemoglobin levels, erythrocyte sedimentation rate, weight, hematocrit score, and the level of serum albumin.

In approximately 10-15% of cases, a definitive diagnosis of ulcerative colitis or Crohn's disease cannot be made and such cases are often referred to as "indeterminate colitis." Two antibody detection tests are available that can help the diagnosis, each of which assays for antibodies in the blood. The antibodies are "perinuclear anti-neutrophil antibody" (pANCA) and "anti-Saccharomyces cervisiae antibody" (ASCA). Most patients with ulcerative colitis have the pANCA antibody but not the ASCA antibody, while most patients with Crohn's disease have the ASCA antibody but not the pANCA antibody. However, these two tests have shortcomings as some patients have neither antibody and some Crohn's disease patients may have only the pANCA antibody.For clinical practice, a reliable test that would indicate the presence and/or progression of an IBD based on molecular markers rather than the measurement of a multitude of variables would be useful for identifying and/or treating individuals with an IBD. Hypothesis free, linkage and association studies have identified genetic loci that have been associated with UC, notably the MHC region on chromosome 6,(Rioux et al. Am J Hum Genet. 2000;66:1863-1870; Stokkers et al. Gut. 1999; 45:395-401; Van Heel et al. Hum Mol Genet. 2004;13:763-770) the IBD2 locus on chromosome 12 (Parkes et al. Am J Hum Genet. 2000;67:1605-1610; Satsangi et al. Nat Genet. 1996;14:199-202) and the IBD5 locus on chromosome 5. (Giallourakis et. al. Am J. Hum Genet. 2003;73:205-211; Palmieri et. al Aliment Pharmacol Ther. 2006;23:497-506; Russell et. al. Gut. 2006;55:1114-1123; Waller et. al. Gut. 2006;55:809-814) Following a UK wide linkage scan identifying a putative loci of association for UC on chromosome 7q, further studies have implicated variants in the ABCB1 (MDR1) gene which is involved in cellular detoxification with UC. (Satsangi et. al. Nat Genet. 1996;14:199-202; Brant et.al. Am J Hum Genet. 2003;73:1282-1292; Ho et. al. Gastroenterology. 2005;128:288-296)

A complementary approach towards the identification and understanding of the complex gene- gene and gene- environment relationships that result in the chronic intestinal inflammation observed in inflammatory bowel disease (IBD) is microarray gene expression analysis. Microarrays allow a comprehensive picture of gene expression at the tissue and cellular level, thus helping understand the underlying patho-physiological processes. (Stoughton et. al. Annu Rev Biochem. 2005;74:53-82) Microarray analysis was first applied to patients with IBD in 1997, comparing expression of 96 genes in surgical resections of patients with CD to synovial tissue of patients with rheumatoid arthritis. (Heller et. al. Proc Natl Acad Sci U S A. 1997;94:2150-2155) Further studies using microarray platforms to interrogate surgical specimens from patients with IBD identified an number of novel genes that were differentially regulated when diseased samples were compared to controls. (Dieckgraefe et.al. Physiol Genomics. 2000;4: 1-11; Lawrance et. al. Hum Mol Genet. 2001;10:445-456)

A complementary approach towards the identification and understanding of the complex gene- gene and gene- environment relationships that result in the chronic intestinal inflammation observed in inflammatory bowel disease (IBD) is microarray gene expression analysis. Microarrays allow a comprehensive picture of gene expression at the tissue and cellular level, thus helping understand the underlying patho-physiological processes. (Stoughton et. al. Annu Rev Biochem. 2005;74:53-82) Microarray analysis was first applied to patients with IBD in 1997, comparing expression of 96 genes in surgical resections of patients with CD to synovial tissue of patients with rheumatoid arthritis. (Heller et. al. Proc Natl Acad Sci U S A. 1997;94:2150-2155) Further studies using microarray platforms to interrogate surgical specimens from patients with IBD identified a number of novel genes that were differentially regulated when diseased samples were compared to controls. (Dieckgraefe et.al. Physiol Genomics. 2000;4:1-11; Lawrance et. al. Hum Mol Genet. 2001;10:445-456)

Endoscopic pinch mucosal biopsies have allowed investigators to microarray tissue from a larger range of patients encompassing those with less severe disease. Langmann et. al. used microarray technology to analyze 22,283 genes in biopsy specimens from macroscopically non affected areas of the colon and terminal ileum. (Langmann et. al. Gastroenterology. 2004;127:26-40) Genes which were involved in cellular detoxification and biotransformation (Pregnane X receptor and MDR1) were significantly downregulated in the colon of patients with UC, however, there was no change in the expression of these genes in the biopsies from patients with CD. Costello and colleagues (Costello et. al. PLoS Med. 2005;2:e199) looked at the expression of 33792 sequences in endoscopic sigmoid colon biopsies obtained from healthy controls, patients with CD and UC. A number of sequences representing novel proteins were differentially regulated and *in silico* analysis suggested that these proteins had putative functions related to disease pathogenesis - transcription factors, signaling molecules and cell adhesion.

In a study of patients with UC, Okahara et al. (Aliment Pharmacol Ther. 2005;21:1091-1097) observed that (migration inhibitory factor- related protein 14 (MRP14), growth- related oncogene gamma (GROγ) and serum amyloid A1 (SAA1) were upregulated where as TIMP1 and elfin were down regulated in the inflamed biopsies when compared to the non- inflamed biopsies. When observing 41 chemokines and 21 chemokine receptors, Puleston et al demonstrated that chemokines CXCLs 1-3 and 8 and CCL20 were upregulated in active colonic CD and UC. (Aliment Pharmacol Ther. 2005;21:109-120) Overall these studies illustrate the heterogeneity of early microarray platforms and tissue collection. However, despite these problems differential expression of a number of genes was consistently observed.

Despite the above identified advances in IBD research, there is a great need for additional diagnostic and therapeutic agents capable of detecting IBD in a mammal and for effectively treating this disorder. Accordingly, the present invention provides polynucleotides and polypeptides that are overexpressed in IBD as compared to normal tissue, and methods of using those polypeptides, and their encoding nucleic acids, for to detect or diagnose the presence of an IBD in mammalian subjects and subsequently to treat those subjects in which an IBD is detected with suitable IBD therapeutic agents.

The present invention provides methods for detecting the presence of and determining the progression of inflammatory bowel disease (IBD), including ulcerative colitis (UC) and Crohn's disease (CD).

The invention disclosed herein provides methods and assays examining expression of one or more gene expression markers in a mammalian tissue or cell sample, wherein the expression of one or more such biomarkers is predictive of whether the mammalian subject from which the tissue or cell sample was taken is more likely to have an IBD. In various embodiments of the invention, the methods and assays examine the expression of gene expression markers such as those listed in Tables 1, 2, and 3 and determine whether expression is higher or lower than a control sample.

These and further embodiments of the present invention will be apparent to those of ordinary skill in the art.

### Summary of the Invention

In one aspect, the invention concerns a method of detecting or diagnosing an inflammatory bowel disease (IBD) in a mammalian subject comprising determining, in a biological sample obtained from the subject, that expression levels of (i) one or more nucleic acids encoding one or more polypeptides selected from Tables 1, 2, or 3, or (ii) RNA transcripts or their expression products of one or more genes selected from Tables 1, 2, or 3, is different relative to the expression level in a control, wherein the difference in expression indicates the subject is more likely to have an IBD.

In one embodiment, the methods of diagnosing or detecting the presence of an IBD in a mammalian subject comprise determining that the expression level of (i) one or more nucleic acids encoding one or more polypeptides selected from Tables 1A, 2, or 3A; or (ii) RNA transcripts or expression products thereof of one or more genes selected from Tables 1A, 2 or 3A in a test sample obtained from the subject is higher relative to the level of expression in a control, wherein the higher level of expression is indicative of the presence of an IBD in the subject from which the test sample was obtained.

In another embodiment, the methods of diagnosing or detecting the presence of an IBD in a mammalian subject comprise determining that the expression level of (i) one or more nucleic acids encoding one or more polypeptides selected from Tables 1B or 3B; or (ii) RNA transcripts or expression products thereof of one or more genes selected from Tables 1B or 3B in a test sample obtained from the subject is lower relative to the level of expression in a control, wherein the lower level of expression is indicative of the presence of an IBD in the subject from which the test sample was obtained.

In one aspect, the methods are directed to diagnosing or detecting a flare-up of an IBD in mammalian subject that was previously diagnosed with an IBD and is currently in remission. The subject may have completed treatment for the IBD or is currently undergoing treatment for the IBD. In one embodiment, the methods comprise determining, in a biological sample obtained from the mammalian subject, that the expression level of (i) one or more nucleic acids encoding one or more polypeptides selected from Tables 1, 2, or 3; or (ii) RNA transcripts or expression products thereof of one or more genes selected from Tables 1, 2 or 3 is different relative to the expression level in a control, wherein the difference in expression indicates the subject is more likely to have an IBD flareup. Alternatively, the test sample may be compared to a prior test sample of the mammalian subject, if available, obtained before, after, or at the time of the intial IBD diagnosis.

In all aspects, the mammalian subject preferably is a human patient, such as a human patient diagnosed with or at risk of developing an IBD. The subject may also be an IBD patient who has received prior treatment for an IBD but is at risk of a recurrence of the IBD.

For all aspects of the method of the invention, determining the expression level of one or more genes described herein (or one or more nucleic acids encoding polypeptide(s) expressed by one or more of such genes) may be obtained, for example, by a method of gene expression profiling. The method of gene expression profiling may be, for example, a PCR-based method.

In various embodiments, the diagnosis includes quantification of the expression level of (i) one or more nucleic acids encoding one or more polypeptides selected from Tables 1, 2, or 3; or (ii) RNA transcripts or expression products thereof of one or more genes selected from Tables 1, 2 or 3, such as by immunohistochemistry (IHC) and/or fluorescence *in situ* hybridization (FISH).

For all aspects of the invention, the expression levels of the genes may be normalized relative to the expression levels of one or more reference genes, or their expression products.

For all aspects of the invention, the method may further comprise determining evidence of the expression levels of at least two, three, four, five, six, seven, eight, nine, ten, eleven, twelve, thirteen, fourteen, fifteen, sixteen, seventeen, eighteen, nineteen, or twenty of said genes, or their expression products.

In another aspect, the methods of present invention also contemplate the use of a "panel" of such genes (i.e. IBD markers as disclosed herein) based on the evidence of their level of expression. In some embodiments, the panel of IBD markers will include at least one, two, three, four, five, six, seven, eight, nine, ten, eleven, twelve, thirteen, fourteen, fifteen, sixteen, seventeen, eighteen, nineteen or twenty IBD markers. The panel may include an IBD marker that is overexpressed in IBD relative to a control, an IBD marker that is underexpressed in IBD relative to a control, or IBD markers that are both overexpressed and underexpressed in IBD relative to a control. Such panels may be used to screen a mammalian subject for the differential expression of one or more IBD markers in order to make a determination on whether an IBD is present in the subject.

In one embodiment, the IBD markers that make up the panel are selected from Tables 1, 2, and 3. In a preferred embodiment, the methods of diagnosing or detecting the presence of an IBD in a mammalian subject comprise determining a differential expression level of RNA transcripts or expression products thereof from a panel of IBD markers in a test sample obtained from the subject relative to the level of expression in a control, wherein the differential level of expression is indicative of the presence of an IBD in the subject from which the test sample was obtained. The differential expression in the test sample may be higher and/or lower relative to a control as discussed herein.

For all aspects of the invention, the method may further comprise the step of creating a report summarizing said prediction.

For all aspects, the IBD diagnosed or detected according to the methods of the present invention is Crohn's disease (CD), ulcerative colitis (UC), or both CD and UC.

For all aspects of the invention, the test sample obtained from a mammalian subject may be derived from a colonic tissue biopsy. In a preferred embodiment, the biopsy is a tissue selected from the group consisting of terminal ileum, the ascending colon, the descending colon, and the sigmoid colon. In other preferred embodiments, the biopsy is from an inflamed colonic area or from a non-inflamed colonic area. The inflamed colonic area may be acutely inflamed or chronically inflamed.

For all aspects, determination of expression levels may occur at more than one time. For all aspects of the invention, the determination of expression levels may occur before the patient is subjected to any therapy before and/or after any surgery. In some embodiments, the determining step is indicative of a recurrence of an IBD in the mammalian subject following surgery or indicative of a flare-up of said IBD in said mammalian subject. In a preferred embodiment, the IBD is Crohn's disease.

In another aspect, the present invention concerns methods of treating a mammalian subject in which the presence of an IBD has been detected by the methods described herein. For example, following a determination that a test sample obtained from the mammalian subject exhibits differential expression relative to a control of one or more of the RNA transcripts or the corresponding gene products of an IBD marker described herein, the mammalian subject may be administered an IBD therapeutic agent.

In one embodiment, the methods of treating an IBD in a mammalian subject in need thereof, comprise (a) determining a differential level of expression of (i) one or more nucleic acids encoding one or more polypeptides selected from Tables 1, 2, or 3; or (ii) RNA transcripts or expression products thereof of one or more genes selected from Tables 1, 2 or 3 in a test sample obtained from said subject relative to the level of expression in a control, wherein said differential level of expression is indicative of the presence of an IBD in the subject from which the test sample was obtained; and (b) administering to said subject an effective amount of an IBD therapeutic agent. In a preferred embodiment, the methods of treating an IBD comprise (a) determining that the expression level of (i) one or more nucleic acids encoding one or more polypeptides selected from Tables 1A, 2, or 3A; or (ii) RNA transcripts or expression products thereof of one or more genes selected from Tables 1A, 2 or 3A in a test sample obtained from the subject is higher relative to the level of expression in a control, wherein the higher level of expression is indicative of the presence of an IBD in the subject from which the test sample was obtained; and (b) administering to said subject an effective amount of an IBD therapeutic agent. In another preferred embodiment, the methods of treating an IBD comprise (a) determining that the expression level of (i) one or more nucleic acids encoding one or more polypeptides selected from Tables 1B or 3B; or (ii) RNA transcripts or expression products thereof of one or more genes selected from Tables 1B or 3B in a test sample obtained from the subject is lower relative to the level of expression in a control, wherein the lower level of expression is indicative of the presence of an IBD in the subject from which the test sample was obtained. In some preferred embodiments, the IBD therapeutic agent is one or more of an aminosalicylate, a corticosteroid, and an immunosuppressive agent.

In one aspect, the panel of IBD markers discussed above is useful in methods of treating an IBD in a mammalian subject. In one embodiment, the mammalian subject is screened against the panel of markers and if the presence of an IBD is determined, IBD therapeutic agent(s) may be administered as discussed herein.

In a different aspect the invention concerns a kit comprising one or more of (I) extraction buffer/reagents and protocol; (2) reverse transcription buffer/reagents and protocol; and (3) qPCR buffer/reagents and protocol suitable for performing the methods of this invention. The kit may comprise data retrieval and analysis software.

In one embodiment, the gene whose differential expression is indicative of an IBD is GLI1. In another embodiment, the GLI1 gene is a GLI1 variant. In a preferred embodiment, the GLI1 variant is rs2228226C→G (Q1100E) as described in Example 4.

All publications mentioned herein are incorporated herein by reference to disclose and describe the methods and/or materials in connection with which the publications are cited. Publications cited herein are cited for their disclosure prior to the filing date of the present application. Nothing here is to be construed as an admission that the inventors are not entitled to antedate the publications by virtue of an earlier priority date or prior date of invention. Further the actual publication dates may be different from those shown and require independent verification.

### Brief Description of Drawings

Figure 1 shows the histologically normal biopsies from control patients that were analysed by unsupervised hierarchical clustering.
Figure 2 shows the expression of defensins alpha 5 and 6 in ulcerative colitis patients and controls.
Figure 3 shows the expression of the matrix metalloproteinases (MMPs) 3 and 7 in ulcerative colitis and controls.
Figure 4 shows the real time PCR expression of SAA1, IL-8, defensin alpha 5, and defensin alpha 6 in control and ulcerative colitis inflamed and non-inflamed sigmoid colon biopsies.
Figure 5 shows the real time PCR expression of MMP3, MMP7, S100A8, and TLR4 in control and ulcerative colitis inflamed and non-inflamed sigmoid colon biopsies.
Figure 6 shows *in situ* hybridization of defensin alpha 5 in the terminal ileum and colon of patients with ulcerative colitis and controls.
Figure 7 shows *in situ* hybridization of defensin alpha 6 in the terminal ileum and colon of patients with ulcerative colitis and controls.

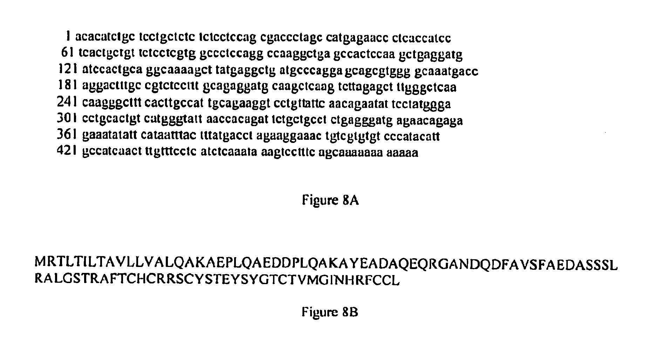

Figure 8A and 8B depict the nucleic acid sequence (SEQ ID NO:1) encoding human DEFA6 polypeptide and the amino acid sequence of human DEFA6 polypeptide (SEQ ID NO:2).

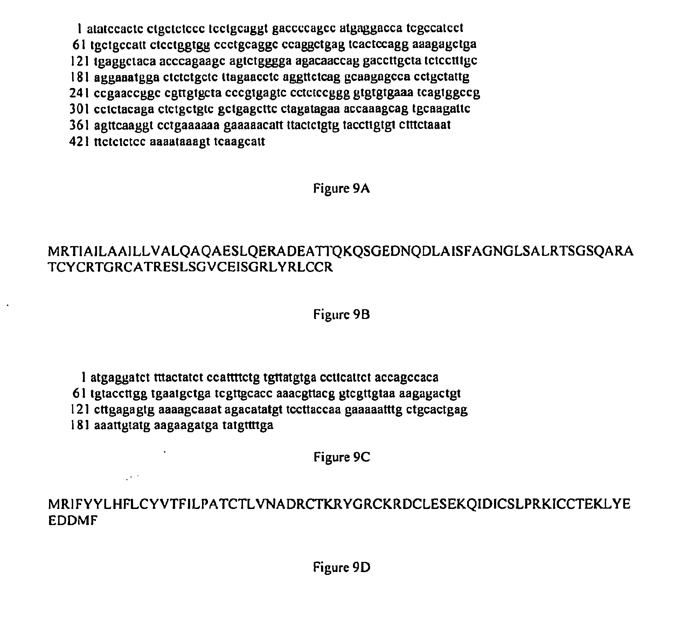

Figure 9A and 9B depict the nucleic acid sequence (SEQ ID NO:3) encoding human DEFA5 polypeptide and the amino acid sequence of human DEFA5 polypeptide (SEQ ID NO:4). Figure 9C and 9D depict the nucleic acid sequence (SEQ ID NO:210) encoding human DEFB14 polypeptide and the amino acid sequence of human DEFB14 polypeptide (SEQ ID NO:211).

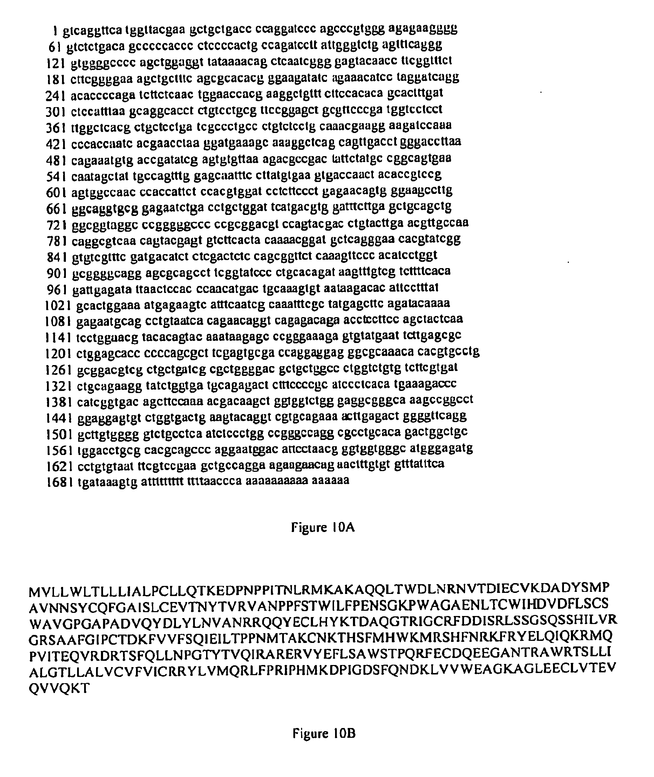

Figure 10A and 10B depict the nucleic acid sequence (SEQ ID NO:5) encoding human IL3RA polypeptide and the amino acid sequence of human IL3RA polypeptide (SEQ ID NO:6).

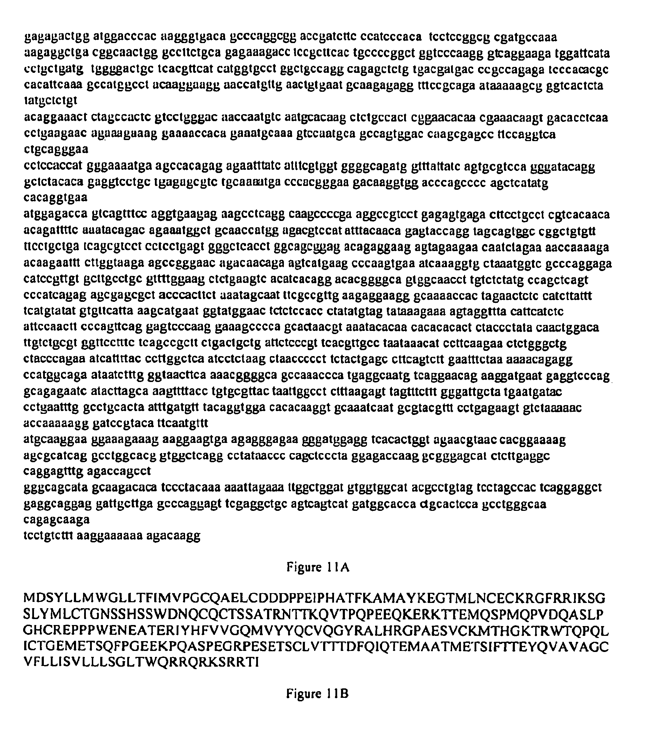

Figure 11A and 11B depict the nucleic acid sequence (SEQ ID NO:7) encoding human IL2RA polypeptide and the amino acid sequence of human IL2RA polypeptide (SEQ ID NO:8).

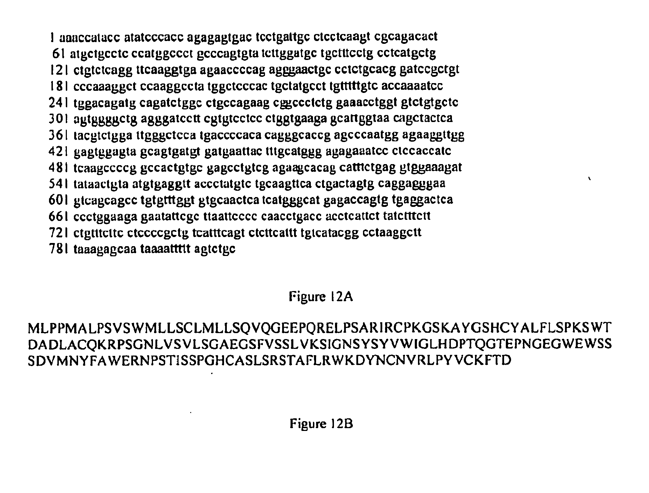

Figure 12A and 12B depict the nucleic acid sequence (SEQ ID NO:9) encoding human REG3G polypeptide and the amino acid sequence of human REG3G polypeptide (SEQ ID NO:10).

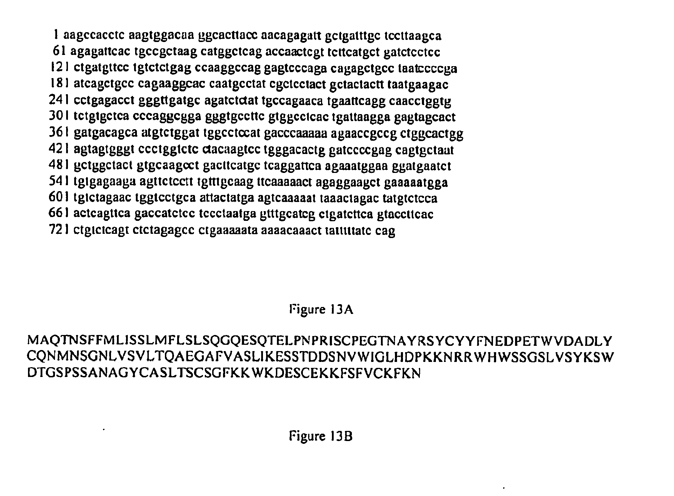

Figure 13A and 13B depict the nucleic acid sequence (SEQ ID NO:11) encoding human REG1B polypeptide and the amino acid sequence of human REG1B polypeptide (SEQ ID NO:12).

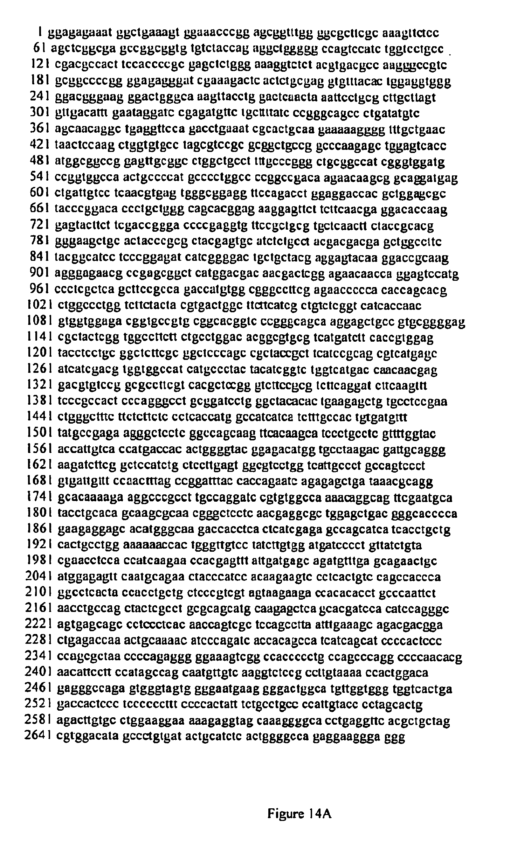

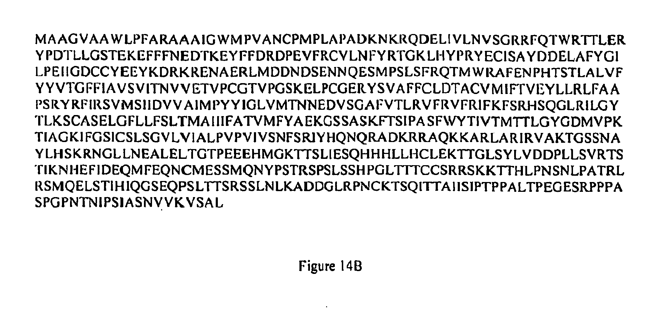

Figure 14A and 14B depict the nucleic acid sequence (SEQ ID NO:13) encoding human KCND3 polypeptide and the amino acid sequence of human KCND3 polypeptide (SEQ ID NO:14).

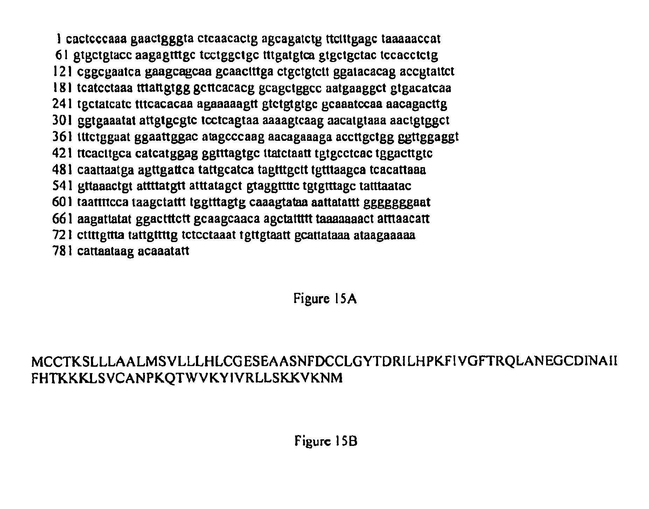

Figure 15A and 15B depict the nucleic acid sequence (SEQ ID NO:15) encoding human MIP-3a polypeptide and the amino acid sequence of human MIP-3a polypeptide (SEQ ID NO:16).

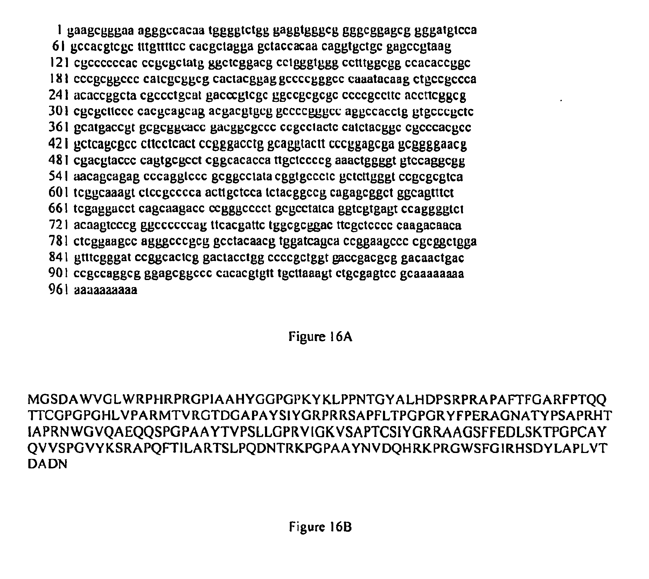

Figure 16A and 16B depict the nucleic acid sequence (SEQ ID NO:17) encoding human ECGF1 polypeptide and the amino acid sequence of human ECGF 1 polypeptide (SEQ ID NO:18).

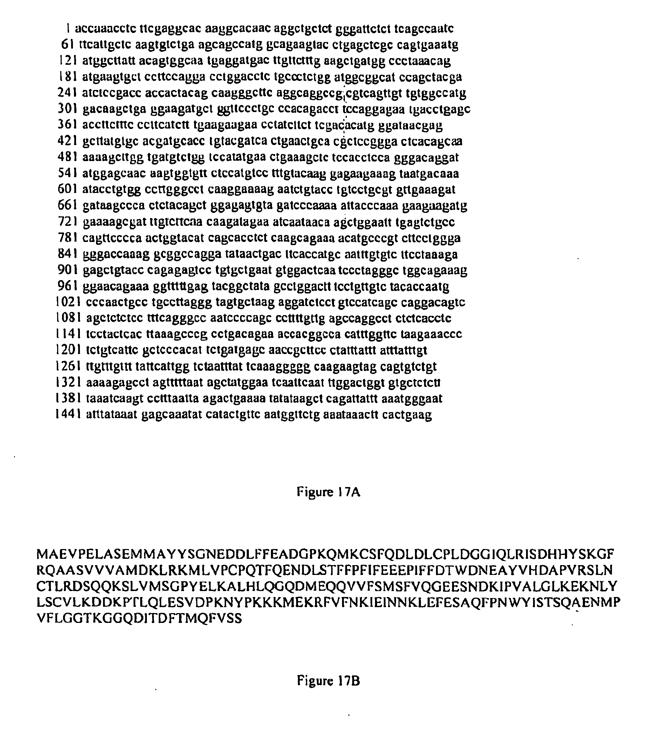

Figure 17A and 17B depict the nucleic acid sequence (SEQ ID NO:19) encoding human IL1B polypeptide and the amino acid sequence of human IL1B polypeptide (SEQ ID NO:20).

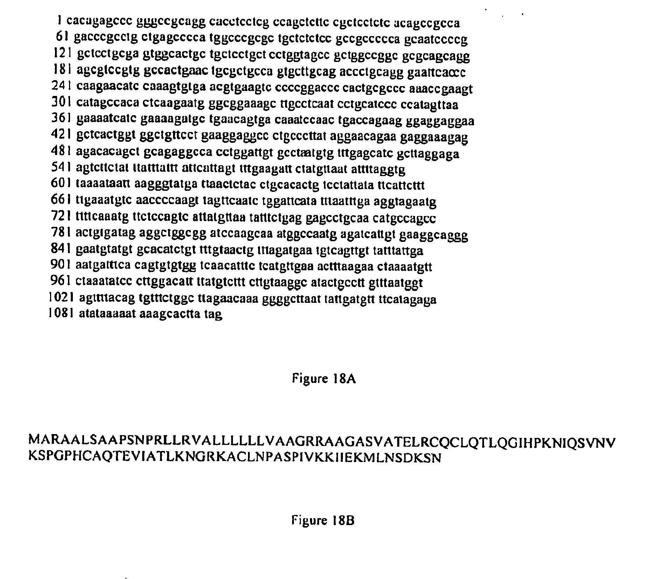

Figure 18A and 18B depict the nucleic acid sequence (SEQ ID NO:21) encoding human MIP2BGRO-g polypeptide and the amino acid sequence of human MIP2BGRO-g polypeptide (SEQ ID NO:22).

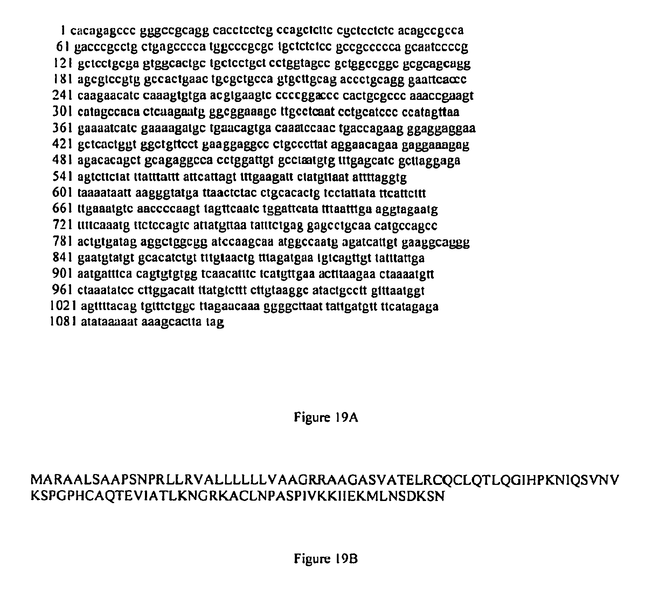

Figure 19A and 19B depict the nucleic acid sequence (SEQ ID NO:23) encoding human CXCL1 polypeptide and the amino acid sequence of human CXCL1 polypeptide (SEQ ID NO:24).

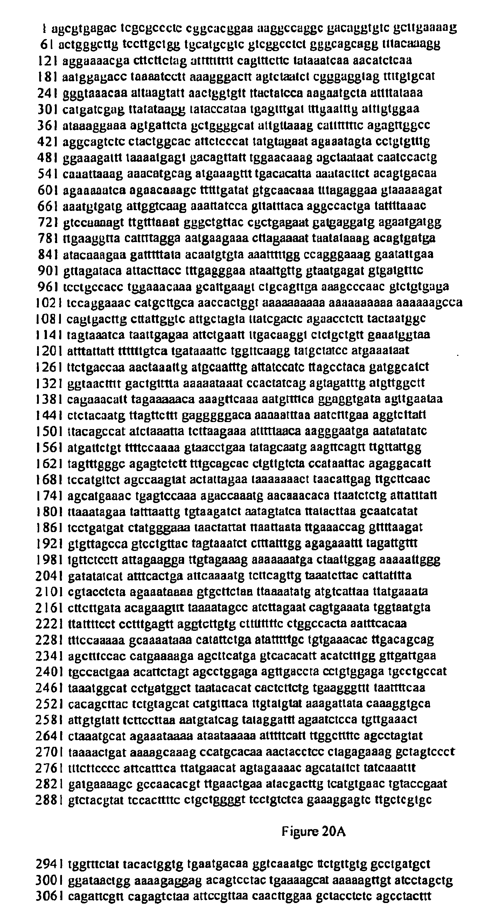

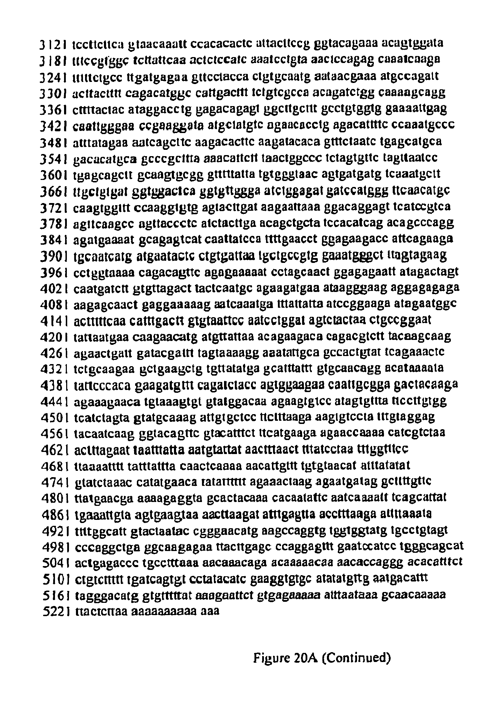

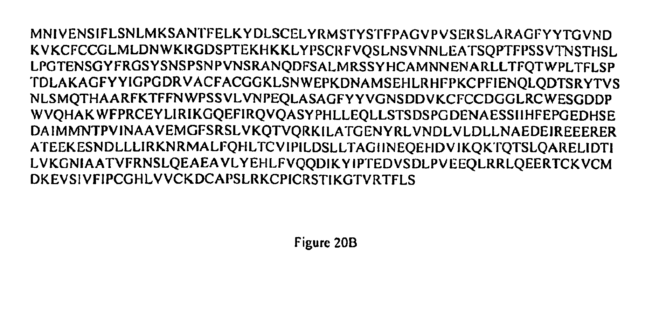

Figure 20A and 20B depict the nucleic acid sequence (SEQ ID NO:25) encoding human IAP1 polypeptide and the amino acid sequence of human IAP1 polypeptide (SEQ ID NO:26).

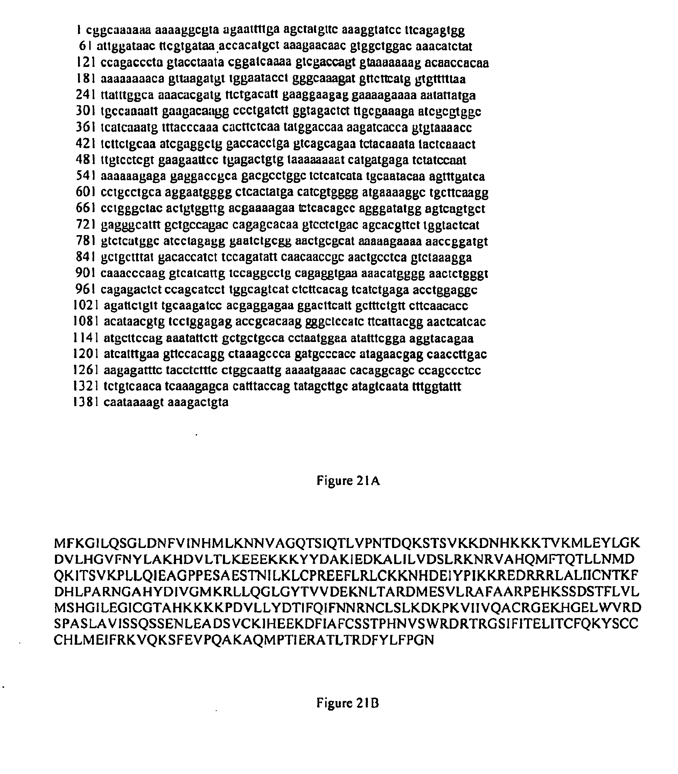

Figure 21A and 21B depict the nucleic acid sequence (SEQ ID NO:27) encoding human CASP5 polypeptide and the amino acid sequence of human CASP5 polypeptide (SEQ ID NO:28).

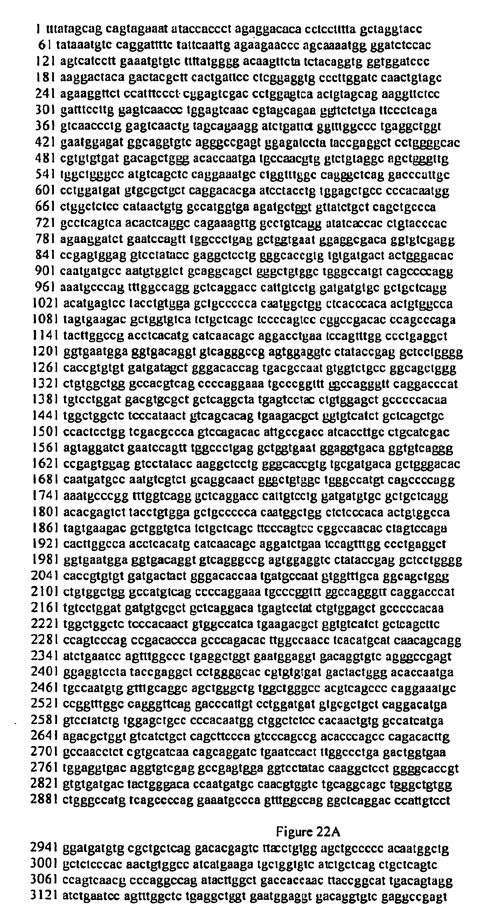

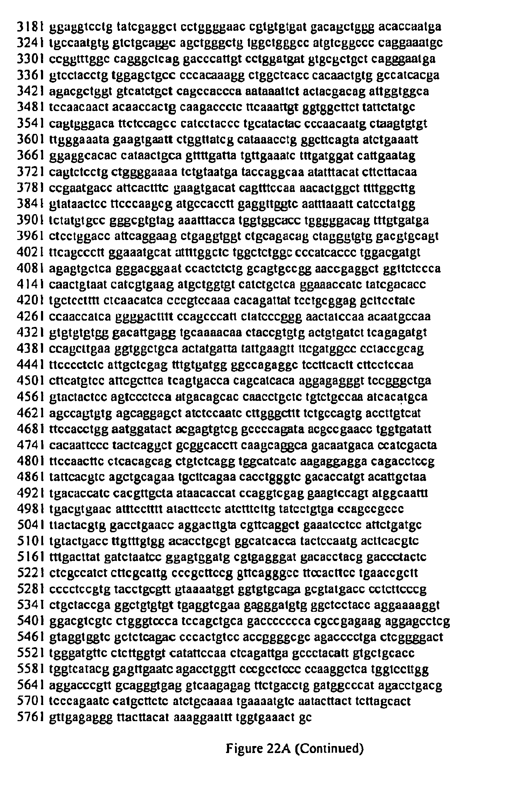

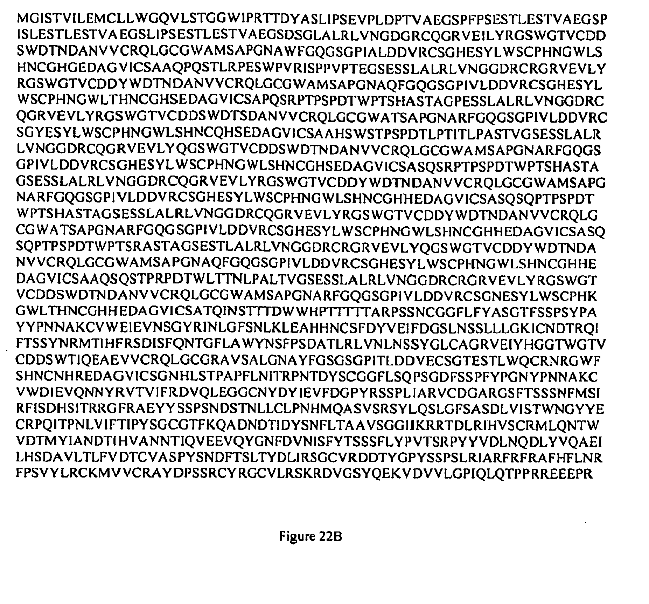

Figure 22A and 22B depict the nucleic acid sequence (SEQ ID NO:29) encoding human DMBT1 polypeptide and the amino acid sequence of human DMBT1 polypeptide (SEQ ID NO:30).

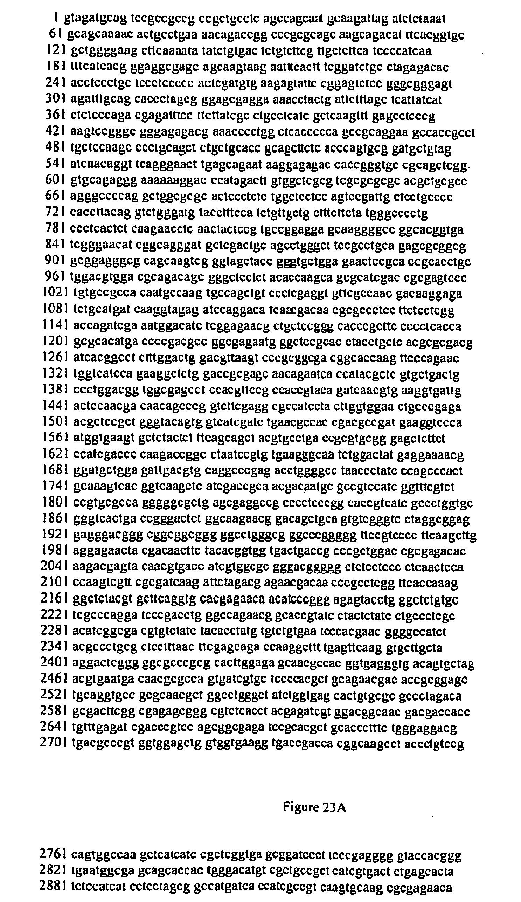

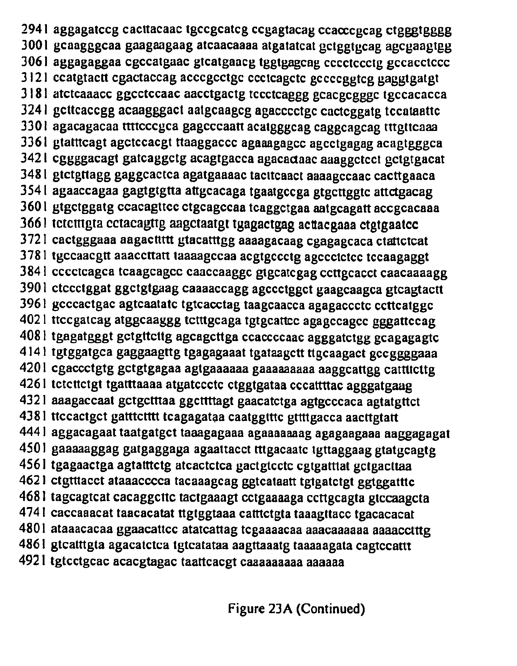

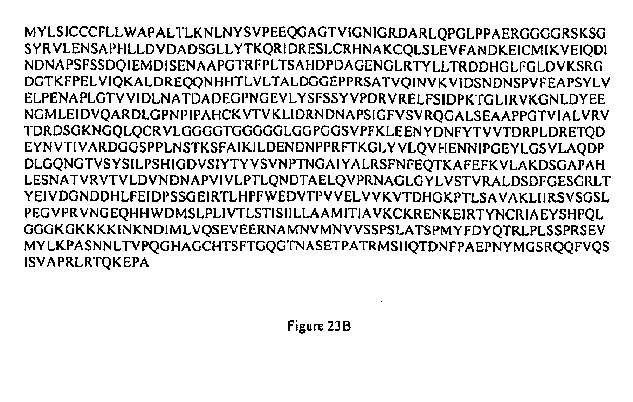

Figure 23A and 23B depict the nucleic acid sequence (SEQ ID NO:31) encoding human PCDH17 polypeptide and the amino acid sequence of human PCDH17 polypeptide (SEQ ID NO:32).

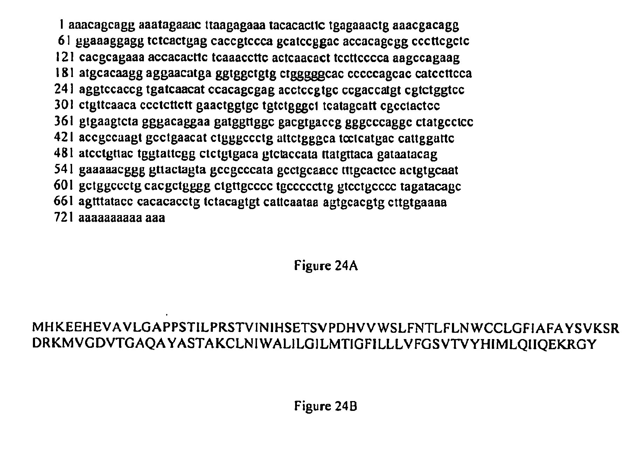

Figure 24A and 24B depict the nucleic acid sequence (SEQ ID NO:33) encoding human IFITM1 polypeptide and the amino acid sequence of human IFITM1 polypeptide (SEQ ID NO:34).

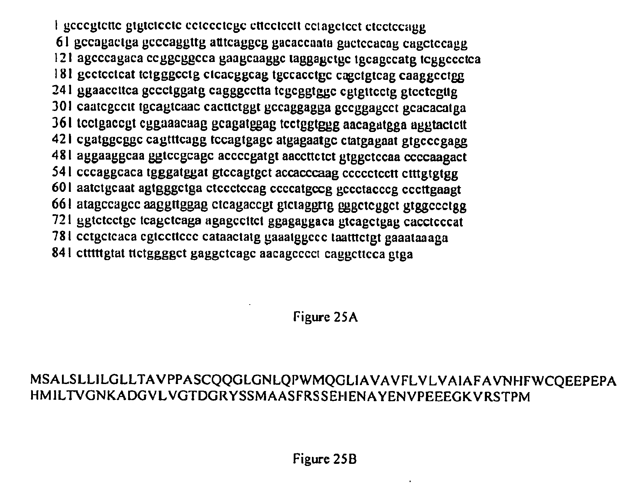

Figure 25A and 25B depict the nucleic acid sequence (SEQ ID NO:35) encoding human PDZK1IP1 polypeptide and the amino acid sequence of human PDZK1IP1 polypeptide (SEQ ID NO:36).

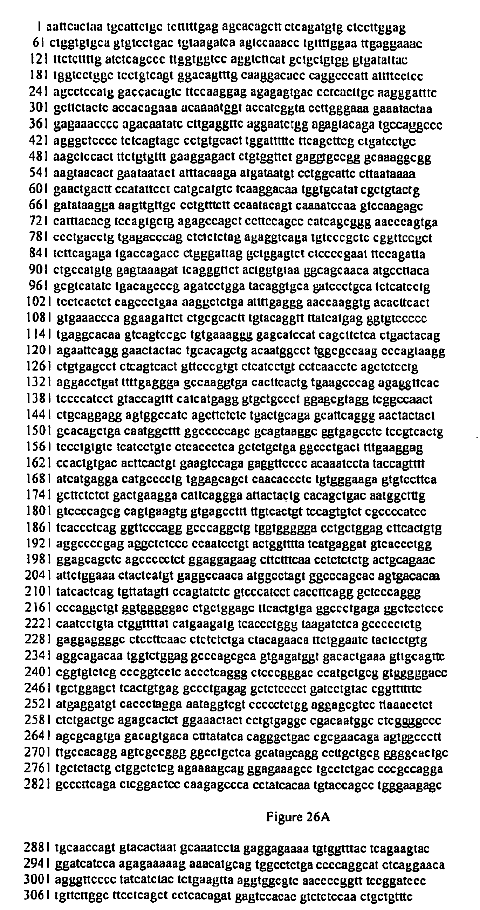

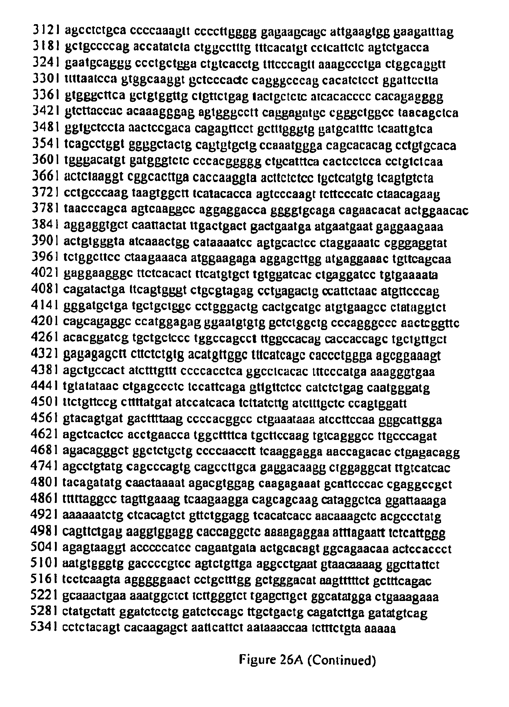

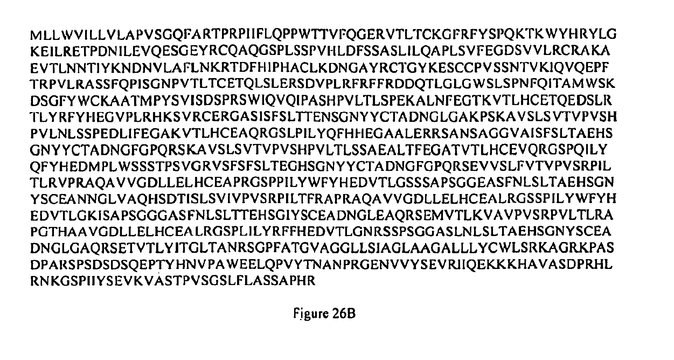

Figure 26A and 26B depict the nucleic acid sequence (SEQ ID NO:37) encoding human IRTA2 polypeptide and the amino acid sequence of human IRTA2 polypeptide (SEQ ID NO:38).

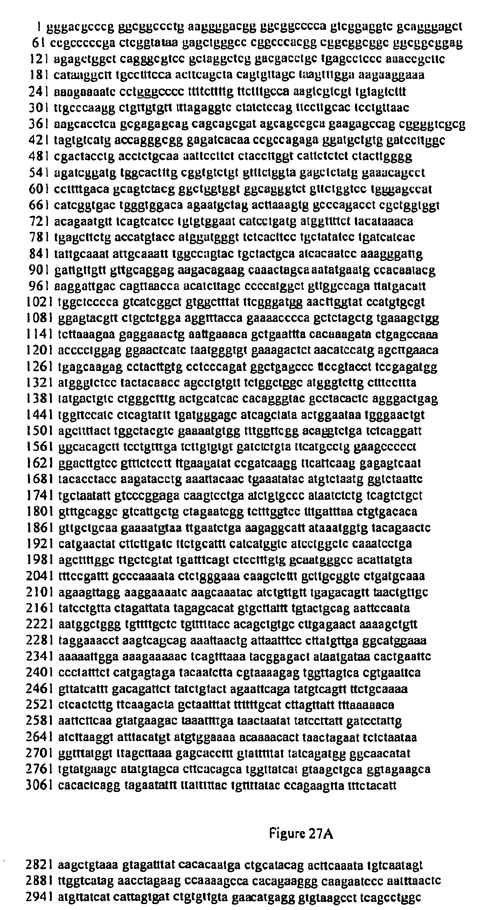

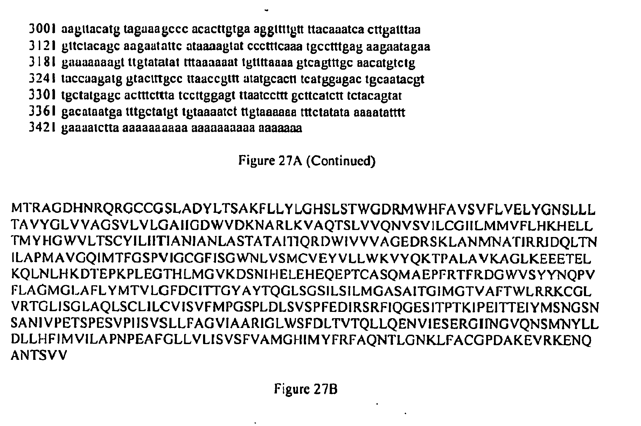

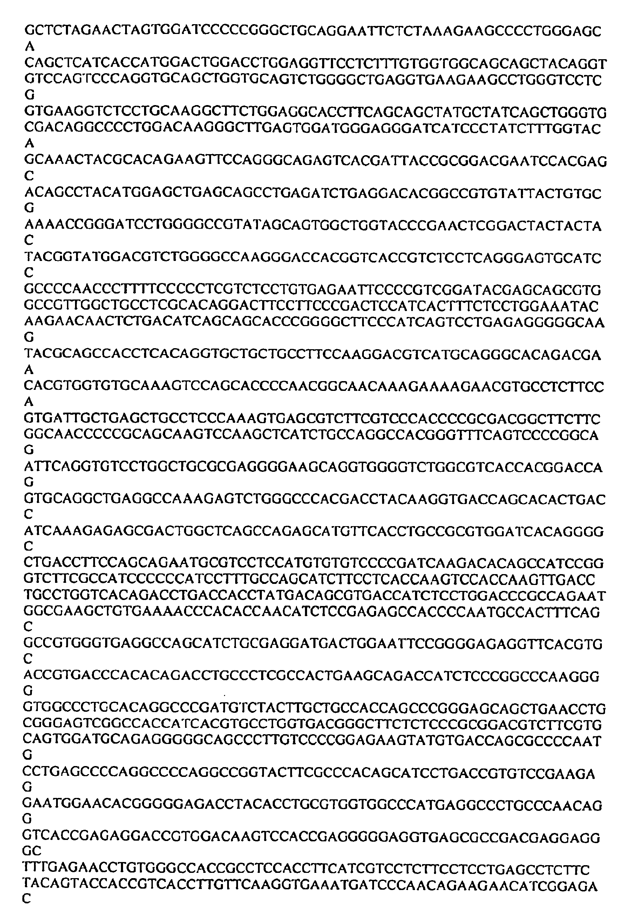

Figure 27A and 27B depict the nucleic acid sequence (SEQ ID NO:39) encoding human SLC40A polypeptide and the amino acid sequence of human SLC40A polypeptide (SEQ ID NO:40).

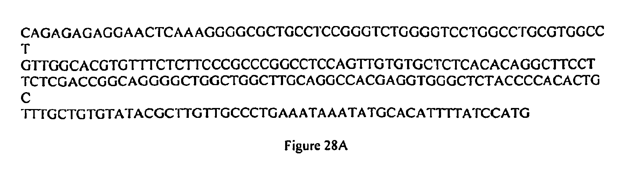

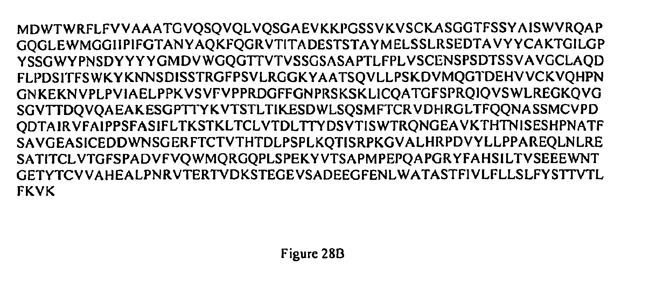

Figure 28A and 28B depict the nucleic acid sequence (SEQ ID NO:41) encoding human IGHV4-4 polypeptide and the amino acid sequence of human IGHV4-4 polypeptide (SEQ ID NO:42).

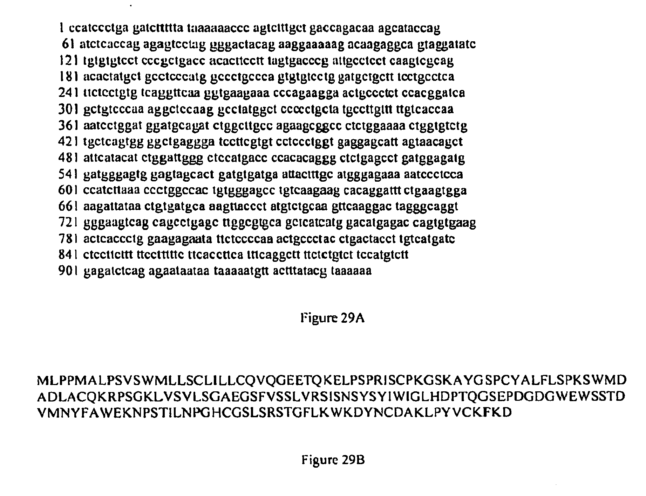

Figure 29A and 29B depict the nucleic acid sequence (SEQ ID NO:43) encoding human REG3G polypeptide and the amino acid sequence of human REG3G polypeptide (SEQ ID NO:44).

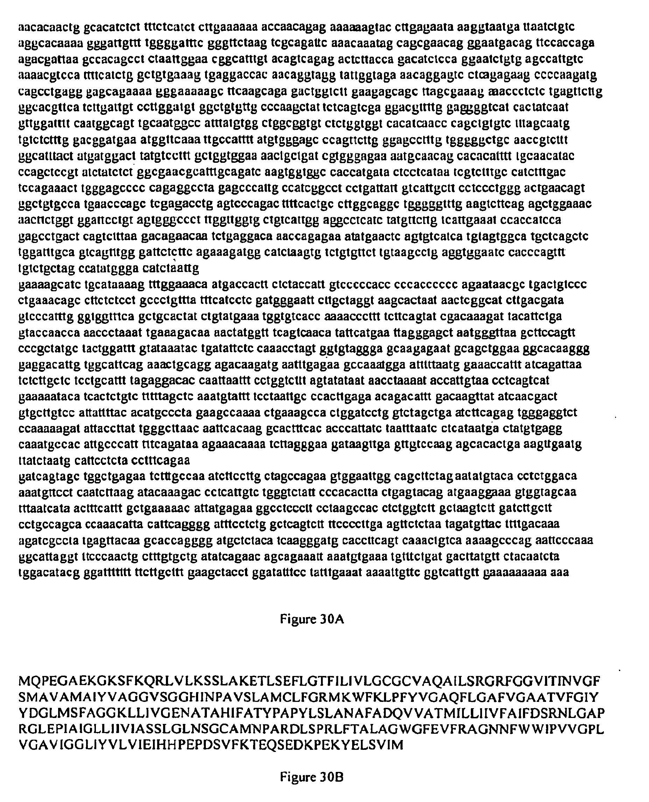

Figure 30A and 30B depict the nucleic acid sequence (SEQ ID NO:45) encoding human AQP9 polypeptide and the amino acid sequence of human AQP9 polypeptide (SEQ ID NO:46).

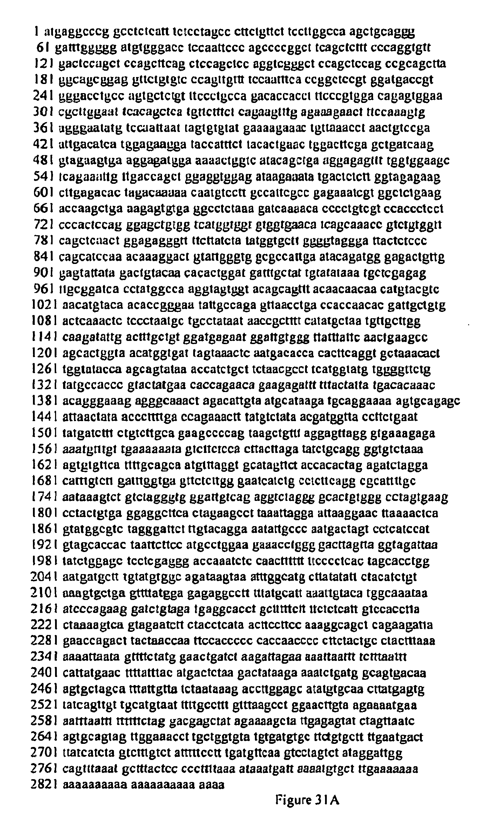

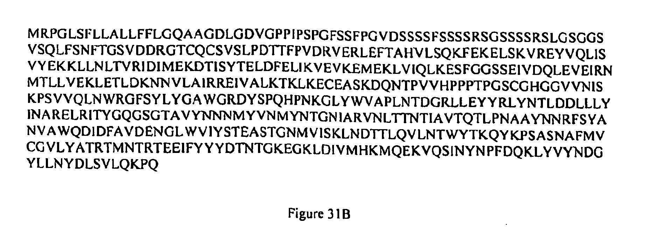

Figure 31A and 31B depict the nucleic acid sequence (SEQ ID NO:47) encoding human OLFM4 polypeptide and the amino acid sequence of human OLFM4 polypeptide (SEQ ID NO:48).

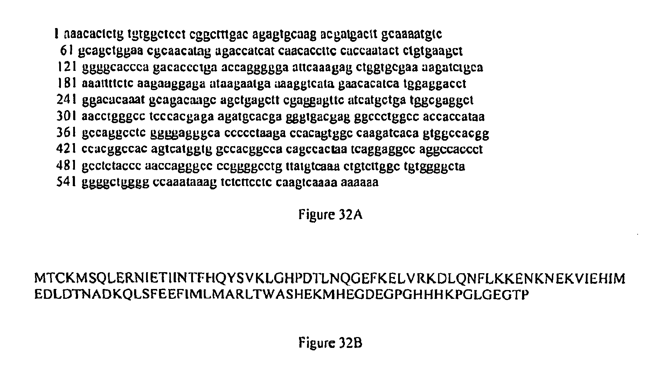

Figure 32A and 32B depict the nucleic acid sequence (SEQ ID NO:49) encoding human S100A9 polypeptide and the amino acid sequence of human S100A9 polypeptide (SEQ ID NO:50).

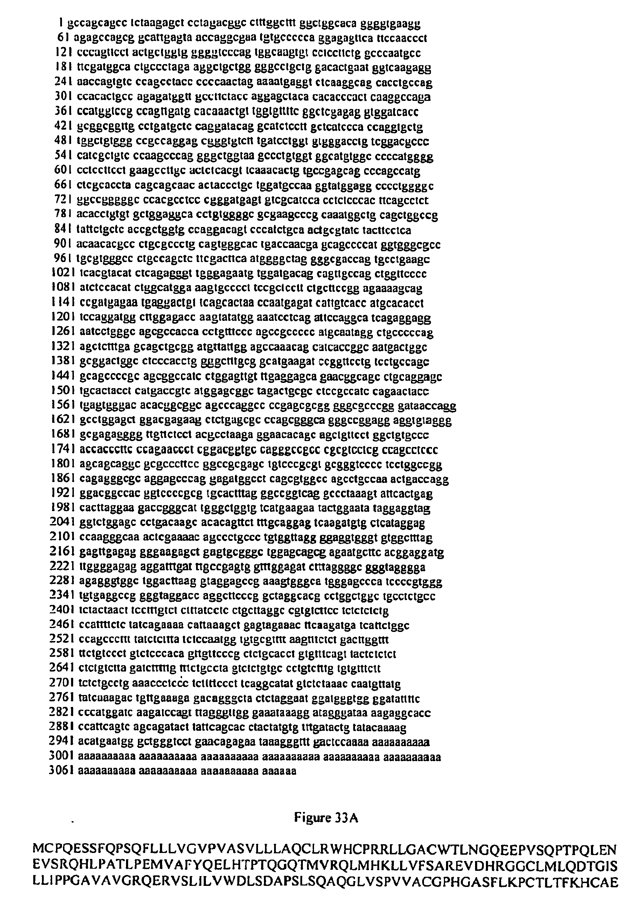

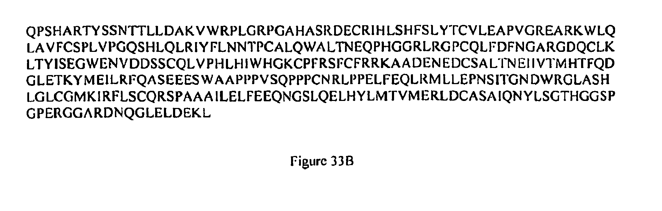

Figure 33A and 33B depict the nucleic acid sequence (SEQ ID NO:51) encoding human UNC5CL polypeptide and the amino acid sequence of human UNC5CL polypeptide (SEQ ID NO:52).

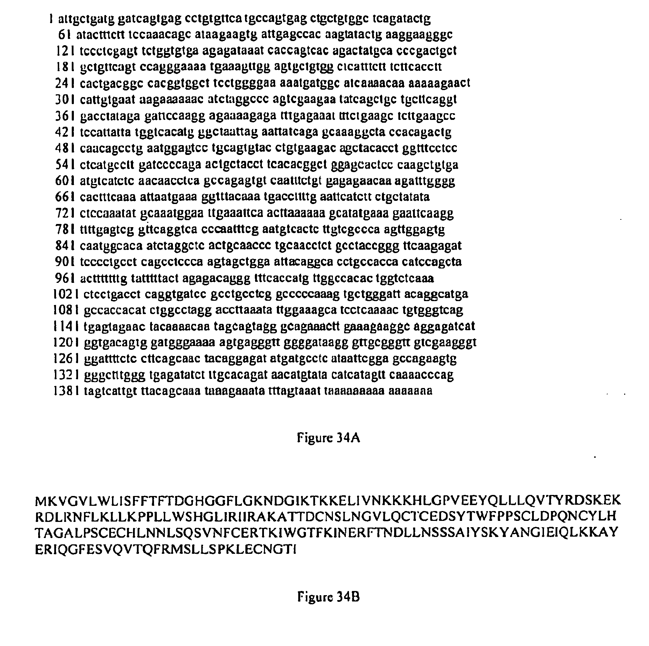

Figure 34A and 34B depict the nucleic acid sequence (SEQ ID NO:53) encoding human GPR110 polypeptide and the amino acid sequence of human GPR110 polypeptide (SEQ ID NO:54).

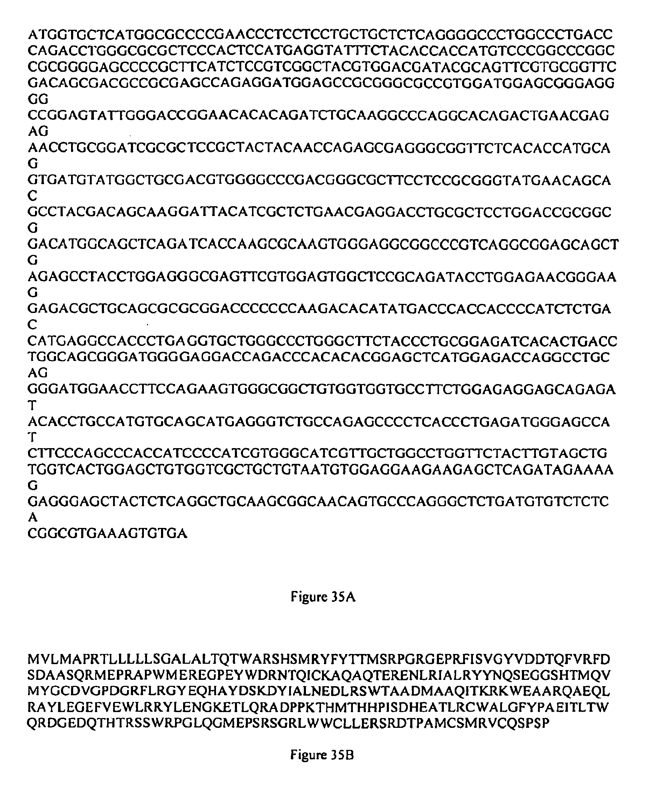

Figure 35A and 35B depict the nucleic acid sequence (SEQ ID NO:55) encoding human HLA-G polypeptide and the amino acid sequence of human HLA-G polypeptide (SEQ ID NO:56).

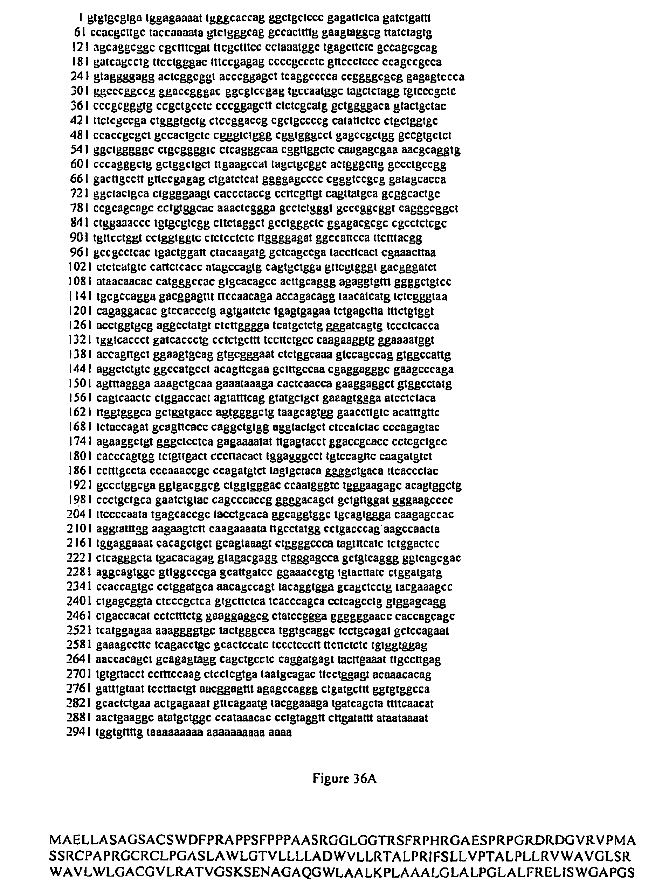

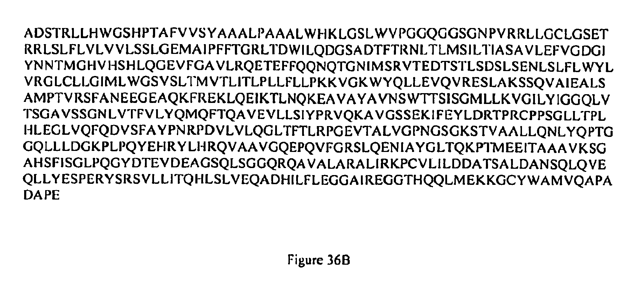

Figure 36A and 36B depict the nucleic acid sequence (SEQ ID NO:57) encoding human TAP1 polypeptide and the amino acid sequence of human TAP1 polypeptide (SEQ ID NO:58).

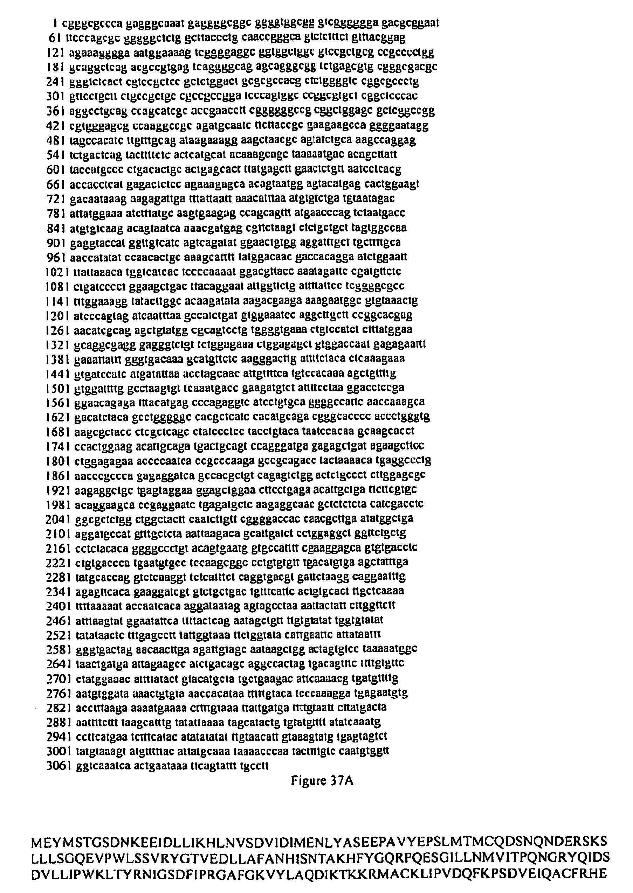

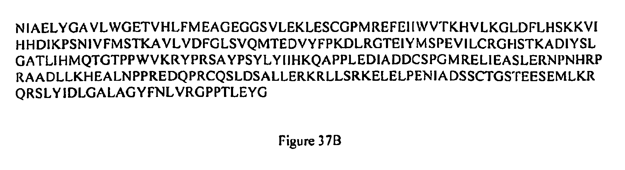

Figure 37A and 37B depict the nucleic acid sequence (SEQ ID NO:59) encoding human MAP3K8 polypeptide and the amino acid sequence of human MAP3K8 polypeptide (SEQ ID NO:60).

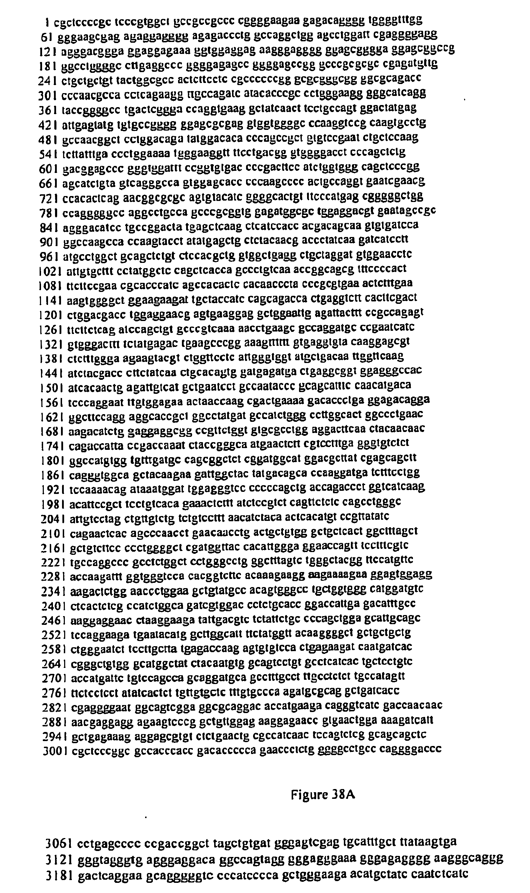

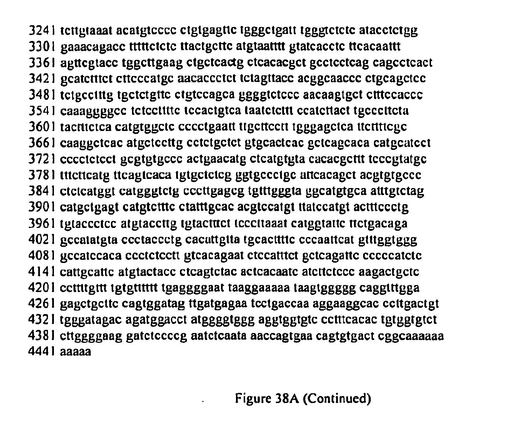

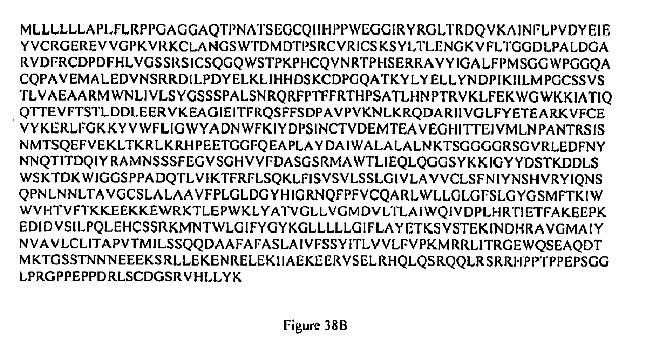

Figure 38A and 38B depict the nucleic acid sequence (SEQ ID NO:61) encoding human UBD|GABBR1 polypeptide and the amino acid sequence of human UBD|GABBR1 polypeptide (SEQ ID NO:62).

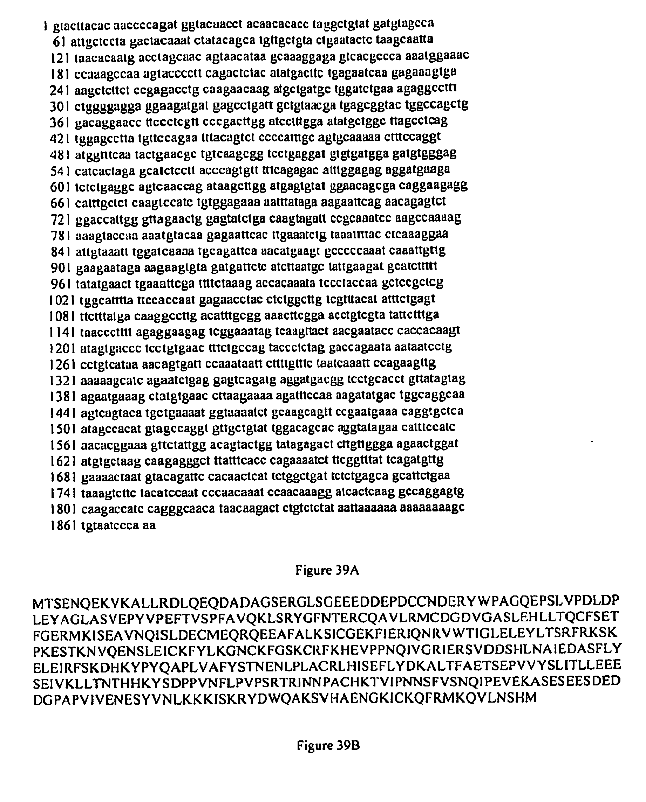

Figure 39A and 39B depict the nucleic acid sequence (SEQ ID NO:63) encoding human DHX57 polypeptide and the amino acid sequence of human DHX57 polypeptide (SEQ ID NO:64).

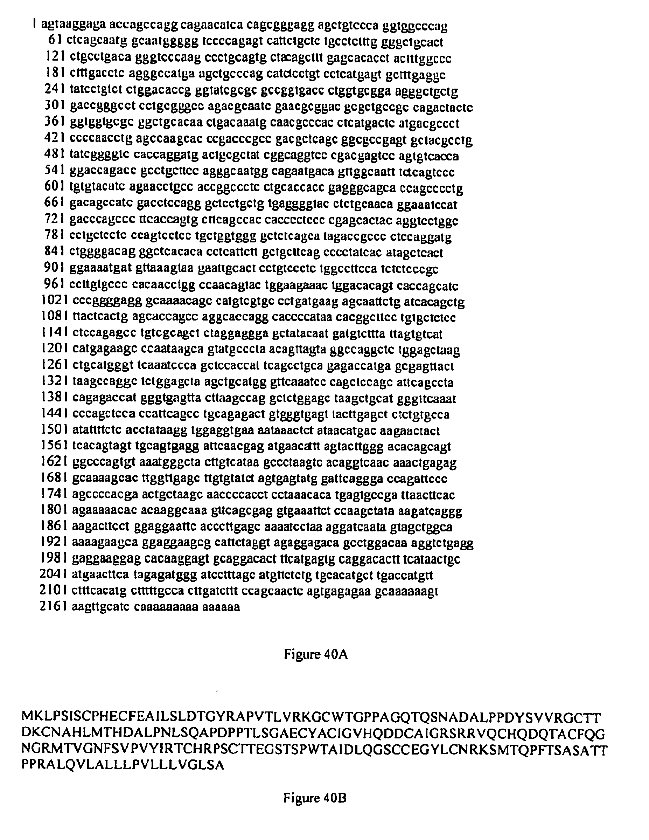

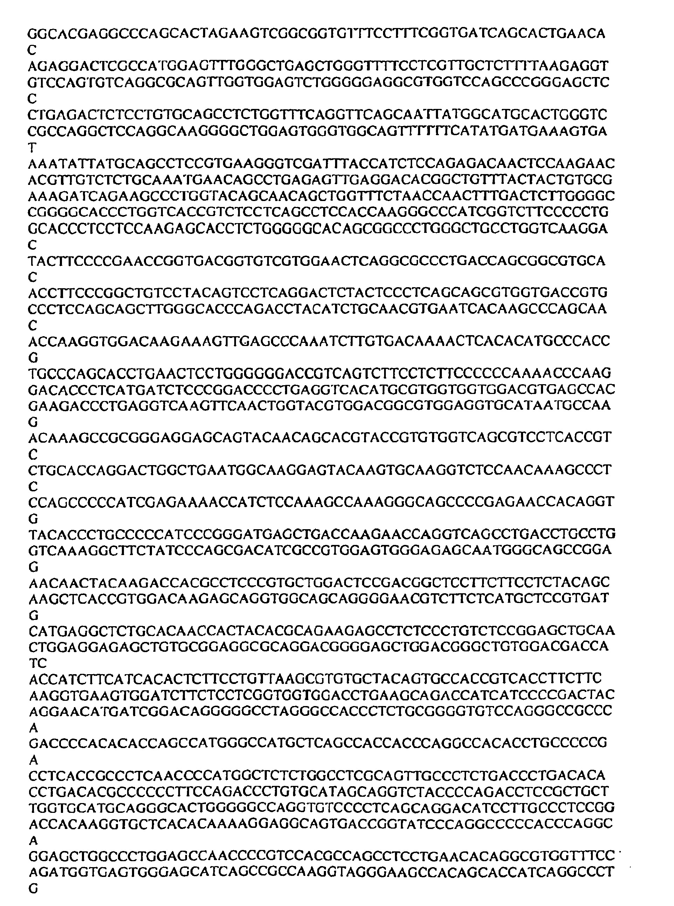

Figure 40A and 40B depict the nucleic acid sequence (SEQ ID NO:65) encoding human MA polypeptide and the amino acid sequence of human MApolypeptide (SEQ ID NO:66).

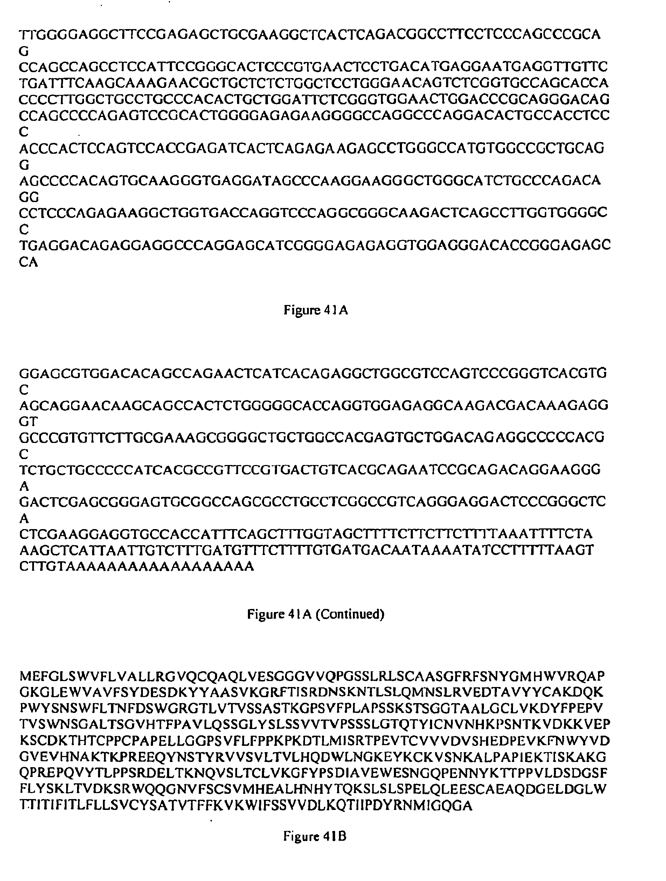

Figure 41A and 41B depict the nucleic acid sequence (SEQ ID NO:67) encoding human IGLJCOR18 polypeptide and the amino acid sequence of human IGLJCOR18 polypeptide (SEQ ID NO:68).

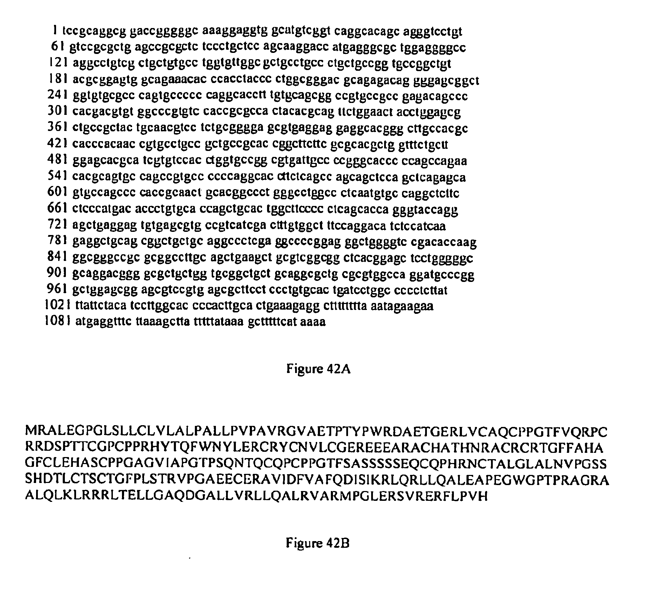

Figure 42A and 42B depict the nucleic acid sequence (SEQ ID NO:69) encoding human HLA-G polypeptide and the amino acid sequence of human HLA-G polypeptide (SEQ ID NO:70).

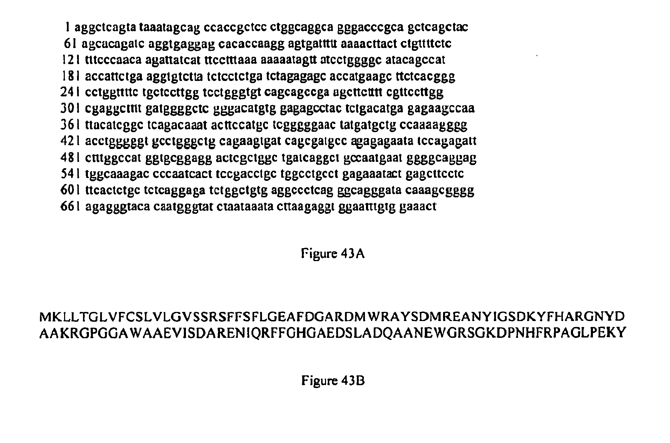

Figure 43A and 43B depict the nucleic acid sequence (SEQ ID NO:71) encoding human SAA1 polypeptide and the amino acid sequence of human SAA1 polypeptide (SEQ ID NO:72).

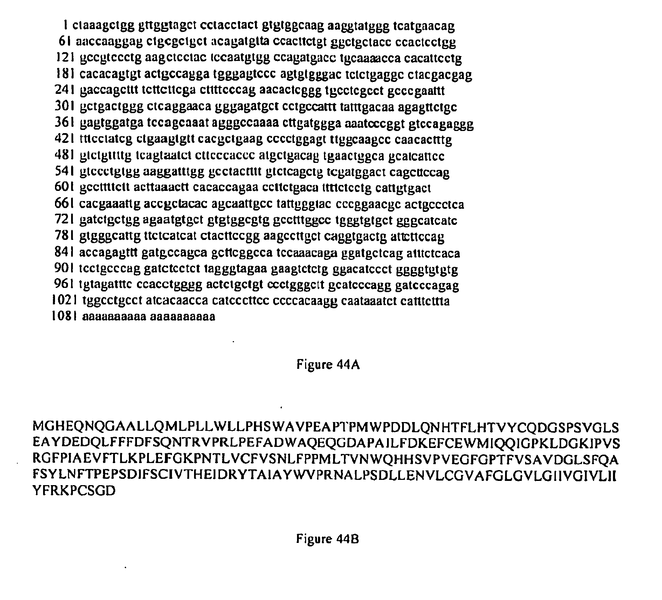

Figure 44A and 44B depict the nucleic acid sequence (SEQ ID NO:73) encoding human TAP2 polypeptide and the amino acid sequence of human TAP2 polypeptide (SEQ ID NO:74).

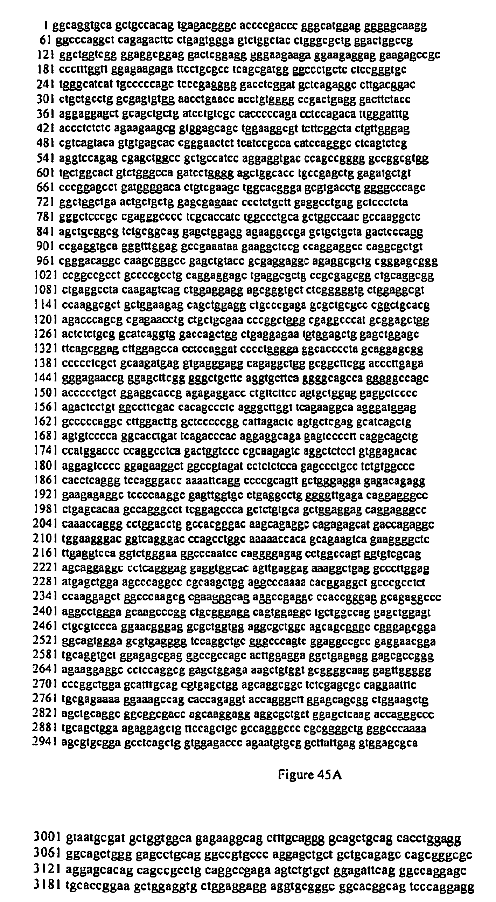

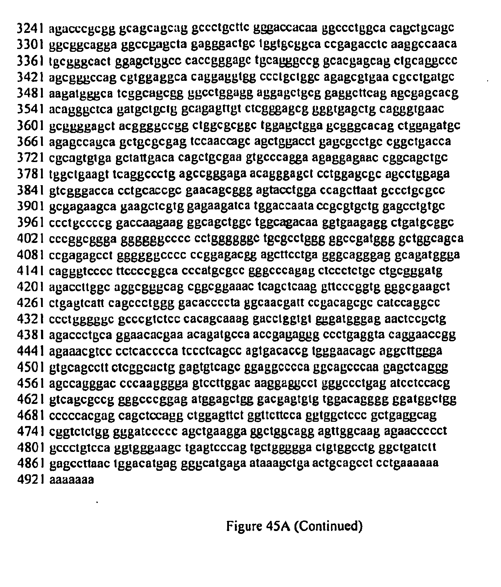

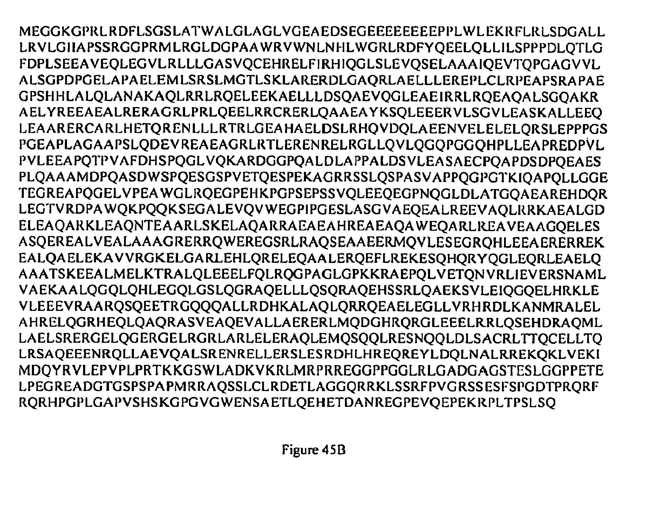

Figure 45A and 45B depict the nucleic acid sequence (SEQ ID NO:75) encoding human PCAA17448 polypeptide and the amino acid sequence of human PCAA17448 polypeptide (SEQ ID NO:76).

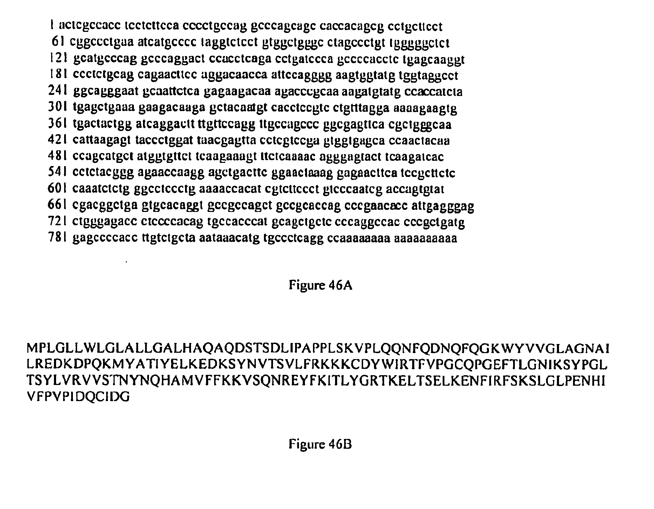

Figure 46A and 46B depict the nucleic acid sequence (SEQ ID NO:77) encoding human LCN2 polypeptide and the amino acid sequence of human LCN2 polypeptide (SEQ ID NO:78).

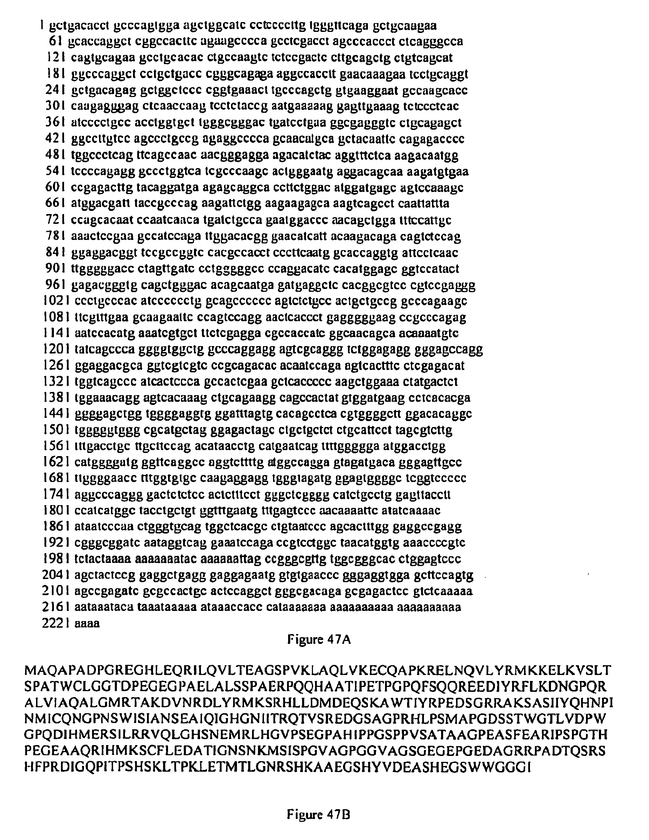

Figure 47A and 47B depict the nucleic acid sequence (SEQ ID NO:79) encoding human ZBP1 polypeptide and the amino acid sequence of human ZBP1 polypeptide (SEQ ID NO:80).

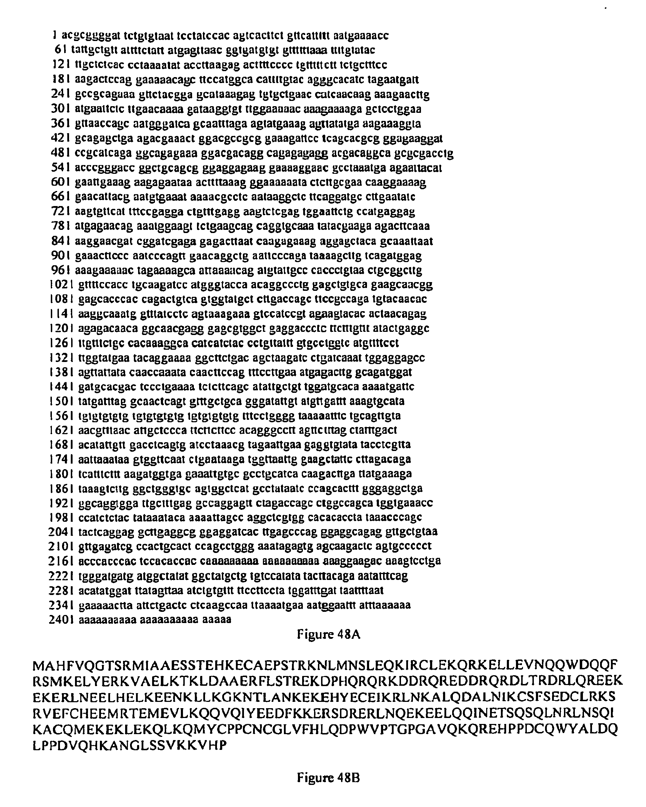

Figure 48A and 48B depict the nucleic acid sequence (SEQ ID NO:81) encoding human TNIP3 polypeptide and the amino acid sequence of human TNIP3 polypeptide (SEQ ID NO:82).

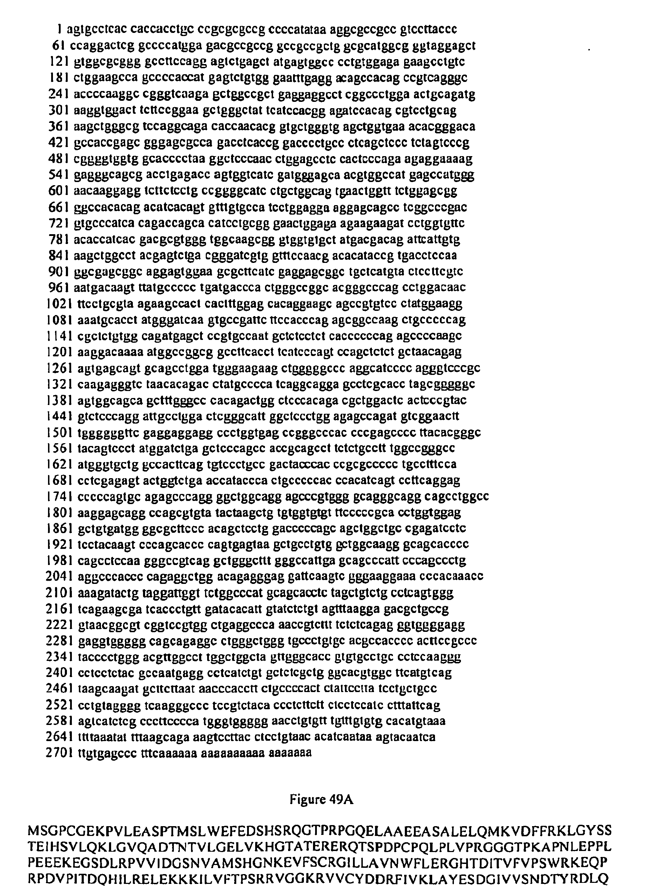

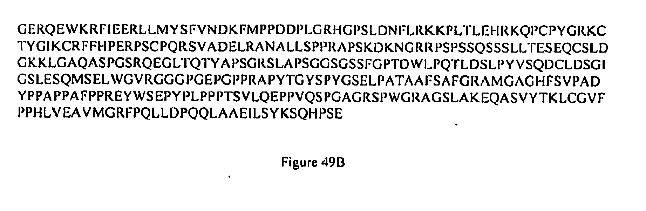

Figure 49A and 49B depict the nucleic acid sequence (SEQ ID NO:83) encoding human ZC3H12A polypeptide and the amino acid sequence of human ZC3H12A polypeptide (SEQ ID NO:84).

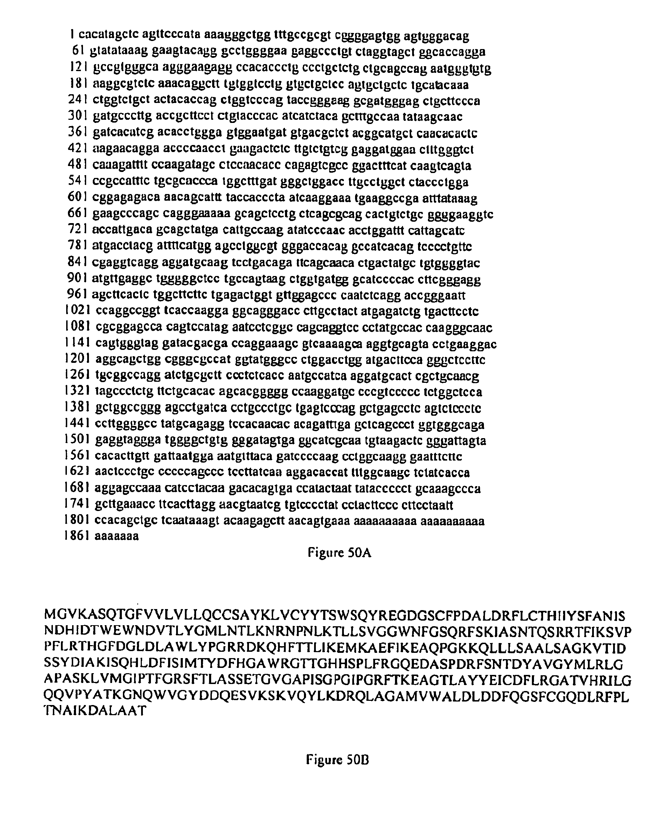

Figure 50A and 50B depict the nucleic acid sequence (SEQ ID NO:85) encoding human CHI3L1 polypeptide and the amino acid sequence of human CHI3L1 polypeptide (SEQ ID NO:86).

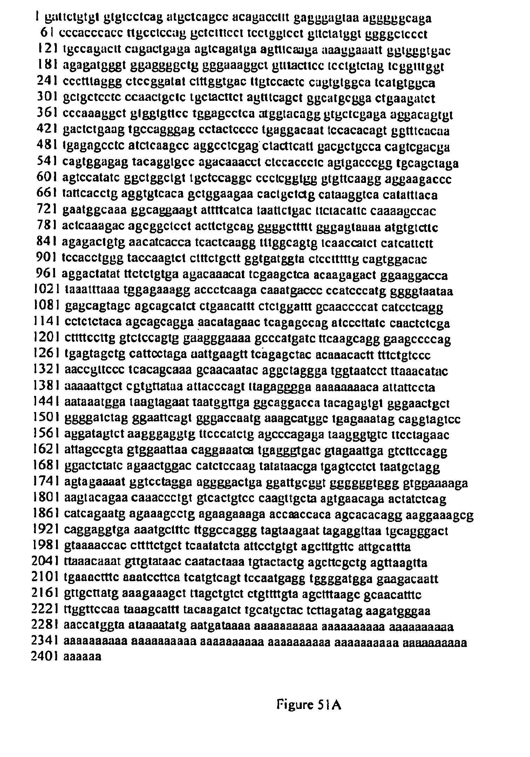

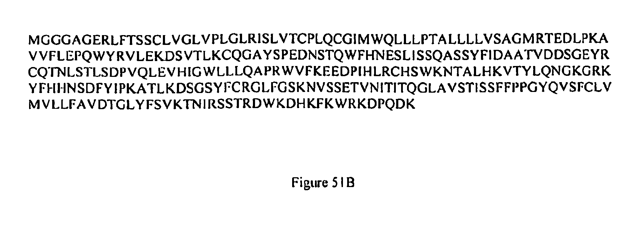

Figure 51A and 51B depict the nucleic acid sequence (SEQ ID NO:87) encoding human FCGR3A polypeptide and the amino acid sequence of human FCGR3A polypeptide (SEQ ID NO:88).

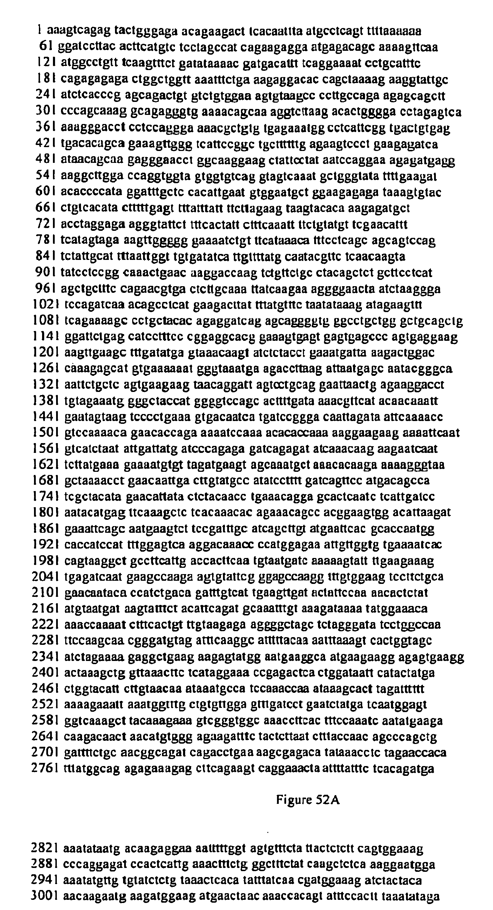

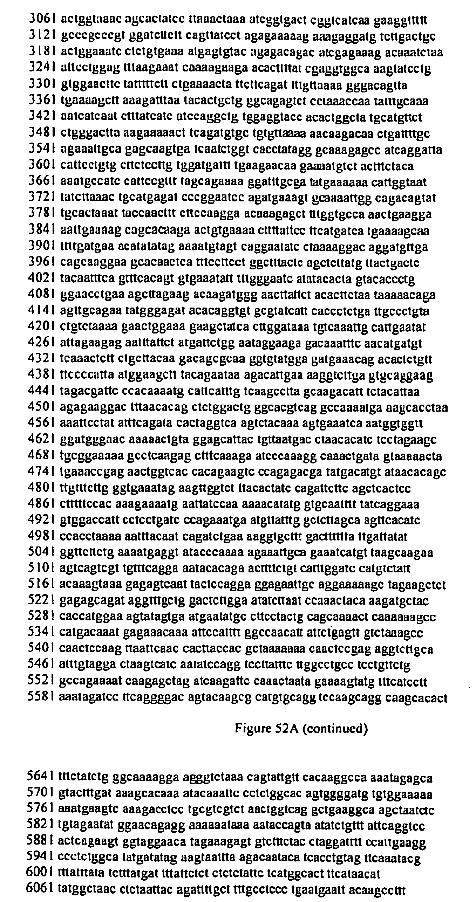

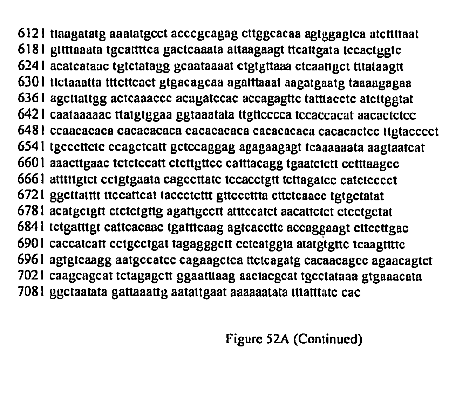

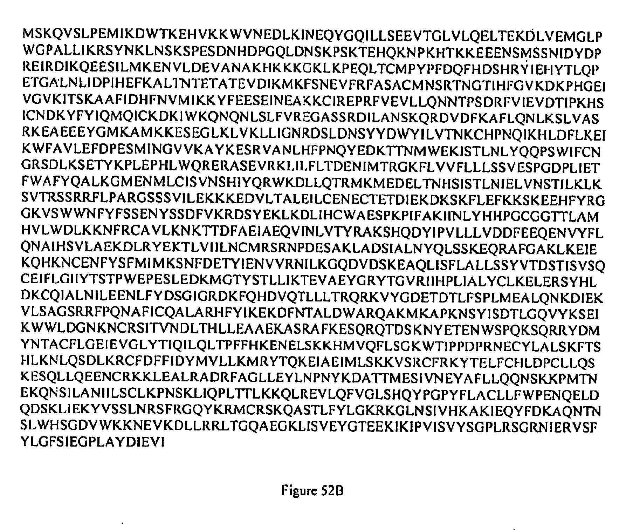

Figure 52A and 52B depict the nucleic acid sequence (SEQ ID NO:89) encoding human SAMD9L polypeptide and the amino acid sequence of human SAMD9L polypeptide (SEQ ID NO:90).

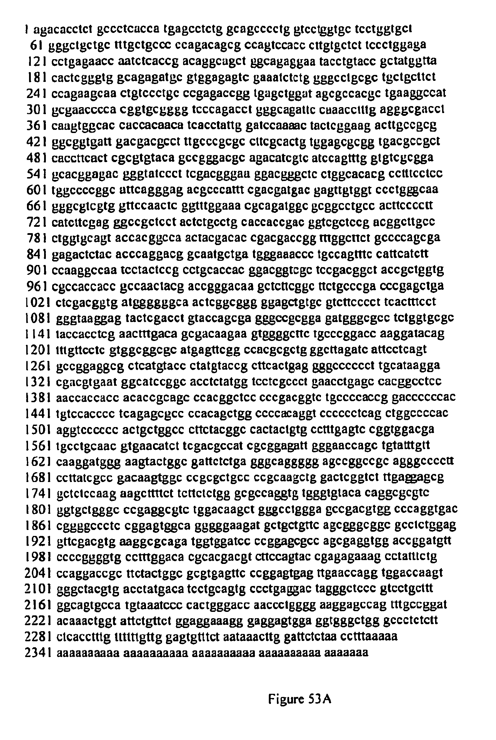

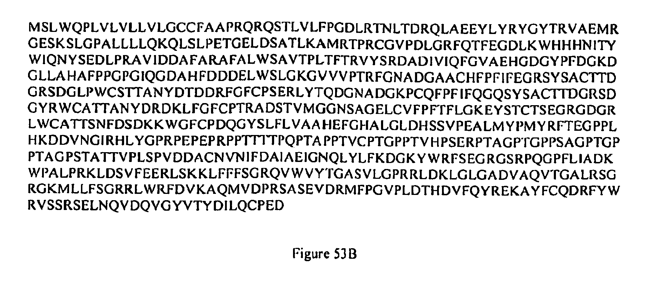

Figure 53A and 53B depict the nucleic acid sequence (SEQ ID NO:91) encoding human MMP9 polypeptide and the amino acid sequence of human MMP9 polypeptide (SEQ ID NO:92).

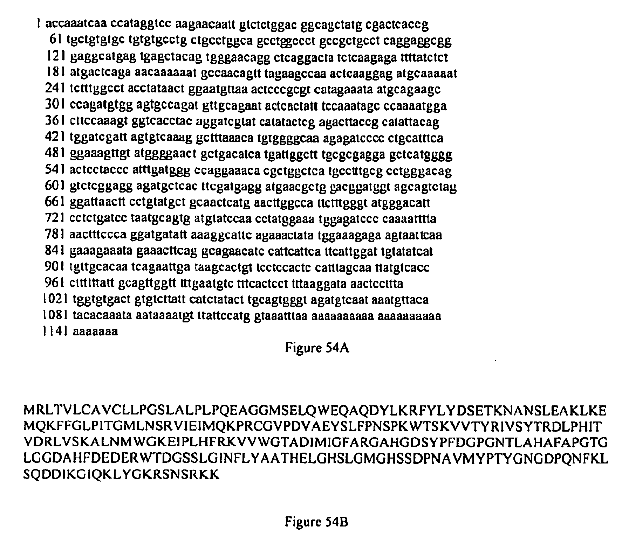

Figure 54A and 54B depict the nucleic acid sequence (SEQ ID NO:93) encoding human MMP7 polypeptide and the amino acid sequence of human MMP7 polypeptide (SEQ ID NO:94).

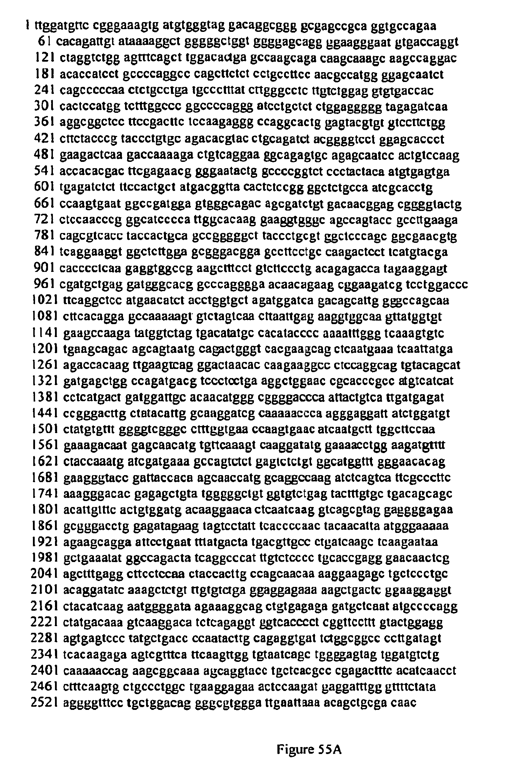

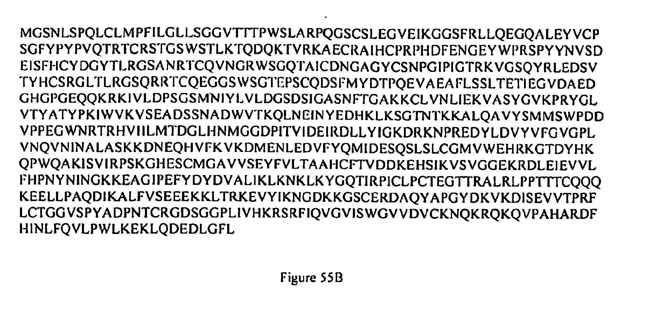

Figure 55A and 55B depict the nucleic acid sequence (SEQ ID NO:95) encoding human BF polypeptide and the am ino acid sequence of human BF polypeptide (SEQ ID NO:96).

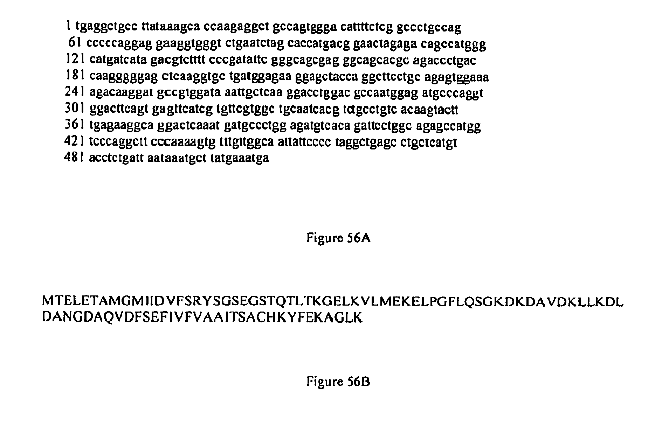

Figure 56A and 56B depict the nucleic acid sequence (SEQ ID NO:97) encoding human S100P polypeptide and the amino acid sequence of human S100P polypeptide (SEQ ID NO:98).

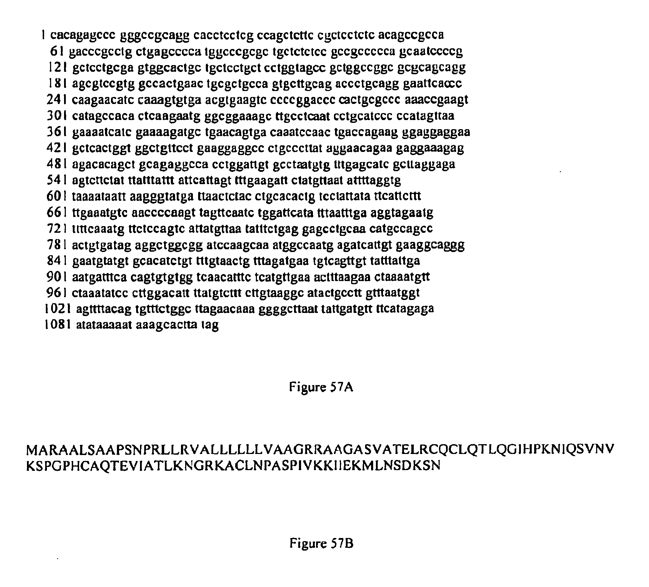

Figure 57A and 57B depict the nucleic acid sequence (SEQ ID NO:99) encoding human GRO polypeptide and the amino acid sequence of human GRO polypeptide (SEQ ID NO:100).

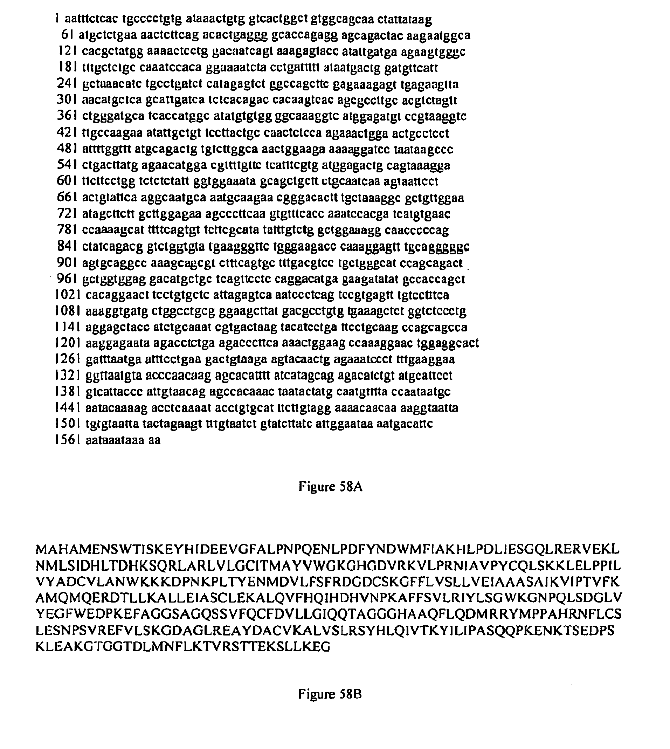

Figure 58A and 58B depict the nucleic acid sequence (SEQ ID NO:101) encoding human INDO polypeptide and the amino acid sequence of human INDO polypeptide (SEQ ID NO:102).

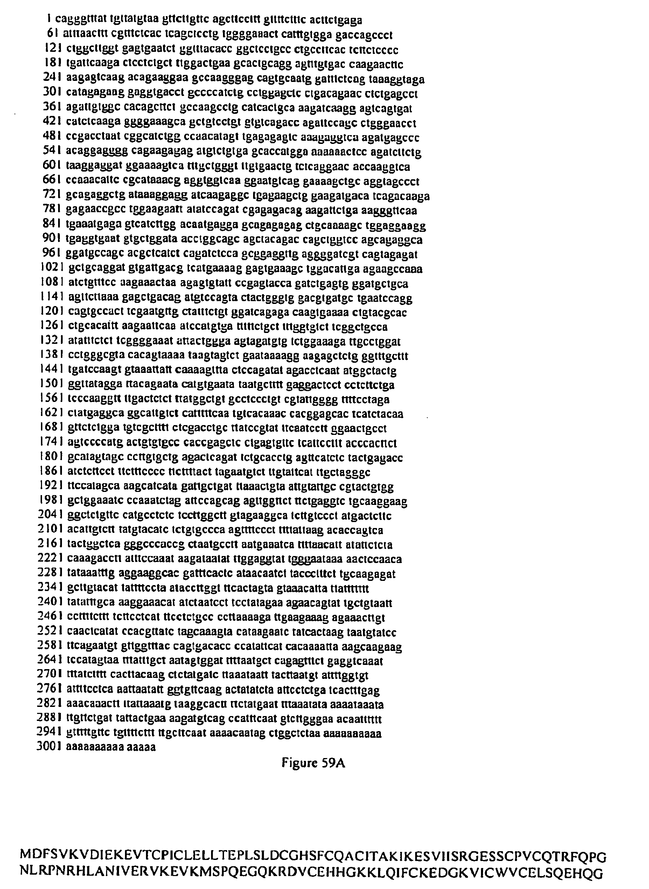

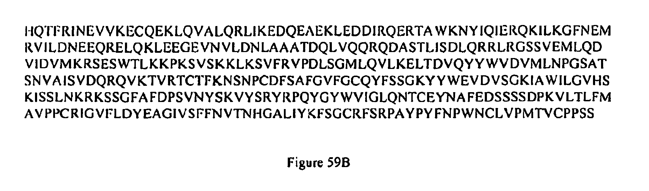

Figure 59A and 59B depict the nucleic acid sequence (SEQ ID NO:103) encoding human TRIM22 polypeptide and the amino acid sequence of human TRIM22 polypeptide (SEQ ID NO:104).

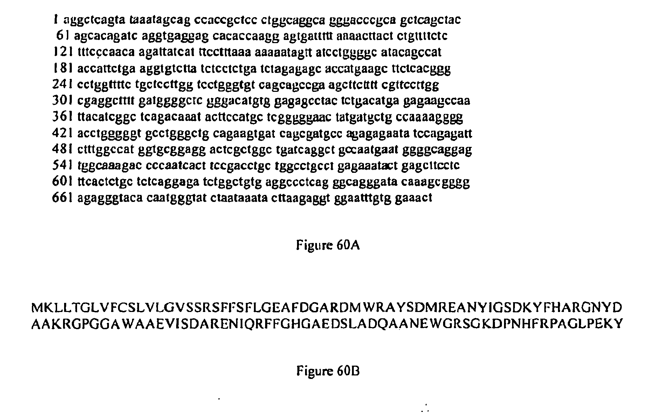

Figure 60A and 60B depict the nucleic acid sequence (SEQ ID NO:105) encoding human SAA2 polypeptide and the amino acid sequence of human SAA2 polypeptide (SEQ ID NO:106).

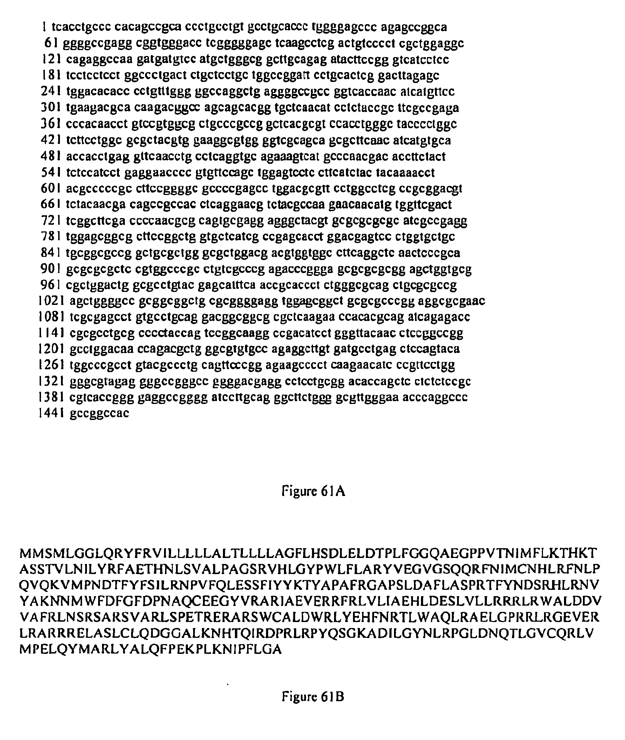

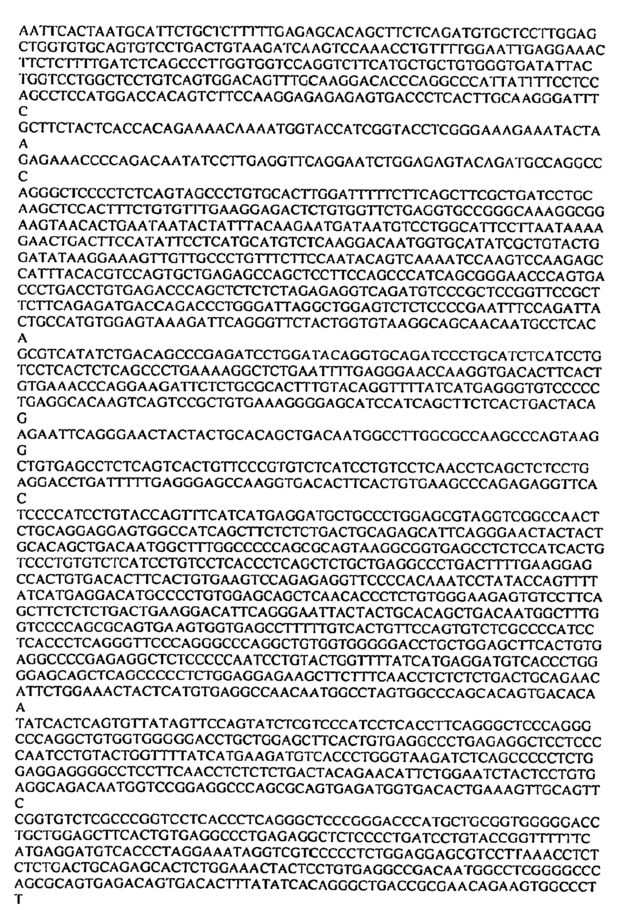

Figure 61A and 61B depict the nucleic acid sequence (SEQ ID NO:107) encoding human NEU4 polypeptide and the amino acid sequence of human NEU4 polypeptide (SEQ ID NO:108).

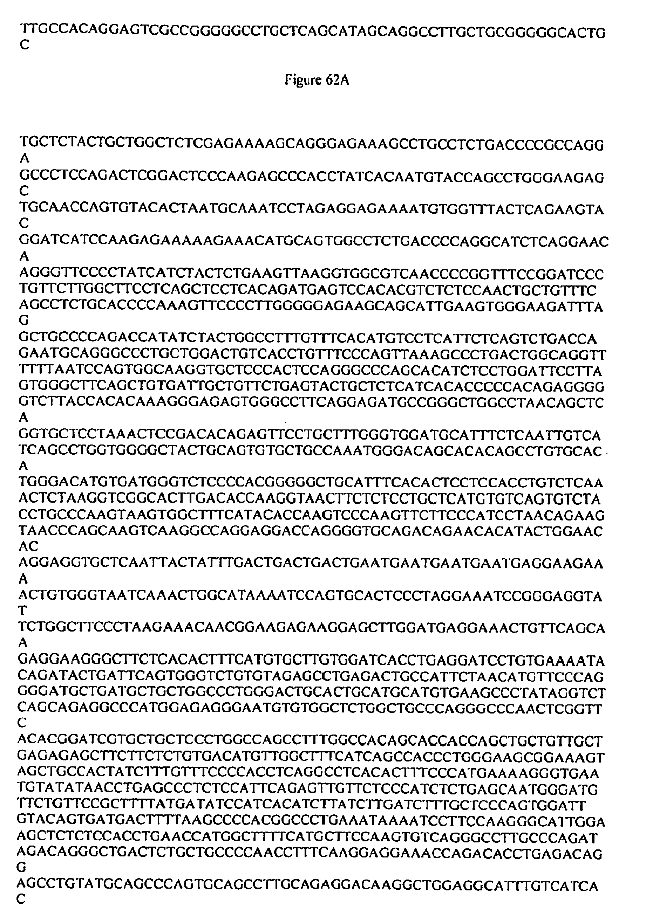

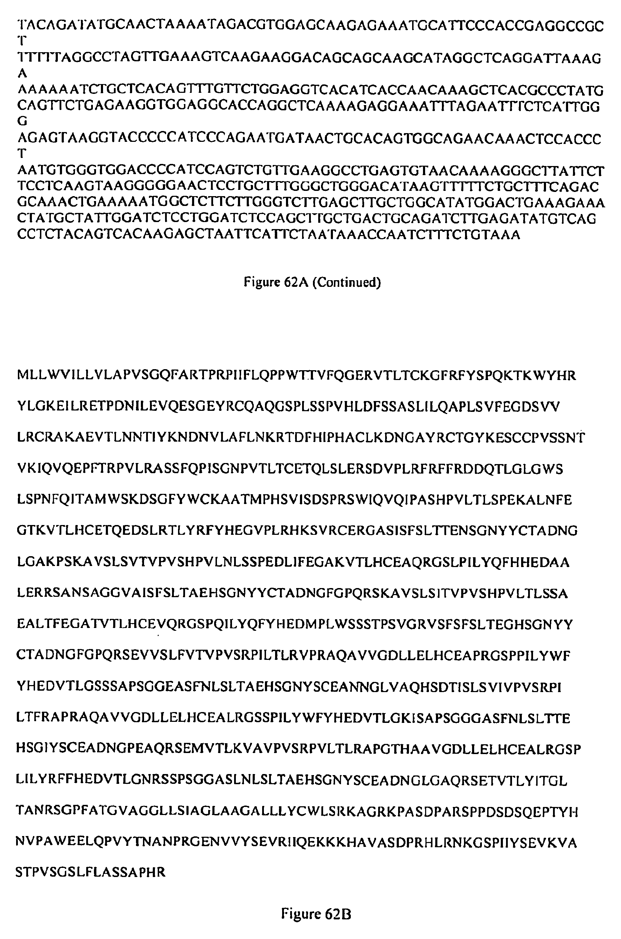

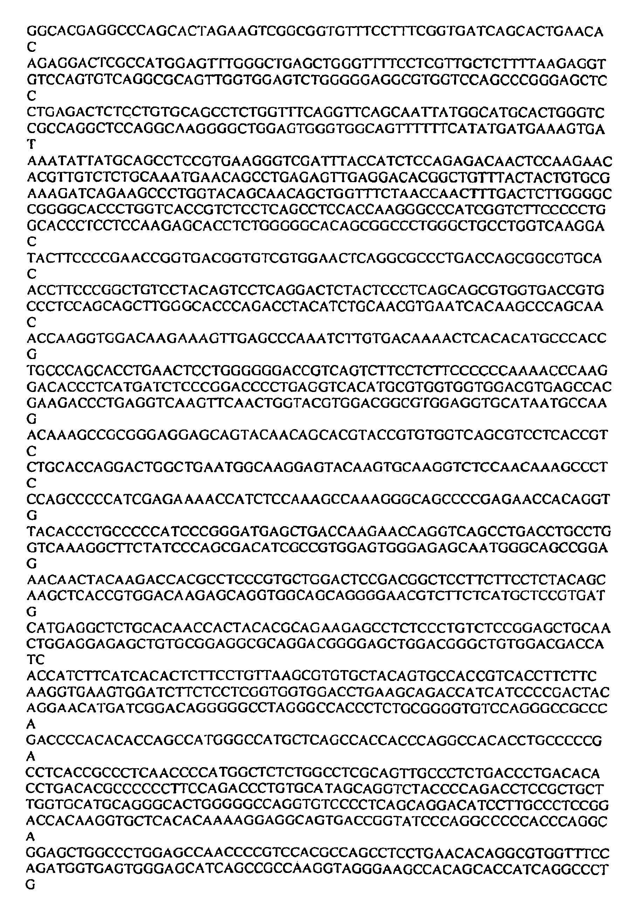

Figure 62A and 62B depict the nucleic acid sequence (SEQ ID NO:109) encoding human IRTA2/FCRH5 polypeptide and the amino acid sequence of human IRTA2/FCRH5 polypeptide (SEQ ID NO:110).

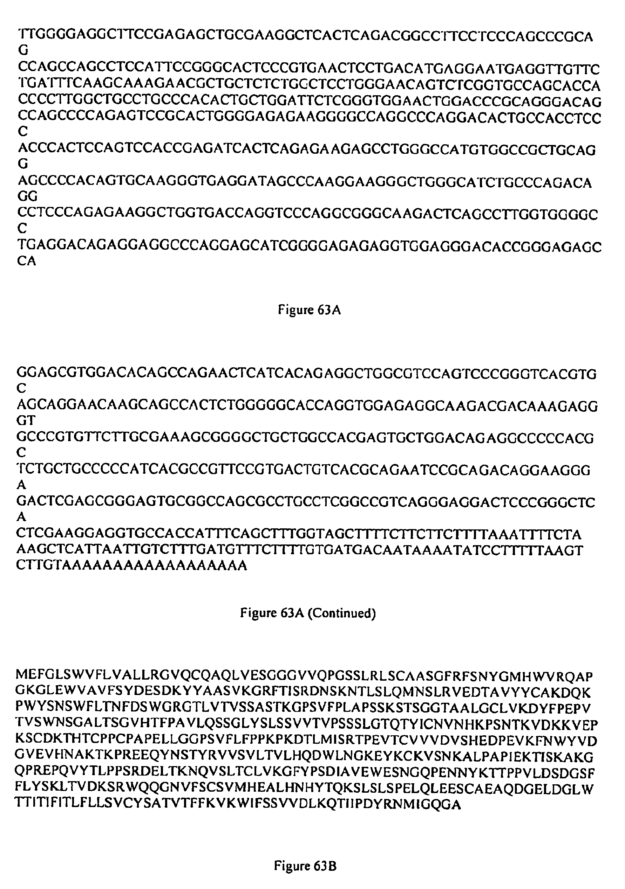

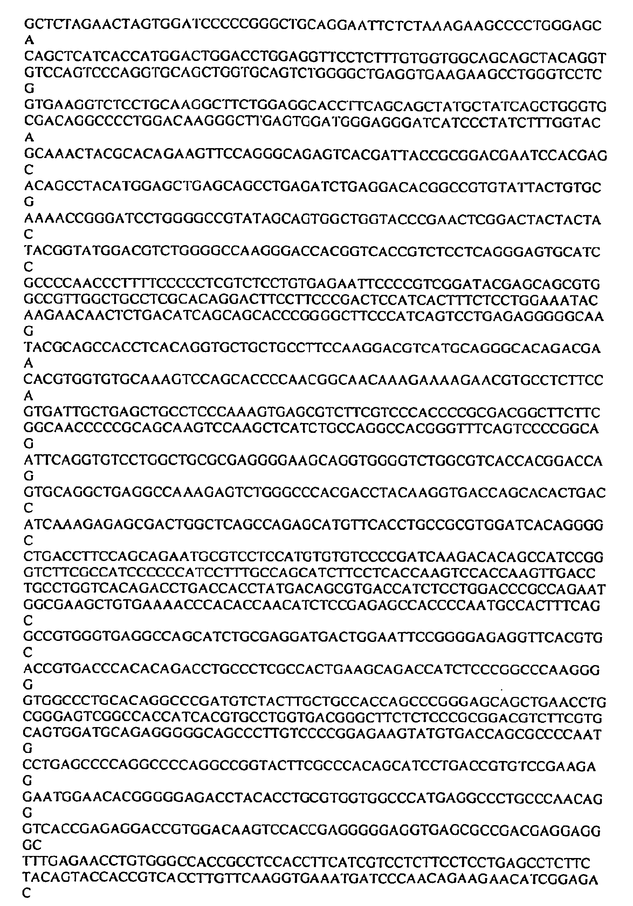

Figure 63A and 63B depict the nucleic acid sequence (SEQ ID NO:111) encoding human IGLJCOR18 polypeptide and the amino acid sequence of human IGLJCOR18 polypeptide (SEQ ID NO:112).

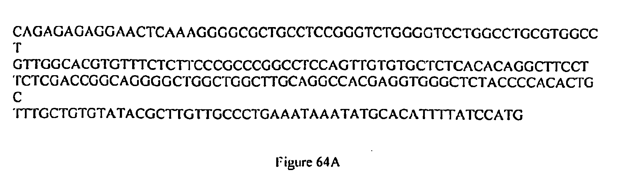

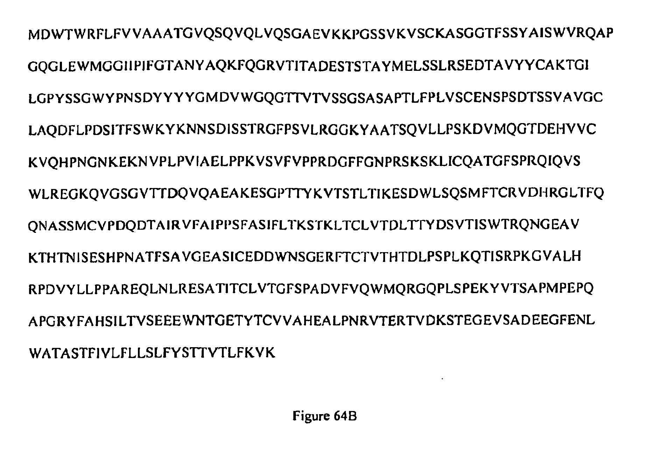

Figure 64A and 64B depict the nucleic acid sequence (SEQ ID NO:113) encoding human IGHV4-4 polypeptide and the amino acid sequence of human IGHV4-4 polypeptide (SEQ ID NO:114).

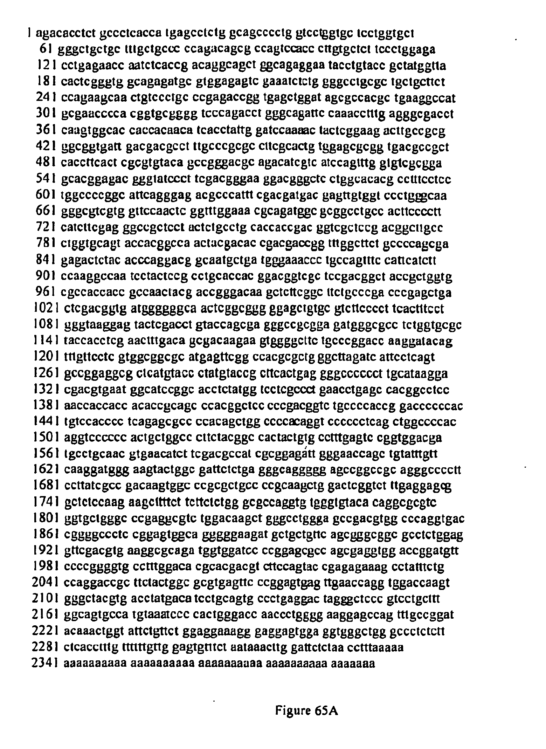

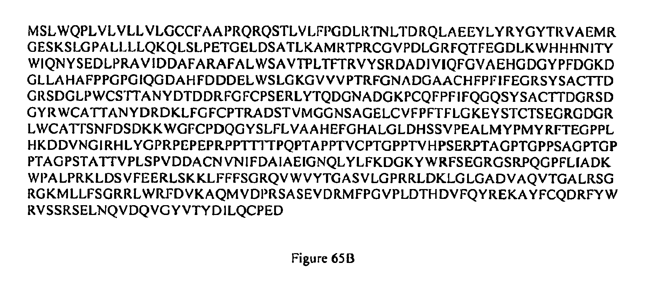

Figure 65A and 65B depict the nucleic acid sequence (SEQ ID NO:115) encoding human MMP9 polypeptide and the amino acid sequence of human MMP9 polypeptide (SEQ ID NO:116).

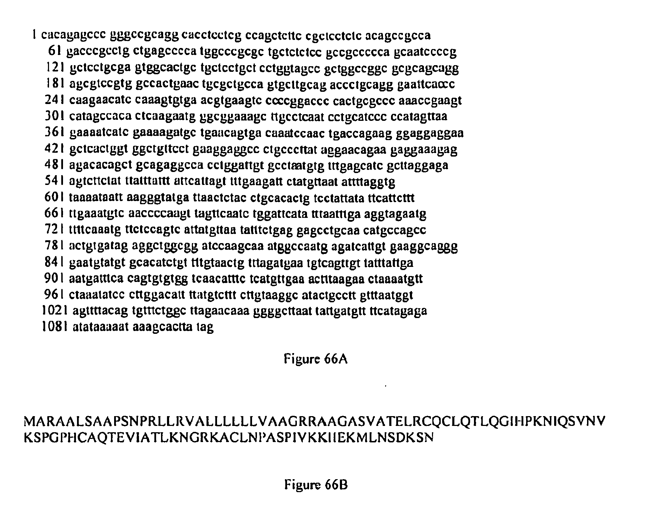

Figure 66A and 66B depict the nucleic acid sequence (SEQ ID NO:117) encoding human GRO polypeptide and the amino acid sequence of human GRO polypeptide (SEQ ID NO:118).

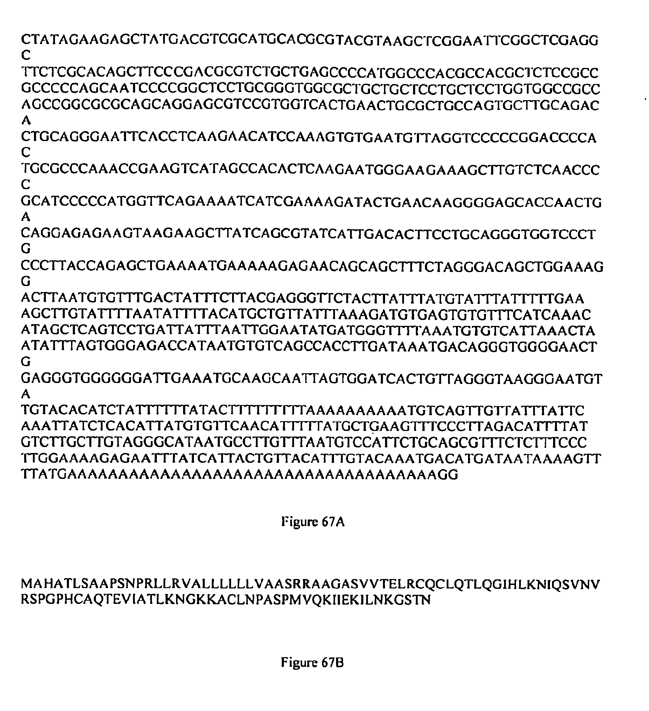

Figure 67A and 67B depict the nucleic acid sequence (SEQ ID NO:119) encoding human MIP2BGRO-g polypeptide and the amino acid sequence of human MIP2BGRO-g polypeptide (SEQ ID NO:120).

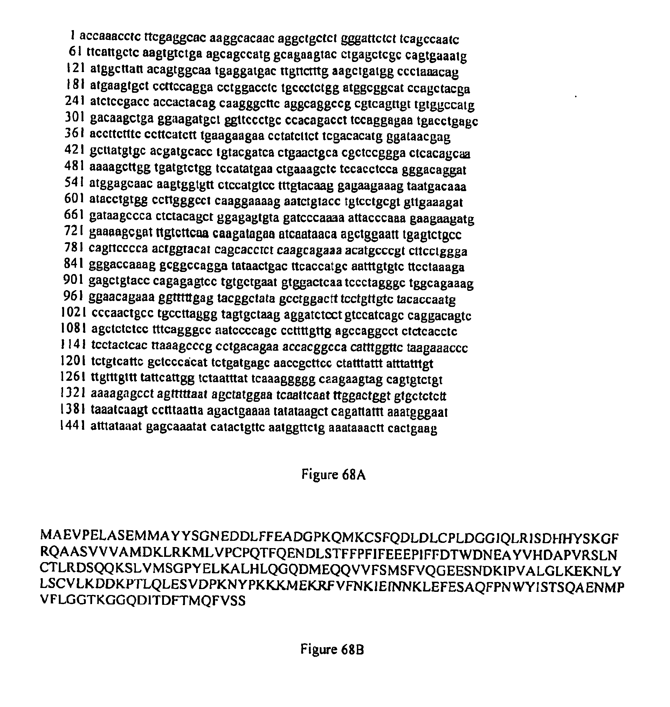

Figure 68A and 68B depict the nucleic acid sequence (SEQ ID NO:121) encoding human IL1B polypeptide and the amino acid sequence of human 1L1B polypeptide (SEQ ID NO:122).

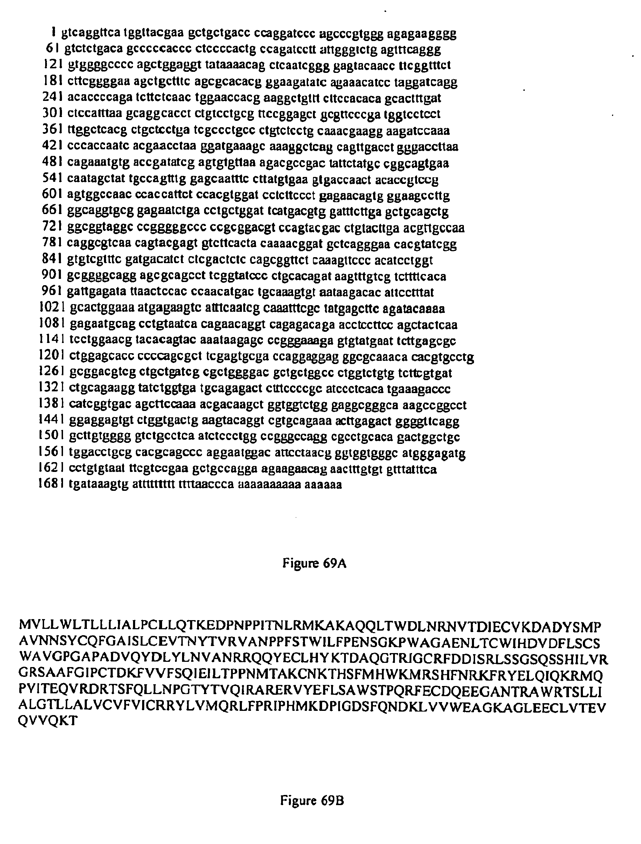

Figure 69A and 69B depict the nucleic acid sequence (SEQ ID NO:123) encoding human IL3RA polypeptide and the amino acid sequence of human IL3RA polypeptide (SEQ ID NO:124).

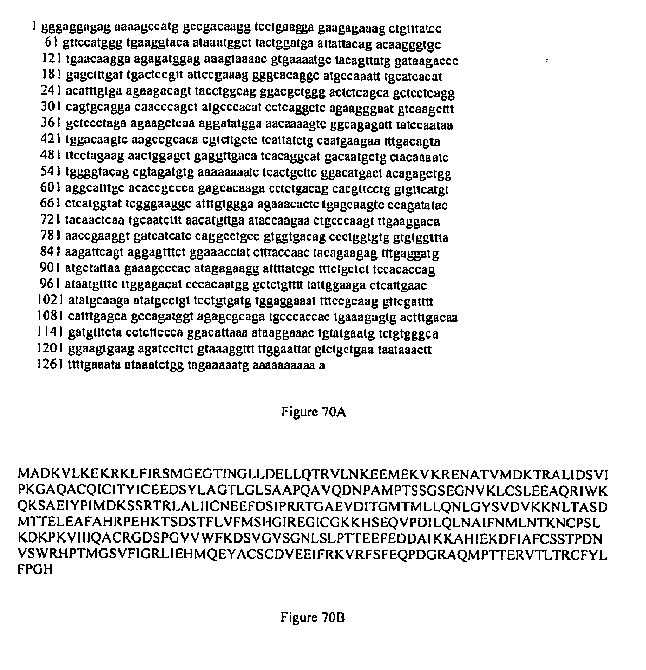

Figure 70A and 70B depict the nucleic acid sequence (SEQ ID NO:125) encoding human CASP1 polypeptide and the amino acid sequence of human CASP1 polypeptide (SEQ ID NO:126).

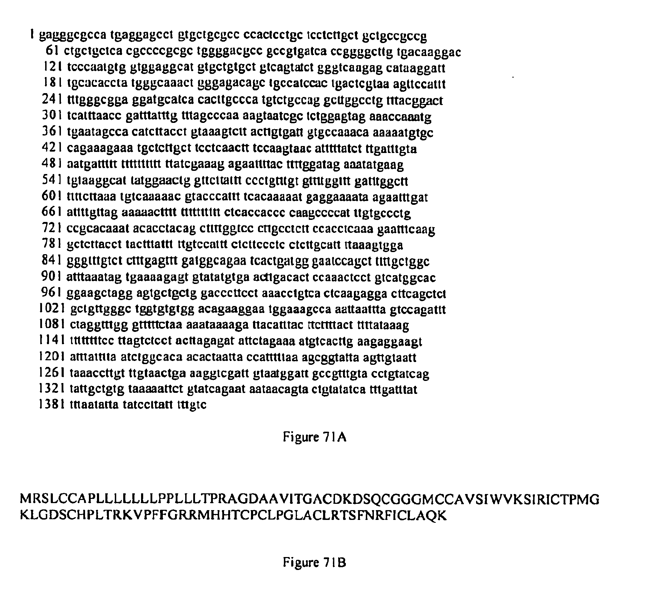

Figure 71A and 71B depict the nucleic acid sequence (SEQ ID NO:127) encoding human BV8 polypeptide and the amino acid sequence of human BV8 polypeptide (SEQ ID NO:128).

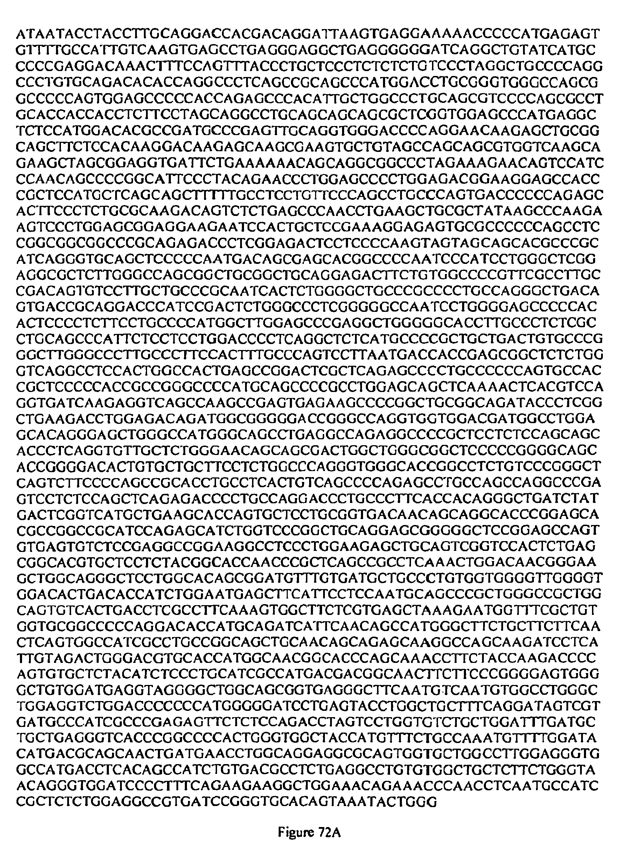

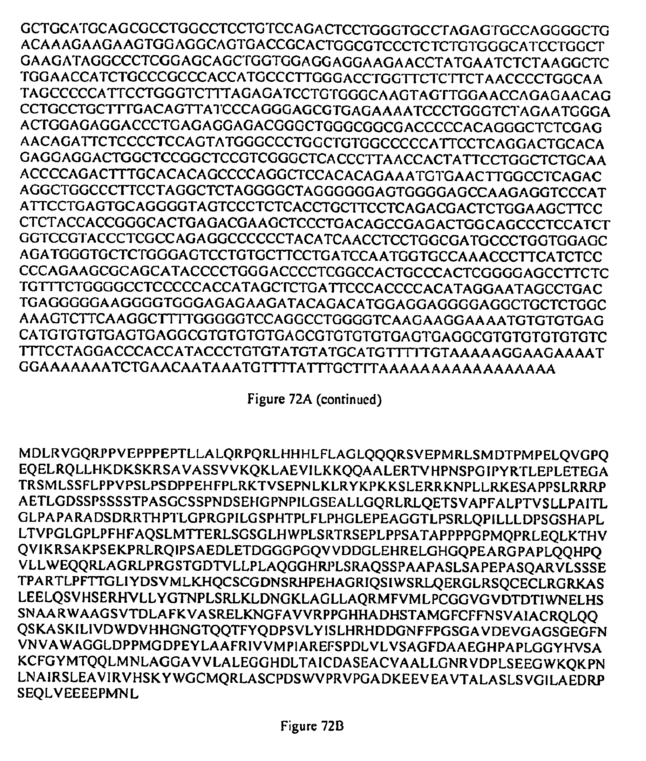

Figure 72A and 72B depict the nucleic acid sequence (SEQ ID NO:129) encoding human HDAC7A polypeptide and the amino acid sequence of human HDAC7A polypeptide (SEQ ID NO:130).

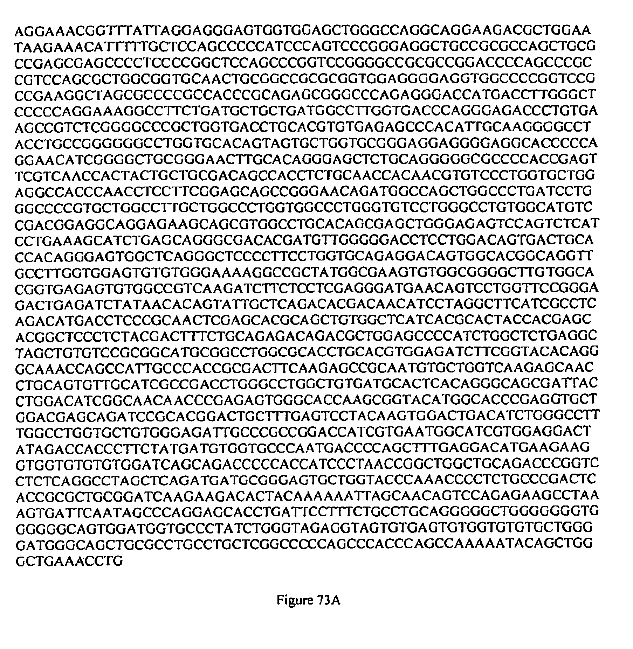

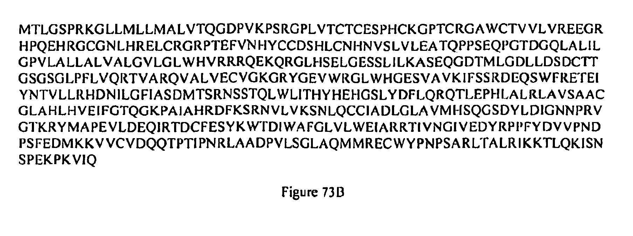

Figure 73A and 73B depict the nucleic acid sequence (SEQ ID NO:131) encoding human ACVRL1 polypeptide and the amino acid sequence of human ACVRL1 polypeptide (SEQ ID NO:132).

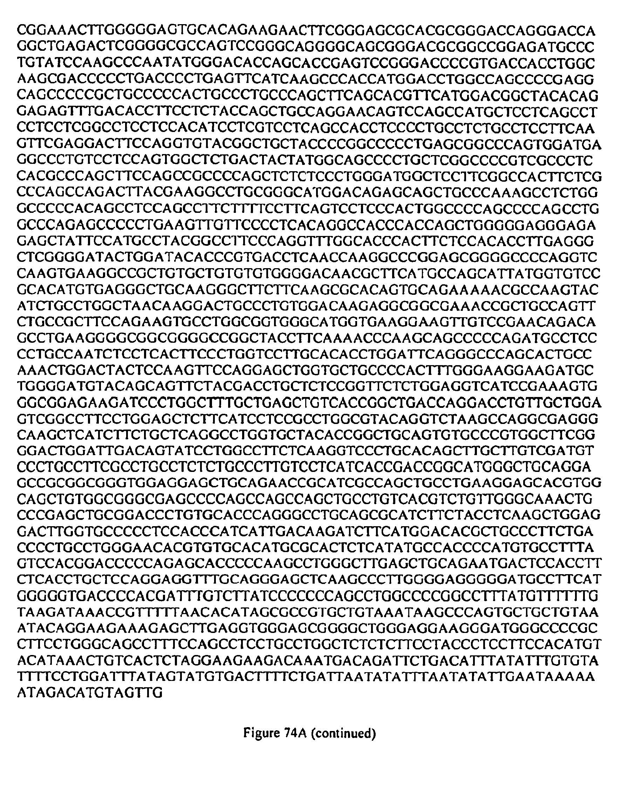

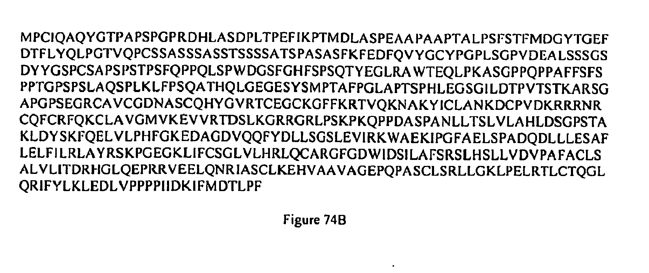

Figure 74A and 74B depict the nucleic acid sequence (SEQ ID NO:133) encoding human NR4A1 polypeptide and the amino acid sequence of human NR4A1 polypeptide (SEQ ID NO:134).

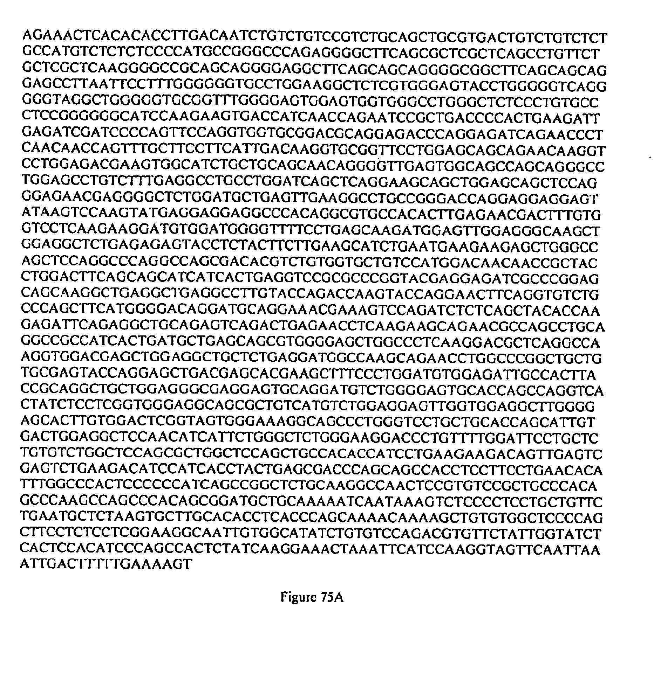

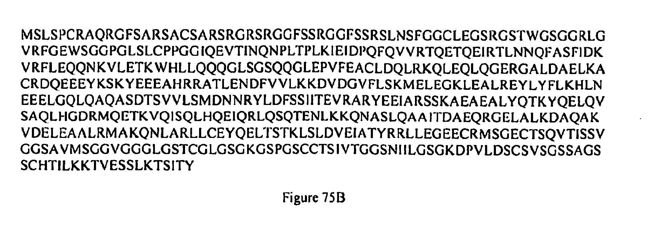

Figure 75A and 75B depict the nucleic acid sequence (SEQ ID NO:135) encoding human K5B polypeptide and the amino acid sequence of human K5B polypeptide (SEQ ID NO:136).

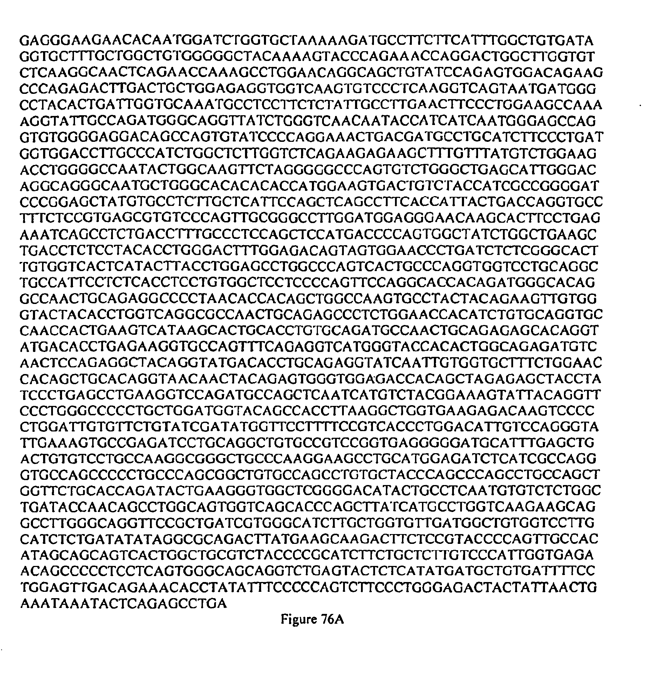

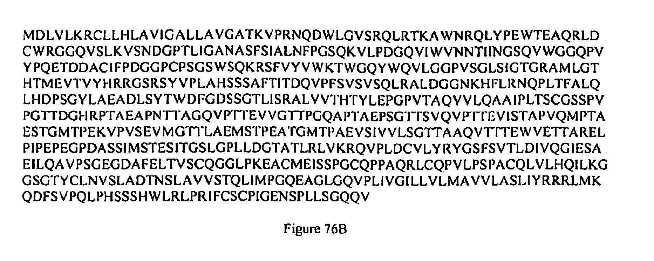

Figure 76A and 76B depict the nucleic acid sequence (SEQ ID NO:137) encoding human SILV polypeptide and the amino acid sequence of human SILV polypeptide (SEQ ID NO:138).

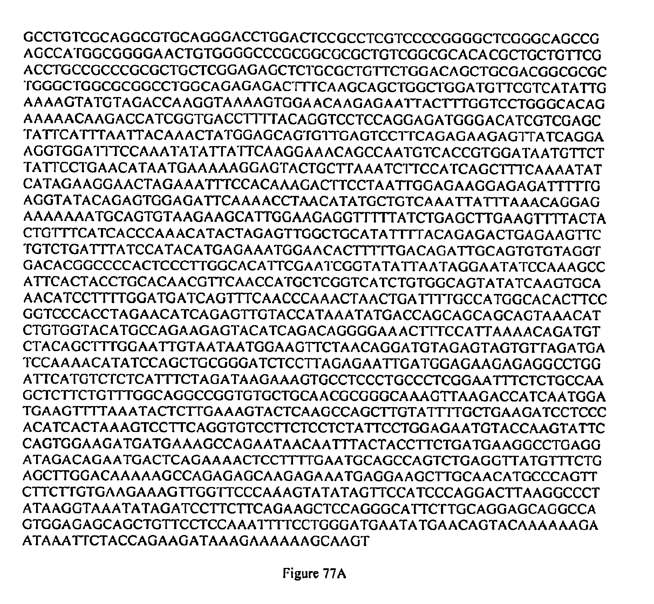

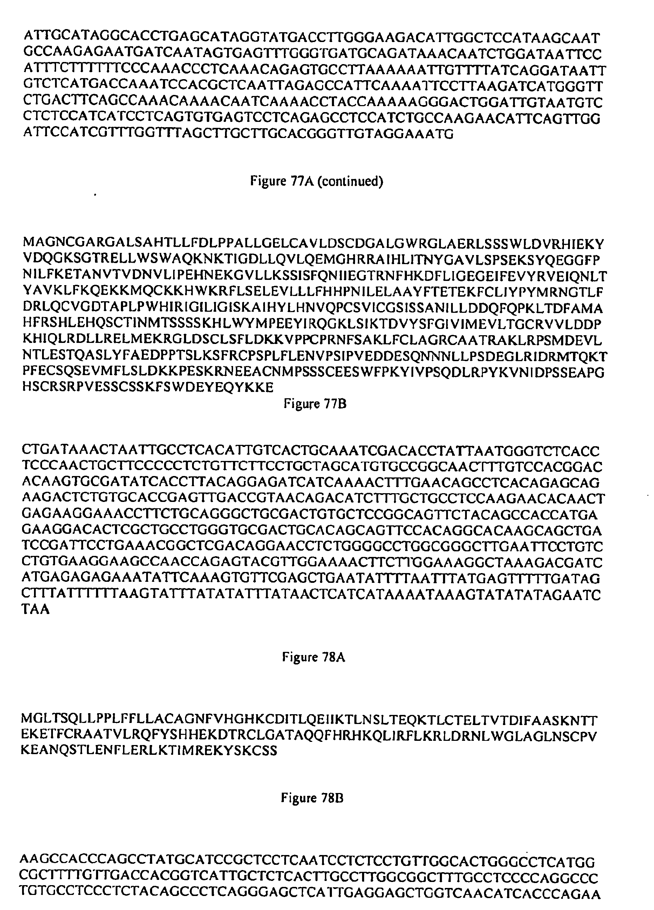

Figure 77A and 77B depict the nucleic acid sequence (SEQ ID NO:139) encoding human IRAK3 polypeptide and the amino acid sequence of human IRAK3 polypeptide (SEQ ID NO:140).
Figure 78A and 78B depict the nucleic acid sequence (SEQ ID NO:141) encoding human IL-4 polypeptide and the amino acid sequence of human IL-4 polypeptide (SEQ ID NO:142).

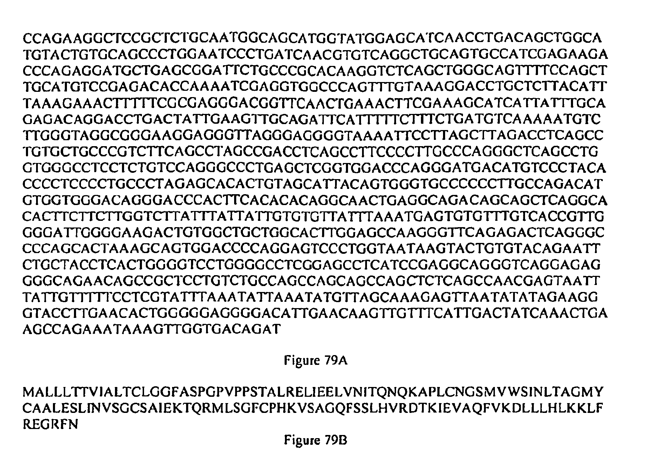

Figure 79A and 79B depict the nucleic acid sequence (SEQ ID NO:143) encoding human IL-13 polypeptide and the amino acid sequence of human IL-13 polypeptide (SEQ ID NO:144).

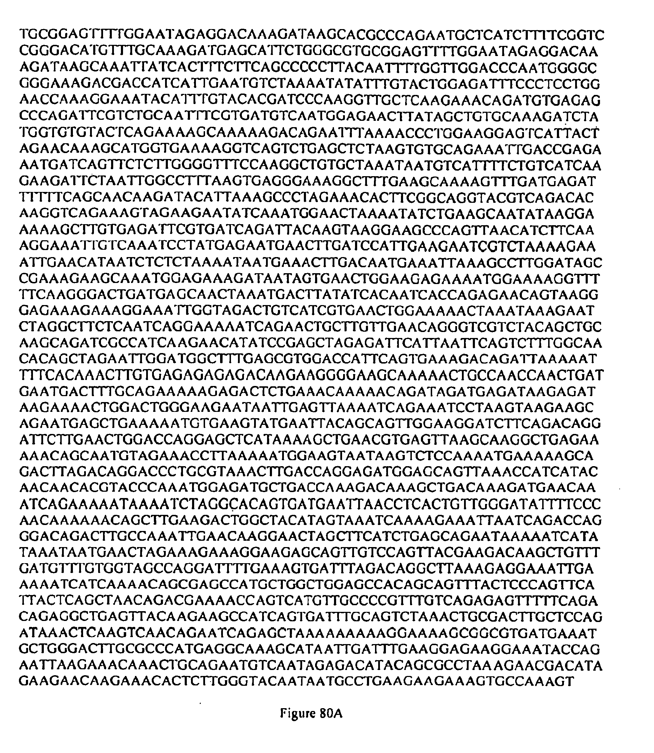

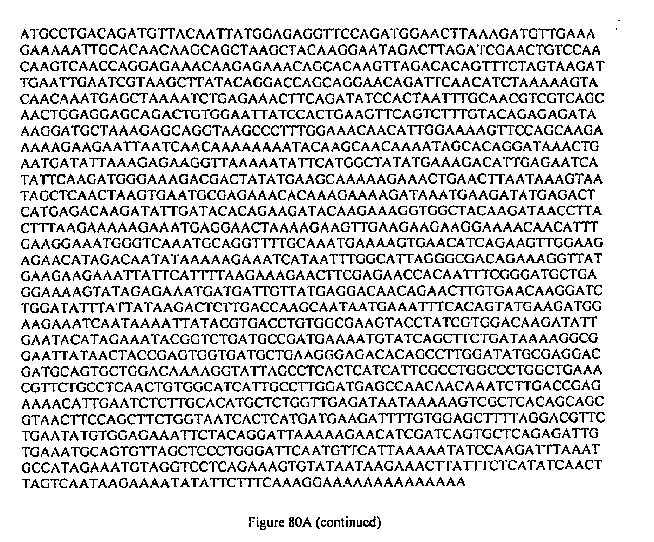

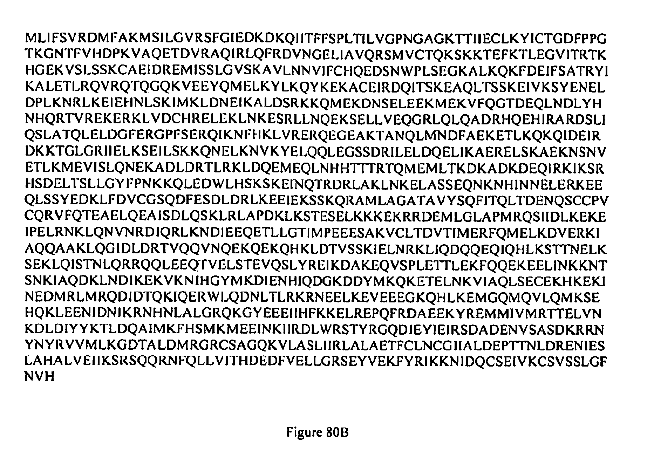

Figure 80A and 80B depict the nucleic acid sequence (SEQ ID NO:145) encoding human RAD50 polypeptide and the amino acid sequence of human RAD50 polypeptide (SEQ ID NO:146).

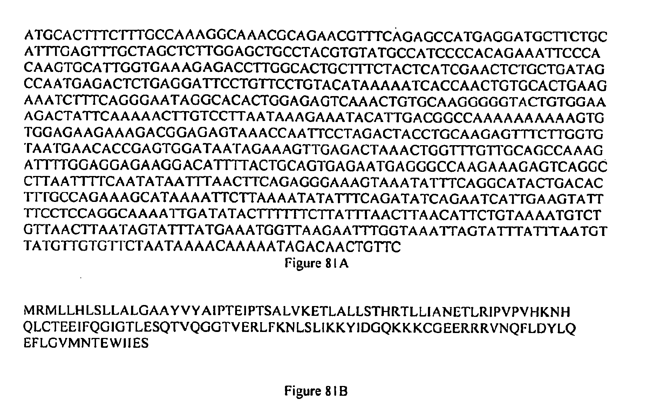

Figure 81A and 81B depict the nucleic acid sequence (SEQ ID NO:147) encoding human IL-5 polypeptide and the amino acid sequence of human IL-5 polypeptide (SEQ ID NO:148).

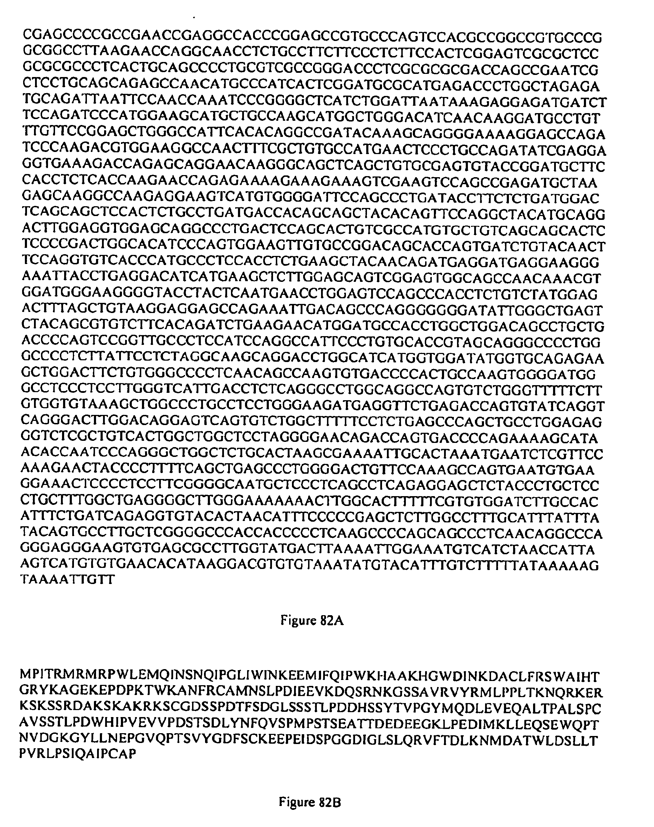

Figure 82A and 82B depict the nucleic acid sequence (SEQ ID NO:149) encoding human IRF1 polypeptide and the amino acid sequence of human IRF1 polypeptide (SEQ ID NO:150).

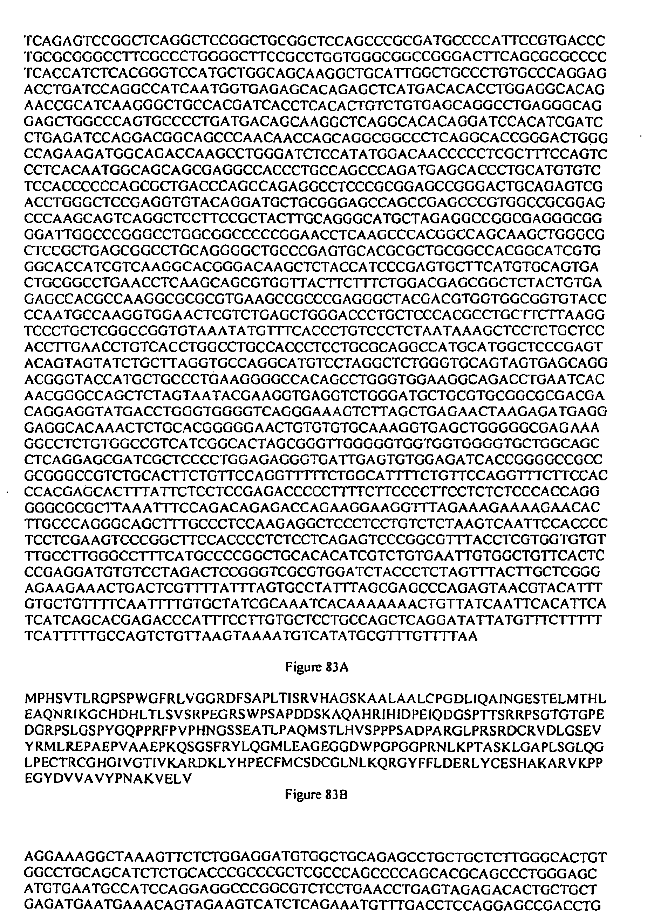

Figure 83A and 83B depict the nucleic acid sequence (SEQ ID NO:151) encoding human PDLIM4 polypeptide and the am ino acid sequence of human PDLIM4 polypeptide (SEQ ID NO:152).

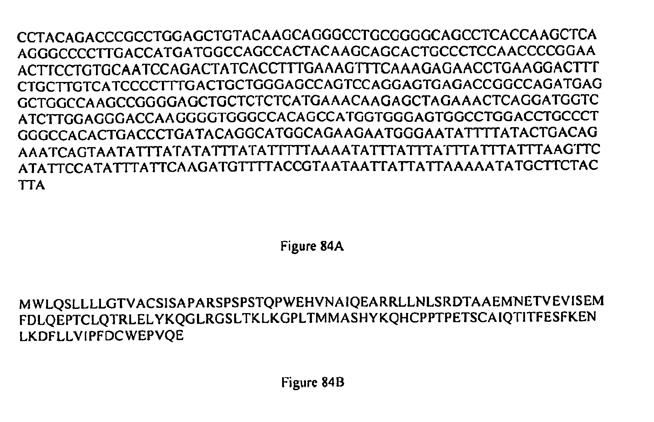

Figure 84A and 84B depict the nucleic acid sequence (SEQ ID NO:153) encoding human CSF2 polypeptide and the amino acid sequence of human CSF2 polypeptide (SEQ ID NO:154).

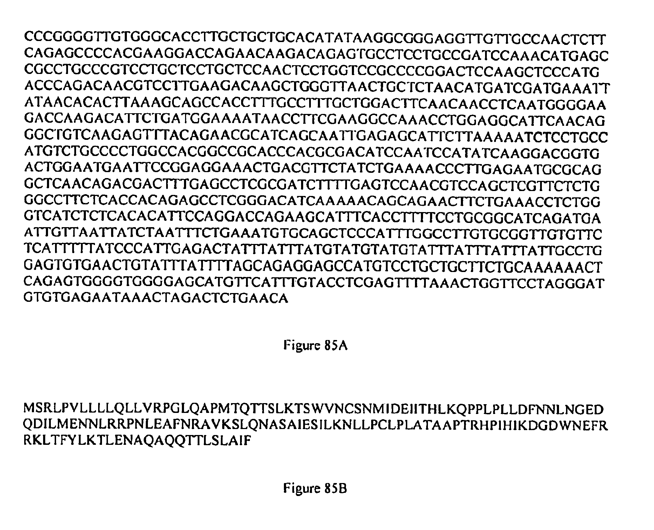

Figure 85A and 85B depict the nucleic acid sequence (SEQ ID NO:155) encoding human IL-3 polypeptide and the amino acid sequence of human IL-3 polypeptide (SEQ ID NO:156).

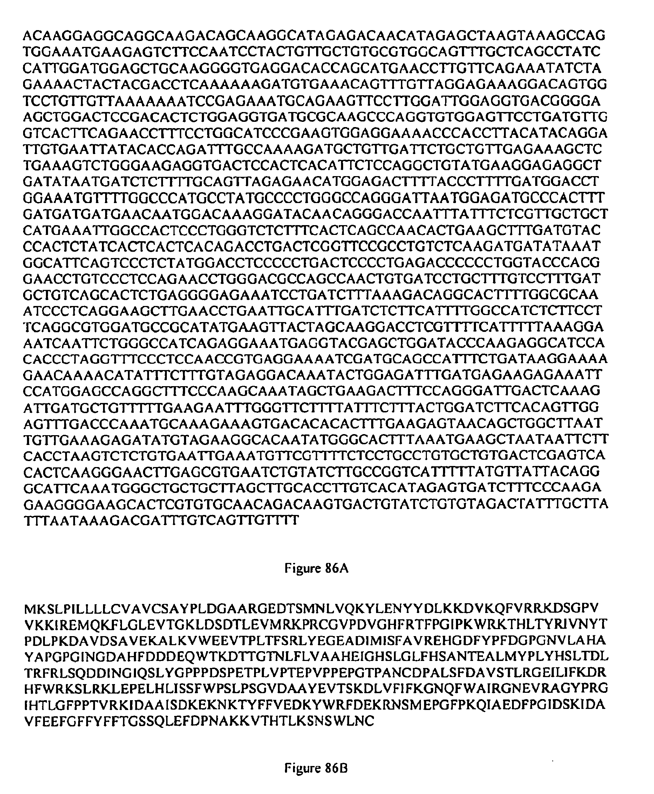

Figure 86A and 86B depict the nucleic acid sequence (SEQ ID NO:157) encoding human MMP3 polypeptide and the amino acid sequence of human MMP3 polypeptide (SEQ ID NO:158).

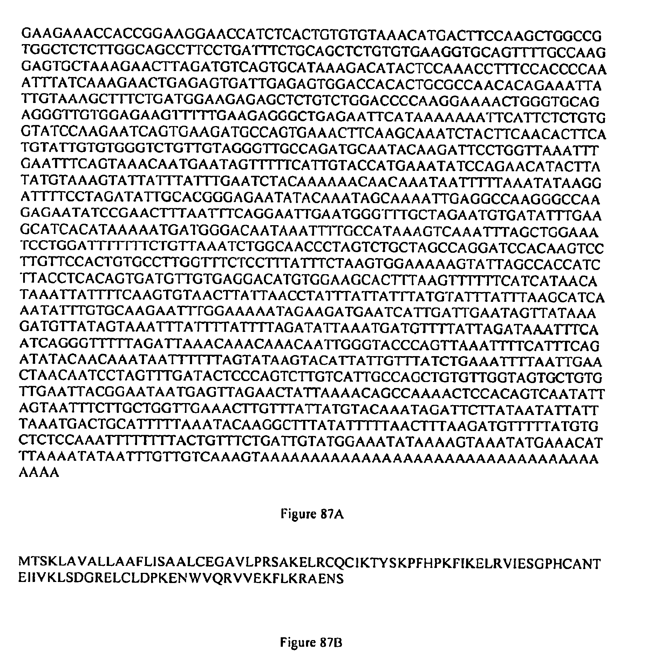

Figure 87A and 87B depict the nucleic acid sequence (SEQ ID NO:159) encoding human IL-8 polypeptide and the amino acid sequence of human IL-8 polypeptide (SEQ ID NO:160).

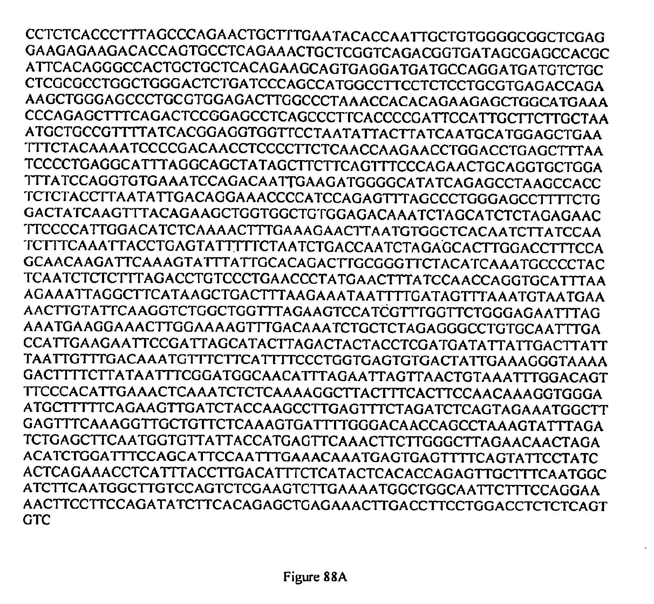

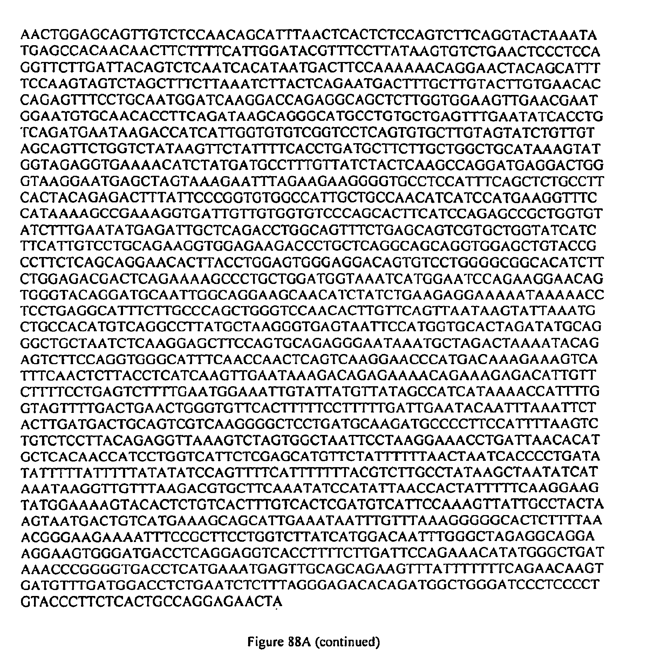

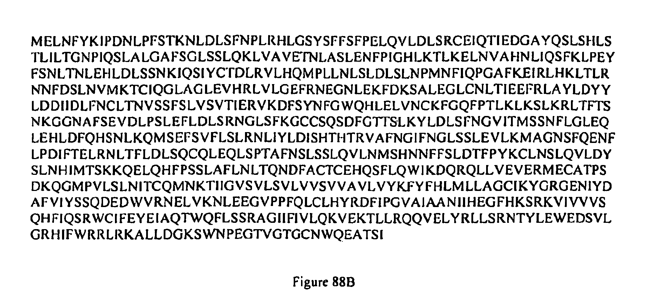

Figure 88A and 88B depict the nucleic acid sequence (SEQ ID NO:161) encoding human TLR4 polypeptide and the amino acid sequence of human TLR4 polypeptide (SEQ ID NO:162).

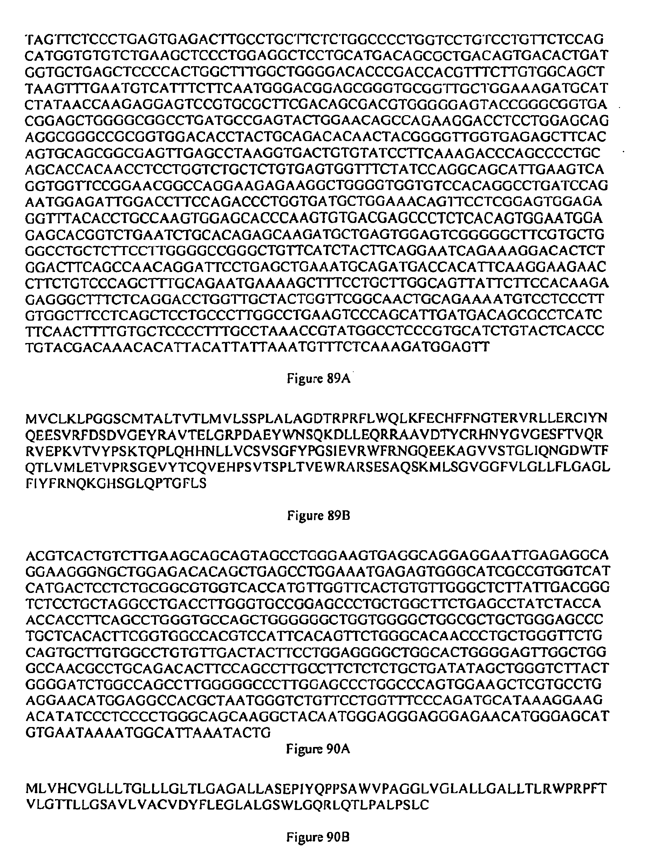

Figure 89A and 89B depict the nucleic acid sequence (SEQ ID NO:163) encoding human HLA-DRB1 polypeptide and the amino acid sequence of human HLA-DRB1 polypeptide (SEQ ID NO:164).
Figure 90A and 90B depict the nucleic acid sequence (SEQ ID NO:165) encoding human MMP19 polypeptide and the amino acid sequence of human MMP19 polypeptide (SEQ ID NO:166).

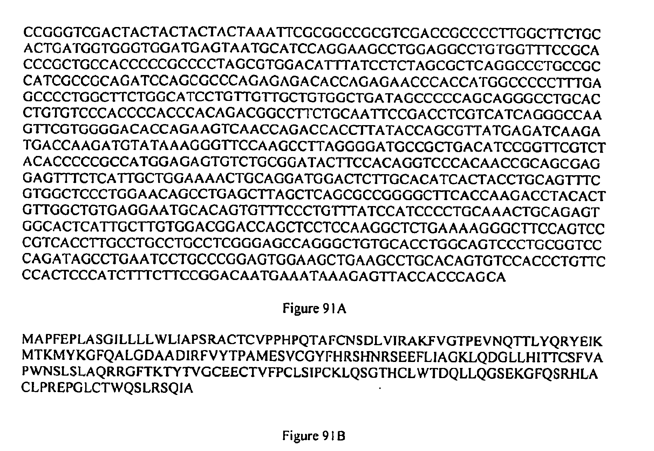

Figure 91A and 91B depict the nucleic acid sequence (SEQ ID NO:167) encoding human TIMP1 polypeptide and the amino acid sequence of human TIMP1 polypeptide (SEQ ID NO:168).

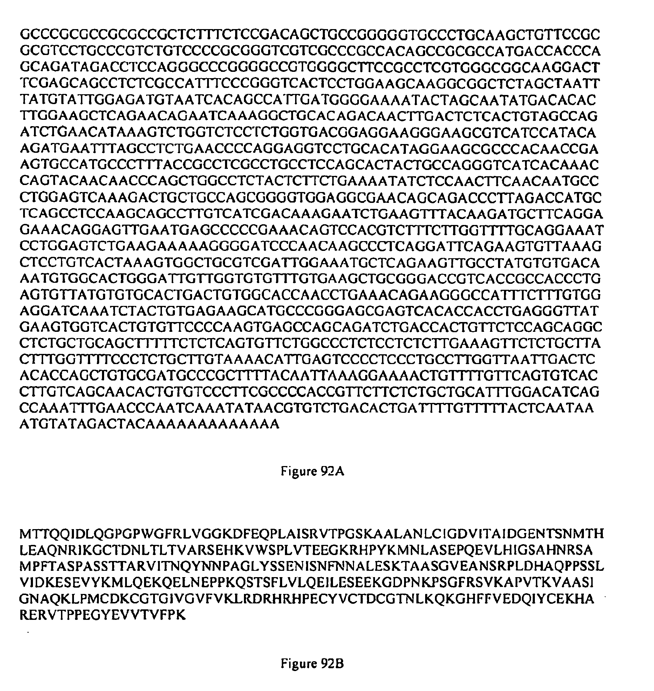

Figure 92A and 92B depict the nucleic acid sequence (SEQ ID NO:169) encoding human Elfin polypeptide and the amino acid sequence of human Elfin polypeptide (SEQ ID NO:170).

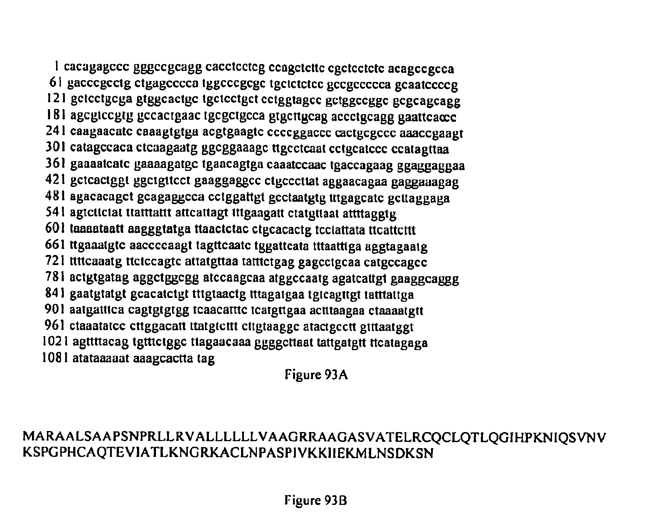

Figure 93A and 93B depict the nucleic acid sequence (SEQ ID NO:171) encoding human CXCL1 polypeptide and the amino acid sequence of human CXCL1 polypeptide (SEQ ID NO:172).

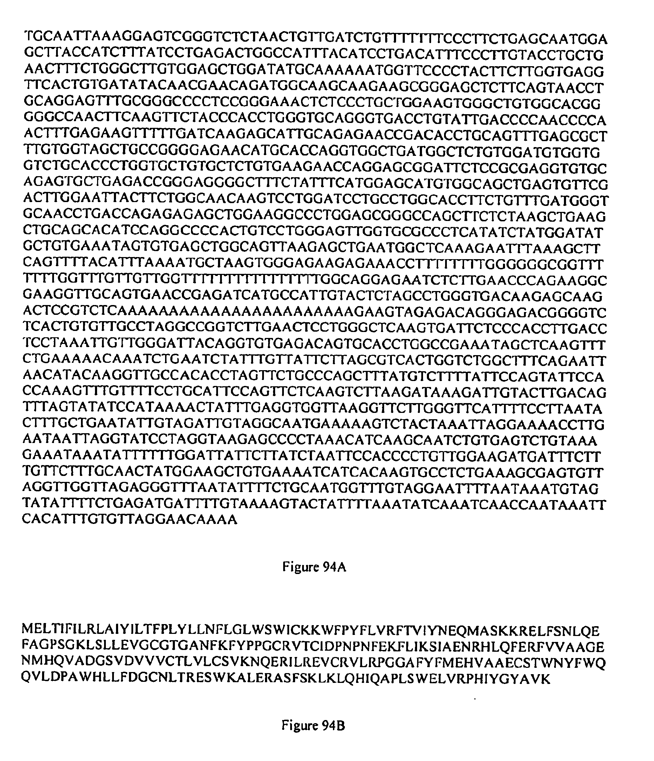

Figure 94A and 94B depict the nucleic acid sequence (SEQ ID NO:173) encoding human DFKZP586A0522 polypeptide and the amino acid sequence of human DFKZP586A0522 polypeptide (SEQ ID NO:174).

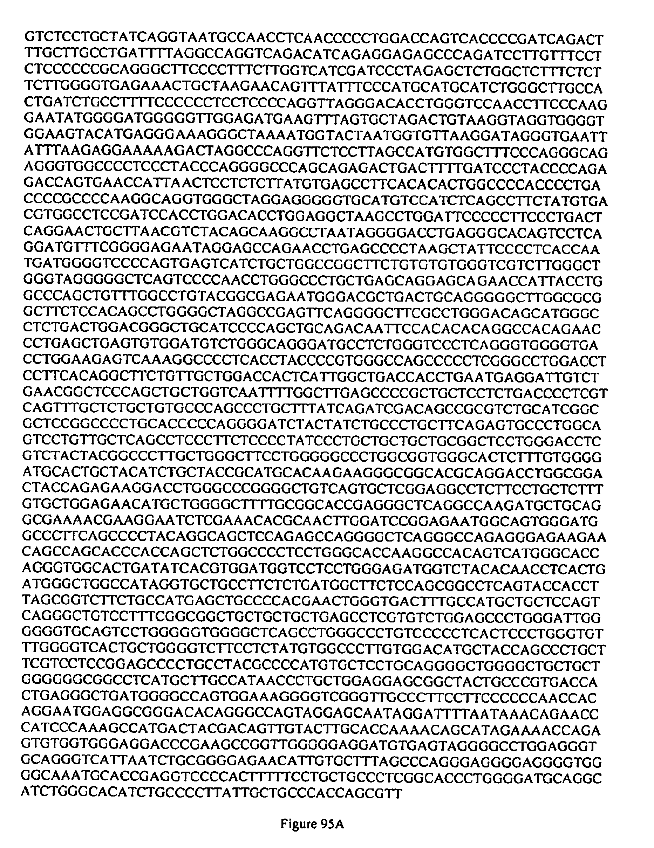

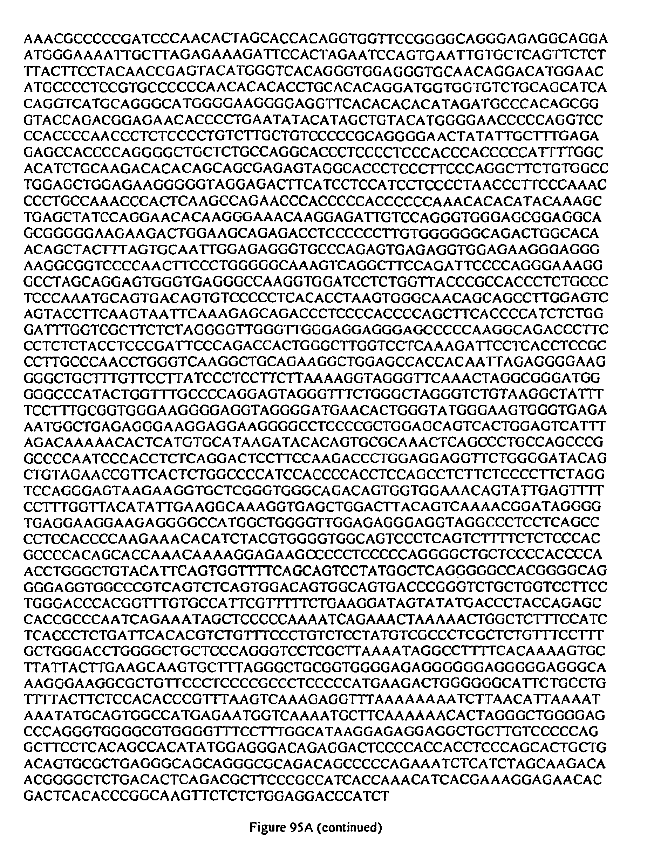

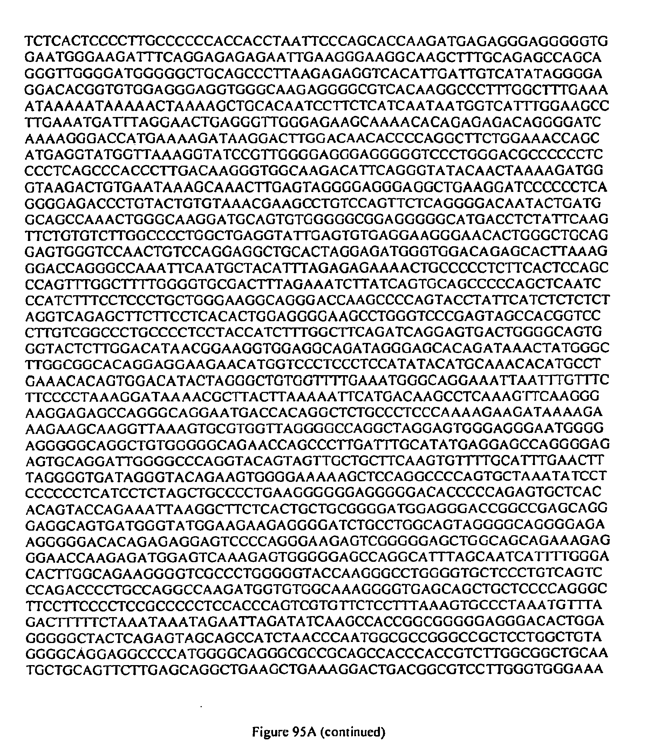

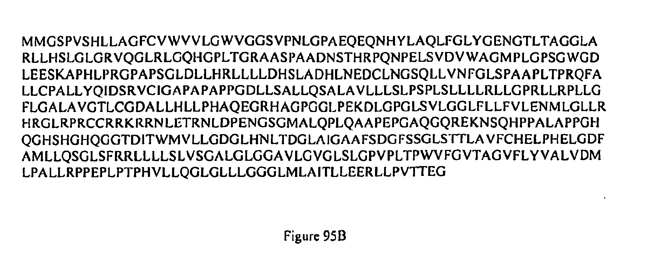

Figure 95A and 95B depict the nucleic acid sequence (SEQ ID NO:175) encoding human SLC39A5 polypeptide and the amino acid sequence of human SLC39A5 polypeptide (SEQ ID NO:176).

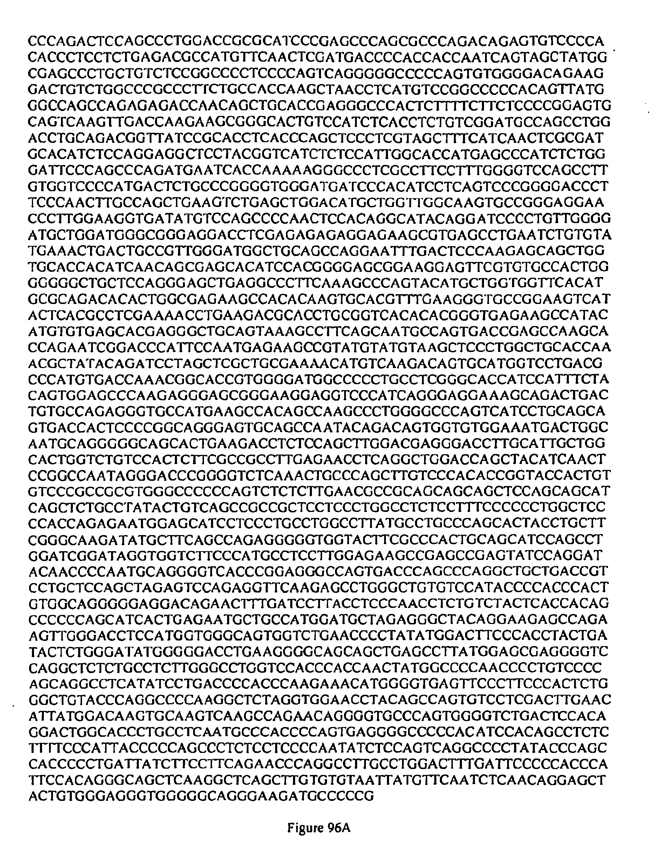

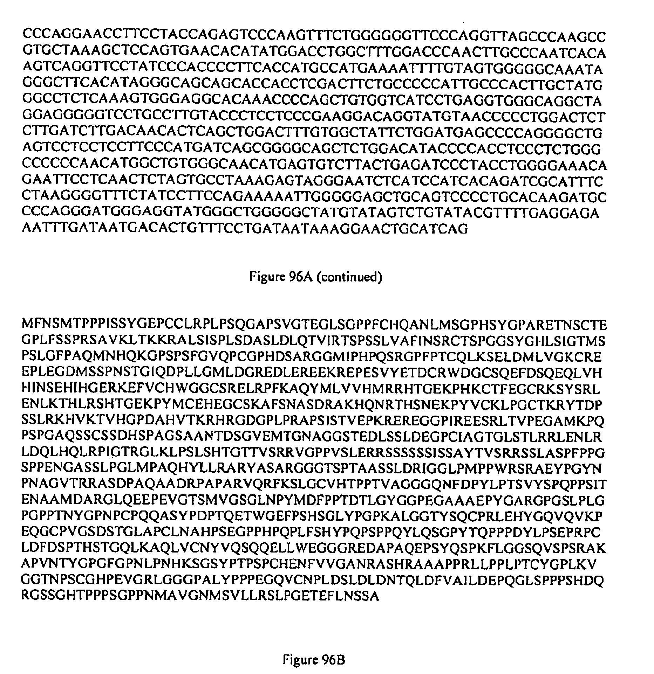

Figure 96A and 96B depict the nucleic acid sequence (SEQ ID NO:177) encoding human GLI-1 polypeptide and the amino acid sequence of human GLI-1 polypeptide (SEQ ID NO:178).

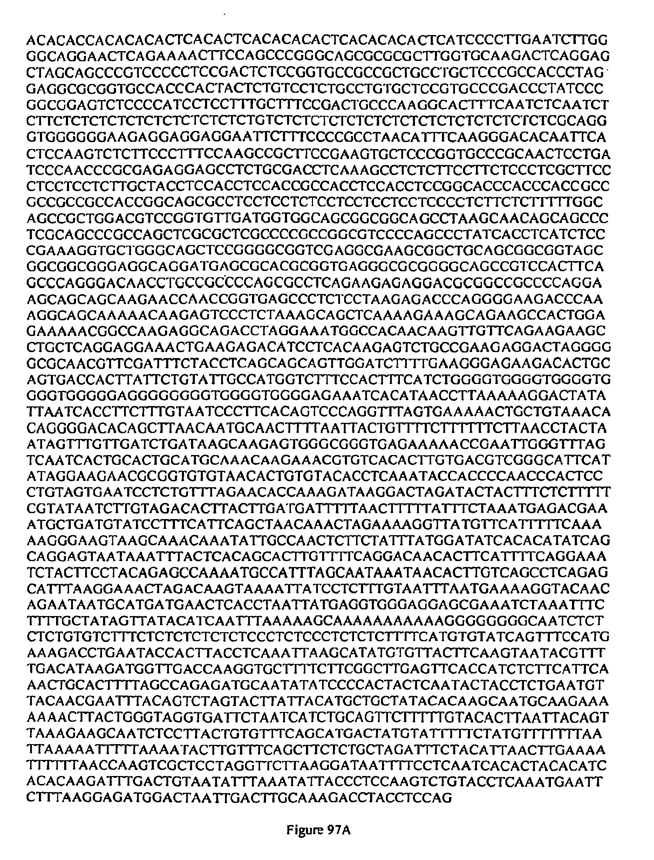

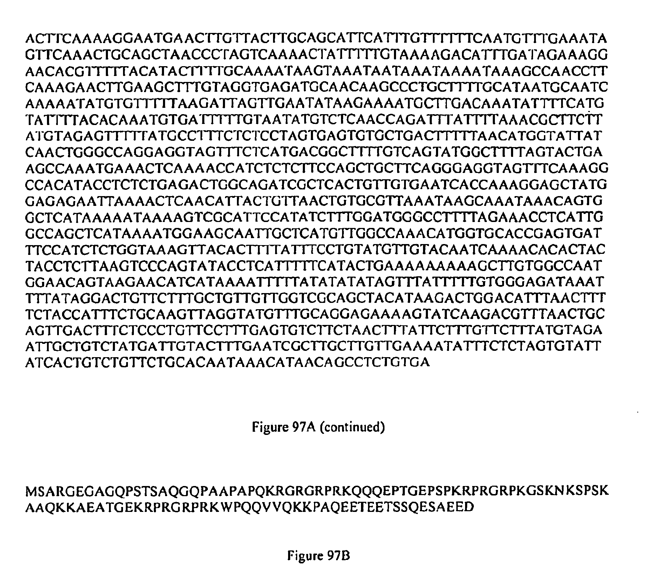

Figure 97A and 97B depict the nucleic acid sequence (SEQ ID NO:179) encoding human HMGA2 polypeptide and the amino acid sequence of human HMGA2 polypeptide (SEQ ID NO:180).

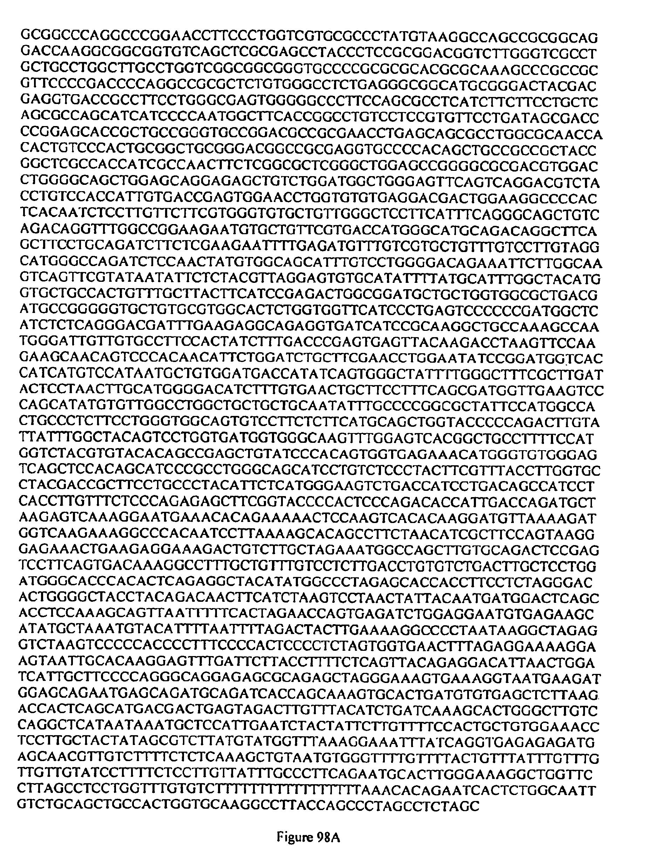

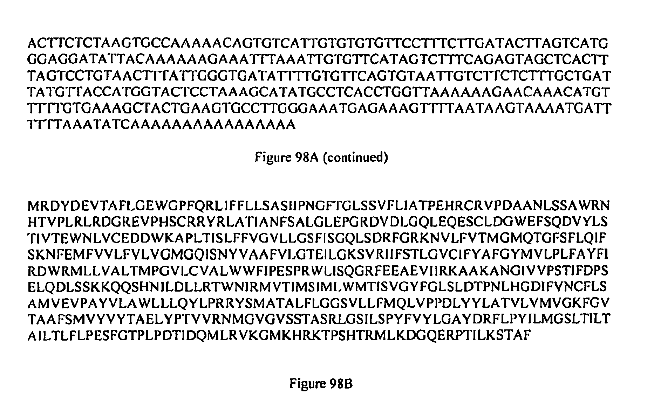

Figure 98A and 98B depict the nucleic acid sequence (SEQ ID NO:181) encoding human SLC22A5 polypeptide and the amino acid sequence of human SLC22A5 polypeptide (SEQ ID NO:182).

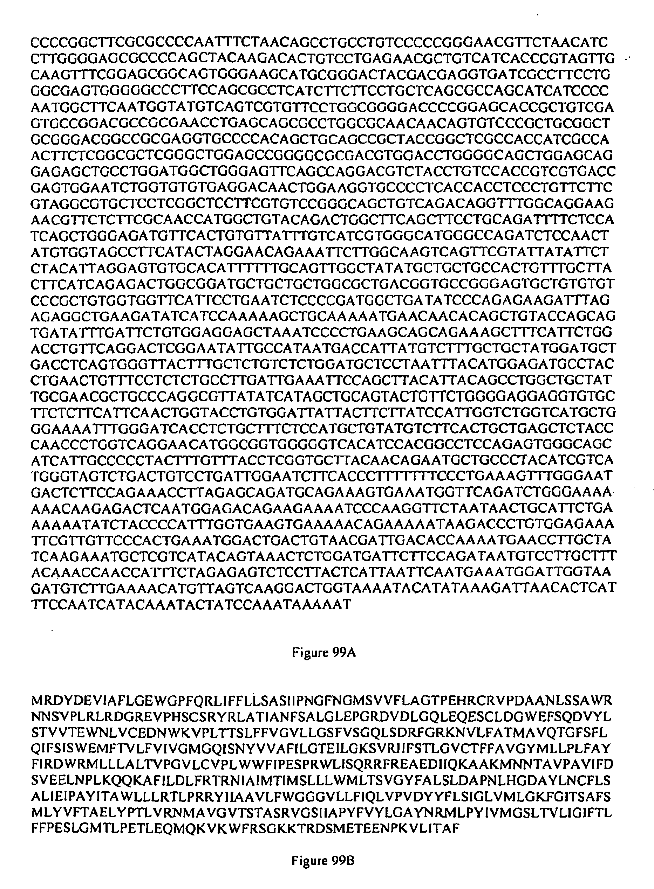

Figure 99A and 99B depict the nucleic acid sequence (SEQ ID NO:183) encoding human SLC22A4 polypeptide and the amino acid sequence of human SLC22A4 polypeptide (SEQ ID NO:184).

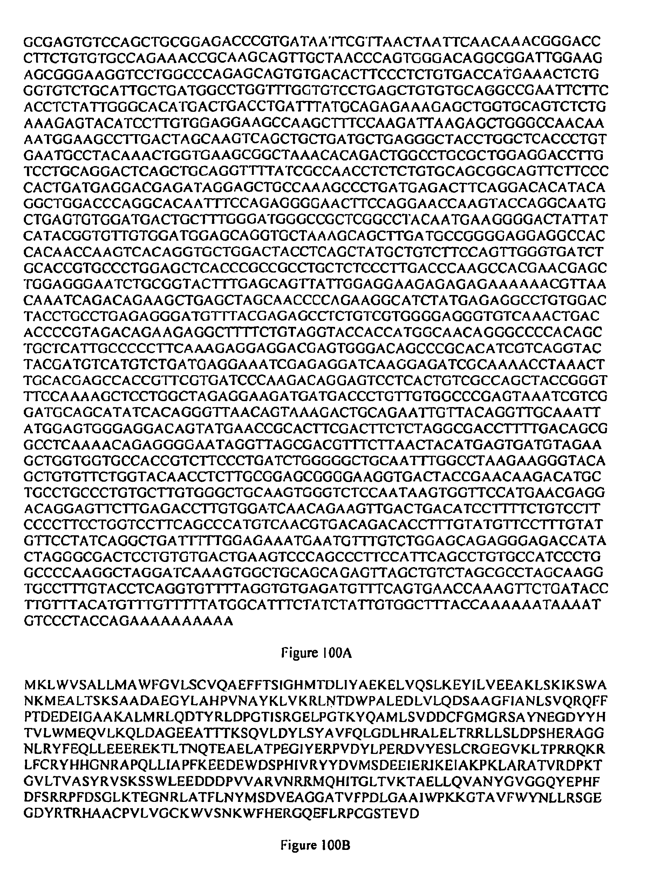

Figure 100A and 100B depict the nucleic acid sequence (SEQ ID NO:185) encoding human P4HA2 polypeptide and the amino acid sequence of human P4HA2 polypeptide (SEQ ID NO:186).

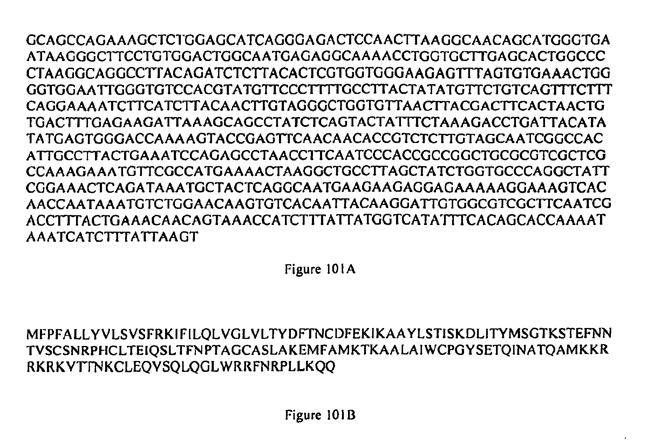

Figure 101A and 101B depict the nucleic acid sequence (SEQ ID NO:187) encoding human TSLP polypeptide and the amino acid sequence of human TSLP polypeptide (SEQ ID NO:188).

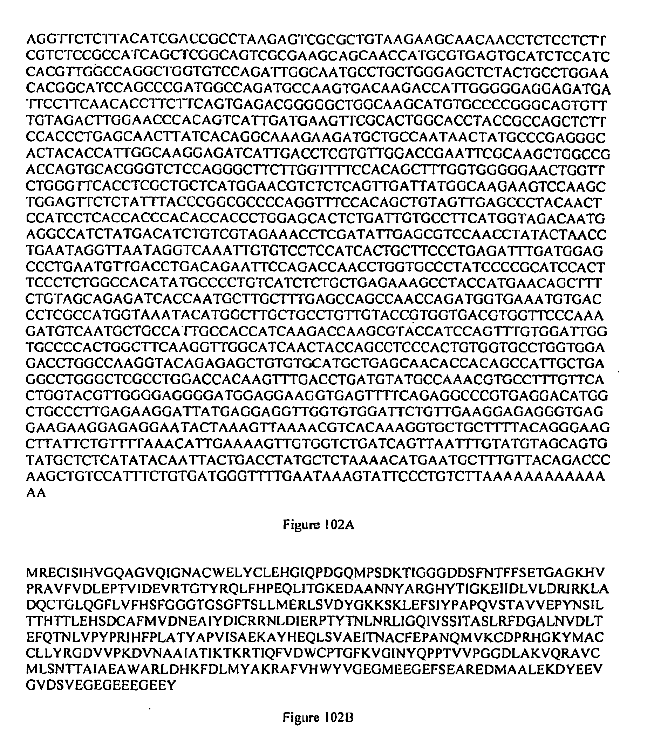

Figure 102A and 102B depict the nucleic acid sequence (SEQ ID NO:189) encoding human tubulin alpha 5/alpha 3 polypeptide and the amino acid sequence of human tubulin alpha 5/alpha 3 polypeptide (SEQ ID NO:190).

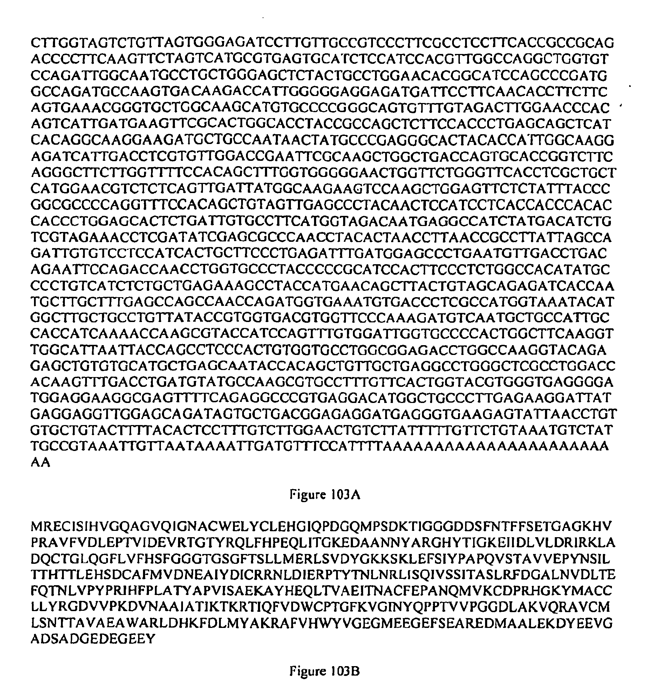

Figure 103A and 103B depict the nucleic acid sequence (SEQ ID NO:191) encoding human tubulin alpha 6 polypeptide and the amino acid sequence of human tubulin alpha 6 polypeptide (SEQ ID NO:192).
Figure 104 shows a meta-analysis of non-synonymous GLI1 SNP rs2228226 in Scotland, Cambridge and Sweden using Mantel-Haenszel method.
Figure 105 shows Q1100E disrupts a conserved region of the GLI1 protein and reduces GLI1 transcriptional activity.
Figure 106 shows expression of hedgehog (HH) signalling components in the healthy human adult colon (HC) and ulcerative colitis (UC).
Figure 107 shows the results in which *Gli1*+/- animals show mortality, severe clinical symptoms, and profound weight loss after DSS treatment.
Figure 108 shows *Gli1*+/- animals demonstrate more severe intestinal inflammation than WT littermates in response to DSS treatment.
Figure 109 shows cytokine analysis of *Gli1+*/*-* and WT mice after DSS treatments demonstrates robust pro-inflammatory cytokine activation.

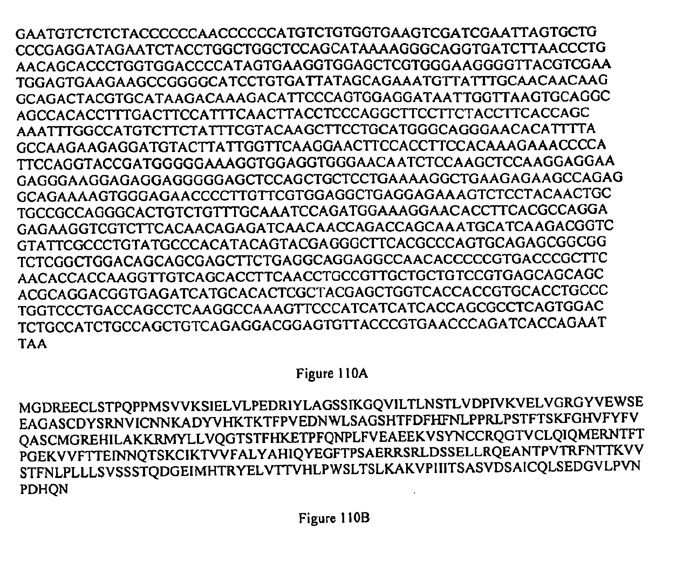

Figure 110A-B shows the (A) nucleic acid sequence encoding human arrestin domain containing 5 (ARRDC5) (LOC342959) and the (B) amino acid sequence of human ARRDC5 polypeptide.

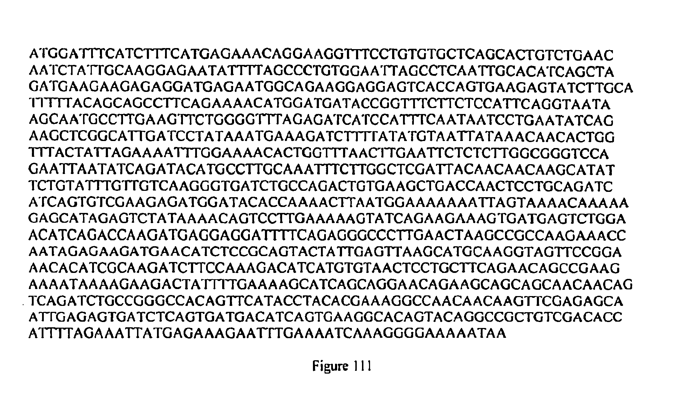

Figure 111 shows the nucleic acid sequence corresponding to human ataxin 3-like (ATXN3L).

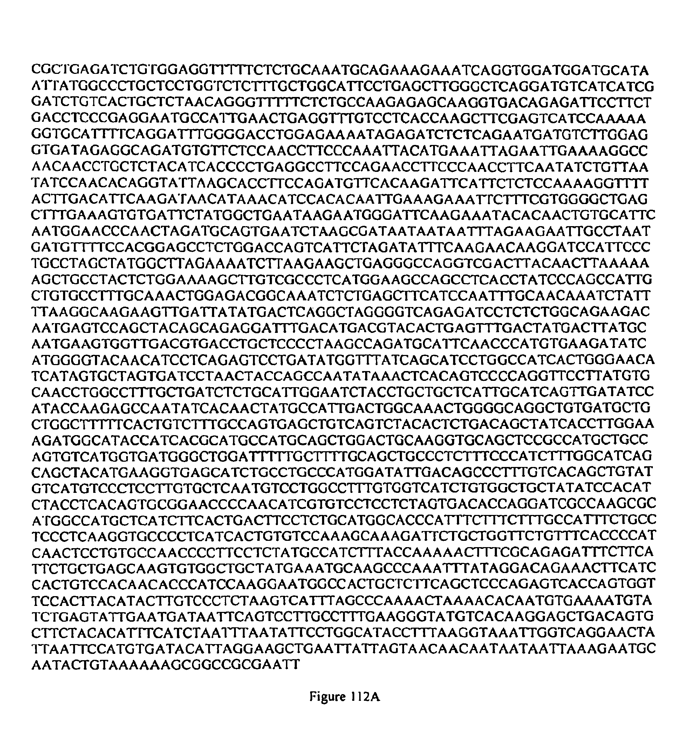

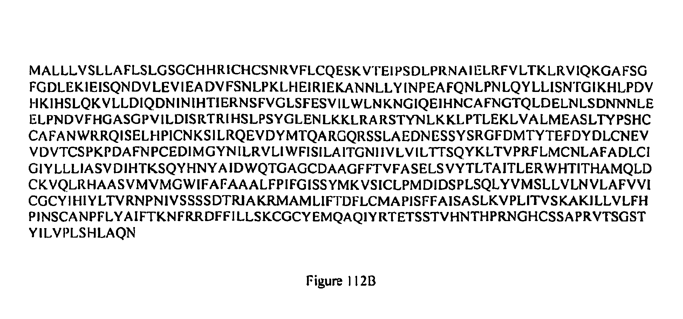

Figure 112A-B shows the (A) nucleic acid sequence encoding human follicle stimulating hormone receptor (FSHR) (LOC92552) and (B) the amino acid sequence of human FSHR polypeptide.

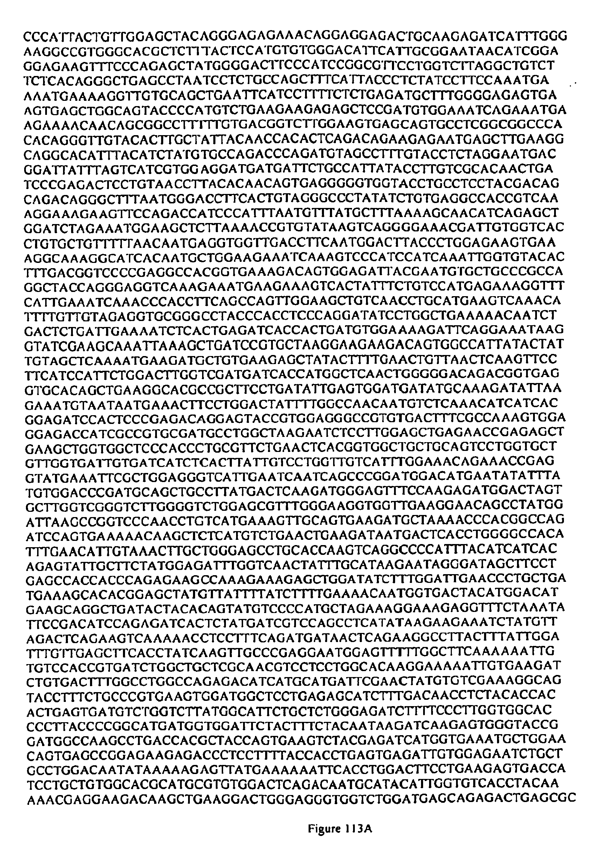

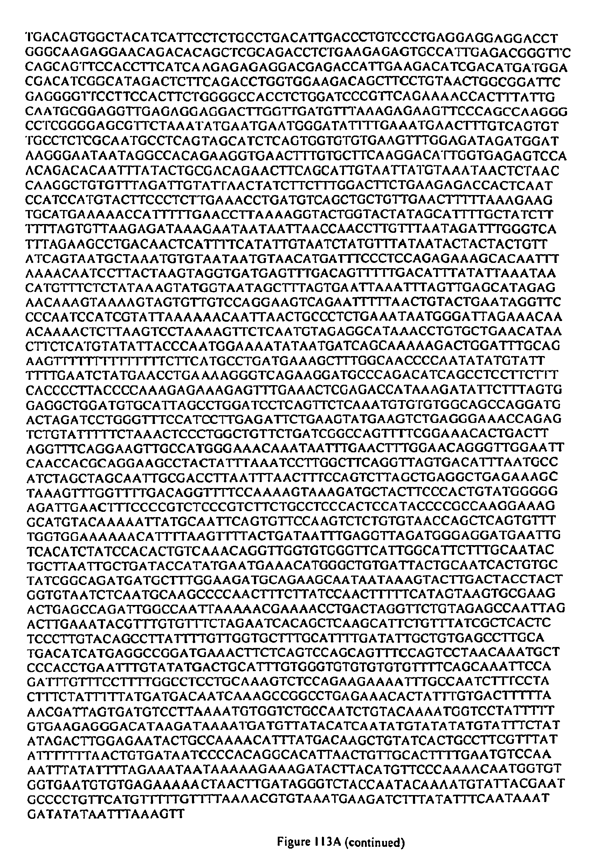

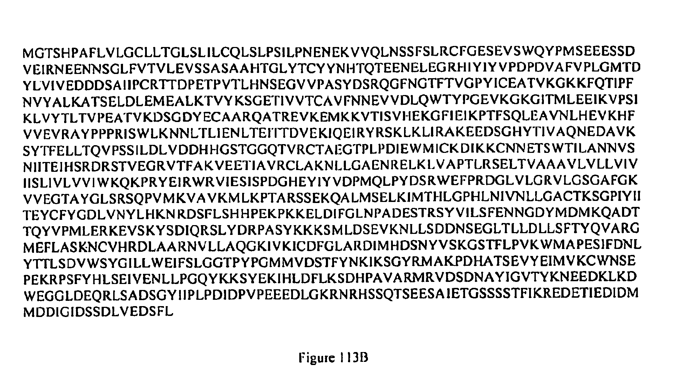

Figure 113A-B shows the (A) nucleic acid sequence encoding human Platelet-derived growth factor receptor, alpha polypeptide (PDGFRA) and (B) the amino acid sequence of human PDGFRA polypeptide.

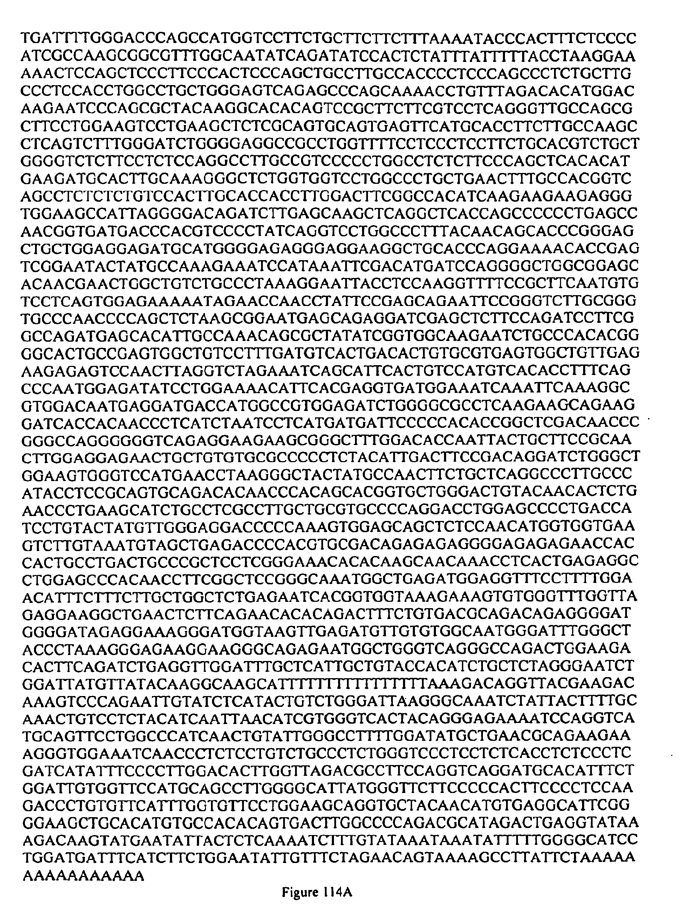

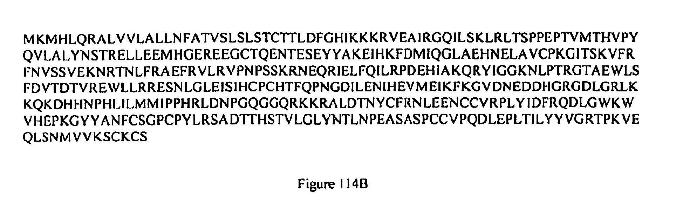

Figure 114A-B shows the (A) nucleic acid sequence encoding human transforming growth factor beta 3 (TGFB3) and (B) the amino acid sequence of human TGFB3 polypeptide.

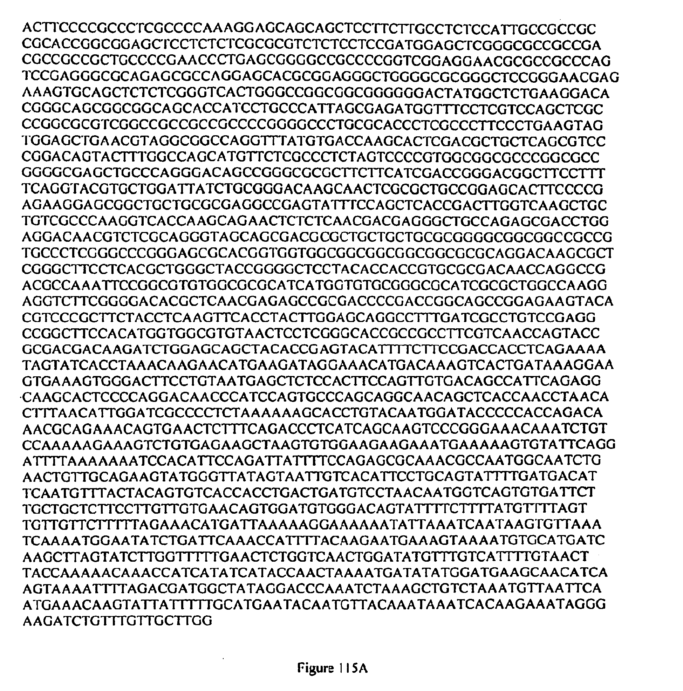

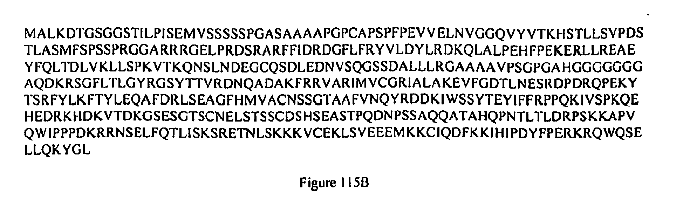

Figure 115A-B shows the (A) nucleic acid sequence encoding human potassium channel tetramerisation domain containing 8 (KCTD8) and (B) the amino acid sequence of human KCTD8 polypeptide.

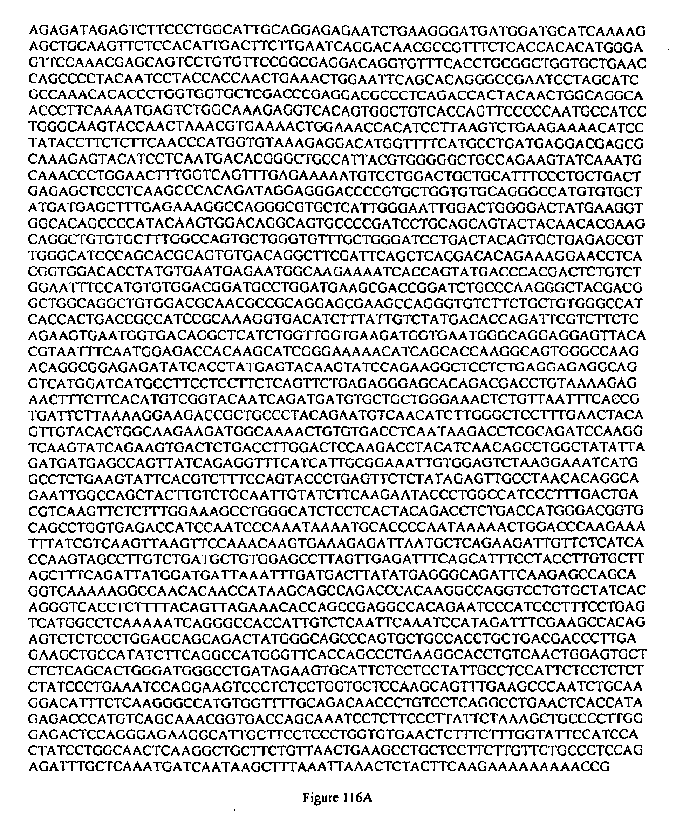

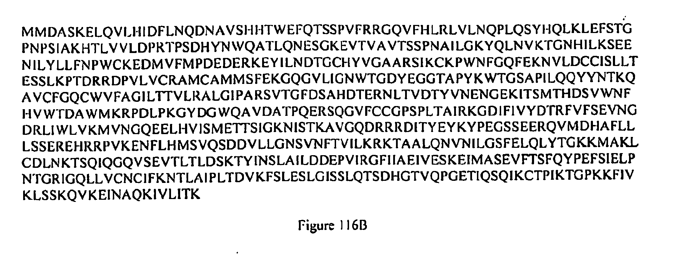

Figure 116A-B shows the (A) nucleic acid sequence encoding human transglutaminase 4 (TGM4) and (B) the amino acid sequence of human TGM4 polypeptide.

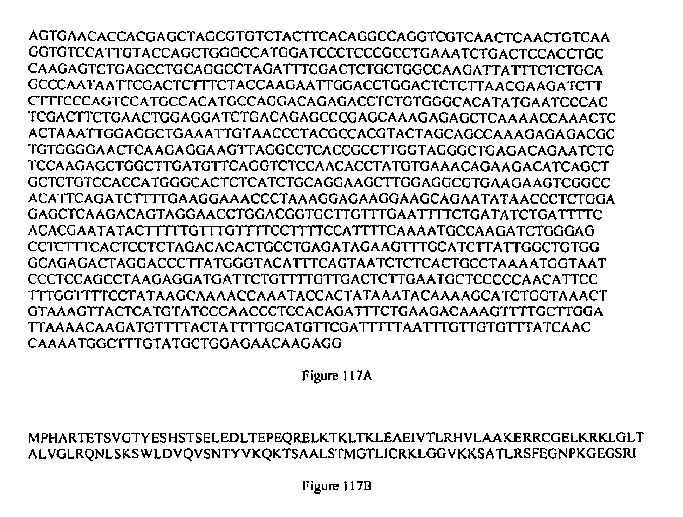

Figure 117A-B shows the (A) nucleic acid sequence encoding human TPD52L3 tumor protein D52-like 3 (NYD-SP25) and (B) the amino acid sequence of human NYD-SP25 polypeptide.

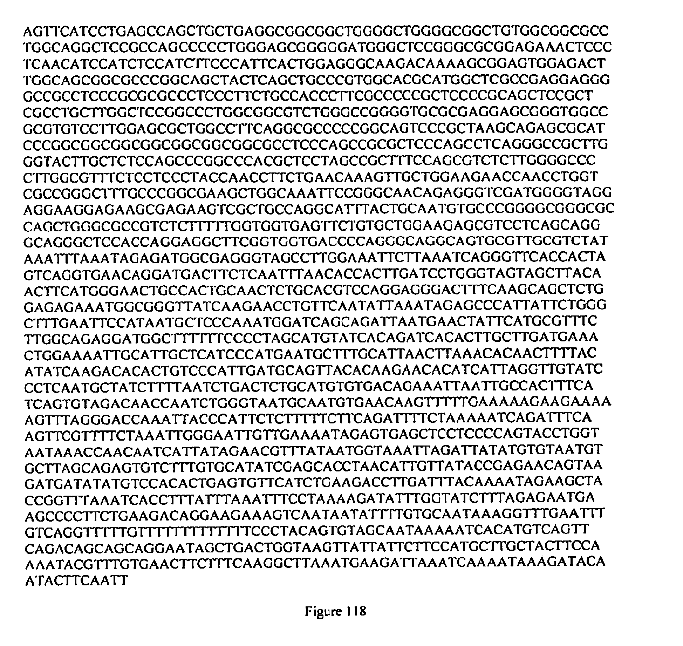

Figure 118 shows the nucleic acid sequence corresponding to misc_RNA (C3orf53), FLJ33651.

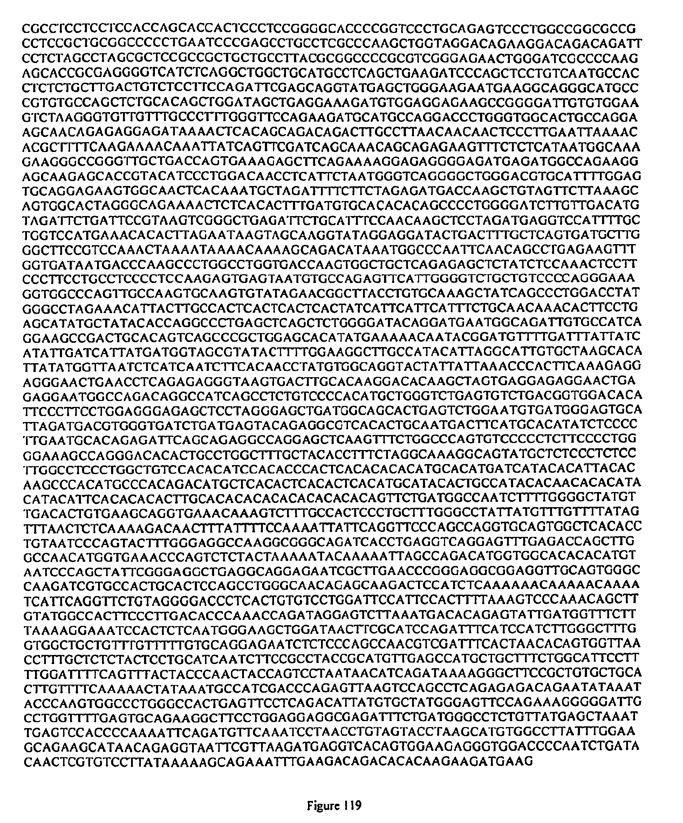

Figure 119 shows the nucleic acid sequence corresponding to EMX2 opposite strand (non-protein coding) (EMX2OS) on chromosome 10.

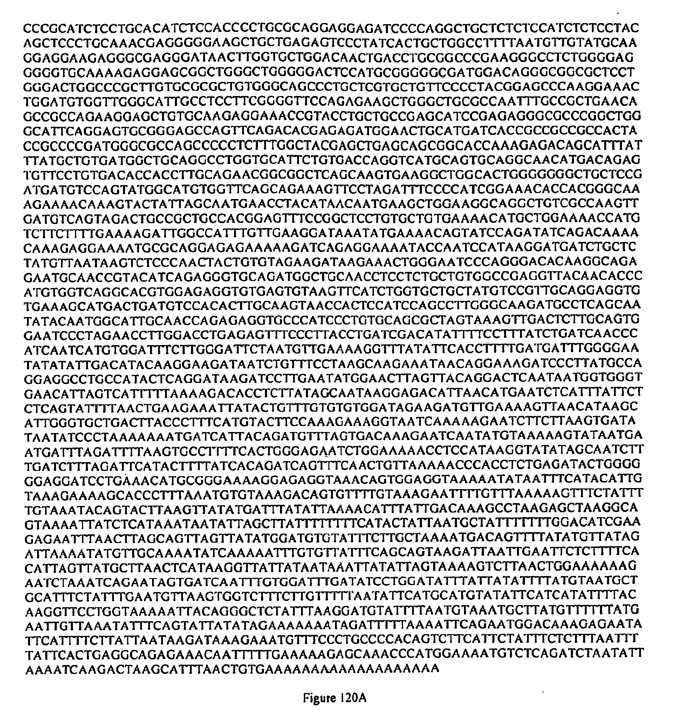

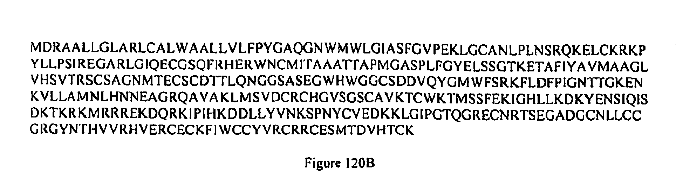

Figure 120A-B shows the (A) nucleic acid sequence encoding human wingless-type MMTV integration site family, member 16 (WNT16) and (B) the amino acid sequence of human WNT16 polypeptide.

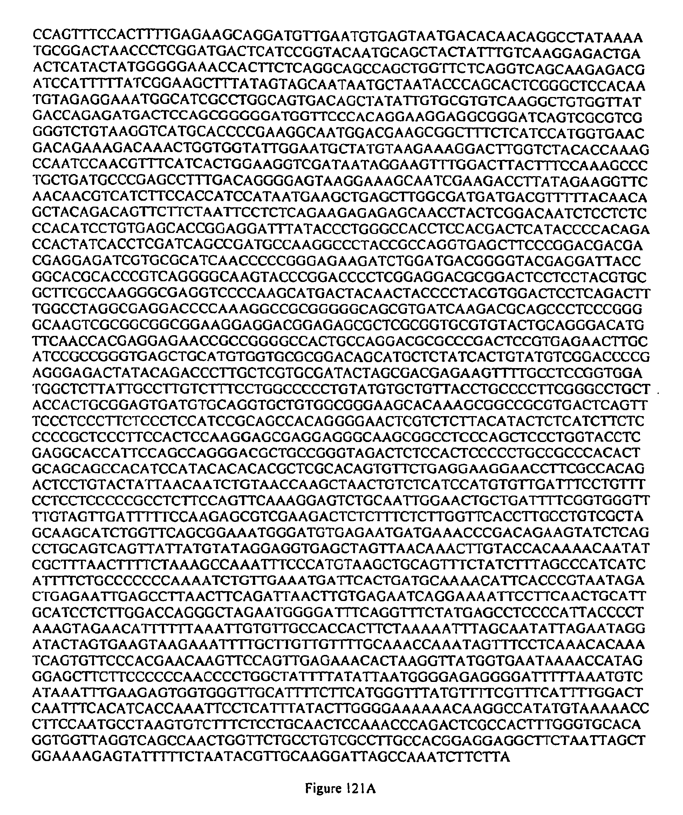

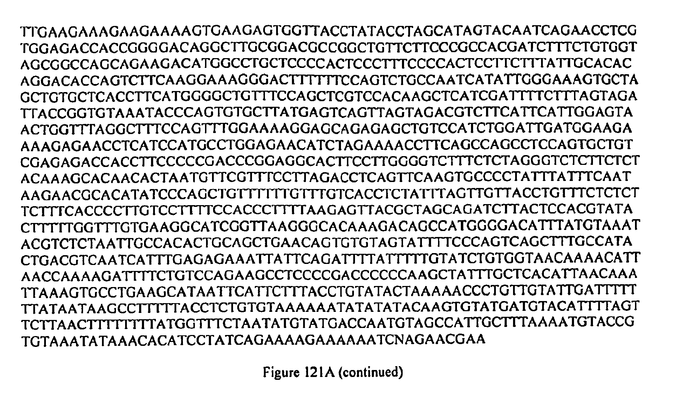

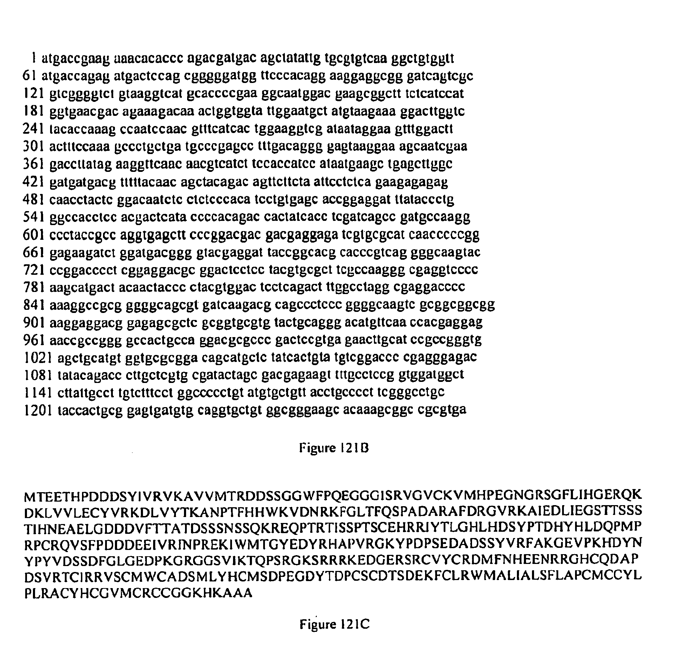

Figure 121A-C shows the (A-B) nucleic acid sequences encoding human sprouty-related, EVH1 domain containing 2 (SPRED2) and (C) the amino acid sequence of human SPRED2.

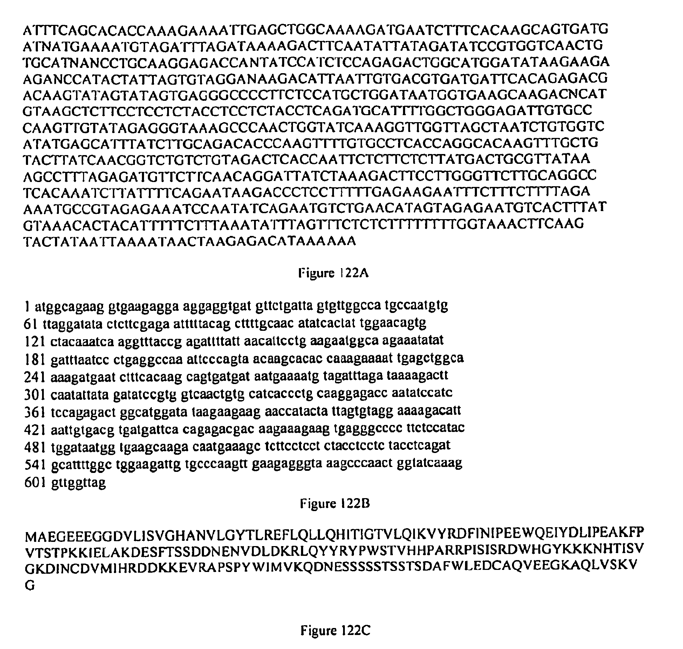

Figure 122A-C shows the (A-B) nucleic acid sequences encoding human chromosome 16 open reading frame 65 (C16orf65) and (C) the amino acid sequence of human chromosome 16 open reading frame 65 (C16orf65).

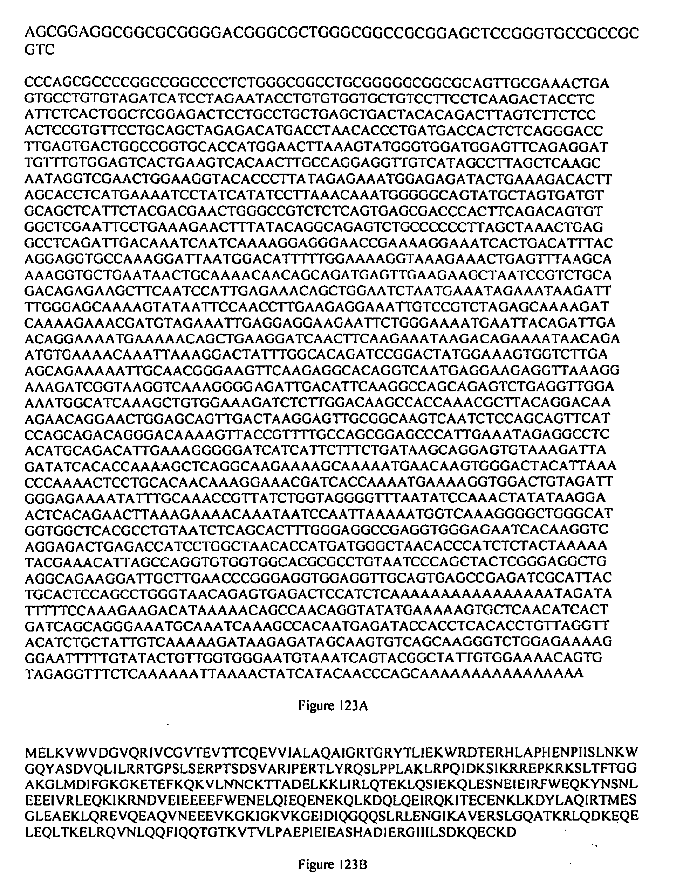

Figure 123A-B shows the (A) nucleic acid sequence encoding human chromosome 12 open reading frame 2 (C12orf2) and (B) the amino acid sequence of human chromosome 12 open reading frame 2 (C12orf2).

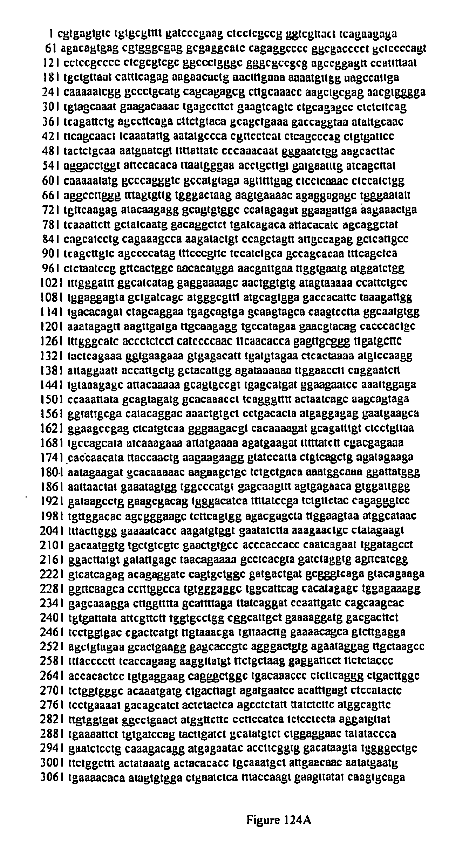

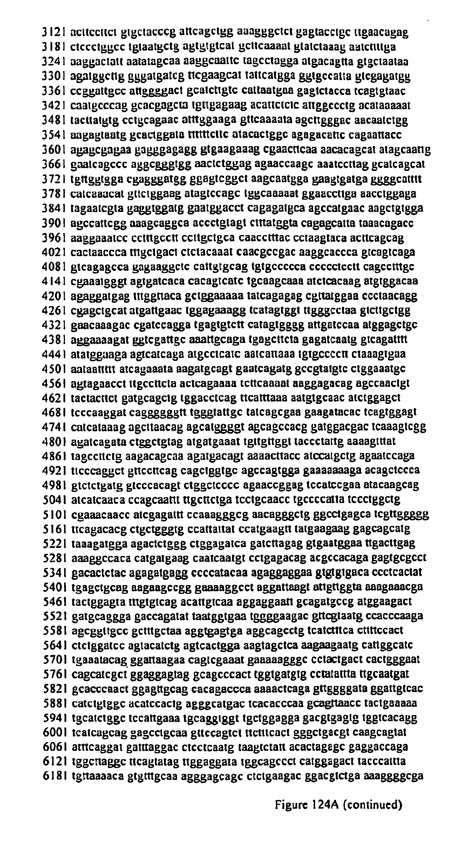

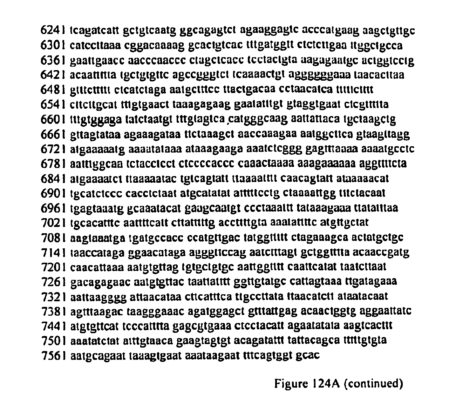

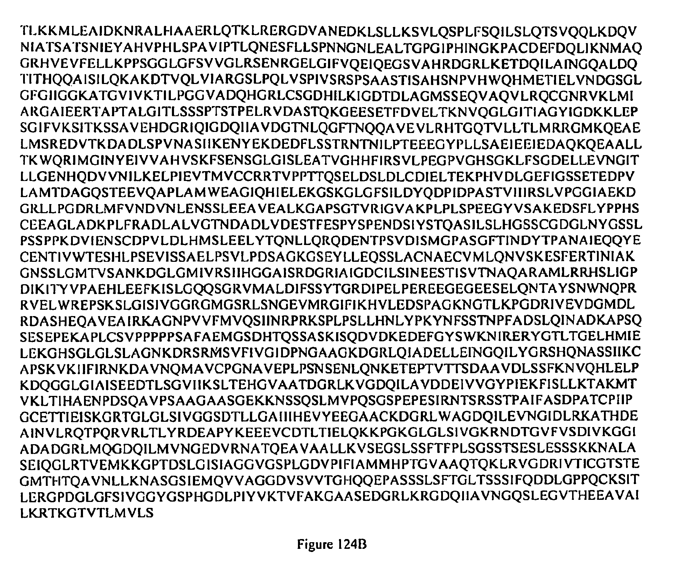

Figure 124A-B shows the (A) nucleic acid sequence encoding human multiple PDZ domain protein (MPDZ) and (B) the amino acid sequence of human MPDZ.

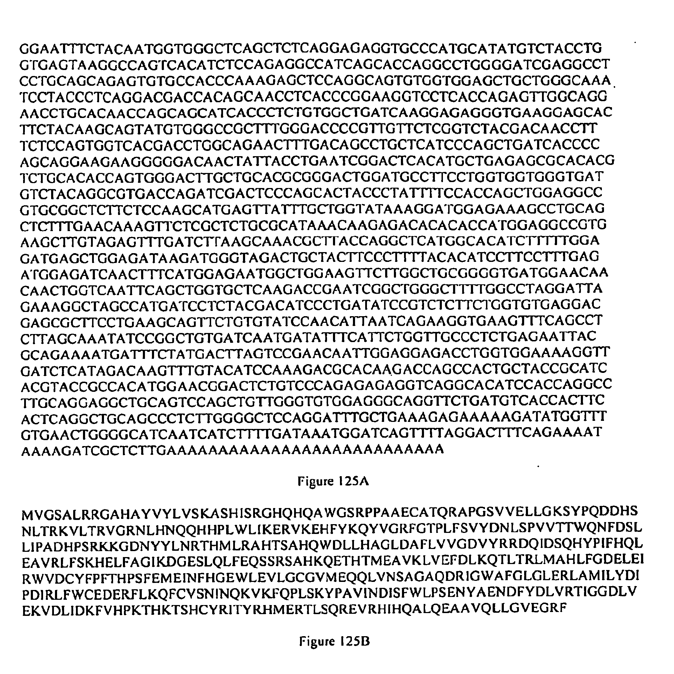

Figure 125A-B shows the (A) nucleic acid sequence encoding human phenylalanine-tRNA synthetase 2 (FARS2) and (B) the amino acid sequence of human FARS2.

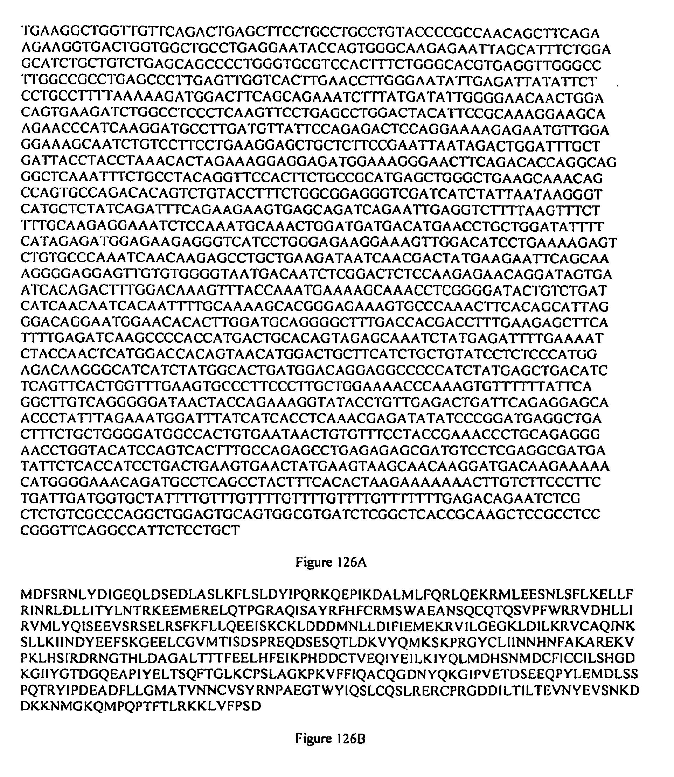

Figure 126A-B shows the (A) nucleic acid sequence encoding human caspase 8, apoptosis-related cysteine protease (CASP8) and (B) the amino acid sequence of human CASP8.

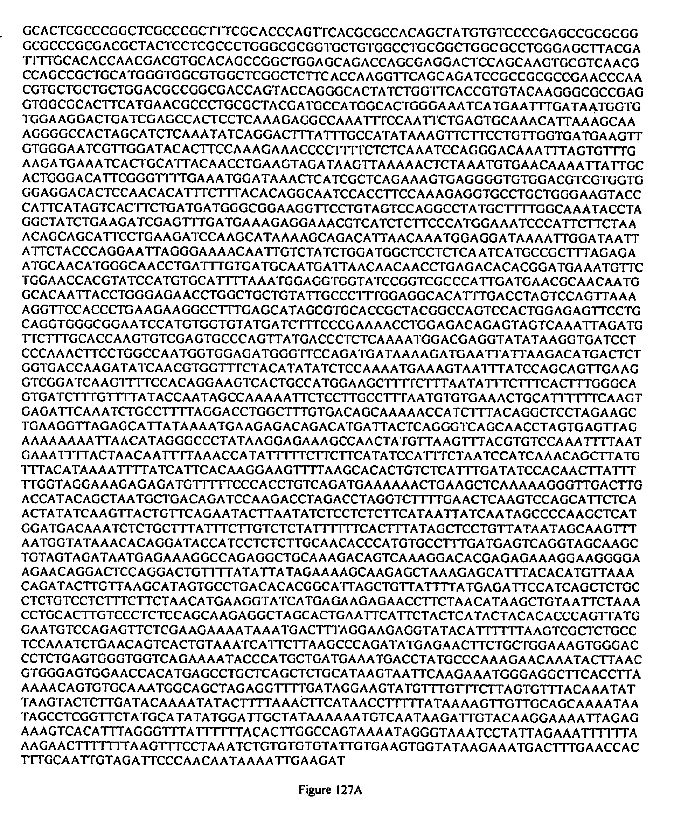

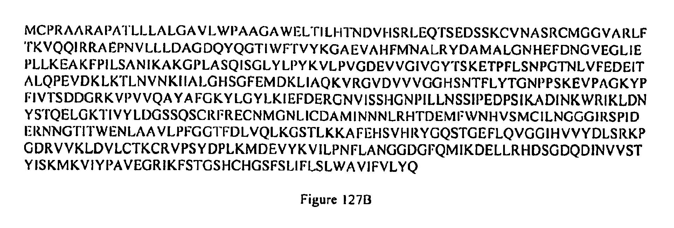

Figure 127A-B shows the (A) nucleic acid sequence encoding human 5'-nucleotidase, ecto (CD73) (NT5E) and (B) the amino acid sequence of human NT5E.

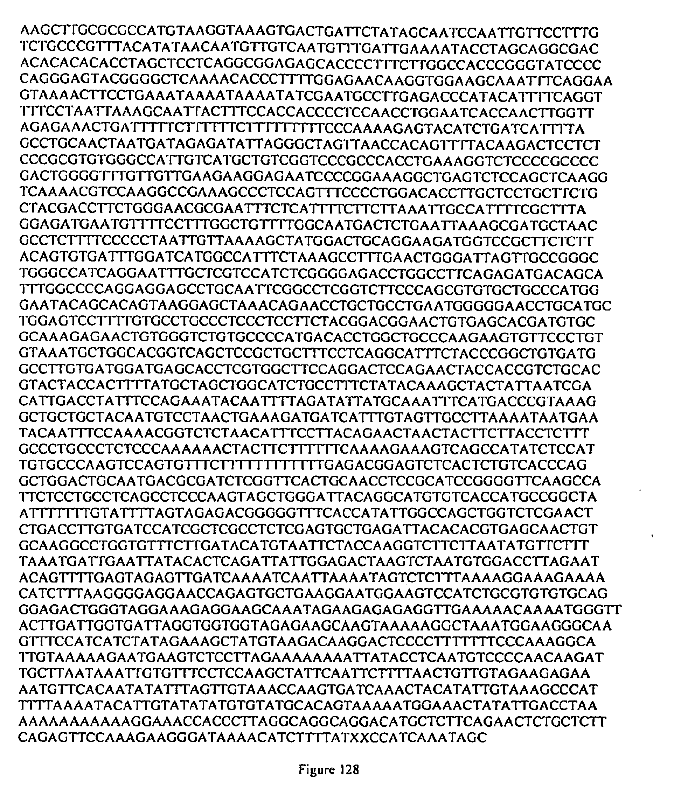

Figure 128 shows the nucleic acid sequence corresponding to human teratocarcinoma-derived growth factor 3 (TDGF3).

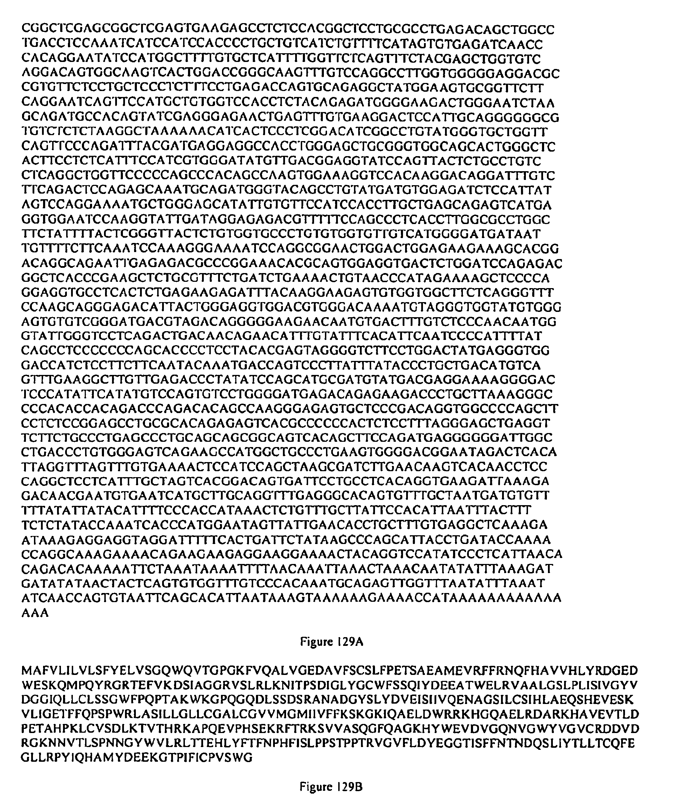

Figure 129A-B shows the (A) nucleic acid sequence encoding human butyrophilin-like 3 (BTNL3) and (B) the amino acid sequence of human BTNL3.

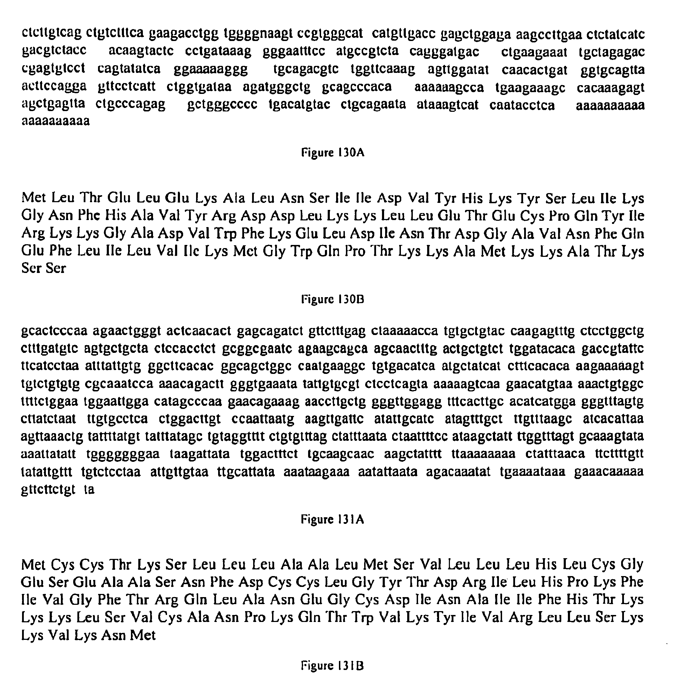

Figure 130A-B shows the (A) nucleic acid sequence encoding human S100A8 and (B) the amino acid sequence of human S100A8.
Figure 131A-B shows the (A) nucleic acid sequence encoding human CCL20 and (B) the amino acid sequence of human CCL20.

### Detailed Description of the Invention

### A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

The term "inflammatory bowel disease" or "IBD" is used as a collective term for ulcerative colitis and Crohn's disease. Although the two diseases are generally considered as two different entities, their common characteristics, such as patchy necrosis of the surface epithelium, focal accumulations of leukocytes adjacent to glandular crypts, and an increased number of intraepithelial lymphocytes (IEL) and certain macrophage subsets, justify their treatment as a single disease group.

The term "Crohn's disease" or "CD" is used herein to refer to a condition involving chronic inflammation of the gastrointestinal tract. Crohn's-related inflammation usually affects the intestines, but may occur anywhere from the mouth to the anus. CD differs from UC in that the inflammation extends through all layers of the intestinal wall and involves mesentery as well as lymph nodes. The disease is often discontinuous, i.e., severely diseased segments of bowel are separated from apparently disease-free areas. In CD, the bowel wall also thickens which can lead to obstructions, and the development of fistulas and fissures are not uncommon. As used herein, CD may be one or more of several types of CD, including without limitation, ileocolitis (affects the ileum and the large intestine); ileitis (affects the ileum); gastroduodenal CD (inflammation in the stomach and the duodenum); jejunoileitis (spotty patches of inflammation in the jejunum); and Crohn's (granulomatous) colitis (only affects the large intestine).

The term "ulcerative colitis" or "UC" is used herein to refer to a condition involving inflammation of the large intestine and rectum. In patients with UC, there is an inflammatory reaction primarily involving the colonic mucosa. The inflammation is typically uniform and continuous with no intervening areas of normal mucosa. Surface mucosal cells as well as crypt epithelium and submucosa are involved in an inflammatory reaction with neutrophil infiltration. Ultimately, this reaction typically progresses to epithelial damage and loss of epithelial cells resulting in multiple ulcerations, fibrosis, dysplasia and longitudinal retraction of the colon.

The term "inactive" IBD is used herein to mean an IBD that was previously diagnosed in an individual but is currently in remission. This is in contrast to an "active" IBD in which an individual has been diagnosed with and IBD but has not undergone treatment. In addition, the active IBD may be a recurrence of a previously diagnosed and treated IBD that had gone into remission (i.e. become an inactive IBD). Such recurrences may also be referred to herein as "flare-ups" of an IBD. Mammalian subjects having an active autoimmune disease, such as an IBD, may be subject to a flare-up, which is a period of heightened disease activity or a return of corresponding symptoms. Flare-ups may occur in response to severe infection, allegic reactions, physical stress, emotional trauma, surgery, or environmental factors.

The term "modulate" is used herein to mean that the expression of the gene, or level of RNA molecule or equivalent RNA molecules encoding one or more proteins or protein subunits, or activity of one or more proteins or protein subunits is up regulated or down regulated, such that expression, level, or activity is greater than or less than that observed in the absence of the modulator.

The terms "inhibit", "down-regulate", "underexpress" and "reduce" are used interchangeably and mean that the expression of a gene, or level of RNA molecules or equivalent RNA molecules encoding one or more proteins or protein subunits, or activity of one or more proteins or protein subunits, is reduced relative to one or more controls, such as, for example, one or more positive and/or negative controls.

The term "up-regulate" or "overexpress" is used to mean that the expression of a gene, or level of RNA molecules or equivalent RNA molecules encoding one or more proteins or protein subunits, or activity of one or more proteins or protein subunits, is elevated relative to one or more controls, such as, for example, one or more positive and/or negative controls.

The term "diagnosis" is used herein to refer to the identification of a molecular or pathological state, disease or condition, such as the identification of IBD.

The term "prognosis" is used herein to refer to the prediction of the likelihood of IBD development or progression, including autoimmune flare-ups and recurrences following surgery. Prognostic factors are those variables related to the natural history of IBD, which influence the recurrence rates and outcome of patients once they have developed IBD. Clinical parameters that may be associated with a worse prognosis include, for example, an abdominal mass or tenderness, skin rash, swollen joints, mouth ulcers, and borborygmus (gurgling or splashing sound over the intestine). Prognostic factors may be used to categorize patients into subgroups with different baseline recurrence risks.

The "pathology" of an IBD includes all phenomena that compromise the well-being of the patient. IBD pathology is primarily attributed to abnormal activation of the immune system in the intestines that can lead to chronic or acute inflammation in the absence of any known foreign antigen, and subsequent ulceration. Clinically, IBD is characterized by diverse manifestations often resulting in a chronic, unpredictable course. Bloody diarrhea and abdominal pain are often accompanied by fever and weight loss. Anemia is not uncommon, as is severe fatigue. Joint manifestations ranging from arthralgia to acute arthritis as well as abnormalities in liver function are commonly associated with IBD. During acute "attacks" of IBD, work and other normal activity are usually impossible, and often a patient is hospitalized.

The aetiology of these diseases is unknown and the initial lesion has not been clearly defined; however, patchy necrosis of the surface epithelium, focal accumulations of leukocytes adjacent to glandular crypts, and an increased number of intraepithelial lymphocytes and certain macrophage subsets have been described as putative early changes, especially in Crohn's disease.

The term "treatment" refers to both therapeutic treatment and prophylactic or preventative measures for IBD, wherein the object is to prevent or slow down (lessen) the targeted pathologic condition or disorder. Those in need of treatment include those already with an IBD as well as those prone to have an IBD or those in whom the IBD is to be prevented. Once the diagnosis of an IBD has been made by the methods disclosed herein, the goals of therapy are to induce and maintain a remission.

Various agents that are suitable for use as an "IBD therapeutic agent" are known to those of ordinary skill in the art. As described herein, such agents include without limitation, aminosalicylates, corticosteroids, and immunosuppressive agents.

The term "test sample" refers to a sample from a mammalian subject suspected of having an IBD, known to have an IBD, or known to be in remission from an IBD. The test sample may originate from various sources in the mammalian subject including, without limitation, blood, semen, serum, urine, bone marrow, mucosa, tissue, etc.

The term "control" or "control sample" refers a negative control in which a negative result is expected to help correlate a positive result in the test sample. Controls that are suitable for the present invention include, without limitation, a sample known to have normal levels of gene expression, a sample obtained from a mammalian subject known not to have an IBD, and a sample obtained from a mammalian subject known to be normal. A control may also be a sample obtained from a subject previously diagnosed and treated for an IBD who is currently in remission; and such a control is useful in determining any recurrence of an IBD in a subject who is in remission. In addition, the control may be a sample containing normal cells that have the same origin as cells contained in the test sample. Those of skill in the art will appreciate other controls suitable for use in the present invention.

The term "microarray" refers to an ordered arrangement of hybridizable array elements, preferably polynucleotide probes, on a substrate.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The terms "differentially expressed gene," "differential gene expression" and their synonyms, which are used interchangeably, refer to a gene whose expression is activated to a higher or lower level in a subject suffering from a disease, specifically an IBD, such as UC or CD, relative to its expression in a normal or control subject. The terms also include genes whose expression is activated to a higher or lower level at different stages of the same disease. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disease, specifically an IBD, or between various stages of the same disease. Differential expression includes both quantitative, as well as qualitative, differences in the temporal or cellular expression pattern in a gene or its expression products among, for example, normal and diseased cells, or among cells which have undergone different disease events or disease stages. For the purpose of this invention, "differential gene expression" is considered to be present when there is at least an about two-fold, preferably at least about four-fold, more preferably at least about six-fold, most preferably at least about ten-fold difference between the expression of a given gene in normal and diseased subjects, or in various stages of disease development in a diseased subject.

The term "over-expression" with regard to an RNA transcript is used to refer to the level of the transcript determined by normalization to the level of reference mRNAs, which might be all transcripts detected in the specimen or a particular reference set of mRNAs.

The phrase "gene amplification" refers to a process by which multiple copies of a gene or gene fragment are formed in a particular cell or cell line. The duplicated region (a stretch of amplified DNA) is often referred to as "amplicon". Usually, the amount of the messenger RNA (mRNA) produced, *i.e.,* the level of gene expression, also increases in the proportion of the number of copies made of the particular gene expressed.

In general, the term "marker" or "biomarker" or refers to an identifiable physical location on a chromosome, such as a restriction endonuclease recognition site or a gene, whose inheritance can be monitored. The marker may be an expressed region of a gene referred to as a "gene expression marker", or some segment of DNA with no known coding function. An "IBD marker" as used herein refers those genes listed in Tables 1, 2, and 3.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate), 50% formamide, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

The terms "splicing" and "RNA splicing" are used interchangeably and refer to RNA processing that removes introns and joins exons to produce mature mRNA with continuous coding sequence that moves into the cytoplasm of an eukaryotic cell.

In theory, the term "exon" refers to any segment of an interrupted gene that is represented in the mature RNA product (B. Lewin. Genes IV Cell Press, Cambridge Mass. 1990). In theory the term "intron" refers to any segment of DNA that is transcribed but removed from within the transcript by splicing together the exons on either side of it. Operationally, exon sequences occur in the mRNA sequence of a gene as defined by Ref. SEQ ID numbers. Operationally, intron sequences are the intervening sequences within the genomic DNA of a gene, bracketed by exon sequences and having GT and AG splice consensus sequences at their 5' and 3' boundaries.

An "interfering RNA" or "small interfering RNA (siRNA)" is a double stranded RNA molecule usually less than about 30 nucleotides in length that reduces expression of a target gene. Interfering RNAs may be identified and synthesized using known methods (Shi Y., Trends in Genetics 19(1):9-12 (2003), WO/2003056012 and WO2003064621), and siRNA libraries are commercially available, for example from Dharmacon, Lafayette, Colorado.

A "native sequence" polypeptide is one which has the same amino acid sequence as a polypeptide derived from nature, including naturally occurring or allelic variants. Such native sequence polypeptides can be isolated from nature or can be produced by recombinant or synthetic means. Thus, a native sequence polypeptide can have the amino acid sequence of naturally occurring human polypeptide, murine polypeptide, or polypeptide from any other mammalian species.

The term "antibody" herein is used in the broadest sense and specifically covers monoclonal antibodies, polyclonal antibodies, multispecific antibodies (*e.g*. bispecific antibodies), and antibody fragments, so long as they exhibit the desired biological activity. The present invention particularly contemplates antibodies against one or more of the IBD markers disclosed herein. Such antibodies may be referred to as "anti-IBD marker antibodies".

The term "monoclonal antibody" as used herein refers to an antibody from a population of substantially homogeneous antibodies, *i.e.,* the individual antibodies comprising the population are identical and/or bind the same epitope(s), except for possible variants that may arise during production of the monoclonal antibody, such variants generally being present in minor amounts. Such monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (*e.g*. Old World Monkey, Ape etc) and human constant region sequences, as well as "humanized" antibodies.

"Humanized" forms of non-human (*e.g*., rodent) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity.

An "intact antibody" herein is one which comprises two antigen binding regions, and an Fc region. Preferably, the intact antibody has a functional Fc region.

"Antibody fragments" comprise a portion of an intact antibody, preferably comprising the antigen binding region thereof. Examples of antibody fragments include Fab, Fab', F(ab')₂, and Fv fragments; diabodies; linear antibodies; single-chain antibody molecules; and multispecific antibodies formed from antibody fragment(s).

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end. The constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)).

The term *"hypervariable region," "HVR,"* or *"HV,"* when used herein refers to the regions of an antibody-variable domain that are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, *e.g.,* Xu et al. Immunity 13:37-45 (2000); Johnson and Wu in Methods in Molecular Biology 248:1-25 (Lo, ed., Human Press, Totowa, NJ, 2003)). Indeed, naturally occurring camelid antibodies consisting of a heavy chain only are functional and stable in the absence of light chain. See, *e.g*., Hamers-Casterman et al., Nature 363:446-448 (1993) and Sheriff et al., Nature Struct. Biol. 3:733-736 (1996).

A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The end of the Chothia CDR-H1 loop when numbered using the Kabat numbering convention varies between H32 and H34 (see below) depending on the length of the loop (this is because the Kabat numbering scheme places the insertions at H35A and H35B; if neither 35A nor 35B is present, the loop ends at 32; if only 35A is present, the loop ends at 33; if both 35A and 35B are present, the loop ends at 34). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these hypervariable regions are noted below.

| Loop | Kabat | AbM | Chothia | Contact |
|---|---|---|---|---|
| | | | | |
| ---- | ----- | ----- | ----- | ----- |
| | | | | |
| L1 | L24-L34 | L24-L34 | L24-L34 | L30-L36 |
| | | | | |
| L2 | L50-L56 | L50-L56 | L50-L56 | L46-L55 |
| | | | | |
| L3 | L89-L97 | L89-L97 | L89-L97 | L89-L96 |
| | | | | |
| H1 | H31-H35B | H26-H35B | H26-H32, 33 or 34 | H30-H35B (Kabat Numbering) |
| | | | | |
| H1 | H31-H35 | H26-H35 | H26-H32 | H30-H35 (Chothia Numbering) |
| | | | | |
| H2 | H50-H65 | H50-H58 | H52-H56 | H47-H58 |
| | | | | |
| H3 | H95-H102 | H95-H102 | H95-H102 | H93-H101. |

Hypervariable regions may comprise "extended hypervariable regions" as follows: 24-36 or 24-34 (L1), 46-56 or 50-56 (L2) and 89-97 (L3) in the VL and 26-35B (H1), 50-65, 47-65 or 49-65 (H2) and 93-102, 94-102 or 95-102 (H3) in the VH. These extended hypervariable regions are typically combinations of the Kabat and Chothia definitions, which may optionally further include residues identified using the Contact definition. The variable domain residues are numbered according to Kabat *et al., supra* for each of these definitions.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa (κ) and lambda (λ), based on the amino acid sequences of their constant domains.

The term "Fc region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue.

Unless indicated otherwise, herein the numbering of the residues in an immunoglobulin heavy chain is that of the EU index as in Kabat el al., Sequences of Proteins of Immunological Interest, 5th Ed. Public Health Service, National Institutes of Health, Bethesda, MD (1991), expressly incorporated herein by reference. The "EU index as in Kabat" refers to the residue numbering of the human IgG1 EU antibody.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "variant Fc region" comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, *e.g*. from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

Depending on the amino acid sequence of the constant domain of their heavy chains, intact antibodies can be assigned to different "classes". There are five major classes of intact antibodies: IgA, IgD, IgE, IgG, and IgM, and several of these may be further divided into "subclasses" (isotypes), *e.g*., IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

"Single-chain Fv" or "scFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a variable heavy domain (V_{H}) connected to a variable light domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger el al., Proc. Natl. Acad. Sci. USA, 90:6444-6448 (1993).

A "naked antibody" is an antibody that is not conjugated to a heterologous molecule, such as a small molecule or radiolabel.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

An "affinity matured" antibody is one with one or more alterations in one or more hypervariable regions thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. BiolTechnology 10:779-783 (1992) describes affinity maturation by VH and VL domain shuffling. Random mutagenesis of HVR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

An "amino acid sequence variant" antibody herein is an antibody with an amino acid sequence which differs from a main species antibody. Ordinarily, amino acid sequence variants will possess at least about 70% homology with the main species antibody, and preferably, they will be at least about 80%, more preferably at least about 90% homologous with the main species antibody. The amino acid sequence variants possess substitutions, deletions, and/or additions at certain positions within or adjacent to the amino acid sequence of the main species antibody. Examples of amino acid sequence variants herein include an acidic variant (*e.g*. deamidated antibody variant), a basic variant, an antibody with an amino-terminal leader extension (*e.g*. VHS-) on one or two light chains thereof, an antibody with a C-terminal lysine residue on one or two heavy chains thereof, etc., and includes combinations of variations to the amino acid sequences of heavy and/or light chains. The antibody variant of particular interest herein is the antibody comprising an amino-terminal leader extension on one or two light chains thereof, optionally further comprising other amino acid sequence and/or glycosylation differences relative to the main species antibody.

A "glycosylation variant" antibody herein is an antibody with one or more carbohydrate moieities attached thereto which differ from one or more carbohydrate moieties attached to a main species antibody. Examples of glycosylation variants herein include antibody with a G1 or G2 oligosaccharide structure, instead a G0 oligosaccharide structure, attached to an Fc region thereof, antibody with one or two carbohydrate moieties attached to one or two light chains thereof, antibody with no carbohydrate attached to one or two heavy chains of the antibody, etc., and combinations of glycosylation alterations.

Where the antibody has an Fc region, an oligosaccharide structure may be attached to one or two heavy chains of the antibody, *e.g*. at residue 299 (298, Eu numbering of residues). For pertuzumab, G0 was the predominant oligosaccharide structure, with other oligosaccharide structures such as G0-F, G-1, Man5, Man6, G1-1, G1(1-6), G1(1-3) and G2 being found in lesser amounts in the pertuzumab composition.

Unless indicated otherwise, a "G1 oligosaccharide structure" herein includes G-1, G1-1, G1(1-6) and G1(1-3) structures.

An "amino-terminal leader extension" herein refers to one or more amino acid residues of the amino-terminal leader sequence that are present at the amino-terminus of any one or more heavy or light chains of an antibody. An exemplary amino-terminal leader extension comprises or consists of three amino acid residues, VHS, present on one or both light chains of an antibody variant.

A "deamidated" antibody is one in which one or more asparagine residues thereof has been derivatized, *e.g*. to an aspartic acid, a succinimide, or an iso-aspartic acid.

### B.1 General Description of the Invention

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

As discussed above, the detection or diagnosis of IBD is currently obtained by various classification systems that rely on a number of variables observed in a patient. The present invention is based on the identification of genes that are associated with IBD. Accordingly, the expression levels of such genes can serve as diagnostic markers to identify patients with IBD. As described in the Examples, the differential expression of a number of genes in IBD patients has been observed. Thus, according to the present invention, the genes listed in Tables 1A-B, 2, and 3A-B have been identified as differentially expressed in IBD.

Table 1A provides a list of genes that are upregulated in IBD.

**Table 1A**

| Gene | Indication(s) | SEQ ID NO nucleic acid, amino acid | Figure |
|---|---|---|---|
| Defensin, alpha 6 (DEFA6) | CD, UC | 1, 2 | 8 |
| Defensin, alpha 5 (DEFA5) | UC | 3, 4 | 9A-B |
| Defensin beta 14 (DEFB 14) | UC | 229,230 | 9C-D |
| IL-3 receptor, alpha low affinity (IL3RA) | CD | 5, 6 | 10 |
| IL-2 receptor, alpha (IL2RA) | CD, UC | 7, 8 | 11 |
| Regenerating islet-derived 3 gamma (REG3G) | CD, UC | 9, 10 | 12 |
| Regenerating islet-derived 1 beta pancreatic stone protein (REG1B) | CD, UC | 11, 12 | 13 |
| Potassium voltage-gated channel, Shal-related subfamily (KCND3) | CD, UC | 13, 14 | 14 |
| Human macrophage inflammatory protein 3 (MIP-3a) | CD, UC | 15, 16 | 15 |
| Endothelial cell growth factor 1 platelet-derived (ECGF1) | CD, UC | 17, 18 | 16 |
| Interleukin I beta (IL1B) | CD, UC | 19,20 | 17 |
| Growth regulated protein gamma (MIP2BGRO-g) | CD, UC | 21,22 | 18 |
| Chemokine C-X-C motif ligand 1 (CXCL1) | CD, UC | 23,24 | 19 |
| Inhibitor of apoptosis protein 1 (IAP1) | CD, UC | 25, 26 | 20 |
| Caspase 5, apoptosis-related cysteine protease (CASP5) | CD, UC | 27,28 | 21 |
| Deleted in malignant brain tumors 1 (DMBT1) | CD, UC | 29, 30 | 22 |
| Protocadherin 17 (PCDH17) | CD, UC | 31, 32 | 23 |
| Interferon-inducible protein 9-27 (IFITM1) | CD, UC | 33,34 | 24 |
| PDZK1 interacting protein 1 (PDZKIIP1) | CD, UC | 35,36 | 25 |
| IRTA2 / FCRHS (IRTA2) | CD | 37, 38 | 26 |
| Solute carrier family 40 iron-regulated transporter, member 1 (SLC40A1) | CD, UC | 39, 40 | 27 |
| Immunoglobulin heavy variable 4-4 (IGHV4-4) | CD, UC | 41,42 | 28 |
| Regenerating islet-derived 3 gamma (REG3G) | CD, UC | 43, 44 | 29 |
| Aquaporin 9 (AQP9) | CD, UC | 45, 46 | 30 |
| Olfactomedin 4 (OLFM4) | CD, UC | 47, 48 | 31 |
| S100 calcium binding protein A9 calgranulin (S 100A9) | B CD, UC | 49,50 | 32 |
| unc-5 homolog C C. elegans-like (UNC5CL) | CD, UC | 51, 52 | 33 |
| G protein-coupled receptor 110 (GPR110) | CD, UC | 53,54 | 34 |
| HLA-G histocompatibility antigen, class I, G (HLA-G) | CD, UC | 55, 56 | 35 |
| Transporter 1, ATP-binding cassette, sub-family MDR/TAP (TAP1) | B CD, UC | 57,58 | 36 |
| Mitogen-activated protein kinase kinase kinase 8 (MAP3K8) | CD, UC | 59, 60 | 37 |
| Ubiquitin D\| Gamma-aminobutyric acid GABA B receptor, 1 (UBD\|GABBR1) | CD, UC | 61,62 | 38 |
| DEAH Asp-Glu-Ala-Asp/His box polypeptide 57 (DHX57) | CD, UC | 63, 64 | 39 |
| Metastasis-associate (MA) LY6/PLAUR domain containing 5 (LYPD5) | CD, UC | 65, 66 | 40 |
| Immunoglobulin lambda joining-constant/OR18 (IGLJCOR18) | CD, UC | 67,68 | 41 |
| TNF R superfamily, member 6b, decoy (TNFRSF6B) | CD, UC | 69, 70 | 42 |
| serum amyloid A1 (SAA1) | CD, UC | 71, 72 | 43 |
| Transporter 2, ATP-binding cassette, sub-family MDR/TAP (TAP2) | B CD, UC | 73, 74 | 44 |
| PCAA 17448 | CD, UC | 75, 76 | 45 |
| lipocalin 2 oncogene 24p3 (LCN2) | CD, UC | 77, 78 | 46 |
| Z-D binding protein 1 (ZBP1) | CD, UC | 79,80 | 47 |
| TNFAIP3 interacting protein 3 (TNIP3) | CD, UC | 81,82 | 48 |
| Zinc finger CCCH-type containing 12A (ZC3H12A) | CD, UC | 83,84 | 49 |
| Chitinase 3-like 1 cartilage glycoprotein-39 (CHI3L1) | CD, UC | 85, 86 | 50 |
| Fc fragment of IgG, low affinity IIIa, receptor CD16a (FCGR3A) | CD, UC | 87, 88 | 51 |
| Sterile alpha motif domain containing 9-like (SAMD9L) | CD, UC | 89,90 | 52 |
| Matrix metalloproteinase 9 gelatinase B, 92kDa gelatinase, 92kDa type IV collagenase (MMP9) | CD | 91, 92 | 53 |
| Matrix metalloproteinase 7 matrilysin, uterine (MMP7) | CD, UC | 93,94 | 54 |
| B-factor, properdin (BF) | CD, UC | 95, 96 | 55 |
| S100 calcium binding protein P (S100P) | CD, UC | 97, 98 | 56 |
| Growth regulated protein (GRO) | CD, UC | 99, 100 | 57 |
| Indoleamine-pyrrole 2,3 dioxygenase INDO (INDO) | CD, UC | 101, 102 | 58 |
| Tripartite motif-containing 22 (TRIM22) | CD, UC | 103, 104 | 59 |
| Serum amyloid A2 (SAA2) | CD, UC | 105, 106 | 60 |
| Arrestin domain containing 5 (ARRDC5) (LOC342959) | CD, UC | 193, 194 | 110 |
| ataxin 3-like (ATXN3L) (A_24_P910246) | CD, UC | 195 | 111 |
| follicle stimulating hormone receptor (FSHR) (LOC92552) | CD, UC | 196, 197 | 112 |
| platelet-derived growth factor receptor, alpha polypeptide (PDGFRA) | CD, UC | 198, 199 | 113 |
| transforming growth factor beta 3 (TGFB3) | CD, UC | 200,201 | 114 |
| potassium channel tetramerisation domain containing 8 (KCTD8) | CD, UC | 202, 203 | 115 |
| transglutaminase 4 (TGM4) | CD, UC | 204, 205 | 116 |
| TPD52L3 tumor protein D52-like 3 (NYD-SP25) | CD, UC | 206, 207 | 117 |
| misc_RNA (C3orf53) (FLJ33651) | CD, UC | 208 | 118 |
| EMX2 opposite strand (non-protein coding) (EMX2OS) on chromosome 10 | CD, UC | 209 | 119A |
| S100A8 | UC | 231, 232 | 130 |
| CCL20 | UC | 233, 234 | 131 |

Table 1B provides a list of genes that are downregulated in IBD.

| Gene | Indication(s) | SEQ ID NO nucleic acid, amino acid | Figure |
|---|---|---|---|
| Sialidase 4 (NFU4) | CD, UC | 107, 108 | 61 |
| wingless-type MMTV integration site family, | CD, UC | 210,211 | 120 |
| member 16 (WNT16) | | | |
| sprouty-related, EVH1 domain containing 2 (SPRED2) | CD, UC | 212, 213 | 121 |
| chromosome 16 open reading frame 65 (C16orf65) (MGC50721) | CD, UC | 214, 215 | 122 |
| chromosome 12 open reading frame 2 (C12orf2) | CD, UC | 216, 217 | 123 |
| multiple PDZ domain protein (MPDZ) | CD, UC | 218, 219 | 124 |
| phenylalanine-tRNA syrithetase 2 (FARS2) | CD, UC | 220, 221 | 125 |
| caspase 8, apoptosis-related cysteine protease (CASP8) | CD, UC | 222, 223 | 126 |
| 5'-nucleotidase, ecto (CD73)(NT5E) | CD, UC | 224, 225 | 127 |
| teratocarcinoma-derived growth factor 3 (TDGF3) | CD, UC | 226 | 128 |
| butyrophilin-like 3 (BTNL3) | CD, UC | 227, 228 | 129 |

Table 2 provides a list of genes that are upregulated in IBD. These genes were identified from the immune response in silico (IRIS) collection of genes (Abbas, A. et al. Genes and Immunity, 6:319-331 (2005) hereby incorporated by reference in its entirety).

**Table 2**

| Gene | Indication(s) | SEQ ID NO nucleic acid, amino acid | Figure |
|---|---|---|---|
| Immunoglobulin domain IRTA2/FCRH5 | CD, UC | 109, 110 | 62 |
| Immunoglobulin lambda joining-constant/OR18 (IGLJCOR18) | CD, UC | 111, 112 | 63 |
| Immunoglobulin heavy variable 4-4 (IGHV4-4) | CD, UC | 113, 114 | 64 |
| Matrix metalloproteinase 9 gelatinase B, 92kDa gelatinase, 92kDa type IV collagenase (MMP9) | CD, UC | 115,116 | 65 |
| Growth regulated protein (GRO) | CD, UC | 117, 118 | 66 |
| Growth regulated protein gamma (MIP2BGRO-g) | CD, UC | 119, 120 | 67 |
| interleukin 1, beta (IL1B) | CD, UC | 121, 122 | 68 |
| IL-3 receptor, alpha low affinity (IL3RA) | CD, UC | 123, 124 | 69 |
| Caspase 1, apoptosis-related cysteine protease | CD, UC | 125, 126 | 70 |
| interleukin 1, beta, convertase (CASP1) | | | |
| Bv8 protein (BV8) | CD, UC | 127, 128 | 71 |

Table 3A provides a list of genes that are upregulated in IBD and were identified based on the experiments described in Example 2. In some instances, the locus/chromosome of the gene is provided.

**Table 3A**

| Gene | Indication(s) | Locus/ Chromosome (if known) | SEQ ID NO nucleic acid, amino acid | Figure |
|---|---|---|---|---|
| HDAC7A | UC | IBD2/12 | 129, 130 | 72 |
| ACVRL1 | UC | IBD2/12 | 131, 132 | 73 |
| NR4A1 | UC | IBD2/12 | 133, 134 | 74 |
| K5B | UC | IBD2/12 | 135, 136 | 75 |
| SILV | UC | IBD2/12 | 137, 138 | 76 |
| IRAK3 | UC | IBD2/12 | 139, 140 | 77 |
| IL-4 | UC | IBD5/5 | 141, 142 | 78 |
| IL-13 | UC | IBD5/5 | 143, 144 | 79 |
| RAD50 | UC | IBD5/5 | 145, 146 | 80 |
| IL-5 | UC | IBD5/5 | 147, 148 | 81 |
| IRF1 | UC | IBD5/5 | 149, 150 | 82 |
| PDLIM4 | UC | IBD5/5 | 151,152 | 83 |
| CSF2 | UC | IBD5/5 | 153, 154 | 84 |
| IL-3 | UC | IBD5/5 | 155, 156 | 85 |
| MMP3 | UC | | 157, 158 | 86 |
| IL-8 | UC | | 159, 160 | 87 |
| TLR4 | UC | | 161, 162 | 88 |
| HLA-DRB1 | UC | | 163, 164 | 89 |
| MMP19 | UC | | 165, 166 | 90 |
| TIMP1 | UC | | 167, 168 | 91 |
| Elfin | UC | | 169, 170 | 92 |
| CXCL1 | UC | | 171,172 | 93 |

Table 3B provides a list of genes that are down-regulated in IBD and were identified based on the experiments described in Example 2. In some instances, the locus/chromosome of the gene is provided.

**Table 3B**

| Gene | Indication(s) | Locus/ Chromosome | SEQ ID NO nucleic acid, amino acid | Figure |
|---|---|---|---|---|
| DFKZP586A0522 | UC | IBD2/12 | 173, 174 | 94 |
| SLC39A5 | UC | IBD2/12 | 175, 176 | 95 |
| GLI-1 | UC | IBD2/12 | 177, 178 | 96 |
| HMGA2 | UC | IBD2/12 | 179, 180 | 97 |
| SLC22A5 | UC | IBD5/5 | 181, 182 | 98 |
| SLC22A4 | UC | IBD5/5 | 183, 184 | 99 |
| P4HA2 | UC | IBD5/5 | 185, 186 | 100 |
| TSLP | UC | | 187, 188 | 101 |
| tubulin alpha 5/alpha 3 | UC | | 189, 190 | 102 |
| tubulin alpha 6 | UC | | 191, 192 | 103 |

### a. Biomarkers of the Invention

The present invention provides numerous gene expression markers or biomarkers for IBD listed in Tables 1A, 1B, 2, 3A, and 3B. In one embodiment of the present invention, the biomarkers are suitable for use in a panel of markers (as described herein). Such panels may include one or more markers from Table 1A; one or more markers from Table 1B; the marker from Table 2; one or markers from Table 3A; and one or more markers from Table 3B. In addition, the present invention also contemplates panels of markers selected from two or more of Tables 1A, 1B, 2, 3A, and 3B. For example, a panel might contain one or more markers from Table 1A and one or more markers from Table 1B; or one or more markers from Table 1A and the marker from Table 2; or one or more markers from Table 1 B and the marker from Table 2; or one or more markers from Table 1A and one or more markers from Table 3A, etc. Those of ordinary skill in the art will appreciate the various combinations of biomarkers from Tables 1-3 that are suitable for use in the panels described herein.

In one embodiment of the present invention, a preferred set of IBD markers identified by microarray analysis, includes markers that are upregulated in an IBD. Preferably, the set of upregulated markers includes DEFA5 (SEQ ID NOS:3-4), DEFA6(SEQ ID NO:1-2), TNIP3(SEQ ID NO:81-82), REG3-gamma(SEQ ID NO:9-10), MMP7(SEQ ID NO:93-94), and SAA1(SEQ ID NO:71-72) in Table 1A; and IL-8(SEQ ID NO:159-160), Keratin 5B (K5B)(SEQ ID NO:135-136), SLC22A4(SEQ ID NO:183-184), SLC22A5(SEQ ID NO:181-182), MMP3(SEQ ID NO:157-158), and MMP19(SEQ ID NO:165-166) in Tables 3A. A preferred downregulated marker is GLI-1 (SEQ ID NO: 175-176) in in Table 3B. A panel of biomarkers as described herein may include one of, more than one of, or all of these markers. Alternatively, the set of markers include 1, 2, 3, 4, 5, 6 of the indicated markers from Table 1A, and/or 1, 2, 3, 4, 5, 6 of the indicated markers from Table 3A and/or 1 or 2 of the indicated markers in Table 3B.

Members of lists provided above, as single markers or in any combination, are preferred for use in prognostic and diagnostic assays of the present invention. The IBD markers of the present invention are differentially expressed genes or regions of genes. A differential level of expression of one or more markers in a test sample from a mammalian subject relative to a control can determined from the level of RNA transcripts or expression products detected by one or more of the methods described in further detail below.

Based on evidence of differential expression of RNA transcripts in normal cells and cells from a mammalian subject having IBD, the present invention provides gene markers for IBD. The IBD markers and associated information provided by the present invention allow physicians to make more intelligent treatment decisions, and to customize the treatment of IBD to the needs of individual patients, thereby maximizing the benefit of treatment and minimizing the exposure of patients to unnecessary treatments, which do not provide any significant benefits and often carry serious risks due to toxic side-effects.

Multi-analyte gene expression tests can measure the expression level of one or more genes involved in each of several relevant physiologic processes or component cellular characteristics. In some instances the predictive power of the test, and therefore its utility, can be improved by using the expression values obtained for individual genes to calculate a score which is more highly correlated with outcome than is the expression value of the individual genes. For example, the calculation of a quantitative score (recurrence score) that predicts the likelihood of recurrence in estrogen receptor-positive, node-negative breast cancer is describe in U.S. Published Patent Application No. 20050048542. The equation used to calculate such a recurrence score may group genes in order to maximize the predictive value of the recurrence score. The grouping of genes may be performed at least in part based on knowledge of their contribution to physiologic functions or component cellular characteristics such as discussed above. The formation of groups, in addition, can facilitate the mathematical weighting of the contribution of various expression values to the recurrence score. The weighting of a gene group representing a physiological process or component cellular characteristic can reflect the contribution of that process or characteristic to the pathology of the IBD and clinical outcome. Accordingly, in an important aspect, the present invention also provides specific groups of the genes identified herein, that together are more reliable and powerful predictors of outcome than the individual genes or random combinations of the genes identified.

In addition, based on the determination of a recurrence score, one can choose to partition patients into subgroups at any particular value(s) of the recurrence score, where all patients with values in a given range can be classified as belonging to a particular risk group. Thus, the values chosen will define subgroups of patients with respectively greater or lesser risk.

The utility of a gene marker in predicting the development or progression of an IBD may not be unique to that marker. An alternative marker having a expression pattern that is closely similar to a particular test marker may be substituted for or used in addition to a test marker and have little impact on the overall predictive utility of the test. The closely similar expression patterns of two genes may result from involvement of both genes in a particular process and/or being under common regulatory control. The present invention specifically includes and contemplates the use of such substitute genes or gene sets in the methods of the present invention.

The markers and associated information provided by the present invention predicting the development and/or progression of an IBD also have utility in screening patients for inclusion in clinical trials that test the efficacy of drug compounds for the treatment of patients with IBD.

The markers and associated information provided by the present invention predicting the presence, development and/or progression of an IBD are useful as criterion for determing whether IBD treatment is appropriate. For example, IBD treatment may be appropriate where the results of the test indicate that an IBD marker is differentially expressed in a test sample from an individual relative to a control sample. The individual may be an individual not known to have an IBD, an individual known to have an IBD, an individual previously diagnosed with an IBD undergoing treatment for the IBD, or an individual previously diagnosed with an IBD and having had surgery to address the IBD. In addition, the present invention contemplates methods of treating an IBD. As described below, the diagnostic methods of the present invention may further comprise the step of administering an IBD therapeutic agent to the mammalian subject that provided the test sample in which the differential expression of one or more IBD markers was observed relative to a control. Such methods of treatment would therefore comprise (a) determining the presence of an IBD in a mammalian subject, and (b) administering an IBD therapeutic agent to the mammalian subject.

In another embodiment, the IBD markers and associated information are used to design or produce a reagent that modulates the level or activity of the gene's transcript or its expression product. Said reagents may include but are not limited to an antisense RNA, a small inhibitory RNA (siRNA), a ribozyme, a monoclonal or polyclonal antibody. In a further embodiment, said gene or its transcript, or more particularly, an expression product of said transcript is used in an (screening) assay to identify a drug compound, wherein said drug compounds is used in the development of a drug to treat an IBD.

In various embodiments of the inventions, various technological approaches described below are available for determination of expression levels of the disclosed genes. In particular embodiments, the expression level of each gene may be determined in relation to various features of the expression products of the gene including exons, introns, protein epitopes and protein activity. In other embodiments, the expression level of a gene may be inferred from analysis of the structure of the gene, for example from the analysis of the methylation pattern of gene's promoter(s).

### b. Diagnostic Methods of the Invention

The present invention provides methods of detecting or diagnosing an IBD in a mammalian subject based on differential expression of an IBD marker. In a one embodiment, the methods comprise the use of a panel of IBD markers as discussed above. The panels may include one or more IBD markers selected from Tables 1-3.

In some embodiments, the panel of IBD markers will include at least 1 IBD marker, at least two IBD markers, at least three IBD markers, at least 4 IBD markers, at least five IBD markers, at least 6 IBD markers, at least 7 IBD marker, at least 8 IBD markers, at least 9 IBD markers, at least 10 IBD markers, at least 11 IBD markers, at least 12 IBD markers, at least 13 IBD markers, at least 14 IBD markers, at least 15 IBD markers, at least 16 IBD markers, at least 17 IBD markers, at least 18 IBD markers, at least 19 IBD markers, or at least 20 IBD markers. In one embodiment, the panel includes markers in increments of five. In another embodiment, the panel includes markers in increments of ten. The panel may include an IBD marker that is overexpressed in IBD relative to a control, an IBD marker that is underexpressed in IBD relative to a control, or IBD markers that are both overexpressed and underexpressed in IBD relative to a control. In a preferred embodiment, the panel includes one or more markers that are upregulated in CD and one or more markers that are downregulated in CD. In another preferred embodiment, the panel includes one or more markers that are upregulated in UC and one or more markers that are downregulated in UC.

In another embodiment, the panels of the present invention may include an IBD marker that is overexpressed in an active IBD relative to a control, underexpressed in an active IBD relative to a control, or IBD markers that are both overexpressed and underexpressed in an active IBD relative to a control. In another embodiment, the panels of the present invention may include an IBD marker that is overexpressed in an inactive IBD relative to a control, underexpressed in an inactive IBD relative to a control, or IBD markers that are both overexpressed and underexpressed in an inactive IBD relative to a control. In a preferred embodiment, the active IBD is CD. In another preferred embodiment, the inactive IBD is CD.

In a preferred embodiment, the methods of diagnosing or detecting the presence of an IBD in a mammalian subject comprise determining a differential expression level of RNA transcripts or expression products thereof from a panel of IBD markers in a test sample obtained from the subject relative to the level of expression in a control, wherein the differential level of expression is indicative of the presence of an IBD in the subject from which the test sample was obtained. The differential expression in the test sample may be higher and/or lower relative to a control as discussed herein.

Differential expression or activity of one or more of the genes provided in the lists above, or the corresponding RNA molecules or encoded proteins in a biological sample obtained from the patient, relative to control, indicates the presence of an IBD in the patient. The control can, for example, be a gene, present in the same cell, which is known to be up-regulated (or down-regulated) in an IBD patient (positive control). Alternatively, or in addition, the control can be the expression level of the same gene in a normal cell of the same cell type (negative control). Expression levels can also be normalized, for example, to the expression levels of housekeeping genes, such as glyceraldehyde-3-phosphate-dehydrogenase (GAPDH) and/or β-actin, or to the expression levels of all genes in the sample tested. In one embodiment, expression of one or more of the above noted genes is deemed positive expression if it is at the median or above, *e.g*. compared to other samples of the same type. The median expression level can be determined essentially contemporaneously with measuring gene expression, or may have been determined previously. These and other methods are well known in the art, and are apparent to those skilled in the art.

Methods for identifying IBD patients are provided herein. Of this patient population, patients with an IBD can be identified by determining the expression level of one or more of the genes, the corresponding RNA molecules or encoded proteins in a biological sample comprising cells obtained from the patient. The biological sample can, for example, be a tissue biopsy as described herein.

The methods of the present invention concern IBD diagnostic assays, and imaging methodologies. In one embodiment, the assays are performed using antibodies as described herein. The invention also provides various immunological assays useful for the detection and quantification of proteins. These assays are performed within various immunological assay formats well known in the art, including but not limited to various types of radioimmunoassays, enzyme-linked immunosorbent assays (ELISA), enzyme-linked immunofluorescent assays (ELIFA), and the like. In addition, immunological imaging methods capable of detecting an IBD characterized by expression of a molecule described herein are also provided by the invention, including but not limited to radioscintigraphic imaging methods using labeled antibodies. Such assays are clinically useful in the detection, monitoring, diagnosis and prognosis of IBD characterized by expression of one or more molecules described herein.

Another aspect of the present invention relates to methods for identifying a cell that expresses a molecule described herein. The expression profile of a molecule(s) described herein make it a diagnostic marker for IBD. Accordingly, the status of the expression of the molecule(s) provides information useful for predicting a variety of factors including susceptibility to advanced stages of disease, rate of progression, and/or sudden and severe onset of symptoms in an active IBD or an inactive IBD, i.e. flare-ups.

In one embodiment, the present invention provides methods of detecting an IBD. A test sample from a mammalian subject and a control sample from a known normal mammal are each contacted with an anti-IBD marker antibody or a fragment thereof. The level of IBD marker expression is measured and a differential level of expression in the test sample relative to the control sample is indicative of an IBD in the mammalian subject from which the test sample was obtained. In some embodiments, the level of IBD marker expression in the test sample is determined to be higher than the level of expression in the control, wherein the higher level of expression indicates the presence of an IBD in the subject from which the test sample was obtained. In another embodiments, the level of IBD marker expression in the test sample is determined to be lower than the level of expression in the control, wherein the lower level of expression indicates the presence of an IBD in the subject from which the test sample was obtained.

In another embodiment, the IBD detected by the methods of the present invention is the recurrence or flareup of an IBD in the mammalian subject.

In preferred embodiments, the methods are employed to detect the flare-up of an IBD or a recurrence of an IBD in a mammalian subject previously determined to have an IBD who underwent treatment for the IBD, such as drug therapy or a surgical procedure. Following initial detection of an IBD, additional test samples may be obtained from the mammalian subject found to have an IBD. The additional sample may be obtained hours, days, weeks, or months after the initial sample was taken. Those of skill in the art will appreciate the appropriate schedule for obtaining such additional samples, which may include second, third, fourth, fifth, sixth, etc. test samples. The intial test sample and the additional sample (and alternately a control sample as described herein) are contacted with an anti-IBD marker antibody. The level of IBD marker expression is measured and a differential level of expression in the additional test sample as compared to the initial test sample is indicative of a flare-up in or a recurrence of an IBD in the mammalian subject from which the test sample was obtained.

In one aspect, the methods of the present invention are directed to a determining step. In one embodiment, the determining step comprises measuring the level of expression of one or more IBD markers in a test sample relative to a control. Typically, measuring the level of IBD marker expression, as described herein, involves analyzing a test sample for differential expression of an IBD marker relative to a control by performing one or more of the techniques described herein. The expression level data obtained from a test sample and a control are compared for differential levels of expression. In another embodiment, the determining step further comprises an examination of the test sample and control expression data to assess whether an IBD is present in the subject from which the test sample was obtained.

In some embodiments, the determining step comprises the use of a software program executed by a suitable processor for the purpose of (i) measuring the differential level of IBD marker expression in a test sample and a control; and/or (ii) analyzing the data obtained from measuring differential level of IBD marker expression in a test sample and a control. Suitable software and processors are well known in the art and are commercially available. The program may be embodied in software stored on a tangible medium such as CD-ROM, a floppy disk, a hard drive, a DVD, or a memory associated with the processor, but persons of ordinary skill in the art will readily appreciate that the entire program or parts thereof could alternatively be executed by a device other than a processor, and/or embodied in firmware and/or dedicated hardware in a well known manner.

Following the determining step, the measurement results, findings, diagnoses, predictions and/or treatment recommendations are typically recorded and communicated to technicians, physicians and/or patients, for example. In certain embodiments, computers will be used to communicate such information to interested parties, such as, patients and/or the attending physicians. In some embodiments, the assays will be performed or the assay results analyzed in a country or jurisdiction which differs from the country or jurisdiction to which the results or diagnoses are communicated.

In a preferred embodiment, a diagnosis, prediction and/or treatment recommendation based on the level of expression of one or more IBD markers disclosed herein measured in a test subject of having one or more of the IBD markers herein is communicated to the subject as soon as possible after the assay is completed and the diagnosis and/or prediction is generated. The results and/or related information may be communicated to the subject by the subject's treating physician. Alternatively, the results may be communicated directly to a test subject by any means of communication, including writing, electronic forms of communication, such as email, or telephone. Communication may be facilitated by use of a computer, such as in case of email communications. In certain embodiments, the communication containing results of a diagnostic test and/or conclusions drawn from and/or treatment recommendations based on the test, may be generated and delivered automatically to the subject using a combination of computer hardware and software which will be familiar to artisans skilled in telecommunications. One example of a healthcare-oriented communications system is described in U.S. Pat. No. 6,283,761; however, the present invention is not limited to methods which utilize this particular communications system. In certain embodiments of the methods of the invention, all or some of the method steps, including the assaying of samples, diagnosing of diseases, and communicating of assay results or diagnoses, may be carried out in diverse (e.g., foreign) jurisdictions.

The invention provides assays for detecting the differential expression of an IBD marker in tissues associated with the gastrointestinal tract including, without limitation, ascending colon tissue, descending colon tissue, sigmoid colon tissue, and terminal ileum tissue; as well expression in other biological samples such as serum, semen, bone, prostate, urine, cell preparations, and the like. Methods for detecting differential expression of an IBD marker are also well known and include, for example, immunoprecipitation, immunohistochemical analysis, Western blot analysis, molecular binding assays, ELISA, ELIFA and the like. For example, a method of detecting the differential expression of an IBD marker in a biological sample comprises first contacting the sample with an anti-IBD marker antibody, an IBD marker-reactive fragment thereof, or a recombinant protein containing an antigen-binding region of an anit-IBD marker antibody; and then detecting the binding of an IBD marker protein in the sample.

In various embodiments of the inventions, various technological approaches are available for determination of expression levels of the disclosed genes, including, without limitation, RT-PCR, microarrays, serial analysis of gene expression (SAGE) and Gene Expression Analysis by Massively Parallel Signature Sequencing (MPSS), which will be discussed in detail below. In particular embodiments, the expression level of each gene may be determined in relation to various features of the expression products of the gene including exons, introns, protein epitopes and protein activity. In other embodiments, the expression level of a gene may be inferred from analysis of the structure of the gene, for example from the analysis of the methylation pattern of gene's promoter(s).

### c. Therapeutic Methods of the Invention

The present invention provides therapeutic methods of treating an IBD in a subject in need that comprise detecting the presence of an IBD in a mammalian subject by the diagnostic methods described herein and then administering to the mammalian subject an IBD therapeutic agent. Those of ordinary skill in the art will appreciate the various IBD therapeutic agents that may be suitable for use in the present invention (see St Clair Jones, Hospital Pharmacist, May 2006, Vol. 13; pages 161-166, hereby incorporated by reference in its entirety). The present invention contemplates methods of IBD treatment in which one or more IBD therapeutic agents are administered to a subject in need. In one embodiment, the IBD therapeutic agent is one or more of an aminosalicylate, a corticosteroid, and an immunosuppressive agent. In a preferred embodiment, the aminosalicylate is one of sulfasalazine, olsalazine, mesalamine, balsalazide, and asacol. In another preferred embodiment, multiple aminosalicylates are co-administered, such as a combination of sulfasalazine and olsalazine. In other preferred embodiments, the corticosteroid may be budesonide, prednisone, prednisolone, methylprednisolone, 6-mercaptopurine (6-MP), azathioprine, methotrexate, and cyclosporin. In other preferred embodiments, the IBD therapeutic agent may an antibiotic, such as ciprofloxacin and/or metronidazole; or an antibody-based agent such as infliximab (Remicade®).

The least toxic IBD therapeutic agents which patients are typically treated with are the aminosalicylates. Sulfasalazine (Azulfidine), typically administered four times a day, consists of an active molecule of aminosalicylate (5-ASA) which is linked by an azo bond to a sulfapyridine. Anaerobic bacteria in the colon split the azo bond to release active 5-ASA. However, at least 20% of patients cannot tolerate sulfapyridine because it is associated with significant side-effects such as reversible sperm abnormalities, dyspepsia or allergic reactions to the sulpha component. These side effects are reduced in patients taking olsalazine. However, neither sulfasalazine nor olsalazine are effective for the treatment of small bowel inflammation. Other formulations of 5-ASA have been developed which are released in the small intestine (e.g. mesalamine and asacol). Normally it takes 6-8 weeks for 5-ASA therapy to show full efficacy. Patients who do not respond to 5-ASA therapy, or who have a more severe disease, are prescribed corticosteroids. However, this is a short term therapy and cannot be used as a maintenance therapy. Clinical remission is achieved with corticosteroids within 2-4 weeks, however the side effects are significant and include Cushing goldface, facial hair, severe mood swings and sleeplessness. The response to sulfasalazine and 5-aminosalicylate preparations is poor in CD, fair to mild in early ulcerative colitis and poor in severe UC. If these agents fail, powerful immunosuppressive agents such as cyclosporine, prednisone, 6-mercaptopurine or azathioprine (converted in the liver to 6-mercaptopurine) are typically tried. For CD patients, the use of corticosteroids and other immunosuppressives must be carefully monitored because of the high risk of intra-abdominal sepsis originating in the fistulas and abscesses common in this disease. Approximately 25% of IBD patients will require surgery (colectomy) during the course of the disease.

Treatment of an IBD may include a surgical procedure, including without limitation, a bowel resection, anastomosis, a colectomy, a proctocolectomy, and an ostomy, or any combination thereof.

In addition to pharmaceutical medicine and surgery, nonconventional treatments for IBD such as nutritional therapy have also been attempted. For example, Flexical®, a semi-elemental formula, has been shown to be as effective as the steroid prednisolone. Sanderson et al., Arch. Dis. Child. 51:123-7 (1987). However, semi-elemental formulas are relatively expensive and are typically unpalatable-thus their use has been restricted. Nutritional therapy incorporating whole proteins has also been attempted to alleviate the symptoms of IBD. Giafer et al., Lancet 335: 816-9 (1990). U.S. Patent No. 5,461,033 describes the use of acidic casein isolated from bovine milk and TGF-2. Beattie et al., Aliment. Pharmacol. Ther. 8: 1-6 (1994) describes the use of casein in infant formula in children with IBD. U.S.P. 5,952,295 describes the use of casein in an enteric formulation for the treatment of IBD. However, while nutrional therapy is non-toxic, it is a palliative treatment and does not treat the underlying cause of the disease.

The present invention contemplates methods of IBD treatment, including for example, in vitro, ex vivo and in vivo therapeutic methods. The invention provides methods useful for treating an IBD in a subject in need upon the detection of an IBD disease state in the subject associated with the expression of one or more IBD markers disclosed herein, such as increased and/or decreased IBD marker expression. In one preferred embodiment, the method comprises (a) determining that the level of expression of (i) one or more nucleic acids encoding one or more polypeptides selected from Tables 1, 2, or 3; or (ii) RNA transcripts or expression products thereof of one or more genes listed in Tables 1, 2, and 3 in a test sample obtained from said subject is higher and/or lower relative to the level of expression in a control, wherein said higher and/or lower level of expression is indicative of the presence of an IBD in the subject from which the test sample was obtained; and (b) administering to said subject an effective amount of an IBD therapeutic agent. The determining step (a) may comprise the measurement of the expression of multiple IBD marker

The method of treatment comprises detecting the IBD and administering an effective amount of an IBD therapeutic agent to a subject in need of such treatment. In some embodiments, the IBD disease state is associated with an increased and/or decrease in expression of one or more IBD markers.

In one aspect, the invention provides methods for treating or preventing an IBD, the methods comprising detecting the presence of an IBD in a subject and administering an effective amount of an IBD therapeutic agent to the subject. It is understood that any suitable IBD therapeutic agent may be used in the methods of treatment, including aminosalicylates, corticosteroids, and immunosuppressive agents as discussed herein.

In any of the methods herein, one may administer to the subject or patient along with a single IBD therapeutic agent discussed herein an effective amount of a second medicament (where the single IBD therapeutic agent herein is a first medicament), which is another active agent that can treat the condition in the subject that requires treatment. For instance, an aminosalicylate may be co-administered with a corticosteroid, an immunsuppressive agent, or another aminosalicylate. The type of such second medicament depends on various factors, including the type of IBD, its severity, the condition and age of the patient, the type and dose of first medicament employed, etc.

Such treatments using first and second medicaments include combined administration (where the two or more agents are included in the same or separate formulations), and separate administration, in which case, administration of the first medicament can occur prior to, and/or following, administration of the second medicament. In general, such second medicaments may be administered within 48 hours after the first medicaments are administered, or within 24 hours, or within 12 hours, or within 3-12 hours after the first medicament, or may be administered over a pre-selected period of time, which is preferably about 1 to 2 days, about 2 to 3 days, about 3 to 4 days, about 4 to 5 days, about 5 to 6 days, or about 6 to 7 days.

The first and second medicaments can be administered concurrently, sequentially, or alternating with the first and second medicament or upon non-responsiveness with other therapy. Thus, the combined administration of a second medicament includes co-administration (concurrent administration), using separate formulations or a single pharmaceutical formulation, and consecutive administration in either order, wherein preferably there is a time period while both (or all) medicaments simultaneously exert their biological activities. All these second medicaments may be used in combination with each other or by themselves with the first medicament, so that the express "second medicament" as used herein does not mean it is the only medicament besides the first medicament, respectively. Thus, the second medicament need not be one medicament, but may constitute or comprise more than one such drug. These second medicaments as set forth herein are generally used in the same dosages and with administration routes as the first medicaments, or about from 1 to 99% of the dosages of the first medicaments. If such second medicaments are used at all, preferably, they are used in lower amounts than if the first medicament were not present, especially in subsequent dosings beyond the initial dosing with the first medicament, so as to eliminate or reduce side effects caused thereby.

Where the methods of the present invention comprise administering one or more IBD therapeutic agent to treat or prevent an IBD, it may be particularly desirable to combine the administering step with a surgical procedure that is also performed to treat or prevent the IBD. The IBD surgical procedures contemplated by the present invention include, without limitation, a bowel resection, anastomosis, a colectomy, a proctocolectomy, and an ostomy, or any combination thereof. For instance, an IBD therapeutic agent described herein may be combined with a colectomy in a treatment scheme, e.g. in treating an IBD. Such combined therapies include and separate administration, in which case, administration of the IBD therapeutic agent can occur prior to, and/or following, the surgical procedure.

Treatment with a combination of one or more IBD therapeutic agents; or a combination of one or more IBD therapeutic agents and a surgical procedure described herein preferably results in an improvement in the signs or symptoms of an IBD. For instance, such therapy may result in an improvement in the subject receiving the IBD therapeutic agent treatment regimen and a surgical procedure, as evidenced by a reduction in the severity of the pathology of the IBD.

The IBD therapeutic agent(s) is/are administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic.

The IBD therapeutic agent(s) compositions administered according to the methods of the invention will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The first medicament(s) need not be, but is optionally formulated with one or more additional medicament(s) (e.g. second, third, fourth, etc. medicaments) described herein. The effective amount of such additional medicaments depends on the amount of the first medicament present in the formulation, the type of disorder or treatment, and other factors discussed above. These are generally used in the same dosages and with administration routes as used hereinbefore or about from 1 to 99% of the heretofore employed dosages.

For the prevention or treatment of an IBD, the appropriate dosage of an IBD therapeutic agent (when used alone or in combination with other agents) will depend on the type of disease to be treated, the type of IBD therapeutic agent(s), the severity and course of the disease, whether the IBD therapeutic agent is administered for preventive or therapeutic purposes, previous therapy, the patient's clinical history and response to the IBD therapeutic agent, and the discretion of the attending physician. The IBD therapeutic agent is suitably administered to the patient at one time or over a series of treatments. Depending on the type and severity of the disease, about 1 ug/kg to 15 mg/kg (e.g. 0.1 mg/kg-10 mg/kg) of IBD therapeutic agent is an initial candidate dosage for administration to the patient, whether, for example, by one or more separate administrations, or by continuous infusion. One typical daily dosage might range from about 1 ug/kg to 100 mg/kg or more, depending on the factors mentioned above. For repeated administrations over several days or longer, depending on the condition, the treatment is sustained until a desired suppression of disease symptoms occurs. One exemplary dosage of the IBD therapeutic agent would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, e.g. about six doses of the IBD therapeutic agent). An initial higher loading dose, followed by one or more lower doses may be administered. An exemplary dosing regimen comprises administering an initial loading dose of about 4 mg/kg, followed by a weekly maintenance dose of about 2 mg/kg of the IBD therapeutic agent. However, other dosage regimens may be useful. The progress of this therapy is easily monitored by conventional techniques and assays.

### B.2. Gene Expression Profiling

In general, methods of gene expression profiling can be divided into two large groups: methods based on hybridization analysis of polynucleotides, and other methods based on biochemical detection or sequencing of polynucleotides. The most commonly used methods known in the art for the quantification of mRNA expression in a sample include northern blotting and in situ hybridization (Parker & Barnes, Methods in Molecular Biology 106:247-283 (1999)); RNAse protection assays (Hod, Biotechniques 13:852-854 (1992)); and reverse transcription polymerase chain reaction (RT-PCR) (Weis et al., Trends in Genetics 8:263-264 (1992)). Alternatively, antibodies may be employed that can recognize specific duplexes, including DNA duplexes, RNA duplexes, and DNA-RNA hybrid duplexes or DNA-protein duplexes. Various methods for determining expression of mRNA or protein include, but are not limited to, gene expression profiling, polymerase chain reaction (PCR) including quantitative real time PCR (qRT-PCR), microarray analysis that can be performed by commercially available equipment, following manufacturer's protocols, such as by using the Affymetrix GenChip technology, serial analysis of gene expression (SAGE) (Velculescu et al., Science 270:484-487 (1995); and Velculescu et al., Cell 88:243-51 (1997)), MassARRAY, Gene Expression Analysis by Massively Parallel Signature Sequencing (MPSS) (Brenner et al., Nature Biotechnology 18:630-634 (2000)), proteomics, immunohistochemistry (IHC), etc. Preferably mRNA is quantified. Such mRNA analysis is preferably performed using the technique of polymerase chain reaction (PCR), or by microarray analysis. Where PCR is employed, a preferred form of PCR is quantitative real time PCR (qRT-PCR).

### a. Reverse Transcriptase PCR (RT-PCR)

Of the techniques listed above, the most sensitive and most flexible quantitative method is RT-PCR, which can be used to compare mRNA levels in different sample populations, in normal and test sample tissues, to characterize patterns of gene expression, to discriminate between closely related mRNAs, and to analyze RNA structure.

The first step is the isolation of mRNA from a target sample. The starting material is typically total RNA isolated from colonic tissue biopsies. Thus, RNA can be isolated from a variety of tissues, including without limitation, the terminal ileum, the ascending colon, the descending colon, and the sigmoid colon. In addition, the colonic tissue from which a biopsy is obtained may be from an inflamed and/or a non-inflamed colonic area.

In one embodiment, the mRNA is obtained from a biopsy as defined above wherein the biopsy is obtained from the left colon or from the right colon. As used herein, the "left colon" refers to the sigmoideum and rectosigmoideum and the "right colon" refers to the cecum.

General methods for mRNA extraction are well known in the art and are disclosed in standard textbooks of molecular biology, including Ausubel et al., Current Protocols of Molecular Biology, John Wiley and Sons (1997). In particular, RNA isolation can be performed using purification kit, buffer set and protease from commercial manufacturers, such as Qiagen, according to the manufacturer's instructions. Total RNA from tissue samples can be isolated using RNA Stat-60 (Tel-Test). RNA prepared from a biopsy can be isolated, for example, by cesium chloride density gradient centrifugation.

As RNA cannot serve as a template for PCR, the first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700TM Sequence Detection SystemTM (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700TM Sequence Detection SystemTM. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Ct).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin.

A more recent variation of the RT-PCR technique is the real time quantitative PCR, which measures PCR product accumulation through a dual-labeled fluorigenic probe (i.e., TaqMan® probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

According to one aspect of the present invention, PCR primers and probes are designed based upon intron sequences present in the gene to be amplified. In this embodiment, the first step in the primer/probe design is the delineation of intron sequences within the genes. This can be done by publicly available software, such as the DNA BLAT software developed by Kent, W.J., Genome Res. 12(4):656-64 (2002), or by the BLAST software including its variations. Subsequent steps follow well established methods of PCR primer and probe design.

In order to avoid non-specific signals, it is important to mask repetitive sequences within the introns when designing the primers and probes. This can be easily accomplished by using the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386).

The most important factors considered in PCR primer design include primer length, melting temperature (Tm), and G/C content, specificity, complementary primer sequences, and 3'-end sequence. In general, optimal PCR primers are generally 17-30 bases in length, and contain about 20-80%, such as, for example, about 50-60% G+C bases. Tm's between 50 and 80 °C, e.g. about 50 to 70 °C are typically preferred.

For further guidelines for PCR primer and probe design see, e.g. Dieffenbach, C.W. et al., "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997), the entire disclosures of which are hereby expressly incorporated by reference.

Further PCR-based techniques include, for example, differential display (Liang and Pardee, Science 257:967-971 (1992)); amplified fragment length polymorphism (iAFLP) (Kawamoto et al., Genome Res. 12:1305-1312 (1999)); BeadArray™ technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex100 LabMAP system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003)).

### b. Microarrays

Differential gene expression can also be identified, or confirmed using the microarray technique. Thus, the expression profile of IBD-associated genes can be measured in either fresh or paraffin-embedded tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from biopsy tissue or cell lines derived from cells obtained from a subject having an IBD, and corresponding normal tissues or cell lines. Thus RNA can be isolated from a variety of colonic tissues or colonic tissue-based cell lines.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Incyte's microarray technology, or Agilent's Whole Human Genome microarray technology.

### c. Serial Analysis of Gene Expression (SAGE)

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

### d. MassARRAY Technology

In the MassARRAY-based gene expression profiling method, developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### e. Gene Expression Analysis by Massively Parallel Signature Sequencing (MPSS)

This method, described by Brenner et al., Nature Biotechnology 18:630-634 (2000), is a sequencing approach that combines non-gel-based signature sequencing with *in vitro* cloning of millions of templates on separate 5 µm diameter microbeads. First, a microbead library of DNA templates is constructed by *in vitro* cloning. This is followed by the assembly of a planar array of the template-containing microbeads in a flow cell at a high density (typically greater than 3 x 10⁶ microbeads/cm²). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

The steps of a representative protocol for profiling gene expression using fixed, paraffin-embedded tissues as the RNA source, including mRNA isolation, purification, primer extension and amplification are given in various published journal articles (for example: Godfrey et al. J. Molec. Diagnostics 2: 84-91 (2000); Specht et al., Am. J. Pathol. 158: 419-29 (2001)). Briefly, a representative process starts with cutting about 10 microgram thick sections of paraffin-embedded tissue samples. The mRNA is then extracted, and protein and DNA are removed. General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tissues can be isolated, for example, by cesium chloride density gradient centrifugation. After analysis of the RNA concentration, RNA repair and/or amplification steps may be included, if necessary, and RNA is reverse transcribed using gene specific promoters followed by PCR. Peferably, real time PCR is used, which is compatible both with quantitative competitive PCR, where internal competitor for each target sequence is used for normalization, and with quantitative comparative PCR using a normalization gene contained within the sample, or a housekeeping gene for RT-PCR. For further details see, e.g. "PCR: The Polymerase Chain Reaction", Mullis et al., eds., 1994; and Held et al., Genome Research 6:986-994 (1996). Finally, the data are analyzed to identify the best treatment option(s) available to the patient on the basis of the characteristic gene expression pattern identified in the sample examined.

### f. Immunohistochemistry

Immunohistochemistry methods are also suitable for detecting the expression levels of the IBD markers of the present invention. Thus, antibodies or antisera, preferably polyclonal antisera, and most preferably monoclonal antibodies specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

Expression levels can also be determined at the protein level, for example, using various types of immunoassays or proteomics techniques.

In immunoassays, the target diagnostic protein marker is detected by using an antibody specifically binding to the markes. The antibody typically will be labeled with a detectable moiety. Numerous labels are available which can be generally grouped into the following categories:
Radioisotopes, such as 35S, 14C, 125I, 3H, and 131I. The antibody can be labeled with the radioisotope using the techniques described in Current Protocols in Immunology, Volumes 1 and 2, Coligen et al. (1991) Ed. Wiley-Interscience, New York, New York, Pubs. for example and radioactivity can be measured using scintillation counting.

Fluorescent labels such as rare earth chelates (europium chelates) or fluorescein and its derivatives, rhodamine and its derivatives, dansyl, Lissamine, phycoerythrin and Texas Red are available. The fluorescent labels can be conjugated to the antibody using the techniques disclosed in Current Protocols in Immunology, supra, for example. Fluorescence can be quantified using a fluorimeter.

Various enzyme-substrate labels are available and U.S. Patent No. 4,275,149 provides a review of some of these. The enzyme generally catalyzes a chemical alteration of the chromogenic substrate which can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al. (1981) Methods for the Preparation of Enzyme-Antibody Conjugates for use in Enzyme Immunoassay, in Methods in Enzym. (ed J. Langone & H. Van Vunakis), Academic press, New York 73:147-166.

Examples of enzyme-substrate combinations include, for example: horseradish peroxidase (HRPO) with hydrogen peroxidase as a substrate, wherein the hydrogen peroxidase oxidizes a dye precursor (e.g.,orthophenylene diamine (OPD) or 3,3',5,5'-tetramethyl benzidine hydrochloride (TMB)); alkaline phosphatase (AP) with para-Nitrophenyl phosphate as chromogenic substrate; and β-D-galactosidase (β-D-Gal) with a chromogenic substrate (e.g., p-nitrophenyl-β-D-galactosidase) or fluorogenic substrate 4-methylumbelliferyl-β-D-galactosidase.

Numerous other enzyme-substrate combinations are available to those skilled in the art. For a general review of these, see U.S. Patent Nos. 4,275,149 and 4,318,980.

Sometimes, the label is indirectly conjugated with the antibody. The skilled artisan will be aware of various techniques for achieving this. For example, the antibody can be conjugated with biotin and any of the three broad categories of labels mentioned above can be conjugated with avidin, or vice versa. Biotin binds selectively to avidin and thus, the label can be conjugated with the antibody in this indirect manner. Alternatively, to achieve indirect conjugation of the label with the antibody, the antibody is conjugated with a small hapten (e.g., digoxin) and one of the different types of labels mentioned above is conjugated with an anti-hapten antibody (e.g., anti-digoxin antibody). Thus, indirect conjugation of the label with the antibody can be achieved.

In other versions of immunoassay techniques, the antibody need not be labeled, and the presence thereof can be detected using a labeled antibody which binds to the antibody.

Thus, the diagnostic immunoassays herein may be in any assay format, including, for example, competitive binding assays, direct and indirect sandwich assays, and immunoprecipitation assays. Zola, Monoclonal Antibodies: A Manual of Techniques, pp.147-158 (CRC Press, Inc. 1987).

Competitive binding assays rely on the ability of a labeled standard to compete with the test sample analyze for binding with a limited amount of antibody. The amount of antigen in the test sample is inversely proportional to the amount of standard that becomes bound to the antibodies. To facilitate determining the amount of standard that becomes bound, the antibodies generally are insolubilized before or after the competition, so that the standard and analyze that are bound to the antibodies may conveniently be separated from the standard and analyze which remain unbound.

Sandwich assays involve the use of two antibodies, each capable of binding to a different immunogenic portion, or epitope, of the protein to be detected. In a sandwich assay, the test sample analyze is bound by a first antibody which is immobilized on a solid support, and thereafter a second antibody binds to the analyze, thus forming an insoluble three-part complex. See, e.g.,U.S. Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

### g. Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the markers of the present invention.

### h. 5'-multiplexed Gene Specific Priming of Reverse Transcription

RT-PCR requires reverse transcription of the test RNA population as a first step. The most commonly used primer for reverse transcription is oligo-dT, which works well when RNA is intact. However, this primer will not be effective when RNA is highly fragmented.

The present invention includes the use of gene specific primers, which are roughly 20 bases in length with a Tm optimum between about 58 °C and 60 °C. These primers will also serve as the reverse primers that drive PCR DNA amplification.

An alternative approach is based on the use of random hexamers as primers for cDNA synthesis. However, we have experimentally demonstrated that the method of using a multiplicity of gene-specific primers is superior over the known approach using random hexamers.

### i. Promoter Methylation Analysis

A number of methods for quantization of RNA transcripts (gene expression analysis) or their protein translation products are discussed herein. The expression level of genes may also be inferred from information regarding chromatin structure, such as for example the methylation status of gene promoters and other regulatory elements and the acetylation status of histones.

In particular, the methylation status of a promoter influences the level of expression of the gene regulated by that promoter. Aberrant methylation of particular gene promoters has been implicated in expression regulation, such as for example silencing of tumor suppressor genes. Thus, examination of the methylation status of a gene's promoter can be utilized as a surrogate for direct quantization of RNA levels.

Several approaches for measuring the methylation status of particular DNA elements have been devised, including methylation-specific PCR (Herman J.G, et al. (1996) Methylation-specific PCR: a novel PCR assay for methylation status of CpG islands. Proc. Natl Acad. Sci. USA. 93, 9821-9826.) and bisulfite DNA sequencing (Frommer M. et al. (1992) A genomic sequencing protocol that yields a positive display of 5-methylcytosine residues in individual DNA strands. Proc. Natl Acad. Sci. USA. 89, 1827-1831.). More recently, microarray-based technologies have been used to characterize promoter methylation status (Chen C.M. (2003) Methylation target array for rapid analysis of CpG island hypermethylation in multiple tissue genomes. Am. J. Pathol. 163, 37-45.).

### j. Coexpression of Genes

A further aspect of the invention is the identification of gene expression clusters. Gene expression clusters can be identified by analysis of expression data using statistical analyses known in the art, including pairwise analysis of correlation based on Pearson correlation coefficients (Pearson K. and Lee A. (1902) Biometrika 2, 357).

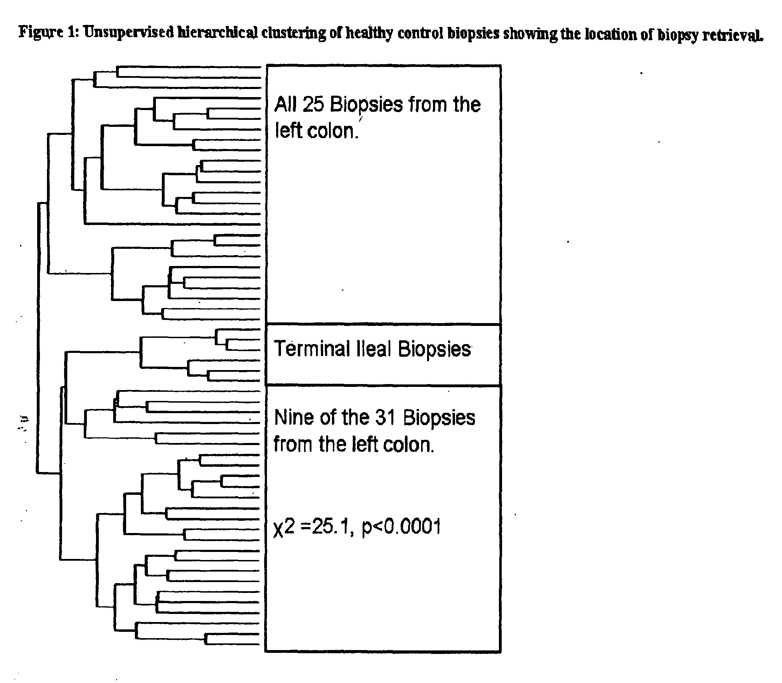

In one embodiment, an expression cluster identified herein includes genes upregulated in the left colon (Fig. 1).

In another embodiment, an expression cluster identified herein includes genes upregulated in the right colon (Fig. 1).

In one other embodiment, an expression cluster identified herein includes genes upregulated in the terminal ileum (Fig. 1).

In other embodiments, the expression cluster identified herein includes genes in the IBD2 locus (Table 7); or in the IBD5 locus (Table 8).

In some embodiments, the expression cluster identified herein includes genes classified under an immune response.

In other embodiments, the expression cluster identified herein includes genes classified under a response to wounding.

### k. Design of Intron-Based PCR Primers and Probes

According to one aspect of the present invention, PCR primers and probes are designed based upon intron sequences present in the gene to be amplified. Accordingly, the first step in the primer/probe design is the delineation of intron sequences within the genes. This can be done by publicly available software, such as the DNA BLAT software developed by Kent, W.J., Genome Res. 12(4):656-64 (2002), or by the BLAST software including its variations. Subsequent steps follow well established methods of PCR primer and probe design.

In order to avoid non-specific signals, it is important to mask repetitive sequences within the introns when designing the primers and probes. This can be easily accomplished by using the Repeat

Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386).

The most important factors considered in PCR primer design include primer length, melting temperature (Tm), and G/C content, specificity, complementary primer sequences, and 3'-end sequence. In general, optimal PCR primers are generally 17-30 bases in length, and contain about 20-80%, such as, for example, about 50-60% G+C bases. Tm's between 50 and 80 °C, e.g. about 50 to 70 °C are typically preferred.

For further guidelines for PCR primer and probe design see, e.g. Dieffenbach, C.W. et al., "General Concepts for PCR Primer Design" in: PCR Primer, A Laboratory Manual, Cold Spring Harbor Laboratory Press, New York, 1995, pp. 133-155; Innis and Gelfand, "Optimization ofPCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997), the entire disclosures of which are hereby expressly incorporated by reference.

### 1. IBD Gene Set, Assayed Gene Subsequences, and Clinical Application of Gene Expression Data

An important aspect of the present invention is to use the measured expression of certain genes by colonic issue to provide diagnostic information. For this purpose it is necessary to correct for (normalize away) both differences in the amount of RNA assayed and variability in the quality of the RNA used. Therefore, the assay typically measures and incorporates the expression of certain normalizing genes, including well known housekeeping genes, such as GAPDH and Cypl. Alternatively, normalization can be based on the mean or median signal (Ct) of all of the assayed genes or a large subset thereof (global normalization approach). On a gene-by-gene basis, measured normalized amount of a patient colonic tissue mRNA is compared to the amount found in an appropriate tissue reference set. The number (N) of tissues in this reference set should be sufficiently high to ensure that different reference sets (as a whole) behave essentially the same way. If this condition is met, the identity of the individual colonic tissues present in a particular set will have no significant impact on the relative amounts of the genes assayed. Usually, the tissue reference set consists of at least about 30, preferably at least about 40 different IBD tissue specimens. Unless noted otherwise, normalized expression levels for each mRNA/tested tissue/patient will be expressed as a percentage of the expression level measured in the reference set. More specifically, the reference set of a sufficiently high number (e.g. 40) of IBD samples yields a distribution of normalized levels of each mRNA species. The level measured in a particular sample to be analyzed falls at some percentile within this range, which can be determined by methods well known in the art. Below, unless noted otherwise, reference to expression levels of a gene assume normalized expression relative to the reference set although this is not always explicitly stated.

### m. Production of antibodies

The present invention further provides anti-IBD marker antibodies. Exemplary antibodies include polyclonal, monoclonal, humanized, bispecific, and heteroconjugate antibodies. As discussed herein, the antibodies may be used in the diagnostic methods for IBD, and in some cases in methods of treatment of IBD.

### (1) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl2, or R1N=C=NR, where R and R1 are different alkyl groups.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (2) Monoclonal antibodies

Various methods for making monoclonal antibodies herein are available in the art. For example, the monoclonal antibodies may be made using the hybridoma method first described by Kohler et al., Nature, 256:495 (1975), by recombinant DNA methods (U.S. Patent No. 4,816,567).

In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional antibody purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as E. coli cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs., 130:151-188 (1992).

In a further embodiment, monoclonal antibodies or antibody fragments can be isolated from antibody phage libraries generated using the techniques described in McCafferly et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy chain and light chain constant domains in place of the homologous murine sequences (U.S. Patent No. 4,816,567; and Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (3) Humanized antibodies

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies. An example of a humanized antibody used to treat IBD is infliximab (Remicade®), an engineered murine-human chimeric monoclonal antibody. The antibody binds the cytokine TNF-alpha and prevents it from binding its receptors to trigger and sustain an inflammatory response. Infliximab is used to treat both CD and UC.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanized antibody (Sims et al., J. Immunol., 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of the humanized antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgG1 antibody.

### (4) Human antibodies

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggermann et al., Year in Immuno., 7:33 (1993); and U.S. Patent Nos. 5,591,669, 5,589,369 and 5,545,807. Alternatively, phage display technology (McCafferty et al., Nature 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats; for their review see, e.g., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patents 5,567,610 and 5,229,275).

### (5) Antibody fragments

Various techniques have been developed for the production of antibody fragments comprising one or more antigen binding regions. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from E. coli and chemically coupled to form F(ab')2 fragments (Carter et al., Bio/Technology 10:163-167 (1992)). According to another approach, F(ab')2 fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; U.S. Patent No. 5,571,894; and U.S. Patent No. 5,587,458. The antibody fragment may also be a Alinear antibody@, *e.g*., as described in U.S. Patent 5,641,870 for example. Such linear antibody fragments may be monospecific or bispecific.

### (6) Bispecific antibodies

Bispecific antibodies are antibodies that have binding specificities for at least two different epitopes. Exemplary bispecific antibodies may bind to two different epitopes of an IBD marker protein. Bispecific antibodies may also be used to localize agents to cells which express an IBD marker protein.

These antibodies possess an IBD marker-binding arm and an arm which binds an agent (e.g. an aminosalicylate). Bispecific antibodies can be prepared as full length antibodies or antibody fragments (e.g. F(ab')2 bispecific antibodies).

Methods for making bispecific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (*e.g*. tyrosine or tryptophan). Compensatory "cavities" of identical or similar size to the large side chain(s) are created on the interface of the second antibody molecule by replacing large amino acid side chains with smaller ones (*e.g*. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then reoxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994). Antibodies with more than two valencies are contemplated. For example, trispecific antibodies can be prepared. Tutt et al. J. Immunol. 147: 60 (1991).

### (7) Other amino acid sequence modifications

Amino acid sequence modification(s) of the antibodies described herein are contemplated. For example, it may be desirable to improve the binding affinity and/or other biological properties of the antibody. Amino acid sequence variants of the antibody are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics. The amino acid changes also may alter post-translational processes of the antibody, such as changing the number or position of glycosylation sites.

A useful method for identification of certain residues or regions of the antibody that are preferred locations for mutagenesis is called "alanine scanning mutagenesis" as described by Cunningham and Wells Science, 244:1081-1085 (1989). Here, a residue or group of target residues are identified (*e.g.,* charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, ala scanning or random mutagenesis is conducted at the target codon or region and the expressed antibody variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include antibody with an N-terminal methionyl residue or the antibody fused to a cytotoxic polypeptide. Other insertional variants of the antibody molecule include the fusion to the N- or C-terminus of the antibody to an enzyme (*e.g*. for ADEPT) or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in Table 1 under the heading of "preferred substitutions". If such substitutions result in a change in biological activity, then more substantial changes, denominated "exemplary substitutions" in the following table, or as further described below in reference to amino acid classes, may be introduced and the products screened.

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)): non-polar: Ala (A), Val (V), Leu (L), Ile (I), Pro (P), Phe (F), Trp (W), Met (M); uncharged polar: Gly (G), Ser (S), Thr (T), Cys (C), Tyr (Y), Asn (N), Gln (Q); acidic: Asp (D), Glu (E); and basic: Lys (K), Arg (R), His(H).

Alternatively, naturally occurring residues may be divided into groups based on common side-chain properties: hydrophobic : Norleucine, Met, Ala, Val, Leu, Ile; neutral hydrophilic: Cys, Ser, Thr, Asn, Gln; acidic: Asp, Glu; basic: His, Lys, Arg; residues that influence chain orientation: Gly, Pro; and aromatic: Trp, Tyr, Phe.

Non-conservative substitutions will entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more hypervariable region residues of a parent antibody (*e.g*. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (*e.g*. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (*e.g*. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and an IBD marker protein. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Engineered antibodies with three or more (preferably four) functional antigen binding sites are also contemplated (U.S. Published Patent Application No. US2002/0004587 A1, Miller et al.).

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

### B.3 Kits of the invention

The materials for use in the methods of the present invention are suited for preparation of kits produced in accordance with well known procedures. The invention thus provides kits comprising agents, which may include gene-specific or gene-selective probes and/or primers, for quantitating the expression of the disclosed genes for IBD. Such kits may optionally contain reagents for the extraction of RNA from samples, in particular fixed paraffin-embedded tissue samples and/or reagents for RNA amplification. In addition, the kits may optionally comprise the reagent(s) with an identifying description or label or instructions relating to their use in the methods of the present invention. The kits may comprise containers (including microtiter plates suitable for use in an automated implementation of the method), each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more probes and primers of the present invention (e.g., appropriate length poly(T) or random primers linked to a promoter reactive with the RNA polymerase).

### B.4 Reports of the invention

The methods of this invention, when practiced for commercial diagnostic purposes generally produce a report or summary of the normalized expression levels of one or more of the selected genes. The methods of this invention will produce a report comprising a prediction of the clinical outcome of a subject diagnosed with an IBD before and after any surgical procedure to treat the IBD. The methods and reports of this invention can further include storing the report in a database. Alternatively, the method can further create a record in a database for the subject and populate the record with data. In one embodiment the report is a paper report, in another embodiment the report is an auditory report, in another embodiment the report is an electronic record. It is contemplated that the report is provided to a physician and/or the patient. The receiving of the report can further include establishing a network connection to a server computer that includes the data and report and requesting the data and report from the server computer.

The methods provided by the present invention may also be automated in whole or in part.

All aspects of the present invention may also be practiced such that a limited number of additional genes that are co-expressed with the disclosed genes, for example as evidenced by high Pearson correlation coefficients, are included in a prognostic or predictive test in addition to and/or in place of disclosed genes.

Having described the invention, the same will be more readily understood through reference to the following Examples, which is provided by way of illustration, and is not intended to limit the invention in any way.

### Examples

### Example 1 - Microarray analysis

Clinically, IBD is characterized by diverse manifestations often resulting in a chronic, unpredictable course. Bloody diarrhea and abdominal pain are often accompanied by fever and weight loss. Anemia is common, as is severe fatigue. Joint manifestations ranging from arthralgia to acute arthritis as well as abnormalities in liver function are commonly associated with IBD. Patients with IBD also have an increased risk of colon carcinomas compared to the general population. During acute "attacks" of IBD, work and other normal activity are usually impossible, and often a patient is hospitalized.

Although the cause of IBD remains unknown, several factors such as genetic, infectious and immunologic susceptibility have been implicated. IBD is much more common in Caucasians, especially those of Jewish descent. The chronic inflammatory nature of the condition has prompted an intense search for a possible infectious cause. Although agents have been found which stimulate acute inflammation, none has been found to cause the chronic inflammation associated with IBD. The hypothesis that IBD is an autoimmune disease is supported by the previously mentioned extraintestinal manifestation of IBD as joint arthritis, and the known positive response to IBD by treatment with therapeutic agents such as adrenal glucocorticoids, cyclosporine and azathioprine, which are known to suppress immune response. In addition, the GI tract, more than any other organ of the body, is continuously exposed to potential antigenic substances such as proteins from food, bacterial byproducts (LPS), etc. The subtypes of IBD are UC and CD.

CD differs from UC in that the inflammation extends through all layers of the intestinal wall and involves mesentery as well as lymph nodes. CD may affect any part of the alimentary canal from mouth to anus. The disease is often discontinuous, i.e., severely diseased segments of bowel are separated from apparently disease-free areas. In CD, the bowel wall also thickens which can lead to obstructions. In addition, fistulas and fissures are not uncommon.

Both UC and CD are typically diagnosed by endoscopy, which shows the afflicted areas. However, the use of microarray technology has shed light on the molecular pathology of UC. A study published in 2000 used microarray technology to analyze eight UC patients. (Dieckgraefe et al., Physiol. Genomics 2000; 4:1-11). 6500 genes were analyzed in this study and the results confirmed increased expression of genes previously implicated in UC pathogenesis, namely IL-1, IL-1RA and IL-8. Endoscopic diagnosis coupled with the ability to take pinch mucosal biopsies have allowed investigators to further diagnose UC using microarray analysis, and allowed investigators to analyze afflicted tissue against normal tissue and to analyze tissue from a larger range of patients with varying degrees of severity. Biopsies of macroscopically unaffected areas of the colon and terminal ileum were microarrayed. (Langman et al., Gastroent. 2004; 127:26-40). Langman analyzed 22,283 genes and found that genes involved in cellular detoxification, such as pregnane X receptor and MDR1 were significantly downregulated in the colon of patients with UC, but there was no change in expression of these genes in CD patients.

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (for example, disease tissue) sample is greater than hybridization signal of a probe from a control, normal tissue sample, the gene or genes overexpressed in the disease tissue are identified. The implication of this result is that an overexpressed protein in a disease tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

Microarray analysis was used to find genes that are overexpressed in CD as compared to normal bowel tissue. For this study, sixty seven patients with CD and thirty-one control patients undergoing colonoscopy were recruited. Patient symptoms were evaluated at the time of colonoscopy using the simple clinical colitis activity index (SCCAI). (Walmsley et al., Gut. 1998; 43:29-32). Quiescent disease showing no histological inflammation was defined as a SCCAI of 2 or less. Active disease with histologially acute or chronic inflammation was defined as a SCCAI of greater than 2. The severity of the CD itself was determined by the criteria of Leonard-Jones. (Lennard-Jones Scand. J. Gastroent. 1989; 170:2-6). The CD patients provided well phenotyped biopsies for analysis of inflammatory pathways of CD at the molecular level, thus identifying novel candidate genes and potential pathways for therapeutic intervention. Paired biopsies were taken from each anatomical location.

All biopsies were stored at -70°C until ready for RNA isolation. The biopsies were homogenized in 600µl of RLT buffer (+ BME) and RNA was isolated using Qiagen™ Rneasy Mini columns (Qiagen ) with on-column DNase treatment following the manufacturer's guidelines. Following RNA isolation, RNA was quantitated using RiboGreen™ (Molecular Probes) following the manufacturer's guidelines and checked on agarose gels for integrity. Appropriate amounts of RNA were labeled for microarray analysis and samples were run on proprietary Genentech microarray and Affymetrics™ microarrays. Genes were compared whose expression was upregulated in UC tissue vs normal bowel, matching biopsies from normal bowel and CD tissue from the same patient. The results of this experiment showed that the nucleic acids as shown in Tables 1A, 1B, and 2A (provided above) are differentially expressed in CD and/or UC tissue in comparision to normal tissue.

The genes listed in Table 1A-1B demonstrated a minimum 1.5 fold difference in expression and also acceptable probe hybridization strength was observed. The genes listed in Table 2 were found to have a minimum within-group Pearson correlation of approximately 0.65 and a three-fold upregulation of gene expression was observed.

More specifically, the SEQ ID NOS listed in Tables 1A and 2 represent polynucleotides and their encoded polypeptide which are significantly up-regulated/overexpressed in CD and/or UC.

The SEQ ID NO listed in Table 1B represents a polynucleotide and its encoded polypeptide which is significantly down-regulated/under-expressed in CD and/or UC.

SEQ ID NOS: listed in Table 3A represent polynucleotides and their encoded polypeptide which are significantly up-regulated/overexpressed in UC.

SEQ ID NOS: listed in Table 3B represent polynucleotides and their encoded polypeptide which are significantly down-regulated/underexpressed in UC.

### Example 2: Characterisation of Distinct Intestinal Gene Expression Profiles in Ulcerative Colitis by Microarray Analysis

Microarray analysis allows a comprehensive picture of gene expression at the cellular level. The aim of this study was to investigate differential intestinal gene expression in patients with ulcerative colitis (UC) and controls.

Methods: 67 UC and 31 control subjects- 23 normal and 8 inflamed non-inflammatory bowel disease patients were studied. Paired endoscopic biopsies were taken from 5 specific anatomical locations for RNA extraction and histology. 41058 expression sequence tags were analyzed in 215 biopsies using the Agilent platform. Confirmation of results was undertaken by real time PCR and immunohistochemistry. Results: In healthy control biopsies, cluster analysis showed differences in gene expression between the right and left colon. (χ²=25.1, p<0.0001). Developmental genes HOXA13, (p=2.3x10⁻¹⁶), HOXB13 (p <1x10⁻⁴⁵), GLI1 (p=4.0x10⁻²⁴), and GLI3 (p=2.1x10⁻²⁸) primarily drove this separation. When all UC biopsies and control biopsies were compared, 143 sequences had a fold change of >1.5 in the UC biopsies (0.01>p>10⁻⁴⁵) and 54 sequences had a fold change of <-1.5 (0.01>p>10⁻²⁰). Differentially upregulated in UC genes included SAA1 (p<10⁻⁴⁵) the alpha defensins, DEFA5&6 (p=0.00003 and p=6.95x10⁻⁷ respectively), MMP3 (p=5.6x10⁻¹⁰) and MMP7 (p=2.3x10⁻⁷). Increased DEFA5&6 expression was further characterized to Paneth cell metaplasia by immunohistochemistry and *in-situ* hybridization. Sub-analysis of the IBD2 & IBD5 loci, and the ABC transporter genes revealed a number of differentially regulated genes in the UC biopsies. Conclusions: These data implicate a number of novel gene families, as well as established candidate genes in the pathogenesis of UC, and may allow characterisation of potential therapeutic targets.

The aim of the current study was to use microarray gene expression analysis to investigate genome wide expression in endoscopic mucosal biopsies of patients with UC and controls. In order to resolve previous inconsistencies and to further delineate inflammatory pathways in UC, substantially more patients and biopsies were included than in previous studies.

### MATERIALS AND METHODS

Patients and Controls. Sixty seven patients with UC and 31 control patients who were undergoing colonoscopy were recruited. Their demographics are shown in Table 4.

**Table 4**

| | ***UC*** |
|---|---|
| Number of patients | 67 |
| Male/ Female | 33/34 |
| Median age at diagnosis (years) | 37 |
| Median duration of follow up (years) | 7.8 |
| **Disease Group** | |
| New Diagnosis (1) | 8 |
| Quiescent disease (2) | 41 |
| Active disease (3) | 18 |
| **Disease extent at time of Endoscopy** | |
| Proctitis | 15 |
| L sided colitis | 27 |
| Extensive colitis | 25 |
| Current Smoker | 6 |
| Family history of IBD | 5 |
| 5 ASA Therapy | 40 |
| Corticosteroid therapy | 10 |
| Immunosuppressant therapy (AZA, 6MP, MTX, MMF) | 11 |

Sixty seven patients with UC and 31 control patients who were undergoing colonoscopy were recruited (Table 4). All UC patients attended the clinic at the Western General Hospital, Edinburgh and the diagnosis of UC adhered to the criteria of Lennard-Jones. (Lennard-Jones JE. Scand J Gastroenterol Suppl 1989;170:2-6) Phenotypic data were collected by interview and case-note review and comprised of demographics, date of diagnosis, disease location, disease behavior, progression, extra-intestinal manifestations, surgical operations, current medication, smoking history, joint symptoms, family history and ethnicity. At the time of colonoscopy patients symptoms were evaluated using the simple clinical colitis activity index (SCCAI). (Walmsley et. al. Gut. 1998;43:29-32)

Patients were recorded as having a 'new diagnosis' of UC if the colonoscopy took place at the time of their index presentation and they had had less than 24 hours of oral/IV therapy. Quiescent disease was defined as a SCCAI of 2 or less and histology showing no inflammation or mild chronic inflammation and active disease was defined as a SCCAI of greater than 2 and histology showing acute or chronic inflammation.

Eleven of the controls were male, 20 were female with a median age of 43 at the time of endoscopy. Six of the controls had normal colonoscopies for colon cancer screening, 9 controls had symptoms consistent with irritable bowel syndrome and had a normal colonoscopic investigation and 7 patients had a colonoscopy for another indication and histologically normal biopsies were obtained. Eight control patients had abnormal inflamed colonic biopsies (1 pseudomembranous colitis, 1 diverticulitis, 1 amoebiasis, 2 microscopic colitis, 1 eosoinophilic infiltrate, 2 scattered lymphoid aggregates and a history of gastroenteritis). Written informed consent was obtained from all patients. Lothian Local Research Ethics Committee approved the study protocol: REC 04/S1103/22.

Biopsy Collection. Anatomical location was confirmed by an experienced operator, distance of endoscope insertion and endoscope configuration using a Scope Guide ^{™}. Paired biopsies were taken from each anatomical location. One biopsy was sent for histological examination and the other was snap frozen in liquid nitrogen for RNA extraction. Each biopsy was graded histologically, by an experienced gastrointestinal pathologist as having no evidence on inflammation, biopsies with evidence of chronic inflammation and predominately chronic inflammatory cell infiltrate or simply those with acute inflammation and an acute inflammatory cell infiltrate. One hundred and thirty nine paired UC biopsies and 76 paired control biopsies were collected. The number of paired biopsies in UC patients and controls from each anatomical location are shown in Table 5.

**Table 5**

| | ***UC (n* =*67)*** | ***Controls (n* =*31)*** |
|---|---|---|
| Total number of paired biopsies | 139 | 76 |
| Terminal Ileum | 4 | 6 |
| Ascending colon | 33 | 17 |
| Descending colon | 35 | 23 |
| Sigmoid colon biopsies. | 57 | 27 |
| Removed from analysis | 10 | 3 |

RNA Isolation. The biopsies weighed between 0.2mg and 16.5 mg with a median weight of 5.5 mg. Total RNA was extracted from each biopsy using the micro total RNA isolation from animal tissues protocol (Qiagen, Valencia, CA), according to the manufacturer's instructions. To evaluate purity and integrity 1 µL of total RNA was assessed each sample with the Agilent technologies 2100 bioanalyzer using the Pico LabChip reagent set (Agilent Technologies, Palo Alto, CA).

**Microarray Analysis.** 1 µg of total RNA was amplified using the Low RNA Input Fluorescent Linear Amplification protocol (Agilent Technologies, Palo Alto, CA). A T7 RNA polymerase single round of linear amplification was carried out to incorporate Cyanine-3 and Cyanine-5 label into cRNA. The cRNA was purified using the RNeasy Mini Kit (Qiagen, Valencia, CA). 1 µl of cRNA was quantified using the NanoDrop ND-1000 spectrophotometer (NanoDrop Technologies, Wilmington, Delaware).

750 ng of Universal Human Reference (Stratagene, La Jolla, CA) cRNA labeled with Cyanine-3 and 750 ng of the test sample cRNA labeled with Cyanine-5 were fragmented for 30 minutes at 60°C before loading onto Agilent Whole Human Genome microarrays (Agilent technologies, Palo Alto, CA). The samples were hybridized for 18 hours at 60°C with constant rotation. Microarrays were washed, dried and scanned on the Agilent scanner according to the manufacturer's protocol (Agilent technologies, Palo Alto, CA). Microarray image files were analyzed using Agilent's Feature Extraction software version 7.5 (Agilent Technologies, Palo Alto, CA). The distribution of log intensities for each sample was plotted and outlier samples (i.e. greater than 2 standard deviations from the mean) were excluded from analysis. 10 UC samples and 3 control samples were designated as outliers using these criteria.

**Real Time PCR.** Confirmation real time PCR analysis was carried out on 8 genes- SAA1, IL8, DEFA5, DEFA6, MMP3, MMP7, S100A8 and TLR4. Ten healthy control sigmoid colon biopsies with normal histology, 9 quiescent UC sigmoid biopsies and 11 UC sigmoid biopsies with an acute (6 biopsies) or chronic (5 biopsies) inflammatory cell infiltrate were selected to represent the different disease groups after stratifying to represent a range of SAA1 and IL-8 expression.

Prior to RTPCR analysis 1 RNA amplification cycle was carried out using the MessageAmp™ II aRNA Amplification Kit protocol (Ambion technologies, Austin, TX). Reverse transcription PCR was then performed on 50ng of RNA using Stratagene model MX4000 (La Jolla, Ca, USA). TaqMan primers and probes were manufactured in house (Genentech Inc, South San Francisco, CA). The sequences for the forward probe, the reverse probe and the TaqMan probe were as follows-
SAA1, forward- agcgatgccagagagaata, reverse- ggaagtgattggggtctttg, Taq- ctttggccatggtgcggagg, [SEQ ID NO:235]

IL-8, forward- actcccagtcttgtcattgc, reverse- caagtttcaaccagcaagaa, Taq-
tgtgttggtagtgctgtgttgaattacgg, [SEQ ID NO:236]

DEFA5, forward- gctacccgtgagtccctct, reverse- tcttgcactgctttggtttc, Taq- tgtgtgaaatcagtggccgcct, [SEQ ID NO:237]

DEFA6, forward- agagctttgggctcaacaag, reverse- atgacagtgcaggtcccata, Taq-
cacttgccattgcagaaggtcctg, [SEQ ID NO:238]

MMP3, forward- aagggaacttgagcgtgaat, reverse- gagtgcttccccttctcttg, Taq- ggcattcaaatgggctgctgc, [SEQ ID NO:239]

MMP7, forward- cacttcgatgaggatgaacg, reverse- gtcccatacccaaagaatgg, Taq-
ctggacggatggtagcagtctaggga, [SEQ ID NO:240]

S100A8, forward- ttgaccgagctggagaaag, reverse- tcaggtcatccctgtagacg, Taq-
tccctgataaaggggaatttccatgc [SEQ ID NO:241]

and
TLR4, forward- agagccgctggtgtatcttt, reverse- ccttctgcaggacaatgaag, Taq- tggcagtttctgagcagtcgtgc [SEQ ID NO:242].

PCR conditions comprised of 48°C for 30 minutes, 95°C hold for 10 minutes, followed by 40 cycles of 30 second 95°C melt and 1 minute 60°C anneal/extend. Absolute quantification of product was calculated by normalizing to RPL19. Results were analyzed using SAS and JMP software (SAS, North Carolina).

*In Situ* Hybridization for Defensin Alpha 5.

PCR primers were designed to amplify a 318 bp fragment of DEFA5 spanning from nt 55-372 of NM_021010 (upper- 5' catcccttgctgccattct [SEQ ID NO:243] and lower- 5' gaccttgaactgaatcttgc [SEQ ID NO:244]). Primers included extensions encoding 27-nucleotide T7 or T3 RNA polymerase initiation sites to allow in vitro transcription of sense or antisense probes, respectively, from the amplified products. Endoscopic biopsies were fixed in 10% neutral buffered formalin and paraffin-embedded. Sections 5 µm thick were deparaffinized, deproteinated in 10 ug/ml Proteinase K (Amresco) for 45 minutes at 37 °C, and further processed for *in situ* hybridization as previously described. (Jubb et. al. Methods Mol Biol. 2006;326:255-264) ³³P-UTP labeled sense and antisense probes were hybridized to the sections at 55°C overnight. Unhybridized probe was removed by incubation in 20µg/ml RNase A for 30 min at 37°C, followed by a high stringency wash at 55°C in 0.1 X SSC for 2 hours and dehydration through graded ethanols. The slides were dipped in NTB nuclear track emulsion (Eastman Kodak), exposed in sealed plastic slide boxes containing desiccant for 4 weeks at 4°C , developed and counterstained with hematoxylin and eosin.

### Immunohistochemistry for Rabbit Anti-Human Lysozyme and Rabbit Anti-Human Defensin Alpha 6

Formalin fixed paraffin embedded tissue sections were rehydrated prior to quenching of endogenous peroxidase activity (KPL, Gathersburg, MD) and blocking of avidin and biotin (Vector, Burlingame, CA). Sections were blocked for 30 minutes with 10% normal goat serum in PBS with 3% BSA. Tissue sections were then incubated with primary antibodies for 60 minutes at room temperature, biotinylated secondary antibodies for 30 min, and incubated in ABC reagent (Vector, Burlingame, CA) for 30 minutes followed by a 5 minute incubation in metal enhanced DAB (Pierce, Rockford, IL). The sections were then counterstained with Mayer's hematoxylin. Primary antibodies used were rabbit anti-human lysozyme at 5.0 µg/ml (Dako, Carpinteria, CA) and rabbit anti-human DEFA6 at 5.0 µg/ml (Alpha Diagnostics, SanAntonio, TX). Secondary antibody used was biotinylated goat anti-rabbit IgG at 7.5 µg/ml (Vector, Burlingame, CA). DEFA6 alpha staining required pre-treatment with Target Retrieval High pH (Dako, Carpenteria,CA) at 99°C for 20 minutes, lysozyme staining did not require pretreatment. All other steps were performed at room temperature.

Data Analysis. Microarray data were analyzed using the Rosetta Resolver software (Rosetta Inpharmatics, Seattle). Statistical significance of the microarray data was determined by Student's unpaired t test. p < 0.01 and a fold change of greater or less than 1.5 were considered statistically significant. Fold change data was calculated using the Rosetta Resolver software. Gene ontology was analyzed using Ingenuity software (Ingenuity Systems, Mountain View, CA). The Mann-Whitney U test was used to analyze the real time PCR data. p < 0.05 was considered significant.

### RESULTS

Influence of anatomical location on gene expression in the healthy colon and terminal ileum. 56 histologically normal biopsies from control patients were analyzed by unsupervised hierarchical clustering. Clear separation by anatomical location was observed on one side of the dendrogram 25/25 biopsies were from the left colon (descending colon or sigmoid colon) where as on the other side of the dendrogram 20/31 biopsies were from the ascending colon (χ² =25.1, p<0.0001) (Figure 1). 6/6 of the terminal ileal biopsies were clustered together. Biopsies from individual patients did not cluster together. The genes driving the differential expression between the right and left colon that were causing the observed clustering were predominately involved in the embryological development of the GI tract- HOXA13, (FC +4.93, p = 2.3x10⁻¹⁶), HOXB13 (FC +16.96, p <1x10⁻⁴⁵), GLI1 (FC +2.2, p = 4.0x10⁻²⁴), and GLI3 (FC +2.3, p = 2.1x10⁻²⁸) were all upregulated in the left colon.

Analysis of expression in UC and control biopsies.

Using unsupervised hierarchical clustering we were unable to differentiate between biopsies from UC patients and controls patients. In addition no clustering based on the inflammation status of the biopsies was observed. The only clustering that was observed was with biopsies from the terminal ileum where both UC and control biopsies clustered together. When all of the UC biopsies (129) and control biopsies (73) were compared, 143 sequences had a fold change of greater than 1.5 in the UC biopsies (0.01 > p > 10⁻⁴⁵) and 54 sequences had a fold change of less than 1.5 (0.01> p > 10⁻²⁰) (data not shown).

Serum amyloid A1 (SAA1) was the most up regulated gene (Fold change (FC) +8.19, p < 10⁻⁴⁵). Other notably upregulated genes were S100A8 (FC +3.50, p = 2.3x10⁻¹⁷), S100A9 (FC +3.06, p = 4.1x10⁻¹³), the alpha defensins, alpha 5 (DEFA5) (FC +3.25, p = 0.00003), alpha 6 (DEFA6) (FC +2.18, p = 6.95x10⁻⁷) and the matrix metalloproteinases MMP3 (FC +2.17, p = 5.6x10⁻¹⁰) and MMP7 (FC +2.29, p = 2.3x10⁻⁷).

A list of the genes found to be differentially expressed in UC patients when compared to normal patients can be found in Tables 1A, 1B, and 2A as described above.

The differential gene expression of a number of candidate genes across more than one experiment is shown in Table 6. Table 6 shows fold changes and p values are shown in a number of different genes in four different experiments. The number of biopsies analyzed in each experiment is shown in brackets. Significant consistent changes in expression across more than one experiment were observed for the genes of interest in this table.

**Table 6**

| ***Genes Analyzed*** | ***All UC (129 biopsies) v controls (73 biopsies) Fold change*** | ***p value*** | ***Non-inflamed UC sigmoid (22) v non- inflamed control sigmoid (18) Fold change*** | ***p value*** | ***Inflamed UC sigmoid (35) v inflamed control sigmoid (8) Fold change*** | ***p value*** | ***Inflamed UC sigmoid (35) v non-inflamed sigmoid UC (22) Fold change*** | ***p value*** |
|---|---|---|---|---|---|---|---|---|
| SAA1 | +8.19 | <10⁻⁴⁵ | +2.0 | 0.00024 | +17.5 | 2.9x10⁻²¹ | +16.51 | <10⁻⁴⁵ |
| Defalpha 5 | +3.25 | 0.00003 | +1.02 | 0.89 | +7.27 | 6.3x10⁻³⁰ | +8.44 | <10⁻⁴⁵ |
| Def alpha 6 | +2.18 | 6.95x10⁻⁷ | -1.09 | 0.34 | +4.41 | 9.7x10⁻⁹ | +6.72 | 4.16x10⁻¹⁹ |
| S100A8 | +3.50 | 2.3x10⁻¹⁷ | +1.21 | 0.19 | +9.75 | 2.4x10⁻²⁴ | +6.84 | 1.16x10⁻¹⁹ |
| S100A9 | +3.06 | 4.1x10⁻¹³ | +1.05 | 0.16 | +7.53 | 6.4x10⁻¹² | +7.11 | 1.96x10⁻³² |
| MMP3 | +2.17 | 5.6x10⁻¹⁰ | -1.55 | 0.0088 | +11.0 | 1.22x10⁻³⁷ | +8.15 | 2.32x10⁻³⁵ |
| MMP7 | +2.29 | 2.3x10⁻⁷ | +1.16 | 0.080 | +7.31 | 4.9x10⁻²⁴ | +5.53 | 1.01x10⁻²³ |
| IL8 | +2.05 | 4.2x10⁻¹¹ | +1.10 | 0.26 | +6.36 | 9.27x10⁻¹⁷ | +7.24 | 8.42x10⁻¹⁹ |
| TLR4 | +1.34 | 4.5x10⁻⁷ | +1.15 | 0.18 | +1.50 | 0.0044 | +1.54 | 0.00073 |
| TNIP3 | +8.02 | 1.1x10⁻¹⁷ | -1.30 | 0.20 | +7.53 | 2.93x10⁻¹³ | +10.5 | 1x10⁻³⁸ |
| CCL20 | +1.30 | 0.00011 | +1.25 | 0.020 | +1.79 | 0.00002 | +2.36 | 4.68x10⁻¹¹ |
| ABCB1 | -1.32 | 0.00091 | +1.10 | 0.40 | -1.82 | 5.6x10⁻⁶ | -1.92 | 9.0x10⁻¹⁰ |
| HLA-DRB1 | +1.03 | 0.88 | -3.0 | 0.0010 | +3.30 | 0.033 | +2.67 | 0.0011 |
| TSLP | -1.12 | 0.31 | -2.73 | 2.7x10⁻¹⁰ | -1.15 | 0.61 | +1.23 | 0.092 |

Gene ontology analysis involving the genes differentially expressed between the UC and control biopsies showed a preponderance of differentially expressed genes were involved in immune response (48 genes out of a total of 679 genes classified under immune response, p = 2.1x10⁻⁹, OR 2.61, CI 1.85-3.56) and response to wounding (30 genes out of a total of 359 genes classified under response to wounding, p = 6.42 x10⁻⁹, OR 3.14, CI 2.09- 4.53) when biological systems were considered.

Analysis of expression in sigmoid colon biopsies in patients with quiescent UC and non-inflamed control biopsies. To compare expression in biopsies without an acute inflammatory signal and to remove the effect of anatomical variation, 22 biopsies from the sigmoid colon with no histological evidence of inflammation from patients with UC were compared to 18 histologically normal control sigmoid colon biopsies. 102 sequences had a fold change greater than 1.5 (0.01 > p > 4.77x10⁻¹³) and 84 sequences had a fold change of less than 1.5 (0.01 > p > 1.8x10⁻²¹) (data not shown).

Upregulated genes included defensin beta 14 (FC +2.11, p = 0.00002) and SAA1 (FC +2.01, p = 0.00024). Interesting genes that were down regulated included HLA-DRB1 (FC -3.0, p = 0.0010) and TSLP (FC -2.73, p = 2.7x10⁻¹⁰) (Table 6).

Analysis of expression in sigmoid colon biopsies in patients with active UC and inflamed control biopsies. Expression of 35 histologically inflamed sigmoid biopsies from patients with UC were compared to 8 histologically inflamed control sigmoid biopsies. Reflecting the more severe inflammation in the UC biopsies a number of genes involved in the acute inflammatory response were upregulated in the UC biopsies v the control biopsies- SAA1 (FC +17.5, p = 2.9x10⁻²¹), MMP3 (FC +11.0, p = 1.22x10⁻³⁷), MMP7 (FC +7.31, p = 4.9x10⁻²⁴) and IL-8 (FC +6.36, p = 9.27x10⁻¹⁷) (Table 6). Overall 623 sequences had a fold change of greater than 1.5 and 509 sequences had a fold change of -1.5 or less (p < 0.01) (data not shown).

Inflamed versus non-inflamed UC sigmoid colon biopsies. When expression signals were compared between 35 histologically inflamed and 22 non- inflamed sigmoid colon UC biopsies 700 sequences had a fold change of greater than 1.5 (0.01 > p > 1x10⁻⁴⁵) and 518 sequences (0.01 > p > 1x10⁻⁴⁵) had a fold change of less than 1.5 in the inflamed biopsies (data not shown).

Notably upregulated genes included SAA1 (FC +16.51, p = <10⁻⁴⁵), TNFAIP3 interacting protein 3 (TNIP3) (FC +10.5, p = 1x10⁻³⁸), DEFA5 (FC +8.44, p = <10⁻⁴⁵), DEFA6 (FC +6.72, p = 4.16x10⁻¹⁹) and regenerating islet-derived 3 gamma (REG3y) (FC +6.99, p = <10⁻⁴⁵).

Analysis of Specific Gene Families- Alpha Defensins 5 and 6.

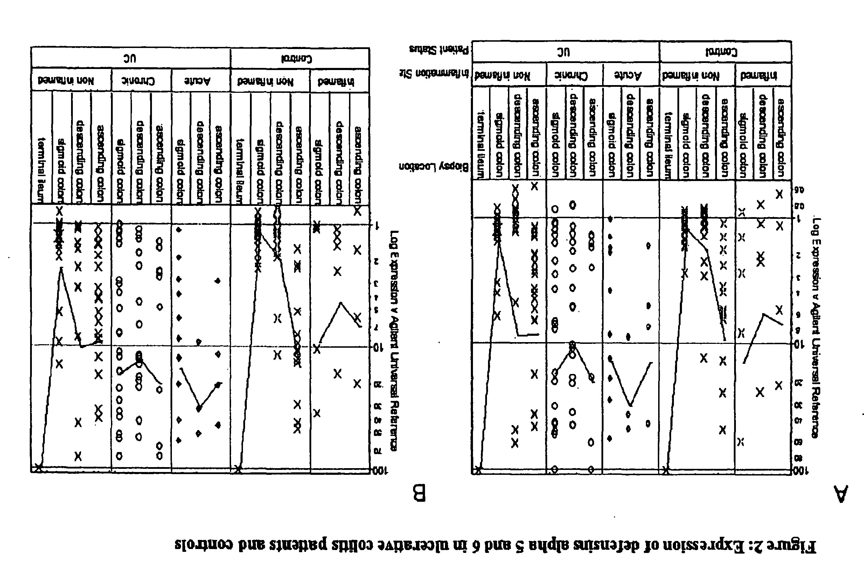

Expression of a number of genes of interest was further analysed, taking into consideration anatomical location and degree of inflammation in the UC samples. When DEFA5 and DEFA6 were analysed expression in the normal controls and the non- inflamed UC biopsies was similar across the different anatomical locations with there being high expression in the terminal ileum, and expression decreasing as the biopsy location became more distal in the colon (Figure 2).

In Figure 2, the expression of each array sample is plotted against the Agilent universal reference. Each endoscopic biopsy has been separated by patient status, biopsy inflammation status and anatomical location. The mean expression levels for each anatomical location are linked in blue. High alpha defensin 5 (panel A) and 6 (B) (DEFA5 and DEFA6) expression levels are seen in the terminal ileum of the controls and the non inflamed UC samples. The expression in these 2 groups decreased the more distally in the colon the biopsies were retrieved from. In the acute and chronically inflamed UC samples and to a lesser extent in the inflamed control samples there was a marked increase in DEFA5 and DEFA6 expression throughout the ascending , descending and sigmoid colon- sigmoid colon inflamed v non-inflamed UC samples (FC +8.44, p = <10⁻⁴⁵) for DEFA5, (FC +6.72, p = 4.16x10⁻¹⁹) for DEFA6.

In the acute and chronically inflamed UC biopsies there was marked upregulation of DEFA5 and DEFA6 expression throughout the ascending, descending and sigmoid colon (Table 6).

Matrix metalloproteinases 3 and 7.

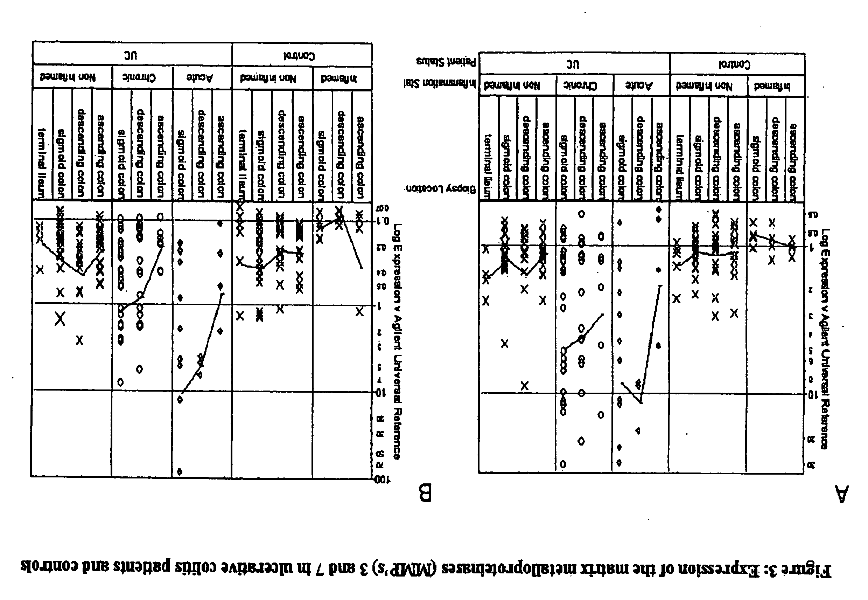

Increased expression of MMP3 and MMP7 was observed in the acutely and chronically inflamed UC biopsies when compared to the non- inflamed UC biopsies- sigmoid colon inflamed v non- inflamed UC samples MMP3 (FC +8.15, p = 2.3x10⁻³⁵) and MMP7 (FC +5.53, p = 1.0x10⁻²³) (Figure 3).

In Figure 3, the expression of each array sample is plotted against the Agilent universal reference. Each endoscopic biopsy has been separated by patient status, biopsy inflammation status and anatomical location. The mean expression levels for each anatomical location are linked in blue. Increased expression of MMP3 (panel A) and MMP7 (B) was observed in the acutely and chronically inflamed UC biopsies when compared to the non inflamed UC biopsies- sigmoid colon inflamed v non inflamed UC samples MMP3 (FC +8.15, p = 2.3x10⁻³⁵) and MMP7 (FC +5.53, p = 1.0x10⁻²³). In contrast when the inflamed and non- inflamed control samples were analysed, a decrease in the expression levels of MMP3 and MMP7 in the inflamed control biopsies was observed (FC -1.62, p = 0.012 and FC -2.0, p = 0.0002 respectively).

ATP- binding cassette (ABC) transporter family and the Xenobiotic- transcription regulators. To further investigate this gene family, expression patterns from probes representing 48 transcriptional genes and their key mediators (PXR, FXR, LXR and CAR) were analysed. When these genes were compared in all the UC and control biopsies, 7 genes were found to significantly down regulated in the UC samples when compared to the control samples- ABCA1 (p= 0.01), ABCA8 (p= 0.0064), ABCB1 (p= 0.0091), ABCC6 (p=0.0050), ABCB7 (p= 0.0068), ABCF1 (p=0.0005) and ABCF2 (p<0.00001). Only one probe representing ABCB2 was significantly upregulated in UC (p= 0.0048).

A number of ABC genes were found to be down-regulated in IBD patients as compared to normal patients (data not shown). The changes observed in ABCB1 expression appeared to be primarily driven by the inflamed UC biopsies which were significantly downregulated when compared to the non-inflamed UC biopsies in the sigmoid colon (FC -1.82, p= 5.6x10⁻⁶)(Table 6). Of interest, no difference in the expression of PXR between UC and controls was observed in any of the analysis including disease location and activity.

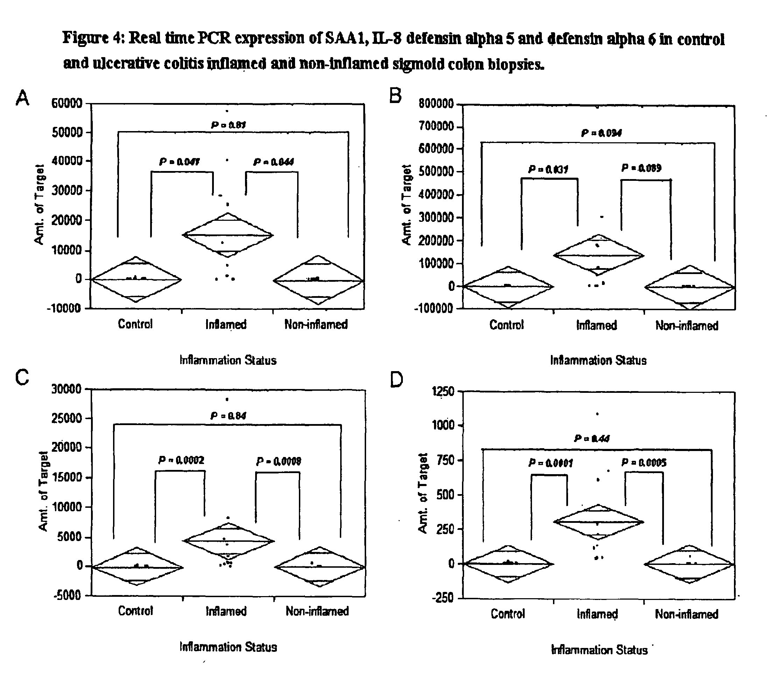

RTPCR Analysis. In the case of 8 genes implicated by microarray expression results, confirmatory real time PCR analysis using 10 healthy control colon sigmoid biopsies with normal histology, 9 quiescent UC sigmoid biopsies and 11 UC sigmoid biopsies with an acute (6 biopsies) or chronic (5 biopsies) inflammatory cell infiltrate was undertaken. Increased SAA1 expression in the inflamed UC sigmoid colon biopsies compared to the normal control sigmoid colon biopsies and the non-inflamed UC sigmoid colon biopsies (p = 0.041 and p = 0.044 respectively) was observed. Elevated IL-8 expression was also confirmed in the inflamed UC sigmoid biopsies when compared to the control sigmoid biopsies (p = 0.031) and a trend was observed towards there being higher IL-8 expression in the inflamed UC sigmoid colon biopsies when compared to the non-inflamed UC biopsies (p = 0.089) (Figure 4).

Figure 4 shows the real time PCR expression data comparing expression in 10 healthy control sigmoid biopsies with normal histology, 9 quiescent UC sigmoid biopsies and 11 UC sigmoid biopsies with an acute or chronic inflammatory cell infiltrate. Expression of SAA1 (A), IL-8 (B), defensin alpha 5 (C) and defensin alpha (D) were compared between the control and the inflamed and non-inflamed UC biopsies. Standard error bars are illustrated in green in each graph.

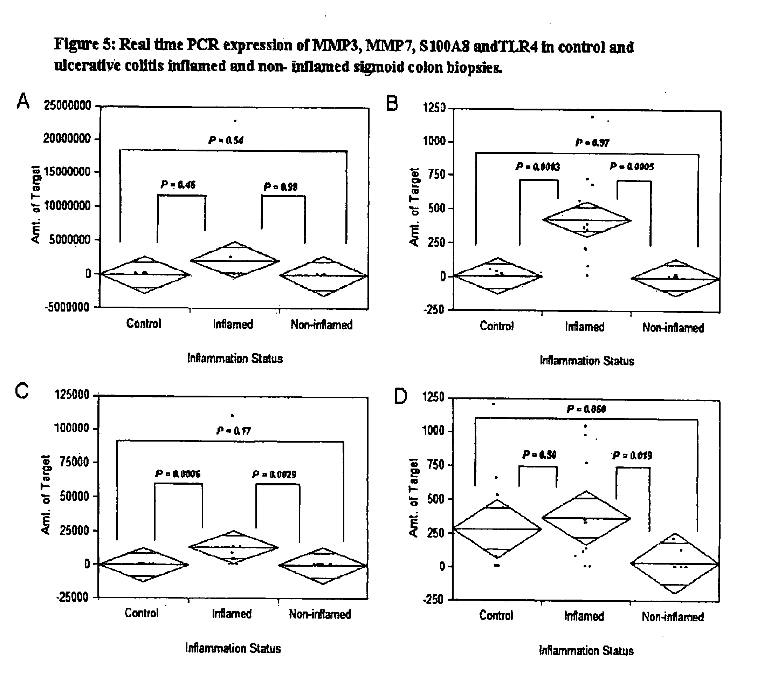

Increased expression of DEFA5 and DEFA6 in the inflamed UC sigmoid colon biopsies when compared to the non- inflamed UC sigmoid colon biopsies (p = 0.0008 and p = 0.0005 respectively) and the control sigmoid colon biopsies (p = 0.0002 and p = 0.0001 respectively) was observed (Figure 4). Increased expression in the inflamed UC sigmoid colon biopsies when compared to the non inflamed UC sigmoid colon biopsies was also observed when MMP7, (p = 0.0005), S100A8, (p = 0.0029) and TLR4, (p = 0.019) were examined (Figure 5).

Figure 5 shows the real time PCR expression data comparing expression in 10 healthy control sigmoid biopsies with normal histology, 9 quiescent UC sigmoid biopsies and 11 UC sigmoid biopsies with an acute or chronic inflammatory cell infiltrate. Expression of MMP3 (A), MMP7 (B), S100A8 (C) and TLR4 (D) were compared between the different patient groups. Standard error bars are illustrated in green in each graph. No significant change in MMP3 expression was observed when the inflamed, non-inflamed UC sigmoid colon biopsies and the control sigmoid colon biopsies were analysed.

*In- Situ* Hybridization and Immunohistochemistry.

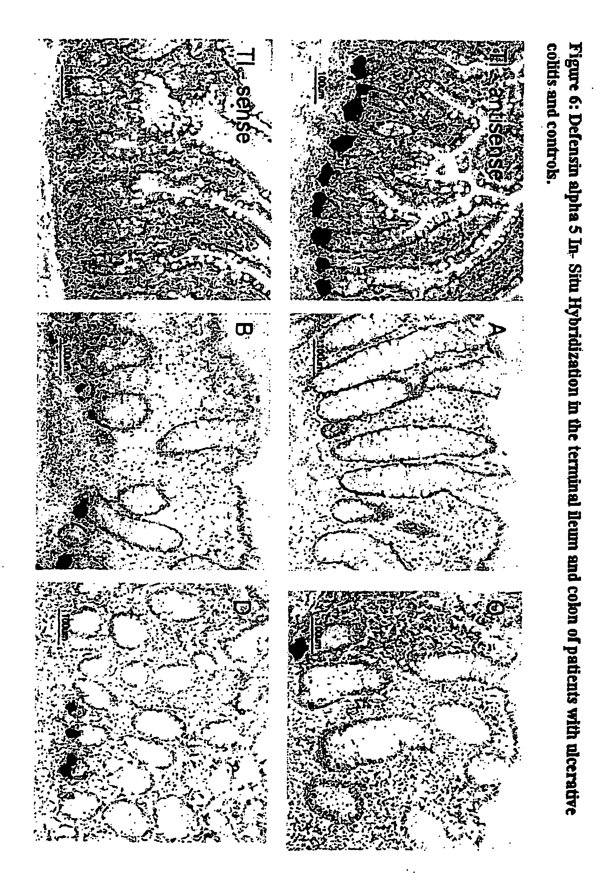

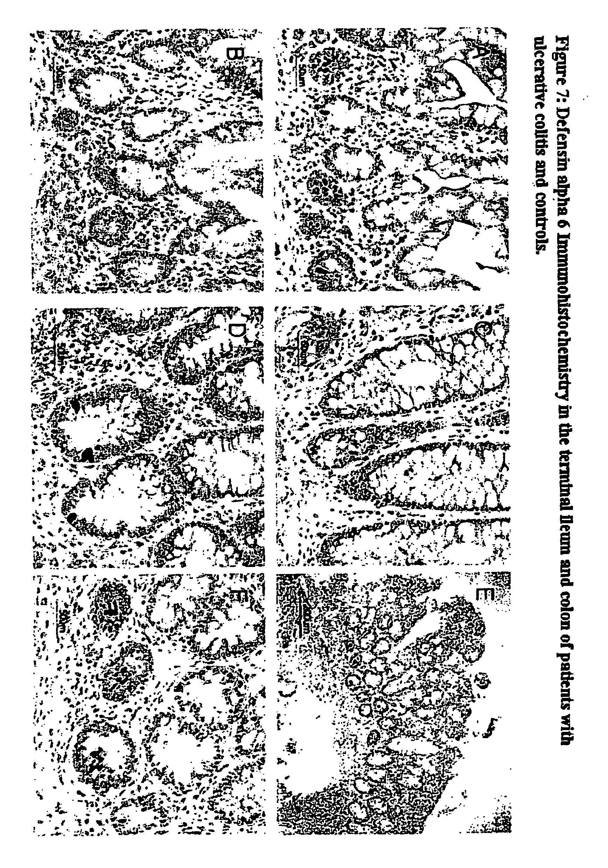

To further investigate the cellular localization of the excess DEFA5& 6 expression in the colon of patients with UC, *in- situ* hybridization and immunohistochemistry was undertaken in a cohort of biopsies of patients with UC and controls (Figures 6 and 7).

Figure 6 shows the *in- situ* hybridization of the terminal ileal biopsies for DEFA5 showed strong hybridization in the basal crypts consistent with Paneth cell location. In the upper panel terminal ileum (TI), the antisense probe shows strong hybridization in the basal crypts consistent with Paneth cell location. In the lower panel terminal ileum (TI), no significant hybridization was observed with sense control probe. Panel A shows the sigmoid colon biopsy of a non- inflamed control patient. Panels B,C, & D show strong, multifocal hybridization in the basal crypt region of UC sigmoid colon biopsies consistent with Paneth cell metaplasia. In the UC biopsies taken from the sigmoid colon strong, multifocal hybridization in the basal crypt region of these biopsies was observed and this would be consistent with Paneth cell metaplasia. This was not observed in the non- inflamed control biopsies.

Figure 7 shows the *in- situ* hybridization of the terminal ileal biopsies for DEFA6. In panel A, terminal ileum immunohistochemistry shows positive staining in the basal crypts consistent with Paneth cell location. In panels B & C, no significant staining was observed in the non- inflamed control patients. In panels D, E, & F, strong, multifocal staining in the basal crypt region of UC sigmoid colon biopsies consistent with Paneth cell metaplasia. Immunohistochemistry for DEFA6 confirmed that in the sigmoid colon UC biopsies, staining was observed in the basal crypt region of these biopsies consistent with Paneth cell metaplasia. Again, this was not observed in the non- inflamed control biopsies (Figure 7).

Expression of Genes within the IBD2 Locus. Using the markers defining the IBD2 locus we identified 526 Agilent probes representing genes or expressed sequence tags within this locus on chromosome 12. 12 probes had a greater or less than 1.5 fold change in expression with p < 0.01 when expression of acute and chronically inflamed UC sigmoid colon biopsies were compared to non- inflamed UC sigmoid colon biopsies (Table 7).

**Table 7**

| ***Agilent Probe*** | ***Gene Symbol*** | **UC sigmoid inflamed (35 biopsies) v non-inflamed (25) fold change** | ***p value*** |
|---|---|---|---|
| A_23_P98876 | SLC39A5 | -1.52855 | 1.36x10⁻⁷ |
| A_24_P647146 | HDAC7A | 1.53876 | 3.15x10⁻¹⁶ |
| A_24_P941773 | DKFZP586A0522 | -2.27306 | 2.31x10⁻¹¹ |
| A_24_P945113 | ACVRL1 | 1.57956 | 6.33x10⁻⁹ |
| A_23_P128230 | NR4A1 | 1.81844 | 0.00005 |
| A_23_P331098 | K5B | 2.3845 | 0.0004 |
| A_24_P246636 | A_24_P246636 | -1.69754 | 7.06x10⁻⁸ |
| A_23_P2233 | SILV | 1.51557 | 9.07x10⁻⁹ |
| A_23_P105251 | GLI | -1.54447 | 7.73x10⁻⁹ |
| A_32_P3783 | HMGA2 | -1.94107 | 2.37x10⁻⁹ |
| A_23_P162300 | IRAK3 | 1.7382 | 3.11x10⁻¹⁵ |
| A_32_P83256 | IRAKM | 1.70847 | 4.56x10⁻¹⁰ |

Analysis of the 526 expression probes located within the IBD2 locus identified 12 probes that were significantly differentially regulated when the inflamed UC sigmoid colon biopsies were compared to the non- inflamed UC sigmoid colon biopsies.

Table 3A (provided above) lists those genes from the IBD2 locus on chromosome 12 that were found to be up-regulated in IBD patients as compared to normal patients.

Table 3B (provided above) lists those genes from the IBD2 locus on chromosome 12 that were found to be down-regulated in IBD patients as compared to normal patients.

Interesting candidate genes that were differentially regulated in the inflamed UC sigmoid samples included keratin 5B (FC +2.38, p = 0.0004), MMP19 (FC +1.95, p = 0.0084), GLI 1(FC -1.54, p = 7.3x10⁻⁹), interleukin-1 receptor-associated kinase 3 (FC +1.74, p = 3.1x10⁻¹⁵) and interleukin-1 receptor-associated kinase M (FC +1.71, p = 0.0014).

When the acute inflammatory signal was removed and non- inflamed UC sigmoid biopsies and non-inflamed control sigmoid biopsies were compared, no sequences had a fold change greater or less than 1.5. However, notably downregulated genes included tubulin alpha 5 (FC -1.32, p = 9.0x10⁻⁶) and tubulin alpha 6 (FC -1.45, p = 1.2x10⁻⁵), molecules involved in the microtubule cytoskeleton and barrier integrity of the bowel.

Expression of Genes within the IBD5 Locus. Agilent probes representing 11 genes within the with IBD5 locus were identified and compared in healthy control, non- inflamed and inflamed UC biopsies (Table 8). Table 8 shows the fold changes in expression of genes within the IBD5 locus comparing controls and patients with UC, who have been stratified for the degree of inflammation observed in their sigmoid biopsies. Significant down regulation of the organic cation transporters SLC22A4 and SLC22A5 was observed when inflamed UC sigmoid biopsies were compared to non-inflamed UC sigmoid biopsies.

**Table 8**

| **Genes Analyzed** | **All UC (129) v controls (73) Fold change** | **p value** | **Inflamed sigmoid (35) v non-inflamed sigmoid (22) (UC) Fold change** | **p value** | **Non-inflamed UC sigmoid (22) v non inflamed control sigmoid (18) Fold change** | ***p value*** |
|---|---|---|---|---|---|---|
| IL4 | +1.00 | 0.96 | +1.06 | 0.62 | +1.14 | 0.40 |
| IL13 | +1.12 | 0.057 | -1.02 | 0.82 | 1.00 | 0.98 |
| RAD50 | +1.02 | 0.20 | -1.20 | 3.5x10⁻⁶ | +1.08 | 0.062 |
| IL5 | +1.09 | 0.037 | +1.10 | 0.17 | +1.04 | 0.53 |
| IRF1 | +1.10 | 0.35 | +1.12 | 2.9x10⁻⁶ | -1.01 | 0.62 |
| SLC22A5 | -1.26 | 3.37x10⁻⁶ | -1.50 | 2.2x10⁻⁶ | +1.02 | 0.75 |
| SLC22A4 | -1.18 | 0.11 | -1.79 | 1.53x10⁻⁹ | +1.22 | 0.63 |
| PDLIM4 | +1.10 | 0.0056 | +1.14 | 0.00039 | +1.01 | 0.78 |
| P4HA2 | -1.05 | 0.13 | 1.00 | 0.98 | -1.05 | 0.28 |
| CSF2 | +1.10 | 0.056 | +1.19 | 0.052 | -1.04 | 0.63 |
| IL3 | -1.01 | 0.39 | +1.02 | 0.67 | +1.01 | 0.75 |

Table 3A (provided above) lists those genes from the IBD5 locus on chromosome 5 that were found to be up-regu lated.

Non significant, but consistent fold increases in expression were observed when IRF1 and PDLIM4 expression was compared in inflamed and non-inflamed UC sigmoid colon biopsies, (FC +1.12, p = 2.9x10⁻⁶ and FC +1.14, p = 0.00039, respectively).

Table 3B (provided above) lists those genes from the IBD5 locus on chromosome 5 that were found to be down-regulated in IBD patients as compared to normal patients. SLC22A5 (OCTN2) was downregulated in UC biopsies compared to controls (FC -1.26 p = 3.37x10⁻⁶) and when inflamed UC sigmoid colon biopsies were compared to non- inflamed UC sigmoid colon biopsies (FC -1.50, p = 2.2x10⁻⁶). Expression of SLC22A4 (OCTN1) was also downregulated in inflamed UC sigmoid colon biopsies compared to non- inflamed UC sigmoid colon biopsies (FC -1.79, p = 1.5x10⁻⁹), however when all UC biopsies were compared to controls, no change was observed (FC -1.18, p = 0.11).

Gene Expression and Disease Activity. Sigmoid colon biopsies from newly diagnosed UC patients who were undergoing endoscopy (8 biopsies) and sigmoid biopsies from patients with established active UC (18 biopsies) were compared. In the newly diagnosed UC sigmoid biopsies, 861 sequences were upregulated with a fold change of greater than 1.5 and p < 0.01, and 373 sequences were downregulated with a fold change of less than 1.5 and p < 0.01 (data not shown).

The 3 most upregulated genes in the newly diagnosed patients were Selectin E (FC +10.95, p = 1.8x10⁻⁶), CCL19 (FC +7.42, p = 4.7x10⁻¹⁵) and IL-8 (FC +7.32, p = 2.9x10⁻⁷), and down regulated genes included S100P (FC -6.4, p = 2.0x10⁻⁹).

Sigmoid colon biopsies from patients from UC patients with a simple clinical colitis activity index (SCCAI) of more than 2 (27 biopsies) were compared to sigmoid colon biopsies from UC patients with a SCCA1 of 2 or less (30 biopsies). 813 sequences were upregulated and 444 sequences were downregulated. Among the most upregulated genes were those that were involved in the acute inflammatory response IL-8 (FC +8.86, p = 4.4x10⁻¹²), MMP3 (FC +6.5, p = 3.2x10⁻¹⁹), MMP7 (FC +5.3, p = 3.4x10⁻²¹) and DEFA6 (FC +4.5, p = 2.7x10⁻¹²). Down regulated genes included UGT2B15 (FC -4.5, p = 0.0013), UGT2B17 (FC -2.8, p = 0.0059) and UGT2B10 (FC -2.2, p = 0.0038), all members of the UGT family which are involved in cellular detoxification and excretion.

Table 3A (provided above) lists additional genes identified in the present study observed to be up-regulated in UC patients as compared to normal patients, while Table 3B (provided above) lists additional genes identified in the present study observed to be down-regulated in UC patients as compared to normal patients.

### DISCUSSION

The present study represents the most rigorous microarray analysis yet reported comparing intestinal gene expression in patients with UC, with healthy controls as well as in patients with other causes of colonic inflammation. The data provide important information on anatomical pattern of gene expression in the healthy colon, with intriguing differences in the right and left colon. Moreover, strong evidence for dysregulated gene expression characteristic of UC has been provided and overall these data provide valuable insight into gene expression in normal physiological homeostasis and during the pathological process ofUC.

The strengths of this data set are the size of the study undertaken, the meticulous assessment of disease phenotype, the documenting of anatomical location for each biopsy and the avoidance of confounding effects due to pooling of samples. Thereby, we have been able to remove a considerable amount of background variability that has hampered previous studies. (Marshall E. Science 2004;306:630-631; Ioannidis JP. Lancet 2005;365:454-455).

Moreover, the present study has addressed the potentially critical confounding effect of the strength of the non- specific acute inflammatory signature. Quiescent biopsies from patients with UC and controls were compared and from these analysis we were able to gain valuable insight into the pathogenesis of UC. In addition, the present study provided access to a proportion of patients with newly diagnosed disease, overcoming treatment related alterations in gene expression. A further strength of our study has been the fact that real time PCR analysis consistently confirmed the significant changes of in expression in all but one of the candidate genes of interest, strongly validating the microarray data and increasing substantially the confidence associated with the interpretation of the data.

This is the first microarray study to show a gradient in expression of a number of genes along the healthy adult colon. These results contrast with data from Costello and colleagues where no significant differences in expression patterns were observed when comparing biopsies from caecum, transverse colon, descending colon and sigmoid colon (Costello CM, et al. PLoS Med 2005;2:e199). The observed differences in these data sets may be explained by our analysis looking only at non- inflamed control samples where as Costello investigated control and diseased patients.

Genes involved in developmental pathways - the HOX family and the hedgehog signaling pathway appeared to be the most differentially regulated along the anatomical length of the healthy colon. HOXA13 has been shown play a crucial role in the development of the tail gut and mutations in the gene result in urogenital abnormalities, (De Santa et. al. Development. 2002;129:551-561) and interestingly it has been shown that HOXB13 expression is down regulated in colorectal tumours from the distal left colon. (Jung et. al. Br J Cancer. 2005;92:2233-2239)

The hedgehog signaling pathway is also crucial to the normal growth and development of the human gastrointestinal system and a number of congenital diseases that affect the gut are due to mutations in genes involved in this pathway. (Lees et. al. Gastroenterology. 2005;129:1696-1710) GLI-1 is one of the major effector molecules of the hedgehog signaling pathway and the GLI-1 gene lies within the IBD2 locus, an area that has previously been shown to be associated with UC. (Parkes et. al. Am J Hum Genet. 2000;67:1605-1610; Satsangi, et. al, Nat. Genet. 1996;14:199-202) Data from the present study would also suggest that GLI-1 is downregulated in inflamed UC biopsies compared to non inflamed biopsies from patients with UC. Recent data from our unit has shown a strong association between mutations in the GLI-1 gene and UC. (Lees et. al. Gastroenterology. 2006;130:A52)

Further recent data published by Varnat and colleagues have suggested that PPARβ negatively regulates Paneth cell differentiation by downregulating the expression of Indian hedgehog, another of the major effector molecules in the hedgehog signaling pathway. (Vamat et. al. Gastroenterology. 2006;131:538-553) Our data have shown upregulation of the alpha defensins 5 and 6 in the colon of patients with inflamed UC compared to non-inflamed biopsies from patients with UC and controls, and immunohistochemistry and *in-situ* hybridization have shown that this is largely mediated by Paneth cell metaplasia. It is intriguing to speculate that in patients with UC, further as yet undetermined defects in the Hedgehog signaling pathway may result in unregulated Paneth cell differentiation, Paneth cell metaplasia, increased alpha defensin 5 and 6 expression, and mucosal inflammation.

Indeed among the most stimulating observations in the present study were the data showing the upregulation of the alpha defensins 5 and 6 in patients with UC. Alpha defensins 5 and 6 are small cationic proteins that are part of the innate immune response and they have potent antimicrobial properties against gram positive and gram negative bacteria. (Ouellette AJ. Springer Semin Immunopathol. 2005;27:133-146) They are stored as pro-molecules in Paneth cells which in the healthy colon are largely restricted to the terminal ileum and on release into the mucosa they are cleaved by trypsin to the active antimicrobial peptide. (Ghosh et.al. Nat Immunol. 2002;3:583-590)

In our data set, high levels of alpha defensin expression were observed in the terminal ileal biopsies of non-inflamed controls and patients with UC. Levels of expression in the control patients and patients with quiescent UC fell as the location that the biopsies were retrieved from became more distal in the colon - ascending colon, descending colon and sigmoid colon. However, in the inflamed UC biopsies increased expression of both alpha defensins 5 and 6 was observed at each anatomical location that was biopsied. Lawrance and colleagues also noted the defensins alpha 5 and 6 were upregulated in patients with UC compared to controls, (Lawrance et. al. Hum Mol Genet. 2001;10:445-456) although, RNA was extracted from surgical resections and no details about the anatomical location of these specimens were given.

Recent data examining the expression of the alpha defensins 5 and 6 in patients with CD in the terminal ileum would suggest that expression levels are reduced irrespective of the degree of inflammation when compared to control terminal ileal tissue and this may be responsible for the terminal ileal phenotype observed in CD. (Wehkamp et. al. Proc Natl Acad Sci U S A. 2005;102:18129-18134) Whether the observed increase in alpha defensin 5 and 6 expression in our data set is a primary phenomenon related to disease pathogenesis, or whether it is secondary phenomenon protecting a previously damaged epithelial surface to microbial invasion may be resolved by looking for critical variants within these genes that are associated with disease susceptibility and altered function of these peptides.

It is of interest that many but not all of our results are broadly in line with two of the landmark microarray papers in IBD. Consistent with data from Lawrance and colleagues (Lawrance et. al. Hum Mol Genet. 2001;10:445-456) we have shown upregulation of S100A8 & A9 and the alpha defensins 5 & 6 in UC. However, no overlap was observed in the differentially expressed probes in the IBD2 locus in the present study and in that of Lawrance. Dieckgraefe and colleagues (Dieckgraefe et.al. Physiol Genomics. 2000;4:1-11) observed upregulation of a number of the MMP genes, consistent with our data and interestingly members of the REG family were shown to be upregulated in the colon of patients with UC, probably as a result of Paneth cell metaplasia.

The downregulation of ABCB1 in our dataset is of significant interest, and consistent with earlier microarray data from Langman, Dieckgraefe and Lawrance and colleagues. (Dieckgraefe et.al. Physiol Genomics. 2000;4:1-11; Lawrance et. al. Hum Mol Genet. 2001;10:445-456; Langmann et. al. Gastroenterology. 2004;127:26-40) In addition to this, we have observed that the expression in ABCB1 displayed a decreasing gradient, with gene expression lowest in the sigmoid colon in UC. This pattern of expression is perhaps consistent with the hypothesis associating loss-of-function of P-glycoprotein (gene product of ABCB 1) in UC, and in line with the clinical presentation of UC. The current data illustrate the importance of ABCB1 in the disease pathogenesis of UC, as highlighted in recent genetic and animal functional data. (Moodie et. al. Glucocorticoid access and action in the rat colon: expression and regulation of multidrug resistance 1a gene (mdr1a), glucocorticoid receptor (GR), mineralocorticoid receptor (MR) and 11-beta-hydroxysteroid dehydrogenase type 2 (11BHDS2). 197 ed. 2003)

ABCB1 encodes P-glycoprotein 170, an efflux epithelial transporter involved in gut barrier defence and xenobiotic metabolism. It is pertinent that when the entire class of proteins sharing homology with ABCB1 were analyzed, a further 6 out of 48 genes (12.5%) of the ABC transporters were significantly dysregulated in UC; suggesting an important role in this class of protein in the aetiopathogenesis of UC.

In contrast to data produced by Langmann we did not observe any changes in expression of the transcriptional regulator Pregnane-X receptor. These negative data are consistent with genetic studies carried out in the IBD population in Edinburgh- using a haplotype tagging approach, there was an association between the ABCB1 gene and UC , (Ho et. al. Hum Mol Genet. 2006;15:797-805) but no association between the Pregnane- X receptor and UC. (Ho GT et. al. Gut. 2006;55:1676-1677) Aspects of study design may explain the differences observed in this context between our data and those of Langmann and colleagues, as our analysis took in to consideration the inflammation status of the biopsies and anatomical location. In our data set we did not pool samples, thus carrying out a large number of microarrays, where as Langmann and colleagues pooled terminal ileal and colon biopsies and this difference in methodology may also account for the different results.

The matrix metalloproteinases (MMPs) comprise of a family of greater than 23 zinc-dependent enzymes that are end stage effecter molecules involved in the degradation of extra cellular matrix components during morphogenesis and tissue remodeling. (Brinckerhoff et. al. Nat Rev Mol Cell Biol. 2002;3:207-214) Consistent with our data, previous studies in patients with IBD have shown a marked increase in expression of MMP3 in the inflamed biopsies when compared to non inflamed biopsies and control biopsies. (Heuschkel et. al. Gut. 2000;47:57-62; von LBet. al. Gut. 2000;47:63-73) Data for MMP3 -/mice have also demonstrated delayed clearance of bacteria, a compensatory increase in MMP7 and reduced CD4⁺ T lymphocyte recruitment to the lamina propria. (Li et. al. J Immunol. 2004;173:5171-5179) Furthermore, recent data have also shown a proinflammatory effect of MMP3 mediated through CXCL7 causing dose dependant neutrphil recruitment in colonic cell lines. (Kruidenier et. al. Gastroenterology. 2006; 130:127-136)

In our data set we observed a decrease in MMP3 expression in our inflamed controls compared to our non-inflamed controls suggesting that the changes observed in MMP3 and MMP7 expression in UC may be disease specific. Transmission disequilibrium testing of the 5A variant of MMP3 in the German population showed an association with CD and not UC that was not replicated in the English population. (Pender et. al. J Med Genet. 2004;41:e112)

We have also used this dataset to analyse the relative expression of genes mapping to susceptibility loci implicated by genome wide analysis, notably within IBD2 and IBD5. The present data will be of use in gene identification, complementing results of fine-mapping studies, and genome- wide case- control studies currently underway. In this context it is of considerable interest to review our data concerning the IBD5 locus which spans a cytokine cluster containing a number of attractive candidate genes was initially discovered by genome wide scanning in 2000. (Rioux et. al. Am J Hum Genet. 2000;66:1863-1870; Ma et. al. Inflamm Bowel Dis. 1999;5:271-278) The tight linkage disequilibrium spanning the IBD5 linkage interval, has limited previous genetic studies, and these have not been sufficiently powered to identify the susceptibility gene within this region. (Waller, et. al. Gut. 2006;55:809-814; Fisher et. al. Hum Mutat. 2006;27:778-785; Noble et. al. Gastroenterology. 2005;129:1854-1864)

Downregulation of the positional candidate gene encoding the organic cation transporter SLC22A5 (OCTN2) was observed when UC biopsies were compared to control biopsies and in both SLC22A4 (OCTN1) and SLC22A5 down regulation was observed in the inflamed UC sigmoid colon biopsies compared to non-inflamed sigmoid biopsies. These provocative data would suggest that decreased expression of these genes may after all be involved in the pathogenesis of UC, and these data thereby may sustain interest in these genes. Peltekova and colleagues suggested that two variants in these genes-SLC22A4 (1672C→T) and the SLC22A5 variant (-207G→C) conferred disease susceptibility to CD, (Peltekova et. al. Nat Genet. 2004;36:471-475) however, when expression was compared in the small number of patients in this study who had been genotyped for these mutations no change in expression was observed between wild type and TC homozygote patients (data not shown). Expression of IRF-1 and PDLIM4, both plausible candidates within IBD5 was also dysregulated, emphasizing the uncertainties pertaining to this locus at present. Within the IBD2 locus, a series of dysregulated genes were identified in the present dataset, of which only GLI 1 has been subjected to detailed mutation analysis.

In conclusion these data have rigorously characterized expression of the whole genome in the terminal ileum and colon of patients which UC and controls. The studies provide new insights into regional variation of gene expression in the healthy colon, and also considerably extend previous studies in UC. These data identify a number of key regulators of intestinal inflammation, notably the alpha defensin family, hedgehog signaling molecules, and matrix metalloproteinases.

### Example 3 - Immunohistochemistry

DefA6 Expression in IBD Biopsies.

Defensin alpha 6 is normally expressed by Paneth cells in the small intestine crypt epithelium and not in colon epithelial cells. We had observed increased DefA6 expression at the RNA level in ulcerative colitis and Crohn's disease patients using Agilent microarray and in taqman on biopsy lysates. This experiment evaluated if increased DefA6 protein expression could be seen in formalin fixed colon biopsies.

Our studies show, as expected, that there was no DefA6 staining in colon biopsies from non-IBD control patients with no histologic evidence of inflammation. We also evaluated one non-IBD control patient with a diagnosis of microscopic colitis. In that patient, DefA6 was present in sigmoid colon crypt epithelial cells.

In ulcerative colitis patients, 21 patients had scattered or clustered DefA6 staining in crypt epithelial cells of the sigmoid colon, descending colon, transverse colon, or rectum. Twenty of 21 positive patients had histologic evidence of chronic or chronic-active inflammation in their biopsy tissue. The remaining patient had predominantly acute (neutrophilic) inflammation. No patients with positive DefA6 staining in the colon had uninflamed biopsies.

There were 18 ulcerative colitis patients with no evidence of DefA6 staining in colon epithelium. The majority of these patients (10) had no histologic evidence of inflammation in the biopsy tissue. Six of the remaining patients had predominantly neutrophilic inflammation (acute inflammation) and two had chronic/chronic-active inflammation.

In summary, DefA6 expression in ulcerative colitis appears to correlated with the local inflammation status observed in the biopsy. None of the uninflamed biopsies had DefA6 staining. In addition, patients with chronic or chronic-active inflammation were more likely to have positive DefA6 staining than patients with acute inflammation.

Experimental Design: The scoring of inflammation status was based on inflammatory cell type predominance: neutrophil predominance = acute inflammation; neutrophils and mononuclear inflammatory cells = chronic active; and predominantly mononuclear inflammatory cells = chronic.

Table 9 shows the histologic findings. The expression of DefA6 is shown as "-", "+", or "++" and the corresponding inflammation score is provided in some cases.

**Table 9**

| **Patient** | **HP #** | **Tissue** | **DefA6** | **Inflammation status** |
|---|---|---|---|---|
| 115 | HP-19891 | sigmoid | + | microscopic colitis |
| 119 | HP-19893 | descending | - | |
| 122 | HP-19898 | sigmoid | - | |
| 124 | HP-19899 | sigmoid | - | |
| 125 | HP-19900 | sigmoid | NA | this is a section of terminal ileum |
| 125 | HP-19901 | sigmoid | - | |
| 130 | HP-19905 | rectum | - | |
| 131 | HP-19906 | sigmoid | - | |
| 135 | HP-19908 | sigmoid | - | |
| 136 | HP-19909 | sigmoid | - | |
| 222 | HP-19912 | rectum | + | chronic active |
| 223 | HP-19914 | sigmoid | + | chronic |
| 224 | HP-19916 | sigmoid | + | minimal chronic active |
| 225 | HP-19919 | sigmoid | - | no inflammation |
| 226 | HP-19920 | descending | - | no inflammation |
| 227 | HP-19922 | sigmoid | + | minimal chronic active |
| 230 | HP-19924 | sigmoid | - | no inflammation |
| 230 | HP-19925 | rectum | - | no inflammation |
| 231 | HP-19926 | descending | - | acute inflammation |
| 233 | HP-19927 | sigmoid | - | no inflammation |
| 234 | HP-19928 | descending | - | minimal chronic active HP |
| 235 | HP-19929 | rectum | - | acute inflammation |
| 238 | HP-19930 | sigmoid | + | chronic active |
| 239 | HP-19932 | rectum | + | minimal chronic |
| 240 | HP-19933 | rectum | - | minimal acute |
| 241 | HP-19934 | rectum | - | no inflammation |
| 244 | HP-19935 | sigmoid | - | no inflammation |
| 246 | HP-19936 | sigmoid | + | minimal chronic |
| 247 | HP-19937 | rectum | + | minimal chronic |
| 248 | HP-19938 | rectum | + | minimal chronic |
| 249 | HP-19939 | rectum | + | chronic active |
| 250 | HP-19940 | sigmoid | + | chronic active |
| 251 | HP-19941 | rectum | + | chronic active |
| 253 | HP-19942 | sigmoid | + | chronic active |
| 255 | HP-19943 | sigmoid | NA | fragmented section |
| 256 | HP-19944 | sigmoid | + | chronic active |
| 257 | HP-19947 | rectum | ++ | chronic active |
| 258 | HP-19948 | rectum | + | chronic |
| 259 | HP-19949 | sigmoid | ++ | chronic active |
| 260 | HP-19950 | sigmoid | + | chronic active |
| 261 | HP-19951 | sigmoid | - | mild acute |
| 262 | HP-19952 | sigmoid | - | acute inflammation |
| 262 | HP-19953 | rectum | - | no inflammation |
| 263 | HP-19954 | rectum | - | no inflammation |
| 265 | HP-19955 | sigmoid | + | chronic active |
| 266 | HP-19956 | rectum | + | acute inflammation |
| 267 | HP-19957 | transverse | ++ | Chronic active |
| 270 | HP-19958 | rectum | - | Chronic active |
| 245 | HP-19965 | sigmoid | - | no inflammation |
| 245 | HP-19966 | rectum | - | acute inflammation |

### Example 4 - Analysis of Germline GLI1 Variation

Ulcerative colitis (UC) and Crohn's disease (CD) are polygenic chronic inflammatory bowel diseases (IBD) of high prevalence that are associated with considerable morbidity. The hedgehog (HH) signalling pathway plays vital roles in gastrointestinal tract development, homeostasis and malignancy. We identified a germline variation in *GLI1* (within the IBD2 linkage region, 12q13) in patients with IBD. Since this IBD-associated variant encodes a GLI1 protein with reduced function, we tested whether mice with reduced Gli1 activity are susceptible to chemically induced colitis. Using a gene-wide haplotype-tagging approach, germline *GLI1* variation was examined in three independent populations of IBD patients and healthy controls from Northern Europe (Scotland, England and Sweden) totalling over 5000 individuals. On log-likelihood analysis, *GLI1* was associated with IBD, predominantly UC, in Scotland and England (p<0.0001). A non-synonymous SNP (rs2228226C→G), in exon 12 of *GLI1* (Q1100E) was strongly implicated, with pooled odds ratio of 1.194 (C.I.=1.09-1.31, p=0.0002). *GLI1* variants were tested in vitro for transcriptional activity in luciferase assays. Q1100E falls within a conserved motif near the C-terminus of GLI1; the variant GLI protein exhibited reduced transactivation function in vitro. In complementary expression studies, we noted the colonic HH response, including GLI1, PTCH and HHIP, to be down-regulated in patients with UC. Finally, *Gli1+*/*-* mice were tested for susceptibility to DSS-induced colitis. Clinical response, histology and expression of inflammatory cytokines were recorded. *Gli1+*/*-* mice rapidly developed severe intestinal inflammation, with considerable morbidity and mortality compared with wild-type. Local myeloid cells were shown to be direct targets of HH signals and cytokine expression studies revealed robust up-regulation of IL-12, IL-17 and IL-23 in this model. HH signalling through GLI1 is required for proper modulation of the intestinal response to acute inflammatory challenge. Reduced GLI1 function predisposes to IBD pathogenesis, suggesting novel therapeutic avenues.

Ulcerative colitis (UC; MIM 191390) and Crohn's disease (CD; MIM 266600) are chronic, relapsing, inflammatory bowel diseases (IBD) of high prevalence (200-400 cases per 100,000 in N Europe and N America [Loftus EV, Jr. (2004) Gastroenterology 126: 1504-1517]) and are associated with considerable morbidity. Precise aetio-pathogenetic mechanisms are not understood but several lines of evidence implicate the central importance of a dysregulated host response to intestinal bacteria [Xavier RJ, Podolsky DK (2007) Nature 448: 427-434]. Epidemiological data, detailed molecular studies, and recent genome-wide association studies strongly suggest that UC and CD are related polygenic diseases that share some susceptibility loci (IL-23R, IL-12B, and NKX2.3 [Wellcome Trust Case Control Consortium (2007) Nature 447: 661-678; Duerr et al. (2006) Science 314: 1461-1463; Parkes et al. (2007) Nat Genet Jul;39(7):830-2. Epub 2007 Jun 6]), but differ at others: NOD2, ATG16L1 and IRGM are specific CD genes; the ECM1 locus is associated with UC [Parkes et al. (2007) Nat Genet Jul;39(7):830-2. Epub 2007 Jun 6; Fisher et al. (2008). Nat Genet 40(6):710-2. Epub 2008 Apr 27; Hampe et al. (2007) Nat Genet 39: 207-211; Hugot et al. (2001). Nature 411: 599-603; Ogura Y et al. (2001). Nature 411: 603-606]. The IBD2 locus (OMIM 601458) on chromosome 12q13 was first identified in a UK genome-wide scan (peak LOD score 5.47 at D12S83) [Satsangi et al. (1996). Nat Genet 14: 199-202] involving both UC and CD patients. Later studies showed that IBD2 contributes significantly to UC, notably extensive disease, but perhaps in a more minor way to CD susceptibility [Achkar et al. (2006). Am J Gastroenterol 101: 572-580; Parkes M et al. (2000) Am J Hum Genet 67: 1605-1610]. A strong candidate gene that maps to the IBD2 locus is *GLI1,* one of three related GLI transcription factors that transduce secreted hedgehog (HH) signals. HH signalling is key in gut development, homeostasis and malignancy, but has not been carefully studied in IBD [Lees et al. (2005) Gastroenterology 129: 1696-1710]. In developing intestine, Sonic (SHH) and Indian Hedgehog (IHH) provide a paracrine signal from epithelium to the mesenchymal receptor patched (PTCH). PTCH controls HH signal transduction through the membrane protein smoothened (SMO) and zinc finger transcription factors GLI1, GLI2, and GLI3 to direct tissue pattern and cell fate [Madison et al. (2005) Development 132: 279-289]. Chronic injury, inflammation and repair are critical aspects of IBD, and thus it is pertinent that the HH pathway is centrally involved in these processes in several other tissues, including muscle [Pola et al. (2003) Circulation 108: 479-485], liver [Jung et al. (2008) Gastroenterology 134: 1532-1543; Omenetti et al. (2008) Gut May;134(5):1532-43. Epub 2008 Feb 14.], and lung [Stewart et al. (2003) J Pathol 199: 488-495; Watkins et al. (2003) Nature 422: 313-317]. Indeed, HH signalling may play a central role in the inflammatory response since SHH is critical for T lymphocyte development [El Andaloussi et al. (2006). Nat Immunol 7: 418-426], adult human CD4+ T cell activation [Lowrey et al. (2002) J Immunol 169: 1869-1875; Stewart et al. (2002). J Immunol 169: 5451-5457], and myeloid cell maturation in the spleen [Varas et al., (2008) J Leukoc Biol Jun;83(6):1476-83. Epub 2008 Mar 11]. Dysregulation of components of the HH pathway has also been noted in inflammatory diseases of the gut, including Barrett's esophagus, chronic gastritis and IBD [Nielsen et al., (2004) Lab Invest 84: 1631-1642]. Using microarray gene expression analyses of colonoscopic biopsies, we recently demonstrated that GLI1 is downregulated in the intestinal mucosa in inflamed UC compared with non-inflamed samples [Noble et al. (2008) Gut. Oct;57(10):1398-405. Epub 2008 Jun 3]. To further explore the possible association between GLI1 and IBD susceptibility, we examined haplotype variation at the *GLI1* locus in several Northern European populations using a gene-wide haplotype-tagging approach. We identified a significant association with IBD strongly implicating a non-synonymous SNP in the C-terminal region of GLI1. Functional analysis of the associated variant in vitro demonstrated a 50% reduction in GLI1 transcriptional activation, evidence that this may be a functional variant increasing disease risk. These findings together led us to hypothesize that a reduced dosage of functional GLI1 protein might play an important role in colonic inflammation. To test this directly, we challenged *Gli1+*/*-* mice and their wild type (WT) littermates with dextran sodium sulphate (DSS) to induce acute intestinal inflammation. *Gli1+*/*-* mice rapidly developed severe colitis, suggesting that functional Gli1 activity is crucial to response to inflammatory stimuli in mouse and man.

Subjects and Samples. Table 10 provides detailed demographics and phenotypic data on Scottish and Cambridge IBD population. (HC - healthy control; CD - Crohn's disease; UC - ulcerative colitis; IC - indeterminate colitis.

**Table 10**

| | ***Scottish Panel*** | ***Cambridge Panel*** |
|---|---|---|
| **Total number** | **2183** **(1374 HC; 474 UC; 335 CD)** | **2337** **(589 HC; 928 UC; 737 CD; 83 IC)** |
| Sex - % male | HC 48.7%; UC 52.0%; CD 39.6% | HC 45.0%; UC 52.6%; CD 37.0% |
| Median age at diagnosis/ recruitment -years (IQR) | HC 50.0 (43.0-55.0) | HC 60.0 (53.0-69.0) |
| | UC 34.1 (25.2-49.9) | UC 36.7 (26.84-50.35) |
| | CD 27.8 (20.8-41.1) | CD 26.1 (20.3-37.2) |
| **CD location** | | |
| Terminal ileum (L1) | 38.4% | 31.8% |
| Colon (L2) | 36.5% | 36.5% |
| Ileocolon (L3) | 25.1% | 31.7% |
| **UC location** | | |
| Proctitis (E1) | 17.3% | 14.8% |
| Left-sided colitis (E2) | 40.5% | 34.1% |
| Extensive colitis (E3) | 42.2% | 51.1% |
| **CD 5 year behaviour** | | |
| Inflammatory (B1) | 64.8% | 52.3% |
| Stricturing (B2) | 14.8% | 35.2% |
| Penetrating (B3) | 20.3% | 12.5% |
| Perianal involvement (p) | 17.4% | 24.4% |
| Surgery CD | CD 59.3% | CD 52.0% |
| | UC 18.5% | UC 11.8% |
| Smoking at diagnosis of CD | YES 40.7% | YES 41.8% |
| | NO 47.4% | NO 45.0% |
| | EX 11.9% | EX 13.2% |
| Smoking at diagnosis of UC | YES 19.0% | YES 12.1% |
| | NO 49.5% | |
| | EX 31.5% | |

**Genotyping.** Scotland and Sweden: Genomic DNA was extracted from blood samples using a modified salting-out technique as previously described, and Nucleon kits. Genotypes were derived using the Taqman system for allelic discrimination; the assays were available from Applied Biosystems as Taqman SNP Genotyping Assays (7900HT sequence detection system; Applied Biosystems), except for SNPs rs10783819, rs3809114, rs507562, rs542278, rs730560, rs1669296, rs775322 which were genotyped on the Illumina platform. The accuracy of each Taqman assay was checked by repeat analysis in 5% of cases, with 100% concordance. Genotype distributions in control populations were consistent with Hardy-Weinberg Equilibrium (p>0.01) for all SNPs. Genotypes, in cases for the four tSNPs that could not be derived by Taqman were obtained by direct sequencing. Cambridge: DNA was extracted using Nucleon kits. Genotyping of CD cases and controls was performed using the Taqman biallelic discrimination system using an ABI 7900HT analyser (Applied Biosystems). Genotyping of UC cases was performed using a 1536 SNP Golden Gate bead array (Illumina). Concordance between platforms was assessed by genotyping 92 UC cases for SNPs rs2228224 and rs2228226 with concordance rates of 100% and 97.9% respectively and no evidence of systematic bias between platforms. Genotype distributions in case and control populations were consistent with Hardy-Weinberg Equilibrium (p>0.01) for all SNPs.

**Gene expression by microarray and QPCR.** The cohort of patients used in the microarray studies consisted of 67 patients with UC, 53 with CD and 31 healthy controls (HC). For demographics, generation of microarray dataset and QPCR methodology [see Noble et al. (2008) Gut. Oct;57(10):1398-405. Epub 2008 Jun 3].

**Induction of DSS-induced colitis and histological analysis.** Groups of two to four wild type *Gli1+*/*lacZ* or wild type littermate controls in mixed cages (on a C57BL/6 background) were administered 3% DSS in drinking water for 4-6 days. The amount of DSS consumed was not significantly different between WT and *Gli1+*/*-* animals (data not shown). *Gli1+*/*-* animals tended to weigh more than their WT littermates (mean = 28g for *Gli1+*/*-* animals and 24g for WT animals). Therefore, DSS-treated weight matched WT C57Bl/6 animals (n= 4) were also tested along with *Gli1+*/*-* animals and WT littermates. No differences were evident between WT littermates and weight matched C57Bl/6. All animals were monitored daily for diarrhoea, bloody stool, and weight loss. Clinical scoring was as follows: 0 = no symptoms, 1 = diarrhoea, 2 = bloody stool, 4 = severe rectal bleeding and morbidity to the point of immobility/death. For histology, a segment of large intestine tissue of equal length and location for all animals was fixed overnight in 4% paraformaldehyde, dehydrated, infiltrated with paraffin, and sectioned at 5µm. Slides were stained with hematoxylin/eosin and scored histologically by a gastrointestinal pathologist (HA) blinded to the source of the tissue.

**Protein Mutagenesis and Luciferase Assay** GLI1 E1100 was amplified from Image Clone #3531657, and cloned into pCMVTag2b. GLI1 Q1100 was obtained by inducing a point mutation using the QuikChange II Site-Directed Mutagenesis Kit (Stratagene) following the manufacturer's protocol. Plasmids encoding 8xGli-Luciferase, m8x Gli-Luciferase (mutated Gli sites) and Gli2ΔN were gifts of Dr. Andrzej Dlugosz. 293T cells were plated in 12 well plates, transfected with 0.7µg/well transcription factor, 0.4µg/well reporter plasmid, and 2ng/well pRL-TK Renilla (Promega) and analyzed for luciferase expression 36 hours after transfection using the Dual-Luciferase Reporter Kit (Promega) following the manufacturer's protocol. Firefly luciferase expression was normalized by well to Renilla, and fold changes were calculated by comparing to 8xGli-Luciferase transfected alone.

Cytokine Expression QPCR Whole colonic mRNA was collected using Trizol, followed by RNA cleanup with DNase digestion using the RNeasy Mini Kit (Qiagen). cDNA was synthesized using the iScript cDNA synthesis kit (Biorad), and SybrGreen QPCR was performed on a Biorad iCycler machine. Expression levels were normalized to GAPDH, and statistical analysis was performed using the Student's T-test.

Statistical analysis. Haplotype frequencies of the tSNPs were inferred using the expectation-maximization algorithm and these used to test whether haplotype frequencies were different in cases and controls as implemented in the EH and PM programmes. The test statistic 2*(In(Lcase) + In(Lcontrol) - In(Lcase/Lcontrol)), which has a χ² distribution with n-1 degrees of freedom (where n = number of possible haplotypes) was calculated and empirical p values obtained by permuting the data 10,000 times. Haplotypes were examined using the Haploview programme v3.2 (www.hapmap.org). Individual SNP analysis was performed using χ² or Fisher's exact test, where appropriate, with twotailed p-values given and odds ratios (OR) presented with 95% confidence intervals (C.I.). The meta-analysis of SNP rs2228226 was performed using the Mantel-Haenszel method using a fixed effects model (R-software package). Details for calculation of false positive report probability are provided in supplementary methods. Expression profiles were analysed using Mann-Whitney U test and Kruskal-Wallis test, assuming a non-parametric distribution of all datasets (GraphPad Prism 4, GraphPad Software Inc.).

### RESULTS

### Gene-wide variation in GLI1 is associated with IBD and attributable to a nonsynonymous SNP (rs2228226) in the Scottish population

Four multi-marker tagging single nucleotide polymorphisms (tSNPs; r²≥0.8) were identified (rs3817474, rs2228225, rs2228224, and rs2228226) to describe haplotypic variation of *GLI1,* detecting haplotypes of a frequency >1%. We genotyped these 4 tSNPs in a Scottish IBD population consisting of 474 UC and 335 CD cases, and 1364 well-matched healthy controls (Table 10). We then used a model-free analysis [Zhao et al. (2000) Hum Hered 50: 133-139] to test the association of *GLI1* and IBD susceptibility. In the Scottish population, we demonstrate a highly significant association in IBD (p<0.0001) and UC (p<0.0001), and an association with CD of borderline significance (p=0.03). On analysis of individual estimated haplotype frequencies in Haploview, 3 common haplotypes were described (DATA NOT SHOWN). We confirmed that this effect was confined to the *GLI1* gene by genotyping an additional 7 haplotypetagging SNPs, chosen from Phase II HapMap data, to tag neighbouring blocks, in 166 CD and 170 UC patients. This confirmed the presence of a *GLI1* spanning haplotype block that did not extend into neighbouring genes (*INHBE* and *ARHGAP9*) (DATA NOT SHOWN).

Table 11 shows minor allelic frequencies for GLI1 non-synonymous SNP rs2228226 (tSNP4) in Scottish, English, and Swedish healthy controls (HC), inflammatory bowel disease (IBD), Crohn's disease (CD) and ulcerative colitis (UC).

**Table 11**

| | HC | | IBD | | | UC | | | CD | | |
|---|---|---|---|---|---|---|---|---|---|---|---|
| | N | % | N | % | p value OR (C.I.) | N | % | p value OR (C.I.) | N | % | p value OR (C.I.) |
| Scotland | 1374 | 30.3 | 884 | 34.8 | 0.0026 | 474 | 33.9 | 0.042 | 335 | 36.1 | 0.0053 |
| | | | | | 1.23 (1.07-1.40) | | | 1.19 (1.01-1.39) | | | 1.30 (1.08-1.55) |
| Cambridge | 589 | 26.4 | 1737 | 29.6 | 0.042 | 928 | 30.8 | 0.017 | 737 | 27.9 | 0.40 |
| | | | | | 1.17 (1.0-136) | | | 1.21 (1.03-1.42) | | | 1.08 (0.90-1.28) |
| Sweden | 281 | 30.6 | 493 | 35 | 0.27 | 288 | 34.4 | 0.43 | 205 | 35.9 | 0.24 |
| | | | | | 1.14 (0.90-1.45) | | | 1.11 (0.90-1.45) | | | 1.19 (0.90-1.58) |

Odds ratios and two-tailed p-values are given for χ2 analysis of IBD vs. HC, UC vs. HC and CD vs. HC in each of these three populations. Meta-analysis of these data are presented in Figure 1. Frequencies of estimated haplotypes in all three populations and the full genotype data for the Scottish population are detailed in supplementary materials (Supplemental Tables 1 and 2).

The association on haplotype testing and log-likelihood analysis was largely attributable to a non-synonymous SNP in exon 12 of *GLI1* (rs2228226C→G; tSNP4). rs2228226 was associated with IBD (allelic frequency OR=1.23, C.I. 1.07-1.40, p=0.0026; homozygotes OR=1.56, C.I. 1.15-2.11, p=0.0047), CD (allelic frequency OR=1.30, C.I. 1.08-1.55, p=0.0053, homozygotes OR=1.79, C.I. 1.21-2.65, p=0.0048) and UC (allelic frequency OR=1.19, C.I. 1.01-1.39, p=0.04) (Table 11 and DATA NOT SHOWN). These data suggest an allele specific dose response with a greater odds ratio for homozygotes than heterozygote patients. Mutation screening of the *GLI1* coding regions by direct sequencing failed to identify any novel SNPs. There was no association between 7 additional *GLI1* variants from dbSNP and IBD (DATA NOT SHOWN).

Replication of *GLI1* association in two independent North European IBD cohorts and meta-analysis. We then sought to replicate these findings in other populations. In a large IBD panel from Cambridge, England (n=928 UC, 737 CD, 83 indeterminate colitis and 589 HC) association with *GLI1* was replicated by log-likelihood analysis in IBD (p=0.009) and UC (p<0.0001). rs2228226 was associated with IBD (OR 1.17, C.I. 1.00-1.36, p=0.042) and UC (OR 1.21, C.I. 1.03-1.42, p=0.017) but not CD in this population (Table 11). As in Scotland, there was no association with tSNPs1-3 (DATA NOT SHOWN). In the smaller Swedish cohort (n=770), there was a non-significant trend to association of rs2228226 with IBD (OR 1.14, C.I. 0.90-1.45) (Table 11).

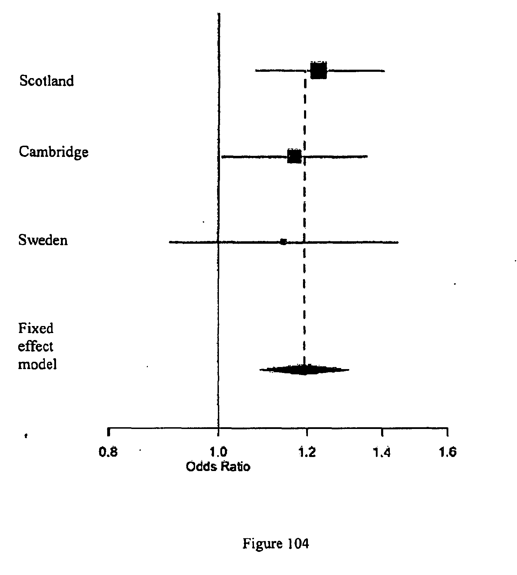

Figure 104 shows a meta-analysis, using the Mantel-Haenszel method with a fixed effects model on the IBD cases and healthy controls in Scotland, England and Sweden confirmed the association with rs2228226 (OR 1.194, C.I. 1.089-1.309, p=0.0002). The meta-analysis is of non-synonymous *GLI1* SNP rs2228226 (tSNP4) in Scotland, Cambridge and Sweden using Mantel-Haenszel method (n=5352 individuals). There was no evidence of heterogeneity in the contribution of rs2228226 between the 3 cohorts (p=0.825). Recognising the current problem with the publication of false positive findings in genetic association studies we estimated the probability that the association with disease risk found in the meta-analysis of *GLI1* SNP rs2228226 represents a true (rather than false positive) association by adopting the false positive report probability (FPRP) approach described by Wacholder et al. (2004) J Natl Cancer Inst 96: 434-442. This gives an estimated probability that these findings represent a true finding of at least 92% (FPRP < 0.08). This method is designed to avoid overinterpretation of statistically significant findings that are not likely to signify a true positive but in our study gives clear support to our interpretation of these data.

The GLI1 variant encoded by rs2228226 is functionally deficient in activating GLI-responsive transcription in vitro rs2228226C→G is a mis-sense mutation in exon 12 of *GLI1,* encoding a change from glutamine to glutamic acid (Q1100E).

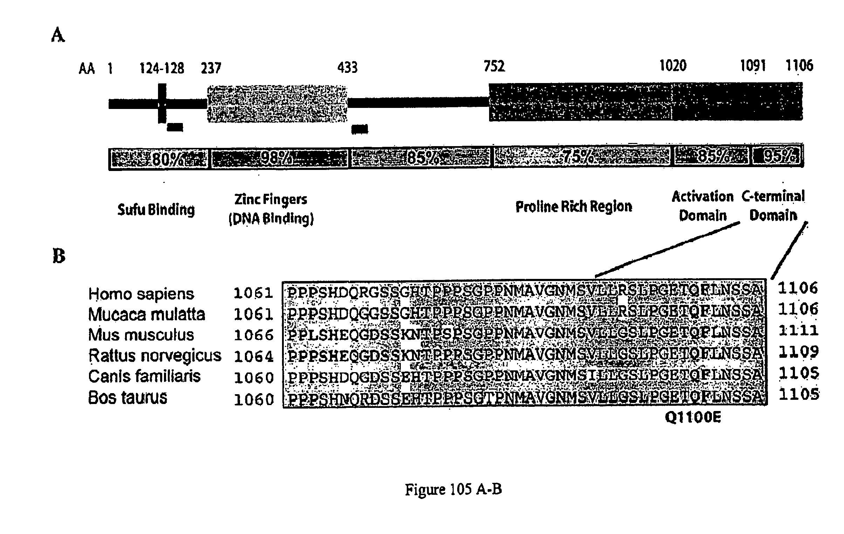

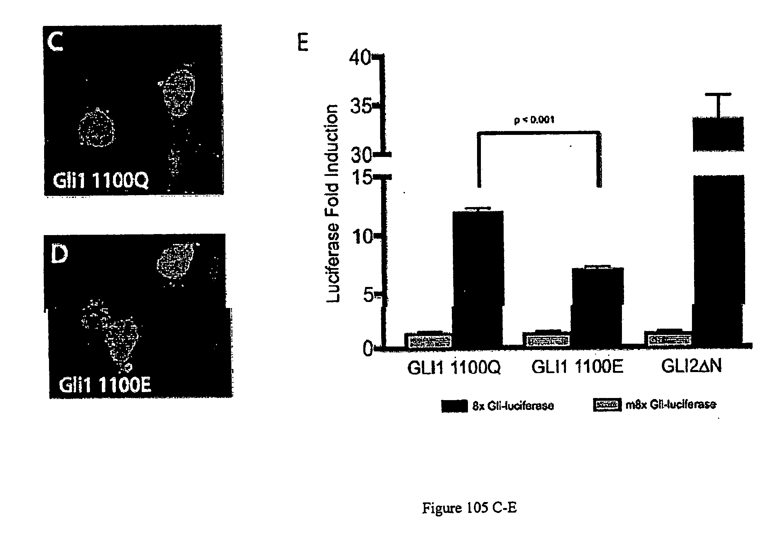

Figure 105 shows Q1100E disrupts a conserved region of the GLI1 protein and reduces GLI1 transcriptional activity. A) Conservation of known functional domains in the Gli1 protein. Previously described Sufu binding, DNA binding, and transactivation domains [Yoon et al. (1998) J Biol Chem 273: 3496-3501; 27 Kinzler et al. (1988) Nature 332:371-374; Kogerman et al. (1999) Nat Cell Biol 1: 312-319] are shown schematically. Amino acid conservation of each domain is represented numerically and by shading of the bar below the domain. Red boxes indicate regions known to regulate GLI1 protein stability [Huntzicker et al. (2006) Genes Dev 20: 276-281]. The conserved C-terminal domain that includes Q1100E is adjacent to a known transactivation domain. B) Alignment of the C-terminus of mammalian Gli1 proteins. This region (AA 1080-1106) is highly conserved in mammalian lineages. C, D) GLI1 Q1100 and E1100 have similar cellular localization in 293T cells. E) GLI1 E1100 is deficient in driving activation of the 8xGli-Luciferase reporter compared to GLI1 Q1100. Gli2ΔN is a strong activator of 8xGli-Luciferase and serves as a positive control for GLI1 activation. The m8xGli-Luciferase construct contains only mutant Gli binding sites and serves as a negative control. Data shown from 6 triplicate experiments done using two different plasmid preparations (N=18).

The mutation falls within a well conserved motif at the C-terminus of mammalian GLI1 proteins, near a recognized transactivation domain (Figure 105a) [Yoon et al. (1998) J Biol Chem 273: 3496-3501]. The Q1100 residue is itself 100% conserved in all mammals examined (Figure 105b). In order to evaluate the functional consequences of the Q1100E mutation, we transfected either GLI1 Q1100 or the variant GLI1 E1100 into 293T cells. No differences in level of expression or cellular localization were detected between these GLI1 variants; both proteins were readily detectable in the nucleus of transfected cells (Figure 105c-d). We further evaluated the ability of each variant to activate the well-characterized GLI reporter 8xGli-Luciferase [Saitsu et al. (2005) Dev Dyn 232: 282-292]. We utilized GliΔ2N, a very strong activator of 8xGli-Luciferase, as a positive control for GLI1 transcriptional activity [Roessler et al. (2005) Hum Mol Genet 14: 2181-2188]. While both GLI1 variants activated 8xGli-Luciferase above baseline, WT GLI1 Q1100 was two-fold more efficient as a transcriptional activator than the variant GLI1 E1100 (Figure 105e).

Hedgehog pathway activity is dysregulated in colonic inflammation. We have previously reported that GLI1 expression is greater in the distal compared with the proximal colon in man [Noble et al. (2008) Gut. Oct;57(10):1398-405. Epub 2008 Jun 3].

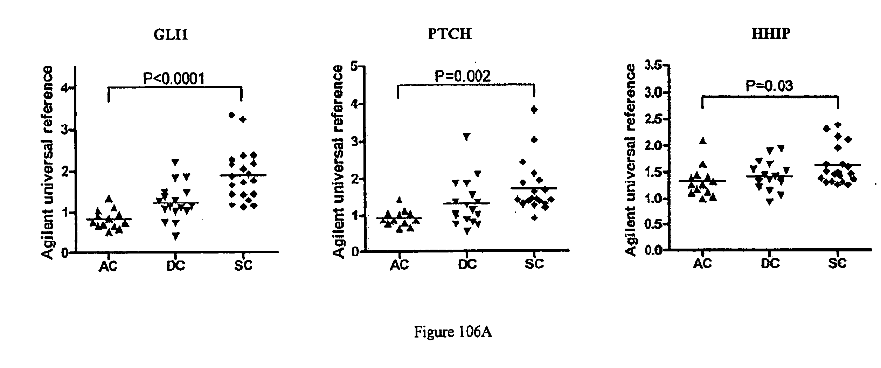

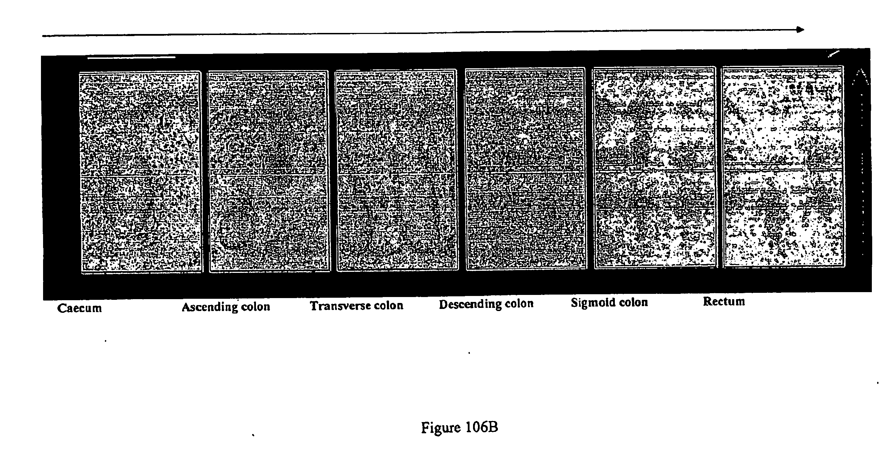

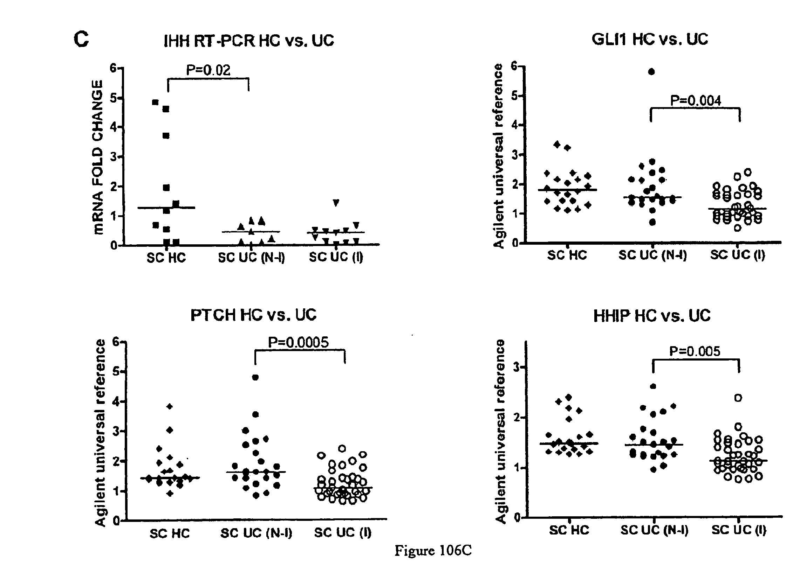

Figure 106 shows expression of hedgehog (HH) signalling components in the healthy human adult colon (HC) and ulcerative colitis (UC). A) Patched (PTCH), Hedgehog-interacting protein (HHIP), and GLI1 mRNA levels increase along the length of the healthy adult colon, from ascending colon (AC) to descending colon (DC) and sigmoid colon (SC). **B)** HH protein expression in terminally differentiated enterocytes at the luminal surface, is greater in the distal colon compared with the proximal. **C)** Quantitative analysis of mRNA levels of Indian hedgehog (IHH), PTCH, GLI1, and HHIP in UC compared with non-inflamed HC. To account for the gradients identified along the length of the healthy colon **(a-b),** the data from SC only are shown. QPCR data is presented for IHH as this gene was not present on the Agilent microarray chip. Disease specimens are sub-categorised into non-inflamed (N-I) and inflamed (I) tissues. There was no change in levels of DHH, PTCH2, GLI2, GLI3, SUFU or DISP1 in either UC or CD compared with HC, or in non-IBD inflammation (data not shown). Analysis of SHH mRNA demonstrated a mild increase in expression levels related to inflammation that is consistent with the known expression of SHH in inflammatory cells (data not shown) [Lowrey et al. (2002) J Immunol 169: 1869-1875].

Individual data points are plotted with horizontal lines representing the medians for each dataset. P-values presented are derived from Kruskal-Wallis test, comparing levels in AC, DC and SC, and from Mann-Whitney U-tests (UC vs. HC (N-I)).

Extended in silico analysis of this microarray dataset now demonstrates that mRNA transcripts of PTCH and HHIP, along with HH protein, mirror this expression gradient (Figure 106a-b). GLI1, PTCH and HHIP are pathway response elements whose expression levels predict pathway activity. GLII (p=0.0003), PTCH (p=0.002), and HHIP (p=0.0003) were lower in inflamed UC compared with HC from equivalent location (Figure 106c). IHH was lower in UC regardless of inflammation (p=0.02). GLI1 expression was lower in CD than HC (p=0.004) irrespective of inflammatory status (DATA NOT SHOWN), a noteworthy finding given that *GLI1* variation was also associated with CD in Scotland. PTCH was lower in inflamed CD compared with non-inflamed CD and HC (p=0.007). GLI1 and PTCH were both lower in non-IBD inflammation versus HC (DATA NOT SHOWN). These data demonstrate overall down-regulation of HH pathway activity, including GLI1, PTCH, and HHIP, in areas of colonic inflammation.

*Gli1+*/*-* animals exhibit mortality and heightened morbidity in response to intestinal inflammation induced by 3% DSS treatment. In vitro analysis of the GLI1 1100E variant demonstrated a 50% deficiency in transactivation function compared to WT GLI1, and our genetic analysis demonstrates an allele-specific dosage response, suggesting that a moderate reduction in GLI1 function was sufficient to predispose to intestinal inflammatory disease. To specifically test this hypothesis, we treated *Gli1*+/- mice [Park et al. (2000) Development 127: 1593-1605], and their WT littermates with 3% DSS for 6 days to induce acute intestinal inflammation. *Gli1l+*/*-* animals were rapidly and severely affected by DSS treatment.

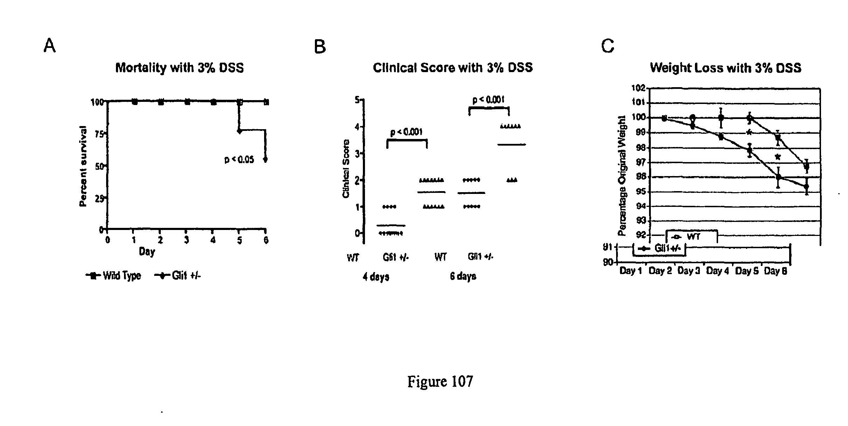

Figure 107 shows the results in which *Gli1+*/*-* animals show mortality, severe clinical symptoms, and profound weight loss after DSS treatment. A) WT animals are 100% viable over the 6 day treatment period (N=14). Nearly 50% of *Gli1+*/*-* animals (4/9) die in response to 3% DSS treatment for 6 days. **B)** *Gli1+*/*-* animals display markedly more severe symptoms than WT animals after 4 or 6 days of 3% DSS treatment. 1=diarrhoea, 2=bloody diarrhoea, 4=severe bleeding/death. Each dot represents an individual animal and the solid line shows the mean observation in each cohort. **C)** *Gli1+*/*-* animals (N=9) have lose weight more rapidly than their WT littermates (N=10). * = p <0.05

After 6 days, 4/9 had died, and 3 of the survivors demonstrated severe morbidity, with significant rectal bleeding and almost complete immobility (Figure 107a). In contrast, no WT animals (N=14) died, all were mobile and showed less morbidity on days 5 and 6 after treatment. *Gli1+*/*-* animals developed bloody diarrhoea and significant weight loss by day 4, whereas WT animals did not develop clinical signs or measurable weight loss until day 6 (Figure 107b-c).

*Gli1+*/*-* animals develop more severe colonic pathology than WT littermates in response to DSS treatment. We examined colonic tissue from *Gli1+*/*-* and WT animals taken after 4 and 6 days of DSS treatment. *Gli1+*/*-* animals developed severe tissue lesions more rapidly than WT.

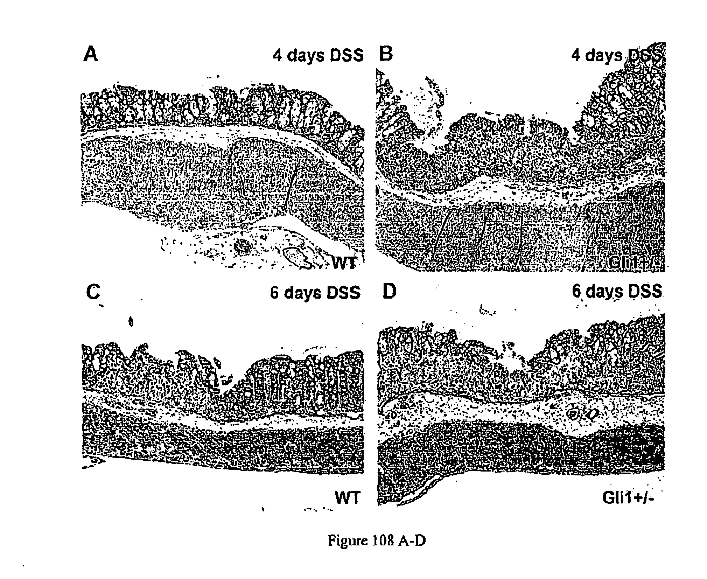

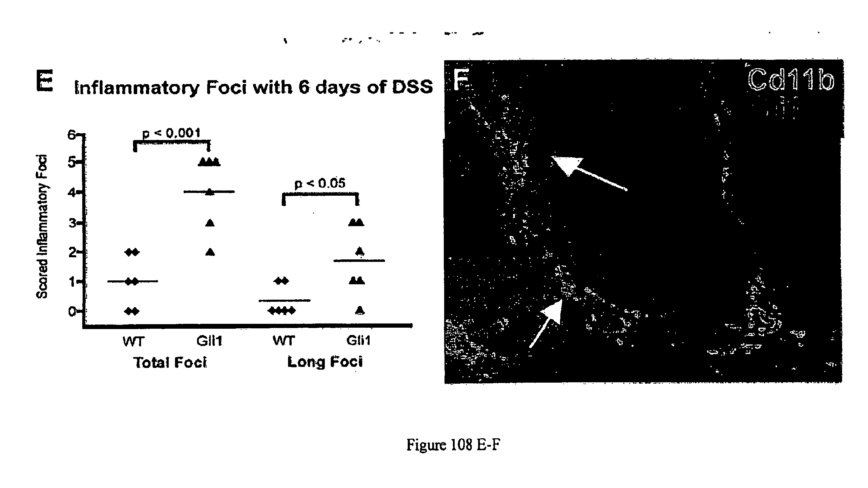

Figure 108 shows *Gli1+*/*-* animals demonstrate more severe intestinal inflammation than WT littermates in response to DSS treatment. A) WT animals (N=4) exhibit mild colonic inflammation but do not develop substantial epithelial or ulcerative inflammatory pathology within 4 days of DSS treatment. **B)** *Gli1+*/*-* animals (N=4) develop significant inflammatory infiltration, epithelial damage, and ulceration within 4 days of DSS treatment. **C-D)** *Gli1+*/*-* animals develop profound intestinal inflammation in response to 3% DSS treatment, with severe epithelial damage in long stretches of their colonic mucosa (N=9). **E)** Blinded histological scoring of colonic damage after 6 days of DSS treatment. Standard lengths of tissue from the mid colon and distal descending colon were scored in each animal. *Gli1+*/*-* animals (N=6) have more overall inflammatory foci and more long foci (10+ crypt units affected) than WT animals (N=6). Each dot represents the number of observed foci in an individual animal; the solid line shows the mean observation in each cohort. Red dots indicate the animals that were analyzed for cytokine expression. **F)** Resident mucosal myeloid cells respond directly to Hh signalling and express LacZ in the homeostatic colon of *Gli1+*/*-* animals. Arrows indicate cells with LacZ-positive nuclei and Cd11b membranes.

After 4 days, WT colons showed evidence of inflammatory change but with few destructive lesions Figure 108a), while extensive inflammatory infiltration and destructive colonic ulcers were prominent in *Gli1+*/*-* mice (Figure 108b). After 6 days, inflammation in surviving *Gli1+*/*-* animals was markedly more severe. The number (Figure 108e), size and invasiveness of inflammatory lesions (Figure 108c-e) were significantly greater in *Gli1+*/*-* animals. Taken together, these results demonstrate that the loss of a single *Gli1* allele leads to increased sensitivity to DSS treatment as reflected by severe intestinal inflammatory pathology and obvious clinical signs.

Intestinal myeloid cells respond directly to HH signals. We have demonstrated that HH signalling is exclusively paracrine in murine intestine and colon [Madison et al. (2005) Development 132: 279-28914]. Here we confirm that Gli1 is expressed in inflammatory cells in mouse, utilizing *Gli1*+/lacZ animals, which allow Hh-responsive cells to be easily visualized [Park et al. (2000) Development 127: 1593-1605]. In resting adult colon, lamina propria resident CD11b-positive myeloid cells express LacZ and are therefore responding directly to Hh signals (Figure 108e).

*Gli1+*/*-* animals have increased IL-23p19 and pro-inflammatory cytokine expression.

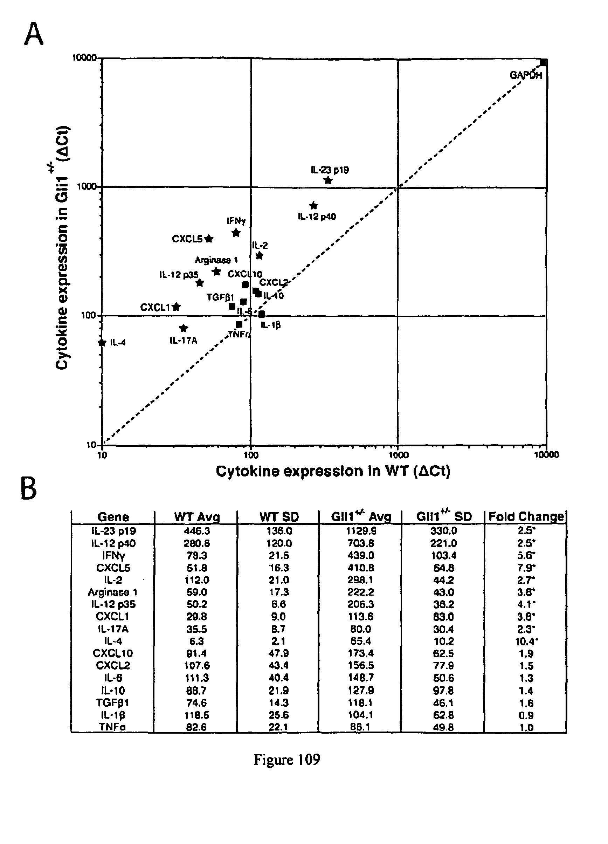

Figure 109 shows cytokine analysis of *Gli1+*/*-* and WT mice after DSS treatments demonstrates robust pro-inflammatory cytokine activation. A) Cytokine expression in WT and *Gli1+*/*-* animals (N=4) after 6 days of 3% DSS treatment. Cytokine expression normalized to GAPDH is plotted on the Y-axis for *Gli1+*/*-* mice, and on the X-axis for WT animals. Gene expression levels that are changed in a statistically significant manner are shown with stars. The dotted diagonal trendline indicates identical expression levels between WT and *Gli1*+/-mice. **B)** Table showing the average cytokine expression, standard deviation, and fold change in *Gli1+*/*-* animals compared to WT controls (* = p<0.05). Several pro-inflammatory cytokines are upregulated in *Gli1+*/*-* animals, but anti-inflammatory cytokines are largely unchanged.

QPCR examination of cytokine expression on whole colonic tissue taken after 6 days of DSS confirms the histological and clinical data; *Gli1+*/*-* animals demonstrate very significant inflammation compared to WT (Figure 109). We detect robust expression of TH1 cytokines, including IFNγ, in *Gli1+*/*-* animals, not surprising given the severity of the inflammation in these animals and the prominence of TH1 cells in DSS-induced colitis. We did not detect a significant difference in TGFβ and IL-10 between *Gli1+*/*-* and WT animals, suggesting that down-regulation of anti-inflammatory cytokines was not the primary mechanism of increased inflammation in this model. The most highly expressed cytokine in *Gli1+l-*animals was IL-23p19, which is known to drive differentiation of TH17 lymphocytes, key mediators of inflammation in several systems, including IBD [Hue et al. (2006) J Exp Med 203: 2473-2483; Yen et al. (2006) J Clin Invest 116: 1310-1316]. IL-12 and IL-17, cytokines closely associated with IL-23, were also upregulated in *Gli1+*/*-* animals. These data are particularly significant since the IL-23 pathway has recently been strongly implicated in IBD pathogenesis both in humans [Duerr et al. (2006) Science 314: 1461-1463] and mice [Yen et al. (2006) J Clin Invest 116: 1310-1316]. Our data provide a potential link between this key inflammatory pathway and the robust inflammation seen with reduced GLI1 dosage or function.

### DISCUSSION

The data presented here provide the first evidence that intact HH signalling is critical in the mammalian gut response to inflammatory challenge, and that reduced GLI1 function is implicated in IBD pathogenesis. We confirm that the HH signaling pathway is downregulated in colonic inflammation in man. We identify a specific *GLI1* variant that is highly associated with UC/IBD, and demonstrate that the variant protein is functionally deficient as a transcriptional activator in vitro. Finally, we demonstrate that a 50% reduction in murine *Gli1* results in a heightened intestinal inflammatory response to DSS with significant upregulation of the IL-23 pathway. Not only do these findings have clear implications for the understanding of IBD pathogenesis with potential for therapeutic intervention, they are the first clear description of a functional role for HH signalling and GLI1 in bowel inflammation. The inherited variation in the *GLI1* gene that we have detected is associated with IBD and UC, in both Scotland and England, with findings for rs2228226 confirmed by meta-analysis of over 5000 individuals, with odds ratio of 1.19. Evidence for an effect in CD is seen in the present study, but the predominant effect is clearly related to UC. The magnitude of this association is entirely in line with the effect size noted in a number of recent studies of complex disease genetics, including CD, colo-rectal cancer [Tenesa et al. (2008) Nat Genet 40: 631-637], and coeliac disease [Hunt et al. (2008) Nat Genet 40: 395-402]. The level of significance attained satisfies suggested criteria of p < 10⁻⁴ - 10⁻⁶ for gene-centric studies [Burton et al. (2007) Nat Genet 39: 1329-1337; Thomas et al. (2004) J Natl Cancer Inst 96: 421-423]. The three Northern European populations studied have previously demonstrated similar contribution of other IBD susceptibility genes / loci, including NOD2 and IBD5 [Gaya et al. (2006) Lancet 367: 1271-1284]. Whilst the minor allelic frequencies for this SNP are very similar in Scotland and Sweden (30.3% and 30.6%), they differ by 3.9% between Scotland and Cambridge (30.3% and 26.4%). This difference is in keeping with that noted for a number of SNPs analysed for population stratification in the recent WTCCC study [Wellcome Trust Case Control Consortium (2007) Nature 447: 661-678]. Whilst our resequencing efforts identify rs2228226 as the only coding variant associated with IBD, the haplotype analysis and log-likelihood analyses raise the possibility that other germ-line variants may also contribute to IBD risk. These need be explored formally - specifically the role of intronic variants, long-range promoter effects and / or copy number variation. In this context, several complex disease genes, including NOD2 [Hugot et al. (2001). Nature 411: 599-603; Ogura Y et al. (2001). Nature 411: 603-606], have multiple independent mutations conferring disease risk, some disease genes have no causative mutations within coding sequences (e.g. *IRGM* in CD [Parkes et al. Am J Hum Genet. 2000;67:1605-16105]), and synonymous SNPs may be associated with functional effects [Kimchi-Sarfaty et al. (2007) Science 315: 525-528]. rs2228226C→G encodes a change from glutamine to glutamic acid (Q1100E). Our in vitro data demonstrates that GLI1 1100E is a subfunctional transcriptional activator compared to WT GLI1, though it is synthesized and localized appropriately. The Q1100E mutation causes a significant charge change in a conserved region directly adjacent to the known transactivation region of GLI1; this change could directly modify transactivation activity, disrupt the structure of the transactivation domain, or affect protein stabilization [Huntzicker et al. (2006) Genes Dev 20: 276-281] decreasing activity. Our data suggest that reductions in GLI1 activity or amount also produce a robust phenotype. *Gli1+*/*-* animals, which have only 50% of the WT level of Gli1, develop severe inflammation rapidly in response to moderate stimuli. These data, to our knowledge the first description of a phenotype for reduced Gli1 function [Park et al. (2000) Development 127: 1593-1605], demonstrate the key role that a full complement of Hh response plays in protection from inflammatory disease. In addition, our in vitro data demonstrates that GLI1 E1100 is capable of activating some Gli response, suggesting that under homeostatic conditions, GLI1 E1100 could perform adequately. Similar to the situation in *Gli1+*/*-* animals, however, under conditions of inflammatory stress, GLI1 E1100 can only function at 50% of the level of WT GLI1. Whether the predisposition to inflammation in these systems is a direct result of lowered HH signal transduction within inflammatory cells or reflects the effect of lower HH signals on stromal target cells that, in turn, release signals that impact the integrity of the epithelial layer is not yet clear. Addressing this question will be a key avenue of future investigation. We have shown that the HH pathway may directly modify the innate immune response through signalling to myeloid target cells. This finding is in accordance with recent data demonstrating a crucial role for Hh signalling in myeloid cell maturation in the spleen [Varas et al., (2008) J Leukoc Biol Jun;83(6):1476-83. Epub 2008 Mar 1123]. Interestingly, myeloid cell populations can differentially modify the intestinal inflammatory milieu through a significant impact on the IL-23/IL-17 pathway [Denning et al. (2007) Nat Immunol 8: 1086-1094]. Together, our findings demonstrating direct HH response by innate immune populations and increased IL-23 after DSS treatment in Gli1+/- animals suggest that HH signalling may normally promote a tolerogenic phenotype in mucosal myeloid cells; reduced HH signal transduction may instead trigger an inflammatory response in these cells.

In conclusion, we demonstrate here for the first time the altered expression of a developmental signalling pathway in IBD, opening up novel lines of investigation. Furthermore, we show that these effects are in part genetically determined, with evidence implicating *GLI1* as an IBD2 gene, and identification of a specific variant with reduced transcriptional activity. The functional relevance of Gli1 is demonstrated by the severe intestinal inflammation that develops in the face of a 50% reduction in Gli1 concentration in an established mouse model of colitis. Taken together, these data strongly argue for the importance of a robust HH pathway activation in the protective response of the intestinal mucosa to inflammatory stimuli. These findings have important implications for the pathogenesis of UC and potentially for other forms of chronic inflammation. [Lees et al. (2006) Gastroenterology 131: 1657-1658].

### Example 5 - IBD gene expression profiles from whole blood samples

Genome wide expression profiles from human endoscopic colonic biopsies have started to help dissect out the pathogenic inflammatory pathways at the cellular level in IBD (Noble CL et al . Gut, 2008 Jun 3. [Epub ahead of print]). Whole blood genome wide expression profiles may complement conventional endoscopic techniques to help in the diagnosis of IBD- Crohn's disease (CD) and ulcerative colitis (UC). The aim of this study was to investigate whole blood gene expression profiles to try to differentiate patients with IBD- CD, UC, and controls (HC).

Patients. 21 UC, 19 CD, and 10 controls (HC) were studied. 2 of the UC patients were newly diagnosed, 15 had quiescent disease and 4 had active disease. In CD 1 new diagnosis, 11 quiescent disease and 7 active disease patients were investigated.

Methods. 41058 expression sequence tags (representing 33296 genes) were analyzed in 50 whole blood samples using the Agilent platform. Total RNA was extracted from the blood using the micro total RNA isolation from animal tissues protocol (*Qiagen, Valencia, CA*). A T7 RNA polymerase single round of linear amplification was carried out to incorporate Cyanine-3 and Cyanine-5 label into cRNA. The samples were hybridized for 18 hours at 60°C with constant rotation. Microarrays were washed, dried and scanned on the Agilent scanner according to the manufacturer's protocol. Microarray image files were analysed using Agilent's Feature Extraction software version 7.5. The genes were normalized using the *Stratagene* Universal Human Reference.

Using clustering analysis with all the IBD and HC patients, and probes that had a > or < than 1.5 fold change in expression, HC patients were more prevalent on one side of the dendrogram 1/21 v 9/29 (p=0.02 OR 1.8) (data not shown). No difference was observed in the distribution of the CD and UC samples. When all of the IBD samples were compared to controls 493 sequences had a fold change of greater than 1.5 (1.7x10-41<p<0.01) and 595 sequences had a fold change of less than 1.5 (4.0x10⁻⁴⁰<p<0.01). When CD and UC were compared, 293 sequences had a fold change of greater than 1.5 (5.4x10⁻²⁷<p<0.01) and 301 sequences had a fold change of less than 1.5 (5.2x10⁻¹⁸<p<0.01).

By using a panel of 10 of the up regulated and down regulated genes sequences we were able to predict with a >90% sensitivity IBD samples from controls. Table 12 below lists 20 differentially regulated genes in IBD when compared to controls.

**Table 12**

| Sequence Name | Sequence Code | Fold Change | P-value |
|---|---|---|---|
| LOC342959 (AARDC5) | A_32_P30271 | 4.92527 | 8.62E-18 |
| A_24_P910246 (ATXN3L) | A_24_P910246 | 2.92405 | 1.41E-07 |
| LOC92552 (FSHR) | A_23_P361744 | 2.78494 | 4.12E-13 |
| PDGFRA | A_23_P332536 | 2.7178 | 0.00004 |
| TGFB3 | A_24_P373096 | 2.67234 | 5.89E-09 |
| KCTD8 | A_23_P94902 | 2.65574 | 0.00005 |
| TGM4 | A_23_P41241 | 2.64262 | 0.0005 |
| NYD-SP25 | A_24_P309216 | 2.62905 | 7.76E-15 |
| FLJ33651 | A_24_P306032 | 2.62873 | 0.0034 |
| EMX2OS | A_24_P892472 | 2.55683 | 0.00029 |
| WNT16 | A_23_P134601 | -2.96773 | 3.46E-10 |
| SPRED2 | A_24_P315535 | -2.97751 | 3.12E-16 |
| MGC50721 (C16orf65) | A_23_P412508 | -3.06148 | 0.0088 |
| C12orf2 | A_24_P78556 | -3.17916 | 0.0129 |
| MPDZ | A_23_P396328 | -3.19437 | 1.87E-40 |
| FARS2 | A_24_P456422 | -3.35391 | 0.00097 |
| CASP8 | A_24_P157087 | -3.41412 | 3.90E-09 |
| NT5E | A_24_P316430 | -3.63046 | 0 |
| TDGF3 | A_24_P179646 | -3.71928 | 7.31E-23 |
| BTNL3 | A_23_P158297 | -5.22514 | 2.09E-12 |

Whole blood genome wide expression signature provides a starting point for differentiating between patients with IBD and controls and this may provide complimentary diagnostic evidence of the diagnosis of IBD.

### Statements of invention

1. A method of diagnosing the presence of an inflammatory bowel disease (IBD) in a mammalian subject, comprising determining that the level of expression of a nucleic acid encoding a polypeptide shown as any one of SEQ ID NOS: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 194, 197, 199, 201, 203, 205, 207, and 230 in a test sample obtained from said subject is higher relative to the level of expression in a control, wherein said higher level of expression is indicative of the presence of an IBD in the subject from which the test sample was obtained.
2. A method of diagnosing the presence of an inflammatory bowel disease (IBD) in a mammalian subject, comprising determining that the level of expression of a nucleic acid encoding a polypeptide shown as any one of SEQ ID NOS: 108, 174, 176, 178, 180, 182, 184, 186, 188, 190, 192, 211, 213, 215, 217, 219, 221, 223, 225, and 228, in a test sample obtained from said subject is lower relative to the level of expression in a control, wherein said lower level of expression is indicative of the presence of an IBD in the subject from which the test sample was obtained.
3. The method of 1 or 2 wherein said mammalian subject is a human patient.
4. The method of 3 wherein evidence of said expression level is obtained by a method of gene expression profiling.
5. The method of 3 wherein said method is a PCR-based method.
6. The method of 4 wherein said expression levels are normalized relative to the expression levels of one or more reference genes, or their expression products.
7. The method of 1 or 2 comprising determining evidence of the expression levels of at least two of said genes, or their expression products.
8. The method of 1 or 2 comprising determining evidence of the expression levels of at least three of said genes, or their expression products.
9. The method of 1 or 2 comprising determining evidence of the expression levels of at least four of said genes, or their expression products.
10. The method of 1 or 2 comprising determining evidence of the expression levels of at least five of said genes, or their expression products.
11. The method of 1 or 2 further comprising the step of creating a report summarizing said IBD detection.
12. The method of 1 or 2, wherein said IBD is ulcerative colitis.
13. The method of 1 or 2, wherein said IBD is Crohn's disease.
14. The method of 1 or 2, wherein said IBD is ulcerative colitis and Crohn's disease.
15. The method of 1 or 2, wherein said test sample is from a colonic tissue biopsy.
16. The method of 15, wherein said biopsy is from a tissue selected from the group consisting of the terminal ileum, the ascending colon, the descending colon, and the sigmoid colon.
17. The method of 15, wherein said biopsy is from an inflamed colonic area.
18. The method of 15, wherein said biopsy is from a non-inflamed colonic area.
19. The method of 1 or 2, wherein said determining step is indicative of a recurrence of an IBD in said mammalian subject, and wherein said mammalian subject was previously diagnosed with an IBD and treated for said previously diagnosed IBD.
20. The method of 19, wherein said treatment comprised surgery.
21. The method of 1 or 2, wherein said determining step is indicative of a flare-up of said IBD in said mammalian subject.
22. A method of treating an inflammatory bowel disorder (IBD) in a mammalian subject in need thereof, the method comprising the steps of
   (a) determining that the level of expression of a nucleic acid encoding a polypeptide shown as any one of SEQ ID NOS: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 194, 197, 199, 201, 203, 205, 207, and 230 in a test sample obtained from said subject is higher relative to the level of expression in a control, wherein said higher level of expression is indicative of the presence of an IBD in the subject from which the test sample was obtained; and
   (b) administering to said subject an effective amount of an IBD therapeutic agent.
23. A method of treating an inflammatory bowel disorder (IBD) in a mammalian subject in need thereof, the method comprising the steps of
   (a) determining that the level of expression of a nucleic acid encoding a polypeptide shown as any one of SEQ ID NOS: 108, 174, 176, 178, 180, 182, 184, 186, 188, 190, 192, 211, 213, 215, 217, 219, 221, 223, 225, and 228, in a test sample obtained from said subject is lower relative to the level of expression in a control, wherein said lower level of expression is indicative of the presence of an IBD in the subject from which the test sample was obtained; and
   (b) administering to said subject an effective amount of an IBD therapeutic agent.
24. The method of 22 or 23 wherein said mammalian subject is a human patient.
25. The method of 24 wherein evidence of said expression level is obtained by a method of gene expression profiling.
26. The method of 24 wherein said method is a PCR-based method.
27. The method of 25 wherein said expression levels are normalized relative to the expression levels of one or more reference genes, or their expression products.
28. The method of 22 or 23 comprising determining evidence of the expression levels of at least two of said genes, or their expression products.
29. The method of 22 or 23 comprising determining evidence of the expression levels of at least three of said genes, or their expression products.
30. The method of 22 or 23 comprising determining evidence of the expression levels of at least four of said genes, or their expression products.
31. The method of 22 or 23 comprising determining evidence of the expression levels of at least five of said genes, or their expression products.
32. The method of 22 or 23 further comprising the step of creating a report summarizing said IBD detection.
33. The method of 22 or 23, wherein said IBD is ulcerative colitis.
34. The method of 22 or 23, wherein said IBD is Crohn's disease.
35. The method of 22 or 23, wherein said IBD is ulcerative colitis and Crohn's disease.
36. The method of 22 or 23, wherein said test sample is from a colonic tissue biopsy.
37. The method of 36, wherein said biopsy is from a tissue selected from the group consisting of the terminal ileum, the ascending colon, the descending colon, and the sigmoid colon.
38. The method of 36, wherein said biopsy is from an inflamed colonic area.
39. The method of 36, wherein said biopsy is from a non-inflamed colonic area.
40. The method of 22 or 23, wherein said determining step is indicative of a recurrence of an IBD in said mammalian subject, and wherein said mammalian subject was previously diagnosed with an IBD and treated for said previously diagnosed IBD.
41. The method of 40, wherein said treatment comprised surgery.
42. The method of 22 or 23, wherein said determining step is indicative of a flare-up of said IBD in said mammalian subject.
43. The method of 22 or 23, wherein said IBD therapeutic agent is an aminosalicylate.
44. The method of 22 or 23, wherein said IBD therapeutic agent is a corticosteroid.
45. The method of 22 or 23, wherein said IBD therapeutic agent is an immunosuppressive agent.

## Claims

1. A method of diagnosing the presence of an inflammatory bowel disease (IBD) in a mammalian subject, comprising determining that the level of expression of a nucleic acid encoding a polypeptide shown as either SEQ ID NO:70 or SEQ ID NO: 78 in a test sample obtained from said subject is higher relative to the level of expression in a control, wherein said higher level of expression is indicative of the presence of an IBD in the subject from which the test sample was obtained.

2. An IBD therapeutic agent for use in a method of treating an inflammatory bowel disorder (IBD) in a mammalian subject in need thereof, the method comprising the steps of
(a) determining that the level of expression of a nucleic acid encoding a polypeptide shown as either SEQ ID NO: 70 or SEQ ID NO: 78 in a test sample obtained from said subject is higher relative to the level of expression in a control, wherein said higher level of expression is indicative of the presence of an IBD in the subject from which the test sample was obtained; and
(b) administering to said subject an effective amount of the IBD therapeutic agent.

3. The method of claim 1 or the agent for use according to claim 2 wherein said mammalian subject is a human patient.

4. The method or agent for use according to claim 3 wherein evidence of said expression level is obtained by a method of gene expression profiling.

5. The method or agent for use according to claim 3 wherein said method is a PCR-based method.

6. The method or agent for use according to claim 4 wherein said expression levels are normalized relative to the expression levels of one or more reference genes, or their expression products.

7. The method of claim 1 or the agent for use according to claim 2 comprising determining evidence of the expression levels of at least one, at least two, at least three or at least four further genes selected from the group:
(a) SEQ ID NOS: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 194, 197, 199, 201, 203, 205, 207, and 230, or its expression product, wherein a higher level of expression is indicative of the presence of an IBD in the subject from which the test sample was obtained; or
(b) SEQ ID NOS: 108, 174, 176, 178, 180, 182, 184, 186, 188, 190, 192, 211, 213, 215, 217, 219, 221, 223, 225, and 228, or its expression product, wherein a lower level of expression is indicative of the presence of an IBD in the subject from which the test sample was obtained.

8. The method or agent for use according to claim 7 wherein the expression levels of SEQ ID NOS: 70 and 78 are determined.

9. The method of claim 1 or the agent for use according to claim 2, wherein said IBD is ulcerative colitis and/or Crohn's disease.

10. The method of claim 1 or the agent for use according to claim 2, wherein said test sample is from a colonic tissue biopsy.

11. The method or agent for use according to claim 10, wherein said biopsy is from a tissue selected from the group consisting of the terminal ileum, the ascending colon, the descending colon, and the sigmoid colon, an inflamed colonic area or a non-inflamed colonic area.

12. The method of claim 1 or the agent for use according to claim 2, wherein said determining step is indicative of a recurrence of an IBD in said mammalian subject, and wherein said mammalian subject was previously diagnosed with an IBD and treated for said previously diagnosed IBD.

13. The method or agent for use according to claim 12, wherein said treatment comprised surgery.

14. The method of claim 1 or the agent for use according to claim 2, wherein said determining step is indicative of a flare-up of said IBD in said mammalian subject.

15. The agent for use according to claim 2, wherein said IBD therapeutic agent is an aminosalicylate, a corticosteroid or an immunosuppressive agent.
